# EUROPEAN PATENT SPECIFICATION

(11) **EP 3 589 733 B1**
(45) Date of publication and mention of the grant of the patent: **07.01.2026**
(21) Application number: 18711459.0
(22) Date of filing: 03.03.2018
(51) Int. Cl.: C12N 15/10, C12N 15/867, C12N 5/0783, C12N 5/10, C07K 14/725, C07K 14/705, C07K 14/715, A61K 48/00, C12N 15/86

(54) **METHODS AND COMPOSITIONS FOR TRANSDUCING AND EXPANDING LYMPHOCYTES AND REGULATING THE ACTIVITY THEREOF**
VERFAHREN UND ZUSAMMENSETZUNGEN ZUR TRANSDUKTION UND EXPANSION VON LYMPHOZYTEN UND REGULIERUNG DER AKTIVITÄT DAVON
PROCÉDÉS ET COMPOSITIONS POUR LA TRANSDUCTION ET L'EXPANSION DE LYMPHOCYTES ET LA RÉGULATION DE L'ACTIVITÉ DE CES DERNIERS

(30) Priority: 03.03.2017 US 201762467039 P; 19.03.2017 US 201715462855; 19.03.2017 WO PCT/US2017/023112; 08.07.2017 US 201715644778; 08.07.2017 WO PCT/US2017/041277; 18.09.2017 US 201762560176 P; 27.09.2017 US 201762564253 P; 28.09.2017 US 201762564991 P
(43) Date of publication of application: 08.01.2020
(73) Proprietor: Exuma Biotech Corp., West Palm Beach, FL 33401 (US)
(72) Inventor: FROST, Gregory Ian, West Palm Beach, FL 33401 (US); ONUFFER, James Joseph Jr., West Palm Beach, FL 33401 (US); GUIBINGA, Ghiabe H., West Palm Beach, FL 33401 (US); HAERIZADEH, Farzad, West Palm Beach, FL 33401 (US); ANIRBAN, Kundu, Georgetown, Grand Cayman KY1-1003 (KY)
(74) Representative: Turner, Craig Robert
(86) International application number: PCT/US2018/020818
(87) International publication number: WO 2018/161064

(56) References cited:
- WO-A1-2006/007539
- WO-A1-2012/138858
- WO-A1-2016/139463
- WO-A1-2018/009923
- WO-A1-2018/033726
- WO-A2-2017/165245
- HAIGUANG YANG ET AL: "Cell Type-Specific Targeting with Surface-Engineered Lentiviral Vectors Co-displaying OKT3 Antibody and Fusogenic Molecule", PHARMACEUTICAL RESEARCH, KLUWER ACADEMIC PUBLISHERS-PLENUM PUBLISHERS, NL, vol. 26, no. 6, 4 March 2009 (2009-03-04), pages 1432 - 1445, XP019686189, ISSN: 1573-904X, DOI: 10.1007/S11095-009-9853-Y
- VERHOEYEN ELS ET AL: "Lentiviral vector gene transfer into human T cells", ANTIBODY-DRUG CONJUGATES IN: METHODS IN MOLECULAR BIOLOGY , ISSN 1064-3745; VOL. 1045; [METHODS IN MOLECULAR BIOLOGY , ISSN 1064-3745; VOL. 1045], HUMANA PRESS, US, vol. 506, 1 January 2009 (2009-01-01), pages 97 - 114, XP009131783, ISBN: 978-1-62703-541-5
- E. VERHOEYEN ET AL: "IL-7 surface-engineered lentiviral vectors promote survival and efficient gene transfer in resting primary T lymphocytes", BLOOD, vol. 101, no. 6, 15 March 2003 (2003-03-15), US, pages 2167 - 2174, XP055272100, ISSN: 0006-4971, DOI: 10.1182/blood-2002-07-2224
- MAURICE M ET AL: "Efficient gene transfer into human primary blood lymphocytes by surface-engineered lentiviral vectors that display a T cell-activating polypeptide", BLOOD, AMERICAN SOCIETY OF HEMATOLOGY, US, vol. 99, no. 7, 1 April 2002 (2002-04-01), pages 2342 - 2350, XP002346143, ISSN: 0006-4971, DOI: 10.1182/BLOOD.V99.7.2342
- S. WILKIE ET AL: "Selective Expansion of Chimeric Antigen Receptor-targeted T-cells with Potent Effector Function using Interleukin-4", JOURNAL OF BIOLOGICAL CHEMISTRY, vol. 285, no. 33, 18 June 2010 (2010-06-18), pages 25538 - 25544, XP055125366, ISSN: 0021-9258, DOI: 10.1074/jbc.M110.127951
- ANN M LEEN ET AL: "Reversal of Tumor Immune Inhibition Using a Chimeric Cytokine Receptor", MOLECULAR THERAPY : THE JOURNAL OF THE AMERICAN SOCIETY OF GENE THERAPY, ACADEMIC PRESS ; NATURE PUBLISHING GROUP, US, vol. 22, no. 6, 1 June 2014 (2014-06-01), pages 1211 - 1220, XP002728753, ISSN: 1525-0016, DOI: 10.1038/MT.2014.47
- TILL BRIAN G ET AL: "CD20-specific adoptive immunotherapy for lymphoma using a chimeric antigen receptor with both CD28 and 4-1BB domains: pilot clinical trial results.", BLOOD, vol. 119, no. 17, 26 April 2012 (2012-04-26), pages 3940 - 3950, XP002771432, ISSN: 1528-0020

## Description

### SEQUENCE LISTING

This application hereby incorporates the material of the electronic Sequencing Listing filed concurrently herewith. The materials in the electronic Sequence Listing is submitted as a text (.txt) file entitled "F1_001_WO_03_Sequence_Listing_2018_03_03.txt" created on March 3, 2018, which has a file size of 526 KB, and is herein incorporated in its entirety.

### FIELD OF INVENTION

This disclosure relates to the field of immunology, or more specifically, to the genetic modification of T lymphocytes or other immune cells, and methods of making replication incompetent recombinant retroviral particles and controlling the expression of genes therein.

### BACKGROUND OF THE DISLOSURE

Lymphocytes isolated from a subject (e.g. patient) can be activated *in vitro* and genetically modified to express synthetic proteins that enable redirected engagement with other cells and environments based upon the genetic programs incorporated. An example of such a synthetic protein is a chimeric antigen receptor (CAR). One CAR that is currently used is a fusion of an extracellular recognition domain (e.g., an antigen-binding domain), a transmembrane domain, and one or more intracellular signaling domains encoded by a replication incompetent recombinant retrovirus.

While recombinant retroviruses have shown efficacy in infecting non-dividing cells, resting CD4 and CD8 lymphocytes are refractory to genetic transduction by these vectors. To overcome this difficulty, these cells are typically activated *in vitro* using stimulation reagents before genetic modification with the CAR gene vector can occur. Following stimulation and transduction, the genetically modified cells are expanded *in vitro* and subsequently reintroduced into a lymphodepleted patient. Upon antigen engagement *in vivo,* the intracellular signaling portion of the CAR can initiate an activation-related response in an immune cell and release of cytolytic molecules to induce tumor cell death.

Such current methods require extensive manipulation and manufacturing of proliferating T cells outside the body prior to their reinfusion into the patient, as well as lymphodepleting chemotherapy to free cytokines and deplete competing receptors to facilitate T cell engraftment. Such CAR therapies further cannot be controlled for propagation rate *in vivo* once introduced into the body, nor safely directed towards targets that are also expressed outside the tumor. As a result, CAR therapies today are typically infused from cells expanded *ex vivo* from 12 to 28 days using doses from 1 x 10⁵ to 1 x 10⁸ cells/kg and are directed towards targets, for example tumor targets, for which off tumor on target toxicity is generally acceptable. These relatively long *ex vivo* expansion times create issues of cell viability and sterility, as well as sample identity in addition to challenges of scalability. Thus, there are significant needs for a safer, more effective scalable T cell or NK cell therapy.

Since our understanding of processes that drive transduction, proliferation and survival of lymphocytes is central to various potential commercial uses that involve immunological processes, there is a need for improved methods and compositions for studying lymphocytes. For example, it would be helpful to identify methods and compositions that can be used to better characterize and understand how lymphocytes can be genetically modified and the factors that influence their survival and proliferation. Furthermore, it would be helpful to identify compositions that drive lymphocyte proliferation and survival. Such compositions could be used to study the regulation of such processes. In addition to methods and compositions for studying lymphocytes, there is a need for improved viral packaging cell lines and methods of making and using the same. For example, such cell lines and methods would be useful in analyzing different components of recombinant viruses, such as recombinant retroviral particles, and for methods that use packaging cells lines for the production of recombinant retroviral particles. WO2016/139463 describes a retroviral or lentiviral vector having a viral envelope which comprises: (i) a mitogenic T-cell activating transmembrane protein which comprises a mitogenic domain and a transmembrane domain; and/or (ii) a cytokine-based T-cell activating transmembrane protein which comprises a cytokine domain and a transmembrane domain, wherein the mitogenic or cytokine-based T-cell activating transmembrane protein is not part of a viral envelope glycoprotein.

### SUMMARY

The present invention is as set out in claims 1 to 16 appended hereto. References to embodiments and aspects and the like in the text which follows which are not encompassed by appended claims 1 to 16 do not form part of the claimed invention but may assist in the understanding thereof. Provided herein are methods, compositions, and kits that help overcome issues related to the effectiveness and safety of methods for transducing and/or genetically modifying lymphocytes such as T cells and/or NK cells. Certain such methods are useful for performing adoptive cell therapy with these cells. Accordingly, in some aspects, provided herein are methods, compositions, and kits for genetically modifying and/or transducing lymphocytes, especially T cell and/or NK cells, and/or for regulating the activity of transduced and/or genetically modified T cells and/or NK cells. Such methods, compositions, and kits provide improved efficacy and safety over current technologies, especially with respect to T cells and/or NK cells that express chimeric antigen receptors (CARs), and in illustrative embodiments microenvironment restricted biologic CARs. Transduced and/or genetically modified T cells and/or NK cells that are produced by and/or used in methods provided herein, include functionality and combinations of functionality, in illustrative embodiments delivered from retroviral (e.g. lentiviral) genomes via retroviral (e.g. lentiviral) particles, that provide improved features for such cells and for methods that utilize such cells, such as research methods, commercial production methods, and adoptive cellular therapy. For example, such cells can be produced in less time ex vivo, and that have improved growth properties that can be better regulated.

Provided herein in some aspects are regulatory elements for regulating the expression of CARs, mRNA, inhibitory RNA(s), and/or lymphoproliferative elements, for example chimeric lymphoproliferative elements, in lymphocytes such as B cells, T cells and NK cells. Furthermore, provided herein in some aspects are recombinant retroviruses that express various functional elements and that carry various functional elements on their surface, and methods and packaging cell lines for producing the recombinant retroviruses. These recombinant retroviruses and methods and cells for producing the same, overcome prior art limitations with respect to the number and size in a genome, of different functional elements that provide benefits when delivered into a T cell and/or NK cells.

In some aspects, methods are provided for transducing and/or genetically modifying lymphocytes such as T cells and/or NK cells, and in illustrative embodiments, *ex vivo* methods for transducing and/or genetically modifying resting T cells and/or NK cells. Some of these aspects can be performed much more quickly than previous methods, which can facilitate more efficient research, more effective commercial production, and improved methods of patient care. Furthermore, provided herein are methods that in some embodiments utilize recombinant retroviruses provided herein in some aspects along with pharmacologic agents, to provide improved safety mechanisms to help modulate the activity of transduced and/or genetically modified lymphocytes such as T cells and/or NK cells. Such methods, compositions, and kits can be used as research tools, in commercial production, and in adoptive cellular therapy with transduced and/or genetically modified T cells and/or NK cells expressing a CAR.

Further details regarding aspects and embodiments of the present disclosure are provided throughout this patent application. Sections and section headers are not intended to limit combinations of methods, compositions, and kits or functional elements therein.

### BRIEF DESCRIPTION OF THE DRAWINGS

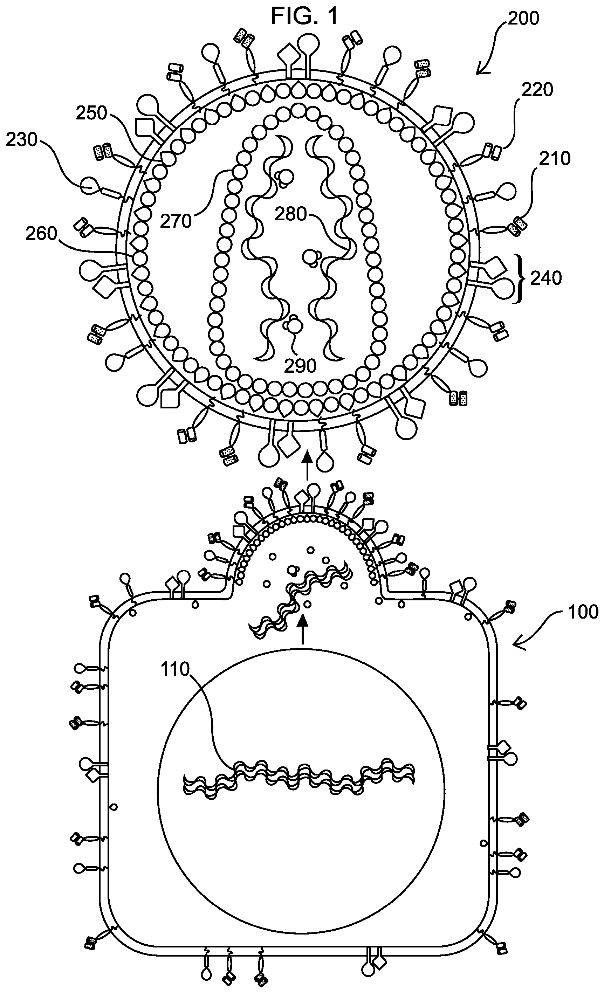

FIG. 1 shows a schematic of illustrative compositions including a packaging cell (100) and a replication incompetent recombinant retroviral particle (200) of one exemplary, non-limiting embodiment of the present disclosure, produced by the packaging cell (100). In FIG. 1, various vectors (referred to as recombinant polynucleotides (110)) capable of encoding aspects of the invention are packaged into a recombinant retroviral particle (200) that includes in its genome a first engineered signaling polypeptide that includes one or more lymphoproliferative elements and in some embodiments, a second engineered signaling polypeptide that is a chimeric antigen receptor, or a CAR. The replication incompetent recombinant retroviral particle expresses on its membrane, a pseudotyping element (in a non-limiting embodiment, a Measles Virus hemagglutinin (H) polypeptide and a Measles Virus fusion (F) polypeptide, or cytoplasmic domain deletion variants thereof) (240) that allows the replication incompetent recombinant retroviral particle to bind to and fuse with a target cell; an activation element (in non-limiting embodiments an activation element that has a polypeptide capable of binding to CD28 and a polypeptide capable of binding to CD3) (210 and 220, respectively) that is capable of binding to and activating a resting T cell; and a membrane-bound cytokine (in a non-limiting embodiment, an IL-7 DAF fusion polypeptide) (230). Parts labeled as (250), (260), (270), (280), and (290) are the Src-FLAG-Vpx, HIV gag matrix, HIV gag capsid, RNA, and HIV pol, respectively.

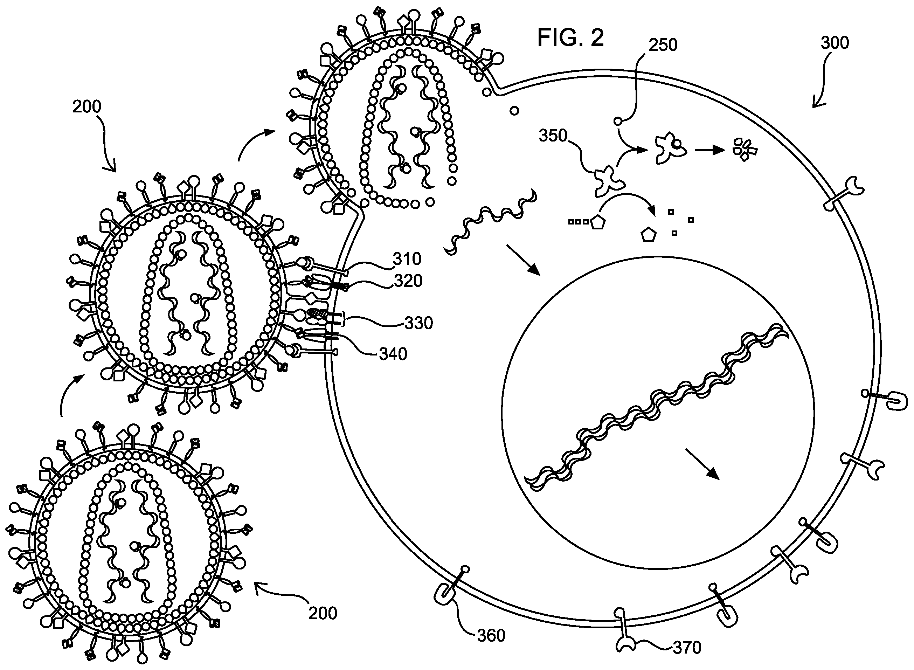

FIG. 2 shows a schematic of illustrative compositions including a replication incompetent recombinant retroviral particle (200), produced by a packaging cell (100) and a resting T cell (300) transfected by the replication incompetent recombinant retroviral particle (200). The elements on the surface of the replication incompetent recombinant retroviral particle (200), bind to receptors and/or ligands on the surface of a resting T cell. The pseudotyping element can include, in non-limiting embodiments, a binding polypeptide and a fusogenic polypeptide (in non-limiting embodiments, a Measles Virus hemagglutinin (H) polypeptide and a Measles Virus fusion (F) polypeptide, or cytoplasmic domain deletion variants thereof) that facilitate the binding and fusion of the replication incompetent recombinant retroviral particle (200), to the T cell. In non-limiting embodiments, the replication incompetent recombinant retroviral particle (200), includes on its surface an activation element (in non-limiting embodiments an activation element that has a polypeptide capable of binding to CD28 and a polypeptide capable of binding to CD3) that is capable of activating the resting T cell by engaging the T-cell receptor complex and optionally a co-receptor (320). Furthermore, membrane-bound cytokines (in non-limiting embodiments, an IL-7 DAF fusion polypeptide) present on the surface of the replication incompetent recombinant retroviral particle (200), bind to IL-7Rα (310) on the surface of the resting T cell. The replication incompetent recombinant retroviral particle (200), fuses with the T cell, and polynucleotides that encode the first engineered signaling polypeptide that includes the lymphoproliferative element (in illustrative embodiments, a constitutively active IL-7Rα) (370), are reverse transcribed in the cytosol prior to migrating to the nucleus to be incorporated into the DNA of the activated T cell. Not to be limited by theory, in some non-limiting embodiments, Src-FLAG-Vpx (250) packaged with the virus enters the cytosol of the resting T cells and promotes the degradation of SAMHD1 (350), resulting in an increased pool of cytoplasmic dNTPs available for reverse transcription. In some embodiments, the polynucleotides can also encode a second engineered signaling polypeptide that includes a CAR (360). In some embodiments, the lymphoproliferative element is expressed when a compound binds to a control element that regulates its expression (in non-limiting example, the control element is a riboswitch that binds a nucleoside analog). In some embodiments, expression of the CAR is also regulated by the control element. Part (330) is SLAM and CD46. Part (340) is CD3.

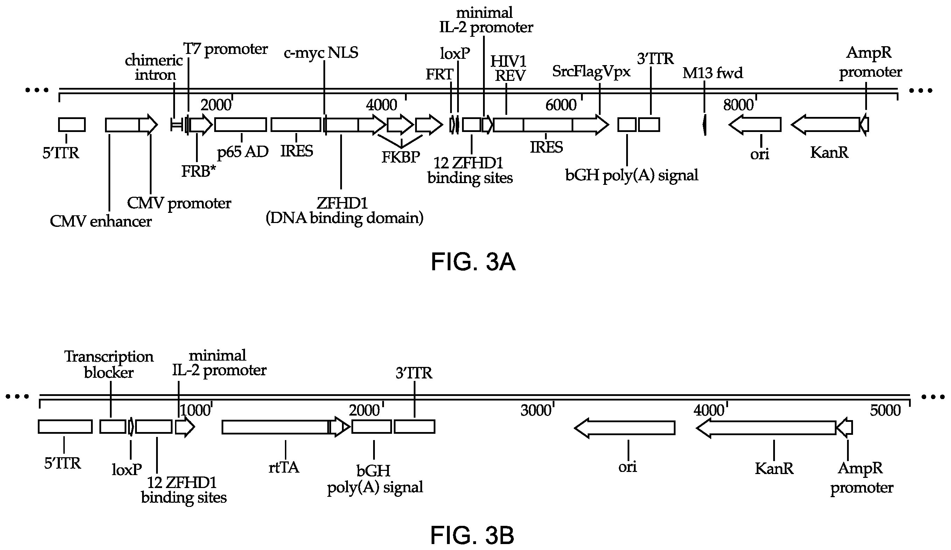

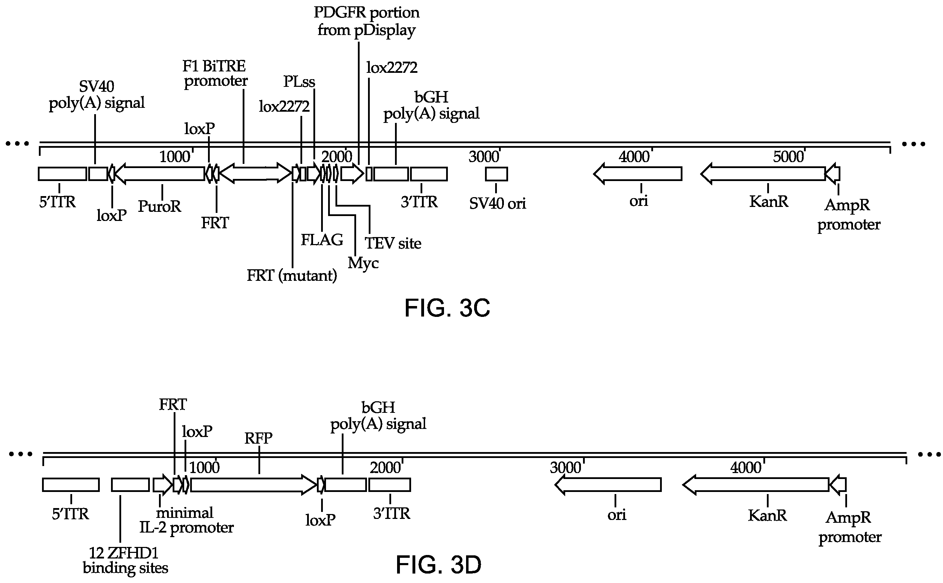

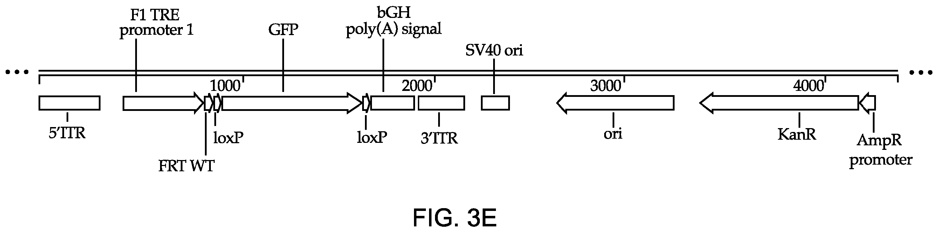

FIGs. 3A-3E show schematics of non-limiting, exemplary vector constructs for transfecting packaging cells to produce replication incompetent recombinant retroviral particles described herein. FIG. 3A shows a construct containing a polynucleotide sequence encoding an FRB domain fused to the NFκB p65 activator domain (p65 AD) and ZFHD1 DNA binding domain fused to three FKBP repeats that is constitutively expressed. The construct in FIG. 3A also includes HIV1 REV and Vpx as a SrcFlagVpx fusion under the rapamycin-inducible ZFHD 1/p65 AD promoter. FIG. 3B shows a construct containing a polynucleotide encoding an rtTA sequence under the control of the ZFHD1/p65 AD promoter. FIG. 3C shows a construct containing a polynucleotide encoding a puromycin resistance gene flanked by loxP sites and the extracellular MYC tag flanked by lox2272 sites. Both selectable markers are under the control of a BiTRE promoter, which is flanked by FRT sites. FIG. 3D shows a construct that contains a polynucleotide encoding RFP flanked by loxP sites that is under the control of a TRE promoter and a single FRT site between the TRE promoter and the 5' loxP site of RFP. FIG. 3E shows a construct containing a polynucleotide encoding GFP flanked by loxP sites that is under the control of the TRE promoter and a single FRT site between the TRE promoter and the 5' loxP site of GFP. The constructs in FIGs. 3C-3E function as landing pads for other polynucleotide sequences to insert into the genome of the packaging cell line.

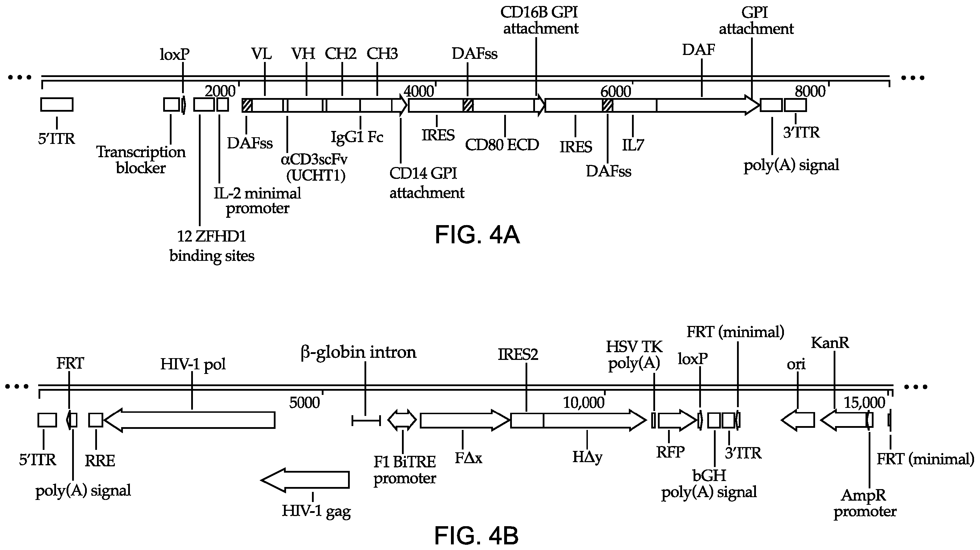

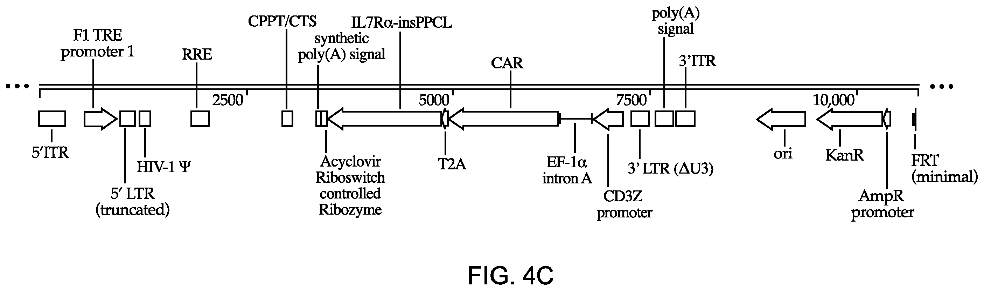

FIGs. 4A-4C show schematics of non-limiting, exemplary vector constructs for transfecting packaging cells to produce replication incompetent recombinant retroviral particles described herein. Fig. 4A shows a construct containing a tricistronic polynucleotide encoding anti-CD3 (clone UCHT1) scFvFc with a CD14 GPI anchor attachment site, CD80 extra cellular domain (ECD) capable of binding CD28 with a CD16B GPI anchor attachment site, and IL-7 fused to decay-accelerating factor (DAF) with transposon sequences flanking the polynucleotide region for integration into the HEK293S genome. FIG. 4B shows a construct containing a polynucleotide with a BiTRE promoter and a polynucleotide region encoding the gag and pol polypeptides in one direction and a polynucleotide region encoding the measles virus FΔx and HΔy proteins in the other direction. FIG. 4C shows a construct containing a polynucleotide sequence encoding a CAR and the lymphoproliferative element IL7Rα-insPPCL under the control of a CD3Z promoter which is not active in HEK293S cells, wherein the CAR and IL7Rα-insPPCL are separated by a polynucleotide sequence encoding a T2A ribosomal skip sequence and the IL7Rα-insPPCL has an acyclovir riboswitch controlled ribozyme. The CAR-containing construct further includes cPPT/CTS, an RRE sequence, and a polynucleotide sequence encoding HIV-1 Psi (Ψ). The entire polynucleotide sequence on the CAR-containing construct to be integrated into the genome is flanked by FRT sites.

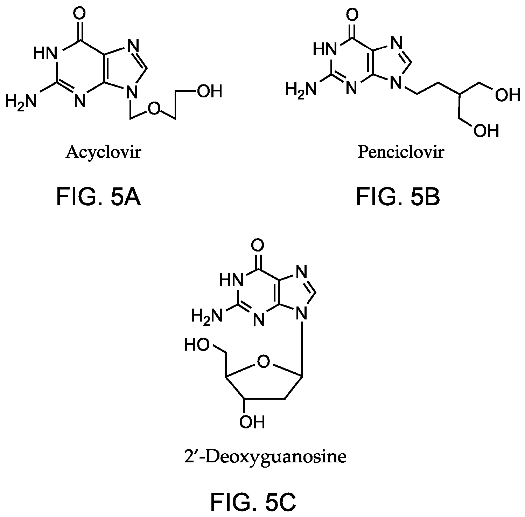

FIGs. 5A-5C show molecular structures of acyclovir (FIG. 5A), penciclovir (FIG. 5B), and 2'-deoxyguanonsine (FIG. 5C) as representative nucleoside analogues for selective riboswitch control.

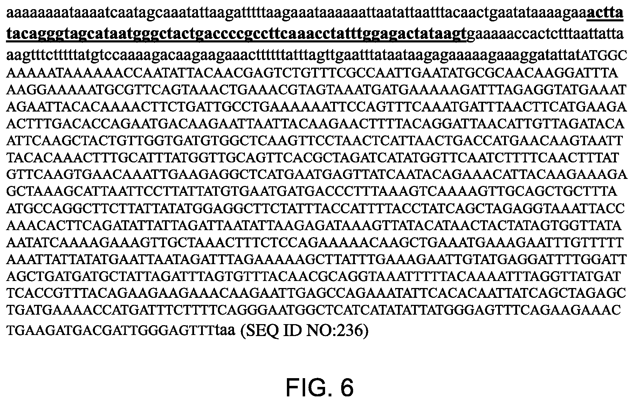

FIG. 6 represents the *Mesoplasma florum* type I-A deoxyguanosine riboswitch regulatory region and associated gene product. The sequence is the reverse complement of *M*. *florum* L1 genomic DNA (AE017263.1) nt624396 to nt625670 which is same as *M*. *florum* W37 genomic DNA (CP006778.1) nt636277 to nt 637550. The deoxyguanosine binding aptamer sequence used for initial screen indicated in bold and underline. The downstream gene product (Ribonucleotide reductase of class Ib (aerobic), beta subunit) is indicated in capital letters.

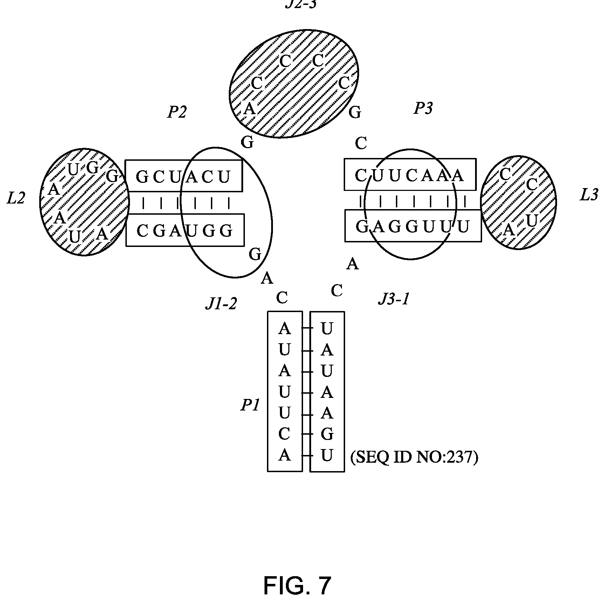

FIG. 7 represents the *M*. *florum* type I-A deoxyguanosine riboswitch aptamer regions targeted for directed evolution strategy. Nucleotides within empty ovals were targeted for randomization. Nucleotides within striped ovals were targeted for insertion/deletion and randomization.

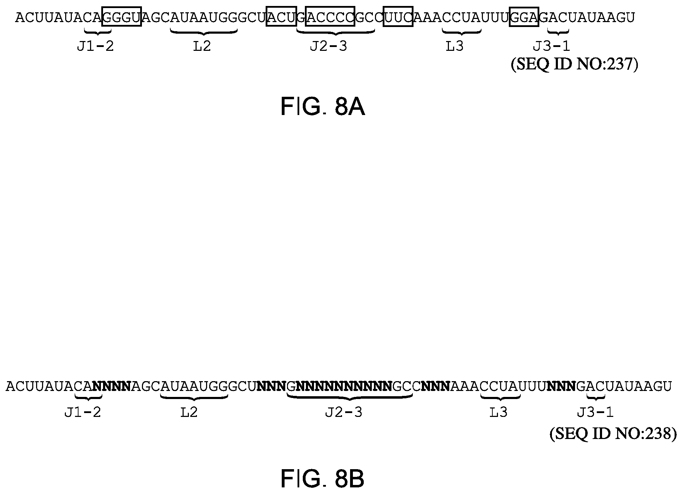

FIGs. 8A and 8B represent the *M*. *florum* type I-A deoxyguanosine riboswitch aptamer screening library. In FIG. 8A, nucleotides within boxes with solid lines are sequence regions targeted for randomization and nucleotides within boxes with dashed lines are sequence regions targeted for insertion/deletion and randomization. FIG. 8B shows possible sequences generated through mutation ("random nucleotides ("N")) and deletion/insertion.

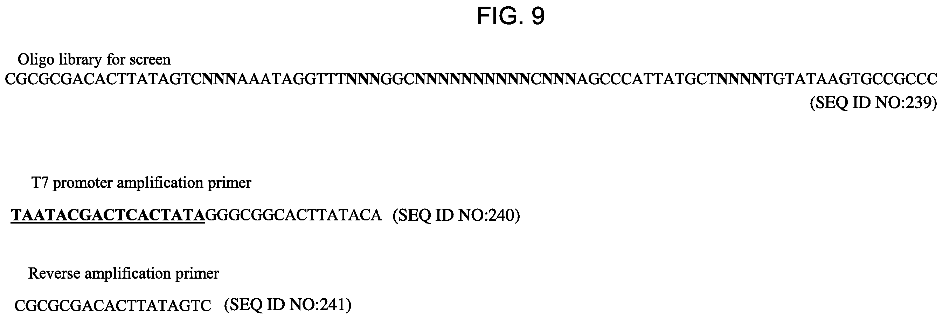

FIG. 9 represents the *M*. *florum* type I-A deoxyguanosine riboswitch aptamer oligo library synthesized as a reverse complement with additional base pairs added to allow for PCR amplification and T7 promoter addition for *in vitro* transcription for library screening. The corresponding T7 promoter amplification primer and reverse amplification primer are also shown.

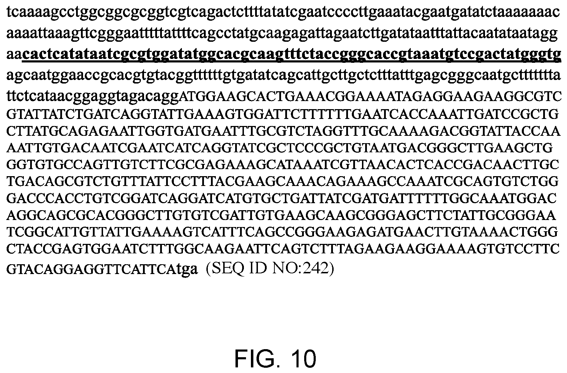

FIG. 10 represents the *Bacillus subtilis* guanosine xpt riboswitch regulatory region and associated gene product. The sequence is the reverse complement of *B. subtilis* subsp. *subtilis* 6051-HGW genomic DNA (CP003329.1) nt2319439 to nt2320353. The guanosine binding aptamer sequence used for initial screen indicated in bold and underline. The downstream gene product (Xanthine phosphoribosyltransferase xpt) is indicated in capital letters.

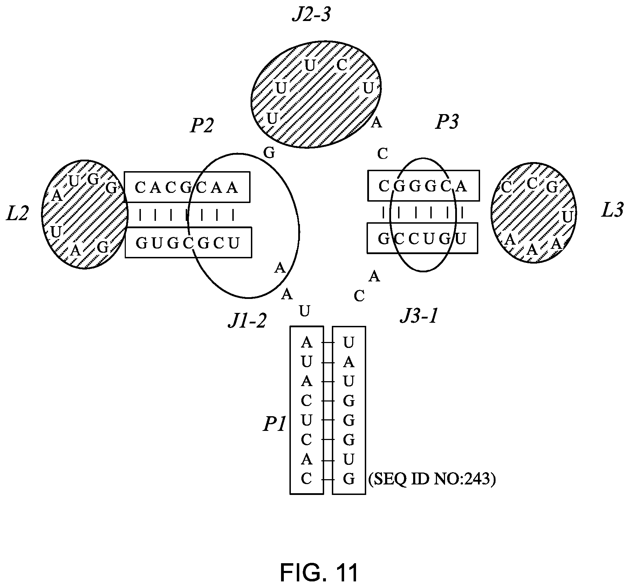

FIG. 11 represents the *B. subtilis* guanosine xpt riboswitch aptamer regions targeted for directed evolution strategy. Nucleotides within empty ovals were targeted for randomization. Nucleotides within striped ovals were targeted for insertion/deletion and randomization.

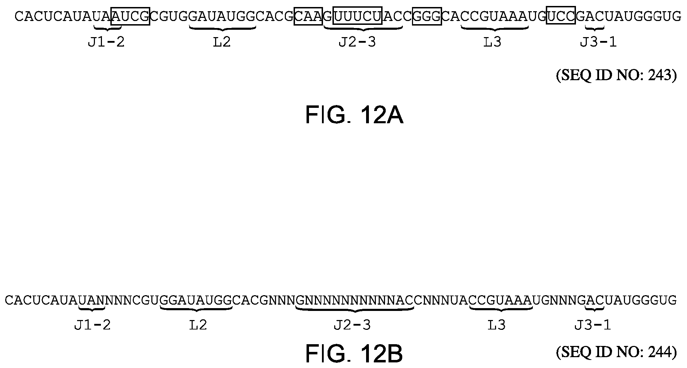

FIGs. 12A and 12B represent the *B. subtilis* guanosine xpt riboswitch aptamer screening library. In FIG. 12A, nucleotides within boxes with solid lines are sequence regions targeted for randomization and nucleotides within boxes with dashed lines are sequence regions targeted for insertion/deletion and randomization. FIG. 12B shows possible sequences generated through mutation (random nucleotides ("N")) and deletion/insertion.

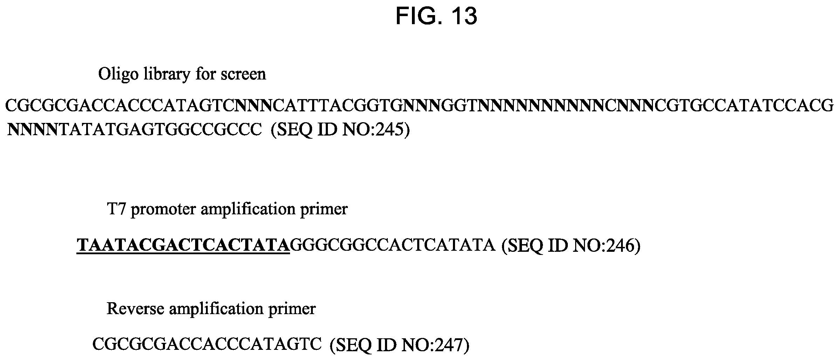

FIG. 13 represents the *B. subtilis* guanosine xpt riboswitch aptamer oligo library synthesized as a reverse complement with additional base pairs added to allow for PCR amplification and T7 promoter addition for *in vitro* transcription for library screening. The corresponding T7 promoter amplification primer and reverse amplification primer are also shown.

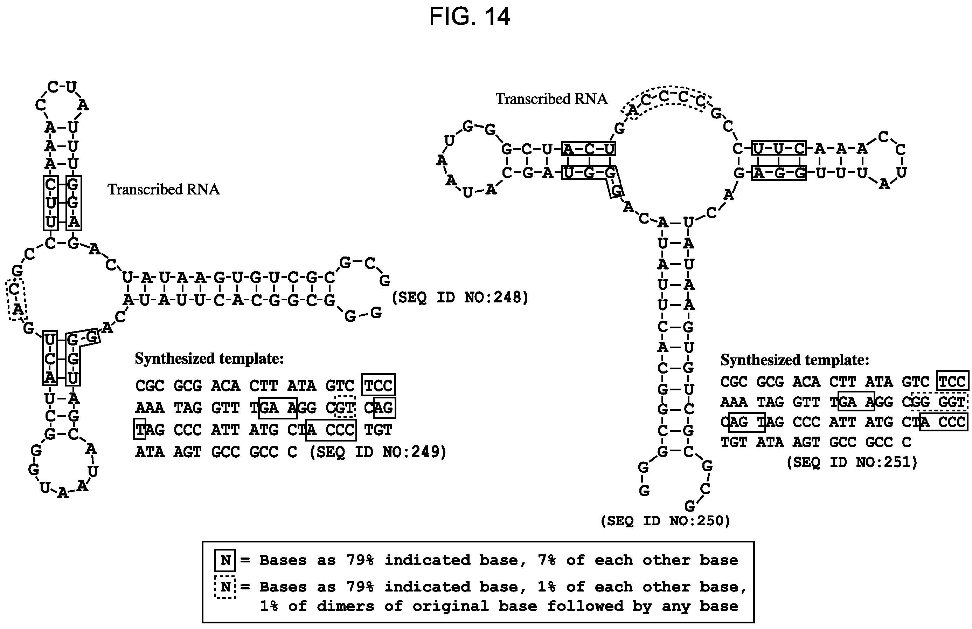

FIG. 14 shows the selection library construction. The library was constructed on the basis of known guanosine- and deoxyguanosine-binding RNA (Pikovskaya, 2013).

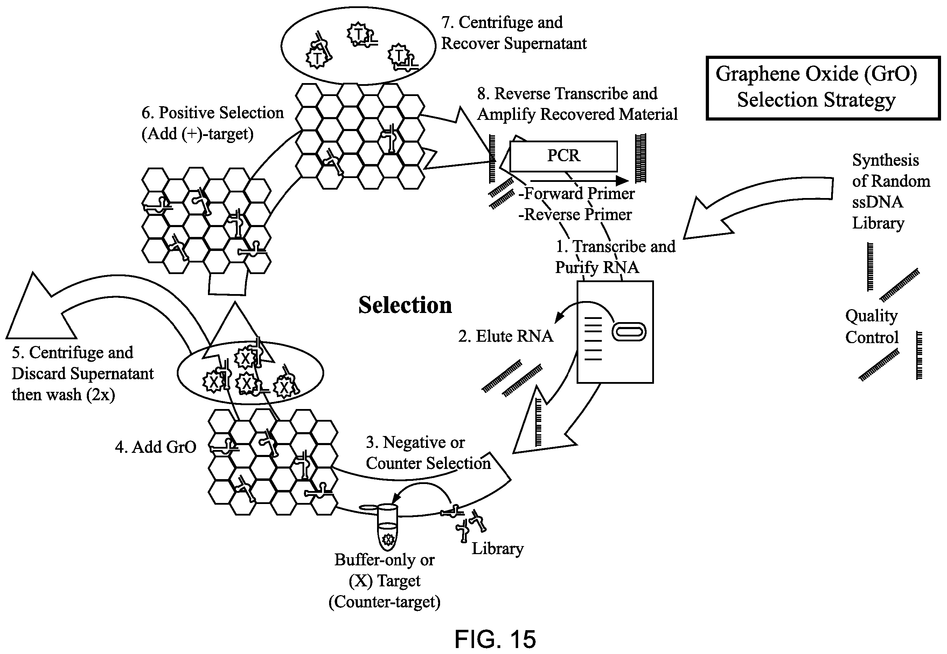

FIG. 15 shows an illustration of graphene oxide (GrO) aptamer selection. In step (1), RNA was transcribed and purified. In step (2), purified RNA was eluted. In step (3), aptamers were incubated with counter-targets and buffer. In step (4), sequences bound to counter-targets or buffer components were removed with graphene oxide. In step (5), centrifugation partitioned the non-specifically-responsive species within the supernatant, which is then discarded. Two additional 5-minute washes removed most of the residual counter-target-binding and buffer-binding sequences. In step (6), a solution of acyclovir in 1X selection buffer was added to the GrO-bound library for positive selection so potential aptamer sequences desorb from the GrO through interaction with the positive target. In step (7), a final centrifugation step separates the target-binding sequences in the supernatant from the non-responsive sequences still adsorbed to the GrO. In step (8) selected sequences were reverse-transcribed, then the library was amplified through PCR, then transcribed to generate library for the next selection round.

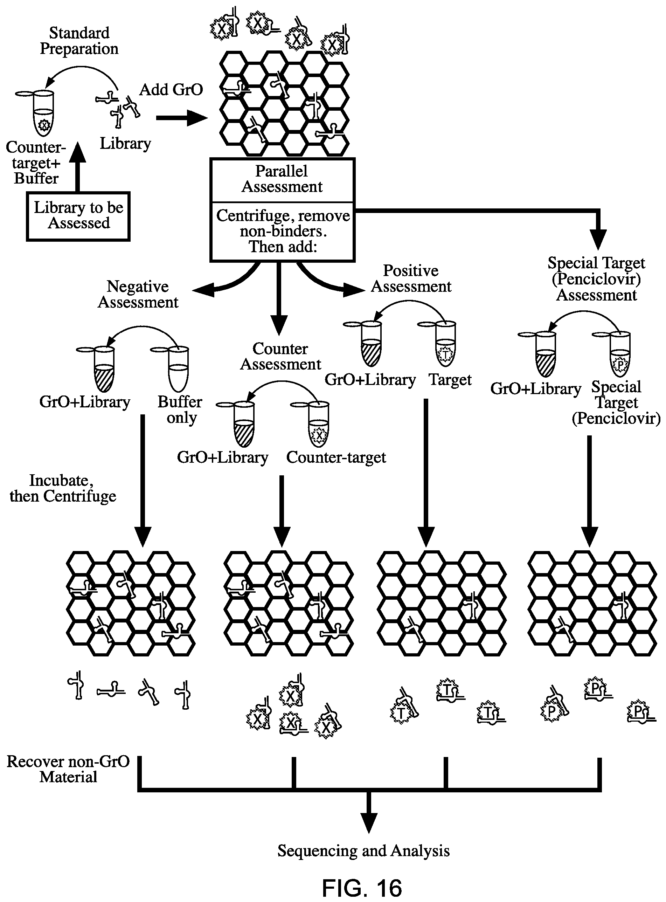

FIG. 16 shows an illustration of graphene oxide parallel assessment. Enriched libraries undergoing parallel assessment were divided into four equal portions. Library samples were then added to graphene oxide and allowed to incubate to load the library on the graphene oxide. Two 5-minute washes were used to remove non-binding material. For the positive (acyclovir) and special target (penciclovir) sample, each target was prepared separately in 1X selection buffer to 1 µM; the counter target replaced the positive target with 10 µM of each counter-target in solution; the negative sample replaced the positive target with an equal volume of nuclease-free water. Samples were then combined with their respective graphene oxide preparations and incubated. Post-incubation, samples were centrifuged to recover their supernatants, and library recovery was determined by NanoDrop-1000 spectrophotometer reading (Thermo Fisher Scientific; Wilmington, DE). Remaining library sample was analyzed on denaturing PAGE. Images of the gels were taken after staining/destaining with Gel-Star. Bands corresponding to expected library size were recovered for a follow-up round of parallel assessment, with positive target acyclovir replacing counter-targets for the negative, counter, and special target samples' pre-loading incubation. Material recovered from the second parallel assessment was used for sequencing and analysis.

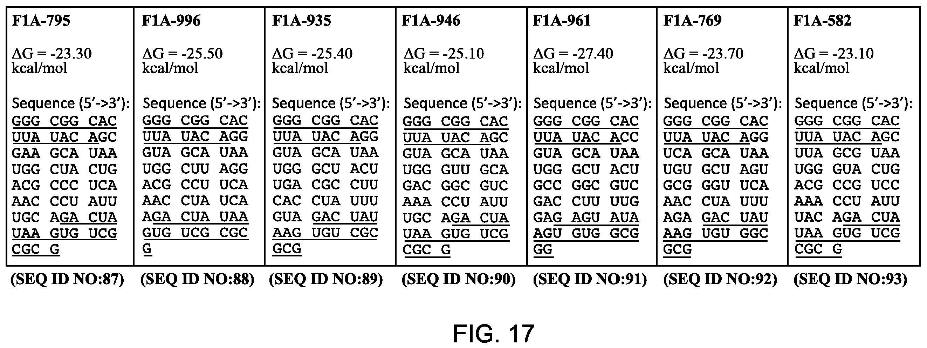

FIG. 17 shows seven aptamer candidates against acyclovir. The free energy for each aptamer was computed at 37 °C and 1 M Na+ by Quikfold 3.0 (Zuker 2003). Sequences were identified using proprietary algorithms. The underlined regions in each sequence are the PCR primer annealing regions.

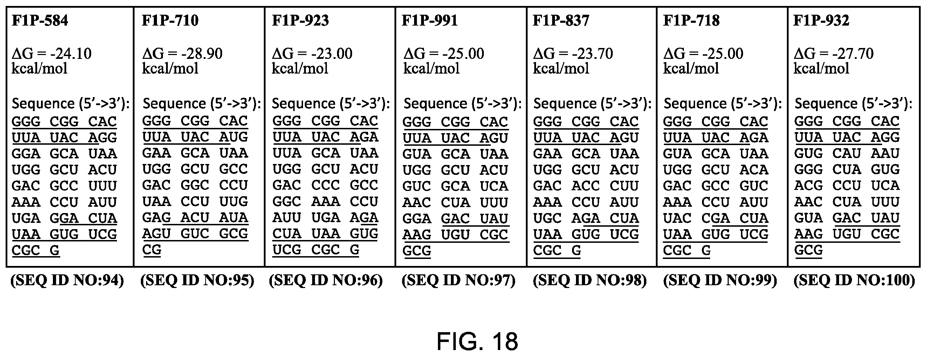

FIG. 18 shows seven aptamer candidates against penciclovir. The free energy for each aptamer was computed at 37 °C and 1 M Na+ by Quikfold 3.0 (Zuker 2003). Sequences were identified using proprietary algorithms. The underlined regions in each sequence are the PCR primer annealing regions.

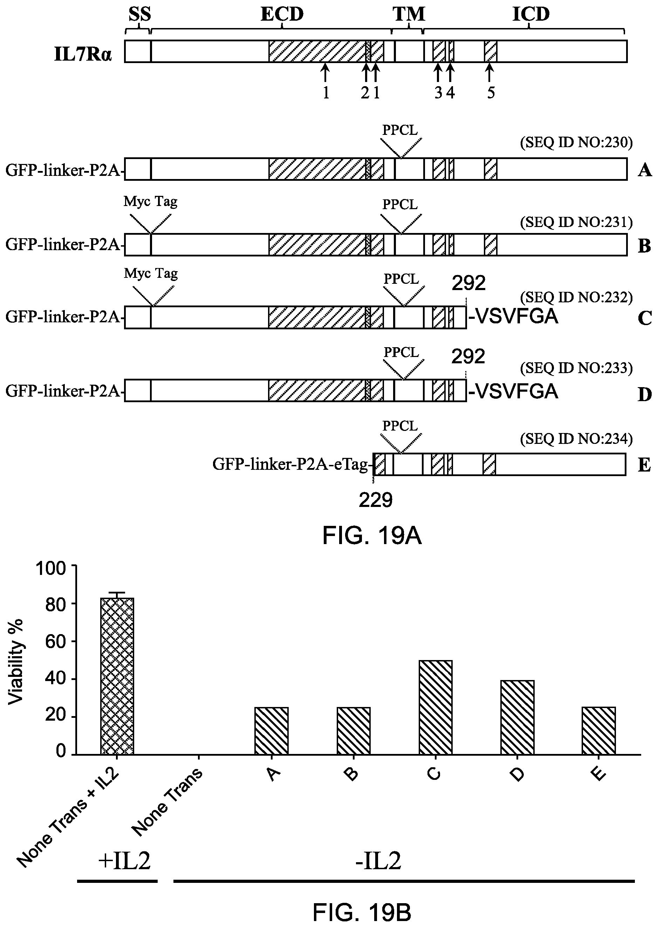

FIG. 19A provides a schematic of IL7Rα variants tested for lymphoproliferative/survival activity when expressed in PBMCs. FIG. 19B provides a bar graph showing percent viability of PBMCs in the presence and absence of IL-2.

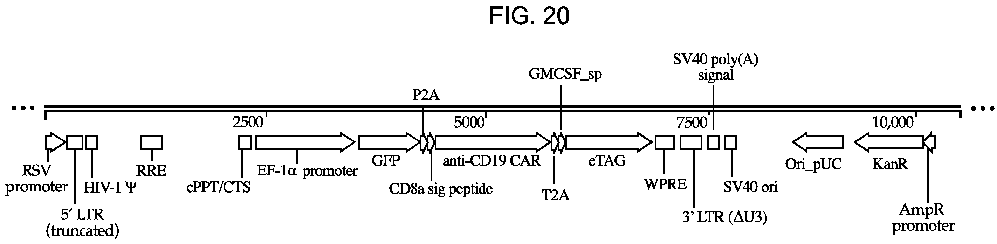

FIG. 20 shows a schematic of the lentiviral expression vector encoding GFP, an anti-CD19 chimeric antigen receptor, and an eTAG referred to herein as F1-0-03.

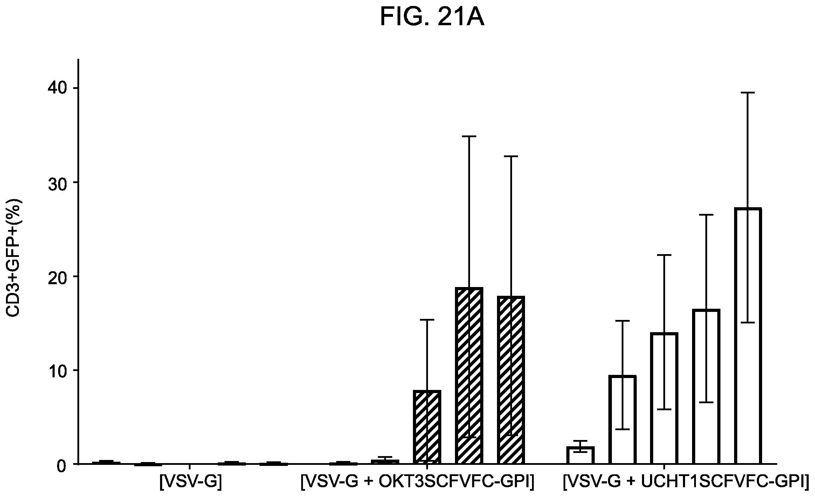

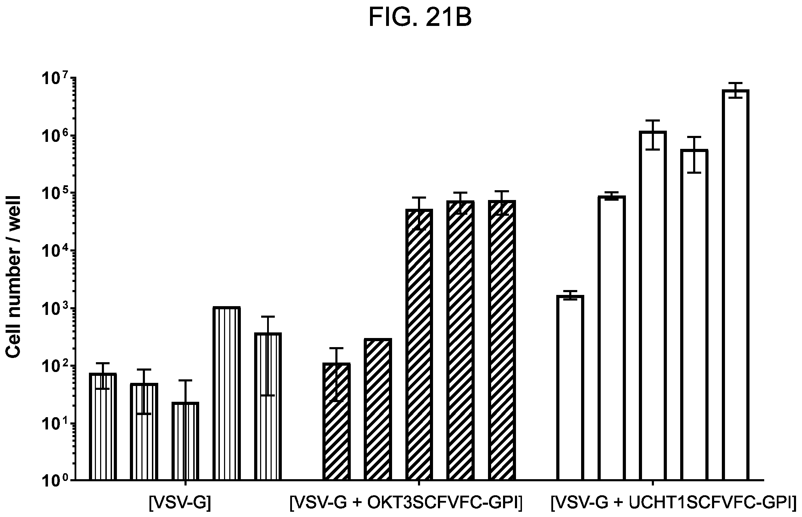

FIG. 21A and FIG. 21B show a histogram of the percentage (%) CD3+GFP+ cells in the total CD3+ population and a histogram of the absolute cell count per well of the CD3+GFP+ population, respectively, at 3, 6, 9, 13 and 17 days after transduction of freshly isolated and unstimulated PBMCs from Donor 12M, for 14h with the indicated lentiviral particles. Each bar represents the mean +/- SD of duplicates.

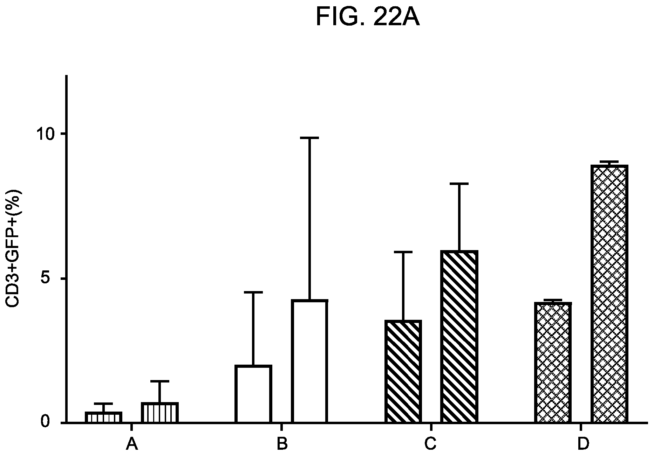

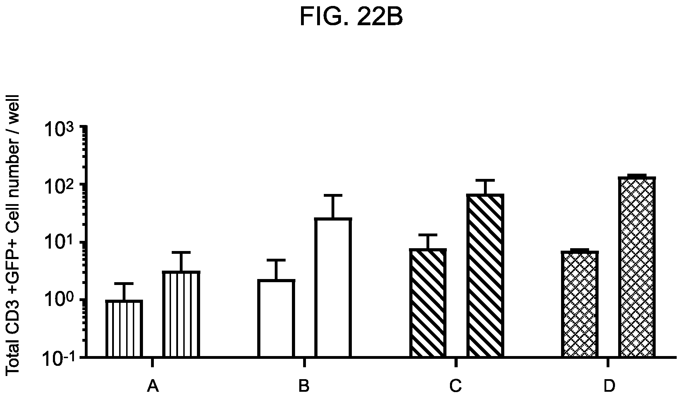

FIG. 22A and FIG. 22B show a histogram of (%)CD3+GFP+ cells in the total CD3+ population and a histogram of the absolute cell count per well of the CD3+GFP+ population, respectively, at 3 and 6 days after transduction of freshly isolated and unstimulated PBMCs from Donor 13F, for 14h, with the indicated lentiviral particles. Please note that "A" shows results using VSV-G pseudotyped lentiviral particles (triplicate experiments); "B" shows results using VSV-G pseudotyped lentiviral particles with OKT3 Ab (1ug/mL) added to the transduction medium (duplicate experiments); "C" shows results using VSV-G pseudotyped lentiviral particles expressing GPI-anchored UCHT1scFvFc on their surface (triplicate experiments); and "D" shows results using VSV-G pseudotyped lentiviral particles expressing GPI anchored UCHT1scFvFc and GPI-anchored CD80, or a functional extracellular fragment thereof, on their surface (duplicate experiments). Each bar represents the mean +/- SD of duplicates or triplicates, as indicated in FIG. 22A.

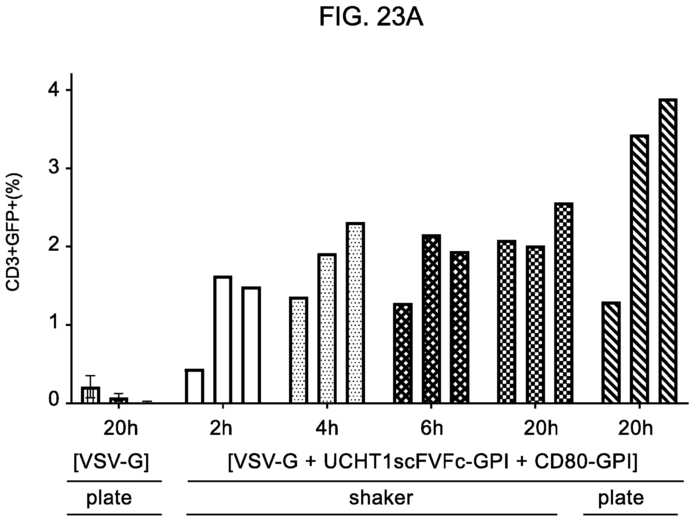

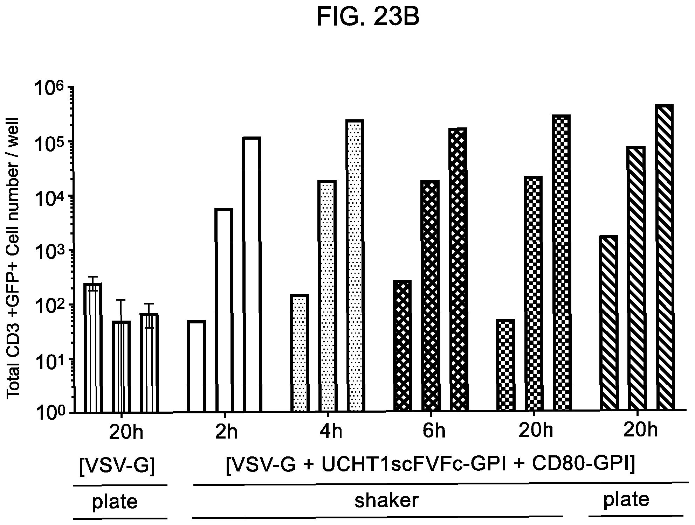

FIG. 23A and FIG. 23B show a histogram of percentage (%) CD3+GFP+ cells in the total CD3+ population and a histogram of the absolute cell count per well of the CD3+GFP+ population, respectively, at 3, 6 and 9 days after transduction of freshly isolated and unstimulated PBMCs from Donor 12M for the indicated time of exposure (2-20h), with the indicated lentiviral particles. Transduction was performed in a plate or a shaker flask as indicated. Each bar represents the mean +/- SD of duplicates for lentiviral particles pseudotyped with VSV-G ("[VSV-G]"); the other experiments did not have replicates.

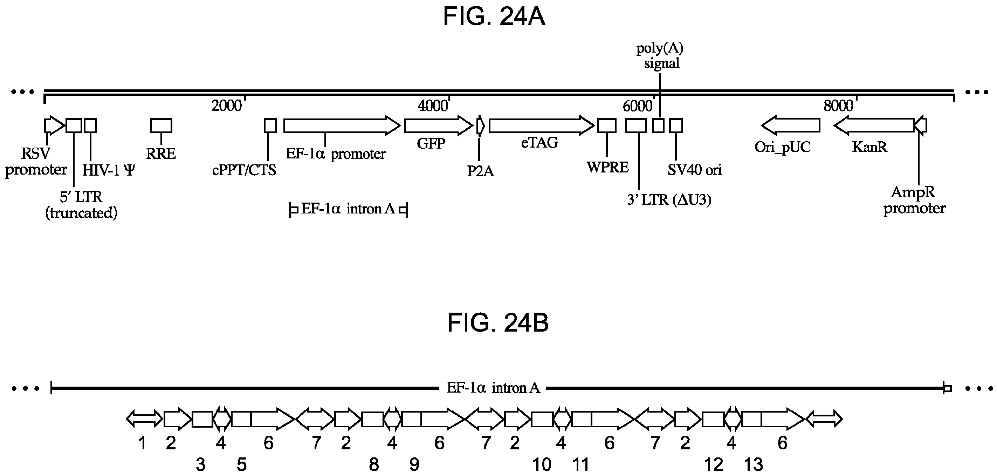

FIG. 24A is a schematic of the lentiviral vector backbone F1-0-02 including a transgene expression cassette driving expression of GFP and eTag and a synthetic EF-1alpha promoter and intron A upstream of the GFP. FIG. 24B shows insertion of the miRNAs into EF1alpha intron A of the F1-0-02 backbone. "1" represents the EF1alpha overlap; "2" represents a 5' arm; "3" represents the miRNA1 5' stem; "4" represents a loop; "5" represents the miRNA1 3' stem; "6" represents a 3' arm; "7" represents a linker; "8" represents the miRNA2 5' stem; "9" represents the miRNA2 3' stem;"10" represents the miRNA3 5' stem; "11" represents the miRNA3 3' stem; "12" represents the miRNA4 5' stem; and "13" represents the miRNA4 3' stem.

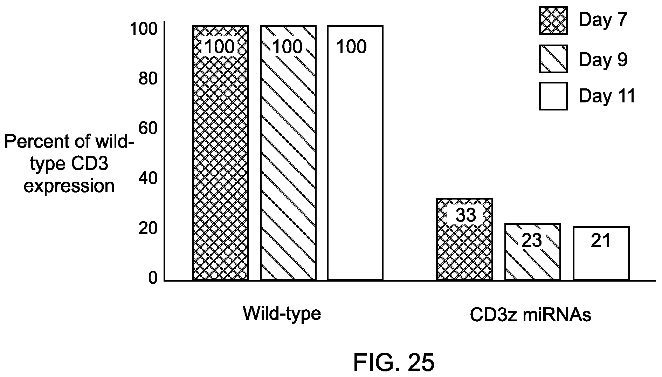

FIG. 25 is a graph showing that the miRNAs targeting CD3zeta that are in the EF-1alpha promoter intron are able to knockdown expression of the CD3 complex.

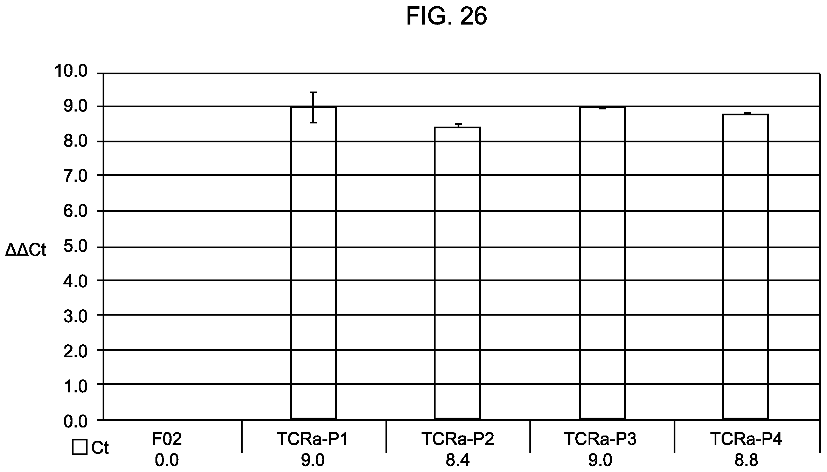

FIG. 26 is a histogram showing the ΔΔCt of samples transduced with miR-TCRα containing replication incompetent lentiviral particles. The ΔΔCt values are representative of the amount of processed miR-TCRa miRNA in each transduced sample relative to the non-transduced control.

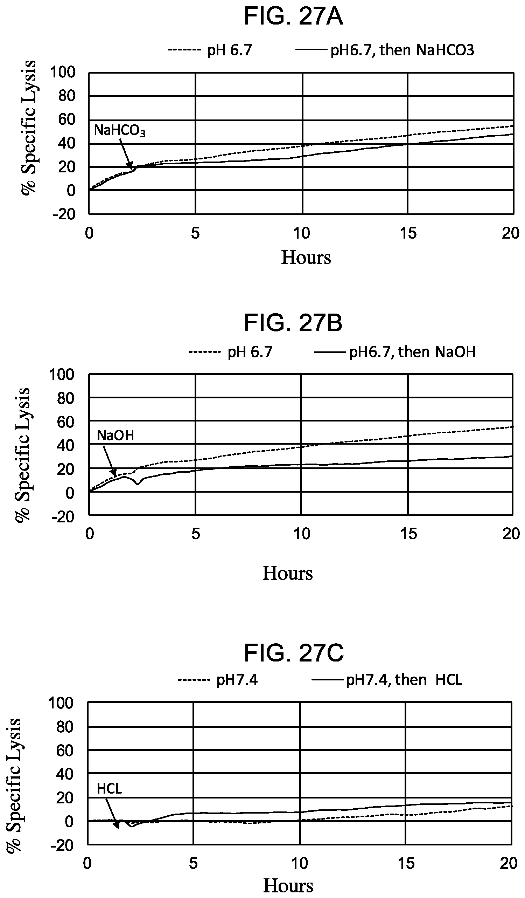

FIGs. 27A-C are graphs showing the percent specific lysis of CHO-Target 1 cells with and without treatment with a pH-modulating pharmacologic agent. In FIG. 27A, the CHO-Target 1 cells were initially at pH 6.7 and experimental wells (solid line) and control cells (dashed line) were treated with or without NaHCO₃, respectively, at the time indicated by the arrow. In FIG. 27B, the CHO-Target 1 cells were initially at pH 6.7 and experimental wells (solid line) and control cells (dashed line) were treated with or without NaOH, respectively, at the time indicated by the arrow. In FIG. 27C, the CHO-Target 1 cells were initially at pH 7.4 and experimental wells (solid line) and control cells (dashed line) were treated with or without HCl, respectively.

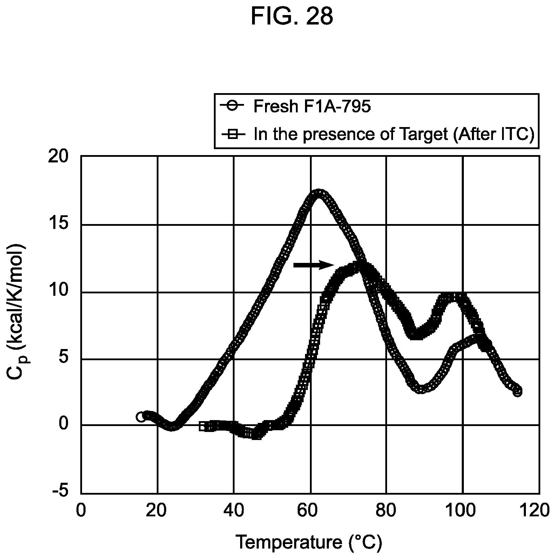

FIG. 28 is a graph showing the heat flux versus time for F1A-795 in the absence (circles) or presence (squares) of acyclovir as measured by DSC.

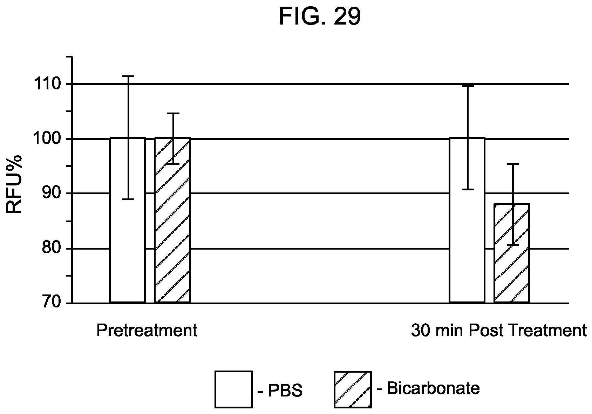

FIG. 29 is a graph showing the RFU percentage from ProSense FAST probe in CHO-xenograft tumor bearing mice before and after administration of PBS or bicarbonate.

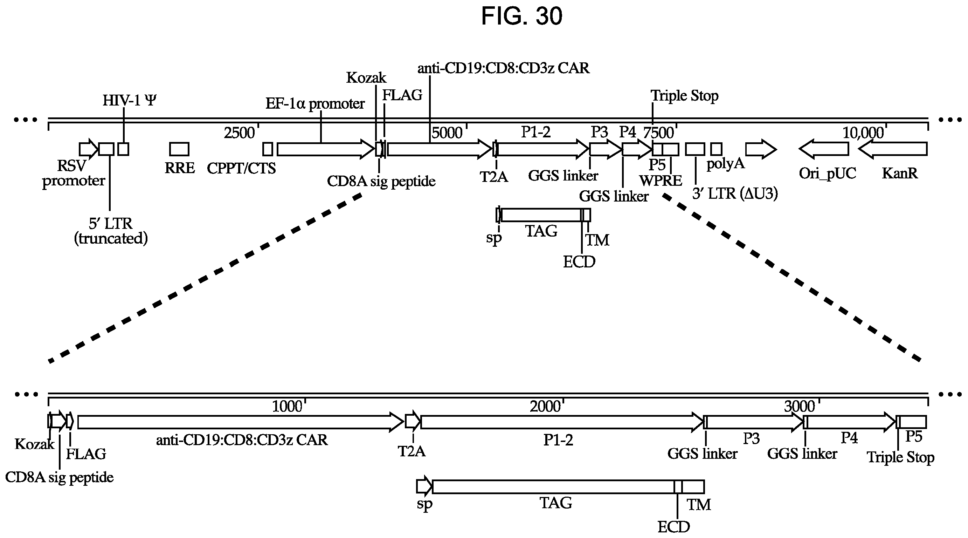

FIG. 30 is a schematic of a non-limiting, exemplary transgene expression cassette containing a polynucleotide sequence encoding a CAR and a candidate chimeric lymphoproliferative element (CLE) of Libraries 1A, 1.1A, and 1.1B.

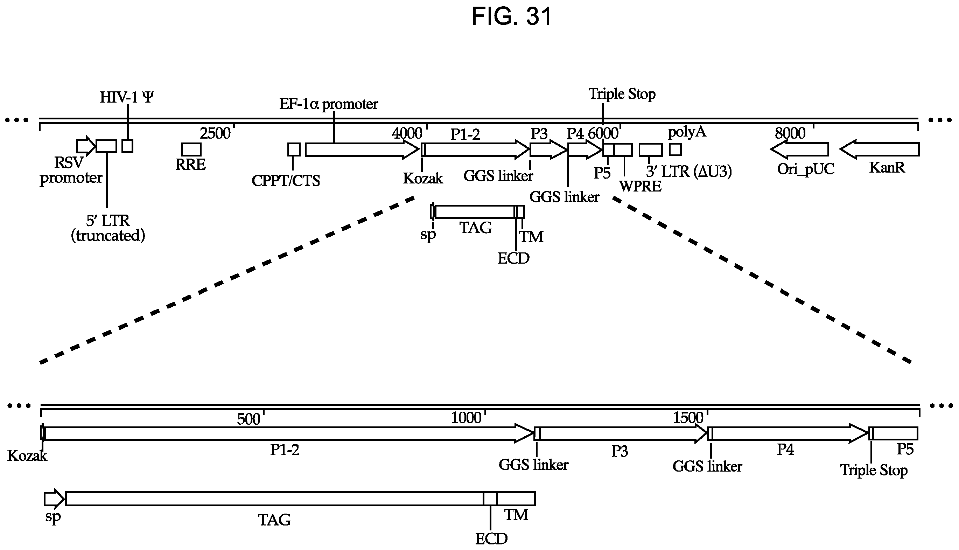

FIG. 31 is a schematic of a non-limiting, exemplary transgene expression cassette containing a polynucleotide sequence encoding a candidate CLE of Libraries 2B and 2.1B.

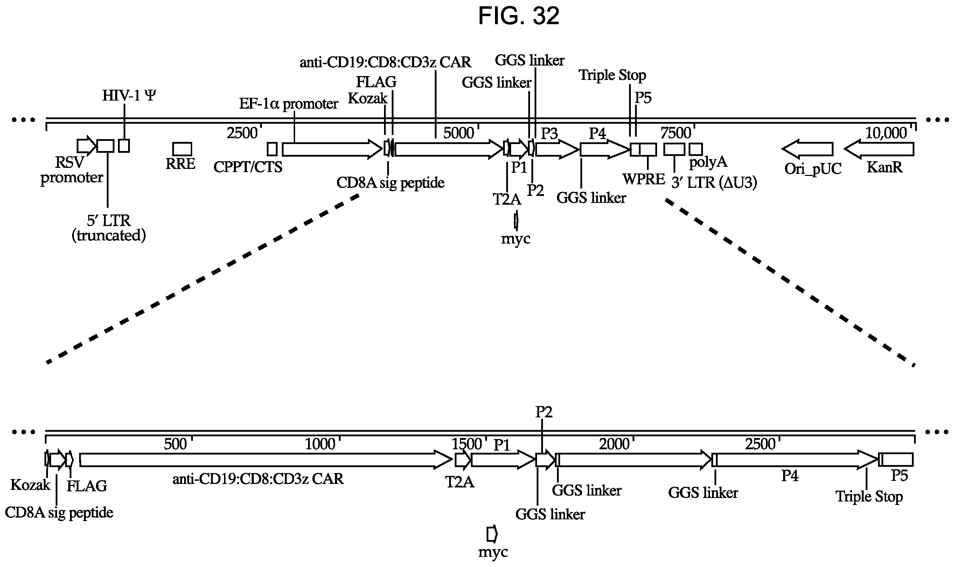

FIG. 32 is a schematic of a non-limiting, exemplary transgene expression cassette containing a polynucleotide sequence encoding a CAR and a candidate CLE of Libraries 3A, 3B, 3.1A, and 3.1B.

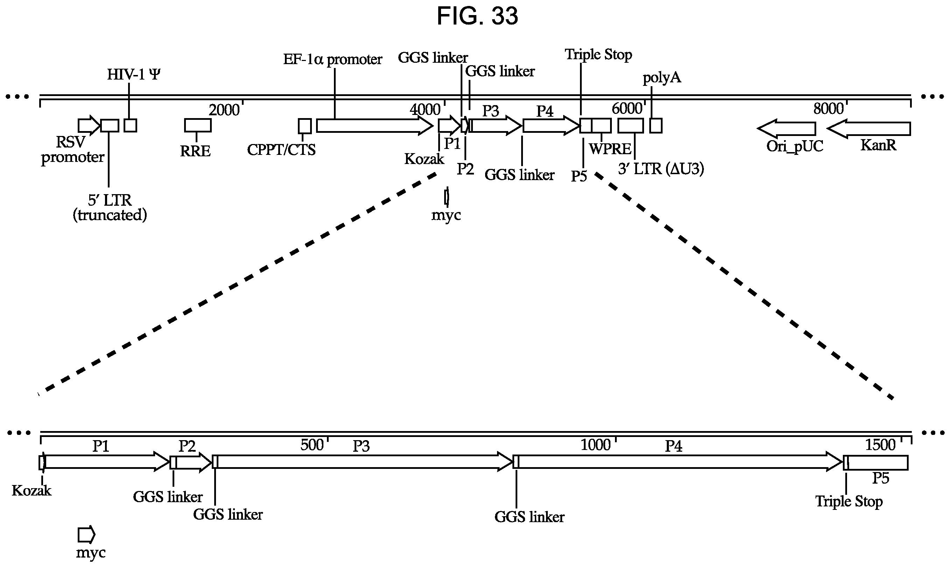

FIG. 33 is a schematic of a non-limiting, exemplary transgene expression cassette containing a polynucleotide sequence encoding a candidate CLE of Libraries 4B and 4.1 B.

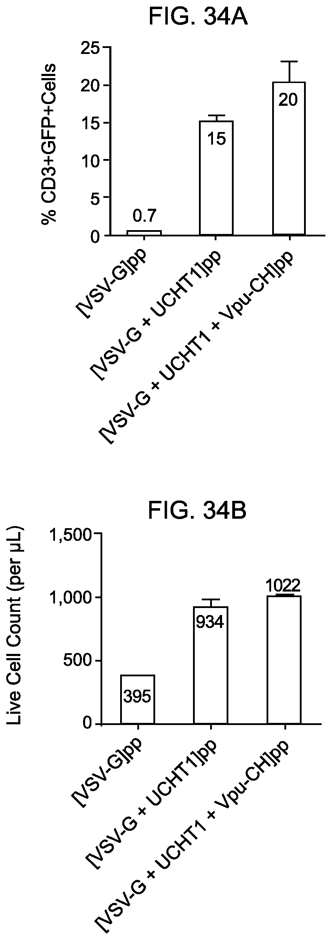

FIG. 34 shows a histogram of the percentage (%)CD3+GFP+ cells in the Live CD3+ population FIG. 34A, and a histogram of the absolute cell count per uL of the total live population 34B, respectively, at day 3 post-transduction of freshly isolated and unstimulated PBMCs from Donor 18, with the indicated lentiviral particles. Each bar represents the mean +/- SD of duplicates.

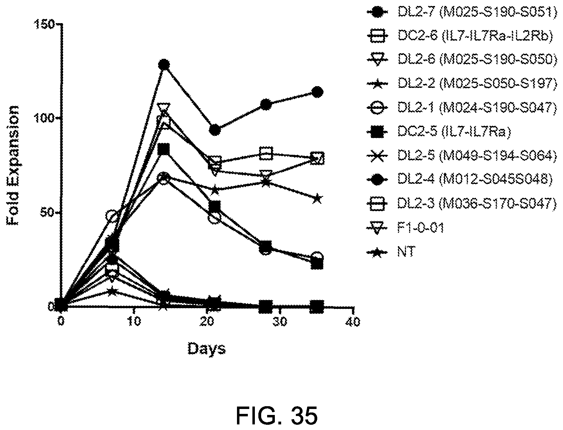

FIG. 35 is a graph showing the fold expansion of PBMCs transduced with lentiviral particles encoding individual CLEs and cultured for 35 days in the absence of exogenous cytokines.

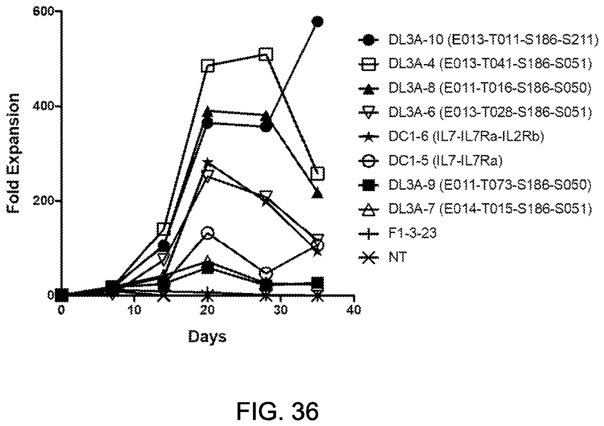

FIG. 36 is a graph showing the fold expansion of PBMCs transduced with lentiviral particles encoding an anti-CD19 CAR construct and individual CLEs and cultured for 35 days in the presence of donor matched PBMCs but in the absence of exogenous cytokines.

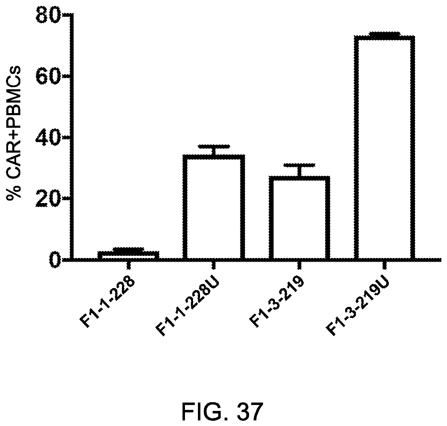

FIG. 37 is a graph showing the efficiency by which the indicated lentiviral particle transduced resting PBMCs in 4 hours. Transduction efficiency was measured as the % CAR+ PBMCs after 6 days in culture in the absence of exogenous cytokines as determined by FACS. Each lentiviral particle encoded a CAR and a CLE. Lentiviral particles transduced with F1-1-228U and F1-3-219U displayed UCHT1scFvFc-GPI on their surface.

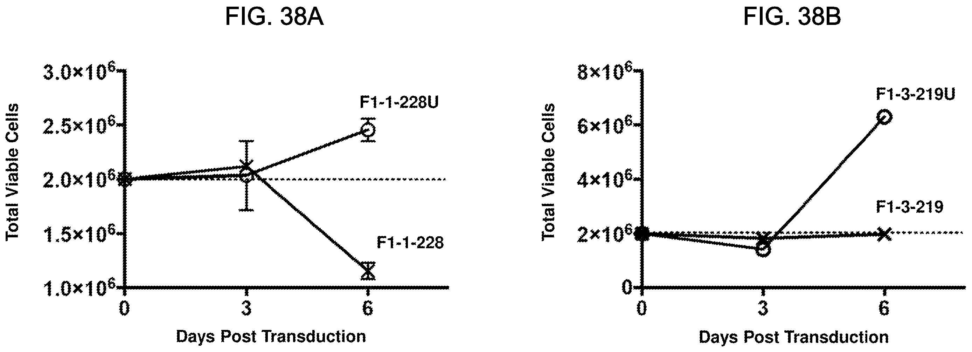

FIG. 38A and FIG. 38B are graphs showing a time course of the total number of viable cells after resting PBMCs were transduced with the indicated lentiviral particle for 4 hours and cultured in vitro in the absence of exogenous cytokines for 6 days. Each lentiviral particle encoded a CAR and a CLE. Lentiviral particles transduced with F1-1-228U and F1-3-219U displayed UCHT1scFvFc-GPI on their surface.

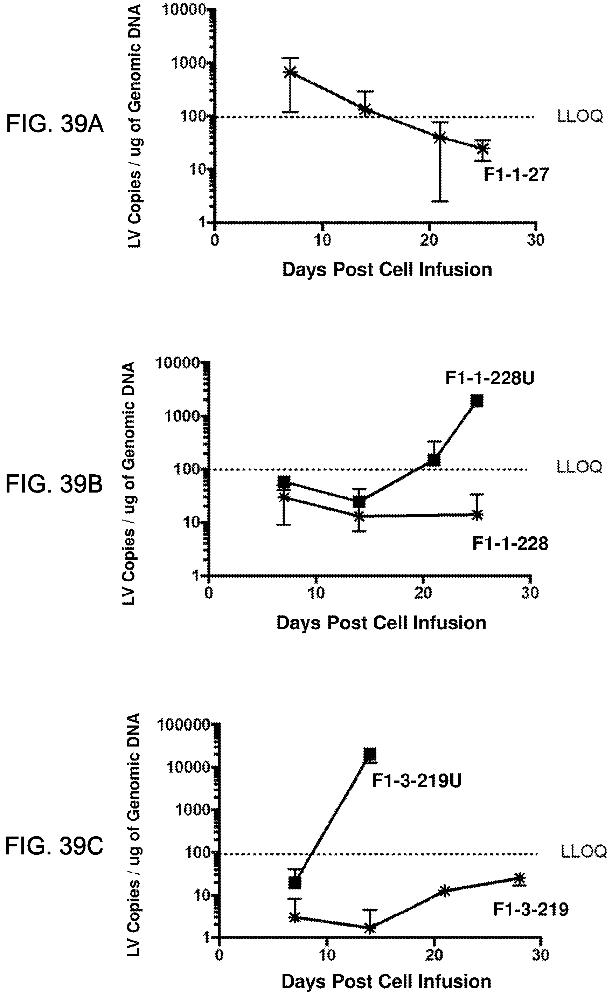

FIGs. 39A, 39B, and 39C are graphs showing a time course of the copies of lentiviral genome per µg of genomic DNA from the blood of tumor-bearing NSG mice dosed with human PBMCs transduced with the indicated lentiviral particle for 4 hours and injected intravenously without the PBMCs having been expanded ex vivo. Each lentiviral particle encoded a CAR. F1-1-228, F1-1-228U, F1-3-219, and F1-3-219U also encoded a CLE. Lentiviral particles transduced with F1-1-228U and F1-3-219U displayed UCHT1scFvFc-GPI on their surface.

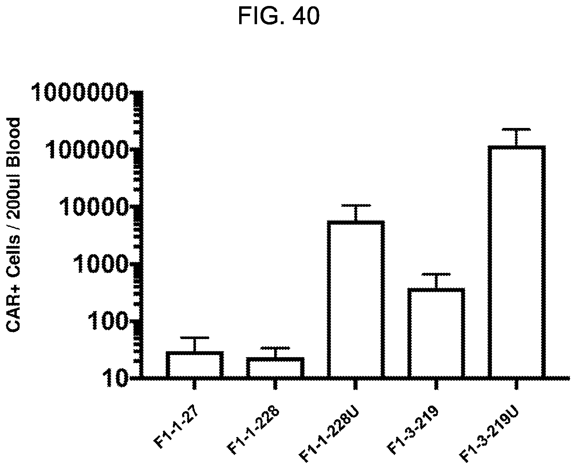

FIG. 40 is a graph showing the number of CAR+ cells per 200µl of blood of tumor-bearing NSG mice dosed with human PBMCs transduced with the indicated lentiviral particle for 4 hours and injected intravenously without the PBMCs having been expanded ex vivo. Blood was sampled at the time the mice were euthanized. Each lentiviral particle encoded a CAR. F1-1-228, F1-1-228U, F1-3-219, and F1-3-219U also encoded a CLE. Lentiviral particles transduced with F1-1-228U and F1-3-219U displayed UCHT1scFvFc-GPI on their surface.

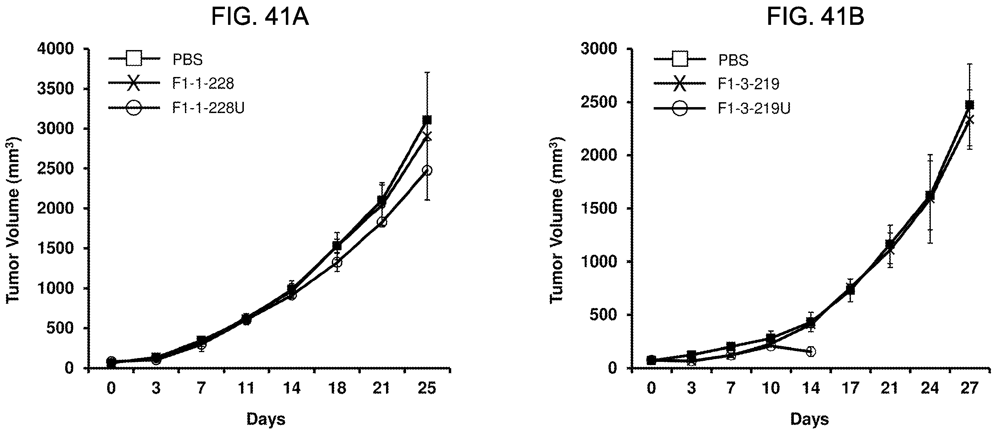

FIG. 41A is a graph showing the mean tumor volume of CHO-ROR2 tumors in NSG mice dosed intravenously with PBS or human PBMCs transduced with the indicated lentiviral particle encoding an anti-ROR2 MRB CAR and a CLE for 4 hours without the PBMCs having been expanded ex vivo. 41B is a graph showing the mean tumor volume of Raji tumors in NSG mice dosed intravenously with PBS or human PBMCs transduced with the indicated lentiviral particle encoding an anti-CD19 CAR and a CLE for 4 hours without the PBMCs having been expanded ex vivo. Lentiviral particles transduced with F1-1-228U and F1-3-219U displayed UCHT1scFvFc-GPI on their surface.

### DEFINITIONS

As used herein, the term "chimeric antigen receptor" or "CAR" or "CARs" refers to engineered receptors, which graft an antigen specificity onto cells, for example T cells, NK cells, macrophages, and stem cells. The CARs as described herein include at least one antigen-specific targeting region (ASTR), a transmembrane domain (TM), and an intracellular activating domain (IAD) and can include a stalk, and one or more co-stimulatory domains (CSDs). In another embodiment, the CAR is a bispecific CAR, which is specific to two different antigens or epitopes. After the ASTR binds specifically to a target antigen, the IAD activates intracellular signaling. For example, the IAD can redirect T cell specificity and reactivity toward a selected target in a non-MHC-restricted manner, exploiting the antigen-binding properties of antibodies. The non-MHC-restricted antigen recognition gives T cells expressing the CAR the ability to recognize an antigen independent of antigen processing, thus bypassing a major mechanism of tumor escape. Moreover, when expressed in T cells, CARs advantageously do not dimerize with endogenous T cell receptor (TCR) alpha and beta chains.

As used herein, the term "microenvironment" means any portion or region of a tissue or body that has constant or temporal, physical, or chemical differences from other regions of the tissue or regions of the body. For example, a "tumor microenvironment" as used herein refers to the environment in which a tumor exists, which is the non-cellular area within the tumor and the area directly outside the tumorous tissue but does not pertain to the intracellular compartment of the cancer cell itself. The tumor microenvironment can refer to any and all conditions of the tumor milieu including conditions that create a structural and or functional environment for the malignant process to survive and/or expand and/or spread. For example, the tumor microenvironment can include alterations in conditions such as, but not limited to, pressure, temperature, pH, ionic strength, osmotic pressure, osmolality, oxidative stress, concentration of one or more solutes, concentration of electrolytes, concentration of glucose, concentration of hyaluronan, concentration of lactic acid or lactate, concentration of albumin, levels of adenosine, levels of R-2-hydroxyglutarate, concentration of pyruvate, concentration of oxygen, and/or presence of oxidants, reductants, or co-factors, as well as other conditions a skilled artisan will understand.

As used interchangeably herein, the terms "polynucleotide" and "nucleic acid" refer to a polymeric form of nucleotides of any length, either ribonucleotides or deoxyribonucleotides. Thus, this term includes, but is not limited to, single-, double-, or multi-stranded DNA or RNA, genomic DNA, cDNA, DNA-RNA hybrids, or a polymer comprising purine and pyrimidine bases or other natural, chemically or biochemically modified, non-natural, or derivatized nucleotide bases.

As used herein, the term "antibody" includes polyclonal and monoclonal antibodies, including intact antibodies and fragments of antibodies which retain specific binding to antigen. The antibody fragments can be, but are not limited to, fragment antigen binding (Fab) fragments, Fab' fragments, F(ab')₂ fragments, Fv fragments, Fab'-SH fragments, (Fab')₂ Fv fragments, Fd fragments, recombinant IgG (rIgG) fragments, single-chain antibody fragments, including single-chain variable fragments (scFv), divalent scFv's, trivalent scFv's, and single domain antibody fragments (e.g., sdAb, sdFv, nanobody). The term includes genetically engineered and/or otherwise modified forms of immunoglobulins, such as intrabodies, peptibodies, chimeric antibodies, single-chain antibodies, fully human antibodies, humanized antibodies, fusion proteins including an antigen-specific targeting region of an antibody and a non-antibody protein, heteroconjugate antibodies, multispecific, e.g., bispecific, antibodies, diabodies, triabodies, and tetrabodies, tandem di-scFv's, and tandem tri-scFv's. Unless otherwise stated, the term "antibody" should be understood to include functional antibody fragments thereof. The term also includes intact or full-length antibodies, including antibodies of any class or sub-class, including IgG and subclasses thereof, IgM, IgE, IgA, and IgD.

As used herein, the term "antibody fragment" includes a portion of an intact antibody, for example, the antigen binding or variable region of an intact antibody. Examples of antibody fragments include Fab, Fab', F(ab')₂, and Fv fragments; diabodies; linear antibodies (Zapata et al., Protein Eng. 8(10): 1057-1062 (1995)); single-chain antibody molecules; and multispecific antibodies formed from antibody fragments. Papain digestion of antibodies produces two identical antigen-binding fragments, called "Fab" fragments, each with a single antigen-binding site, and a residual "Fe" fragment, a designation reflecting the ability to crystallize readily. Pepsin treatment yields an F(ab')₂ fragment that has two antigen combining sites and is still capable of cross-linking antigen.

As used interchangeably herein, the terms "single-chain Fv," "scFv," or "sFv" antibody fragments include the V_{H} and V_{L} domains of antibody, wherein these domains are present in a single polypeptide chain. In some embodiments, the Fv polypeptide further includes a polypeptide linker or spacer between the V_{H} and V_{L} domains, which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994*).*

As used herein, "naturally occurring" VH and VL domains refer to VH and VL domains that have been isolated from a host without further molecular evolution to change their affinities when generated in an scFv format under specific conditions such as those disclosed in US patent 8709755 B2 and application WO/2016/033331A1.

As used herein, the term "affinity" refers to the equilibrium constant for the reversible binding of two agents and is expressed as a dissociation constant (Kd). Affinity can be at least 1-fold greater, at least 2-fold greater, at least 3-fold greater, at least 4-fold greater, at least 5-fold greater, at least 6-fold greater, at least 7-fold greater, at least 8-fold greater, at least 9-fold greater, at least 10-fold greater, at least 20-fold greater, at least 30-fold greater, at least 40-fold greater, at least 50-fold greater, at least 60-fold greater, at least 70-fold greater, at least 80-fold greater, at least 90-fold greater, at least 100-fold greater, or at least 1000-fold greater, or more, than the affinity of an antibody for unrelated amino acid sequences. Affinity of an antibody to a target protein can be, for example, from about 100 nanomolar (nM) to about 0.1 nM, from about 100 nM to about 1 picomolar (pM), or from about 100 nM to about 1 femtomolar (fM) or more. As used herein, the term "avidity" refers to the resistance of a complex of two or more agents to dissociation after dilution. The terms "immunoreactive" and "preferentially binds" are used interchangeably herein with respect to antibodies and/or antigen-binding fragments.

As used herein, the term "binding" refers to a direct association between two molecules, due to, for example, covalent, electrostatic, hydrophobic, and ionic and/or hydrogen-bond interactions, including interactions such as salt bridges and water bridges. Non-specific binding would refer to binding with an affinity of less than about 10⁻⁷ M, e.g., binding with an affinity of 10⁻⁶ M, 10⁻⁵ M, 10⁻⁴ M, etc.

As used herein, reference to a "cell surface expression system" or "cell surface display system" refers to the display or expression of a protein or portion thereof on the surface of a cell. Typically, a cell is generated that expresses proteins of interest fused to a cell-surface protein. For example, a protein is expressed as a fusion protein with a transmembrane domain.

As used herein, the term "element" includes polypeptides, including fusions of polypeptides, regions of polypeptides, and functional mutants or fragments thereof and polynucleotides, including microRNAs and shRNAs, and functional mutants or fragments thereof.

As used herein, the term "region" is any segment of a polypeptide or polynucleotide.

As used herein, a "domain" is a region of a polypeptide or polynucleotide with a functional and/or structural property.

As used herein, the terms "stalk" or "stalk domain" refer to a flexible polypeptide connector region providing structural flexibility and spacing to flanking polypeptide regions and can consist of natural or synthetic polypeptides. A stalk can be derived from a hinge or hinge region of an immunoglobulin (e.g., IgGl) that is generally defined as stretching from Glu216 to Pro230 of human IgGl (Burton (1985) Molec. Immunol., 22:161-206). Hinge regions of other IgG isotypes may be aligned with the IgG1 sequence by placing the first and last cysteine residues forming inter-heavy chain disulfide (S-S) bonds in the same positions. The stalk may be of natural occurrence or non-natural occurrence, including but not limited to an altered hinge region, as disclosed in U.S. Pat. No. 5,677,425. The stalk can include a complete hinge region derived from an antibody of any class or subclass. The stalk can also include regions derived from CD8, CD28, or other receptors that provide a similar function in providing flexibility and spacing to flanking regions.

As used herein, the term "isolated" means that the material is removed from its original environment (e.g., the natural environment if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

As used herein, a "polypeptide" is a single chain of amino acid residues linked by peptide bonds. A polypeptide does not fold into a fixed structure nor does it have any posttranslational modification. A "protein" is a polypeptide that folds into a fixed structure. "Polypeptides" and "proteins" are used interchangeably herein.

As used herein, a polypeptide may be "purified" to remove contaminant components of a polypeptide's natural environment, e.g. materials that would interfere with diagnostic or therapeutic uses for the polypeptide such as, for example, enzymes, hormones, and other proteinaceous or nonproteinaceous solutes. A polypeptide can be purified (1) to greater than 90%, greater than 95%, or greater than 98%, by weight of antibody as determined by the Lowry method, for example, more than 99% by weight, (2) to a degree sufficient to obtain at least 15 residues of N-terminal or internal amino acid sequence by use of a spinning cup sequenator, or (3) to homogeneity by sodium dodecyl sulfate-polyacrylamide gel electrophoresis (SDS-PAGE) under reducing or nonreducing conditions using Coomassie blue or silver stain.

As used herein, the term "immune cells" generally includes white blood cells (leukocytes) which are derived from hematopoietic stem cells (HSC) produced in the bone marrow. "Immune cells" includes, e.g., lymphocytes (T cells, B cells, natural killer (NK) cells) and myeloid-derived cells (neutrophil, eosinophil, basophil, monocyte, macrophage, dendritic cells).

As used herein, "T cell" includes all types of immune cells expressing CD3 including T-helper cells (CD4⁺ cells), cytotoxic T cells (CD8⁺ cells), T-regulatory cells (Treg) and gamma-delta T cells.

As used herein, a "cytotoxic cell" includes CD8⁺ T cells, natural-killer (NK) cells, NK-T cells, γδ T cells, a subpopulation of CD4⁺ cells, and neutrophils, which are cells capable of mediating cytotoxicity responses.

As used herein, the term "stem cell" generally includes pluripotent or multipotent stem cells. "Stem cells" includes, e.g., embryonic stem cells (ES); mesenchymal stem cells (MSC); induced-pluripotent stem cells (iPS); and committed progenitor cells (hematopoietic stem cells (HSC); bone marrow derived cells, etc.).

As used herein, the terms "treatment," "treating," and the like, refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment," as used herein, covers any treatment of a disease in a mammal, e.g., in a human, and includes: (a) preventing the disease from occurring in a subject which may be predisposed to the disease but has not yet been diagnosed as having it; (b) inhibiting the disease, i.e., arresting its development; and (c) relieving the disease, i.e., causing regression of the disease.

As used interchangeably herein, the terms "individual", "subject", "host", and "patient" refer to a mammal, including, but not limited to, humans, murines (e.g., rats, mice), lagomorphs (e.g., rabbits), non-human primates, humans, canines, felines, ungulates (e.g., equines, bovines, ovines, porcines, caprines), etc.

As used herein, the terms "therapeutically effective amount" or "efficacious amount" refers to the amount of an agent, or combined amounts of two agents, that, when administered to a mammal or other subject for treating a disease, is sufficient to affect such treatment for the disease. The "therapeutically effective amount" will vary depending on the agent(s), the disease and its severity and the age, weight, etc., of the subject to be treated.

As used herein, the term "evolution" or "evolving" refers to using one or more methods of mutagenesis to generate a different polynucleotide encoding a different polypeptide, which is itself an improved biological molecule and/or contributes to the generation of another improved biological molecule. "Physiological" or "normal" or "normal physiological" conditions are conditions such as, but not limited to, pressure, temperature, pH, ionic strength, osmotic pressure, osmolality, oxidative stress, concentration of one or more solutes, concentration of electrolytes, concentration of glucose, concentration of hyaluronan, concentration of lactic acid or lactate, concentration of albumin, levels of adenosine, levels of R-2-hydroxyglutarate, concentration of pyruvate, concentration of oxygen, and/or presence of oxidants, reductants, or co-factors, as well as other conditions, that would be considered within a normal range at the site of administration, or at the tissue or organ at the site of action, to a subject.

As used herein, a "genetically modified cell" includes cells that contain exogenous nucleic acids whether or not the exogenous nucleic acids are integrated into the genome of the cell.

A "polypeptide" as used herein can include part of or an entire protein molecule as well as any posttranslational or other modifications.

A pseudotyping element as used herein can include a "binding polypeptide" that includes one or more polypeptides, typically glycoproteins, that identify and bind the target host cell, and one or more "fusogenic polypeptides" that mediate fusion of the retroviral and target host cell membranes, thereby allowing a retroviral genome to enter the target host cell. The "binding polypeptide" as used herein, can also be referred to as a "T cell and/or NK cell binding polypeptide" or a "target engagement element," and the "fusogenic polypeptide" can also be referred to as a "fusogenic element".

A "resting" lymphocyte, such as for example, a resting T cell, is a lymphocyte in the G0 stage of the cell cycle that does not express activation markers such as Ki-67. Resting lymphocytes can include naive T cells that have never encountered specific antigen and memory T cells that have been altered by a previous encounter with an antigen. A "resting" lymphocyte can also be referred to as a "quiescent" lymphocyte.

As used herein, "lymphodepletion" involves methods that reduce the number of lymphocytes in a subject, for example by administration of a lymphodepletion agent. Lymphodepletion can also be attained by partial body or whole body fractioned radiation therapy. A lymphodepletion agent can be a chemical compound or composition capable of decreasing the number of functional lymphocytes in a mammal when administered to the mammal. One example of such an agent is one or more chemotherapeutic agents. Such agents and dosages are known, and can be selected by a treating physician depending on the subject to be treated. Examples of lymphodepletion agents include, but are not limited to, fludarabine, cyclophosphamide, cladribine, denileukin diftitox, or combinations thereof.

RNA interference (RNAi) is a biological process in which RNA molecules inhibit gene expression or translation by neutralizing targeted RNA molecules. The RNA target may be mRNA, or it may be any other RNA susceptible to functional inhibition by RNAi. As used herein, an "inhibitory RNA molecule" refers to an RNA molecule whose presence within a cell results in RNAi and leads to reduced expression of a transcript to which the inhibitory RNA molecule is targeted. An inhibitory RNA molecule as used herein has a 5' stem and a 3' stem that is capable of forming an RNA duplex. The inhibitory RNA molecule can be, for example, a miRNA (either endogenous or artificial) or a shRNA, a precursor of a miRNA (i.e. a Pri-miRNA or Pre-miRNA) or shRNA, or a dsRNA that is either transcribed or introduced directly as an isolated nucleic acid, to a cell or subject.

As used herein, "double stranded RNA" or "dsRNA" or "RNA duplex" refers to RNA molecules that are comprised of two strands. Double-stranded molecules include those comprised of two RNA strands that hybridize to form the duplex RNA structure or a single RNA strand that doubles back on itself to form a duplex structure. Most, but not necessarily all of the bases in the duplex regions are base-paired. The duplex region comprises a sequence complementary to a target RNA. The sequence complementary to a target RNA is an antisense sequence, and is frequently from 18 to 29, from 19 to 29, from 19 to 21, or from 25 to 28 nucleotides long, or in some embodiments between 18, 19, 20, 21, 22, 23, 24, 25 on the low end and 21, 22, 23, 24, 25, 26, 27, 28 29, or 30 on the high end, where a given range always has a low end lower than a high end. Such structures typically include a 5' stem, a loop, and a 3' stem connected by a loop which is contiguous with each stem and which is not part of the duplex. The loop comprises, in certain embodiments, at least 3, 4, 5, 6, 7, 8, 9, or 10 nucleotides. In other embodiments the loop comprises from 2 to 40, from 3 to 40, from 3 to 21, or from 19 to 21 nucleotides, or in some embodiments between 2, 3, 4, 5, 6, 7, 8, 9, 10, 11, 12, 13, 14, 15, 16, 17, 18, 19, or 20 on the low end and 10, 11, 12, 13, 14, 15, 16, 17, 18, 19, 20, 25, 30, 35, or 40 on the high end, where a given range always has a low end lower than a high end.

The term "microRNA flanking sequence" as used herein refers to nucleotide sequences including microRNA processing elements. MicroRNA processing elements are the minimal nucleic acid sequences which contribute to the production of mature microRNA from precursor microRNA. Often these elements are located within a 40 nucleotide sequence that flanks a microRNA stem-loop structure. In some instances the microRNA processing elements are found within a stretch of nucleotide sequences of between 5 and 4,000 nucleotides in length that flank a microRNA stem-loop structure.

The term "linker" when used in reference to a multiplex inhibitory RNA molecule refers to a connecting means that joins two inhibitory RNA molecules.

As used herein, a "recombinant retrovirus" refers to a non-replicable, or "replication incompetent", retrovirus unless it is explicitly noted as a replicable retrovirus. The terms "recombinant retrovirus" and "recombinant retroviral particle" are used interchangeably herein. Such retrovirus/retroviral particle can be any type of retroviral particle including, for example, gamma retrovirus, and in illustrative embodiments, lentivirus. As is known, such retroviral particles, for example lentiviral particles, typically are formed in packaging cells by transfecting the packing cells with plasmids that include packaging components such as Gag, Pol and Rev, an envelope or pseudotyping plasmid that encodes a pseudotyping element, and a transfer, genomic, or retroviral (e.g. lentiviral) expression vector, which is typically a plasmid on which a gene(s) or other coding sequence of interest is encoded. Accordingly, a retroviral (e.g. lentiviral) expression vector includes sequences (e.g. a 5' LTR and a 3' LTR flanking e.g. a psi packaging element and a target heterologous coding sequence) that promote expression and packaging after transfection into a cell. The terms "lentivirus" and "lentiviral particle" are used interchangeably herein.

A "framework" of a miRNA consists of "5' microRNA flanking sequence" and/or "3' microRNA flanking sequence" surrounding a miRNA and, in some cases, a loop sequence that separates the stems of a stem-loop structure in a miRNA. In some examples, the "framework" is derived from naturally occurring miRNAs, such as, for example, miR-155. The terms "5' microRNA flanking sequence" and "5' arm" are used interchangeably herein. The terms "3' microRNA flanking sequence" and "3' arm" are used interchangeably herein.

As used herein, the term "miRNA precursor" refers to an RNA molecule of any length which can be enzymatically processed into an miRNA, such as a primary RNA transcript, a pri-miRNA, or a pre-miRNA.

It is to be understood that the present disclosure and the aspects and embodiments provided herein, are not limited to particular examples disclosed, as such may, of course, vary. It is also to be understood that the terminology used herein is for the purpose of disclosing particular examples and embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention. When multiple low and multiple high values for ranges are given that overlap, a skilled artisan will recognize that a selected range will include a low value that is less than the high value. All headings in this specification are for the convenience of the reader and are not limiting.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein can be referred to for further details to disclose and describe the methods and/or materials in connection with which the publications are cited.

It must be noted that as used herein and in the appended claims, the singular forms "a," "an," and "the" include plural referents unless the context clearly dictates otherwise. Thus, for example, reference to "a chimeric antigen receptor" includes a plurality of such chimeric antigen receptors and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

### DETAILED DESCRIPTION

The present disclosure overcomes prior art challenges by providing improved methods and compositions for genetically modifying lymphocytes, for example NK cells and in illustrative embodiments, T cells. For example, some of these methods do not include prior-activation of the lymphocyte, and some of these methods are performed in less time than prior methods. Furthermore, compositions that have many uses, including their use in these improved methods, are provided. Some of these compositions are genetically modified lymphocytes that have improved proliferative and survival qualities, including in *in vitro* culturing, for example in the absence of growth factors. Such genetically modified lymphocytes will have utility for example, as research tools to better understand factors that influence T cell proliferation and survival, and for commercial production, for example for the production of certain factors, such as growth factors and immunomodulatory agents, that can be harvested and tested or used in commercial products.

Some embodiments provided herein are methods for performing adoptive cellular therapy that include transducing T cells and/or NK cells, that require far less time *ex vivo,* for example, 24, 12, or 8 hours or less, and in some embodiments without prior *ex vivo* stimulation. These methods are well-suited for closed system *ex vivo* processing of blood from a subject, and can be performed with the subject present in the same room as and/or in some embodiments, within their line of sight of their blood or isolated blood cells thereof at all times during performance of the method. More specifically, the aspects and embodiments of the disclosure herein overcome problems associated with current adoptive cellular therapies by providing methods for transducing resting T cells and/or resting NK cells, that typically utilize a pseudotyping element that facilitates binding and fusion of a replication incompetent recombinant retroviral particle to a resting T cell and/or a resting NK cell, to facilitate genetic modification of the resting T cells and/or NK cells by the replication incompetent recombinant retroviral particles. Furthermore, methods provided herein overcome problems of the art by utilizing in illustrative embodiments, a chimeric antigen receptor and one or more lymphoproliferative elements whose expression is under the control of a control element, such that exposure of the subject to a compound that binds the control element, or termination of such exposure, promotes expansion of the genetically modified T cells and/or NK cells *in vivo.*

As a result of these and other improvements disclosed in detail herein, in one aspect, provided herein is a method for genetically modifying resting T cells and/or resting NK cells of a subject, such as a patient having a disease or disorder, wherein blood from the subject is collected; resting T cells and/or NK cells are genetically modified by contacting them with a replication incompetent recombinant retroviral particle; and the genetically modified cells are reintroduced into the subject typically within a shorter period of time than prior methods, for example within 24 hours and in some non-limiting embodiments, within 12 hours and/or without further expanding the population of genetically modified T cells and/or NK cells *ex vivo,* for example such that the genetically modified resting T cells and/or NK cells do not undergo more than 4 cell divisions *ex vivo.* Thus, methods provided herein can be performed in much less time than current CAR therapies, thereby providing processes by which a subject can remain in a clinic for the entire time of the *ex vivo* steps. This facilitates performance of the *ex vivo* steps in a closed system, which reduces the chances for contamination and mixing of patient samples and can be performed more readily by clinical labs.

Accordingly, FIGs. 1 and 2 provide schematic diagrams of illustrative compositions used in methods provided herein. FIG. 1 provides a diagram of a packaging cell (100) and a replication incompetent recombinant retroviral particle, produced by such a packaging cell (200). The packaging cell (100) includes recombinant polynucleotides (110) incorporated into its genome that include recombinant transcriptional elements that express retroviral proteins and various different membrane-bound polypeptides under the control of inducible promoters that are regulated by transactivators, which bind and are activated by ligands. These transactivators, inducible promoters, and ligands are used to induce the sequential expression and accumulation of cell membrane-bound polypeptides that will be incorporated into the membrane of the replication incompetent recombinant retroviral particle as well as retroviral components necessary for packaging and assembly of the replication incompetent recombinant retroviral particles.

As a result of the sequential induced expression of the various polynucleotides as discussed in detail herein below, the illustrative packaging cell (100) illustrated in FIG. 1 is produced, and can be used in illustrative methods to produce replication incompetent recombinant retroviral particles used in methods of transfecting resting T cells and/or NK cells ((300) in FIG. 2) provided herein. The packaging cell (100), in non-limiting illustrative embodiments, includes in its genome nucleic acids encoding a packageable retroviral RNA genome that includes at least some of the elements of a retroviral genome necessary for packaging and assembly of the replication incompetent recombinant retroviral particle (as non-limiting illustrative examples, a retroviral psi element, a retroviral gag polypeptide and a retroviral pol polypeptide).

Some membrane bound polypeptides incorporated or associated with the cell membrane of the packaging cell will become incorporated or associated into the replication incompetent recombinant retroviral particles, but are not encoded by the retroviral genome. For example, the packaging cell and replication incompetent recombinant retroviral particles formed therefrom, can include a retroviral Vpx polypeptide (250), which in non-limiting illustrative examples can be expressed as a membrane associated fusion protein, for example a Src-Flag-Vpx polypeptide; a pseudotyping element that can include a binding polypeptide and a fusogenic polypeptide (240), which in a non-limiting embodiment includes a Measles Virus hemagglutinin (H) polypeptide and a Measles Virus fusion (F) polypeptide, or cytoplasmic domain deletion variants thereof; optionally, one or more activation elements (210, 220), which in a non-limiting embodiment includes a membrane-bound polypeptide capable of binding to CD3 and a membrane-bound polypeptide capable of binding to CD28; and/or optionally a membrane-bound cytokine (230), a non-limiting embodiment of which is a fusion polypeptide that includes IL-7 fused to DAF, or a fragment thereof. Various other specific types of these membrane bound polypeptides are provided herein.

As a result of the sequential expression of the transcriptional elements by the packaging cell, a replication incompetent recombinant retroviral particle is produced. The RNA retroviral genome inside of and typically integrated into the genome of the packaging cell that becomes the genome of the replication incompetent recombinant retroviral particle, includes retroviral components (as non-limiting illustrative examples, retroviral Gag and Pol polynucleotides) that are necessary for retroviral production, infection and integration into the genome of a host cell, which is typically a resting T cell and/or NK cell. Furthermore, the retroviral genome furthermore includes polynucleotides encoding one or typically two engineered signaling polypeptides provided herein. One of the engineered signaling polypeptides typically encodes at least one lymphoproliferative element (in non-limiting examples a constitutive interleukin 7 receptor mutant) and the other engineered signaling polypeptide typically encodes a chimeric antigen receptor.

The replication incompetent recombinant retroviral particle, (200) is then used to transduce a resting T cell and/or resting NK cell (300) in methods provided herein. As shown in FIG. 2, after the resting T cell and/or NK cell (300) is contacted with the replication incompetent recombinant retroviral particle (200), membrane polypeptides discussed above on the surface of the replication incompetent recombinant retroviral particle bind to receptors and/or ligands on the surface of the resting T cell and/or NK cell (300). For example, the pseudotyping element, which as indicated above can include a binding polypeptide that binds to molecules on the surface of resting T cells and/or resting NK cells and a fusogenic polypeptide, facilitates the binding and fusion of replication incompetent recombinant retroviral particle (200) to the T cell and/or NK cell membrane. The activation element(s) (210, 220) activate the resting T cell and/or NK cell (300) by engaging the T-cell receptor complex, a process which occurs over the time course of the contacting or an incubation thereafter. Furthermore, the membrane-bound cytokines (230) can be present on the surface of replication incompetent recombinant retroviral particle and bind cytokine receptors (310) on the surface of the resting T cell and/or NK cell (300), thus further promoting binding and activation. Thus, not to be limited by theory, in illustrative embodiments provided herein, as a result of one or more of these replication incompetent recombinant retroviral particles (200) components, *ex vivo* stimulation or activation by an element that is not already in or on the replication incompetent recombinant retroviral particle (200) is not required. This in turn, helps to cut down the *ex vivo* time that is required for completion of the methods in these illustrative methods provided herein.

Upon binding to the T cell and/or NK cell (200), the replication incompetent recombinant retroviral particle then fuses with the T cell and/or NK cell (300), and polypeptides and nucleic acids in the replication incompetent recombinant retroviral particle enter the T cell and/or NK cell (300). As indicated above, one of these polypeptides in the replication incompetent recombinant retroviral particle is the Vpx polypeptide (250). The Vpx polypeptide (250) binds to and induces the degradation of the SAMHD1 restriction factor (350), which degrades free dNTPs in the cytoplasm. Thus, the concentration of free dNTPs in the cytoplasm increases as Vpx degrades SAMHD1, and reverse transcription activity is increased, thus facilitating reverse transcription of the retroviral genome and integration into the T cell and/or NK cell genome.

After integration of the retroviral genome into the T cell and/or NK cell (200), the T cell and/or NK cell genome includes nucleic acids encoding the signaling polypeptide encoding the lymphoproliferative element (370) and optionally the signaling polypeptide encoding the CAR (360). Expression of the lymphoproliferative element and optionally the CAR are under the control of a control element. Exposure to a compound that binds the control element, which can occur in vitro or *in vivo* by administering it to a subject whose T cell and/or NK cell (300) was transduced, promotes proliferation of the T cell and/or NK cell (300) *in vitro or in vivo* by expressing the lymphoproliferative element and optionally as a result of expression of the CAR and binding of the CAR to its target cell. Thus, T cells and/or NK cells that are transduced with replication incompetent recombinant retroviral particles herein, have one or more signals that drive proliferation and/or inhibit cell death, which in turn in illustrative embodiments, avoids the requirements of prior methods to lymphodeplete a host before returning transduced T cells and/or NK cells back into the subject. This in turn, in illustrative embodiments, further reduces the requirement for days of processing before transduced T cells and/or NK cells are reintroduced into a subject. Thus, in illustrative embodiments, no more than 36 hours, 24 hours, 12 hours, or in some instances even 8 hours, of time is required from collection of blood from the subject to reintroduction of the blood to the subject, which fundamentally changes the CAR-T process from prior methods. Furthermore, the control element provides one of the safety mechanisms provided herein as well. For example, ceasing administration of the compound can down-regulate or even terminate expression of the lymphoproliferative element and optionally the CAR, thus ending a proliferation and/or survival signal to the transduced T cell and/or NK cell and its progeny.

### METHODS FOR TRANSDUCING AND/OR GENETICALLY MODIFYING LYMPHOCYTES

Provided herein in certain aspects, is a method of transducing and/or genetically modifying a lymphocyte, typically a T cell and/or an NK cell, and typically a resting T cell and/or resting NK cell, that includes contacting the lymphocyte with a replication incompetent recombinant retroviral particle, wherein the replication incompetent recombinant retroviral particle typically comprises a pseudotyping element on its surface, wherein said contacting (and incubation under contacting conditions) facilitates transduction of the resting T cell and/or NK cell by the replication incompetent recombinant retroviral particle, thereby producing the genetically modified T cell and/or NK cell. The pseudotyping element is typically capable of binding the resting T cell and/or NK cell and typically facilitating membrane fusion on its own or in conjunction with other protein(s) of the replication incompetent recombinant retroviral particles.

In some embodiments, the replication incompetent recombinant retroviral particle can further include an activation element, which can be any activation element provided herein. In illustrative embodiments, the activation element can be anti-CD3, such as anti-CD3 scFv, or anti-CD3 scFvFc. In some embodiments, the contacting can be performed for between 1 and 24 hours, for example, between 1 and 12 hours, or between 1 and 6 hours. In some embodiments, the contacting can be performed for less than 24 hours, for example, less than 12 hours, less than 8 hours, or less than 4 hours. A packing cell, and in illustrative embodiments a packaging cell line, and in particularly illustrative embodiments a packaging cell provided in certain aspects herein, is used to produce the replication incompetent recombinant retroviral particle. In exemplary embodiments of such methods, the genetically modified T cell or NK cell is capable of survival in ex vivo culture for at least 7, 14, 21, 28, 35, 42, or 60 days in the absence of a target antigen recognized by the CAR and in the absence of cytokines such as IL-2, IL-15, or IL-7. In exemplary embodiments of such methods, the genetically modified T cell or NK cell is capable of engraftment *in vivo* in mice and/or enrichment *in vivo* in mice for at least 7, 14, or 28 days. A skilled artisan will recognize that such mice may be treated or otherwise genetically modified so that any immunological differences between the genetically modified T cell and/or NK cell do not result in an immune response being elicited in the mice against any component of the lymphocyte transduced by the replication incompetent recombinant retroviral particle. In some embodiments, the packaging cell line can be a suspension cell line. In illustrative embodiments, the packaging cell line can be grown in serum-free media. In some embodiments, the lymphocyte can be from a subject. In illustrative embodiments, the lymphocyte can be from blood of a subject.

Further embodiments of any of the above aspects for transducing and/or genetically modifying a lymphocyte, for example an NK cell or in illustrative embodiments, a T cell, can include any of the embodiments of replication incompetent recombinant retroviral particles, lymphoproliferative elements, CARs, pseudotyping elements, riboswitches, activation elements, membrane-bound cytokines, miRNAs, and/or other elements disclosed herein, and can be combined with methods herein for producing retroviral particles using a packaging cell. In certain illustrative embodiments, the retroviral particle is a lentiviral particle. Such a method for transducing a T cell and/or NK cell can be performed *in vitro* or *ex vivo.* A skilled artisan will recognize that details provided herein for transducing and/or genetically modifying T cell(s) and/or NK cell(s) can apply to any aspect that includes such step(s), including aspects that are directed to methods for transducing and/or genetically modifying a lymphocyte such as T cell(s) and/or NK cell(s).

Accordingly, provided in one aspect herein is a method for transducing (and/or genetically modifying) lymphocytes, typically resting T cells and/or resting NK cells from isolated blood, comprising:
A. collecting blood from a subject;
B. isolating peripheral blood mononuclear cells (PBMCs) comprising resting T cells and/or resting NK cells; and
C. contacting the resting T cells and/or resting NK cells of the subject *ex vivo,* with replication incompetent recombinant retroviral particles, wherein the replication incompetent recombinant retroviral particles comprise a pseudotyping element on their surface that is capable of binding a resting T cell and/or resting NK cell and facilitating membrane fusion of the replication incompetent recombinant retroviral particles thereto, wherein said contacting facilitates transduction of at least 5% of the resting T cells and/or resting NK cells by the replication incompetent recombinant retroviral particles, thereby producing genetically modified T cells and/or NK cells, thereby transducing resting T cells and/or NK cells.

In some embodiments of any method herein that includes a step of transducing T cells and/or NK cells, the cells can be contacted with a retroviral particle without prior activation. In some embodiments of any method herein that includes a step of transducing T cells and/or NK cells, the T cells and/or NK cells have not been incubated on a substrate that adheres to monocytes for more than 4 hours in one embodiment, or for more than 6, hours in another embodiment, or for more than 8 hours in another embodiment before the transduction. In one illustrative embodiment, the T cells and/or NK cells have been incubated overnight on an adherent substrate to remove monocytes before the transduction. In another embodiment, the method can include incubating the T cells and/or NK cells on an adherent substrate that binds monocytes for no more than 30 minutes, 1 hour, or 2 hours before the transduction. In another embodiment, the T cells and/or NK cells are exposed to no step of removing monocytes by an incubation on an adherent substrate before said transduction step. In another embodiment, the T cells and/or NK cells are not incubated with or exposed to a bovine serum, such as a cell culturing bovine serum, for example fetal bovine serum before or during the transduction.

Accordingly, provided in another aspect herein is a method for genetically modifying or transducing a lymphocyte of a subject, in illustrative embodiments, a T cell and/or and NK cell or a population of T cells or NK cells, that includes contacting the T cell(s) and/or NK cell(s) of, typically of a subject *ex vivo,* with a replication incompetent recombinant retroviral particle comprising in its genome a polynucleotide comprising one or more nucleic acid sequences operatively linked to a promoter active in T cells and/or NK cells, wherein a first nucleic acid sequence of the one or more nucleic acid sequences encodes two or more inhibitory RNA molecules directed against one or more RNA targets and a second nucleic acid sequence of the one or more nucleic acid sequences encodes a chimeric antigen receptor (CAR) comprising an antigen-specific targeting region (ASTR), a transmembrane domain, and an intracellular activating domain, wherein said contacting facilitates transduction of the, or at least some of the resting T cells and/or NK cells by the replication incompetent recombinant retroviral particle, thereby producing a genetically modified T cell and/or NK cell.

Provided herein in another aspect is a method for genetically modifying or transducing a lymphocyte (e.g. a T cell or an NK cell) or a population thereof, of a subject, comprising contacting the lymphocyte (e.g. the T cell or NK cell) or a population thereof, of the subject ex vivo, with a replication incompetent recombinant retroviral particle comprising in its genome a polynucleotide comprising one or more nucleic acid sequences operatively linked to a promoter active in lymphocytes (e.g. T cells and/or NK cells), wherein a first nucleic acid sequence of the one or more nucleic acid sequences encodes one or more (e.g. two or more) inhibitory RNA molecules directed against one or more RNA targets and a second nucleic acid sequence of the one or more nucleic acid sequences encodes a chimeric antigen receptor (CAR) comprising an antigen-specific targeting region (ASTR), a transmembrane domain, and an intracellular activating domain, wherein said contacting facilitates genetic modification and/or transduction of the lymphocyte (e.g. T cell or NK cell), or at least some of the lymphocytes (e.g. T cells and/or NK cells) by the replication incompetent recombinant retroviral particle, thereby producing a genetically modified and/or transduced lymphocyte (e.g. T cell and/or NK cell).

In some embodiments of the method provided immediately above, the genetically modified and/or transduced lymphocyte (e.g. T cell and/or NK cell) or population thereof, is introduced into the subject. In some embodiments, the genetically modified and/or transduced lymphocyte (e.g. T cell and/or NK cell) or population thereof, undergoes 4 or fewer cell divisions ex vivo prior to being introduced or reintroduced into the subject. In some embodiments, the lymphocyte(s) are resting T cells and/or resting NK cells that are in contact with the replication incompetent recombinant retroviral particles for between 1 hour and 12 hours. In some embodiments, no more than 8 hours pass between the time blood is collected from the subject and the time the genetically modified T cells and/or NK cells are reintroduced into the subject. In some embodiments, all steps after the blood is collected and before the blood is reintroduced, are performed in a closed system in which a person monitors the closed system throughout the processing.

In any of the method aspects provided immediately above that include a polynucleotide comprising one or more nucleic acid sequences operatively linked to a promoter active in T cells and/or NK cells, wherein a first nucleic acid sequence of the one or more nucleic acid sequences encodes one or more (e.g. two or more) inhibitory RNA molecules directed against one or more RNA targets, and a second nucleic acid sequence of the one or more nucleic acid sequences encodes a chimeric antigen receptor (CAR) comprising an antigen-specific targeting region (ASTR), a transmembrane domain, and an intracellular activating domain, the polynucleotide may further include a third nucleic acid sequence that encodes at least one lymphoproliferative element that is not an inhibitory RNA molecule. In some embodiments, the lymphoproliferative element can be a cytokine or cytokine receptor polypeptide, or a fragment thereof comprising a signaling domain. In some embodiments, the lymphoproliferative element is constitutively active. In certain embodiments, the lymphoproliferative element can be an IL-7 receptor or a fragment thereof. In illustrative embodiments, the lymphoproliferative element can be a constitutively active IL-7 receptor or a constitutively active fragment thereof.

In any of the method aspects provided immediately above that include a polynucleotide comprising one or more nucleic acid sequences operatively linked to a promoter active in T cells and/or NK cells, wherein a first nucleic acid sequence of the one or more nucleic acid sequences encodes one or more (e.g. two or more) inhibitory RNA molecules directed against one or more RNA targets, an inhibitory RNA molecule can in some embodiments include a 5' strand and a 3' strand that are partially or fully complementary to one another, wherein said 5' strand and said 3' strand are capable of forming an 18-25 nucleotide RNA duplex. In some embodiments, the 5' strand can be 18, 19, 20, 21, 22, 23, 24, or 25 nucleotides in length, and the 3' strand can be 18, 19, 20, 21, 22, 23, 24, or 25 nucleotides in length. In some embodiments, the 5' strand and the 3' strand can be the same or different lengths. In some embodiments, the RNA duplex can include one or more mismatches. In alternate embodiments, the RNA duplex has no mismatches.

In any of the method aspects provided immediately above that include a polynucleotide comprising one or more nucleic acid sequences operatively linked to a promoter active in T cells and/or NK cells, wherein a first nucleic acid sequence of the one or more nucleic acid sequences encodes one or more (e.g. two or more) inhibitory RNA molecules directed against one or more RNA targets, an inhibitory RNA molecule can be a miRNA or an shRNA. In some embodiments, the inhibitory molecule can be a precursor of a miRNA, such as for example, a Pri-miRNA or a Pre-miRNA, or a precursor of an shRNA. In some embodiments, the inhibitory molecule can be an artificially derived miRNA or shRNA. In other embodiments, the inhibitory RNA molecule can be a dsRNA (either transcribed or artificially introduced) that is processed into an siRNA or the siRNA itself. In some embodiments, the inhibitory RNA molecule can be a miRNA or shRNA that has a sequence that is not found in nature, or has at least one functional segment that is not found in nature, or has a combination of functional segments that are not found in nature. In illustrative embodiments, at least one or all of the inhibitory RNA molecules are miR-155.

In any of the method aspects provided immediately above that include a polynucleotide comprising one or more nucleic acid sequences operatively linked to a promoter active in T cells and/or NK cells, wherein a first nucleic acid sequence of the one or more nucleic acid sequences encodes one or more (e.g. two or more) inhibitory RNA molecules directed against one or more RNA targets, an inhibitory RNA molecule, in some embodiments, can comprises from 5' to 3' orientation: a 5' arm, a 5' stem, a loop, a 3' stem that is partially or fully complementary to said 5' stem, and a 3' arm. In some embodiments, at least one of two or more inhibitory RNA molecules has this arrangement. In other embodiments, all of two or more inhibitory molecules have this arrangement. In some embodiments, the 5' stem can be 18, 19, 20, 21, 22, 23, 24 or 25 nucleotides in length. In some embodiments, the 3' stem can be 18, 19, 20, 21, 22, 23, 24, or 25 nucleotides in length. In some embodiments, the loop can be 3, 4,5, 6, 7, 8, 9, 10, 11, 12, 13, 14, 15, 16, 17, 18, 19, 20, 21, 22, 23, 24,2 5, 26, 27, 28, 29, 30, 31, 32, 33, 34, 35, 36, 37, 38, 39, or 40 nucleotides in length. In some embodiments, the 5' arm, 3' arm, or both, are derived from a naturally occurring miRNA. In some embodiments, the 5' arm, 3' arm, or both, are derived from a naturally occurring miRNA is selected from the group consisting of: miR-155, miR-30, miR-17-92, miR-122, and miR-21. In illustrative embodiments, the 5' arm, 3' arm, or both are derived from miR-155. In some embodiments, the 5' arm, 3' arm, or both are derived from Mus musculus miR-155 or Homo sapiens miR-155. In some embodiments, the 5' arm has the sequence set forth in SEQ ID NO:256 or is a functional variant thereof, such as, for example, a sequence that is the same length as SEQ ID NO:256, or 95%, 90%, 85%, 80%,75%, or 50% as long as SEQ ID NO: 256 or is 100 nucleotides or less, 95 nucleotides or less, 90 nucleotides or less, 85 nucleotides or less, 80 nucleotides or less, 75 nucleotides or less, 70 nucleotides or less, 65 nucleotides or less, 60 nucleotides or less, 55 nucleotides or less, 50 nucleotides or less, 45 nucleotides or less, 40 nucleotides or less, 35 nucleotides or less, 30 nucleotides or less, or 25 nucleotides or less; and is at least 50%, 55%, 60%, 65%, 70%, 75%, 80%, 85%, 90%, or 95% identical to SEQ ID NO:256. In some embodiments, the 3' arm has the sequence set forth in SEQ ID NO:260 or is a functional variant thereof, such as, for example, the same length as SEQ ID NO:260, or 95%, 90%, 85%, 80%,75%, or 50% as long as SEQ ID NO: 260 or is a sequence that is 100 nucleotides or less, 95 nucleotides or less, 90 nucleotides or less, 85 nucleotides or less, 80 nucleotides or less, 75 nucleotides or less, 70 nucleotides or less, 65 nucleotides or less, 60 nucleotides or less, 55 nucleotides or less, 50 nucleotides or less, 45 nucleotides or less, 40 nucleotides or less, 35 nucleotides or less, 30 nucleotides or less, or 25 nucleotides or less; and is at least 50%, 55%, 60%, 65%, 70%, 75%, 80%, 85%, 90%, or 95% identical to SEQ ID NO:260. In some embodiments, the 3' arm comprises nucleotides 221-283 of the Mus musculus BIC.

In any of the method aspects provided immediately above that include a polynucleotide comprising one or more nucleic acid sequences operatively linked to a promoter active in T cells and/or NK cells, wherein a first nucleic acid sequence of the one or more nucleic acid sequences encodes two or more inhibitory RNA molecules directed against one or more RNA targets, the two or more inhibitory RNA molecules, in some embodiments, can be positioned in the first nucleic acid sequence in series. In some embodiments, the inhibitory RNA molecules can be adjoined to one another either directly or indirectly by non-functional linker sequence(s). In some embodiments, the linker sequences can be between 5 and 120 nucleotides in length, or between 10 and 40 nucleotides in length.

In any of the method aspects provided immediately above that include a polynucleotide comprising one or more nucleic acid sequences operatively linked to a promoter active in T cells and/or NK cells, wherein a first nucleic acid sequence of the one or more nucleic acid sequences encodes two or more inhibitory RNA molecules directed against one or more RNA targets, in some embodiments, the first nucleic acid sequence encodes two to four inhibitory RNA molecules. In illustrative embodiments, between 2 and 10, 2 and 8, 2 and 6, 2 and 5, 2 and 4, 3 and 5, or 3 and 6 inhibitory RNA molecules are included in the first nucleic acid sequence. In an illustrative embodiment, four inhibitory RNA molecules are included in the first nucleic acid sequence.

In any of the method aspects provided immediately above that include a polynucleotide comprising one or more nucleic acid sequences operatively linked to a promoter active in T cells and/or NK cells, wherein a first nucleic acid sequence of the one or more nucleic acid sequences encodes one or more (e.g. two or more) inhibitory RNA molecules directed against one or more RNA targets, the one or more (e.g. two or more) inhibitory RNA molecules can be in an intron. In some embodiments, the intron is in a promoter. In illustrative embodiments, the intron is EF-1alpha intron A. In some embodiments, the intron is adjacent to and downstream of a promoter, which in illustrative embodiments, is inactive in a packaging cell used to produce the replication incompetent recombinant retroviral particle.

In any of the method aspects provided immediately above that include a polynucleotide comprising one or more nucleic acid sequences operatively linked to a promoter active in T cells and/or NK cells, wherein a first nucleic acid sequence of the one or more nucleic acid sequences encodes two or more inhibitory RNA molecules directed against one or more RNA targets, the two or more inhibitory RNA molecules, in some embodiments, can be directed against different targets. In an alternate embodiment, the two or more inhibitory RNA molecules are directed against the same target. In some embodiments, the RNA targets are mRNAs transcribed from genes that are expressed by T cells such as but not limited to PD-1 (prevent inactivation); CTLA4 (prevent inactivation); TCRa (safety - prevent autoimmunity); TCRb (safety - prevent autoimmunity); CD3Z (safety - prevent autoimmunity); SOCS1 (prevent inactivation); SMAD2 (prevent inactivation); a miR-155 target (promote activation); IFN gamma (reduce CRS); cCBL (prolong signaling); TRAIL2 (prevent death); PP2A (prolong signaling); ABCG1 (increase cholesterol microdomain content by limiting clearance of cholesterol). In some embodiments, the RNA targets are mRNAs transcribed from genes that encode components of the T cell receptor (TCR) complex. In some embodiments, at least one of the two or more of inhibitory RNA molecules can decrease expression of T cell receptors, in illustrative embodiments, one or more endogenous T cell receptor(s) of a T cell. In certain embodiments, the RNA target can be mRNA transcribed from the endogenous TCRα or TCRβ gene of the T cell whose genome comprises the first nucleic acid sequence encoding the one or more miRNAs. In illustrative embodiments, the RNA target is mRNA transcribed from the TCRα gene.

In any of the method aspects provided immediately above that include a polynucleotide comprising one or more nucleic acid sequences operatively linked to a promoter active in T cells and/or NK cells, wherein a first nucleic acid sequence of the one or more nucleic acid sequences encodes one or more (e.g. two or more) inhibitory RNA molecules directed against one or more RNA targets, and a second nucleic acid sequence of the one or more nucleic acid sequences encodes a chimeric antigen receptor (CAR) comprising an antigen-specific targeting region (ASTR), a transmembrane domain, and an intracellular activating domain, in some embodiments, the CAR is a microenvironment restricted biologic (MRB)-CAR. In other embodiments, the ASTR of the CAR binds to a tumor associated antigen. In other embodiments, the ASTR of the CAR is a microenvironment-restricted biologic (MRB)-ASTR.

In any of the method aspects provided immediately above that include a polynucleotide comprising one or more nucleic acid sequences operatively linked to a promoter active in T cells and/or NK cells, wherein a first nucleic acid sequence of the one or more nucleic acid sequences encodes one or more (e.g. two or more) inhibitory RNA molecules directed against one or more RNA targets, and a second nucleic acid sequence of the one or more nucleic acid sequences encodes a chimeric antigen receptor (CAR) comprising an antigen-specific targeting region (ASTR), a transmembrane domain, and an intracellular activating domain, and in some instances a third nucleic acid sequence of the one or more nucleic acid sequences that encodes at least one lymphoproliferative element that is not an inhibitory RNA molecule, in some embodiments, any or all of the first nucleic acid sequence, second nucleic acid sequence, and third nucleic acid sequence is operably linked to a riboswitch. In some embodiments, the riboswitch is capable of binding a nucleoside analog. In some embodiments, the nucleoside analog is an antiviral drug.

In some embodiments, methods are provided for activating and/or genetically modifying, and typically transducing resting T cells or NK cells, in illustrative embodiments resting T cells, by contacting the cells with a retroviral particle disclosed herein and soluble anti-CD3 antibodies at 25-200, 50-150, 75-125, or 100 ng/ml. In illustrative embodiments, such methods are performed without prior activation, and can be carried out, for example, for 8 hours or less, 4 hours or less, or between 2 and 8 hours, 2 and 4 hours, or between 2 and 3 hours.

In certain aspects, provided herein are methods for performing adoptive cell therapy on a subject, As an illustrative example, the method can include the following:
A. collecting blood from a subject;
B. isolating peripheral blood mononuclear cells (PBMCs) comprising resting T cells and/or resting NK cells;
C. contacting the resting T cells and/or resting NK cells of the subject *ex vivo,* with replication incompetent recombinant retroviral particles, wherein the replication incompetent recombinant retroviral particles comprise a pseudotyping element on their surface that is capable of binding a resting T cell and/or NK cell and facilitating membrane fusion of the replication incompetent recombinant retroviral particles thereto, wherein said contacting facilitates transduction of the resting T cells and/or NK cells by the replication incompetent recombinant retroviral particles, thereby producing genetically modified T cells and/or NK cells; and
D. reintroducing the genetically modified cells into the subject within 36, 24, 12, or even 8 hours of collecting blood from the subject, thereby performing adoptive cell therapy in the subject.

In some aspects provided herein, methods with similar steps are referred to as methods for genetically modifying and expanding lymphocytes of a subject. A skilled artisan will understand that the discussion herein as it applies to methods and compositions for performing adoptive cell therapy apply to methods for genetically modifying and expanding lymphocytes of a subject as well.

Typically, the adoptive cell therapy methods of the present disclosure are carried out by autologous transfer, in which the cells are isolated and/or otherwise prepared from the subject who is to receive the cell therapy, or from a sample derived from such a subject. Thus, in some aspects, the cells are derived from a subject, e.g., patient, in need of a treatment and the cells, following isolation and processing are administered to the same subject. In some embodiments of the methods and compositions disclosed herein, a subject having a disease or disorder enters a medical facility where the subject's blood is drawn using known methods, such as venipuncture. In certain embodiments, the volume of blood drawn from a subject is between 10, 15, 20, 25, 30, 35, 40, 50, 75, or 100 ml on the low end of the range and 200, 250, 300, 350, 400, 500, 750, 1000, 2000, or 2500 ml on the high end of the range. In some embodiments, between 10 and 400 ml are drawn from the subject. In some embodiments, between 20 and 250 ml of blood are drawn from the subject. In some embodiments, the blood is fresh when it is processed. In any of the embodiments disclosed herein, fresh blood can be blood that was withdrawn from a subject less than 15, 30, 45, 60, 90, 120, 150, or 180 minutes prior. In some embodiments, the blood is processed in the methods provided herein without storage.

Contact between the T cells and/or NK cells and the replication incompetent recombinant retroviral particles typically facilitates transduction of the T cells and/or NK cells by the replication incompetent recombinant retroviral particles. Throughout this disclosure, a transduced T cell and/or NK cell includes progeny of *ex vivo* transduced cells that retain at least some of the nucleic acids or polynucleotides that are incorporated into the cell during the *ex vivo* transduction. In methods herein that recite "reintroducing" a transduced cell, it will be understood that such cell is typically not in a transduced state when it is collected from the blood of a subject. A subject in any of the aspects disclosed herein can be for example, an animal, a mammal, and in illustrative embodiments a human.

Not to be limited by theory, in non-limiting illustrative methods, the delivery of a polynucleotide encoding a lymphoproliferative element, such as an IL7 constitutively active mutant, to a resting T cell and/or NK cell *ex vivo,* which can integrate into the genome of the T cell or NK cell, provides that cell with a driver for *in vivo* expansion without the need for lymphodepleting the host. Thus, in illustrative embodiments, the subject is not exposed to a lymphodepleting agent within 1, 2, 3, 4, 5, 6, 7, 10, 14, 21, or 28 days, or within 1 month, 2 months, 3 months or 6 months of performing the contacting, during the contacting, and/or within 1, 2, 3, 4, 5, 6, 7, 10, 14, 21, or 28 days, or within 1 month, 2 months, 3 months or 6 months after the modified T cells and/or NK cells are reintroduced back into the subject. Furthermore, in non-limiting illustrative embodiments, methods provided herein can be performed without exposing the subject to a lymphodepleting agent during a step wherein a replication incompetent recombinant retroviral particle is in contact with resting T cells and/or resting NK cells of the subject and/or during the entire *ex vivo* method.

Hence, methods of expanding genetically modified T cells and/or NK cells in a subject in vivo is a feature of some embodiments of the present disclosure. In illustrative embodiments, such methods are ex vivo propagation-free or substantially propagation-free.

This entire method/process from blood draw from a subject to reintroduction of blood back into the subject after *ex vivo* transduction of T cells and/or NK cells, in non-limiting illustrative embodiments herein, can occur over a time period less than 48 hours, less than 36 hours, less than 24 hours, less than 12 hours, less than 11 hours, less than 10 hours, less than 9 hours, less than 8 hours, less than 7 hours, less than 6 hours, less than 5 hours, less than 4 hours, less than 3 hours, 2 hours, or less than 2 hours. In other embodiments, the entire method/process from blood draw/collection from a subject to reintroduction of blood back into the subject after *ex vivo* transduction of T cells and/or NK cells, in non-limiting illustrative embodiments herein, occurs over a time period between 1 hour and 12 hours, or between 2 hours and 8 hours, or between 1 hour and 3 hours, or between 2 hours and 4 hours, or between 2 hours and 6 hours, or between 4 hours and 12 hours, or between 4 hours and 24 hours, or between 8 hours and 24 hours, or between 8 hours and 36 hours, or between 8 hours and 48 hours, or between 12 hours and 24 hours, or between 12 hours and 36 hours, or between 12 hours and 48 hours, or over a time period between 15, 30, 60, 90, 120, 180, and 240 minutes on the low end of the range, and 120, 180, and 240, 300, 360, 420, and 480 minutes on the high end of the range. In other embodiments, the entire method/process from blood draw/collection from a subject to reintroduction of blood back into the subject after *ex vivo* transduction of T cells and/or NK cells, occurs over a time period between 1, 2, 3, 4, 6, 8, 10, and 12 hours on the low end of the range, and 8, 9, 10, 11, 12, 18, 24, 36, or 48 hours on the high end of the range. In some embodiments, the genetically modified T cells and/or NK cells are separated from the replication incompetent recombinant retroviral particles after the time period in which contact occurs.

In some embodiments of any method herein that includes a step of blood collection and a step of transduction of lymphocytes, in illustrative embodiments T cells and/or NK cells, including resting T cell and NK cells, the method from blood collection through transduction of T cells and/or NK cells does not include a step of removing monocytes by an incubation on an adherent substrate of more than 4 hours in one embodiment, or for more than 6, hours in another embodiment, or for more than 8 hours in another embodiment. In one illustrative embodiment, the method from blood collection through transduction of T cells and/or NK cells does not include an overnight incubation on an adherent substrate to remove monocytes. In another embodiment, the method from blood collection through transduction of T cells and/or NK cells includes a step of removing monocytes by an incubation on an adherent substrate for no more than 30 minutes, 1 hour, or 2 hours. In another embodiment, the method from blood collection from a subject through transduction of lymphocytes, in illustrative embodiments T cells and/or NK cells, including resting T cells and/or NK cells, include no step of removing monocytes by an incubation on an adherent substrate. In another embodiment, the method from blood collection from a subject through transduction of lymphocytes, in illustrative embodiments T cells and/or NK cells, including resting T cells and/or NK cells, includes, the T cells and/or NK cells are not incubated with or exposed to a bovine serum such as a cell culturing bovine serum, for example fetal bovine serum during the method.

In some embodiments of any method herein that includes a step of blood collection and a step of transduction of lymphocytes, in illustrative embodiments T cells and/or NK cells, including resting T cell and NK cells, the method from blood collection from a subject through reintroduction of T cells and/or NK cells into the subject does not include a step of removing monocytes by an incubation on an adherent substrate of more than 4 hours in one embodiment, or for more than 6, hours in another embodiment, or for more than 8 hours in another embodiment. In one illustrative embodiment, the method from blood collection from a subject through reintroduction of T cells and/or NK cells into the subject does not include an overnight incubation on an adherent substrate to remove monocytes. In another embodiment, the method from blood collection from a subject through reintroduction of T cells and/or NK cells into the subject includes a step of removing monocytes by an incubation on an adherent substrate for no more than 30 minutes, 1 hour, or 2 hours. In another embodiment, the method from blood collection from a subject through reintroduction of T cells and/or NK cells into the subject includes no step of removing monocytes by an incubation on an adherent substrate. In another embodiment, the method from blood collection from a subject through reintroduction of T cells and/or NK cells into the subject, the T cells and/or NK cells are not incubated with or exposed to a bovine serum, such as a cell culturing bovine serum, for example fetal bovine serum during the method.

Because methods provided herein for adoptive cell therapy and related methods for modifying resting T cells and/or resting NK cells *ex vivo* before expanding them *in vivo,* can be performed in significantly less time than prior methods, fundamental improvements in patient care and safety as well as product manufacturability are made possible. Therefore, such processes are expected to be favorable in the view of regulatory agencies responsible for approving such processes when carried out *in vivo* for therapeutic purposes. For example, the subject in non-limiting examples, can remain in the same building (e.g. infusion clinic) or room as the instrument processing their blood or sample for the entire time that the sample is being processed before modified T cells and/or NK cells are reintroduced into the patient. In non-limiting illustrative embodiments, a subject remains within line of site and/or within 100, 50, 25, or 12 feet or arm's distance of their blood or cells that are being processed, for the entire method/process from blood draw/collection from the subject to reintroduction of blood to the subject after *ex vivo* transduction of T cells and/or NK cells. In other non-limiting illustrative embodiments, a subject remains awake and/or at least one person can continue to monitor the blood or cells of the subject that are being processed, throughout and/or continuously for the entire method/process from blood draw/collection from the subject to reintroduction of blood to the subject after *ex vivo* transduction of T cells and/or NK cells. Because of improvements provided herein, the entire method/process for adoptive cell therapy and/or for transducing resting T cells and/or NK cells from blood draw/collection from the subject to reintroduction of blood to the subject after *ex vivo* transduction of T cells and/or NK cells can be performed with continuous monitoring by a human. In other non-limiting illustrative embodiments, at no point the entire method/process from blood draw/collection from the subject to reintroduction of blood to the subject after *ex vivo* transduction of T cells and/or NK cells, are blood cells incubated in a room that does not have a person present. In other non-limiting illustrative embodiments, the entire method/process from blood draw/collection from the subject to reintroduction of blood to the subject after *ex vivo* transduction of T cells and/or NK cells, is performed next to the subject and/or in the same room as the subject and/or next to the bed or chair of the subject. Thus, sample identity mix-ups can be avoided, as well as long and expensive incubations over periods of days or weeks. This is further provided by the fact that methods provided herein are readily adaptable to closed and automated blood processing systems, where a blood sample and its components that will be reintroduced into the subject, only make contact with disposable, single-use components.

Methods for performing adoptive cell therapy provided herein, typically include 1) methods of transducing lymphocytes, such as T cell(s) or NK cell(s), which in illustrative embodiments are resting T cell(s) and/or NK cell(s), and/or include 2) methods for genetically modifying a lymphocyte such as T cell(s) and/or an NK cell(s), which in illustrative embodiments are resting T cell(s) and/or NK cell(s), both (1 and 2) of which themselves each form distinct aspects of the present disclosure. Such methods can be performed with or without other steps identified herein for performing adoptive cell therapy. In methods for adoptive cell therapy and any method provided herein that include transducing resting T cells and/or resting NK cells *ex vivo,* typically, neutrophils/granulocytes are separated away from the blood cells before the cells are contacted with replication incompetent recombinant retroviral particles. In some embodiments, peripheral blood mononuclear cells (PBMCs) including peripheral blood lymphocytes (PBLs) such as T cell and/or NK cells, are isolated away from other components of a blood sample using for example, apheresis, and/or density gradient centrifugation. In some embodiments, neutrophils are removed before PBMCs and/or T cells and/or NK cells are processed, contacted with a replication incompetent recombinant retroviral particle, transduced, or transfected. With reference to the subject to be treated, the cells may be allogeneic and/or autologous.

As non-limiting examples, in some embodiments for methods of transducing or genetically modifying herein, PBMCs are isolated using a Sepax or Sepax 2 cell processing system (BioSafe). In some embodiments, the PBMCs are isolated using a CliniMACS Prodigy cell processor (Miltenyi Biotec). In some embodiments, an automated apheresis separator is used which takes blood from the subject, passes the blood through an apparatus that sorts out a particular cell type (such as, for example, PBMCs), and returns the remainder back into the subject. Density gradient centrifugation can be performed after apheresis. In some embodiments, the PBMCs are isolated using a leukoreduction filter device. In some embodiments, magnetic bead activated cell sorting is then used for purifying a specific cell population from PBMCs, such as, for example, PBLs or a subset thereof, according to a cellular phenotype (i.e. positive selection). Other methods for purification can also be used, such as, for example, substrate adhesion, which utilizes a substrate that mimics the environment that a T cell encounters during recruitment, allowing them to adhere and migrate, or negative selection, in which unwanted cells are targeted for removal with antibody complexes that target the unwanted cells. In some embodiments, red blood cell rosetting can be used to purify cells.

In some illustrative embodiments of any of the relevant aspects herein, the PBLs include T cells and/or NK cells. The T cells and/or NK cells that are contacted by replication incompetent recombinant retroviral particles of the present disclosure during certain embodiments herein, for example in methods of modifying lymphocytes and methods of performing adoptive cellular therapy, are mainly resting T cells. In some embodiments, the T cells and/or NK cells consist of between 95 and 100% resting cells (Ki-67⁻). In some embodiments, the T cell and/or NK cells that are contacted by replication incompetent recombinant retroviral particles include between 90, 91, 92, 93, 94, and 95% resting cells on the low end of the range and 96, 97, 98, 99, or 100% resting cells on the high end of the range. In some embodiments, the T cells and/or NK cells include naive cells.

In some embodiments of the methods and compositions disclosed herein, T cells and/or NK cells are contacted *ex vivo* with replication incompetent recombinant retroviral particles to genetically modify T cells and/or NK cells to illicit a targeted immune response in the subject when reintroduced into the subject. During the period of contact, the replication incompetent recombinant retroviral particles identify and bind to T cells and/or NK cells at which point the retroviral and host cell membranes start to fuse. Then, through the process of transduction, genetic material from the replication incompetent recombinant retroviral particles enters the T cells and/or NK cells and is incorporated into the host cell DNA. Methods of lentiviral transduction are known. Exemplary methods are described in, e.g., Wang et al. (2012) J. Immunother. 35(9): 689-701; Cooper et al. (2003) Blood. 101:1637-1644; Verhoeyen et al. (2009) Methods Mol Biol. 506: 97-114; and Cavalieri et al. (2003) Blood. 102(2): 497-505.

Many of the methods provided herein include transduction of T cells and/or NK cells. Methods are known in the art for transducing T cells and/or NK cells *ex vivo* with replication incompetent recombinant retroviral particles, such as replication incompetent recombinant lentiviral particles. Methods provided herein, in illustrative embodiments, do not require *ex vivo* stimulation or activation. Thus, this common step in prior methods can be avoided in the present method, although *ex vivo* stimulatory molecule(s) such as anti-CD3 and/or anti-CD28 beads, can be present during the transduction. However, with illustrative methods provided herein, *ex vivo* stimulation is not required. In certain exemplary methods, between 3 and 10 multiplicity of infection (MOI), and in some embodiments, between 5 and 10 MOI units of replication incompetent recombinant retroviral particles, for example lentivirus, can be used.

The transduction reaction can be carried out in a closed system, such as a Sepax system, as discussed herein, wherein the transduction reaction can be carried out in disposable bags loaded on the system. Blood cells, such as PBMCs, from the collected blood sample from the subject, can be contacted with replication incompetent recombinant retroviral particles disclosed herein, in a bag as soon as these blood cells are separated, isolated, and/or purified away from granulocytes, including neutrophils, which are typically not present during the contacting step (i.e. the transduction reaction).

The replication incompetent recombinant retroviral particles can be introduced into the bag that contains the isolated PBMCs, thereby contacting the PBMCs. The time from blood collection from the subject to the time when blood cells, such as PBMCs are added to the transduction reaction bag, can be between 30 minutes and 4 hours, between 30 minutes and 2 hours, or around 1 hour, in some examples. Additives such as media, human serum albumin, human AB+ serum, and/or serum derived from the subject can be added to the transduction reaction mixture. Media is typically present, such as those known in the art for *ex vivo* processes (as non-limiting examples, X-VIVO 15 (Lonza) or CTS media (Thermo Fisher Scientific). Supportive cytokines can be added to the transduction reaction mixture, such as IL2, IL7, or IL15, or those found in HSA.

The transduction reaction mixture can be incubated at between 23 and 39 °C, and in some illustrative embodiments at 37 °C. In certain embodiments, the transduction reaction can be carried out at 37-39 °C for faster fusion/transduction. dGTP can be added to the transduction reaction. The transduction reaction mixture can be incubated for 1 to 12 hours, and in some embodiments, 6 to 12 hrs before a wash is performed regardless of whether such cells will be studied in vitro, ex vivo or introduced into a subject. Accordingly, in certain embodiments where cells are infused into a subject, after transduction, before the transduced T cells and/or NK cells are infused back into the subject, the cells are washed to remove the transduction reaction mixture before being infused back into the subject. For example, the system, such as a Sepax instrument, can be used to wash cells, for example with 10-50 ml of wash solution, before the transduced cells are infused back into the subject. In some embodiments, neutrophils are removed before PBMCs and/or T cells and/or NK cells are processed, contacted with replication incompetent recombinant retroviral particles, transduced, or transfected.

In an illustrative embodiment for performing adoptive cell therapy, blood is collected from a subject into a blood bag and the blood bag is attached to a cell processing system such as a Sepax cell processing system. PBMCs isolated using the cell processing system are collected into a bag, contacted with the replication incompetent recombinant retroviral particles in conditions sufficient to transduce T cells and/or NK cells, and incubated. After incubation, the bag containing the mixture of PBMCs and replication incompetent recombinant retroviral particles is attached to a cell processing system and the PBMCs are washed. The washed PBMCs are collected into a bag and reinfused into the subject. In some embodiments, the entire method, from collecting blood to reinfusing transduced T and/or NK cells, is performed within 1, 2, 3, 4, 5, 6, 7, 8, 9, 10, 11, 12, 15, 18, or 24 hours. In illustrative embodiments, the entire method is performed within 12 hours.

In some embodiments, the target cells for the replication incompetent recombinant retroviral particles are PBLs. In some embodiments, the target cells are T cells and/or NK cells. In some embodiments, the T cells are helper T cells and/or killer T cells.

In some embodiments, the replication incompetent recombinant retroviral particles provided herein have pseudotyping elements on their surface that are capable of binding to T cells and/or NK cells and facilitating membrane fusion of the replication incompetent recombinant retroviral particles thereto. In other embodiments, the replication incompetent recombinant retroviral particles have activation elements on their surface that are capable of binding to resting T cells and/or NK cells. In still other embodiments, the replication incompetent recombinant retroviral particles have membrane-bound cytokines on their surface. In some embodiments, the replication incompetent recombinant retroviral particles include a polynucleotide having one or more transcriptional units encoding one or more engineered signaling polypeptides, one or more of which includes one or more lymphoproliferative elements. In other embodiments, when two signaling polypeptides are utilized, one includes at least one lymphoproliferative element and the other is typically a chimeric antigen receptor (CAR) that includes an antigen-specific targeting region (ASTR), a transmembrane domain, and an intracellular activating domain. As indicated herein, an activation element(s) that is typically associated with the surface of a replication incompetent recombinant retroviral particle provided herein, is capable of, and as a resulting of contacting resting T cells and/or NK cells for a sufficient period of time and under appropriate conditions, activates resting T cells and/or NK cells. It will be understood that such activation occurs over time during a contacting step of methods herein. Furthermore, it will be understood that in some embodiments where a pseudotyping element is found on the surface of a replication incompetent recombinant retroviral particle, that binds a T cell and/or an NK cell, in methods herein, activation can be induced by binding of the pseudotyping element. An activation element is optional in those embodiments.

Further details regarding a pseudotyping element, an activation element, a membrane-bound cytokine, an engineered signaling polypeptide, a lymphoproliferative element, and a CAR are provided in other sections herein.

In some embodiments of the methods and compositions disclosed herein, between 5% and 90% of the total lymphocytes collected from the blood are transduced. In some embodiments, the percent of lymphocytes that are transduced is between 5, 10, 15, 20, 25, 30, 35, 40, 45, 50, 55, and 60% on the low end of the range, and 50, 55, 60, 65, 70, 75, 80, 85, and 90% on the high end of the range. In some embodiments, the percent of lymphocytes that are transduced is at least 5%, at least 10%, at least 15%, at least 20%, at least 25%, at least 30%, at least 35%, at least 40%, at least 45%, at least 50%, at least 55%, or at least 60%.

In some embodiments of the methods and compositions disclosed herein, the genetically modified T cells and/or NK cells are introduced back, reintroduced, or reinfused into the subject without additional *ex vivo* manipulation, such as stimulation and/or activation of T cells and/or NKs. In the prior art methods, *ex vivo* manipulation is used for stimulation/activation of T cells and/or NK cells and for expansion of genetically modified T cells and/or NK cells prior to introducing the genetically modified T cells and/or NK cells into the subject. In prior art methods, this generally takes days or weeks and requires a subject to return to a clinic for a blood infusion days or weeks after an initial blood draw. In some embodiments of the methods and compositions disclosed herein, T cells and/or NK cells are not stimulated *ex vivo* by exposure to anti-CD3/anti-CD28 solid supports such as, for example, beads coated with anti-CD3/anti-CD28, prior to contacting the T cells and/or NK cells with the replication incompetent recombinant retroviral particles. As such provided herein is an *ex vivo* propagation-free method. In other embodiments, genetically modified T cells and/or NK cells are not expanded *ex vivo,* or only expanded for a small number of cell divisions (e.g. 1, 2, 3, 4, 5, 6, 7, 8, 9, or 10 rounds of cell division), but are rather expanded, or predominantly expanded, *in vivo,* i.e. within the subject. In some embodiments, no additional media is added to allow for further expansion of the cells. In some embodiments, no cell manufacturing of the PBLs occurs while the PBLs are contacted with the replication incompetent recombinant retroviral particles. In illustrative embodiments, no cell manufacturing of the PBLs occurs while the PBLs are *ex vivo.* In previous methods of adoptive cell therapy, subjects were lymphodepleted prior to reinfusion with genetically modified T cells and or NK cells. In some embodiments, patients or subjects are not lymphodepleted prior to blood being withdrawn. In some embodiments, patients or subjects are not lymphodepleted prior to reinfusion with genetically modified T cells and or NK cells.

In any of the embodiments disclosed herein, the number of T cells and/or NK cells to be reinfused into a subject can be between 1 x 10³, 2.5 x 10³, 5 x 10³, 1 x 10⁴, 2.5 x 10⁴, 5 x 10⁴, 1 x 10⁵, 2.5 x 10⁵, 5 x 10⁵, 1 x 10⁶, 2.5 x 10⁶, 5 x 10⁶, and 1 x 10⁷ cells/kg on the low end of the range and 5 x 10⁴, 1 x 10⁵, 2.5 x 10⁵, 5 x 10⁵, 1 x 10⁶, 2.5 x 10⁶, 5 x 10⁶, 1 x 10⁷, 2.5 x 10⁷, 5 x 10⁷, and 1 x 10⁸ cells/kg on the high end of the range. In illustrative embodiments, the number of T cells and/or NK cells to be reinfused into a subject can be between 1 x 10⁴, 2.5 x 10⁴, 5 x 10⁴, and 1 x 10⁵ cells/kg on the low end of the range and 2.5 x 10⁴, 5 x 10⁴, 1 x 10⁵, 2.5 x 10⁵, 5 x 10⁵, and 1 x 10⁶ cells/kg on the high end of the range. In some embodiments, the number of PBLs to be reinfused into a subject can be fewer than 5 x 10⁵, 1 x 10⁶, 2.5 x 10⁶, 5 x 10⁶, 1 x 10⁷, 2.5 x 10⁷, 5 x 10⁷, and 1 x 10⁸ cells and the low end of the range and 2.5 x 10⁶, 5 x 10⁶, 1 x 10⁷, 2.5 x 10⁷, 5 x 10⁷, 1 x 10⁸, 2.5 x 10⁸, 5 x 10⁸, and 1 x 10⁹ cells on the high end of the range. In some embodiments, the number of T cells and/or NK cells available for reinfusion into a 70 kg subject or patient is between 7 x 10⁵ and 2.5 x 10⁸ cells. In other embodiments, the number of T cells and/or NK cells available for transduction is approximately 7 x 10⁶ plus or minus 10%.

In the methods disclosed herein, the entire adoptive cell therapy procedure, from withdrawing blood to the reinfusion of genetically modified T cells and/or NK cells, can advantageously be performed in a shorter time than previous methods. In some embodiments, the entire adoptive cell therapy procedure can be performed in less than 1, 2, 3, 4, 5, 6, 7, 8, 9, 10, 11, 12, 15, 18, or 24 hours. In illustrative embodiments, the entire adoptive cell therapy procedure can be performed in less than 1, 2, 3, 4, 5, 6, 7, 8, 9, 10, 11, or 12 hours. In some embodiments, the entire adoptive cell therapy procedure can be performed in between 1, 2, 3, 4, 5, 6, 7, 8, 9, 10, 11, 12, or 15 hours on the low end of the range and 1, 2, 3, 4, 5, 6, 7, 8, 9, 10, 11, 12, 15, 18, or 24 hours on the high end of the range.

In some embodiments herein, a closed system is used to process PBMCs, for example in methods that include genetically modifying PBMCs, NK cells and in illustrative embodiments T cells, for example by transducing the PBMCs or subset(s) thereof. Such methods can be used to genetically modify lymphocytes to be used in scientific research, commercial production, or therapeutic methods. For example, such methods can include transferring peripheral blood mononuclear cells (PBMCs) including NK cells, T cells, or both, and in some embodiments resting T cells, and/or resting NK cells, from a vessel into a transduction reaction mixture within a closed system, which thus is without environmental exposure. The vessel, for example, can be a tube, bag, syringe, or other container. In some embodiments, the vessel is a vessel that is used in a research facility. In some embodiments, the vessel is a vessel used in commercial production. In other embodiments, the vessel can be a collection vessel used in a blood collection process. Methods for genetically modifying herein, typically involve a contacting step wherein lymphocytes are contacted with a replication incompetent recombinant retroviral particle. The contacting in some embodiments, can be performed in the vessel, for example, within a blood bag. In other embodiments, PBMCs or a subfraction thereof, can be transferred from the vessel to another vessel (for example from a first vessel to a second vessel) within the closed system for the contacting. The second vessel can be a cell processing compartment of a closed device, such as a G-Rex device. In some embodiments, after the contacting the genetically modified (e.g. transduced) cells can be transferred to a different vessel within the closed system (i.e. without exposure to the environment). Either before or after this transfer the cells are typically washed within the closed system to remove substantially all or all of the retroviral particles. In some embodiments, the process disclosed in this paragraph from transfer of the PBMCs or a fraction thereof, into the vessel in which the contacting (e.g. transduction) will occur through washing the cells after the contacting, is performed within 12 hours. In some embodiments it is performed for between 1, 2, 3, or 4 hours on the low end of the range, and 4, 8, 10, or 12 hours on the high end of the range.

In some embodiments provided herein, the steps of withdrawing a blood sample from a subject, contacting T cells and/or NK cells with replication incompetent recombinant retroviral particles, and/or introducing genetically modified T cells and/or NK cells into the subject, occur in a closed system. A closed system is a culture process that is generally closed or fully closed to contamination. As such, such a system or process does not expose cells to an environment. An advantage of the present invention, is that provided herein are methods for performing CAR therapy in a closed system. One of the greatest risks to safety and regulatory control in the cell processing procedure is the risk of contamination through frequent exposure to the environment as is found in traditional open cell culture systems. To mitigate this risk, particularly in the absence of antibiotics, some commercial processes have been developed that focus on the use of disposable (single-use) equipment. However even with their use under aseptic conditions, there is always a risk of contamination from the opening of flasks to sample or add additional growth media. To overcome this problem, provided herein is a closed-system process, a process that is designed and can be operated such that the product is not exposed to the outside environment. This is important because the outside environment is typically not sterile. Material transfer occurs via sterile connections or tube welding. Air for gas exchange occurs via a gas permeable membrane or like other additions, via 0.2 µm filter to prevent environmental exposure.

In some embodiments, the closed system includes an *ex vivo* circulating system connected to the *in vivo* circulatory system of the subject such that blood is drawn and then circulated to the *ex vivo* circulatory system before being introduced back into the subject. In some embodiments, the *ex vivo* circulatory system includes a system or apparatus for isolating PBLs and/or a system or apparatus for isolating T cells and/or NK cells, in combination with the system or apparatus for exposing the cells to the replication incompetent recombinant retroviral particles. In some embodiments, the closed system does not allow the T cells and/or NK cells to be exposed to air.

Such closed system methods can be performed with commercially available devices. For example, the method can be carried out in devices adapted for closed system T cell production. Such devices include a G-Rex^{™}, a WAVE Bioreactor^{™}, an OriGen PermaLife^{™} bags, and a VueLife^{®} bags.

In some embodiments of the methods and compositions disclosed herein, genetically modified T cells and/or NK cells within a subject are exposed to a compound that binds to an *in vivo* control element present therein, in which the control element is a part of the genetic material introduced by the replication incompetent recombinant retroviral particles. In some embodiments, the control element can be a riboswitch and the compound can bind the aptamer domain of the riboswitch. In some embodiments, the control element can be a molecular chaperone. In any of the embodiments disclosed herein, the compound can be a nucleoside analogue. In some embodiments, the nucleoside analogue can be a nucleoside analogue antiviral drug, wherein an antiviral drug is a compound approved by the Food and Drug Administration for antiviral treatment or a compound in an antiviral clinical trial in the United States. In illustrative embodiments, the compound can be acyclovir or penciclovir. In some embodiments, the compound can be famciclovir, the oral prodrug of penciclovir, or valaciclovir, the oral prodrug of acyclovir. Binding of the compound to the control element affects expression of the introduced genetic material and hence, propagation of genetically modified T cells and/or NK cells.

In some embodiments, the nucleoside analogue antiviral drug or prodrug, for example acyclovir, valaciclovir, penciclovir or famciclovir, is administered to the subject prior to, concurrent with, and/or following PBLs being isolated from the blood of the subject and before T cells and/or NK cells are contacted with replication incompetent recombinant retroviral particles. In some embodiments, the nucleoside analogue antiviral drug or prodrug is administered to the subject for between 5, 10, 15, 30, and 60 minutes on the low end of the range and 1.5, 2, 3, 4, 5, 6, 8, 12, or 24 hours on the high end of the range prior to PBLs being isolated from the blood or prior to T cells and/or NK cells being contacted with replication incompetent recombinant retroviral particles. In other embodiments, the nucleoside analogue antiviral drug or prodrug is administered to the subject for between 1.5, 2, 3, 4, 5, 6, 8, 12, or 24 hours on the low end of the range and ½, 1, 2, 3, 4, 5, 6, 7, 10, 14, 21, or 28 days on the high end of the range after PBLs are isolated from the blood and T cells and/or NK cells are contacted with replication incompetent recombinant retroviral particles in methods provided herein. In some embodiments, the nucleoside analogue antiviral drug or prodrug is administered to the subject for at least 1.5, 2, 3, 4, 5, 6, 8, 12, or 24 hours, or at least 2, 3, 4, 5, 6, 7, 10, 14, 21, or 28 days after PBLs are isolated from the blood and T cells and/or NK cells are contacted with replication incompetent recombinant retroviral particles in methods provided herein. In some embodiments, the nucleoside analogue antiviral drug or prodrug is administered to the subject for at least 1, 2, 3, 4, 5, 7, 10, 14, 21, 28, 30, 60, 90, or 120 days or 5, 6, 9, 12, 24, 36, 48, 60, 72, 84, 96, 120 months or indefinitely after the PBLs have been reinfused into the subject. In any of the embodiments disclosed herein, the nucleoside analogue antiviral drug or prodrug can be administered before and/or during the reinfusion of the PBLs and/or after the PBLs have been reinfused.

In some embodiments, the compound that binds to the control element is administered once, twice, three times, or four times daily to the subject. In some embodiments, daily doses of the compound are provided for 1 week, 2 weeks, 4 weeks, 3 months, 6 months, 1 year, until a subject is disease free, such as cancer free, or indefinitely. The drug, in illustrative embodiments is a nucleoside analogue antiviral drug that binds to a nucleoside analog, such as a riboswitch, as disclosed in further detail herein.

Methods are known in the art for delivering drugs, whether small molecules or biologics, and can be used in methods provided herein. Any such methods can be used to deliver drugs or candidate compounds or antibodies for use in methods as presently described herein. For example, common routes of administration include non-invasive peroral (through the mouth), topical (skin), transmucosal (nasal, buccal/sublingual, vaginal, ocular and rectal) and inhalation routes. Many protein and peptide drugs, such as monoclonal antibodies, have to be delivered by injection or a nanoneedle array. For example, many immunizations are based on the delivery of protein drugs and are often done by injection.

### ENGINEERED SIGNALING POLYPEPTIDE(S)

In some embodiments, the replication incompetent recombinant retroviral particles used to contact T cells and/or NK cells have a polynucleotide having one or more transcriptional units that encode one or more engineered signaling polypeptides. In some embodiments, an engineered signaling polypeptide includes any combination of an extracellular domain (e.g. an antigen-specific targeting region or ASTR), an optional a stalk and a transmembrane domain, combined with one or more intracellular activating domains, one or more modulatory domains (such as a co-stimulatory domain), and one or more T cell survival motifs. In illustrative embodiments, at least one, two, or all of the engineered signaling polypeptides is a chimeric antigen receptor (CAR) or a lymphoproliferative element including a chimeric lymphoproliferative element (CLE). In some embodiments, when two signaling polypeptides are utilized, one encodes a lymphoproliferative element and the other encodes a chimeric antigen receptor (CAR) that includes an antigen-specific targeting region (ASTR), a transmembrane domain, and an intracellular activating domain. One of ordinary skill in the art would be able to configure the system to put the lymphoproliferative element and the CAR on distinct polynucleotides with similar or dissimilar control elements for the methods and compositions disclosed herein. A skilled artisan will recognize that such engineered polypeptides can also be referred to as recombinant polypeptides.

### Extracellular domain

In some embodiments, an engineered signaling polypeptide includes an extracellular domain that is a member of a specific binding pair. For example, in some embodiments, the extracellular domain can be the extracellular domain of a cytokine receptor, or a mutant thereof, or a hormone receptor, or a mutant thereof. Such mutant extracellular domains in some embodiments have been reported to be constitutively active when expressed at least in some cell types. In illustrative embodiments, such extracellular and transmembrane domains do not include a ligand binding region. It is believed that such domains do not bind a ligand when present in an engineered signaling polypeptide and expressed in B cells, T cells, and/or NK cells. Mutations in such receptor mutants can occur in the extracellular juxtamembrane region. Not to be limited by theory, a mutation in at least some extracellular domains (and some extracellular-transmembrane domains) of engineered signaling polypeptides provided herein, are responsible for signaling of the engineered signaling polypeptide in the absence of ligand, by bringing activating chains together that are not normally together. Further embodiments regarding extracellular domains that comprise mutations in extracellular domains can be found, for example, in the Lymphoproliferative Element section herein.

In certain illustrative embodiments, the extracellular domain comprises a dimerizing motif. In an illustrative embodiment the dimerizing motif comprises a leucine zipper. In some embodiments, the leucine zipper is from a jun polypeptide, for example c-jun. Further embodiments regarding extracellular domains that comprise a dimerizing motif can be found, for example, in the Lymphoproliferative Element section herein.

In certain embodiments, the extracellular domain is an antigen-specific targeting region (ASTR), sometimes called an antigen binding domain herein. Specific binding pairs include, but are not limited to, antigen-antibody binding pairs; ligand-receptor binding pairs; and the like. Thus, a member of a specific binding pair suitable for use in an engineered signaling polypeptide of the present disclosure includes an ASTR that is an antibody, an antigen, a ligand, a receptor binding domain of a ligand, a receptor, a ligand binding domain of a receptor, and an affibody.

An ASTR suitable for use in an engineered signaling polypeptide of the present disclosure can be any antigen-binding polypeptide. In certain embodiments, the ASTR is an antibody such as a full-length antibody, a single-chain antibody, an Fab fragment, an Fab' fragment, an (Fab')2 fragment, an Fv fragment, and a divalent single-chain antibody or a diabody.

In some embodiments, the ASTR is a single chain Fv (scFv). In some embodiments, the heavy chain is positioned N-terminal of the light chain in the engineered signaling polypeptide. In other embodiments, the light chain is positioned N-terminal of the heavy chain in the engineered signaling polypeptide. In any of the disclosed embodiments, the heavy and light chains can be separated by a linker as discussed in more detail herein. In any of the disclosed embodiments, the heavy or light chain can be at the N-terminus of the engineered signaling polypeptide and is typically C-terminal of another domain, such as a signal sequence or peptide.

Other antibody-based recognition domains (cAb VHH (camelid antibody variable domains) and humanized versions, IgNAR VH (shark antibody variable domains) and humanized versions, sdAb VH (single domain antibody variable domains) and "camelized" antibody variable domains are suitable for use with the engineered signaling polypeptides and methods using the engineered signaling polypeptides of the present disclosure. In some instances, T cell receptor (TCR) based recognition domains such as single chain TCR (scTv, single chain two-domain TCR containing VαVβ) are also suitable for use.

In some embodiments, the ASTR can be multispecific, e.g. bispecific antibodies. Multispecific antibodies have binding specificities for at least two different sites. In certain embodiments, one of the binding specificities is for one target antigen and the other is for another target antigen. In certain embodiments, bispecific antibodies may bind to two different epitopes of ta target antigen. Bispecific antibodies may also be used to localize cytotoxic agents to cells which express a target antigen. Bispecific antibodies can be prepared as full length antibodies or antibody fragments.

An ASTR suitable for use in an engineered signaling polypeptide of the present disclosure can have a variety of antigen-binding specificities. In some cases, the antigen-binding domain is specific for an epitope present in an antigen that is expressed by (synthesized by) a target cell. In one example, the target cell is a cancer cell associated antigen. The cancer cell associated antigen can be an antigen associated with, e.g., a breast cancer cell, a B cell lymphoma, a Hodgkin lymphoma cell, an ovarian cancer cell, a prostate cancer cell, a mesothelioma, a lung cancer cell (e.g., a small cell lung cancer cell), a non-Hodgkin B-cell lymphoma (B-NHL) cell, an ovarian cancer cell, a prostate cancer cell, a mesothelioma cell, a lung cancer cell (e.g., a small cell lung cancer cell), a melanoma cell, a chronic lymphocytic leukemia cell, an acute lymphocytic leukemia cell, a neuroblastoma cell, a glioma, a glioblastoma, a medulloblastoma, a colorectal cancer cell, etc. A cancer cell associated antigen may also be expressed by a non-cancerous cell.

Non-limiting examples of antigens to which an ASTR of an engineered signaling polypeptide can bind include, e.g., CD19, CD20, CD38, CD30, ERBB2, CA125, MUC-1, prostate-specific membrane antigen (PSMA), CD44 surface adhesion molecule, mesothelin, carcinoembryonic antigen (CEA), epidermal growth factor receptor (EGFR), EGFRvIII, vascular endothelial growth factor receptor-2 (VEGFR2), high molecular weight-melanoma associated antigen (HMW-MAA), MAGE-Al, IL-13R-a2, GD2, Axl, Ror2, and the like.

In some cases, a member of a specific binding pair suitable for use in an engineered signaling polypeptide is an ASTR that is a ligand for a receptor. Ligands include, but are not limited to, hormones (e.g. erythropoietin, growth hormone, leptin, etc.); cytokines (e.g., interferons, interleukins, certain hormones, etc.); growth factors (e.g., heregulin; vascular endothelial growth factor (VEGF); and the like); an integrin-binding peptide (e.g., a peptide comprising the sequence Arg-Gly-Asp); and the like.

Where the member of a specific binding pair in an engineered signaling polypeptide is a ligand, the engineered signaling polypeptide can be activated in the presence of a second member of the specific binding pair, where the second member of the specific binding pair is a receptor for the ligand. For example, where the ligand is VEGF, the second member of the specific binding pair can be a VEGF receptor, including a soluble VEGF receptor.

As noted above, in some cases, the member of a specific binding pair that is included in an engineered signaling polypeptide is an ASTR that is a receptor, e.g., a receptor for a ligand, a co-receptor, etc. The receptor can be a ligand-binding fragment of a receptor. Suitable receptors include, but are not limited to, a growth factor receptor (e.g., a VEGF receptor); a killer cell lectin-like receptor subfamily K, member 1 (NKG2D) polypeptide (receptor for MICA, MICB, and ULB6); a cytokine receptor (e.g., an IL-13 receptor; an IL-2 receptor; etc.); CD27; a natural cytotoxicity receptor (NCR) (e.g., NKP30 (NCR3/CD337) polypeptide (receptor for HLA-B-associated transcript 3 (BAT3) and B7-H6); etc.); etc.

In certain embodiments of any of the aspects provided herein that include an ASTR, the ASTR can be directed to an intermediate protein that links the ASTR with a target molecule expressed on a target cell. The intermediate protein may be endogenously expressed or introduced exogenously and may be natural, engineered, or chemically modified. In certain embodiments the ASTR can be an anti-tag ASTR such that at least one tagged intermediate, typically an antibody-tag conjugate, is included between a tag recognized by the ASTR and a target molecule, typically a protein target, expressed on a target cell. Accordingly, in such embodiments, the ASTR binds a tag and the tag is conjugated to an antibody directed against an antigen on a target cell, such as a cancer cell. Non-limiting examples of tags include fluorescein isothiocyanate (FITC), streptavidin, biotin, histidine, dinitrophenol, peridinin chlorophyll protein complex, green fluorescent protein, phycoerythrin (PE), horse radish peroxidase, palmitoylation, nitrosylation, alkaline phosphatase, glucose oxidase, and maltose binding protein. As such, the ASTR comprises a molecule that binds the tag.

### Stalk

In some embodiments, the engineered signaling polypeptide includes a stalk which is located in the portion of the engineered signaling polypeptide lying outside the cell and interposed between the ASTR and the transmembrane domain. In some cases, the stalk has at least 85, 90, 95, 96, 97, 98, 99, or 100% identity to a wild-type CD8 stalk region (TTTPAPRPPTPAPTIASQPLSLRPEACRPAAGG AVHTRGLDFA (SEQ ID NO:79), has at least 85, 90, 95, 96, 97, 98, 99, or 100% identity to a wild-type CD28 stalk region (FCKIEVMYPPPYLDNEKSNGTIIHVKGKHLCPSPLFPGPSKP (SEQ ID NO:80)), or has at least 85, 90, 95, 96, 97, 98, 99, or 100% identity to a wild-type immunoglobulin heavy chain stalk region. In an engineered signaling polypeptide, the stalk employed allows the antigen-specific targeting region, and typically the entire engineered signaling polypeptide, to retain increased binding to a target antigen.

The stalk region can have a length of from about 4 amino acids to about 50 amino acids, e.g., from about 4 aa to about 10 aa, from about 10 aa to about 15 aa, from about 15 aa to about 20 aa, from about 20 aa to about 25 aa, from about 25 aa to about 30 aa, from about 30 aa to about 40 aa, or from about 40 aa to about 50 aa.

In some cases, the stalk of an engineered signaling polypeptide includes at least one cysteine. For example, in some cases, the stalk can include the sequence Cys-Pro-Pro-Cys (SEQ ID NO:62). If present, a cysteine in the stalk of a first engineered signaling polypeptide can be available to form a disulfide bond with a stalk in a second engineered signaling polypeptide.

Stalks can include immunoglobulin hinge region amino acid sequences that are known in the art; see, e.g., Tan et al. (1990) *Proc. Natl. Acad. Sci. USA* 87:162; and Huck et al. (1986) *Nucl. Acids Res.* 14:1779. As non-limiting examples, an immunoglobulin hinge region can include a domain with at least 50, 60, 70, 75, 80, 85, 90, 95, 96, 97, 98, 99 or 100% sequence identity to a stretch of at least 10, 15, 20, or all of the amino acids of any of the following amino acid sequences: DKTHT (SEQ ID NO:63); CPPC (SEQ ID NO:62); CPEPKSCDTPPPCPR (SEQ ID NO:64) (see, e.g., Glaser et al. (2005) *J. Biol. Chem.* 280:41494); ELKTPLGDTTHT (SEQ ID NO:65); KSCDKTHTCP (SEQ ID NO:66); KCCVDCP (SEQ ID NO:67); KYGPPCP (SEQ ID NO:68); EPKSCDKTHTCPPCP (SEQ ID NO:69) (human IgGl hinge); ERKCCVECPPCP (SEQ ID NO:70) (human IgG2 hinge); ELKTPLGDTTHTCPRCP (SEQ ID NO:71) (human IgG3 hinge); SPNMVPHAHHAQ (SEQ ID NO:72) (human IgG4 hinge); and the like. The stalk can include a hinge region with an amino acid sequence of a human IgG1, IgG2, IgG3, or IgG4, hinge region. The stalk can include one or more amino acid substitutions and/or insertions and/or deletions compared to a wild-type (naturally-occurring) hinge region. For example, His229 of human IgG 1 hinge can be substituted with Tyr, so that the stalk includes the sequence EPKSCDKTYTCPPCP (see, e.g., Yan et al. (2012) J. Biol. Chem. 287:5891). The stalk can include an amino acid sequence derived from human CD8; e.g., the stalk can include the amino acid sequence:
TTTPAPRPPTPAPTIASQPLSLRPEACRPAAGGAVHTRGLDFACD (SEQ ID NO:73), or a variant thereof.

### Transmembrane domain

An engineered signaling polypeptide of the present disclosure can include transmembrane domains for insertion into a eukaryotic cell membrane. The transmembrane domain can be interposed between the ASTR and the co-stimulatory domain. The transmembrane domain can be interposed between the stalk and the co-stimulatory domain, such that the chimeric antigen receptor includes, in order from the amino terminus (N-terminus) to the carboxyl terminus (C-terminus): an ASTR; a stalk; a transmembrane domain; and an activating domain.

Any transmembrane (TM) domain that provides for insertion of a polypeptide into the cell membrane of a eukaryotic (e.g., mammalian) cell is suitable for use in aspects and embodiments disclosed herein. Non-limiting examples of TM domains suitable for any of the aspects or embodiments provided herein, include a domain with at least 50, 60, 70, 75, 80, 85, 90, 95, 96, 97, 98, 99 or 100% sequence identity to a stretch of at least 10, 15, 20, or all of the amino acids of any of the following TM domains or combined stalk and TM domains: a) CD8 alpha TM (SEQ ID NO:46); b) CD8 beta TM (SEQ ID NO:47); c) CD4 stalk (SEQ ID NO:48); d) CD3Z TM (SEQ ID NO:49); e) CD28 TM (SEQ ID NO:50); f) CD134 (OX40) TM: (SEQ ID NO:51); g) CD7 TM (SEQ ID NO:52); h) CD8 stalk and TM (SEQ ID NO:75); and i) CD28 stalk and TM (SEQ ID NO:76).

As non-limiting examples, a transmembrane domain as described herein can have at least 80, 90, or 95% sequence identity to the SEQ ID NO:46 transmembrane domain, the CD8 beta transmembrane domain, the CD4 transmembrane domain, the CD3 zeta transmembrane domain, the CD28 transmembrane domain, the CD134 transmembrane domain, or the CD7 transmembrane domain.

### Intracellular activating domain

Intracellular activating domains suitable for use in an engineered signaling polypeptide of the present disclosure when activated, typically induce the production of one or more cytokines; increased cell death; and/or increased proliferation of CD8⁺ T cells, CD4⁺ T cells, NKT cells, γδ T cells, and/or neutrophils. Activating domains can also be referred to as activation domains herein. Activating domains can be used in CARs or in lymphoproliferative elements provided herein.

In some embodiments, the intracellular activating domain includes at least one (e.g., one, two, three, four, five, six, etc.) ITAM motifs as described below. In some embodiments, an intracellular activating domain can have at least 80%, 90%, or 95% sequence identity to the CD3Z, CD3D, CD3E, CD3G, CD79A, CD79B, DAP12, FCERIG, FCGR2A, FCGR2C. DAP10/CD28, or ZAP70 domains as described below.

Intracellular activating domains suitable for use in an engineered signaling polypeptide of the present disclosure include immunoreceptor tyrosine-based activation motif (ITAM)-containing intracellular signaling polypeptides. An ITAM motif is YX₁X₂L/I, where X₁ and X₂ are independently any amino acid. In some cases, the intracellular activating domain of an engineered signaling polypeptide includes 1, 2, 3, 4, or 5 ITAM motifs. In some cases, an ITAM motif is repeated twice in an intracellular activating domain, where the first and second instances of the ITAM motif are separated from one another by 6 to 8 amino acids, e.g., (YX₁X₂L/I)(X₃)ₙ(YX₁X₂L/I), where n is an integer from 6 to 8, and each of the 6-8 X₃ can be any amino acid. In some cases, the intracellular activating domain of an engineered signaling polypeptide includes 3 ITAM motifs.

A suitable intracellular activating domain can be an ITAM motif-containing portion that is derived from a polypeptide that contains an ITAM motif. For example, a suitable intracellular activating domain can be an ITAM motif-containing domain from any ITAM motif-containing protein. Thus, a suitable intracellular activating domain need not contain the entire sequence of the entire protein from which it is derived. Examples of suitable ITAM motif-containing polypeptides include, but are not limited to: CD3Z (CD3 zeta); CD3D (CD3 delta); CD3E (CD3 epsilon); CD3G (CD3 gamma); CD79A (antigen receptor complex-associated protein alpha chain); DAP12; and FCERlG (Fc epsilon receptor I gamma chain).

In some cases, the intracellular activating domain is derived from T cell surface glycoprotein CD3 zeta chain (also known as CD3Z, T cell receptor T3 zeta chain, CD247, CD3-ZETA, CD3H, CD3Q, T3Z, TCRZ, etc.). For example, a suitable intracellular activating domain can include a domain with at least 50%, 60%, 70%, 75%, 80%, 85%, 90%, 95%, 96%, 97%, 98%, 99% or 100% sequence identity to a stretch of at least 10, 15, 20, or all amino acids in the following sequences or to a contiguous stretch of from about 100 amino acids to about 110 amino acids (aa), from about 110 aa to about 115 aa, from about 115 aa to about 120 aa, from about 120 aa to about 130 aa, from about 130 aa to about 140 aa, from about 140 aa to about 150 aa, or from about 150 aa to about 160 aa, of either of the following amino acid sequences (2 isoforms): 
MKWKALFTAAILQAQLPITEAQSFGLLDPKLCYLLDGILFIYGVILTALFLRVKFSRSADAPAYQQ
GQNQL[YNELNLGRREEYDVL]DKRRGRDPEMGGKPRRKNPQEGL[YNELQKDKMAEAYSEI]G
MKGERRRGKGHDGL[YQGLSTATKDTYDAL]HMQALPPR (SEQ ID NO:11) or
 
MKWKALFTAAILQAQLPITEAQSFGLLDPKLCYLLDGILFIYGVILTALFLRVKFSRSADAPAYQQ
GQNQL[YNELNLGRREEYDVL]DKRRGRDPEMGGKPQRRKNPQEGL[YNELQKDKMAEAYSEI]
GMKGERRRGKGHDGL[YQGLSTATKDTYDAL]HMQALPPR (SEQ ID NO:12),
where the ITAM
motifs are set out with brackets.

Likewise, a suitable intracellular activating domain polypeptide can include 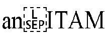 motif-containing a portion of the full length CD3 zeta amino acid sequence. Thus, a suitable intracellular activating domain can include a domain with at least 50%, 60%, 70%, 75%, 80%, 85%, 90%, 95%, 96%, 97%, 98%, 99% or 100% sequence identity to a stretch of at least 10, 15, 20, or all amino acids in the following sequences or to a contiguous stretch of from about 100 amino acids to about 110 amino acids (aa), from about 110 aa to about 115 aa, from about 115 aa to about 120 aa, from about 120 aa to about 130 aa, from about 130 aa to about 140 aa, from about 140 aa to about 150 aa, or from about 150 aa to about 160 aa, of either of the following amino acid sequences: 
RVKFSRSADAPAYQQGQNQL[YNELNLGRREEYDVL]DKRRGRDPEMGGKPRRKNPQEGL[YNE
LQKDKMAEAYSEI]GMKGERRRGKGHDGL[YQGLSTATKDTYDAL]HMQALPPR (SEQ ID
NO:13);
 
RVKFSRSADAPAYQQGQNQL[YNELNLGRREEYDVL]DKRRGRDPEMGGKPQRRKNPQEGL[YN
ELQKDKMAEAYSEI]GMKGERRRGKGHDGL[YQGLSTATKDTYDAL]HMQALPPR (SEQ ID
NO:81); NQL[YNELNLGRREEYDVL]DKR SEQ ID NO:14); EGL[YNELQKDKMAEAYSEI]GMK
(SEQ ID NO:15); or DGL[YQGLSTATKDTYDAL]HMQ (SEQ ID NO:16),
where the ITAM motifs are
set out in brackets.

In some cases, the intracellular activating domain is derived from T cell surface glycoprotein CD3 delta chain (also known as CD3D; CD3-DELTA; T3D; CD3 antigen, delta subunit; CD3 delta; CD3d antigen, delta polypeptide (TiT3 complex); OKT3, delta chain; T cell receptor T3 delta chain; T cell surface glycoprotein CD3 delta chain; etc.). Thus, a suitable intracellular activating domain can include a domain with at least 50%, 60%, 70%, 75%, 80%, 85%, 90%, 95%, 96%, 97%, 98%, 99% or 100% sequence identity to a stretch of at least 10, 15, 20, or all amino acids in the following sequences or to a contiguous stretch of from about 100 amino acids to about 110 amino acids (aa), from about 110 aa to about 115 aa, from about 115 aa to about 120 aa, from about 120 aa to about 130 aa, from about 130 aa to about 140 aa, from about 140 aa to about 150 aa, or from about 150 aa to about 160 aa, of 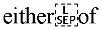 the following amino acid sequences: 
MEHSTFLSGLVLATLLSQVSPFKIPIEELEDRVFVNCNTSITWVEGTVGTLLSDITRLDLGKRILDP
RGIYRCNGTDIYKDKESTVQVHYRMCQSCVELDPATVAGIIVTDVIATLLLALGVFCFAGHETGR
LSGAADTQALLRNDQV[YQPLRDRDDAQYSHL]GGNWARNK (SEQ ID NO:17) or
 
MEHSTFLSGLVLATLLSQVSPFKIPIEELEDRVFVNCNTSITWVEGTVGTLLSDITRLDLGKRILDP
RGIYRCNGTDIYKDKESTVQVHYRTADTQALLRNDQV[YQPLRDRDDAQYSHL]GGNWARNK
(SEQ ID NO:18),
 where the ITAM motifs are set out in brackets.

Likewise, a suitable intracellular activating domain polypeptide can comprise an ITAM motif-containing portion of the full length CD3 delta amino acid sequence. Thus, a suitable intracellular activating domain can include a domain with at least 50%, 60%, 70%, 75%, 80%, 85%, 90%, 95%, 96%, 97%, 98%, 99% or 100% sequence identity to a stretch of at least 10, 15, 20, or all amino acids in the following sequence: DQV[YQPLRDRDDAQYSHL]GGN (SEQ ID NO: 19), where the ITAM motifs are set out in brackets.

In some cases, the intracellular activating domain is derived from T cell surface glycoprotein CD3 epsilon chain (also known as CD3e, T cell surface antigen T3/Leu-4 epsilon chain, T cell surface glycoprotein CD3 epsilon chain, AI504783, CD3, CD3epsilon, T3e, etc.). Thus, a suitable intracellular activating domain can include a domain with at least 50%, 60%, 70%, 75%, 80%, 85%, 90%, 95%, 96%, 97%, 98%, 99% or 100% sequence identity to a stretch of at least 10, 15, 20, or all amino acids in the following sequences or to a contiguous stretch of from about 100 amino acids to about 110 amino acids (aa), from about 110 aa to about 115 aa, from about 115 aa to about 120 aa, from about 120 aa to about 130 aa, from about 130 aa to about 140 aa, from about 140 aa to about 150 aa, or from about 150 aa to about 160 aa, of the following amino acid sequence:
MQSGTHWRVLGLCLLSVGVWGQDGNEEMGGITQTPYKVSISGTTVILTCPQYPGSEILWQHNDK NIGGDEDDKNIGSDEDHLSLKEFSELEQSGYYVCYPRGSKPEDANFYLYLRARVCENCMEMDMS VATIVIVDICITGGLLLLVYYWSKNRKAKAKPVTRGAGAGGRQRGQNKERPPPVPNPD[YEPIRK GQRDLYSGL]NQRRI (SEQ ID NO:20), where the ITAM motifs are set out in brackets.

Likewise, a suitable intracellular activating domain polypeptide can comprise an ITAM motif-containing portion of the full length CD3 epsilon amino acid sequence. Thus, a suitable intracellular activating domain can include a domain with at least 50%, 60%, 70%, 75%, 80%, 85%, 90%, 95%, 96%, 97%, 98%, 99% or 100% sequence identity to a stretch of at least 10, 15, 20, or all amino acids in the following sequence: NPD[YEPIRKGQRDLYSGL]NQR (SEQ ID NO:21), where the ITAM motifs are set out in brackets.

In some cases, the intracellular activating domain is derived from T cell surface glycoprotein CD3 gamma chain (also known as CD3G, T cell receptor T3 gamma chain, CD3-GAMMA, T3G, gamma polypeptide (TiT3 complex), etc.). Thus, a suitable intracellular activating domain can include a domain with at least 50%, 60%, 70%, 75%, 80%, 85%, 90%, 95%, 96%, 97%, 98%, 99% or 100% sequence identity to a stretch of at least 10, 15, 20, or all amino acids in the following sequences or to a contiguous stretch of from about 100 amino acids to about 110 amino acids (aa), from about 110 aa to about 115 aa, from about 115 aa to about 120 aa, from about 120 aa to about 130 aa, from about 130 aa to about 140 aa, from about 140 aa to about 150 aa, or from about 150 aa to about 160 aa, of the following amino acid sequence:
MEQGKGLAVLILAIILLQGTLAQSIKGNHLVKVYDYQEDGSVLLTCDAEAKNITWFKDGKMIGF LTEDKKKWNLGSNAKDPRGMYQCKGSQNKSKPLQVYYRMCQNCIELNAATISGFLFAEIVSIFV LAVGVYFIAGQDGVRQSRASDKQTLLPNDQL[YQPLKDREDDQYSHL]QGNQLRRN (SEQ ID NO:22), where the ITAM motifs are set out in brackets.

Likewise, a suitable intracellular activating domain polypeptide can comprise an ITAM motif-containing portion of the full length CD3 gamma amino acid sequence. Thus, a suitable intracellular activating domain can include a domain with at least 50%, 60%, 70%, 75%, 80%, 85%, 90%, 95%, 96%, 97%, 98%, 99% or 100% sequence identity to a stretch of at least 10, 15, 20, or all amino acids in the following sequence: DQL[YQPLKDREDDQYSHL]QGN (SEQ ID NO:23), where the ITAM motifs are set out in brackets.

In some cases, the intracellular activating domain is derived from CD79A (also known as B-cell antigen receptor complex-associated protein alpha chain; CD79a antigen (immunoglobulin-associated alpha); MB-1 membrane glycoprotein; Ig-alpha; membrane-bound immunoglobulin-associated protein; surface IgM-associated protein; etc.). Thus, a suitable intracellular activating domain can include a domain with at least 50%, 60%, 70%, 75%, 80%, 85%, 90%, 95%, 96%, 97%, 98%, 99% or 100% sequence identity to a stretch of at least 10, 15, 20, or all amino acids in the following sequences or to a contiguous stretch of from about 100 amino acids to about 110 amino acids (aa), from about 110 aa to about 115 aa, from about 115 aa to about 120 aa, from about 120 aa to about 130 aa, from about 130 aa to about 140 aa, from about 140 aa to about 150 aa, or from about 150 aa to about 160 aa, of either of the following amino acid sequences: 



 (SEQ ID NO:24)
 or 
MPGGPGVLQALPATIFLLFLLSAVYLGPGCQALWMHKVPASLMVSLGEDAHFQCPHNSSNNAN
VTWWRVLHGNYTWPPEFLGPGEDPNEPPPRPFLDMGEGTKNRIITAEGIILLFCAVVPGTLLLFRK
RWQNEKLGLDAGDEYEDENL[YEGLNLDDCSMYEDI]SRGLQGTYQDVGSLNIGDVQLEKP
(SEQ ID NO:25),
 where the ITAM motifs are set out in brackets.

Likewise, a suitable intracellular activating domain polypeptide can comprise an ITAM motif-containing portion of the full length CD79A amino acid sequence. Thus, a suitable intracellular activating domain can include a domain with at least 50%, 60%, 70%, 75%, 80%, 85%, 90%, 95%, 96%, 97%, 98%, 99% or 100% sequence identity to a stretch of at least 10, 15, 20, or all amino acids in the following sequence: ENL[YEGLNLDDCSMYEDI[SRG (SEQ ID NO:26), where the ITAM motifs are set out in brackets.

In some cases, the intracellular activating domain is derived from DAP12 ( 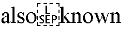 as TYROBP; TYRO protein tyrosine kinase binding protein; KARAP; PLOSL; DNAX-activation protein 12; KAR-associated protein; TYRO protein tyrosine 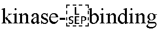 protein; killer activating receptor associated protein; killer-activating 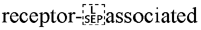 protein; etc.). For example, a suitable intracellular activating domain can include a domain with at least 50%, 60%, 70%, 75%, 80%, 85%, 90%, 95%, 96%, 97%, 98%, 99% or 100% sequence identity to a stretch of at least 10, 15, 20, or all amino acids in the following sequences or to a contiguous stretch of from about 100 amino acids to about 110 amino acids (aa), from about 110 aa to about 115 aa, from about 115 aa to about 120 aa, from about 120 aa to about 130 aa, from about 130 aa to about 140 aa, from about 140 aa to about 150 aa, or from about 150 aa to about 160 aa, of 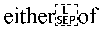 the following amino acid sequences (4 isoforms): 
MGGLEPCSRLLLLPLLLAVSGLRPVQAQAQSDCSCSTVSPGVLAGIVMGDLVLTVLIALAVYFLG
RLVPRGRGAAEAATRKQRITETESP[YQELQGQRSDVYSDL]NTQRPYYK (SEQ ID NO:27),
 
MGGLEPCSRLLLLPLLLAVSGLRPVQAQAQSDCSCSTVSPGVLAGIVMGDLVLTVLIALAVYFLG
RLVPRGRGAAEATRKQRITETESP[YQELQGQRSDVYSDL]NTQ (SEQ ID NO:28),
 
MGGLEPCSRLLLLPLLLAVSDCSCSTVSPGVLAGIVMGDLVLTVLIALAVYFLGRLVPRGRGAAE
AATRKQRITETESP[YQELQGQRSDVYSDL]NTQRPYYK (SEQ ID NO:29),
 or MGGLEPCSRLLLLPLLLAVSDCSCSTVSPGVLAGIVMGDLVLTVLIALAVYFLGRLVPRGRGAAE ATRKQRITETESP[YQELQGQRSDVYSDL]NTQRPYYK (SEQ ID NO:30), where the ITAM motifs are set out in brackets.

Likewise, a suitable intracellular activating domain polypeptide can comprise an ITAM motif-containing portion of the full length DAP12 amino acid sequence. Thus, a suitable intracellular activating domain can include a domain with at least 50%, 60%, 70%, 75%, 80%, 85%, 90%, 95%, 96%, 97%, 98%, 99% or 100% sequence identity to a stretch of at least 10, 15, 20, or all amino acids in the following sequence: ESP[YQELQGQRSDVYSDL]NTQ (SEQ ID NO:31), where the ITAM motifs are set out in brackets.

In some cases, the intracellular activating domain is derived from FCERlG (also known as FCRG; Fc epsilon receptor I gamma chain; Fc receptor gamma-chain; fc-epsilon RI-gamma; fcRgamma; fceRI gamma; high affinity immunoglobulin epsilon receptor subunit gamma; immunoglobulin E receptor, high affinity, gamma chain; etc.). For example, a suitable intracellular activating domain can include a domain with at least 50%, 60%, 70%, 75%, 80%, 85%, 90%, 95%, 96%, 97%, 98%, 99% or 100% sequence identity to a stretch of at least 10, 15, 20, or all amino acids in the following sequences or to a contiguous stretch of from about 50 amino acids to about 60 amino acids (aa), from about 60 aa to about 70 aa, from about 70 aa to about 80 aa, or from about 80 aa to about 88 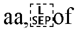 the following amino acid sequence:
MIPAVVLLLLLLVEQAAALGEPQLCYILDAILFLYGIVLTLLYCRLKIQVRKAAITSYEKSDGV[YT GLSTRNQETYETL]KHEKPPQ (SEQ ID NO:32), where the ITAM motifs are set out in brackets.

Likewise, a suitable intracellular activating domain polypeptide can comprise an ITAM motif-containing portion of the full length FCER1G amino acid sequence. Thus, a suitable intracellular activating domain can include a domain with at least 50%, 60%, 70%, 75%, 80%, 85%, 90%, 95%, 96%, 97%, 98%, 99% or 100% sequence identity to a stretch of at least 10, 15, 20, or all amino acids in the following sequence: DGV[YTGLSTRNQETYETL]KHE (SEQ ID NO:33), where the ITAM motifs are set out in brackets.

Intracellular activating domains suitable for use in an engineered signaling polypeptide of the present disclosure include a DAP 10/CD28 type signaling chain. An example of a DAP10 signaling chain is the amino acid SEQ ID NO:34. In some embodiments, a suitable intracellular activating domain includes a domain with at least 50%, 60%, 70%, 75%, 80%, 85%, 90%, 95%, 96%, 97%, 98%, 99% or 100% sequence identity to a stretch of at least 10, 15, 20, or all amino acids in SEQ ID NO:34.

An example of a CD28 signaling chain is the amino acid sequence is SEQ ID NO:35. In some embodiments, a suitable intracellular domain includes a domain with at least 50%, 60%, 70%, 75%, 80%, 85%, 90%, 95%, 96%, 97%, 98%, 99% or 100% sequence identity to a stretch of at least 10, 15, 20, or all amino acids of SEQ ID NO:35.

Intracellular activating domains suitable for use in an engineered signaling polypeptide of the present disclosure include a ZAP70 polypeptide, For example, a suitable intracellular activating domain can include a domain with at least 50%, 60%, 70%, 75%, 80%, 85%, 90%, 95%, 96%, 97%, 98%, 99% or 100% sequence identity to a stretch of at least 10, 15, 20, or all amino acids in the following sequences or to a contiguous stretch of from about 300 amino acids to about 400 amino acids, from about 400 amino acids to about 500 amino acids, or from about 500 amino acids to 619 amino acids, of SEQ ID NO:36.

### Modulatory domains

Modulatory domains can change the effect of the intracellular activating domain in the engineered signaling polypeptide, including enhancing or dampening the downstream effects of the activating domain or changing the nature of the response. Modulatory domains suitable for use in an engineered signaling polypeptide of the present disclosure include co-stimulatory domains. A modulatory domain suitable for inclusion in the engineered signaling polypeptide can have a length of from about 30 amino acids to about 70 amino acids (aa), e.g., a modulatory domain can have a length of from about 30 aa to about 35 aa, from about 35 aa to about 40 aa, from about 40 aa to about 45 aa, from about 45 aa to about 50 aa, from about 50 aa to about 55 aa, from about 55 aa to about 60 aa, from about 60 aa to about 65 aa, or from about 65 aa to about 70 aa. In other cases, modulatory domain can have a length of from about 70 aa to about 100 aa, from about 100 aa to about 200 aa, or greater than 200 aa.

Co-stimulatory domains typically enhance and/or change the nature of the response to an activation domain. Co-stimulatory domains suitable for use in an engineered signaling polypeptide of the present disclosure are generally polypeptides derived from receptors. In some embodiments, co-stimulatory domains homodimerize. A subject co-stimulatory domain can be an intracellular portion of a transmembrane protein (i.e., the co-stimulatory domain can be derived from a transmembrane protein). Non-limiting examples of suitable co-stimulatory polypeptides include, but are not limited to, 4-lBB (CD137), CD27, CD28, CD28 deleted for Lck binding (ICΔ), ICOS, OX40, BTLA, CD27, CD30, GITR, and HVEM. For example, a co-stimulatory domain of as described herein can have at least 80%, 90%, or 95% sequence identity to the co-stimulatory domain of 4-lBB (CD137), CD27, CD28, CD28 deleted for Lck binding (ICΔ), ICOS, OX40, BTLA, CD27, CD30, GITR, or HVEM. For example, a co-stimulatory domain as described herein can have at least 80%, 90%, or 95% sequence identity to the co-stimulatory domain of non-limiting examples of suitable co-stimulatory polypeptides include, but are not limited to, 4-lBB (CD137), CD27, CD28, CD28 deleted for Lck binding (ICΔ), ICOS, OX40, BTLA, CD27, CD30, GITR, and HVEM. For example, a co-stimulatory domain as described herein can have at least 80%, 90%, or 95% sequence identity to the co-stimulatory domain of 4-lBB (CD137), CD27, CD28, CD28 deleted for Lck binding (ICΔ), ICOS, OX40, BTLA, CD27, CD30, GITR, or HVEM.

A co-stimulatory domain suitable for inclusion in an engineered signaling polypeptide can have a length of from about 30 amino acids to about 70 amino acids (aa), e.g., a co-stimulatory domain can have a length of from about 30 aa to about 35 aa, from about 35 aa to about 40 aa, from about 40 aa to about 45 aa, from about 45 aa to about 50 aa, from about 50 aa to about 55 aa, from about 55 aa to about 60 aa, from about 60 aa to about 65 aa, or from about 65 aa to about 70 aa. In other cases, the co-stimulatory domain can have a length of from about 70 aa to about 100 aa, from about 100 aa to about 200 aa, or greater than 200 aa.

In some cases, the co-stimulatory domain is derived from an intracellular portion of the transmembrane protein CD137 (also known as TNFRSF9; CD137; 4-lBB; CDwl37; ILA; etc.). For example, a suitable co-stimulatory domain can include a domain with at least 50%, 60%, 70%, 75%, 80%, 85%, 90%, 95%, 96%, 97%, 98%, 99% or 100% sequence identity to a stretch of at least 10, 15, 20, or all of the amino acids in SEQ ID NO: 1. In some of these embodiments, the co-stimulatory domain has a length of from about 30 aa to about 35 aa, from about 35 aa to about 40 aa, from about 40 aa to about 45 aa, from about 45 aa to about 50 aa, from about 50 aa to about 55 aa, from about 55 aa to about 60 aa, from about 60 aa to about 65 aa, or from about 65 aa to about 70 aa.

In some cases, the co-stimulatory domain is derived from an intracellular portion of the transmembrane protein CD28 (also known as Tp44). For example, a suitable co-stimulatory domain can include a domain with at least 50%, 60%, 70%, 75%, 80%, 85%, 90%, 95%, 96%, 97%, 98%, 99% or 100% sequence identity to a stretch of at least 10, 15, 20, or all of the amino acids in SEQ ID NO:2. In some of these embodiments, the co-stimulatory domain has a length of from about 30 aa to about 35 aa, from about 35 aa to about 40 aa, from about 40 aa to about 45 aa, from about 45 aa to about 50 aa, from about 50 aa to about 55 aa, from about 55 aa to about 60 aa, from about 60 aa to about 65 aa, or from about 65 aa to about 70 aa.

In some cases, the co-stimulatory domain is derived from an intracellular portion of the transmembrane protein CD28 deleted for Lck binding (ICΔ). For example, a suitable co-stimulatory domain can include a domain with at least 50%, 60%, 70%, 75%, 80%, 85%, 90%, 95%, 96%, 97%, 98%, 99% or 100% sequence identity to a stretch of at least 10, 15, 20, or all of the amino acids in SEQ ID NO:3. In some of these embodiments, the co-stimulatory domain has a length of from about 30 aa to about 35 aa, from about 35 aa to about 40 aa, from about 40 aa to about 45 aa, from about 45 aa to about 50 aa, from about 50 aa to about 55 aa, from about 55 aa to about 60 aa, from about 60 aa to about 65 aa, or from about 65 aa to about 70 aa.

In some cases, the co-stimulatory domain is derived from an intracellular portion of the transmembrane protein ICOS (also known as AILIM, CD278, and CVIDl). For example, a suitable co-stimulatory domain can include a domain with at least 50%, 60%, 70%, 75%, 80%, 85%, 90%, 95%, 96%, 97%, 98%, 99% or 100% sequence identity to a stretch of at least 10, 15, 20, or all of the amino acids in SEQ ID NO:4. In some of these embodiments, the co-stimulatory domain has a length of from about 30 aa to about 35 aa, from about 35 aa to about 40 aa, from about 40 aa to about 45 aa, from about 45 aa to about 50 aa, from about 50 aa to about 55 aa, from about 55 aa to about 60 aa, from about 60 aa to about 65 aa, or from about 65 aa to about 70 aa.

In some cases, the co-stimulatory domain is derived from an intracellular portion of the transmembrane protein OX40 (also known as TNFRSF4, RP5-902P8.3, ACT35, CD134, OX-40, TXGPlL). For example, a suitable co-stimulatory domain can include a domain with at least 50%, 60%, 70%, 75%, 80%, 85%, 90%, 95%, 96%, 97%, 98%, 99% or 100% sequence identity to a stretch of at least 10, 15, 20, or all of the amino acids in SEQ ID NO:5. In some of these embodiments, the co-stimulatory domain has a length of from about 30 aa to about 35 aa, from about 35 aa to about 40 aa, from about 40 aa to about 45 aa, from about 45 aa to about 50 aa, from about 50 aa to about 55 aa, from about 55 aa to about 60 aa, from about 60 aa to about 65 aa, or from about 65 aa to about 70 aa.

In some cases, the co-stimulatory domain is derived from an intracellular portion of the transmembrane protein CD27 (also known as S 152, T 14, TNFRSF7, and Tp55). For example, a suitable co-stimulatory domain can include a domain with at least 50%, 60%, 70%, 75%, 80%, 85%, 90%, 95%, 96%, 97%, 98%, 99% or 100% sequence identity to a stretch of at least 10, 15, 20, or all of the amino acids in SEQ ID NO:6. In some of these embodiments, the co-stimulatory domain has a length of from about 30 aa to about 35 aa, from about 35 aa to about 40 aa, from about 40 aa to about 45 aa, or from about 45 aa to about 50 aa.

In some cases, the co-stimulatory domain is derived from an intracellular portion of the transmembrane protein BTLA (also known as BTLAl and CD272). For example, a suitable co-stimulatory domain can include a domain with at least 50%, 60%, 70%, 75%, 80%, 85%, 90%, 95%, 96%, 97%, 98%, 99% or 100% sequence identity to a stretch of at least 10, 15, 20, or all of the amino acids in SEQ ID NO:7.

In some cases, the co-stimulatory domain is derived from an intracellular portion of the transmembrane protein CD30 (also known as TNFRSF8, DlS166E, and Ki-1). For example, a suitable co-stimulatory domain can include a domain with at least 50%, 60%, 70%, 75%, 80%, 85%, 90%, 95%, 96%, 97%, 98%, 99% or 100% sequence identity to a stretch of from about 100 amino acids to about 110 amino acids (aa), from about 110 aa to about 115 aa, from about 115 aa to about 120 aa, from about 120 aa to about 130 aa, from about 130 aa to about 140 aa, from about 140 aa to about 150 aa, from about 150 aa to about 160 aa, or from about 160 aa to about 185 aa of SEQ ID NO:8.

In some cases, the co-stimulatory domain is derived from an intracellular portion of the transmembrane protein GITR (also known as TNFRSF18, RP5-902P8.2, AITR, CD357, and GITR-D). For example, a suitable co-stimulatory domain can include a domain with at least 50%, 60%, 70%, 75%, 80%, 85%, 90%, 95%, 96%, 97%, 98%, 99% or 100% sequence identity to a stretch of at least 10, 15, 20, or all of the amino acids in SEQ ID NO:9. In some of these embodiments, the co-stimulatory domain has a length of from about 30 aa to about 35 aa, from about 35 aa to about 40 aa, from about 40 aa to about 45 aa, from about 45 aa to about 50 aa, from about 50 aa to about 55 aa, from about 55 aa to about 60 aa, from about 60 aa to about 65 aa, or from about 65 aa to about 70 aa.

In some cases, the co-stimulatory domain derived from an intracellular portion of the transmembrane protein HVEM (also known as TNFRSF14, RP3-395M20.6, 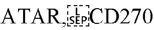 , HVEA, HVEM, LIGHTR, and TR2). For example, a suitable co-stimulatory domain can include a domain with at least 50%, 60%, 70%, 75%, 80%, 85%, 90%, 95%, 96%, 97%, 98%, 99% or 100% sequence identity to a stretch of at least 10, 15, 20, or all of the amino acids in SEQ ID NO:10. In some of these embodiments, the co-stimulatory domain of both the first and the second polypeptide has a length of from about 30 aa to about 35 aa, from about 35 aa to about 40 aa, from about 40 aa to about 45 aa, from about 45 aa to about 50 aa, from about 50 aa to about 55 aa, from about 55 aa to about 60 aa, from about 60 aa to about 65 aa, or from about 65 aa to about 70 aa.

### Linker

In some cases, the engineered signaling polypeptide includes a linker between any two adjacent domains. For example, a linker can be between the transmembrane domain and the first co-stimulatory domain. As another example, the ASTR can be an antibody and a linker can be between the heavy chain and the light chain. As another example, a linker can be between the ASTR and the transmembrane domain and a co-stimulatory domain. As another example, a linker can be between the co-stimulatory domain and the intracellular activating domain of the second polypeptide. As another example, the linker can be between the ASTR and the intracellular signaling domain.

The linker peptide may have any of a variety of amino acid sequences. Proteins can be joined by a spacer peptide, generally of a flexible nature, although other chemical linkages are not excluded. A linker can be a peptide of between about 1 and about 100 amino acids in length, or between about 1 and about 25 amino acids in length. These linkers can be produced by using synthetic, linker-encoding oligonucleotides to couple the proteins. Peptide linkers with a degree of flexibility can be used. The linking peptides may have virtually any amino acid sequence, bearing in mind that suitable linkers will have a sequence that results in a generally flexible peptide. The use of small amino acids, such as glycine and alanine, are of use in creating a flexible peptide. The creation of such sequences is routine to those of skill in the art.

Suitable linkers can be readily selected and can be of any of a suitable of different lengths, such as from 1 amino acid (e.g., Gly) to 20 amino acids, from 2 amino acids to 15 amino acids, from 3 amino acids to 12 amino acids, including 4 amino acids to 10 amino acids, 5 amino acids to 9 amino acids, 6 amino acids to 8 amino acids, or 7 amino acids to 8 amino acids, and may be 1, 2, 3, 4, 5, 6, or 7 amino acids.

Exemplary flexible linkers include glycine polymers (G)ₙ, glycine-serine polymers (including, for example, (GS)ₙ, GSGGSₙ, GGGSₙ, and GGGGSₙ where n is an integer of at least one), glycine-alanine polymers, alanine-serine polymers, and other flexible linkers known in the art. Glycine and glycine-serine polymers are of interest since both of these amino acids are relatively unstructured, and therefore may serve as a neutral tether between components. Glycine polymers are of particular interest since glycine accesses significantly more phi-psi space than even alanine, and is much less restricted than residues with longer side chains (see Scheraga, Rev. Computational Chem. 11173-142 (1992)). Exemplary flexible linkers include, but are not limited GGGGSGGGGSGGGGS (SEQ ID NO:53),
GGGGSGGGGSGGGGSGGGGSGGGGSGGGGS (SEQ ID NO:54), GGGGSGGGSGGGGS (SEQ ID NO:55), GGSG (SEQ ID NO:56), GGSGG (SEQ ID NO:57), GSGSG (SEQ ID NO:58), GSGGG (SEQ ID NO:59), GGGSG (SEQ ID NO:60), GSSSG (SEQ ID NO:61), and the like. The ordinarily skilled artisan will recognize that design of a peptide conjugated to any elements described above can include linkers that are all or partially flexible, such that the linker can include a flexible linker as well as one or more portions that confer less flexible structure.

### Combinations

In some embodiments, a polynucleotide provided by the replication incompetent recombinant retroviral particles has one or more transcriptional units that encode certain combinations of the one or more engineered signaling polypeptides. In some methods and compositions provided herein, genetically modified T cells include the combinations of the one or more engineered signaling polypeptides after transduction of T cells by the replication incompetent recombinant retroviral particles. It will be understood that the reference of a first polypeptide, a second polypeptide, a third polypeptide, etc. is for convenience and elements on a "first polypeptide" and those on a "second polypeptide" means that the elements are on different polypeptides that are referenced as first or second for reference and convention only, typically in further elements or steps to that specific polypeptide.

In some embodiments, the first engineered signaling polypeptide includes an extracellular antigen binding domain, which is capable of binding an antigen, and an intracellular signaling domain. In other embodiments, the first engineered signaling polypeptide also includes a T cell survival motif and/or a transmembrane domain. In some embodiments, the first engineered signaling polypeptide does not include a co-stimulatory domain, while in other embodiments, the first engineered signaling polypeptide does include a co-stimulatory domain.

In some embodiments, a second engineered signaling polypeptide includes a lymphoproliferative gene product and optionally an extracellular antigen binding domain. In some embodiments, the second engineered signaling polypeptide also includes one or more of the following: a T cell survival motif, an intracellular signaling domain, and one or more co-stimulatory domains. In other embodiments, when two engineered signaling polypeptides are used, at least one is a CAR.

In one embodiment, the one or more engineered signaling polypeptides are expressed under a T cell specific promoter or a general promoter under the same transcript wherein in the transcript, nucleic acids encoding the engineered signaling polypeptides are separated by nucleic acids that encode one or more internal ribosomal entry sites (IREs) or one or more protease cleavage peptides.

In certain embodiments, the polynucleotide encodes two engineered signaling polypeptides wherein the first engineered signaling polypeptide includes a first extracellular antigen binding domain, which is capable of binding to a first antigen, and a first intracellular signaling domain but not a co-stimulatory domain, and the second polypeptide includes a second extracellular antigen binding domain, which is capable of binding VEGF, and a second intracellular signaling domain, such as for example, the signaling domain of a co-stimulatory molecule. In a certain embodiment, the first antigen is PSCA, PSMA, or BCMA. In a certain embodiment, the first extracellular antigen binding domain comprises an antibody or fragment thereof (e.g., scFv), e.g., an antibody or fragment thereof specific to PSCA, PSMA, or BCMA. In a certain embodiment, the second extracellular antigen binding domain that binds VEGF is a receptor for VEGF, i.e., VEGFR. In certain embodiments, the VEGFR is VEGFR1, VEGFR2, or VEGFR3. In a certain embodiment, the VEGFR is VEGFR2.

In certain embodiments, the polynucleotide encodes two engineered signaling polypeptides wherein the first engineered signaling polypeptide includes an extracellular tumor antigen binding domain and a CD3ζ signaling domain, and the second engineered signaling polypeptide includes an antigen-binding domain, wherein the antigen is an angiogenic or vasculogenic factor, and one or more co-stimulatory molecule signaling domains. The angiogenic factor can be, e.g., VEGF. The one or more co-stimulatory molecule signaling motifs can comprise, e.g., co-stimulatory signaling domains from each of CD27, CD28, OX40, ICOS, and 4-1BB.

In certain embodiments, the polynucleotide encodes two engineered signaling polypeptides wherein the first engineered signaling polypeptide includes an extracellular tumor antigen-binding domain and a CD3ζ signaling domain, the second polypeptide comprises an antigen-binding domain, which is capable of binding to VEGF, and co-stimulatory signaling domains from each of CD27, CD28, OX40, ICOS, and 4-1BB. In a further embodiment, the first signaling polypeptide or second signaling polypeptide also has a T cell survival motif. In some embodiments, the T cell survival motif is, or is derived from, an intracellular signaling domain of IL-7 receptor (IL-7R), an intracellular signaling domain of IL-12 receptor, an intracellular signaling domain of IL-15 receptor, an intracellular signaling domain of IL-21 receptor, or an intracellular signaling domain of transforming growth factor β (TGFβ) receptor or the TGFβ decoy receptor (TGF-β-dominant-negative receptor II (DNRII)).

In certain embodiments, the polynucleotide encodes two engineered signaling polypeptides wherein the first engineered signaling polypeptide includes an extracellular tumor antigen-binding domain and a CD3ζ signaling domain, and the second engineered signaling polypeptide includes an antigen-binding domain, which is capable of binding to VEGF, an IL-7 receptor intracellular T cell survival motif, and co-stimulatory signaling domains from each of CD27, CD28, OX40, ICOS, and 4-1BB.

In some embodiments, more than two signaling polypeptides are encoded by the polynucleotide. In certain embodiments, only one of the engineered signaling polypeptides includes an antigen binding domain that binds to a tumor-associated antigen or a tumor-specific antigen; each of the remainder of the engineered signaling polypeptides comprises an antigen binding domain that binds to an antigen that is not a tumor-associated antigen or a tumor-specific antigen. In other embodiments, two or more of the engineered signaling polypeptides include antigen binding domains that bind to one or more tumor-associated antigens or tumor-specific antigens, wherein at least one of the engineered signaling polypeptides comprises an antigen binding domain that does not bind to a tumor-associated antigen or a tumor-specific antigen.

In some embodiments, the tumor-associated antigen or tumor-specific antigen is Her2, prostate stem cell antigen (PSCA), PSMA (prostate-specific membrane antigen), B cell maturation antigen (BCMA), alpha-fetoprotein (AFP), carcinoembryonic antigen (CEA), cancer antigen-125 (CA-125), CA19-9, calretinin, MUC-1, epithelial membrane protein (EMA), epithelial tumor antigen (ETA), tyrosinase, melanoma-associated antigen (MAGE), CD34, CD45, CD99, CD117, chromogranin, cytokeratin, desmin, glial fibrillary acidic protein (GFAP), gross cystic disease fluid protein (GCDFP-15), HMB-45 antigen, protein melan-A (melanoma antigen recognized by T lymphocytes; MART-1), myo-D1, muscle-specific actin (MSA), neurofilament, neuron-specific enolase (NSE), placental alkaline phosphatase, synaptophysin, thyroglobulin, thyroid transcription factor-1, the dimeric form of the pyruvate kinase isoenzyme type M2 (tumor M2-PK), CD19, CD22, CD27, CD30, CD70, GD2 (ganglioside G2), EphA2, CSPG4, CD138, FAP (Fibroblast Activation Protein), CD171, kappa, lambda, 5T4, αvβ6 integrin, integrin αvβ3 (CD61), galactin, K-Ras (V-Ki-ras2 Kirsten rat sarcoma viral oncogene), Ral-B, B7-H3, B7-H6, CAIX, CD20, CD33, CD44, CD44v6, CD44v7/8, CD123, EGFR, EGP2, EGP40, EpCAM, fetal AchR, FRα, GD3, HLA-A1+MAGE1, HLA-A1+NY-ESO-1, IL-11Rα, IL-13Rα2, Lewis-Y, Muc16, NCAM, NKG2D Ligands, NY-ESO-1, PRAME, ROR1, Survivin, TAG72, TEMs, VEGFR2, EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein.

In some embodiments, the first engineered signaling polypeptide includes a first extracellular antigen binding domain that binds a first antigen, and a first intracellular signaling domain; and a second engineered signaling polypeptide includes a second extracellular antigen binding domain that binds a second antigen, or a receptor that binds the second antigen; and a second intracellular signaling domain, wherein the second engineered signaling polypeptide does not comprise a co-stimulatory domain. In a certain embodiment, the first antigen-binding domain and the second antigen-binding domain are independently an antigen-binding portion of a receptor or an antigen-binding portion of an antibody. In a certain embodiment, either or both of the first antigen binding domain or the second antigen binding domain are scFv antibody fragments. In certain embodiments, the first engineered signaling polypeptide and/or the second engineered signaling polypeptide additionally comprises a transmembrane domain. In a certain embodiment, the first engineered signaling polypeptide or the second engineered signaling polypeptide comprises a T cell survival motif, e.g., any of the T cell survival motifs described herein.

In another embodiment, the first engineered signaling polypeptide includes a first extracellular antigen binding domain that binds HER2 and the second engineered signaling polypeptide includes a second extracellular antigen binding domain that binds MUC-1.

In another embodiment, the second extracellular antigen binding domain of the second engineered signaling polypeptide binds an interleukin.

In another embodiment, the second extracellular antigen binding domain of the second engineered signaling polypeptide binds a damage associated molecular pattern molecule (DAMP; also known as an alarmin). In other embodiments, a DAMP is a heat shock protein, chromatin-associated protein high mobility group box 1 (HMGB1), S100A8 (also known as MRP8, or calgranulin A), S100A9 (also known as MRP14, or calgranulin B), serum amyloid A (SAA), deoxyribonucleic acid, adenosine triphosphate, uric acid, or heparin sulfate.

In certain embodiments, said second antigen is an antigen on an antibody that binds to an antigen presented by a tumor cell.

In some embodiments, signal transduction activation through the second engineered signaling polypeptide is non-antigenic, but is associated with hypoxia. In certain embodiments, hypoxia is induced by activation of hypoxia-inducible factor-1α (HIF-1α), HIF-1β, HIF-2α, HIF-2β, HIF-3α, or HIF-3β.

In some embodiments, expression of the one or more engineered signaling polypeptides is regulated by a control element, which is disclosed in more detail herein.

### Additional sequences

The engineered signaling polypeptides, such as CARs, can further include one or more additional polypeptide domains, where such domains include, but are not limited to, a signal sequence; an epitope tag; an affinity domain; and a polypeptide whose presence or activity can be detected (detectable marker), for example by an antibody assay or because it is a polypeptide that produces a detectable signal. Non-limiting examples of additional domains for any of the aspects or embodiments provided herein, include a domain with at least 50%, 60%, 70%, 75%, 80%, 85%, 90%, 95%, 96%, 97%, 98%, 99% or 100% sequence identity to any of the following sequences as described below: a signal sequence, an epitope tag, an affinity domain, or a polypeptide that produces a detectable signal.

Signal sequences that are suitable for use in a subject CAR, e.g., in the first polypeptide of a subject CAR, include any eukaryotic signal sequence, including a naturally-occurring signal sequence, a synthetic (e.g., man-made) signal sequence, etc. In some embodiments, for example, the signal sequence can be the CD8 signal sequence MALPVTALLLPLALLLHAARP (SEQ ID NO:74).

Suitable epitope tags include, but are not limited to, hemagglutinin (HA; e.g., YPYDVPDYA; SEQ ID NO:37); FLAG (e.g.,DYKDDDDK; SEQ ID NO:38); c-myc (e.g., EQKLISEEDL; SEQ ID NO:39), and the like.

Affinity domains include peptide sequences that can interact with a binding partner, e.g., such as one immobilized on a solid support, useful for identification or purification. DNA sequences encoding multiple consecutive single amino acids, such as histidine, when fused to the expressed protein, may be used for one-step purification of the recombinant protein by high affinity binding to a resin column, such as nickel sepharose. Exemplary affinity domains include His5 (HHHHH; SEQ ID NO:40), HisX6 (HHHHHH; SEQ ID NO:41), c-myc (EQKLISEEDL; SEQ ID NO:39), Flag (DYKDDDDK; SEQ ID NO:38), Strep Tag (WSHPQFEK; SEQ ID NO:42), hemagglutinin, e.g., HA Tag (YPYDVPDYA; SEQ ID NO:37), GST, thioredoxin, cellulose binding domain, RYIRS (SEQ ID NO:43), Phe-His-His-Thr (SEQ ID NO:44), chitin binding domain, S-peptide, T7 peptide, SH2 domain, C-end RNA tag, WEAAAREACCRECCARA (SEQ ID NO:45), metal binding domains, e.g., zinc binding domains or calcium binding domains such as those from calcium-binding proteins, e.g., calmodulin, troponin C, calcineurin B, myosin light chain, recoverin, S-modulin, visinin, VILIP, neurocalcin, hippocalcin, frequenin, caltractin, calpain large-subunit, S 100proteins, parvalbumin, calbindin D9K, calbindin D28K, and calretinin, inteins, biotin, streptavidin, MyoD, Id, leucine zipper sequences, and maltose binding protein.

Suitable detectable signal-producing proteins include, e.g., fluorescent proteins; enzymes that catalyze a reaction that generates a detectable signal as a product; and the like.

Suitable fluorescent proteins include, but are not limited to, green fluorescent protein (GFP) or variants thereof, blue fluorescent variant of GFP (BFP), cyan fluorescent variant of GFP (CFP), yellow fluorescent variant of GFP (YFP), enhanced GFP (EGFP), enhanced CFP (ECFP), enhanced YFP (EYFP), GFPS65T, Emerald, Topaz (TYFP), Venus, Citrine, mCitrine, GFPuv, destabilized EGFP (dEGFP), destabilized ECFP (dECFP), destabilized EYFP (dEYFP), mCFPm, Cerulean, T-Sapphire, CyPet, YPet, mKO, HcRed, t-HcRed, DsRed, DsRed2, DsRed-monomer, J-Red, dimer2, t-dimer2(12), mRFPl, pocilloporin, Renilla GFP, Monster GFP, paGFP, Kaede protein and kindling protein, Phycobiliproteins and Phycobiliprotein conjugates including B-Phycoerythrin, R-Phycoerythrin and Allophycocyanin. Other examples of fluorescent proteins include mHoneydew, mBanana, mOrange, dTomato, tdTomato, mTangerine, mStrawberry, mCherry, 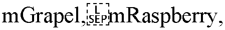 mGrape2, mPlum (Shaner et al. (2005) Nat. Methods 2:905-909), and the like. Any of a variety of fluorescent and colored proteins from Anthozoan species, as described in, e.g., Matz et al. (1999) Nature Biotechnol. 17:969-973, is suitable for use.

Suitable enzymes include, but are not limited to, horse radish peroxidase (HRP), alkaline phosphatase (AP), beta-galactosidase (GAL), glucose-6-phosphate dehydrogenase, beta-N-acetylglucosaminidase, β-glucuronidase, invertase, Xanthine Oxidase, firefly luciferase, glucose oxidase (GO), and the like.

### Recognition and/or elimination domain

Any of the replication incompetent recombinant retroviral particles provided herein can include nucleic acids that encode a recognition or elimination domain as part of, or separate from, nucleic acids encoding any of the engineered signaling polypeptides provided herein. Thus, any of the engineered signaling polypeptides provided herein, can include a recognition or elimination domain. For example, any of the CARs disclosed herein can include a recognition or elimination domain. Moreover, a recognition or elimination domain can be expressed together with, or even fused with any of the lymphoproliferative elements disclosed herein. The recognition or elimination domains are expressed on the T cell and/or NK cell but are not expressed on the replication incompetent recombinant retroviral particles.

In some embodiments, the recognition or elimination domain can be derived from herpes simplex virus-derived enzyme thymidine kinase (HSV-tk) or inducible caspase-9. In some embodiments, the recognition or elimination domain can include a modified endogenous cell-surface molecule, for example as disclosed in U.S. Patent 8,802,374. The modified endogenous cell-surface molecule can be any cell-surface related receptor, ligand, glycoprotein, cell adhesion molecule, antigen, integrin, or cluster of differentiation (CD) that is modified. In some embodiments, the modified endogenous cell-surface molecule is a truncated tyrosine kinase receptor. In one aspect, the truncated tyrosine kinase receptor is a member of the epidermal growth factor receptor (EGFR) family (e.g., ErbB1, ErbB2, ErbB3, and ErbB4). In some embodiments, the recognition domain can be a polypeptide that is recognized by an antibody that recognizes the extracellular domain of an EGFR member. In some embodiments, the recognition domain can be at least 20 contiguous amino acids of an EGFR family member, or for example, between 20 and 50 contiguous amino acids of an EGFR family member. For example, SEQ ID NO:78, is an exemplary polypeptide that is recognized by, and under the appropriate conditions bound by an antibody that recognizes the extracellular domain of an EGFR member. Such extracellular EGFR epitopes are sometimes referred to herein as eTags. In illustrative embodiments, such epitopes are recognized by commercially available anti-EGFR monoclonal antibodies.

Epidermal growth factor receptor, also known as EGFR, ErbB1 and HER1, is a cell-surface receptor for members of the epidermal growth factor family of extracellular ligands. Alterations in EGFR activity have been implicated in certain cancers. In some embodiments, a gene encoding an EGFR polypeptide including human epidermal growth factor receptor (EGFR) is constructed by removal of nucleic acid sequences that encode polypeptides including the membrane distal EGF-binding domain and the cytoplasmic signaling tail, but retains the extracellular membrane proximal epitope recognized by an anti-EGFR antibody. Preferably, the antibody is a known, commercially available anti-EGFR monoclonal antibody, such as cetuximab, matuzumab, necitumumab or panitumumab.

Others have shown that application of biotinylated-cetuximab to immunomagnetic selection in combination with anti-biotin microbeads successfully enriches T cells that have been lentivirally transduced with EGFRt-containing constructs from as low as 2% of the population to greater than 90% purity without observable toxicity to the cell preparation. Furthermore, others have shown that constitutive expression of this inert EGFR molecule does not affect T cell phenotype or effector function as directed by the coordinately expressed chimeric antigen receptor (CAR), CD19R. In addition, others have shown that through flow cytometric analysis, EGFR was successfully utilized as an *in vivo* tracking marker for T cell engraftment in mice. Furthermore, EGFR was demonstrated to have suicide gene potential through Erbitux^{®} mediated antibody dependent cellular cytotoxicity (ADCC) pathways. The inventors of the present disclosure have successfully expressed eTag in PBMCs using lentiviral vectors, and have found that expression of eTag *in vitro* by PBMCs exposed to Cetuximab, provided an effective elimination mechanism for PBMCs. Thus, EGFR may be used as a non-immunogenic selection tool, tracking marker, and suicide gene for transduced T cells that have immunotherapeutic potential. The EGFR nucleic acid may also be detected by means well known in the art.

In some embodiments provided herein, EGFR is expressed as part of a single polypeptide that also includes the CAR or as part of a single polypeptide that includes the lymphoproliferative element. In some embodiments, the amino acid sequence encoding the EGFR recognition domain can be separated from the amino acid sequence encoding the chimeric antigen receptor by a cleavage signal and/or a ribosomal skip sequence. The ribosomal skip and/or cleavage signal can be any ribosomal skip and/or cleavage signal known in the art. Not to be limited by theory, the ribosomal skip sequence can be, for example T2A (also referred to as 2A-1 herein) with amino acid sequence GSGEGRGSLLTCGDVEENPGP (SEQ ID NO:77). Not to be limited by theory, other examples of cleavage signals and ribosomal skip sequences include FMDV 2A (F2A); equine rhinitis A virus 2A (abbreviated as E2A); porcine teschovirus-1 2A (P2A); and *Thoseaasigna* virus 2A (T2A). In some embodiments, the polynucleotide sequence encoding the recognition domain can be on the same transcript as the CAR or lymphoproliferative element but separated from the polynucleotide sequence encoding the CAR or lymphoproliferative element by an internal ribosome entry site.

In other embodiments as exemplified empirically herein, a recognition domain can be expressed as part of a fusion polypeptide, fused to a lymphoproliferative element. Such constructs provide the advantage, especially in combination with other "space saving" elements provided herein, of taking up less genomic space on an RNA genome compared to separate polypeptides. In one illustrative embodiment, an eTag is expressed as a fusion polypeptide, fused to an IL7Rα mutant, as experimentally demonstrated herein.

### Chimeric antigen receptor

In some aspects as described herein, an engineered signaling polypeptide is a chimeric antigen receptor (CAR) or a polynucleotide encoding a CAR, which, for simplicity, is referred to herein as "CAR." A CAR of the present disclosure includes: a) at least one antigen-specific targeting region (ASTR); b) a transmembrane domain; and c) an intracellular activating domain. In illustrative embodiments, the antigen-specific targeting region of the CAR is a scFv portion of an antibody to the target antigen. In illustrative embodiments, the intracellular activating domain is from CD3z.

A CAR of the present disclosure can be present in the plasma membrane of a eukaryotic cell, e.g., a mammalian cell, where suitable mammalian cells include, but are not limited to, a cytotoxic cell, a T lymphocyte, a stem cell, a progeny of a stem cell, a progenitor cell, a progeny of a progenitor cell, and an NK cell, an NK-T cell, and a macrophage. When present in the plasma membrane of a eukaryotic cell, a CAR of the present disclosure is active in the presence of one or more target antigens that, in certain conditions, binds the ASTR. The target antigen is the second member of the specific binding pair. The target antigen of the specific binding pair can be a soluble (e.g., not bound to a cell) factor; a factor present on the surface of a cell such as a target cell; a factor presented on a solid surface; a factor present in a lipid bilayer; and the like. Where the ASTR is an antibody, and the second member of the specific binding pair is an antigen, the antigen can be a soluble (e.g., not bound to a cell) antigen; an antigen present on the surface of a cell such as a target cell; an antigen presented on a solid surface; an antigen present in a lipid bilayer; and the like.

In some instances, a CAR of the present disclosure, when present in the plasma membrane of a eukaryotic cell, and when activated by one or more target antigens, increases expression of at least one nucleic acid in the cell. For example, in some cases, a CAR of the present disclosure, when present in the plasma membrane of a eukaryotic cell, and when activated by the one or more target antigens, increases expression of at least one nucleic acid in the cell by at least about 10%, at least about 15%, at least about 20%, at least about 25%, at least about 30%, at least about 40%, at least about 50%, at least about 75%, at least about 2-fold, at least about 2.5-fold, at least about 5-fold, at least about 10-fold, or more than 10-fold, compared with the level of transcription of the nucleic acid in the absence of the one or more target antigens.

As an example, the CAR of the present disclosure can include an immunoreceptor tyrosine-based activation motif (ITAM)-containing intracellular signaling polypeptide.

A CAR of the present disclosure, when present in the plasma membrane of a eukaryotic cell, and when activated by one or more target antigens, can, in some instances, result in increased production of one or more cytokines by the cell. For example, a CAR of the present disclosure, when present in the plasma membrane of a eukaryotic cell, and when activated by the one or more target antigens, can increase production of a cytokine by the cell by at least about 10%, at least about 15%, at least about 20%, at least about 25%, at least about 30%, at least about 40%, at least about 50%, at least about 75%, at least about 2-fold, at least about 2.5-fold, at least about 5-fold, at least about 10-fold, or more than 10-fold, compared with the amount of cytokine produced by the cell in the absence of the one or more target antigens. Cytokines whose production can be increased include, but are not limited to interferon gamma (IFN-γ), tumor necrosis factor-alpha (TNF-a), IL-2, IL-15, IL-12, IL-4, IL-5, IL-10; a chemokine; a growth factor; and the like.

In some cases, a CAR of the present disclosure, when present in the plasma membrane of a eukaryotic cell, and when activated by one or more target antigens, can result in both an increase in transcription of a nucleic acid in the cell and an increase in production of a cytokine by the cell.

In some instances, a CAR of the present disclosure, when present in the plasma membrane of a eukaryotic cell, and when activated by one or more target antigens, results in cytotoxic activity by the cell toward a target cell that expresses on its cell surface an antigen to which the antigen-binding domain of the first polypeptide of the CAR binds. For example, where the eukaryotic cell is a cytotoxic cell (e.g., an NK cell or a cytotoxic T lymphocyte), a CAR of the present disclosure, when present in the plasma membrane of the cell, and when activated by the one or more target antigens, increases cytotoxic activity of the cell toward a target cell that expresses on its cell surface the one or more target antigens. For example, where the eukaryotic cell is an NK cell or a T lymphocyte, a CAR of the present disclosure, when present in the plasma membrane of the cell, and when activated by the one or more target antigens, increases cytotoxic activity of the cell by at least about 10%, at least about 15%, at least about 20%, at least about 25%, at least about 30%, at least about 40%, at least about 50%, at least about 75%, at least about 2-fold, at least about 2.5-fold, at least about 5-fold, at least about 10-fold, or more than 10-fold, compared to the cytotoxic activity of the cell in the absence of the one or more target antigens.

In some cases, a CAR of the present disclosure, when present in the plasma membrane of a eukaryotic cell, and when activated by one or more target antigens, can result in other CAR activation related events such as proliferation and expansion (either due to increased cellular division or anti-apoptotic responses).

In some cases, a CAR of the present disclosure, when present in the plasma membrane of a eukaryotic cell, and when activated by one or more target antigens, can result in other CAR activation related events such as intracellular signaling modulation, cellular differentiation, or cell death.

In some embodiments, CARs of the present disclosure are microenvironment restricted. This property is typically the result of the microenvironment restricted nature of the ASTR domain of the CAR. Thus, CARs of the present disclosure can have a lower binding affinity or, in illustrative embodiments, can have a higher binding affinity to one or more target antigens under a condition(s) in a microenvironment than under a condition in a normal physiological environment.

### Lymphoproliferative elements

Peripheral T lymphocyte numbers are maintained at remarkably stable levels throughout adulthood, despite the continuing addition of cells, due to emigration from the thymus and proliferation in response to antigen encounter, and loss of cells owing to the removal of antigen-specific effectors after antigen clearance (Marrak, P. et al. 2000. Nat Immunol 1: 107-111; Freitas, A.A. et al. 2000. Annu Rev Immunol 18:83-111). The size of the peripheral T cell compartment is regulated by multiple factors that influence both proliferation and survival. However, in a lymphopenic environment, T lymphocytes divide independently of cognate antigen, due to "acute homeostatic proliferation" mechanisms that maintain the size of the peripheral T cell compartment. Conditions for lymphopenia have been established in subjects or patients during adoptive cell therapy by proliferating T cells *in vitro* and introducing them into lymphodepleted subjects, resulting in enhanced engraftment and antitumor function of transferred T cells. However, lymphodepletion of a subject is not desirable because it can cause serious side effects, including immune dysfunction and death.

Studies have shown that lymphodepletion removes endogenous lymphocytes functioning as cellular sinks for homeostatic cytokines, thereby freeing cytokines to induce survival and proliferation of adoptively transferred cells. Some cytokines, such as for example, IL-7 and IL-15, are known to mediate antigen-independent proliferation of T cells and are thus capable of eliciting homeostatic proliferation in non-lymphopenic environments. However, these cytokines and their receptors have intrinsic control mechanisms that prevent lymphoproliferative disorders at homeostasis.

Many of the aspects provided herein include a lymphoproliferative element, or a nucleic acid encoding the same, typically as part of an engineered signaling polypeptide. Accordingly, in some aspects as described herein, an engineered signaling polypeptide is a lymphoproliferative element such as a chimeric lymphoproliferative element (CLE). Typically, the CLE contains an extracellular domain, a transmembrane domain, and at least one intracellular signaling domain that drives proliferation. A CLE does not comprise both an ASTR and an activation domain. In illustrative embodiments herein, one or more lymphoproliferative elements is introduced into a resting T cell and/or resting NK cell, typically by transducing the resting T cell and/or resting NK cell with replication incompetent recombinant retroviral particles whose genome encodes the lymphoproliferative element as part of an engineered signaling polypeptide. The lymphoproliferative element can include a single intracellular domain or can include more than one intracellular domain. In certain illustrative embodiments, a lymphoproliferative element includes two intracellular domains. Tables 7-23 provided herein and discussed in Examples 17 and 18 provide CLEs with one intracellular domain and CLEs with two intracellular domains that were identified using experimental methods provided therein that tested candidate chimeric polypeptides that included different combinations of extracellular domains and intracellular domains that are identified in Table 6.

In some illustrative embodiments, a lymphoproliferative element can be or can comprise a cytokine or in further illustrative embodiments, a cytokine receptor, or a fragment that includes a signaling domain thereof, that activates a STAT3 pathway, a STAT4 pathway, or in even further illustrative embodiments, a Jak/STAT5 pathway. As such, a lymphoproliferative element, can be, in a non-limiting example, a cytokine receptor, or active fragment that includes a signaling domain thereof, such as an interleukin receptor, or an active fragment that includes a signaling domain thereof, that activates STAT5. Thus, a lymphoproliferative element is a polypeptide that promotes proliferation, and optionally survival (anti-apoptotic), and optionally provides a co-stimulatory signal that enhances a differentiation state, proliferative potential or resistance to cell death of a lymphocyte. In certain illustrative embodiments, a lymphoproliferative element is a polypeptide that induces proliferation of a T cell and/or NK cell. Illustrative lymphoproliferative elements induce proliferation by activating STAT5. Thus, fragments of such lymphoproliferative elements retain the ability to induce proliferation of T cells and/or NK cells, in illustrative embodiments, by activating STAT5.

In illustrative embodiments, lymphoproliferative elements when present in genetically modified PBMCs, lymphocytes, or genetically modified T cells and/or NK cells are capable of promoting lymphocyte proliferation/expansion and optionally survival *ex vivo* or *in vitro* in culture in the absence of exposure of the cells to cytokines such as IL-15, IL-7, and in illustrative embodiments IL-2 and the target for an ASTR of a CAR expressed by the cells during culturing for 6, 7, 14, 21, or 35 days.

In some of the methods and compositions presented herein, a lymphoproliferative element is used to promote proliferation or expansion of genetically modified T cells *in vivo* without having to lymphodeplete subjects. As such, non-limiting illustrative embodiments of methods provided herein that include inserting a lymphoproliferative element into a resting T cell and/or NK cell of a subject, typically by transducing such T cell and/or NK cell can be performed without lymphodepleting the subject before, during and/or after performing the method, or without lymphodepleting the subject before, during and/or after collecting blood from a subject before performing such method, or without lymphodepleting the subject before, during, and/or after genetically modifying T cells or NK cells *ex vivo* from the subject, and/or before, during, or after reintroducing the genetically modified T cells and/or NK cells into the subject. Factors that promote proliferation of T cells *in vivo* include cytokines and their receptors, in which a receptor typically includes a ligand binding domain and a signaling domain. In some embodiments, the lymphoproliferative element used in the methods and compositions disclosed herein is a cytokine and/or a cytokine receptor. The cytokine can be an interleukin, and the cytokine receptor can be an interleukin receptor. The lymphoproliferative element can be a functional fragment of a cytokine and/or a functional fragment of a cytokine receptor, such as a signaling domain thereof, wherein the fragment is capable of promoting proliferation of T cells, for example by activating STAT5.

In some embodiments, the cytokine lymphoproliferative element in the methods and compositions herein include one or more of the following: Interleukin-7 (IL-7) or its receptor (IL-7R), or a signaling domain thereof; Interleukin-12 (IL-12) or its receptor (IL-12R), or a signaling domain thereof; Interleukin-23 (IL-23) or its receptor composed of IL-12R β1 and IL-23R, or a signaling domain thereof; Interleukin-27 (IL-27) or its receptor (IL-27R), or a signaling domain thereof; Interleukin-15 (IL-15) or its receptor (IL-15R), or a signaling domain thereof; Interleukin-21 (IL-21) or its receptor (IL-21R), or a signaling domain thereof; or transforming growth factor β (TGFβ) or its receptor (TGFβR) or a signaling domain thereof; or the TGFβ decoy receptor (TGF-β-dominant-negative receptor II (DNRII)). In some embodiments, the lymphoproliferative element is the IL-12R or the TGFβ decoy receptor (TGF-β-dominant-negative receptor II (DNRII)).

IL-7 binds to the IL-7 receptor, a heterodimer consisting of IL-7R alpha and common gamma chain receptor. Binding results in a cascade of signals important for T cell development within the thymus and survival within the periphery. Binding of IL-7 to the IL-7 receptor is known to activate the Jak/STAT5 pathway.

IL-12 is involved in the differentiation of naïve T cells into Th1 cells (Hsieh CS et al. 1993. Science. 260(5107):547-9) and is known as a T cell-stimulating factor. IL-12 binds to the IL-12 receptor, which is a heterodimeric receptor formed by IL-12R-β1 and IL-12R-β2. IL12 can act by activating STAT4, but has been shown to activate STAT5 in T cells as well (Ahn, H., et al. 1998. J. Immun. 161:5893-5900). The IL-12 family is composed of the cytokines IL-12, IL-23, and IL-27. The receptor for IL-23 is composed of IL-12R β1 and IL-23R. IL-27 is a heterodimeric cytokine that is composed of two distinct genes, Epstein-Barr virus-induced gene 3(EBI3) and IL-27p28. IL-27 interacts with IL-27 receptor.

IL-15 is a T and NK cell stimulatory factor that is similar in structure and function to IL-2. Both cytokines induce proliferation of T cells; and their shared functions are thought to result from both receptors using the IL-2/IL-15Rβ and common γ chains. Signaling pathway of IL-15 begins with binding to IL-15Rα receptor, with subsequent presentation to surrounding cells bearing IL-15Rβγc complex on their cell surface. Upon binding IL-15β subunit activates Janus kinase 1 (Jak1) and γc subunit Janus kinase 3 (Jak3), which leads to phosphorylation and activation of STAT3 and STAT5.

IL-21 is expressed in activated human CD4+ T cells and in NKT cells, and IL-21 expression is up-regulated in Th2 and Th17 subsets of T helper cells. The IL-21 receptor (IL-21R) is expressed on the surface of T, B and NK cells and is similar in structure to the receptors for other type I cytokines like IL-2R or IL-15. IL-21R requires dimerization with the common gamma chain (γc) in order to bind IL-21. When bound to IL-21, the IL-21 receptor acts through the Jak/STAT pathway, activating STAT1, STAT3, and STATS.

TGFβ decoy receptors (TGF-β-dominant-negative receptor II (DNRII)) block TGFβ signaling by competing with the natural receptors for TGFβ binding. TGFβ-DNRII is a kinase-dead truncated form of RII that contains the extracellular TGFβ binding domain and the transmembrane domain of RII. TGFβ-DNRII binds the ligand but does not phosphorylate and activate RI, which thereby diminishes or eliminates Smad phosphorylation.

Gain-of-function mutations in IL-7Rα have been identified in subjects with B and T cell acute lymphoblastic leukemias (B-ALL and T-ALL) (Zenatti PP, et al. 2011. Nat Genet 43:932-939; Snochat, C. et al. 2011. J Exp Med 208:901-908; McElroy, C.A. et al. 2012. PNAS 109(7):2503-2508). The mutations included insertions and deletions in the N-terminal region of the IL-7Rα TMD, with nearly all of the sequences containing an extra Cys residue, and an S165-to-C165 mutation. The cysteine resulted in constitutive activation of the receptor. Some of the mutations in the T-all group activated JAK1. These gain-of-function IL-7R mutants can be used in any of the aspects provided herein as one of the lymphoproliferative element(s).

Accordingly, in some embodiments, the lymphoproliferative element is a mutated IL-7 receptor. In other embodiments, the mutated IL-7 receptor is constitutively active, activating the JAK-STAT5 pathway in the absence of the cytokine ligand. In still other embodiments, the mutated IL-7 receptor comprises a 1 to 10 amino acid insertion at a position between 237 and 254 that includes a cysteine residue that includes the ability to constitutively activate the STAT5 pathway. In some embodiments, the mutated IL-7 receptor is IL-7Rα-insPPCL (represented by SEQ ID NO:82). Furthermore, in some chimeric lymphoproliferative element (CLE) embodiments provided herein, one or more, but not all, of the domains is from IL-7Rα-insPPCL.

In some embodiments, the lymphoproliferative element is a chimeric cytokine receptor such as but not limited to a cytokine tethered to its receptor that typically constitutively activates the same STAT pathway as a corresponding activated wild-type cytokine receptor such as STAT3, STAT4, and in illustrative embodiments, STATS. In some embodiments, the chimeric cytokine receptor is an interleukin, or a fragment thereof, tethered to or covalently attached to its cognate receptor, or a fragment thereof, via a linker. In some embodiments, the chimeric cytokine receptor is IL-7 tethered to IL-7Rα. In other embodiments, the chimeric cytokine receptor is IL-7 tethered to a domain of IL-7Rα, such as for example, the extracellular domain of IL-7Rα and/or the transmembrane domain of IL-7Rα. In some embodiments, the lymphoproliferative element is a cytokine receptor that is not tethered to a cytokine, and in fact in illustrative embodiments, provided herein a lymphoproliferative element is a constitutively active cytokine receptor that is not tethered to a cytokine. These chimeric IL-7 receptors typically constitutively activate STAT5 when expressed.

In illustrative embodiments of any of the methods and compositions provided herein that include a lymphoproliferative element, wherein the lymphoproliferative element is a cytokine or cytokine receptor polypeptide, or a fragment thereof comprising a signaling domain, the lymphoproliferative element can comprise an interleukin polypeptide covalently attached to a portion of its cognate interleukin receptor polypeptide via a linker. Typically, this portion of the cognate interleukin receptor includes a functional portion of the extracellular domain capable of binding the interleukin cytokine and the transmembrane domain. In some embodiments, the intracellular domain is an intracellular portion of the cognate interleukin receptor. In some embodiments, the intracellular domain is an intracellular portion of a different cytokine receptor that is capable of promoting lymphocyte proliferation. In some embodiments the lymphoproliferative element is an interleukin polypeptide covalently attached to its full length cognate interleukin receptor polypeptide via a linker.

In some embodiments, the lymphoproliferative element can include a cytokine receptor or a fragment that includes a signaling domain thereof. In some embodiments, the cytokine receptor can be CD40, CRLF2, CSF2RA, CSF2RB, CSF3R, EPOR, GHR, IFNAR1, IFNAR2, IFNGR1, IFNGR2, IFNLR1, IL1R1, IL1RAP, IL1RL1, IL1RL2, IL2RA, IL2RG, IL2RB, IL3RA, IL4R, IL5RA, IL6R, IL6ST, IL7RA, IL9R, IL10RA, IL10RB, IL12RB1, IL13RA2, IL15RA, IL17RA, IL17RB, IL17RC, IL17RE, IL18R1, IL18RAP, IL20RA, IL20RB, IL21R, IL22RA1, IL23R, IL27R, IL31RA, LEPR, LIFR, MPL, OSMR, PRLR, TNFRSF4, TNFRSF8, TNFRSF9, TNFRSF14, or TNFRSF18. Exemplary embodiments of chimeric cytokine receptors that include functional intracellular domains of the above-listed cytokine receptors were tested in Examples 17 and 18 to confirm that chimeric cytokine receptors that included these functional intracellular domains could function as lymphoproliferative elements.

In certain illustrative embodiments, the lymphoproliferative element comprises a cytokine receptor, or a fragment thereof that includes a signaling domain, that activates a Jak/STAT5 pathway. For example, such lymphoproliferative element can include an intracellular domain of IL21R, IL27R, IL31RA, LIFR, and OSMR. As shown in the experiments of Examples 17 and 18 and Tables 7 to 17, chimeric lymphoproliferative elements that include intracellular domains of these genes were found among the candidate chimeric polypeptides that induced the highest magnitude of proliferation in PBMCs cultured in the absence of exogenous cytokines such as IL-2.

In some embodiments, the lymphoproliferative element can comprise an intracellular domain that is an interleukin or an interleukin receptor and that was a part of a Top Construct identified in Libraries 1A, 1.1A, 2B, 2.1B, 3A, 3.1A, 3B, 3.1B, 4B, and/or 4.1B of Examples 17 and 18 (Tables 7 to 17). In some embodiments, the lymphoproliferative element can comprise an intracellular domain that is a cytokine receptor and that was a part of a Top Construct identified in Libraries 1A, 1.1A, 2B, 2.1B, 3A, 3.1A, 3B, 3.1B, 4B, and/or 4.1B of Examples 17 and 18 (Tables 7 to 17). In some embodiments, the lymphoproliferative element can comprise an intracellular domain that includes at least one ITAM motif and that was a part of a Top Construct identified in Libraries 1A, 1.1A, 2B, 2.1B, 3A, 3.1A, 3B, 3.1B, 4B, and/or 4.1B of Examples 17 and 18 (Tables 7 to 17).

In illustrative embodiments, the lymphoproliferative element can comprise an intracellular domain from IL7R, IL12RB1, IL15RA, or IL27RA, which were present in constructs that showed particularly noteworthy enrichments (i.e. elicited highest magnitude of proliferation) in an initial screen and a repeated screen as detailed in Examples 17 and 18 (Tables 19 to 23).

In illustrative embodiments, the lymphoproliferative element can comprise an intracellular domain from the cytokine receptors CD27, CD40, CRLF2, CSF2RA, CSF3R, EPOR, FCER1G, FCGR2A, FCGR2C, GHR, IFNAR1, IFNAR2, IFNGR2, IL1R1, IL1RL1, IL2RA, IL2RG, IL3RA, IL5RA, IL6R, IL7R, IL9R, IL10RB, IL11RA, IL12RB1, IL13RA1, IL13RA2, IL15RA, IL17RB, IL18R1, IL18RAP, IL20RB, IL22RAT1, IL27RA, IL31RA, LEPR, MPL, OSMR, PRLR, TNFRSF4, TNFRSF8, TNFRSF9, TNFRSF14, or TNFRSF18, which were present in constructs that showed particularly noteworthy enrichments (i.e. elicited highest magnitude of proliferation) in an initial screen and a repeated screen as detailed in Examples 17 and 18 (Tables 19 to 23).

In illustrative embodiments, the lymphoproliferative element comprises an intracellular domain from CD3D, CD3E, CD3G, CD79A, CD79B, FCER1G, FCGR2A, or FCGR2C, which include at least one ITAM motif and were present in constructs that showed particularly noteworthy enrichments (i.e. elicited highest magnitude of proliferation) in an initial screen and a repeated screen as detailed in Examples 17 and 18 (Tables 19 to 23).

In some embodiments, the lymphoproliferative element in this paragraph, which were shown in examples 17 and 18 to be active in constructs with only a single intracellular domain, can be the intracellular domain of a lymphoproliferative element with either two or more intracellular domains, or in illustrative embodiments a single intracellular domain, i.e. the lymphoproliferative element does not comprise two or more intracellular domains. In illustrative embodiments, the intracellular domain in a lymphoproliferative element comprises a domain from CD40, CRLF2, CSF2RA, CSF3R, EPOR, FCGR2A, IFNAR2, IFNGR2, IL1R1, IL3RA, IL7R, IL10RB, IL11RA, IL12RB1, IL13RA2, IL18RAP, IL31RA, MPL, MYD88, TNFRSF14, or TNFRSF18, which were present in constructs that showed particularly noteworthy enrichments (i.e. elicited highest magnitude of proliferation) in an initial screen and a repeated screen as detailed in Example 18 as single intracellular signaling domains (Tables 22 and 23). In illustrative embodiments, the intracellular domain in a lymphoproliferative element comprises a domain from IL7R or IL12RB1, which were present in constructs that showed particularly noteworthy enrichments (i.e. elicited highest magnitude of proliferation) in an initial screen and a repeated screen as detailed in Example 18 (Tables 22 and 23). In some embodiments, the intracellular domain in a lymphoproliferative element with a single intracellular domain can be a cytokine receptor. In illustrative embodiments, the cytokine receptor in a lymphoproliferative element with a single intracellular domain comprises a domain from CD40, CRLF2, CSF2RA, CSF3R, EPOR, FCGR2A, IFNAR2, IFNGR2, IL1R1, IL3RA, IL7R, IL10RB, IL11RA, IL12RB1, IL13RA2, IL18RAP, IL31RA, MPL, TNFRSF14, or TNFRSF18, which were present in constructs that showed particularly noteworthy enrichments (i.e. elicited highest magnitude of proliferation) in an initial screen and a repeated screen as detailed in Example 18 (Tables 22 and 23). In some embodiments, the intracellular domain in a lymphoproliferative element can include at least one ITAM motif. In illustrative embodiments, the intracellular domain in a lymphoproliferative element that includes at least one ITAM motif comprises a domain from FCGR2A, which was present in constructs that showed particularly noteworthy enrichments (i.e. elicited highest magnitude of proliferation) in an initial screen and a repeated screen as detailed in Example 18 (Table 22).

In illustrative embodiments, the lymphoproliferative element can comprise a costimulatory domain from CD27, CD28, OX40 (also referred to as TNFRSF4), GITR (also referred to as TNFRSF18), or HVEM (also referred to as TNFRSF14), which were present in constructs that showed particularly noteworthy enrichments (i.e. elicited highest magnitude of proliferation) in an initial screen and a repeated screen as detailed in Examples 17 and 18 (Tables 19 to 23).

In some embodiments, the lymphoproliferative element can be one of the constructs M024-S190-S047, M025-5050-S197, M036-S170-S047, M012-5045-5048, M049-S194-S064, M025-S190-S050, M025-S190-S05, E013-T041-S186-S051, E013-T028-S186-S051, E014-T015-S186-S051, E011-T016-S186-S050, E011-T073-S186-S050, or E013-T011-S186-S211, all of which stimulated proliferation of resting lymphocytes after transduction as shown in Example 21 (FIGs. 35 and 36). In certain embodiments, lymphoproliferative elements comprise an extracellular domain from CSF3R, IL3RA, ICOS, CRLF, CSF2RA, LIFR, or CD40; a first intracellular domain from MyD88, CD40, or MPL, and/or a second intracellular domain from CD27 or MyD88.

In some embodiments, the lymphoproliferative element can be the construct E013-T041-S186-S051, which stimulated proliferation of resting lymphocytes after transduction with a replication incompetent recombinant retroviral particle displaying UCHT1scFvFc-GPI as shown and analyzed in Example 22 (FIGs. 38A and B). In other embodiments the lymphoproliferative element is IL7-IL7RAIL2RB, as shown and analyzed in Example 22.

As detailed in Example 17, for Libraries 1A and 1.1A, constructs with domains from the cytokine receptors CD27, IL1RL1, IL6R, IL31RA, TNFRSF4, or TNFRSF18, showed particularly noteworthy enrichments (i.e. elicited highest magnitude of proliferation) in both Libraries 1A and 1.1A (Table 19).

As detailed in Example 17, for Libraries 2B and 2.1B, constructs with domains from the cytokine receptors CD40, FCER1G, FCGR2C, IFNGR2, GHR, IL10RB, IL11RA, IL13RA2, IL17RB, IL22RA1, TNFRSF14, or TNFRSF9, showed particularly noteworthy enrichments (i.e. elicited highest magnitude of proliferation) in both Libraries 2B and 2.1B (Table 20).

As detailed in Example 18, for Libraries 3A and 3.1A, constructs with domains from the cytokine receptors CD27, CD40, CSF2RA, FCGR2A, MPL, OSMR, TNFRSF4, or TNFRSF18 showed particularly noteworthy enrichments (i.e. elicited highest magnitude of proliferation) in both Libraries 3A and 3.1A (Table 21).

As detailed in Example 18, for Libraries 3B and 3.1B, constructs with domains from the cytokine receptors CD27, CD40, CRLF2, CSF2RA, CSF3R, EPOR, FCER1G, FCGR2A, FCGR2C, IFNAR1, IFNAR2, IFNGR2, IL1RL1, IL2RG, IL3RA, IL5RA, IL6R, IL7R, IL9R, IL10RB, IL11RA, IL12RB1, IL13RA1, IL13RA2, IL15RA, IL18RAP, IL20RB, IL27RA, IL31RA, LEPR, MPL, OSMR, PRLR, TNFRSF4, TNFRSF8, TNFRSF9, TNFRSF14, or TNFRSF18, showed particularly noteworthy enrichments (i.e. elicited highest magnitude of proliferation) in both Libraries 3B and 3.1B (Table 22).

As detailed in Example 18, for Libraries 4B and 4.1B, constructs with domains from the cytokine receptors CD27, CD40, CRLF2, CSF2RA, CSF3R, EPOR, FCGR2A, FCGR2C, IFNAR2, IFNGR2, IL1R1, IL2RA, IL3RA, IL2RG, IL6R, IL7R, IL10RB, IL11RA, IL31RA, IL13RA1, IL13RA2, IL18R1, MPL, OSMR, TNFRSF4, TNFRSF9, or TNFRSF18, showed particularly noteworthy enrichments (i.e. elicited highest magnitude of proliferation) in both Libraries 4B and 4.1B (Table 23).

In certain illustrative embodiments, the lymphoproliferative element comprises an intracellular domain of CD40, MPL and IL2Rb, which are demonstrated in the Examples herein to promote PBMC proliferation.

In some embodiments, the lymphoproliferative element can be other than a cytokine receptor. In some embodiments, the lymphoproliferative element other than a cytokine receptor can include an intracellular signaling domain from CD2, CD3D, CD3G, CD3Z, CD4, CD8RA, CD8RB, CD28, CD79A, CD79B, FCER1G, FCGR2A, FCGR2C, or ICOS. Exemplary embodiments of chimeric lymphoproliferative elements that include these recited genes are provided in Examples 17 and 18, and the tables cited therein.

In some embodiments, CLE is other than IL-15 tethered to the IL-2/IL-15 receptor.

In some of the methods and compositions disclosed herein, expression of the lymphoproliferative element is induced by and can even dependent on binding of a compound to a control element (as discussed elsewhere herein), which in non-limiting embodiments is a ribowsitch. In some embodiments, the lymphoproliferative element is expressed from a promoter active in a T cell and/or an NK cell. For methods and compositions provided herein, a skilled artisan will recognize that promoters are known that are active in T cells and/or NK cells and can be used to express a first engineered signaling polypeptide or a second engineered signaling polypeptide, or any component thereof. In illustrative embodiments, such a promoter is not active in a packaging cell line, such as the packaging lines disclosed herein. In some embodiments, the promoter is the EF1a promoter or the murine stem cell virus (MSCV) promoter (Jones et al., Human Gene Therapy (2009) 20: 630-40). In illustrative embodiments, the promoter is the T cell specific CD3 zeta promoter.

In some embodiments, the lymphoproliferative element is microenvironment restricted. For example, the lymphoproliferative element can be a mutated receptor that binds its respective cytokine differentially in aberrant versus physiological conditions. For example, an IL-7R that can bind IL7 more strongly in a tumor environment than in a normal physiological environment can be used.

In some embodiments, the lymphoproliferative element is fused to a recognition or elimination domain. Such recognition or elimination domains are disclosed in more detail herein. Such fusion provides the advantage, especially when a truncated or other mutated lymphoproliferative element is used, of requiring less polynucleotides in the retroviral genome. This is important in illustrative embodiments provided herein, because it helps to permit more nucleic acids encoding functional elements to be included in the retroviral genome and because it adds a mechanism by which cells expressing the lymphoproliferative element can be killed if their proliferation is no longer wanted or is detrimental to an organism.

In some embodiments, a lymphoproliferative element, including a CLE, comprises an intracellular activating domain as disclosed hereinabove. In some illustrative embodiments a lymphoproliferative element is a CLE comprising an intracellular activating domain comprising an ITAM-containing domain, As such, the CLE can comprise an intracellular activating domain having at least 80%, 90%, 95%, 98%, or 100% sequence identity to the CD3Z, CD3D, CD3E, CD3G, CD79A, CD79B, DAP12, FCERlG, FCGR2A, FCGR2C. DAP10/CD28, or ZAP70 domains provided herein. In certain illustrative embodiments, the intracellular activating domain is an ITAM-containing domain from CD3D, CD3G, CD3Z, CD79A, CD79B, FCER1G, FCGR2A, or FCGR2C. CLEs comprising these intracellular activating domains are demonstrated in Examples 17 and 18 herein, and associated tables 18-23, as being effective at promoting proliferation of PBMCs ex vivo in cultures in the absence of exogenous cytokines such as exogenous IL-2. In some embodiments, provided herein are CLEs comprising an intracellular domain from CD3D, CD3G, CD3Z, CD79A, FCER1G.

In some embodiments, one or more domains of a lymphoproliferative element is fused to a co-stimulatory domain and/or an intracellular activating domain of a CAR. A chimeric lymphoproliferative element as disclosed herein, is not a chimeric antigen receptor (CAR) because it does not comprise an ASTR. However, in some embodiments, one or more intracellular domains of a lymphoproliferative element can be part of the same polypeptide as a CAR or can be fused and optionally functionally connected to some components of CARs. For example, a lymphoproliferative element can be fused to an antigen-specific targeting region (ASTR) and activated by binding of the ASTR to its antigen. In still other embodiments, an engineered signaling polypeptide can include an ASTR, an intracellular activation domain (such as a CD3 zeta signaling domain), a co-stimulatory domain, and a lymphoproliferative domain. Further details regarding co-stimulatory domains, intracellular activating domains, ASTRs and other CAR domains, are disclosed elsewhere herein.

In illustrative embodiments herein, a T cell and/or NK cell survival element is introduced into a resting T cell and/or resting NK cell, typically by transducing the resting T cell and/or resting NK cell with a replication incompetent recombinant retroviral particle whose genome encodes the T cell and/or NK cell survival element as part of an engineered signaling polypeptide. In some embodiments, a lymphoproliferative element is also a T cell and/or NK cell survival element. As discussed above, some of the lymphoproliferative elements not only promote proliferation, but they promote cell survival as well. In some embodiments, the T cell and/or NK survival cell motif is not a lymphoproliferative element. In some embodiments, the T cell and/or NK cell survival motif can be a CD28 T cell survival motif or a CD137 cell survival motif. Such T cell survival motifs can be found on engineered signaling polypeptides that include an ASTR, such as an scFv. In an illustrative embodiment, the T cell survival motif is a CD28 T cell survival motif or a CD137 motif connected to an scFv through a CD8a transmembrane domain or a CD28 transmembrane domain. In certain embodiments, said intracellular signaling domain comprises a polypeptide sequence comprising an immunoreceptor tyrosine-based activation motif (ITAM). In a certain embodiment, said polypeptide sequence is a CD3ζ signaling domain.

In some embodiments, the lymphoproliferative element is not a polypeptide, but rather comprises an inhibitory RNA. In some embodiments, methods, uses, compositions, and products of processes according to any aspect herein include both a lymphoproliferative element comprising an inhibitory RNA and a lymphoproliferative element that is an engineered signaling polypeptide. In embodiments where a lymphoproliferative element is an inhibitory RNA, that inhibitory RNA can be a miRNA that stimulates the STAT5 pathway typically by potentiating activation of STAT5 by degrading or causing down-regulation of a negative regulator in the SOCS pathway. In some embodiments, the miRNA is directed to mRNA encoding proteins that affect proliferation such as but not limited to ABCG1, SOCS1, TGFbR2, SMAD2, cCBL, and PD1. In illustrative embodiments, as exemplified herein, such inhibitory RNA (e.g. miRNAs) can be located in introns in packaging cells and/or a replication incompetent recombinant retroviral particle genome and/or a retroviral vector, typically with expression driven by a promoter that is active in a T cell and/or NK cell. Not to be limited by theory, inclusion of introns in transcription units are believed to result in higher expression and/or stability of transcripts. As such, the ability to place miRNAs within introns of a retroviral genome adds to the teachings of the present disclosure that overcome challenges in the prior art of trying to get maximum activities into the size restrictions of a retroviral, such as a lentivirus genome. In some embodiments, 1, 2, 3, 4, 5, 6, 7, 8, 9, or 10 miRNAs, in illustrative embodiments between 2 and 5, for example 4 miRNAs, one or more of which each bind nucleic acids encoding one or more of ABCG1, SOCS1, TGFbR2, SMAD2, cCBL, and PD1, can be included in the recombinant retroviral genome and delivered to a target cell, for example T cells and/or NK cells, using methods provided herein. In fact, as provided herein 1, 2, 3, or 4 miRNAs can be delivered in a single intron such as the EF1a intron.

ABCG1 is an ATP-binding cassette transporter that negatively regulates thymocyte and peripheral lymphocyte proliferation (Armstrong et al. 2010. J Immunol 184(1):173-183).

SOCS1 is a member of the SOCS (Suppressor of cytokine signaling) family of negative regulators of cytokine signal transduction that inhibit the Jak/Stat pathway such as STAT5. SOCS1 is also known as JAB (Janus Kinase binding protein), SSI-1 (Stat-induced Stat inhibitor-1), and TIP3 (Tec-interacting protein).

TGFbR2 is a member of the serine/threonine protein kinase family that binds TGF-β, forming a complex that phosphorylates proteins that then enter the nucleus and regulate transcription of genes related to proliferation.

SMAD2 mediates the signal of the transforming growth factor (TGF)-β and regulates multiple cellular processes, such as cell proliferation, apoptosis, and differentiation.

cCBL is an E3 ubiquitin ligase that inhibits TCR signaling by dephosphorylation and inactivation of ZAP-70 and through internalization of the TCR.

PD1 (CD279) is a cell surface receptor expressed on T cells and ProB cells. PD-1 binds two ligands, PD-L1 and PD-L2. Signaling through PD-1 functions to prevent activation of cells.

In some embodiments, herein the lymphoproliferative element is a polypeptide comprising the intracellular region of any of the genes of Table 18. In some embodiments, the lymphoproliferative element comprises or is an intracellular domain identified in the chimeric polypeptides in Table 18. In these embodiments, the lymphoproliferative element can be a polypeptide that is a chimeric polypeptide (See CLEs below), or is not a CLE but comprises or is an intracellular domain of a gene identified in the P3 (first intracellular domain) position of Table 18. Table 18 identifies CLE constructs that promoted cell proliferation of PBMCs between day 7 and the last day where a second intracellular domain was not present on the construct. In some subembodiments of this embodiment, the lymphoproliferative element is a chimeric polypeptide (i.e. a CLE - see below) that includes a combination of transmembrane domain and intracellular domain, with or without an extracellular domain comprising a dimerizing motif.

In some embodiments, the lymphoproliferative element comprises MPL, or is MPL, or a variant and/or fragment thereof, including a variant and/or fragment that includes at least 75, 80, 85, 90, 95, 96, 97, 98, 99, or 100% of the intracellular domain of MPL, with or without a transmembrane and/or extracellular domain of MPL, and/or has at least 75, 80, 85, 90, 95, 96, 97, 98, 99, or 100% sequence idnentity to the intracellular domain of MPL, with or without a transmembrane and/or extracellular domain of MPL, wherein the variant and/or fragment retains the ability to promote cell proliferation of PBMCs, and in some embodiments T cells. In some embodiments, an MPL fragment included in the compositions and methods herein has and/or retains a JAK-2 binding domain. In some embodiments, an MPL fragment included herein has or retains the ability to activate a STAT. The full intracellular domain of MPL is SEQ ID NO:491 (part S186 in Tables 7 to 18). MPL is the receptor for thrombopoietin. Several cytokines such as thrombopoietin and EPO are referred to in the literature and herein as either a hormone or a cytokine.

In some embodiments, which provide separate aspects of the present disclosure, provided herein are chimeric polypeptides that are chimeric lymphoproliferative elements (CLEs), as well as isolated polynucleotides and nucleic acid sequences that encode the same. Exemplary CLEs are illustrated in FIGs. 30-33. CLEs herein promote cell proliferation of T cells and/or NK cells and can optionally also promote survival of T cells and/or NK cells. Some CLEs promote proliferation and optionally also survival of other types of PBMCs, for example B cells. Embodiments provided herein that include nucleic acid sequences that encode a CLE and a CAR can be referred to herein as CLE CAR polynucleotide embodiments for drafting convenience. In some embodiments, CLEs can include a transmembrane domain and a first intracellular domain. Furthermore, in illustrative embodiments, as shown in FIGs. 30-33, CLEs include an extracellular domain and/or a second intracellular domain (and in further embodiments, third, fourth, etc. intracellular domains). Chimeric polypeptides herein are chimeric because at least one domain is from a different polypeptide than at least one of the other domains and such chimera are not found naturally in an organism without human intervention. Some CLEs include a ligand for a receptor and some CLEs do not include a ligand for a receptor.

Not to be limited by theory, such CLEs were designed to promote proliferation and optionally cell survival in B cells, NK cells, and/or T cells in a constitutive manner (i.e. without the requirement of ligand binding for activation). None of the CLEs are found in nature, and many of the CLEs have components that are not usually expressed in B cells, T cells, and/or NK cells in vivo and/or some candidate CLEs are not generally known to specifically promote cell proliferation and/or cell survival signaling in B cells, T cells, and/or NK cells. For example, MPL is usually not expressed in B cells, T cells and/or NK cells. Surprisingly, new CLEs were identified by screening a large number of candidate chimeric polypeptides as set out in Examples 17 and 18, that promoted PBMC cell proliferation. Such CLEs help to meet the long-felt need of identifying mechanisms to stimulate proliferation and optionally survival as well, of B cells, T cells, and/or NK cells, such as would be beneficial for important clinically-relevant technologies, such as CAR-T and T-cells that are genetically engineered to express a defined TCR. As such, it is believed that some CLEs will provide a T cell and/or NK cell the ability to expand in vivo without the need for lymphodepleting the host.

Chimeric lymphoproliferative elements provided herein, and isolated polynucleotides and nucleic acids encoding the same can be included in any aspect provided herein that includes a lymphoproliferative element. For example, a first engineered signaling polypeptide can be, or can include a CLE in aspects provided herein that include one or more transcriptional units that encode a first engineered signaling polypeptide regulated by a control element. Furthermore, separate aspects as described herein are provided that specifically include a CLE. For example, such aspects include isolated chimeric lymphoproliferative polypeptides, isolated polynucleotides and nucleic acid sequences encoding the same, and vectors, including plasmids, viral, and retroviral vectors including such nucleic acid sequences or isolated polynucleotides. Such aspects further include, methods for transducing or transfecting PBMCs, such as B cells, or especially T cells and NK cells, with the isolated polynucleotides and vectors comprising the same. Such cells can be isolated, unaltered cells, or they can be cells that have been modified such as cells that are genetically modified, such as to express a defined TCR, or CAR-T cells.

In some embodiments, CLEs include both an extracellular portion and a transmembrane portion that is from the same protein, in illustrative embodiments the same receptor, either of which in illustrative embodiments is a mutant, thus forming an extracellular and transmembrane domain. These domains can be from a cytokine receptor, or a mutant thereof, or a hormone receptor, or a mutant thereof in some embodiments that have been reported to be constitutively active when expressed at least in some cell types. In illustrative embodiments, such extracellular and transmembrane domains do not include a ligand binding region. It is believed that such domains do not bind a ligand when present in CLEs and expressed in B cells, T cells, and/or NK cells. Mutations in such receptor mutants can occur in the transmembrane region or in the extracellular juxtamembrane region. Not to be limited by theory, a mutation in at least some extracellular - transmembrane domains of CLEs provided herein, are responsible for signaling of the CLE in the absence of ligand, by bringing activating chains together that are not normally together, or by changing the confirmation of a linked transmembrane and/or intracellular domain.

One aspect that utilizes such transmembrane domains of receptor mutants, provided herein is an isolated polynucleotide comprising one or more nucleic acid sequences, wherein:
a first nucleic acid sequence of the one or more nucleic acid sequences encodes a chimeric polypeptide comprising in amino to carboxy orientation,
(a) an extracellular and transmembrane domain from a cytokine receptor or a hormone receptor, wherein at least one of the extracellular domain and the transmembrane domain comprise a mutation that is found on a constitutively active mutant of the cytokine receptor and wherein the extracellular sequence does not bind a ligand of the cytokine receptor; and
(b) a first intracellular domain selected from an intracellular domain of a gene having a first intracellular domain and optionally a second domain of a selected polypeptide identified in Tables 7 to 11, wherein said chimeric polypeptide promotes cell proliferation of B cells, T cells, and/or NK cells.

The extracellular region of such extracellular and transmembrane domains in these embodiments, is typically long enough to form a linker, in illustrative embodiments a flexible linker between a transmembrane domain and another functional peptide region, such as a clearance domain, that in some embodiments, is linked to the amino terminus of the extracellular region. Accordingly, the extracellular region when present to form an extracellular and transmembrane domain, can be between 1 and 1000 amino acids, and is typically between 4 and 400 amino acids. between 4 and 200 amino acids, between 4 and 100 amino acids, between 4 and 50 amino acids, between 4 and 25 or between 4 and 20 amino acids in length. In one embodiment, the extracellular region is GGGS for an extracellular and transmembrane domain of this aspect.

The transmembrane domain whether as part of embodiments that include an extracellular and transmembrane domain as one part, for example as shown in Libraries 1A, 1.1A, 1.1B, 2B and 2.1B of Example 17, or as part of embodiments that include an extracellular dimerizing motif, as shown in Libraries 3A, 3B, 3.1A, 3.1B, 4B, and 4.1B of Example 18, as discussed in more detail below, are typically at least long enough to cross a plasma membrane. Accordingly, transmembrane domains or transmembrane regions of extracellular and transmembrane domains, can be between 10 and 250 amino acids, and are more typically at least 15 amino acids in length, and can be for example between 15 and 100, 15 and 75, 15 and 50, 15 and 40, or 15 and 30 amino acids.

Exemplary extracellular and transmembrane domains for CLEs of embodiments that include such domains, in illustrative embodiments, are extracellular regions, typically less than 30 amino acids of the membrane-proximal extracellular domains along with transmembrane domains from mutant receptors that have been reported to be constitutive, that is not require ligand binding for activation of an associated intracellular domain. In illustrative embodiments, such extracellular and transmembrane domains include IL7RA Ins PPCL, CRLF2 F232C, CSF2RB V449E, CSF3R T640N, EPOR L251C I252C, GHR E260C I270C, IL27RA F523C, and MPL S505N. Further non-limiting examples of such extracellular and transmembrane domains are provided in Table 6 and exemplified in Example 17 and the corresponding tables. In some embodiments, the extracellular and transmembrane domain does not comprise more than 10, 20, 25 30 or 50 consecutive amino acids that are identical in sequence to a portion of the extracellular and/or transmembrane domain of IL7RA, or a mutant thereof. In some embodiments, the extracellular and transmembrane domain is other than IL7RA Ins PPCL. In some embodiments, the extracellular and transmembrane does not comprise more than 10, 20, 25, 30, or 50 consecutive amino acids that are identical in sequence to a portion of the extracellular and/or transmembrane domain of IL15R.

Additional exemplary transmembrane domains are provided in Example 18. These transmembrane domains in illustrative embodiments are from type I transmembrane proteins.

Accordingly provided herein in one aspect is an isolated polynucleotide comprising one or more nucleic acid sequences, wherein:
a first nucleic acid sequence of the one or more nucleic acid sequences encodes a chimeric polypeptide comprising in amino to carboxy orientation,
a) a transmembrane domain from a type I transmembrane protein; and
b) a first intracellular domain selected from an intracellular domain of a gene having a first intracellular domain of a selected polypeptide identified in Tables 12 to 17, wherein said chimeric polypeptide promotes cell cell proliferation of B cells, T cells, and/or NK cells.

In illustrative embodiments, said chimeric polypeptide promotes cell proliferation of PBMCs, for example B cells and/or NK cells, and/or in illustrative embodiments T cells. As shown in Example 18, such chimeric polypeptides are capable of promoting cell proliferation of PBMCs in the absence of exposure of the PBMCs to exogenous cytokines such as IL-2, IL-15, or IL-7 during culturing, for example in the absence of adding IL-2 to culture media of PBMCs (e.g. B cells, T cells, or NK cells) that express a nucleic acid encoding the chimeric polypeptide. As illustrative in Example 18, for such embodiments IL-2 can be added during transduction of PBMCs, but not in subsequent culturing. For example, chimeric polypeptides disclosed herein are lymphoproliferative elements because they are capable of promoting cell proliferation and optionally cell survival of PBMCs, and in illustrative embodiments, T cells, without adding IL-2 during culturing of PBMCs (e.g. T cells) after day 1, 2, 3, 4, 5, or 7 of culturing optionally after transduction of the PBMCs with a nucleic acid encoding the chimeric lymphoproliferative element. Example 21 provides another example of a method that can be used to identify and analyze lymphoproliferative elements. Such analysis can include, for example, measuring the relative lymphoproliferative activity of such lymphoproliferative elements, for example by analyzing the magnitude of enrichment provided by genetically modified lymphocytes expressing such lymphoproliferative elements compared to control lymphocytes that do not express such lymphoproliferative elements.

In one embodiment of this aspect, the first nucleic acid sequence further encodes an extracellular domain, and in illustrative embodiments, the extracellular domain comprises a dimerizing motif. In illustrative embodiments of this aspect, the extracellular domain comprises a leucine zipper. In some embodiments, the leucine zipper is from a jun polypeptide, for example c-jun. In certain embodiments the c-jun polypeptide is the c-jun polypeptide region of ECD-11.

In embodiments of any of these aspects and embodiments wherein the transmembrane domain is a type I transmembrane protein, the transmembrane domain can be a Type I growth factor receptor, a hormone receptor, a T cell receptor, or a TNF-family receptor. In an embodiment of any of the aspects and embodiments wherein the chimeric polypeptide comprises an extracellular domain and wherein the extracellular domain comprises a dimerizing motif, the transmembrane domain can be a Type I cytokine receptor, a hormone receptor, a T cell receptor, or a TNF-family receptor.

Exemplary transmembrane domains include any transmembrane domain that was used in Example 18. In some embodiments, the transmembrane domain is from CD4, CD8RB, CD40, CRLF2, CSF2RA, CSF3R, EPOR, FCGR2C, GHR, ICOS, IFNAR1, IFNGR1, IFNGR2, IL1R1, IL1RAP, IL2RG, IL3RA, IL5RA, IL6ST, IL7RA, IL10RB, IL11RA, IL13RA2, IL17RA, IL17RB, IL17RC, IL17RE, IL18R1, IL18RAP, IL20RA, IL22RA1, IL31RA, LEPR, PRLR, and TNFRSF8, or mutants thereof that are known to promote signaling activity in certain cell types if such mutants are present in the constructs provided in Example 18. In some embodiments, the transmembrane domain is from CD40, ICOS, FCGR2C, PRLR, IL3RA, or IL6ST. In some embodiments, the transmembrane is the specific transmembrane domain provided for these genes in the constructs of any one or more of Tables 12 to 17, or a transmembrane domain from any of the constructs in the first 50, 25 or 10 listed constructs of those tables. In illustrative embodiments, the transmembrane domain is from CD40 or ICOS, as non-limiting examples the transmembrane domains for these genes provided in Table 6, or a fragment thereof that retains the ability to be transmembrane, and/or mutants thereof that are known to promote constitutive signaling activity in certain cell types.

Exemplary transmembrane domains from this aspect are shown as P2 in Tables 12 to 17. For Library 3A, transmembrane domains (P2) from CD40, CD8B , CRLF2, CSF2RA, GCGR2C, ICOS, IFNAR1, IFNGR1, IL10RB, IL18R1, IL18RAP, IL3RA, LEPR, and PRLR, or mutants thereof that are known to promote constitutive signaling activity in certain cell types, if such mutants are present in the constructs provided in Example 18, when found at the transmembrane domain position (P2) of candidate chimeric polypeptides elements herein, promoted proliferation of PBMCs between day 7 and the last day, when data is considered in a combined manner for all constructs with a transmembrane domain derived from that gene. In the screens provided in Examples 17 and 18, cells were transduced with replication incompetent recombinant retroviral particles containing a library and then either fed PBMCs, or not fed PBMCs, as discussed in Examples 17 and 18. Screens where the cells were fed PBMCs are identified with an "A" after the library number, e.g. Library 1A and screens where the cells were not fed PBMCs are identified with a "B" after the library number, e.g. Library 1.1B. Additionally, screens identified as libraries containing ".1", e.g. Library 1.1A, were performed in an identical manner to screens of the corresponding library without the ".1" e.g. Library 1.1A was a repeat screen of Library 1A. For Library 3B, transmembrane domains from CD40, ICOS, CSF2RA, IL18R1, IL3RA, and TNFRSF14, or mutants thereof that are known to promote constitutive signaling activity in certain cell types, if such mutants are present in the constructs provided in Example 18, were most capable of promoting cell proliferation. For Library 4B, transmembrane domains from IL3RA, CSFR2RA, IL18R1, CD8B, CD40. ICOS, or mutants thereof that are known to promote constitutive signaling activity in certain cell types, if such mutants are present in the constructs provided in Example 18, were most capable of promoting cell proliferation. In illustrative embodiments, a transmembrane domain in a CLE provided herein is a transmembrane domain from CD40 and ICOS. Examples of transmembrane domains or portions and/or mutants thereof, that were present in constructs that promoted cell proliferation for Libraries 3A, 3B, 3,1A, 3,1B, 4B, and 4.1B are provided in the tables provided in Example 18.

In some embodiments of any aspect herein a chimeric polypeptide comprises a transmembrane domain identified in any of the constructs shown in Tables 12 to 17 other than a transmembrane domain mutant V449E of CSF2RB.

In some embodiments, the extracellular and transmembrane domain is the viral protein LMP1, or a mutant and/or fragment thereof. LMP1 is a multispan transmembrane protein that is known to activate cell signaling independent of ligand when targeted to lipid rafts or when fused to CD40 (Kaykas et al. EMBO J. 20: 2641 (2001)). A fragment of LMP1 is typically long enough to span a plasma membrane and to activate a linked intracellular domain(s). For example, the LMP1 can be between 15 and 386, 15 and 200, 15 and 150, 15 and 100, 18 and 50, 18 and 30, 20 and 200, 20 and 150, 20 and 50, 20 and 30, 20 and 100, 20 and 40, or 20 and 25 amino acids. A mutant and/or fragment of LMP1 when included in a CLE provided herein, retains its ability to activate an intracellular domain. Furthermore, if present, the extracellular domain includes at least 1, but typically at least 4 amino acids and is typically linked to another functional polypeptide, such as a clearance domain, for example, an eTag.

In other embodiments of CLEs provided herein, the extracellular domain includes a dimerizing moiety. Many different dimerizing moieties disclosed herein can be used for these embodiments. In illustrative embodiments, the dimerizing moieties are capable of homodimerizing. Not to be limited by theory, dimerizing moieties can provide an activating function on intracellular domains connected thereto via transmembrane domains. Such activation can be provided, for example, upon dimerization of a dimerizing moiety, which can cause a change in orientation of intracellular domains connected thereto via a transmembrane domain, or which can cause intracellular domains to come into proximity. An extracellular domain with a dimerizing moiety can also serve a function of connecting a recognition tag to a cell expressing a CLE. In some embodiments, the dimerizing agent can be located intracellularly rather than extracellularly. In some embodiments, more than one or multiples of dimerizing domains can be used.

Extracellular domains for embodiments where extracellular domains have a dimerizing motif, are long enough to form dimers, such as leucine zipper dimers. As such, extracellular domains that include a dimerizing moiety can be from 15 to 100, 20 to 50, 30 to 45, or 35 to 40 amino acids, of in illustrative embodiments is a c-Jun portion of a c-Jun extracellular domain provided in Table 6. Extracellular domains of polypeptides that include a dimerizing moiety, may not retain other functionalities. For example, for leucine zippers embodiments, such leucine zippers are capable of forming dimers because they retain a motif of leucines spaced 7 residues apart along an alpha helix. However, leucine zipper moieties of certain embodiments of CLEs provided herein, may or may not retain their DNA binding function.

One exemplary extracellular domain of this type, is a leucine zipper domain from a Jun protein, such as c-Jun. For example, an extracellular domain can be a variant of c-Jun found in NM_002228_3 (Table 6). Such an extracellular domain was used in the constructs discussed in Example 18.

A spacer of between 1 and 4 alanine residues can be included in CLEs between the extracellular domain that has a dimerizing moiety, and the transmembrane domain. Not to be limited by theory, it is believed that the alanine spacer affects signaling of intracellular domains connected to the leucine zipper extracellular region via the transmembrane domain, by changing the orientation of the intracellular domains.

The first and second intracellular domains of CLEs provided herein, are intracellular signaling domains of genes that are known in at least some cell types, to promote proliferation, survival (anti-apoptotic), and/or provide a co-stimulatory signal that enhances a differentiation state, proliferative potential or resistance to cell death. As such, these intracellular domains can be intracellular domains from lymphoproliferative elements and co-stimulatory domains provided herein. Some of the intracellular domains of candidate chimeric polypeptides are known to activate JAK1/JAK2 signaling and STATS. The genes and intracellular domains thereof that are found in a first intracellular domain are the same as the second intracellular domain, except that if the first and second intracellular domain are identical, then at least one, and typically both the transmembrane domain and the extracellular domain are not from the same gene.

Exemplary intracellular domains include those from the constructs listed in Tables 7 to 11. Exemplary intracellular domains from these genes that were empirically determined to be active in the experiment of Example 17, are provided in the first intracellular domain (P3) and second intracellular domain (P4) locations of tables provided in Example 17 and as further listed in this section below. Illustrative intracellular domains identified in the top hits for the Example 17 screen included CD40, CSF2RA, IFNAR1, IL1RAP, IL4R, IL6ST, IL11RA, IL12RB2, IL17RA, IL17RD, IL17RE, IL18R1, IL21R, IL23R, MPL, and MyD88. Illustrative intracellular domains identified in the top hits for the Example 18 screen included CD40, LEPR, MyD88, IFNAR2, MPL, IL18R1, IL13RA2, IL10RB, IL23R, or CSF2RA. In certain illustrative embodiments, the first intracellular domain is MPL, LEPR, MyD88, or IFNAR2. For these certain illustrative embodiments non-limiting exemplary second intracellular domain (P4) domains are those of genes that are linked to the corresponding MPL, LEPR, MyD88, IFNAR2, CD40, CD79B, or CD27 first intracellular domain (P3) provided in Tables 12 to 17, including in some non-limiting examples the first and/or second intracellular domains for those genes provided in these tables. For these certain illustrative embodiments other non-limiting exemplary second intracellular (P4) domains are those of genes that are linked to the corresponding MPL, LEPR, MYD88, IFNAR2, CD40, CD79B, or CD27 first intracellular domain in tables provided in Example 18, including as non-limiting examples, the first and/or second intracellular domains of those genes provided in these tables. In some embodiments, the chimeric lymphoproliferative element can be any of the polypeptides in Tables 19-23 of Examples 17 and 18.

In certain illustrative embodiments, the second intracellular domain is from CD3D, CD3G, CD27, CD40, CD79A, CD79B, FCER1G, FCGRA2, ICOS, TNFRSF4, and TNFRSF8, or mutants thereof that are known to promote signaling activity in certain cell types if such mutants are present in the constructs provided in Example 18, In certain illustrative embodiments, the second intracellular domain is CD40, CD79B, TNFRSF4, TNFRSF9, TNFRSF14, FCGRA2, CD3G, or CD27, including in illustrative examples an intracellular domain of CD40, CD79B, and CD27. For these certain illustrative embodiments non-limiting exemplary first intracellular domains (P3) are those of genes that are linked to CD40, CD79B, TNFRSF4, TNFRSF9, TNFRSF14, FCGRA2, CD3G, or CD27, including in illustrative examples an intracellular domain of CD40, CD79B, or CD27 in Tables 12 to 17, including as non-limiting examples, the first and/or second intracellular domains of those genes provided in these tables. For these certain illustrative embodiments, other non-limiting exemplary first intracellular (P3) domains are those of genes that are linked to CD40, CD79B, TNFRSF4, TNFRSF9, TNFRSF14, FCGRA2, CD3G, or CD27, including in illustrative examples an intracellular domain of CD40, CD79B, and CD27 second intracellular domains in tables for these genes as P3 provided in Example 18, including as non-limiting examples, the first and/or second intracellular domains of those genes provided in these tables.

Detailed information, including sequence information, about exemplary intracellular domains of the above genes that were identified in Examples 17 and 18 within constructs that promoted cell survival and proliferation are provided in Table 6. Intracellular domains that can be included in CLE embodiments provided herein include mutants and/or fragments of the intracellular domains of the recited genes provided that such mutants and/or fragments retain the ability to promote proliferation, survival (anti-apoptotic), and/or provide a co-stimulatory signal that enhances a differentiation state, proliferative potential or resistance to cell death. As such, the intracellular domains can be, for example, between 10 and 1000, 10 and 750, 10 and 500, 10 and 250, 10 and 100 amino acids.

In some embodiments, all domains of a CLE are other than an IL-7 receptor, or a mutant thereof, and/or a fragment thereof that has at least 10, 15, 20, or 25 contiguous amino acids of IL-7 receptor, or other than an IL-15 receptor, or a mutant thereof, and/or a fragment thereof that has at least 10, 15, 20, or 25 contiguous amino acids of IL-15 receptor. In some embodiments, a CLE does not comprise a combination of first intracellular domain and second intracellular domain of CD40 and MyD88.

In illustrative embodiments, CLEs include a recognition and/or elimination domain. Details regarding recognition and/or elimination domains are provided in other sections herein. Any of the recognition and/or elimination domains provided herein can be part of a CLE. Typically the recognition domain is linked to the N terminus of the extracellular domain. Not to be limited by theory, in some embodiments, the extracellular domain includes the function of providing a linker, in illustrative embodiments a flexible linker, linking a recognition domain to a cell that expresses the CLE.

In illustrative embodiments, as illustrated in FIG. 30 and FIG. 32, a CLE provided herein is co-expressed with a CAR which can be designed according to any of the CAR embodiments provided herein, or otherwise known in the art.

Some embodiments provided herein, are isolated polynucleotides and nucleic acid sequences encoding any of the CLEs provided herein, as exemplified in FIGs. 30-33. Such isolated polynucleotides can include any elements known in the art for providing expression of a transcript, such as a transcript encoding a CLE. For example, the isolated polynucleotide can include a promoter element that is active in B cells, T cells, and/or NK cells. Such promoters that are appropriate for these embodiments are known in the art, some of which are identified in other sections of this disclosure. A skilled artisan will understand as discussed in more detail in Examples 17 and 18, for example, how to design isolated polynucleotides and vectors containing the same, for expressing any of the CLE embodiments provided herein. For example, in some embodiments, a Kozak sequence is provided within 10 nucleotides upstream of an ATG start site encoding the amino terminal amine acid of a CLE or a CAR located upstream a CLE on the same transcription unit. Accordingly, in polynucleotide embodiments comprising a nucleic acid encoding a CLE provided herein, the polynucleotide can further comprise one or more of a Kozak-related sequence, a WPRE element, and a multiple stop sequence, for example a double stop sequence or a triple stop sequence.

Furthermore, polynucleotides that include a nucleic acid sequence encoding a CLE provided herein, also typically comprise a signal sequence to direct expression to the plasma membrane. Exemplary signal sequences are provided herein in other sections. Elements can be provided on the transcript such that both a CAR and CLE are expressed from the same transcript. Such a construct is advantageous in requiring less nucleic acids than if these elements were expressed from different transcripts, which is an important feature that allows such constructs to be present in vectors, such as retroviral genomes, used to transfect or transduce B cells, T cells, and/or NK cells, for example.

Numerous chimeric polypeptide candidates were designed and identified as lymphoproliferative elements in Examples 17 and 18 herein. For Library 1A, which included constructs that encoded the chimeric polypeptides and a CAR, 172 top candidate chimeric polypeptides were identified (See Table 7) that promoted PBMC proliferation between day 7 and the last day when cultured in the absence of IL-2. For Library 2B, which included constructs that encoded the chimeric polypeptides but did not include a CAR, 167 top candidate chimeric polypeptides were identified (See Table 8) that promoted PBMC proliferation between day 7 and the last day when cultured in the absence of IL-2. Certain illustrative CLEs, as well as polynucleotides, and nucleic acid sequences encoding the same, and methods that include any of these, include intracellular domains from genes having matched domains (e.g. transmembrane gene and first intracellular gene and optionally second intracellular gene) on constructs provided in Tables 7 to 11, as well as, in non-limiting examples, the specific matched domains provided on these tables, some of which are set out in the Exemplary Embodiments section herein. In illustrative embodiments, the intracellular domains from CLEs having matched domains (e.g. transmembrane gene and first intracellular gene and optionally second intracellular gene) on constructs that had particularly noteworthy enrichments in a first screen and a repeated screen with the same P1-P2, P3 and P4 domains, e.g. Libraries 1A and 1.1A, can be used, as shown in Tables 7 to 11. For Libraries 1A and 1.1A, the constructs M001-S116-S044, M024-S192-S045, M001-5047-S 102, M048-S195-S043, M012-S216-S211, and M030-S170-S194 had particularly noteworthy enrichments in both screens.

Further information about the first and second intracellular domains in constructs with particularly noteworthy enrichments in screens of both Library 1A and Library 1.1A is provided in Table 19, including the gene(s) the first and second intracellular domain are derived from, whether the first and/or second intracellular domain are cytokine receptors, and whether the first and/or second intracellular domain have at least one ITAM motif. As shown in Example 17, constructs with intracellular domains from IL6R, MYD88, CD27, MYD88, TNFRSF18, or IL31RA, when present at the first intracellular domain (P3), and constructs with intracellular domains from CD8B, IL1RL1, CD8A, TNFRSF4, or MYD88, when present at the second intracellular domain (P4), showed particularly noteworthy enrichments in both Libraries 1A and 1.1A (Table 19). Constructs with domains from the cytokine receptors IL6R, CD27, TNFRSF18, or IL31RA, when present at the first intracellular domain (P3), and constructs with domains from the cytokine receptors IL1RL1 or TNFRSF4, when present at the second intracellular domain (P4), showed particularly noteworthy enrichments in both Libraries 1A and 1.1A (Table 19).

For Libraries 2B and 2.1B, the constructs M007-S049-S051, M007-S050-S039, M012-S050-S043, M012-S161-S213, M030-S142-S049, M001-S145-S130, M018-5085-5039, M018-5075-5053, M012-S135-S074, and M007-S214-S077 had particularly noteworthy enrichments in both screens. For the purposes of the repeated screens, constructs with particularly noteworthy enrichments were those that had a log₂((normalized count data on the last day + 1)/(normalized count data on day 7 + 1)) value above 2.

Further information about the first and second intracellular domains in constructs with particularly noteworthy enrichments in screens of both Library 2B and Library 2.1B is provided in Table 20, including the gene(s) the first and second intracellular domain are derived from, whether the first and/or second intracellular domain are cytokine receptors, and whether the first and/or second intracellular domain have at least one ITAM motif. Constructs with intracellular domains from CD28, CD40, IL22RA1, IL13RA2, IL17RB, IFNGR2, FCGR2C, IL11RA, or TNFRSF14, when present at the first intracellular domain (P3), and constructs with intracellular domains from CD40, CD3G, CD8A, TNFRSF9, CD28, IL10RB, CD79B, FCER1G, or GHR, when present at the second intracellular domain (P4), showed particularly noteworthy enrichments in both Libraries 2B and 2.1B (Table 20). Constructs with domains from the cytokine receptors CD40, IL22RA1, IL13RA2, IL17RB, IFNGR2, FCGR2C, IL11RA, or TNFRSF14, when present at the first intracellular domain (P3), and constructs with domains from the cytokine receptors TNFRSF9, IL10RB, FCER1G, GHR, or CD40, when present at the second intracellular domain (P4), showed particularly noteworthy enrichments in both Libraries 2B and 2.1B (Table 20). Constructs with domains containing ITAM motifs from FCGR2C, when present at the first intracellular domain (P3), and constructs with domains containing ITAM motifs from CD3G, CD79B, or FCER1G, when present at the second intracellular domain (P4), showed particularly noteworthy enrichments in both Libraries 2B and 2.1B (Table 20).

In an initial interim analysis, for Library 3A, which included constructs that encoded the chimeric polypeptides and a CAR, 126 top candidate chimeric polypeptides were identified (See Table 12) that promoted PBMC proliferation between day 7 and the last day in transduced PBMCs stimulated with untransduced PBMCs as set out in Example 18, when cultured in the absence of IL-2. For the initial interim analysis of Library 3B, which included constructs that encoded the chimeric polypeptides and a CAR, 127 top candidate chimeric polypeptides were identified (See Table 13) that promoted PBMC proliferation between day 7 and the last day in transduced PBMCs that were not stimulated with untransduced PBMCs as set out in Example 18, when cultured in the absence of IL-2. For Library 4B, which included constructs that encoded the chimeric polypeptides but not a CAR, 154 top candidate chimeric polypeptides were identified (See Table 16) that promoted PBMC proliferation between day 7 and the last day in transduced PBMCs that were not stimulated with untransduced PBMCs as set out in Example 18, when cultured in the absence of IL-2.

After further decoding, which provided deep decoding, in Libraries 3A and 3B, which included constructs that encoded the chimeric polypeptides and a CAR and transduced PBMCs that were supplemented with (Library 3A) or without (Library 3B) fresh untransduced PBMCs, 134 top candidates were identified for Library 3A at day 21, 124 top candidates were identified for Library 3A at day 35, and 131 top candidates were identified for Library 3B at day 21 (See Tables 12 and 13, respectively) that promoted PBMC proliferation between day 7 and the last day when cultured the absence of IL-2 (i.e. IL-2 was not added to the culture medium during culturing after the initial transduction), IL-7, or any other exogenous cytokine.

Certain illustrative CLEs, as well as polynucleotides, and nucleic acid sequences encoding the same, and methods that include any of these, include intracellular domains from genes having matched domains (e.g. transmembrane gene and first intracellular gene and optionally second intracellular gene) on constructs provided in Tables 12 to 17, or mutants thereof that retain signaling activity, as well as, in non-limiting examples, the specific matched domains provided on these tables, some of which are set out in the Exemplary Embodiments section herein. Intracellular domains that are identified in the data provided in Example 17 and Example 18, from either the first intracellular domain or the second domain position, are envisioned in exemplary CLE embodiments, interchangeably as either the first or second intracellular domain. In illustrative embodiments, the intracellular domains from genes having matched domains (e.g. transmembrane gene and first intracellular gene and optionally second intracellular gene) on constructs that had particularly noteworthy enrichments in a first screen and a repeated screen with the same P1, P2, P3, and P4 domains, e.g. Libraries 3A and 3.1A, can be used. For Libraries 3A and 3.1A, the constructs E008/E013-T041-S186-S050, E006/E011-T077-S186-5211, E007/E012-T021-S186-S051, E009/E014-T041-S186-S053, E007/E012-T073-S186-S053, E006/E011-T017-S186-S051, E006/E011-T031-S186-S211, E006/E011-T011-S186-S050, E006/E011-T011-S186-S047, E007/E012-T001-S 186-S050, E006/E011-T041-S186-S051, E008/E013-T028-S186-S076, E009/E014-T029-S199-S053, E009/E014-T062-S186-S216, E007/E012-T006-S058-S051, E009/E014-T076-S186-S211, and E007/E012-T001-S186-S047 had particularly noteworthy enrichments in both screens, where the P1 parts separated by a slash here included different tags for Libraries 3A and 3.1A as shown in Tables 6, 12, and 14. For the purposes of the repeated screens, constructs with particularly noteworthy enrichments were those that had a log₂((normalized count data on the last day + 1)/(normalized count data on day 7 + 1)) value above 2.

Further information about the first and second intracellular domains in constructs with particularly noteworthy enrichments in screens of both Library 3A and Library 3.1A is provided in Table 21, including the gene(s) the first and second intracellular domain are derived from, whether the first and/or second intracellular domain are cytokine receptors, and whether the first and/or second intracellular domain have at least one ITAM motif. Constructs with intracellular domains from MPL, OSMR, or CSF2RA, when present at the first intracellular domain (P3), and constructs with intracellular domains from CD40, TNFRSF4, CD79B, CD27, FCGR2A, or TNFRSF18, when present at the second intracellular domain (P4), showed particularly noteworthy enrichments in both Libraries 3A and 3.1A (Table 21). Constructs with domains from the cytokine receptors MPL, OSMR, or CSF2RA, when present at the first intracellular domain (P3), and constructs with domains from the cytokine receptors CD40, TNFRSF4, CD27, FCGR2A, or TNFRSF18, when present at the second intracellular domain (P4), showed particularly noteworthy enrichments in both Libraries 3A and 3.1A (Table 21). Constructs with domains containing ITAM motifs from MPL or OSMR, when present at the second intracellular domain (P4), showed particularly noteworthy enrichments in both Libraries 3A and 3.1A (Table 21).

For Libraries 3B and 3.1B, the constructs E007/E012-T017-S186-S051, E007/E012-T073-S 186-S053, E008/E013-T028-S186-S047, E006/E011-T011-S186-S047, E007/E012-T082-S176-S214, E006/E011-T046-S186-S052, E008/E013-T029-S186-S052, E009/E014-T011-S186-S053, E008/E013-T032-S186-S039, E007/E012-T034-S186-S051, E007/E012-T041-S192-S213, E006/E011-T014-S069-S213, E006/E011-T022-S 186-S053, E006/E011-T023-S115-S075, E006/E011-T029-S106-S213, E006/E011-T032-S155-S080, E006/E011-T041-S186-S216, E006/E011-T057-S135-S080, E006/E011-T072-S191-X002, E006/E011-T077-S186-S216, E006/E011-T080-S141-S080, E007/E012-T001-X001-S214, E007/E012-T007-5059-5211, E007/E012-T016-S186-S052, E007/E012-T031-S186-S053, E007/E012-T044-S102-S052, E007/E012-T044-S142-X002, E007/E012-T055-S069-S053, E007/E012-T063-S176-S216, E007/E012-T065-S157-S075, E008/E013-T008-5085-X002, E008/E013-T011-S085-S048, E008/E013- T021-S109-X002, E008/E013-T021-S168-S211, E008/E013-T032-S064-S214, E008/E013-T037-S170-S215, E008/E013-T038-S176-S048, E008/E013-T039-S137-S216, E008/E013-T041-S141-S053, E008/E013-T045-S177-S048, E008/E013-T048-S109-S074, E008/E013-T073-S199-S075, E009/E014-T001-S157-S074, E009/E014-T005-S196-S049, E009/E014-T011-S130-X002, E009/E014-T013-S155-X002, E009/E014-T017-S186-S076, E009/E014-T021-S142-S080, E009/E014-T023-S082-S076, E009/E014-T038-S196-S037, E009/E014-T055-S186-S052, E009/E014-T060-S175-S053, E009/E014-T070-S085-S212, E008/E013-T026-S054-S213, E009/E014-T007-S120-S053, E007/E012-T045-S186-S211, E008/E013-T073-S186-X002, E008/E013-T074-S186-X002, E007/E012-T055-S186-S053, E008/E013-T036-S186-S053, E007/E012-T017-S058-S053, E008/E013-T030-S189-S080, E006/E011-T029-S081-S047, E009/E014-T044-S194-S050, E006/E011-T028-S121-X002, E008/E013-T028-S186-S053, E009/E014-T078-S142-S213, E009/E014-T041-S186-S051, E008/E013-T006-S186-S050, E006/E011-T028-S186-S075, E006/E011-T040-S120-S038, E007/E012-T044-S115-S211, E009/E014-T039-S176-S075, E007/E012-T028-S186-S050, E008/E013-T031-S202-S050, E007/E012-T072-S192-S053, E006/E011-T065-X001-S051, E007/E012-T030-S062-X002, E007/E012-T073-S186-X002, E009/E014-T056-S186-S053, E008/E013-T046-S137-X002, E006/E011-T016-S136-S076, E007/E012-T032-S142-S037, E007/E012-T065-S120-S215, E009/E014-T077-S186-S047, E009/E014-T001-S126-S051,E006/E011-T030-S121-S039, E008/E013-T006-S176-S213, E009/E014-T032-S130-S215, E008/E013-T041-S186-S039, E009/E014-T021-S186-S047, E008/E013-T026-S137-S214, E007/E012-T029-S116-S075, E008/E013-T026-S106-S049, and E007/E012-T032-S168-S075 had particularly noteworthy enrichments in both screens, where the P1 parts separated by a slash here included different tags for Libraries 3B and 3.1B as shown in Tables 6, 13, and 15. For the purposes of the repeated screens, constructs with particularly noteworthy enrichments were those that had a log₂((normalized count data on the last day + 1)/(normalized count data on day 7 + 1)) value above 2.

Further information about the first and second intracellular domains in constructs with particularly noteworthy enrichments in screens of both Library 3B and Library 3.1B is provided in Table 22, including the gene(s) the first and second intracellular domain are derived from, whether the first and/or second intracellular domain are cytokine receptors, and whether the first and/or second intracellular domain have at least one ITAM motif. Constructs with intracellular domains from MPL, LEPR, MYD88, EPOR, IL5RA, IL2RG, IL18RAP, IL11RA, IL13RA1, CSF2RA, IL1RL1, IL13RA2, IL20RB, IFNGR2, IL3RA, IL27RA, CSF3R, IL31RA, IL12RB1, OSMR, IL10RB, IFNAR2, CRLF2, IL7R, IFNAR1, PRLR, IL9R, IL6R, or IL15RA, when present at the first intracellular domain (P3), and constructs with intracellular domains from CD40, CD79B, CD27, TNFRSF14, CD79A, CD3G, TNFRSF9, FCGR2C, ICOS, TNFRSF18, TNFRSF4, CD28, FCER1G, FCGR2A, CD3D, TNFRSF8, or CD3E, when present at the second intracellular domain (P4), showed particularly noteworthy enrichments in both Libraries 3B and 3.1B (Table 22). Constructs with domains from the cytokine receptors MPL, LEPR, EPOR, IL5RA, IL2RG, IL18RAP, IL11RA, IL13RA1, CSF2RA, IL1RL1, IL13RA2, IL20RB, IFNGR2, IL3RA, IL27RA, CSF3R, IL31RA, IL12RB1, OSMR, IL10RB, IFNAR2, CRLF2, IL7R, IFNAR1, PRLR, IL9R, IL6R, or IL15RA, when present at the first intracellular domain (P3), and constructs with domains from the cytokine receptors CD40, CD27, TNFRSF14, TNFRSF9, FCGR2C, TNFRSF18, TNFRSF4, FCER1G, FCGR2A, or TNFRSF8, when present at the second intracellular domain (P4), showed particularly noteworthy enrichments in both Libraries 3B and 3.1B (Table 22). Constructs with domains containing ITAM motifs from CD79B, CD79A, CD3G, FCGR2C, FCER1G, FCGR2A, CD3D, or CD3E, when present at the second intracellular domain (P4), showed particularly noteworthy enrichments in both Libraries 3B and 3.1B (Table 22).

For Libraries 4B and 4.1B, the constructs E007/E012-T078-S154-S047, E008/E013-T062-S186-X002, E008/E013-T055-S186-S050, E009/E014-T057-S186-S050, E007/E012-T077-S054-S053, E007/E012-T034-S135-S211, E009/E014-T071-X001-5216, E009/E014-T011-S141-S037, E008/E013-T041-S186-S037, E006/E011-T038-S106-S039, E006/E011-T011-S121-X002, E007/E012-T007-5085-S215, E006/E011-T041-S186-S050, E007/E012-T008-S064-S051, E006/E011-T041-S186-S047, E008/E013-T045-S186-S051, E008/E013-T003-S104-S216, E006/E011-T019-S186-S053, E008/E013-T071-S064-S080, E006/E011-T021-S054-X002, E006/E011-T003-S135-X002, E009/E014-T020-S199-S213, E008/E013-T027-S121-S211, E009/E014-T032-S195-X002, E009/E014-T050-S171-X002, E008/E013-T069-S083-X002, E008/E013-T026-S116-S038, E009/E014-T072-S195-X002, E007/E012-T047-S058-S211, E008/E013-T046-S142-S080, E006/E011-T065-S186-S076, E006/E011-T062-S069-X002, E007/E012-T047-5098-X002, E009/E014-T069-S099-S048, E008/E013-T039-S141-S050, E006/E011-T052-S130-S052, E008/E013-T041-S186-X002, E007/E012-T019-S120-X002, E008/E013-T045-S186-S053, E006/E011-T003-S170-5039, E007/E012-T047-S058-S051, E007/E012-T069-S109-X002, E009/E014-T019-S130-S075, and E006/E011-T047-S054-S053 had particularly noteworthy enrichments in both screens, where the P1 parts separated by a slash here included different tags for Libraries 4B and 4.1B as shown in Tables 6, 16, and 17. For the purposes of the repeated screens, constructs with particularly noteworthy enrichments were those that had a log₂((normalized count data on the last day + 1)/(normalized count data on day 7 + 1)) value above 2.Further information about the first and second intracellular domains in constructs with particularly noteworthy enrichments in screens of both Library 4B and Library 4.1B is provided in Table 23, including the gene(s) the first and second intracellular domain are derived from, whether the first and/or second intracellular domain are cytokine receptors, and whether the first and/or second intracellular domain have at least one ITAM motif. Constructs with intracellular domains from IL18R1, MPL, CRLF2, IL11RA, IL13RA1, IL2RG, IL7R, IFNGR2, CSF3R, IL2RA, OSMR, MYD88, IL31RA, IFNAR2, IL6R, CSF2RA, IL13RA2, EPOR, IL1R1, IL10RB, or IL3RA, when present at the first intracellular domain (P3), and constructs with intracellular domains from CD27, CD40, CD79B, TNFRSF4, TNFRSF18, CD3D, CD3G, CD27, ICOS, TNFRSF9, CD3E, FCGR2A, CD28, CD79A, or FCGR2C, when present at the second intracellular domain (P4), showed particularly noteworthy enrichments in both Libraries 4B and 4.1B (Table 23). Constructs with domains from the cytokine receptors IL18R1, MPL, CRLF2, IL11RA, IL13RA1, IL2RG, IL7R, IFNGR2, CSF3R, IL2RA, OSMR, IL31RA, IFNAR2, IL6R, CSF2RA, IL13RA2, EPOR, IL1R1, IL10RB, or IL3RA, when present at the first intracellular domain (P3), and constructs with domains from the cytokine receptors CD27, CD40, TNFRSF4, TNFRSF18, TNFRSF9, FCGR2A, or FCGR2C, when present at the second intracellular domain (P4), showed particularly noteworthy enrichments in both Libraries 4B and 4.1B (Table 23). Constructs with domains containing ITAM motifs from CD79B, CD3D, CD3G, CD3E, FCGR2A, CD79A, or FCGR2C, when present at the second intracellular domain (P4), showed particularly noteworthy enrichments in both Libraries 4B and 4.1B (Table 23).

In some illustrative embodiments, the CLE comprises an intracellular domain from a cytokine receptor in any of the constructs of Tables 19-23. In some illustrative embodiments, the CLE comprises an intracellular domain from any of the constructs of Tables 19-23 with only a single intracellular domain (other P3 or P4 was a linker or stop).

Information about various exemplary CLE domains provided herein, is found in Table 6. For example, the first CLE identified in Table 7 is M008-S212-S075. For this CLE, the extracellular and transmembrane domain (P1-2) is M008, the first intracellular domain (P3) is S212, and the second intracellular domain (P4) is S075. Detailed information about the identity of these domains or modules when considering nucleic acids encoding the same, for example M008, is found in Table 6. Table 6 discloses that M008 is ECDTM-8 and more specifically that this is an interleukin 7 receptor alpha (IL7RA) mutant with a PPCL insert and that the construct includes an eTag.

For Library 1A, intracellular domains from CD3D, CD3E, CD8A, CD27, CD40, CD79B, IFNAR1, IL2RA, IL3RA, IL13RA2, TNFRSF8, and TNFRSF9, or mutants thereof that are known to have signaling activity, when found at the first intracellular domain position (P3) of candidate chimeric polypeptides promoted proliferation of PBMCs between day 7 and the last day, when data was considered in a combined manner for all constructs with first intracellular domains (P3) derived from that gene. Accordingly, exemplary embodiments of CLEs provided herein have intracellular domains from CD3D, CD3E, CD8A, CD27, CD40, CD79B, IFNAR1, IL2RA, IL3RA, IL13RA2, TNFRSF8, and TNFRSF9, including mutants thereof that retain signaling activity, as either the second intracellular domain, or in illustrative embodiments, the first intracellular domain.

For Library 1A, intracellular domains from CD3D, CD3G, CD8A, CD8B, CD27, CD40, CD79B, CRLF2, FCGR2C, ICOS , IL2RA, IL13RA1, IL13RA2, IL15RA, TNFRSF9, and TNFRSF18, or mutants thereof that are known to have signaling activity, when found at the second intracellular domain position (P4) of candidate chimeric polypeptides promoted proliferation of PBMCs between day 7 and the last day, when data was considered in a combined manner for all constructs with a second intracellular domains (P4) derived from that gene. Examples of second intracellular domains or portions and/or mutants thereof, that were present in constructs that promoted cell proliferation are provided in tables in Example 17. Accordingly, exemplary embodiments of CLEs provided herein have intracellular domains from CD3D, CD3G, CD8A, CD8B, CD27, CD40, CD79B, CRLF2, FCGR2C, ICOS, IL2RA, IL13RA1, IL13RA2, IL15RA, TNFRSF9, and TNFRSF18, including mutants thereof that retain signaling activity, as either the first intracellular domain, or in illustrative embodiments, the second intracellular domain.

For Library 3A, first intracellular (P3) domains from CSF2RA, IFNAR1, IL1RAP, IL4R, IL6ST, IL11RA, IL12RB2, IL17RA, IL17RD, IL17RE, IL18R1, IL21R, IL23R, MPL, and MyD88, or mutants thereof that are known to promote signaling activity in certain cell types if such mutants are present in the constructs provided in Example 18, when found at the first intracellular domain (P3) of candidate chimeric polypeptides elements herein, promoted proliferation of PBMCs between day 7 and the last day or between day 7 and day 21, when data is considered in a combined manner for all constructs with a first intracellular domain derived from that gene. This conclusion is based on enrichment of sequence counts of constructs in mixed cultured PBMC cell populations, such that enrichment, calculated as the logarithm in base 2 of the ratio between normalized count at the last day indicated on the table plus one and normalized count at day 7 plus one, was at least 2 for Library 3A, when results for all constructs for a gene are combined. For Library 3B, first intracellular domains from CSF2RB, IL4R, and MPL, or mutants thereof that are known to promote signaling activity in certain cell types if such mutants are present in the constructs provided in Example 18, were the best performers when analyzed in this way. Examples of first intracellular domains or portions and/or mutants thereof that were present in constructs that promoted cell proliferation for Libraries 3A, 3B, 3.1A, and 3.1B are provided in Tables 12-15.

For Library 3A, second intracellular (P4) domains from CD3D, CD3G, CD27, CD40, CD79A, CD79B, FCER1G, FCGRA2, ICOS, TNFRSF4, and TNFRSF8, or mutants thereof that are known to promote signaling activity in certain cell types if such mutants are present in the constructs provided in Example 18, when found at the second intracellular domain (P4) of candidate chimeric polypeptides elements herein, promoted proliferation of PBMCs between day 7 and day 21 or between day 7 and the last day indicated on the table, when data is considered in a combined manner for all constructs with a second intracellular domain derived from that gene. This conclusion is based on enrichment of sequence counts of constructs in mixed cultured PBMC cell populations, such that enrichment, calculated as the logarithm in base 2 of the ratio between normalized count at the last day indicated on the table plus one and normalized count at day 7 plus one, was at least 2 for Library 3A, when results for all constructs for a gene are combined. Examples of second intracellular domains or portions and/or mutants thereof that were present in constructs that promoted cell proliferation for Libraries 3A and 3B are provided in Tables 12 to 17.

After an initial interim decoding analysis, for Library 3A, first intracellular (P3) domains from IL17RD, IL17RE, IL1RAP, IL23R, and MPL, or mutants thereof that are known to promote signaling activity in certain cell types, when found at the first intracellular domain (P3) of candidate chimeric polypeptides elements herein, promoted proliferation of PBMCs between day 7 and day 21, when data is considered in a combined manner for all constructs with a first intracellular domain derived from that gene. For Library 3B, first intracellular domains from IL21R, MPL, and OSMR, or mutants thereof that are known to promote signaling activity in certain cell types, were the best performers when analyzed in this way. Examples of first intracellular domains or portions and/or mutants thereof, that were present in constructs that promoted cell proliferation for Libraries 3A and 3B are provided in tables in Example 18. Accordingly, exemplary embodiments of CLEs provided herein have intracellular domains from IL17RD, IL17RE, IL1RAP, IL23R, and MPL F9, including mutants thereof that retain signaling activity, as either the second intracellular domain, or in illustrative embodiments, the first intracellular domain.

After an initial decoding analysis, for Library 3A, second intracellular (P4) domains from CD27, CD3G, CD40, and CE79B, or mutants thereof that are known to promote signaling activity in certain cell types, when found at the second intracellular domain (P4) of candidate chimeric polypeptides elements herein, promoted proliferation of PBMCs between day 7 and the last day indicated on the table, when data is considered in a combined manner for all constructs with a second intracellular domain derived from that gene. For Library 3B, second intracellular domains from CD40, or mutants thereof that are known to promote signaling activity in certain cell types, were the best performance when analyzed in this way. Accordingly, exemplary embodiments of CLEs provided herein have intracellular domains from CD27, CD3G, CD40, and CE79B, including mutants thereof that retain signaling activity, as either the first intracellular domain, or in illustrative embodiments, the second intracellular domain.

As shown in Tables 12 to 17, first intracellular (P3) domains from MPL, LEPR, MYD88, IFNAR2, or in some cases mutants thereof that retain signaling activity, when found at the first intracellular domain (P3) of candidate chimeric polypeptides herein, promoted proliferation of PBMCs between day 7 and the end of the experiment, when considering the first intracellular domains that occur most frequently in top candidate constructs. Examples of first intracellular domains or portions and/or mutants thereof, that were present in constructs that promoted cell proliferation for Libraries 3A, 3B, 3.1A, 3,1B, 4B, and 4.1B are provided in tables in Example 18. Accordingly, exemplary embodiments of CLEs provided herein have intracellular domains from MPL, LEPR, MYD88, IFNAR2, including mutants thereof that retain signaling activity, as either the second intracellular domain, or in illustrative embodiments, the first intracellular domain.

As shown in Tables 12 to 17, second intracellular (P4) domains from CD40, CD79B, CD27, or in some cases mutants thereof that retain signaling activity, when found at the second intracellular domain (P4) of candidate chimeric polypeptides herein, promoted proliferation of PBMCs between day 7 and the last day indicated on the table, when considering the first intracellular domains that occur most frequently in top candidate constructs. Examples of first intracellular domains or portions and/or mutants thereof, that were present in constructs that promoted cell proliferation for Libraries 3A, 3B, 3.1A, 3,1B, 4B, and 4.1B are provided in tables in Example 18. Accordingly, exemplary embodiments of CLEs provided herein have intracellular domains from CD40, CD79B, CD27, including mutants thereof that retain signaling activity, as either the first intracellular domain, or in illustrative embodiments, the second intracellular domain.

In noteworthy embodiments of any of the isolated polynucleotide or vector aspects and embodiments provided herein that comprise a first nucleic acid sequence that encodes a chimeric polypeptide that promotes cell proliferation of PBMCs, B cells, T cells, and/or NK cells (i.e. a CLE), and a second nucleic acid sequence that encodes a chimeric antigen receptor (CAR) comprising an antigen-specific targeting region (ASTR), a transmembrane domain, and an intracellular activating domain, the first nucleic acid sequence and the second nucleic acid sequence can be separated by a ribosomal skip sequence. In some embodiments, the ribosomal skip sequence is F2A, E2A, P2A, or T2A.

As disclosed herein, lymphoproliferative elements, as exemplified herein with CLEs, in some illustrative embodiments can include a dimerizing motif to help enhance and/or regulate activity thereof, for example to affect signals in a cell, such as signals that affect protein activity or gene expression. Such dimerizing motif is typically a portion of, or the entire extracellular domain. In some embodiments, a dimerizing moiety can be attached to a recognition and clearance sequence in the extracellular domain of a CLE herein. In fact, in some embodiments, a lymphoproliferative element can include an entire lymphoproliferative element from one protein except that the extracellular domain includes a dimerizing moiety.

In some embodiments of lymphoproliferative elements and CLEs that include dimerizing agents, and polynucleotides and nucleic acid sequences encoding the same, the dimerizing motif can include an amino acid sequence from transmembrane homodimeric polypeptides that naturally exist as homodimers. For example, the dimerizing motif can be a leucine zipper polypeptide, for example a Jun polypeptide as exemplified in Example 18 herein. In some embodiments, these transmembrane homodimeric polypeptides can include early activation antigen CD69 (CD69), Transferrin receptor protein 1 (CD71), B-cell differentiation antigen (CD72), T-cell surface protein tactile (CD96), Endoglin (Cd105), Killer cell lectin-like receptor subfamily B member 1 (Cd161), P-selectin glycoprotein ligand 1 (Cd162), Glutamyl aminopeptidase (Cd249), Tumor necrosis factor receptor superfamily member 16 (CD271), Cadherin-1 (E-Cadherin) (Cd324), or active fragments thereof. In some embodiments, the dimerizing motif can include an amino acid sequence from transmembrane proteins that dimerize upon ligand (also referred to herein as a dimerizer or dimerizing agent) binding. In some embodiments, the dimerizing motif and dimerizer can include (where the dimerizer is in parentheses following the dimerizer-binding pair): FKBP and FKBP (rapamycin); GyrB and GyrB (coumermycin); DHFR and DHFR (methotrexate); or DmrB and DmrB (AP20187). As noted above, rapamycin can serve as a dimerizer. Alternatively, a rapamycin derivative or analog can be used (see, e.g., WO96/41865; WO 99/36553; WO 01/14387; and Ye et al (1999) Science 283:88-91). For example, analogs, homologs, derivatives, and other compounds related structurally to rapamycin ("rapalogs") include, among others, variants of rapamycin having one or more of the following modifications relative to rapamycin: demethylation, elimination or replacement of the methoxy at C7, C42 and/or C29; elimination, derivatization or replacement of the hydroxy at C13, C43 and/or C28; reduction, elimination or derivatization of the ketone at C14, C24 and/or C30; replacement of the 6-membered pipecolate ring with a 5-membered prolyl ring; and alternative substitution on the cyclohexyl ring or replacement of the cyclohexyl ring with a substituted cyclopentyl ring. Additional information is presented in, e.g., U.S. Pat. Nos. 5,525,610; 5,310,903 5,362,718; and 5,527,907. Selective epimerization of the C-28 hydroxyl group has been described (see, e.g., WO 01/14387). Additional synthetic dimerizing agents suitable for use as an alternative to rapamycin include those described in U.S. Patent Publication No. 2012/0130076. As noted above, coumermycin can serve as a dimerizing agent. Alternatively, a coumermycin analog can be used (see, e.g., Farrar et al. (1996) Nature 383:178-181; and U.S. Pat. No. 6,916,846). As noted above, in some cases, the dimerizing agent is methotrexate, e.g., a non-cytotoxic, homo-bifunctional methotrexate dimer (see, e.g., U.S. Pat. No. 8,236,925).

In some embodiments, when present in the plasma membrane of a eukaryotic cell, a lymphoproliferative element, an in illustrative embodiments a CLE, including a dimerizing motif can be active in the absence of a dimerizing agent. For example, lymphoproliferative elements that include a leucine zipper-containing dimerization motif, or that include a dimerizing motif from transmembrane homodimeric polypeptides including CD69, CD71, CD72, CD96, Cd105, Cd161, Cd162, Cd249, CD271, Cd324, active mutants thereof, and/or active fragments thereof can be active in the absence a dimerizing agent. In some embodiments, when present in the plasma membrane of a eukaryotic cell, a lymphoproliferative element including a dimerizing motif can be active in the presence of a dimerizing agent. For example, lymphoproliferative elements including a dimerizing motif from FKBP, GyrB, DHFR, or DmrB can be active in the presence of the respective dimerizing agents or analogs thereof, e.g. rapamycin, coumermycin, methotrexate, and AP20187, respectively.

In method embodiments including lymphoproliferative elements, e.g. CLE, that include a dimerizer for dimerizing a polypeptide comprising a dimerizing motif,a dimerizer can be administered to the host (subject) using any convenient means capable of resulting in the desired effect. Thus, the dimerizer can be incorporated into a variety of formulations for administration. More particularly, a dimerizer can be formulated into pharmaceutical compositions by combination with appropriate, pharmaceutically acceptable carriers or diluents, and may be formulated into preparations in solid, semisolid, liquid or gaseous forms, such as tablets, capsules, powders, granules, ointments, solutions, suppositories, injections, inhalants and aerosols.

Those of skill will readily appreciate that dose levels can vary as a function of the specific dimerizer, the severity of the symptoms, and the susceptibility of the subject to side effects. Preferred dosages for a given compound are readily determinable by those of skill in the art by a variety of means.

A dimerizer is administered to an individual using any available method and route suitable for drug delivery, including *in vivo* and *ex vivo* methods, as well as systemic and localized routes of administration.

In any aspects or embodiments wherein the extracellular domain of a CLE comprises a dimerizing motif, the dimerizing motif can be selected from the group consisting of: a leucine zipper motif-containing polypeptide, CD69, CD71, CD72, CD96, Cd105, Cd161, Cd162, Cd249, CD271, and Cd324, as well as mutants and/or active fragments thereof that retain the ability to dimerize. In any of the aspects and embodiments herein wherein the extracellular domain of a CLE comprises a dimerizing motif, the dimerizing motif can require a dimerizing agent, and the dimerizing motif and associated dimerizing agent can be selected from the group consisting of: FKBP and rapamycin or analogs thereof, GyrB and coumermycin or analogs thereof, DHFR and methotrexate or analogs thereof, or DmrB and AP20187 or analogs thereof, as well as mutants and/or active fragments of the recited dimerizing proteins that retain the ability to dimerize.

In illustrative embodiments of any aspects or embodiments herein wherein the extracellular domain of a CLE comprises a dimerizing motif, the extracellular domain can comprises a leucine zipper motif. In some embodiments, the leucine zipper motif is from a jun polypeptide, for example c-jun. In certain embodiments the c-jun polypeptide is the c-jun polypeptide region of ECD-11.

In any of the aspects and embodiments provided herein that include lymphoproliferative element, the lymphoproliferative element can be a polypeptide comprising a membrane targeting region, a dimerizing (or multimerizing) domain, a first intracellular domain, and optionally a second intracellular domain, and further optionally a third intracellular domain, and optionally a fourth intracellular domain, wherein at least one of the first and optional second, third, and fourth intracellular domains are from any gene with an intracellular domain identified in Tables 7 to 17, or selected from any of the first or second intracellular domains identified in Tables 7 to 17, and wherein said internally dimerizing and/or multimerizing lymphoproliferative element promotes cell proliferation of PBMCs, and in illustrative embodiments, of B cells, T cells, and/or NK cells. Such polypeptide can be referred to herein as an internally dimerizing and/or multimerizing lymphoproliferative element. In certain embodiments, linkers can be added between any of the domains. In certain embodiments, the first and second intracellular domains are other than MyD88 and CD40. In some embodiments, the intracellular domain is MPL, or an intracellular fragment thereof that retains the ability to promote PBMC cell proliferation. In some embodiments, the intracellular domain is other than MPL or an intracellular fragment thereof that retains the ability to promote PBMC cell proliferation. In some embodiments, the intracellular domain is other than MyD88 or an intracellular fragment thereof that retains the ability to promote PBMC cell proliferation.

The dimerizing domain of such internally dimerizing and/or multimerizing polypeptide lymphoproliferative elements in illustrative embodiments is located between the membrane targeting region and the first intracellular domain. In certain embodiments the dimerizing (or multimerizing) domain comprises a multimerizing or dimerizing ligand binding sites, such as, for example, an FKBP region, for example FKBP12. In some embodiments, the polypeptide can comprise an extracellular domain, such as any of the extracellular domains provided herein, for example in Tables 7 to 11 of Example 17 or in Tables 12 to 17 of Example 18, with or without the recognition and clearance domain. In certain embodiments, the membrane targeting region is selected from the group consisting of a myristoylation region, palmitoylation region, prenylation region, and transmembrane sequences of receptors. In certain embodiments, the membrane targeting region is a myristoylation region. The internally dimerizing and/or multimerizing lymphoproliferative element is typically tethered to the plasma membrane via the membrane targeting region, for example tethered to the membrane with an N-terminal myristate. In illustrative embodients, the FKBP region binds FK506.

Internally dimerizing and/or multimerizing lymphoproliferative elements in one embodiment are an integral part of a system that uses a dimeric analog of the lipid permeable immunosuppressant drug, FK506, which loses its normal bioactivity while gaining the ability to crosslink molecules genetically fused to the FK506-binding protein, FKBP12. By fusing one or more FKBPs and a myristoylation sequence to the cytoplasmic signaling domain of a target receptor, one can stimulate signaling in a dimerizer drug-dependent, but ligand and ectodomain-independent manner. This provides the system with temporal control, reversibility using monomeric drug analogs, and enhanced specificity. The high affinity of third- generation AP20187/AP1903 dimerizer drugs for their binding domain, FKBP12 permits specific activation of the recombinant receptor in vivo without the induction of non-specific side effects through endogenous FKBP12. FKBP12 variants having amino acid substitutions and deletions, such as FKBP12V36, that bind to a dimerizer drug, may also be used. In addition, the synthetic ligands are resistant to protease degradation, making them more efficient at activating receptors in vivo than most delivered protein agents.

### PSEUDOTYPING ELEMENTS

Many of the methods and compositions provided herein include pseudotyping elements. The pseudotyping of replication incompetent recombinant retroviral particles with heterologous envelope glycoproteins typically alters the tropism of a virus and facilitates the transduction of host cells. A pseudotyping element as used herein can include a "binding polypeptide" that includes one or more polypeptides, typically glycoproteins, that identify and bind the target host cell, and one or more "fusogenic polypeptides" that mediate fusion of the retroviral and target host cell membranes, thereby allowing a retroviral genome to enter the target host cell. In some embodiments provided herein, pseudotyping elements are provided as polypeptide(s)/protein(s), or as nucleic acid sequences encoding the polypeptide(s)/protein(s).

In some embodiments, the pseudotyping element is the feline endogenous virus (RD114) envelope protein, the oncoretroviral amphotropic envelope protein, the oncoretroviral ecotropic envelope protein, the vesicular stomatitis virus envelope protein (VSV-G), and/or the paramyxovirus Measles envelope proteins H and F.

In some embodiments, the pseudotyping elements include a binding polypeptide and a fusogenic polypeptide derived from different proteins. For example, the replication incompetent recombinant retroviral particles of the methods and compositions disclosed herein can be pseudotyped with the fusion (F) and hemagglutinin (H) polypeptides of the measles virus (MV), as non-limiting examples, clinical wildtype strains of MV, and vacccine strains including the Edmonston strain (MV-Edm) or fragments thereof. Not to be limited by theory, both hemagglutinin (H) and fusion (F) polypeptides are believed to play a role in entry into host cells wherein the H protein binds MV to receptors CD46, SLAM, and Nectin-4 on target cells and F mediates fusion of the retroviral and host cell membranes. In an illustrative embodiment, especially where the target cell is a T cell and/or NK cell, the binding polypeptide is a Measles Virus H polypeptide and the fusogenic polypeptide is a Measles Virus F polypeptide.

In some studies, lentiviral particles pseudotyped with truncated F and H polypeptides had a significant increase in titers and transduction efficiency (Funke et al. 2008. Molecular Therapy. 16(8):1427-1436), (Frecha et al. 2008. Blood. 112(13):4843-4852). The highest titers were obtained when the F cytoplasmic tail was truncated by 30 residues (referred to as MV(Ed)-FΔ30 (SEQ ID NO:105)). For the H variants, optimal truncation occurred when 18 or 19 residues were deleted (MV(Ed)-HΔ18 (SEQ ID NO:106) or MV(Ed)-HΔ19), although variants with a truncation of 24 residues with and without replacement of deleted residues with alanine (MV(Ed)-HΔ24 (SEQ ID NO:235) and MV(Ed)-HΔ24+A) also resulted in optimal titers.

In some embodiments, including those directed to transducing T cells and/or NK cells, the replication incompetent recombinant retroviral particles of the methods and compositions disclosed herein are pseudotyped with mutated or variant versions of the measles virus fusion (F) and hemagglutinin (H) polypeptides, in illustrative examples, cytoplasmic domain deletion variants of measles virus F and H polypeptides. In some embodiments, the mutated F and H polypeptides are "truncated H" or "truncated F" polypeptides, whose cytoplasmic portion has been truncated, i.e. amino acid residues (or coding nucleic acids of the corresponding nucleic acid molecule encoding the protein) have been deleted. "HΔY" and "FΔX" designate such truncated H and F polypeptide, respectively, wherein "Y" refers to 1-34 residues that have been deleted from the amino termini and "X" refers to 1-35 residues that have been deleted from the carboxy termini of the cytoplasmic domains. In a further embodiment, the "truncated F polypeptide" is FΔ24 or FΔ30 and/or the "truncated H protein" is selected from the group consisting of HΔ14, HΔ15, HΔ16, HΔ17, HΔ18, HΔ19, HΔ20, HΔ21+A, HΔ24 and HΔ24+4A, more preferably HΔ18 or HΔ24. In an illustrative embodiment, the truncated F polypeptide is MV(Ed)-FΔ30 and the truncated H polypeptide is MV(Ed)-HΔ18.

In some embodiments, the fusogenic polypeptide includes multiple elements expressed as one polypeptide. In some embodiments, the binding polypeptide and fusogenic polypeptide are translated from the same transcript but from separate ribosome binding sites; in other embodiments, the binding polypeptide and fusogenic polypeptide are separated by a cleavage peptide site, which not to be bound by theory, is cleaved after translation, as is common in the literature, or a ribosomal skip sequence. In some embodiments, the translation of the binding polypeptide and fusogenic polypeptide from separate ribosome binding sites results in a higher amount of the fusogenic polypeptide as compared to the binding polypeptide. In some embodiments, the ratio of the fusogenic polypeptide to the binding polypeptide is at least 2:1, at least 3:1, at least 4:1, at least 5:1, at least 6:1, at least 7:1, or at least 8:1. In some embodiments, the ratio of the fusogenic polypeptide to the binding polypeptide is between 1.5:1, 2:1, or 3:1, on the low end of the range, and 3:1, 4:1, 5:1, 6:1, 7:1, 8:1. 9:1 or 10: 1 on the high end of the range.

### ACTIVATION ELEMENTS

Many of the methods and composition aspects of the present disclosure include an activation element, also referred to herein as a T cell activation element, or a nucleic acid encoding an activation element. The restrictions associated with lentiviral (LV) transduction into resting T cells are attributed to a series of pre-entry and post-entry barriers as well as cellular restrictive factors (Strebel et al 2009. BMC Medicine 7:48). One restriction is the inability for the envelope pseudotyped-LV particles to recognize potential receptors and mediate fusion with the cellular membrane. However, under certain conditions, the transduction of resting T cells with HIV-1-based lentiviral vectors is possible mostly upon T cell receptor (TCR) CD3 complex and CD28 co-stimulation (Korin & Zack. 1998. Journal of Virology. 72:3161-8, Maurice et al. 2002. Blood 99:2342-50), as well as through exposure to cytokines (Cavalieri et al 2003).

Cells of the immune system such as T lymphocytes recognize and interact with specific antigens through receptors or receptor complexes which, upon recognition or an interaction with such antigens, cause activation of the cell and expansion in the body. An example of such a receptor is the antigen-specific T lymphocyte receptor complex (TCR/CD3). The T cell receptor (TCR) is expressed on the surface of T lymphocytes. One component, CD3, is responsible for intracellular signaling following occupancy of the TCR by ligand. The T lymphocyte receptor for antigen-CD3 complex (TCR/CD3) recognizes antigenic peptides that are presented to it by the proteins of the major histocompatibility complex (MHC). Complexes of MHC and peptide are expressed on the surface of antigen presenting cells and other T lymphocyte targets. Stimulation of the TCR/CD3 complex results in activation of the T lymphocyte and a consequent antigen-specific immune response. The TCR/CD3 complex plays a central role in the effector function and regulation of the immune system. Thus, activation elements provided herein, activate T cells by binding to one or more components of the T cell receptor associated complex, for example by binding to CD3. In some cases, the activation element can activate alone. In other cases, the activation requires activation through the TCR receptor complex in order to further activate cells.

T lymphocytes also require a second, co-stimulatory signal to become fully active. Without such a signal, T lymphocytes are either non-responsive to antigen binding to the TCR, or become anergic. Such a co-stimulatory signal, for example, is provided by CD28, a T lymphocyte protein, which interacts with CD80 and CD86 on antigen-producing cells. As used herein, a functional extracellular fragment of CD80 retains its ability to interact with CD28. ICOS (Inducible COStimulator), another T lymphocyte protein, provides a co-stimulatory signal when bound to ICOS ligand.

Activation of the T cell receptor (TCR) CD3 complex and co-stimulation with CD28 can occur by *ex vivo* exposure to solid surfaces (e.g. beads) coated with anti-CD3 and anti-CD28. In some embodiments of the methods and compositions disclosed herein, resting T cells are activated by exposure to solid surfaces coated with anti-CD3 and anti-CD28 *ex vivo.* In other embodiments, resting T cells or NK cells, illustrative embodiments T cells, are activated by exposure to soluble anti-CD3 antibodies (e.g. at 50-150, or 75-125, or 100 ng/ml). In such embodiments, which can be part of methods for genetically modifying or transducing, in illustrative embodiments without prior activation, such activation and/or contacting can be carried out, for example, for 8 hours or less, 4 hours or less or between 2 and 8 hours, 2 and 4 hours, or between 2 and 3.

In certain illustrative embodiments of the methods and compositions provided herein, polypeptides that are capable of binding CD3 and/or CD28, are presented as "activation elements" on the surface of replication incompetent recombinant retroviral particles of the methods and compositions disclosed herein.. Polypeptides that bind CD3 and/or CD28 are referred to as "activation elements" because of their ability to activate resting T cells.

In some embodiments, the activation element is a polypeptide capable of binding to CD3. In some embodiments, the polypeptide capable of binding to CD3 is an anti-CD3 antibody, or a fragment thereof that retains the ability to bind to CD3. In illustrative embodiments, the anti-CD3 antibody or fragment thereof is a single chain anti-CD3 antibody, such as but not limited to, an anti-CD3 scFv. In another illustrative embodiment, the polypeptide capable of binding to CD3 is anti-CD3scFvFc.

A number of anti-human CD3 monoclonal antibodies and antibody fragments thereof are available, and can be used in the present invention, including but not limited to UCHT1, OKT-3, HIT3A, TRX4, X35-3, VIT3, BMA030 (BW264/56), CLB-T3/3, CRIS7, YTH12.5, F111409, CLB-T3.4.2, TR-66, WT31, WT32, SPv-T3b, 11D8, XIII-141, XIII46, XIII-87, 12F6, T3/RW2-8C8, T3/RW24B6, OKT3D, M-T301, SMC2 and F101.01.

In some embodiments, the activation element is a polypeptide capable of binding to CD28. In some embodiments, the polypeptide capable of binding to CD28 is an anti-CD28 antibody, or a fragment thereof that retains the ability to bind to CD28. In other embodiments, the polypeptide capable of binding to CD28 is CD80, CD86, or a functional fragment thereof that is capable of binding CD28 and inducing CD28-mediated activation of Akt, such as an external fragment of CD80. In some aspects herein, an external fragment of CD80 means a fragment that is typically present on the outside of a cell in the normal cellular location of CD80, that retains the ability to bind to CD28. In illustrative embodiments, the anti-CD28 antibody or fragment thereof is a single chain anti-CD28 antibody, such as, but not limited to, an anti-CD28 scFv. In another illustrative embodiment, the polypeptide capable of binding to CD28 is CD80, or a fragment of CD80 such as an external fragment of CD80.

Anti-CD28 antibodies are known in the art and can include, as non-limiting examples, monoclonal antibody 9.3, an IgG2a antibody (Dr. Jeffery Ledbetter, Bristol Myers Squibb Corporation, Seattle, Wash.), monoclonal antibody KOLT-2, an IgG1 antibody, 15E8, an IgG1 antibody, 248.23.2, an IgM antibody and EX5.3D10, an IgG2a antibody.

In an illustrative embodiment, an activation element includes two polypeptides, a polypeptide capable of binding to CD3 and a polypeptide capable of binding to CD28.

In certain embodiments, the polypeptide capable of binding to CD3 or CD28 is an antibody, a single chain monoclonal antibody or an antibody fragment, for example a single chain antibody fragment. Accordingly, the antibody fragment can be, for example, a single chain fragment variable region (scFv), an antibody binding (Fab) fragment of an antibody, a single chain antigen-binding fragment (scFab), a single chain antigen-binding fragment without cysteines (scFabΔC), a fragment variable region (Fv), a construct specific to adjacent epitopes of an antigen (CRAb), or a single domain antibody (VH or VL).

In any of the embodiments disclosed herein, an activation element, or a nucleic acid encoding the same, can include a dimerizing or higher order multimerizing motif. Dimerizing and multimerizing motifs are well-known in the art and a skilled artisan will understand how to incorporate them into the polypeptides for effective dimerization or multimerization. For example, in some embodiments, the activation element that includes a dimerizing motif can be one or more polypeptides capable of binding to CD3 and/or CD28. In some embodiments, the polypeptide capable of binding to CD3 is an anti-CD3 antibody, or a fragment thereof that retains the ability to bind to CD3. In illustrative embodiments, the anti-CD3 antibody or fragment thereof is a single chain anti-CD3 antibody, such as but not limited to, an anti-CD3 scFv. In another illustrative embodiment, the polypeptide capable of binding to CD3 is anti-CD3scFvFc, which in some embodiments is considered an anti-CD3 with a dimerizing motif without any additional dimerizing motif, since anti-CD3scFvFc constructs are known to be capable of dimerizing without the need for a separate dimerizing motif.

In some embodiments, the dimerizing or multimerizing motif, or a nucleic acid sequence encoding the same, can be an amino acid sequence from transmembrane polypeptides that naturally exist as homodimers or multimers. In some embodiments, the dimerizing or multimerizing motif, or a nucleic acid sequence encoding the same, can be an amino acid sequence from a fragment of a natural protein or an engineered protein. In one embodiment, the homodimeric polypeptide is a leucine zipper motif-containing polypeptide (leucine zipper polypeptide). For example, a leucine zipper polypeptide derived from c-JUN, non-limiting examples of which are disclosed related to chimeric lymphoproliferative elements (CLEs) herein.

In some embodiments, these transmembrane homodimeric polypeptides can include early activation antigen CD69 (CD69), Transferrin receptor protein 1 (CD71), B-cell differentiation antigen (CD72), T-cell surface protein tactile (CD96), Endoglin (Cd105), Killer cell lectin-like receptor subfamily B member 1 (Cd161), P-selectin glycoprotein ligand 1 (Cd162), Glutamyl aminopeptidase (Cd249), Tumor necrosis factor receptor superfamily member 16 (CD271), Cadherin-1 (E-Cadherin) (Cd324), or active fragments thereof. In some embodiments, the dimerizing motif, and nucleic acid encoding the same, can include an amino acid sequence from transmembrane proteins that dimerize upon ligand (also referred to herein as a dimerizer or dimerizing agent) binding. In some embodiments, the dimerizing motif and dimerizer can include (where the dimerizer is in parentheses following the dimerizer-binding pair): FKBP and FKBP (rapamycin); GyrB and GyrB (coumermycin); DHFR and DHFR (methotrexate); or DmrB and DmrB (AP20187). As noted above, rapamycin can serve as a dimerizer. Alternatively, a rapamycin derivative or analog can be used (see, e.g., WO96/41865; WO 99/36553; WO 01/14387; and Ye et al (1999) Science 283:88-91). For example, analogs, homologs, derivatives, and other compounds related structurally to rapamycin ("rapalogs") include, among others, variants of rapamycin having one or more of the following modifications relative to rapamycin: demethylation, elimination or replacement of the methoxy at C7, C42 and/or C29; elimination, derivatization or replacement of the hydroxy at C13, C43 and/or C28; reduction, elimination or derivatization of the ketone at C14, C24 and/or C30; replacement of the 6-membered pipecolate ring with a 5-membered prolyl ring; and alternative substitution on the cyclohexyl ring or replacement of the cyclohexyl ring with a substituted cyclopentyl ring. Additional information is presented in, e.g., U.S. Pat. Nos. 5,525,610; 5,310,903 5,362,718; and 5,527,907. Selective epimerization of the C-28 hydroxyl group has been described (see, e.g., WO 01/14387). Additional synthetic dimerizing agents suitable for use as an alternative to rapamycin include those described in U.S. Patent Publication No. 2012/0130076. As noted above, coumermycin can serve as a dimerizing agent. Alternatively, a coumermycin analog can be used (see, e.g., Farrar et al. (1996) Nature 383:178-181; and U.S. Pat. No. 6,916,846). As noted above, in some cases, the dimerizing agent is methotrexate, e.g., a non-cytotoxic, homo-bifunctional methotrexate dimer (see, e.g., U.S. Pat. No. 8,236,925).

In some embodiments, when present on the surface of replication incompetent recombinant retroviral particles, an activation element including a dimerizing motif can be active in the absence of a dimerizing agent. For example, activation elements including a dimerizing motif from transmembrane homodimeric polypeptides including CD69, CD71, CD72, CD96, Cd105, Cd161, Cd162, Cd249, CD271, Cd324, active mutants thereof, and/or active fragments thereof can be active in the absence a dimerizing agent. In some embodiments, the activation element can be an anti-CD3 single chain fragment and include a dimerizing motif selected from the group consisting of CD69, CD71, CD72, CD96, Cd105, Cd161, Cd162, Cd249, CD271, Cd324, active mutants thereof, and/or active fragments thereof.

In some embodiments, when present on the surface of replication incompetent recombinant retroviral particles, an activation element including a dimerizing motif can be active in the presence of a dimerizing agent. For example, activation elements including a dimerizing motif from FKBP, GyrB, DHFR, or DmrB can be active in the presence of the respective dimerizing agents or analogs thereof, e.g. rapamycin, coumermycin, methotrexate, and AP20187, respectively. In some embodiments, the activation element can be a single chain antibody fragment against anti-CD3 or anti-CD28, or another molecule that binds CD3 or CD28, and the dimerizing motif and dimerizing agent can be selected from the group consisting of FKBP and rapamycin or analogs thereof, GyrB and coumermycin or analogs thereof, DHFR and methotrexate or analogs thereof, or DmrB and AP20187 or analogs thereof.

In some embodiments, an activation element is fused to a heterologous signal sequence and/or a heterologous membrane attachment sequence, both of which help direct the activation element to the membrane. The heterologous signal sequence targets the activation element to the endoplasmic reticulum, where the heterologous membrane attachment sequence covalently attaches to one or several fatty acids (also known as posttranslational lipid modification) such that the activation elements that are fused to the heterologous membrane attachment sequence are anchored in the lipid rafts of the plasma membrane. In some embodiments, posttranslational lipid modification can occur via myristoylation, palmitoylation, or GPI anchorage. Myristoylation is a post-translational protein modification which corresponds to the covalent linkage of a 14-carbon saturated fatty acid, the myristic acid, to the N-terminal glycine of a eukaryotic or viral protein. Palmitoylation is a post-translational protein modification which corresponds to the covalent linkage of a C16 acyl chain to cysteines, and less frequently to serine and threonine residues, of proteins. GPI anchorage refers to the attachment of glycosylphosphatidylinositol, or GPI, to the C-terminus of a protein during posttranslational modification.

In some embodiments, the heterologous membrane attachment sequence is a GPI anchor attachment sequence. The heterologous GPI anchor attachment sequence can be derived from any known GPI-anchored protein (reviewed in Ferguson MAJ, Kinoshita T, Hart GW. Glycosylphosphatidylinositol Anchors. In: Varki A, Cummings RD, Esko JD, et al., editors. Essentials of Glycobiology. 2nd edition. Cold Spring Harbor (NY): Cold Spring Harbor Laboratory Press; 2009. Chapter 11). In some embodiments, the heterologous GPI anchor attachment sequence is the GPI anchor attachment sequence from CD14, CD16, CD48, CD55 (DAF), CD59, CD80, and CD87. In some embodiments, the heterologous GPI anchor attachment sequence is derived from CD16. In illustrative embodiments, the heterologous GPI anchor attachment sequence is derived from Fc receptor FcγRIIIb (CD16b) or decay accelerating factor (DAF), otherwise known as complement decay-accelerating factor or CD55.

In some embodiments, one or both of the activation elements include a heterologous signal sequence to help direct expression of the activation element to the cell membrane. Any signal sequence that is active in the packaging cell line can be used. In some embodiments, the signal sequence is a DAF signal sequence. In illustrative embodiments, an activation element is fused to a DAF signal sequence at its N terminus and a GPI anchor attachment sequence at its C terminus.

In an illustrative embodiment, the activation element includes anti-CD3 scFvFc fused to a GPI anchor attachment sequence derived from CD14 and CD80 fused to a GPI anchor attachment sequence derived from CD16b; and both are expressed on the surface of a replication incompetent recombinant retroviral particle provided herein. In some embodiments, the anti-CD3 scFvFc is fused to a DAF signal sequence at its N terminus and a GPI anchor attachment sequence derived from CD14 at its C terminus and the CD80 is fused to a DAF signal sequence at its N terminus and a GPI anchor attachment sequence derived from CD16b at its C terminus; and both are expressed on the surface of a replication incompetent recombinant retroviral particle provided herein. In some embodiments, the DAF signal sequence includes amino acid residues 1-30 of the DAF protein.

### MEMBRANE-BOUND CYTOKINES

Some embodiments of the method and composition aspects provided herein, include a membrane-bound cytokine, or polynucleotides encoding a membrane-bound cytokine. Cytokines are typically, but not always, secreted proteins. Cytokines that are naturally secreted can be engineered as fusion proteins to be membrane-bound. Membrane-bound cytokine fusion polypeptides are included in methods and compositions disclosed herein. In some embodiments, replication incompetent recombinant retroviral particles have a membrane-bound cytokine fusion polypeptide on their surface that is capable of binding a T cell and/or NK cell and promoting proliferation and/or survival thereof. Typically, membrane-bound polypeptides are incorporated into the membranes of replication incompetent recombinant retroviral particles, and when a cell is transduced by the replication incompetent recombinant retroviral particles, the fusion of the retroviral and host cell membranes results in the polypeptide being bound to the membrane of the transduced cell.

In some embodiments, the cytokine fusion polypeptide includes IL-7, IL-15, or an active fragment thereof. The membrane-bound cytokine fusion polypeptides are typically a cytokine fused to heterologous signal sequence and/or a heterologous membrane attachment sequence. In some embodiments, the heterologous membrane attachment sequence is a GPI anchor attachment sequence. The heterologous GPI anchor attachment sequence can be derived from any known GPI-anchored protein (reviewed in Ferguson MAJ, Kinoshita T, Hart GW. Glycosylphosphatidylinositol Anchors. In: Varki A, Cummings RD, Esko JD, et al., editors. Essentials of Glycobiology. 2nd edition. Cold Spring Harbor (NY): Cold Spring Harbor Laboratory Press; 2009. Chapter 11). In some embodiments, the heterologous GPI anchor attachment sequence is the GPI anchor attachment sequence from CD14, CD16, CD48, CD55 (DAF), CD59, CD80, and CD87. In some embodiments, the heterologous GPI anchor attachment sequence is derived from CD16. In an illustrative embodiment, the heterologous GPI anchor attachment sequence is derived from Fc receptor FcγRIIIb (CD16b). In some embodiments, the GPI anchor is the GPI anchor of DAF.

In illustrative embodiments, the membrane-bound cytokine is a fusion polypeptide of a cytokine fused to DAF. DAF is known to accumulate in lipid rafts that are incorporated into the membranes of replication incompetent recombinant retroviral particles budding from packaging cells. Accordingly, not to be limited by theory, it is believed that DAF fusion proteins are preferentially targeted to portions of membranes of packaging cells that will become part of a recombinant retroviral membrane.

In non-limiting illustrative embodiments, the cytokine fusion polypeptide is an IL-7, or an active fragment thereof, fused to DAF. In a specific non-limiting illustrative embodiment, the fusion cytokine polypeptide includes in order: the DAF signal sequence (residues 1-31 of DAF), IL-7 without its signal sequence, and residues 36-525 of DAF.

### RIBOSWITCH CONTROL ELEMENT

### Riboswitches

Some of the compositions and methods provided herein include one or more riboswitches or polynucleotides that include one or more riboswitch, which themselves form distinct aspects of the present disclosure. Riboswitches are a common feature in bacteria to regulate gene expression and are a means to achieve RNA control of biological functions. Riboswitches are polynucleotides that can be present in the 5'-untranslated region of mRNAs and allow for regulatory control over gene expression through binding of a small molecule ligand that induces or suppresses a riboswitch activity. Typically, the riboswitch controls a gene product involved in the generation of the small molecule ligand, thus forming a feedback loop. Riboswitches typically act in a cis-fashion, although riboswitches have been identified that act in a trans-fashion. Natural riboswitches consist of two domains: an aptamer domain that binds the ligand through a three-dimensional folded RNA structure and a function switching domain that induces or suppresses an activity in the riboswitch based on the absence or presence of the ligand. Thus, there are two ligand sensitive conformations achieved by the riboswitch, representing on and off states (Garst et al., 2011). The function switching domain can affect the expression of a polynucleotide by regulating: an internal ribosome entry site, pre-mRNA splice donor accessibility in the retroviral gene construct, pre-mRNA splicing, translation, termination of transcription, transcript degradation, miRNA expression, or shRNA expression (Dambach and Winkler 2009). The aptamer and function switching domains can be used as modular components allowing for synthetic RNA devices to control gene expression either as native aptamers, mutated/evolved native aptamers, or totally synthetic aptamers that are identified from screening random RNA libraries (McKeague et al 2016).

The purine riboswitch family represents one of the largest families with over 500 sequences found (Mandal et al 2003; US20080269258; and WO2006055351). The purine riboswitches share a similar structure consisting of three conserved helical elements/stem structures (P1, P2, P3) with intervening loop/junction elements (J1-2, L2, J2-3, L3, J3-1). The aptamer domains of the purine family of riboswitches naturally vary in their affinity/regulation by various purine compounds such as adenine, guanine, adenosine, guanosine, deoxyadenosine, deoxyguanosine (FIG. 5), etc. due to sequence variation (Kim et al. 2007).

In one aspect, provided herein is an isolated polynucleotide for regulating expression of a target polynucleotide, including: a polynucleotide encoding the target polynucleotide operably linked to a promoter and a riboswitch, wherein the riboswitch includes: a.) an aptamer domain capable of binding a nucleoside analogue antiviral drug and having reduced binding to guanine or 2'-deoxyguanosine relative to the nucleoside analogue antiviral drug; and b.) a function switching domain capable of regulating expression of the target polynucleotide, wherein binding of the nucleoside analogue by the aptamer domain induces or suppresses the expression regulating activity of the function switching domain, thereby regulating expression of the target gene. In some embodiments, the target polynucleotide can be a polypeptide encoding region, an miRNA, or an shRNA. In a non-limiting example, the riboswitch is operably linked to a nucleic acid encoding a polypeptide, miRNA, or shRNA with *in vivo* activity, for example that is effective at treating a disease. For example, in such a non-limiting example, the riboswitch is operably linked to a nucleic acid encoding a chimeric antigen receptor. In non-limiting illustrative examples provided herein, the target polynucleotide encodes one or more engineered signaling polypeptides included in various other aspects of the present disclosure. In these non-limiting illustrative examples, the riboswitch and the target polynucleotide encoding one or more engineered signaling polypeptides can be found in the genome of a packaging cell, in a replication incompetent recombinant retroviral particle, in a T cell and/or in an NK cell.

In some embodiments, the aptamer domain can be between 30, 35, 40, 45, 50, 55, 60, 65, and 70 nucleotides in length on the low end of the range and 35, 40, 45, 50, 55, 60, 65, 70, 75, 80, 85, 90, 95, and 100 nucleotides in length on the high end of the range, for example between 45 and 80 nucleotides in length, between 45 and 60 nucleotides in length, or between 45 and 58 nucleotides in length. In illustrative embodiments, the nucleoside analogue antiviral drug can be the pharmaceutical ligand acyclovir (also known as aciclovir and acycloguanosine) or penciclovir (FIG. 5). In some embodiments, the aptamer domain can have a binding affinity to the nucleoside analogue antiviral drug greater than, for example at least 2, 3, 4, 5, 6, 7, 8, 9, 10, 20, 30, 40, 50, 60, 70, 80, 90, or 100-fold greater than the binding affinity to the nucleoside or nucleotide.

The control element, for example a riboswitch, in some embodiments is operably linked to a target gene and can control expression of the target gene *in vitro* and/or *in vivo.* promotes expansion of transduced T cells *in vivo.* In some embodiments, expansion is dependent on the presence of the control element. However, in other embodiments, expansion of the transduced T cells can be at least partially driven by other factors such as the presence of interleukins within the subject and binding of the ASTR of a CAR on the recombinant T cell to its ligand.

In some embodiments, a nucleoside analogue antiviral drug, for example acyclovir or penciclovir, is administered to a subject before, during, and/or after PBLs are isolated from the blood and before T cells and/or NK cells are contacted with a replication incompetent recombinant retroviral particle that includes a control element, which in illustrative non-limiting examples is a riboswitch, that binds to the nucleoside analogue antiviral drug and regulates expression of one or more target polynucleotides. The one or more target polynucleotides can encode one or more polypeptides that in non-limiting illustrative examples are one or more engineered signaling polypeptides, at least one of which encodes at least one lymphoproliferative element. In some embodiments, the nucleoside analogue antiviral drug, for example acyclovir or penciclovir, is administered to the subject for between 5, 10, 15, 30, and 60 minutes on the low end of the range, and 1.5, 2, 3, 4, 5, 6, 8, 12, 24, 48, or 72 hours on the high end of the range, before PBLs are isolated from the blood or before T cells and/or NK cells are contacted with replication incompetent recombinant retroviral particles. In some embodiments, the nucleoside analogue antiviral drug, for example acyclovir or penciclovir, is administered to the subject for between 1.5, 2, 3, 4, 5, 6, 8, 12, or 24 hours on the low end of the range, ½, 1, 2, 3, 4, 5, 6, 7, 10, 14, 21, or 28 days on the high end of the range, after PBLs are isolated from the blood or after T cells and/or NK cells are contacted with replication incompetent recombinant retroviral particles in methods provided herein. In some embodiments, the nucleoside analogue antiviral drug, for example acyclovir or penciclovir, is administered to the subject for at least 1.5, 2, 3, 4, 5, 6, 8, 12, or 24 hours, or at least 2, 3, 4, 5, 6, 7, 10, 14, 21, or 28 days after PBLs are isolated from the blood or after T cells and/or NK cells are contacted with replication incompetent recombinant retroviral particles in methods provided herein. In some embodiments, the nucleoside analogue antiviral drug, for example acyclovir or penciclovir, is administered to the subject for at least 1, 2, 3, 4, 5, 7, 10, 14, 21, 28, 30, 60, 90, or 120 days or 5, 6, 9, 12, 24, 36, 48, 60, 72, 84, 96, 120 months or indefinitely after the PBLs have been reinfused into the subject. In any of the embodiments disclosed herein, the nucleoside analogue antiviral drug can be administered before and/or during the reinfusion of the PBLs and/or after the PBLs have been reinfused. In some embodiments, the nucleoside analogue antiviral drug is administered until a subject no longer experiences symptoms of, or is afflicted by, a disease for which the target polynucleotide is related.

In some embodiments, the aptamer domain can preferentially bind penciclovir over acyclovir or alternatively another antiviral agent, such that concomitant antiviral therapy may be utilized without affecting the riboswitch. In some embodiments, the aptamer domain can bind penciclovir with a binding affinity greater than, for example at least 2, 3, 4, 5, 6, 7, 8, 9, 10, 20, 30, 40, 50, 60, 70, 80, 90, or 100-fold greater than the aptamer domain binds acyclovir or another antiviral agent. In some embodiments, the aptamer domain can preferentially bind acyclovir over penciclovir or alternatively another antiviral agent, such that concomitant antiviral therapy may be utilized without affecting the riboswitch. In some embodiments, the aptamer domain can bind acyclovir with a binding affinity greater than, for example at least 2, 3, 4, 5, 6, 7, 8, 9, 10, 20, 30, 40, 50, 60, 70, 80, 90, or 100-fold greater than the aptamer domain binds penciclovir or another antiviral agent. In some embodiments, the oral prodrugs of penciclovir (famciclovir) and acyclovir (valaciclovir) can be given to a subject.

In some embodiments, the aptamer domain of an isolated polynucleotide can share at least 80%, 85%, 90%, 91%, 92%, 93%, 94%, 95%, 96%, 97%, 98%, or 99% sequence identity or be identical to any one of the sequences of SEQ ID NOs:87-93 and retain the ability to bind acyclovir and a reduced ability to bind to guanine or 2'-deoxyguanosine relative to the nucleoside analogue antiviral drug, and wherein the aptamer domain retains the ability to induce or suppress the expression regulating activity of the function switching domain when bound by acyclovir. In some embodiments, the aptamer domain of an isolated polynucleotide can share at least 80%, 85%, 90%, 91%, 92%, 93%, 94%, 95%, 96%, 97%, 98%, or 99% sequence identity or be identical to the aptamer domain of SEQ ID NOs:94-100 and retain the ability to bind penciclovir and a reduced ability to bind to guanine or 2'-deoxyguanosine relative to the nucleoside analogue antiviral drug, and wherein the aptamer domain retains the ability to induce or suppress the expression regulating activity of the function switching domain when bound by penciclovir. In some embodiments, a region of an isolated polynucleotide or a region of a riboswitch can share at least 80%, 85%, 90%, 91%, 92%, 93%, 94%, 95%, 96%, 97%, 98%, or 99% sequence identity or be identical to any one of the sequences of SEQ ID NOs:87-100.

In some embodiments, a DNA sequence containing a region of an aptamer domain of an isolated polynucleotide can share at least 80%, 85%, 90%, 91%, 92%, 93%, 94%, 95%, 96%, 97%, 98%, or 99% sequence identity or be identical to any one of the sequences of SEQ ID NOs:108-221. In some embodiments, a region of an isolated polynucleotide can share at least 80%, 85%, 90%, 91%, 92%, 93%, 94%, 95%, 96%, 97%, 98%, or 99% sequence identity or be identical to any one of the sequences of SEQ ID NOs:108-221.

In some embodiments, a DNA sequence containing a region of an aptamer domain of an isolated polynucleotide can share at least 80%, 85%, 90%, 91%, 91.84%, 92%, 93%, 94%, 95%, 96%, 97%, 98%, or 99% sequence identity or be identical to SEQ ID NO: 108. In some embodiments, a DNA sequence containing a region of an aptamer domain of an isolated polynucleotide can share at least 80%, 85%, 90%, 91%, 92%, 93%, 94%, 95%, 95.83%, 96%, 97%, 98%, or 99% sequence identity or be identical to SEQ ID NO:147. In some embodiments, a DNA sequence containing a region of an aptamer domain of an isolated polynucleotide can share at least 80%, 85%, 90%, 91%, 92%, 93%, 93.88%, 94%, 95%, 96%, 97%, 98%, or 99% sequence identity or be identical to SEQ ID NO:164. In some embodiments, a DNA sequence containing a region of an aptamer domain of an isolated polynucleotide can share at least 80%, 85%, 90%, 91%, 92%, 93%, 94%, 95%, 95.83%96%, 97%, 98%, or 99% sequence identity or be identical to SEQ ID NO:183. In some embodiments, a DNA sequence containing a region of an aptamer domain of an isolated polynucleotide can share at least 80%, 85%, 90%, 91%, 91.84%, 92%, 93%, 94%, 95%, 95.83%96%, 97%, 98%, or 99% sequence identity or be identical to SEQ ID NO:198.

In some embodiments, a region of an isolated polynucleotide can include any one of the consensus sequences of SEQ ID NOs:222-226. In some embodiments, a region of an isolated polynucleotide can share at least 80%, 85%, 90%, 91%, 92%, 93%, 94%, 95%, 95.83%, 96%, 97%, 98%, or 99% sequence identity or be identical to any one of the sequences of SEQ ID NOs:222-226.

In any of the embodiments disclosed herein, the isolated polynucleotide can retain the ability to bind acyclovir and/or penciclovir. In any of the embodiments disclosed herein, an isolated polynucleotide can be the reverse complement of any one of the sequences of SEQ ID NOs: 87-100 or SEQ ID NOs:108-221. In any of the embodiments disclosed herein, an isolated polynucleotide can be a transcription or RNA version of either the DNA sequences of SEQ ID NOs: 108-221 or the DNA sequences complementary to SEQ ID NOs:108-221. In any of the embodiments disclosed herein, an isolated polynucleotide can be a reverse transcription or DNA version of any one of the RNA sequences of SEQ ID NOs:87-100 or the DNA strand complementary to a reverse transcription of any one of the RNA sequences of SEQ ID NOs:87-100.

In some embodiments provided herein, riboswitch scaffolds can be used for mutational analysis or molecular evolution. The riboswitches selected for mutational analysis or molecular evolution can be from any known organism, for example, bacteria. In some embodiments, the type I-A deoxyguanosine riboswitch from *Mesoplasma florum* can be used for molecular evolution. In some embodiments, the derived aptamer domain can be at least 50%, 60%, 70%, 75%, 80%, 85%, 90%, or 95% identical to the aptamer domain from the type I-A deoxyguanosine riboswitch from *Mesoplasma florum* (SEQ ID NO:237). In other embodiments, the xpt riboswitch from *Bacillus subtilis* can be used. In some embodiments, the derived aptamer domain can be at least 50%, 60%, 70%, 75%, 80%, 85%, 90%, or 95% identical to the aptamer domain from the xpt riboswitch from *Bacillus subtilis* (SEQ ID NO:243).

The aptamer domains can be used as modular components and combined with any of the function switching domains to affect the RNA transcript. In any of the embodiments disclosed herein, the riboswitch can affect the RNA transcript by regulating any of the following activities: internal ribosomal entry site (IRES), pre-mRNA splice donor accessibility, pre-mRNA splicing, translation, termination of transcription, transcript degradation, miRNA expression, or shRNA expression. In some embodiments, the function switching domain can control binding of an anti-IRES to an IRES (see, e.g. Ogawa, RNA (2011), 17:478-488, the disclosure of which can be referred to for further details). In any of the embodiments disclosed herein, the presence or absence of the small molecule ligand can cause the riboswitch to affect the RNA transcript. In some embodiments, the riboswitch can include a ribozyme. Riboswitches with ribozymes can inhibit or enhance transcript degradation of target polynucleotides in the presence of the small molecule ligand. In some embodiments, the ribozyme can be a pistol class of ribozyme, a hammerhead class of ribozyme, a twisted class of ribozyme, a hatchet class of ribozyme, or the HDV (hepatitis delta virus) ribozyme.

In any of the embodiments disclosed herein, the riboswitch can be located in various positions relative to the target polynucleotide, as is known generally for riboswitches. In some embodiments, the riboswitch can regulate pre-mRNA splice donor accessibility or pre-mRNA splicing and be located before the target polynucleotide. In some embodiments, the riboswitch can regulate the inclusion of a poly(A) tail and be located after the target polynucleotide. In some embodiments, the riboswitch can regulate an anti-IRES and be located upstream of an IRES. In non-limiting illustrative embodiments, a riboswitch provided herein can be located in any of these positions relative to a nucleic acid encoding one or more engineered signaling polypeptides provided herein.

In any of the embodiments disclosed herein that include a polynucleotide including a riboswitch, the polynucleotide can further include one or more of a Kozak-type sequence, a WPRE element, and a double stop codon or a triple stop codon, wherein one or more of the stop codons of the double stop codon or the triple stop codon define a termination of a reading from of at least one of the one or more transcriptional units. In certain embodiments, the polynucleotide includes a Kozak-type sequence selected from CCACCAUG(G) (SEQ ID NO:515), CCGCCAT/UG(G) (SEQ ID NO:516), GCCGCCGCCAT/UG(G) (SEQ ID NO:517), or GCCGCCGCCAT/UG. In certain embodiments, both the -3 and +4 nucleotides relative to a start codon of the first nucleic acid sequence include a G. In another embodiment, which can be combined with the preceding embodiment that includes a Kozak-type sequence and/or the following embodiment that includes triple stop codon, the polynucleotide includes a WPRE element. In some embodiments, the WPRE element is located 3' of a stop codon of the one or more transcriptional units and 5' to a 3' LTR of the polynucleotide. In another embodiment, which can be combined with either or both of the preceding embodiments (i.e. an embodiment wherein the polynucleotide includes a Kozak-type sequence and/or an embodiment wherein the polynucleotide included a WPRE), the one or more transcriptional units terminates with one or more stop codons of a double stop codon or a triple stop codon.

In some embodiments, the riboswitch can be destabilized at temperatures above 37.5 °C, 38 °C, 38.5 °C, 39 °C, 39.5 °C, or 40 °C such that the riboswitch is no longer responsive to the ligand. In some embodiments, molecular evolution can be used to select riboswitches that are destabilized at temperatures above 37.5 °C, 38 °C, 38.5 °C, 39 °C, 39.5 °C, or 40 °C.

In some embodiments, the target polynucleotide can encode a miRNA, shRNA, and/or a polypeptide, wherein the target polynucleotide is operably linked to a promoter. In some embodiments, the target polynucleotide can encode a lymphoproliferative element. In some embodiments, the target polynucleotide can be an miRNA or shRNA. In some embodiments, the miRNA or shRNA can potentiate the STAT5 pathway or inhibit the SOCS pathway. In some embodiments, the miRNA or shRNA can target transcripts from SOCS1, SMAD2, TGFb, or PD-1. In some embodiments, the miRNA is miR-155. In some embodiments, the target polynucleotide encodes a polypeptide and the polypeptide can include a CAR including an antigen-specific targeting region, a transmembrane domain, and an intracellular activating domain.

In another aspect, provided herein is an isolated polynucleotide for regulating expression of a target polynucleotide, including: a polynucleotide encoding the target polynucleotide operably linked to a promoter and a riboswitch, wherein the riboswitch includes: a.) an aptamer domain capable of binding a nucleoside analogue antiviral drug with a binding affinity at least two-fold greater affinity than the aptamer domain binds guanine or 2'-deoxyguanosine; and b.) a function switching domain capable of regulating expression of the target polynucleotide, wherein binding of the nucleoside analogue by the aptamer domain induces or suppresses the expression regulating activity of the function switching domain. In some embodiments, the aptamer domain can bind the nucleoside analogue antiviral drug with a binding affinity at least 3, 4, 5, 6, 7, 8, 9, 10, 20, 30, 40, 50, 60, 70, 80, 90, or 100-fold greater affinity than the aptamer domain binds guanine or 2'-deoxyguanosine. In some embodiments, the aptamer domain can be between 30, 35, 40, 45, 50, 55, 60, 65, and 70 nucleotides in length on the low end of the range and 35, 40, 45, 50, 55, 60, 65, 70, 75, 80, 85, 90, 95, and 100 nucleotides in length on the high end of the range, for example between 45 and 80 nucleotides in length or between 45 and 58 nucleotides in length. In illustrative embodiments, the nucleoside analogue antiviral drug can be the pharmaceutical ligand acyclovir (also known as aciclovir and acycloguanosine) or penciclovir. In some embodiments, the aptamer domain can have a binding affinity to the nucleoside analogue antiviral drug that is greater than, for example at least 2, 3, 4, 5, 6, 7, 8, 9, 10, 20, 30, 40, 50, 60, 70, 80, 90, or 100-fold greater than the binding affinity to the nucleoside or nucleotide. In some embodiments, binding of the nucleoside analogue by the aptamer domain can induce an activity in the riboswitch.

In some embodiments, the aptamer domain can be specific for penciclovir and lack reactivity to acyclovir or alternatively another antiviral agent, such that concomitant antiviral therapy may be utilized without affecting the riboswitch. In some embodiments, the aptamer domain can bind penciclovir with a binding affinity at least 2, 3, 4, 5, 6, 7, 8, 9, 10, 20, 30, 40, 50, 60, 70, 80, 90, or 100-fold greater than the aptamer domain binds acyclovir or another antiviral agent. In some embodiments, the aptamer domain can be specific for acyclovir and lack reactivity to penciclovir or alternatively another antiviral agent, such that concomitant antiviral therapy may be utilized without affecting the riboswitch. In some embodiments, the aptamer domain can bind acyclovir with a binding affinity at least 2, 3, 4, 5, 6, 7, 8, 9, 10, 20, 30, 40, 50, 60, 70, 80, 90, or 100-fold greater than the aptamer domain binds penciclovir or another antiviral agent. In some embodiments, the oral prodrugs of penciclovir (famciclovir) and acyclovir (valaciclovir) can be given to a subject. In some embodiments, the derived aptamer domain can be at least 50%, 60%, 70%, 75%, 80%, 85%, 90%, or 95% identical to the aptamer domain from the type I-A deoxyguanosine riboswitch from *Mesoplasma florum.* In some embodiments, the derived aptamer domain can be at least 50%, 60%, 70%, 75%, 80%, 85%, 90%, or 95% identical to the aptamer domain from the xpt riboswitch from *Bacillus subtilis.* In any of the embodiments disclosed herein, the riboswitch can affect the RNA transcript by regulating any of the following activities: internal ribosomal entry site, pre-mRNA splice donor accessibility in the retroviral gene construct, pre-mRNA splicing, translation, termination of transcription, transcript degradation, miRNA expression, or shRNA expression. In some embodiments, the function switching domain cahn control binding of an anti-IRES to an IRES. In any of the embodiments disclosed herein, the presence or absence of the small molecule ligand can cause the riboswitch to affect the RNA transcript. In some embodiments, the riboswitch can include a ribozyme. Riboswitches with ribozymes can inhibit or enhance transcript degradation of genes of interest in the presence of the small molecule ligand. In some embodiments, the ribozyme can be a pistol class of ribozyme, a hammerhead class of ribozyme, a twisted class of ribozyme, a hatchet class of ribozyme, or the HDV (hepatitis delta virus) ribozyme. In some embodiments, the riboswitch can be destabilized at temperatures above 37.5 °C, 38 °C, 38.5 °C, 39 °C, 39.5 °C, or 40 °C such that the riboswitch is no longer responsive to the ligand. In some embodiments, molecular evolution can be used to select riboswitches that are destabilized at temperatures above 37.5 °C, 38 °C, 38.5 °C, 39 °C, 39.5 °C, or 40 °C. In some embodiments, the target polynucleotide can encode a miRNA, shRNA, and/or a polypeptide, wherein the target polynucleotide is operably linked to a promoter. In some embodiments, the target polynucleotide can encode a lymphoproliferative element. In some embodiments, the target polynucleotide can be an miRNA and, optionally, the miRNA can stimulate the STAT5 pathway or inhibit the SOCS pathway. In some embodiments, the miRNA can target transcripts from SOCS1, SHP, SMAD2, TGFb, or PD-1. In these embodiments, the miRNA can be miR-155. In some embodiments, the target polynucleotide encodes a polypeptide and the polypeptide can include a CAR including an antigen-specific targeting region, a transmembrane domain, and an intracellular activating domain. Further embodiments of CARs are disclosed elsewhere herein.

In some embodiments, the evolution of aptamers can be performed via aptamer selection from randomized native purine or guanine aptamer libraries using SELEX (Systematic Evolution of Ligands by EXponential enrichment) methods including, but not limited to, those methods that employ graphene oxide in the selection process and screening. In other embodiments, random mutagenesis methodology such as error prone PCR can be used to evolve aptamer constructs or riboswitch constructs where the aptamer is incorporated in the context of any of the riboswitch activities described herein by screening *in vitro* or in mammalian cells. In other embodiments, random libraries of nucleotides can be used in the evolution of the riboswitch. In any of the embodiments disclosed herein, riboswitches can be identified from screening such libraries *in vitro* or in mammalian cells.

In some embodiments, the evolved or derived aptamer domain can have increased binding to analogues of the native ligand and decreased binding to the native ligand. In some embodiments, the aptamer domain can be configured to have increased binding to analogues of the native ligand and decreased binding to the native ligand. In some embodiments, the aptamer domain can be derived from the purine riboswitch family. In some embodiments, the native ligand can be a nucleoside or nucleotide and the analogue can be a nucleoside analogue or nucleotide analogue. In some embodiments, the nucleoside analogue is an antiviral drug. In illustrative embodiments, the aptamer domains can be derived from 2'-deoxyguanosine and guanine riboswitch scaffolds and the derived aptamer domains can show reduced binding to 2'-deoxyguanosine and guanine relative to the wild-type riboswitch.

In some embodiments, the riboswitch can regulate pre-mRNA splice donor accessibility in the retroviral gene construct, wherein the retroviral construct drives the CAR genes or other genes of interest from the reverse strand under a general promoter or a T cell specific promoter. In other embodiments, the riboswitch can regulate an IRES in the retroviral gene construct, wherein the retroviral construct drives the translation of CAR genes or other genes of interest. In other embodiments, the riboswitch can control transcription termination of the RNA, miRNA, or gene transcripts or can control translation of the transcript. In other embodiments, the nucleoside analogue riboswitch can be integrated with a ribozyme to inhibit or enhance transcript degradation of the CAR genes or other genes of interest in the presence of the nucleoside analogue.

In some embodiments, the isolated polynucleotide for regulating expression of a target polynucleotide that includes a polynucleotide encoding the target polynucleotide operably linked to a promoter and a riboswitch that binds a nucleoside analogue antiviral drug, is a molecular cloning vector. The molecular cloning vector can be any type of molecular cloning vector known in the art. As non-limiting examples, the vector can be a plasmid, a virus, a replication incompetent viral particle, a retrovirus, or a replication incompetent recombinant retroviral particle, any of which can be an expression vector. Such an expression vector can encode any of the target polynucleotides provided hereinabove. One or more restriction and/or multiple cloning sites can be included on a molecular cloning vector 5' or 3' to a riboswitch provided herein such that the riboswitch is operably linked to a target polynucleotide inserted into the restriction and/or multiple cloning site.

### Molecular Chaperones

In one aspect, provided herein is a method for genetically modifying and expanding lymphocytes of a subject, comprising:
A. contacting resting T cells and/or NK cells of the subject *ex vivo,* typically without requiring prior *ex vivo* stimulation, with replication incompetent recombinant retroviral particles comprising:
   i. a pseudotyping element on its surface that is capable of binding to a T cell and/or NK cell and facilitating membrane fusion of the replication incompetent recombinant retroviral particles thereto; and
   ii. a polynucleotide comprising one or more transcriptional units operatively linked to a promoter active in T cells and/or NK cells, wherein the one or more transcriptional units encode a first engineered signaling polypeptide regulated by a control element, wherein said first engineered signaling polypeptide comprises at least one lymphoproliferative element and/or a chimeric antigen receptor,
   wherein said contacting facilitates transduction of at least some of the resting T cells and/or NK cells by the replication incompetent recombinant retroviral particles, thereby producing genetically modified T cells and/or NK cells;
B. introducing the genetically modified T cells and/or NK cells into the subject; and
C. exposing the genetically modified T cells and/or NK cells *in vivo* to a compound that acts as the control element to affect expression of the first engineered signaling polypeptide and promote expansion of the lymphocytes *in vivo,* thereby genetically modifying and expanding lymphocytes of the subject.

In illustrative embodiments, the transduction is carried out without *ex vivo* stimulation. In illustrative embodiments, the compound is a molecular chaperone, such as a small molecule molecular chaperone. In illustrative embodiments, binding of the molecular chaperone to the lymphoproliferative element and/or CAR component increases the proliferative activity of the lymphoproliferative element and/or the CAR. The molecular chaperone can be administered to the subject before the blood is collected, during the contacting, and/or after the T cells and/or NK cells are introduced into the subject. Some embodiments of this aspect include collecting blood from the subject. In these embodiments, the introducing is a reintroducing of the cells that were collected and genetically modified before reintroduction. The entire process, in illustrative embodiments, is a shorter process than prior art methods, as for other aspects herein. For example, the entire process can be completed in less than 48 hours, less than 24 hours, or less than 12 hours. The entire process in other embodiments, can be completed in 2, 4, 6, or 8 hours on the low end of the range, and 12, 24, 36, or 48 hours on the high end of the range.

Accordingly, in some embodiments of the methods and compositions provided herein, the control element is a molecular chaperone. As compared to other embodiments herein with other *in vivo* control elements, such as riboswitches that typically bind a compound to affect expression of a lymphoproliferative element or other component of a first or second engineered signaling polypeptide herein, the molecular chaperones are compounds that are the control elements and as such, directly affect activity of, typically by binding to, a lymphoproliferative element or other component of a first or second engineered signaling polypeptide herein. In illustrative examples of such embodiments of methods herein that include the administration of molecular chaperones, a lymphoproliferative element, membrane-bound cytokine, and/or CAR component, can be a less active or inactive lymphoproliferative element, membrane-bound cytokine, and/or CAR component, that is bound by the molecular chaperone to increase its activity. Thus, the target bound by a molecular chaperone is typically a target polypeptide. In some embodiments, as indicated the polypeptide can be a first and/or a second engineered signaling polypeptide, or a polypeptide component thereof, whose activity is affected by binding to the molecular chaperone, which in illustrative embodiments is a small molecule molecular chaperone. In some embodiments, the polypeptide can include a lymphoproliferative element whose activity is regulated, in illustrative embodiments, up-regulated by a molecular chaperone, preferably a small molecule molecular chaperone. The molecular chaperone in the methods provided herein can be a compound that binds to the mutant lymphoproliferative element and/or inactive CAR component, thus rendering them active.

In other embodiments, a lymphoproliferative element or other signaling domain has been mutated to permit transit to the plasma membrane only in the presence of a small molecular synthetic chaperone. In other embodiments, the chaperone promotes stability of the lymphoproliferative element or other signaling domain or protein and half-life as a potentiator.

It will be understood that aspects and embodiments as described herein include many of the same steps and compositions provided in detail herein. Accordingly, it will be understood that the teachings throughout this specification that relate to these common elements apply to aspects and embodiments that utilize a molecular chaperone as the control element, which typically binds a lymphoproliferative element or other target molecule directly, in addition to, or instead of other *in vivo* control elements provided herein, such as riboswitches, which typically utilize a molecule, such as a drug, that binds the riboswitch.

In some embodiments, the molecular chaperone is a compound that can regulate sub-cellular localization of a target, for example, the proper folding and transit of a target protein, such as a lymphoproliferative element and/or a component of a CAR, from the endoplasmic reticulum to the plasma membrane or its half-life on the surface. In other embodiments, the molecular chaperone can promote the functional conformation of a dysfunctional target, thus acting as a potentiator. Examples of molecules that act as chaperones or potentiators to naturally mutated proteins include lumacaftor and ivacaftor. These proteins act upon the mutant CFTR chloride channel variants such as G551D or F508del. Ivacaftor potentiates the activity of the G551D or F508del mutated ion channel, whereas lumacaftor promotes stabilization of mutant chloride channels and subsequent potentiation by ivacaftor. Such chaperone dependent proteins can be generated from naturally functional proteins and screening for functional activity only in the presence of the molecular chaperones. Thus, such proteins are only active when the chaperone is present. Examples of such molecules which can be screened for specific chaperone activity include small molecule antivirals or anti-infectives that show no activity to normal human proteins. Accordingly, in one embodiment, the molecular chaperone used in methods herein is a small molecule antiviral or anti-infective compound that shows no activity to normal human proteins.

In some embodiments, genetically modified lymphocytes can be exposed and/or a subject can be administered the molecular chaperone. In some embodiments, the compound is administered to the subject before, during, and/or after PBLs are isolated from the blood and before T cells and/or NK cells are contacted with a replication incompetent recombinant retroviral particle. The replication incompetent recombinant retroviral particle in such embodiments includes a less active or inactive lymphoproliferative element and/or CAR component that binds to, and is regulated by, the molecular chaperone compound.

For any of the embodiments provided herein for modifying and expanding lymphocytes, which can be part of methods of adoptive cell therapy, the compound can be administered to the subject for between 5, 10, 15, 30, and 60 minutes on the low end of the range, and 1.5, 2, 3, 4, 5, 6, 8, 12, or 24 hours on the high end of the range, before PBLs are isolated from the blood or before T cells and/or NK cells are contacted with a replication incompetent recombinant retroviral particle. In some embodiments, the compound is administered to the subject for between 1.5, 2, 3, 4, 5, 6, 8, 12, or 24 hours on the low end of the range, ½, 1, 2, 3, 4, 5, 6, 7, 10, 14, 21, or 28 days on the high end of the range, after PBLs are isolated from the blood or after T cells and/or NK cells are contacted with a replication incompetent recombinant retroviral particle in methods provided herein. In some embodiments, the compound is administered to the subject for at least 1.5, 2, 3, 4, 5, 6, 8, 12, or 24 hours, or at least 2, 3, 4, 5, 6, 7, 10, 14, 21, or 28 days after PBLs are isolated from the blood or after T cells and/or NK cells are contacted with a replication incompetent recombinant retroviral particle in methods provided herein. In some embodiments, the compound is administered to the subject for at least 1, 2, 3, 4, 5, 7, 10, 14, 21, 28, 30, 60, 90, or 120 days or 5, 6, 9, 12, 24, 36, 48, 60, 72, 84, 96, 120 months or indefinitely after the PBLs have been reinfused into the subject. In any of the embodiments disclosed herein, the compound can be administered before and/or during the reinfusion of the PBLs and/or after the PBLs have been reinfused.

For any of the embodiments herein, molecular chaperones are not in the control elements that are bound by compounds that regulate and/or activate them. Molecular chaperones are compounds, preferably small molecule compounds, that are the control elements and regulate the activity of lymphoproliferative elements and/or functional components of CARs.

### PACKAGING CELL LINES/METHODS OF MAKING RECOMBINANT RETROVIRAL PARTICLES

The present disclosure provides mammalian packaging cells and packaging cell lines that produce replication incompetent recombinant retroviral particles. The cell lines that produce replication incompetent recombinant retroviral particles are also referred to herein as packaging cell lines. A non-limiting example of such method is provided in Example 7 herein. The cells of the packaging cell line can be adherent or suspension cells. In illustrative embodiments, the packaging cell line can be a suspension cell line, i.e. a cell line that does not adhere to a surface during growth. The cells can be grown in a chemically-defined media and/or a serum-free media. In some embodiments, the packaging cell line can be a suspension cell line derived from an adherent cell line, for example, the HEK293 cell line can be grown in conditions to generate a suspension-adapted HEK293 cell line according to methods known in the art. The packaging cell line is typically grown in a chemically defined media. In some embodiments, the packaging cell line media can include serum. In some embodiments, the packaging cell line media can include a serum replacement, as known in the art. In illustrative embodiments, the packaging cell line media can be serum-free media. Such media can be a chemically defined, serum-free formulation manufactured in compliance with Current Good Manufacturing Practice (CGMP) regulations of the US Food and Drug Administration (FDA). The packaging cell line media can be xeno-free and complete. In some embodiments, the packaging cell line media has been cleared by regulatory agencies for use in *ex vivo* cell processing, such as an FDA 510(k) cleared device. It will be understood herein where it is recited that the media includes the composition of the basal media with a media supplement of a catalog number such as A1048501 or A 1048503 that includes a media supplement, that the recited composition is intended to mean the composition of the media with the added supplement. Typically, the manufacture of the media and supplement provides instructions for volumes of supplement to be added.

Accordingly, in one aspect, provided herein is a method of making a replication incompetent recombinant retroviral particle including: A. culturing a packaging cell in suspension in serum-free media, wherein the packaging cell comprises nucleic acid sequences encoding a packagable RNA genome of the replication incompetent retroviral particle, a REV protein, a gag polypeptide, a pol polypeptide, and a pseudotyping element; and B. harvesting the replication incompetent recombinant retroviral particle from the serum-free media. In another aspect, provided herein is a method of transducing a lymphocyte with a replication incompetent recombinant retroviral particle comprising: A. culturing a packaging cell in suspension in serum-free media, wherein the packaging cell comprises nucleic acid sequences encoding a packagable RNA genome of the replication incompetent retroviral particle, a REV protein, a gag polypeptide, a pol polypeptide, and a pseudotyping element; B. harvesting the replication incompetent recombinant retroviral particle from the serum-free media; and C. contacting the lymphocyte with the replication incompetent recombinant retroviral particle, wherein the contacting is performed for less than 24 hours, 20 hours, 18 hours, 12 hours, 8 hours, 4 hours or 2 hours (or between 1, 2, 3, or 4 hours on the low end of the range and 4, 6, 8, 12, 18, 20, or 24 hours on the high end of the range), thereby transducing the lymphocyte.

In another aspect, provided herein is a retroviral packaging system including: a mammalian cell including: a) a first transactivator expressed from a constitutive promoter and capable of binding a first ligand and a first inducible promoter for affecting expression of a nucleic acid sequence operably linked thereto in the presence versus absence of the first ligand; b) a second transactivator capable of binding a second ligand and a second inducible promoter, and affecting expression of a nucleic acid sequence operably linked thereto in the presence versus absence of a second ligand; and c) a packageable RNA genome for a retroviral particle, wherein the first transactivator regulates expression of the second transactivator, and wherein the second transactivator regulates expression of retroviral polypeptides involved in viral packaging, such as, for example, a gag polypeptide, a pol polypeptide, and/or a pseudotyping element, and optionally other polypeptides that will become incorporated in or on the replication incompetent recombinant retroviral particle and are believed to be toxic to packaging cell lines, such as, for example, HEK-293. In certain aspects, the second transactivator itself is cytotoxic to packaging cell lines. Pseudotyping elements are typically capable of binding to a cell membrane of a target cell and facilitating fusion thereto, as discussed in detail herein. Thus, not to be limited by theory, the system provides the ability to accumulate certain polypeptides/proteins that do not inhibit, or do not substantially inhibit, or are not believed to inhibit proliferation or survival of the mammalian cells, for example, non-toxic proteins, while culturing a population of the mammalian cells for days or indefinitely, and controlling induction of polypeptides that are desired for retroviral product but that are inhibitory or can be inhibitory or have been reported to be inhibitory to the survival and/or proliferation of the mammalian cell, for example toxic polypeptides, until a later time closer to the time of when replication incompetent recombinant retroviral particles will be produced and harvested. The packageable RNA genome is typically encoded by a polynucleotide operably linked to a promoter, sometimes referred to herein as a third promoter for convenience, wherein said third promoter is typically inducible by either the first transactivator or the second transactivator. In illustrative embodiments, the packageable RNA genome is encoded by a polynucleotide operably linked to a third promoter, wherein said third promoter is inducible by the second transactivator. As such, the packageable RNA genome can be produced at the later time point, closer to when the replication incompetent recombinant retroviral particles will be harvested.

A skilled artisan will appreciate many different transactivators, ligands, and inducible promoters can be used in the retroviral packaging system. Such inducible promoters can be isolated and derived from many organisms, e.g., eukaryotes and prokaryotes. Modification of inducible promoters derived from a first organism for use in a second organism, e.g., a first prokaryote and a second a eukaryote, a first eukaryote and a second a prokaryote, etc., is well known in the art. Such inducible promoters, and systems based on such inducible promoters but also including additional control proteins, include, but are not limited to, alcohol regulated promoters (e.g., alcohol dehydrogenase I (alcA) gene promoter, promoters responsive to alcohol transactivator proteins (AlcR), etc.), tetracycline regulated promoters, (e.g., promoter systems including TetActivators, TetON, TetOFF, etc.), steroid regulated promoters (e.g., rat glucocorticoid receptor promoter systems, human estrogen receptor promoter systems, retinoid promoter systems, thyroid promoter systems, ecdysone promoter systems, mifepristone promoter systems, etc.), metal regulated promoters (e.g., metallothionein promoter systems, etc.), pathogenesis-related regulated promoters (e.g., salicylic acid regulated promoters, ethylene regulated promoters, benzothiadiazole regulated promoters, etc.), temperature regulated promoters (e.g., heat shock inducible promoters (e.g., HSP-70, HSP-90, soybean heat shock promoter, etc.), light regulated promoters, synthetic inducible promoters, and the like. In some embodiments, a mifepristone-regulated system can be used. In some embodiments, a mifepristone-inducible system with an autoregulatory feedback loop can be used. In some embodiments, a GAL4 regulatory fusion protein is expressed from one construct that also contains the transposon terminal repeats and lox and FRT sites. In some embodiments, the GAL4 regulatory fusion protein controls expression of a reverse tet transactivator (rtTA) and BiTRE. In some embodiments, another construct with lox and FRT sites contains a GAL4 upstream activating sequences (UAS) and an E1b TATA box promoter driving a reporter like mCherry. In some embodiments, a GAL4 regulatory fusion protein binds to GAL4 upstream activating sequences (UAS) in both the promoter controlling expression of the GAL4 regulatory fusion protein and the promoter controlling expression of a target polynucleotide. In some embodiments, mifepristone, doxycycline, and puromycin will be used for induction and selection of packaging cell line.

In some embodiments, either or both transactivators can be split into two or more polypeptides. In some embodiments, the two or more polypeptides can include a DNA binding domain and an activation domain capable of stimulating transcription on separate polypeptides. This "activation domain" is not to be confused with an "activation element," such as a polypeptide that binds CD3, which is capable of activating a T cell and/or NK cell, and typically does activate such T cell and/or NK cell when contacted with it, as discussed in detail herein. The separate polypeptides can further include fusions with polypeptides capable of dimerization through the addition of a ligand. In some embodiments, the activation domain can be the p65 activation domain or a functional fragment thereof. In illustrative embodiments of the packaging systems herein, the DNA binding domain can be the DNA binding domain from ZFHD 1 or a functional fragment thereof. In some embodiments, one polypeptide can be a fusion with FKBP, or functional mutants and/or fragments thereof, or multiple FKBPs and another polypeptide can be a fusion with the FRB domain of mTOR, or functional mutants and/or fragments thereof, and the ligand can be rapamycin or a functional rapalog. In some embodiments, the FRB contains the mutations K2095P, T2098L, and/or W2101F. In some embodiments, the separate polypeptides can be FKBP, or functional fragments thereof, and CalcineurinA, or functional fragments thereof, and the dimerizing agent can be FK506. In some embodiments, the separate polypeptides can be FKBP, or functional fragments thereof, and CyP-Fas, or functional fragments thereof, and the dimerizing agent can be FKCsA. In some embodiments, the separate polypeptides can be GAI, or functional fragments thereof, and GID1, or functional fragments thereof, and the dimerizing agent can be gibberellin. In some embodiments, the separate polypeptides can be Snap-tag and HaloTag, or functional fragments thereof, and the dimerizing agent can be HaXS. In some embodiments, the separate polypeptides can include the same polypeptide. For example, the DNA binding domain and activation domain can be expressed as fusion proteins with FKBP or GyrB and the dimerizing agent can be FK1012 or coumermycin, respectively. In some embodiments, the inducible promoter can be the DNA sequence where the DNA binding domain typically binds. In some embodiments, the inducible promoter can vary from the DNA sequence where the DNA binding domain typically binds. In some embodiments, either transactivator can be an rtTA, the ligand can be tetracycline or doxycycline, and the inducible promoter can be a TRE. In illustrative embodiments, the first transactivator is the p65 activation domain fused to FRB and the ZFHD1 DNA binding domain fused to three FKBP polypeptides and the first ligand is rapamycin. In further illustrative embodiments, the second transactivator can be an rtTA, the second ligand can be tetracycline or doxycycline, and the inducible promoter can be a TRE.

In some embodiments, the first transactivator can regulate expression of an element to control the nuclear export of transcripts containing a consensus sequence, such as an HIV Rev and the consensus sequence can be the Rev response element. In illustrative embodiments, the target cell is a T cell.

In some embodiments, the pseudotyping element is a retroviral envelope polypeptide. The pseudotyping element typically includes a binding polypeptide and a fusogenic polypeptide for binding to and facilitating membrane fusion of the target cell and viral membranes, as discussed in more detail herein. In some embodiments, the pseudotyping element is the feline endogenous virus (RD114) envelope protein, the oncoretroviral amphotropic envelope protein, the oncoretroviral ecotropic envelope protein, and/or vesicular stomatitis virus envelope protein (VSV-G) . In illustrative embodiments, the pseudotyping element includes a binding polypeptide and a fusogenic polypeptide derived from different proteins, as discussed in further detail herein. For example, in an illustrative embodiment, especially where the target cell is a T cell and/or NK cell, the binding polypeptide is a hemagglutinin (H) polypeptide of a Measles Virus (such as the Edmonston strain of the Measles Virus), or a cytoplasmic domain deletion variant thereof, and the fusogenic polypeptide other is a fusion (F) polypeptide of a Measles Virus (such as the Edmonston strain of the Measles Virus), or a cytoplasmic domain deletion variant thereof. In some embodiments, the fusogenic polypeptide can include multiple elements expressed as one polypeptide. In some embodiments, the binding polypeptide and the fusogenic polypeptide can be translated from the same transcript but from separate ribosome binding sites, or the polypeptide is cleaved after translation using a peptide cleavage signal or a ribosomal skip sequence, as disclosed elsewhere herein, to generate the binding polypeptide and the fusogenic polypeptide. In some embodiments, where the binding polypeptide is a Measles Virus H polypeptide, or a cytoplasmic domain deletion thereof, and the fusogenic polypeptide is a Measles Virus F polypeptide, or a cytoplasmic domain deletion thereof, translation of the F and H polypeptides from separate ribosome binding sites results in a higher amount of the F polypeptide as compared to the H polypeptide. In some embodiments, the ratio of the F polypeptides (or cytoplasmic domain deletions thereof) to H polypeptides (or cytoplasmic domain deletions thereof) is at least 2:1, at least 3:1, at least 4:1, at least 5:1, at least 6:1, at least 7:1, or at least 8:1.

In some embodiments, the first transactivator can regulate the expression of an activation element capable of binding to and activating a target cell, such as a T cell or NK cell. Any of the activation elements disclosed herein can be expressed. For example, in these embodiments, the activation element can include: a.) a membrane-bound polypeptide capable of binding to and activating CD3; and/or b.) a membrane-bound polypeptide capable of binding to and activating CD28. In some embodiments, the membrane-bound polypeptide capable of binding to and activating CD3 can be an anti-CD3 antibody. In further embodiments, the anti-CD3 antibody can be anti-CD3 scFvFc. In some embodiments, the membrane-bound polypeptide capable of binding to and activating CD28 is CD80, CD86, or functional fragments thereof, such as an extracellular domain of CD80.

In some embodiments, the second transactivator can regulate the expression of a packageable RNA genome that can include an RNA that encodes one or more target polypeptides, including as a non-limiting example, any of the engineered signaling polypeptides disclosed herein and/or one or more (e.g. two or more) inhibitory RNA molecules. It should be noted that it is envisioned that the retroviral packaging system aspect, and the method of making a replication incompetent recombinant retroviral particle aspect, are not limited to making replication incompetent recombinant retroviral particles for transduction of T cell and/or NK cells, but rather for any cell type that can be transduced by replication incompetent recombinant retroviral particles. The packageable RNA genome, in certain illustrative embodiments, is designed to express one or more target polypeptides, including as a non-limiting example, any of the engineered signaling polypeptides disclosed herein and/or one or more (e.g. two or more) inhibitory RNA molecules in opposite orientation (e.g., encoding on the opposite strand and in the opposite orientation), from retroviral components such as gag and pol. For example, the packageable RNA genome can include from 5' to 3': a 5' long terminal repeat, or active truncated fragment thereof; a nucleic acid sequence encoding a retroviral cis-acting RNA packaging element; a nucleic acid sequence encoding a first and optionally second target polypeptide, such as, but not limited to, an engineered signaling polypeptide(s) in opposite orientation, which can be driven off a promoter in this opposite orientation with respect to the 5' long terminal repeat and the cis-acting RNA packaging element, which in some embodiments is called a "fourth" promoter for convenience only (and sometimes referred to herein as the promoter active in T cells and/or NK cells). which is active in a target cell such as a T cell and/or an NK cell but in illustrative examples is not active in the packaging cell or is only inducibly or minimally active in the packaging cell; and a 3' long terminal repeat, or active truncated fragment thereof. In some embodiments, the packageable RNA genome can include a central polypurine tract (cPPT)/central termination sequence (CTS) element. In some embodiments, the retroviral cis-acting RNA packaging element can be HIV Psi. In some embodiments, the retroviral cis-acting RNA packaging element can be the Rev Response Element. The engineered signaling polypeptide driven by the promoter in the opposite orientation from the 5' long terminal repeat, in illustrative embodiments, is one or more of the engineered signaling polypeptides disclosed herein and can optionally express one or more inhibitory RNA molecules as disclosed in more detail herein.

It will be understood that promoter number, such as a first, second, third, fourth, etc. promoter is for convenience only. A promoter that is called a "fourth" promoter should not be taken to imply that there are any additional promoters, such as first, second or third promoters, unless such other promoters are explicitly recited. It should be noted that each of the promoters are capable of driving expression of a transcript in an appropriate cell type and such transcript forms a transcription unit.

In some embodiments, the engineered signaling polypeptide can include a first lymphoproliferative element. Suitable lymphoproliferative elements are disclosed in other sections herein. As a non-limiting example, the lymphoproliferative element can be expressed as a fusion with a recognition domain, such as an eTag, as disclosed herein. In some embodiments, the packageable RNA genome can further include a nucleic acid sequence encoding a second engineered polypeptide including a chimeric antigen receptor, encoding any CAR embodiment provided herein. For example, the second engineered polypeptide can include a first antigen-specific targeting region, a first transmembrane domain, and a first intracellular activating domain. Examples of antigen-specific targeting regions, transmembrane domains, and intracellular activating domains are disclosed elsewhere herein. In some embodiments where the target cell is a T cell, the promoter that is active in a target cell is active in a T cell, as disclosed elsewhere herein.

In some embodiments, the engineered signaling polypeptide can include a CAR, and the nucleic acid sequence can encode any CAR embodiment provided herein. For example, the engineered polypeptide can include a first antigen-specific targeting region, a first transmembrane domain, and a first intracellular activating domain. Examples of antigen-specific targeting regions, transmembrane domains, and intracellular activating domains are disclosed elsewhere herein. In some embodiments, the packageable RNA genome can further include a nucleic acid sequence encoding a second engineered polypeptide. In some embodiments, the second engineered polypeptide can be a lymphoproliferative element. In some embodiments where the target cell is a T cell or NK cell, the promoter that is active in a target cell is active in a T cell or NK cell, as disclosed elsewhere herein.

In some embodiments, the packageable RNA genome can further include a riboswitch, as discussed in other sections herein. In some embodiments, the nucleic acid sequence encoding the engineered signaling polypeptide can be in reverse orientation. In further embodiments, the packageable RNA genome can further include a riboswitch and, optionally, the riboswitch can be in reverse orientation. In any of the embodiments disclosed herein, a polynucleotide including any of the elements can include a primer binding site. In illustrative embodiments, transcription blockers or polyA sequences can be placed near genes to prevent or reduce unregulated transcription. In any of the embodiments disclosed herein, a nucleic acid sequence encoding Vpx can be on the second or an optional third transcriptional unit, or on an additional transcriptional unit that is operably linked to the first inducible promoter.

Provided in another aspect herein is a mammalian packaging cell line comprising a packageable RNA genome for a replication incompetent retroviral particle, wherein said packageable RNA genome comprises:
a. a 5' long terminal repeat, or active fragment thereof;
b. a nucleic acid sequence encoding a retroviral cis-acting RNA packaging element;
c. a polynucleotide comprising one or more nucleic acid sequences operatively linked to a promoter active in T cells and/or NK cells, wherein a first nucleic acid sequence of the one or more nucleic acids encodes one or more (e.g. two or more) inhibitory RNA molecules directed against one or more RNA targets and a second nucleic acid sequence of the one or more nucleic acid sequences encodes a chimeric antigen receptor (CAR) comprising an antigen-specific targeting region (ASTR), a transmembrane domain, and an intracellular activating domain; and
d. a 3' long terminal repeat, or active fragment thereof.

The inhibitory RNA molecules in the above aspect can include any of the inhibitory RNA molecules, as non-limiting examples, shRNA or miRNA, provided herein in other sections of this disclosure.

In some embodiments of the mammalian packaging cell line aspect, the polynucleotide of (c) can be in reverse orientation to the nucleic acid sequence encoding the retroviral cis-acting RNA packaging element (b), the 5' long terminal repeat (a), and/or the 3' long terminal repeat (d).

In some embodiments of the mammalian packaging cell line aspect, expression of the packageable RNA genome is driven by an inducible promoter active in the mammalian packaging cell line.

The promoter active in T cells and/or NK cells that drives expression of the inducible RNA and the CAR in these aspects provided immediately above, in illustrative embodiments is not active or is only minimally or inducibly active in the packaging cell line. This promoter active in T cells and/or NK cells in illustrative embodiments is located on the packageable RNA genome between the nucleic acids encoding the one (e.g. two) or more inducible RNAs and the CAR and the 3' LTR.

In any of the aspects directed to packageable cells or cell lines herein, that encode one or more inhibitory RNA molecules directed against one or more RNA targets, at least one and in some embodiments all inhibitory RNA molecules can include a 5' strand and a 3' strand that are partially or fully complementary to one another, wherein said 5' strand and said 3' strand are capable of forming an 18-25 nucleotide RNA duplex. In some embodiments, the 5' strand can be 18, 19, 20, 21, 22, 23, 24, or 25 nucleotides in length, and the 3' strand can be 18, 19, 20, 21, 22, 23, 24, or 25 nucleotides in length. In some embodiments, the 5' strand and the 3' strand can be the same or different lengths. In some embodiments, the RNA duplex can include one or more mismatches. In alternate embodiments, the RNA duplex has no mismatches.

In any of the aspects provided immediately above directed to packageable cells or cell lines herein, that encode inhibitory RNA molecules directed against one or more RNA targets, the inhibitory RNA molecule can be a miRNA or an shRNA. In some embodiments, the inhibitory molecule can be a precursor of a miRNA, such as for example, a Pri-miRNA or a Pre-miRNA, or a precursor of an shRNA. In some embodiments, the one or more inhibitory RNA molecules can be an artificially derived miRNA or shRNA. In other embodiments, the inhibitory RNA molecule can be a dsRNA (either transcribed or artificially introduced) that is processed into an siRNA or the siRNA itself. In some embodiments, the inhibitory RNA molecule can be a miRNA or shRNA that has a sequence that is not found in nature, or has at least one functional segment that is not found in nature, or has a combination of functional segments that are not found in nature. In illustrative embodiments, at least one or all of the inhibitory RNA molecules are miR-155.

In any of the aspects provided immediately above directed to packageable cells or cell lines herein, that encode inhibitory RNA molecules directed against one or more RNA targets, the one or more inhibitory RNA molecule(s), in some embodiments, can comprises from 5' to 3' orientation: a 5' arm, a 5' stem, a loop, a 3' stem that is partially or fully complementary to said 5' stem, and a 3' arm. In some embodiments, at least one of the two or more inhibitory RNA molecules has this arrangement. In other embodiments, all of the two or more inhibitory RNA molecules have this arrangement. In some embodiments, the 5' stem can be 18, 19, 20, 21, 22, 23, 24 or 25 nucleotides in length. In some embodiments, the 3' stem can be 18, 19, 20, 21, 22, 23, 24, or 25 nucleotides in length. In some embodiments, the loop can be 3, 4,5, 6, 7, 8, 9, 10, 11, 12, 13, 14, 15, 16, 17, 18, 19, 20, 21, 22, 23, 24,2 5, 26, 27, 28, 29, 30, 31, 32, 33, 34, 35, 36, 37, 38, 39, or 40 nucleotides in length. In some embodiments, the 5' arm, 3' arm, or both, are derived from a naturally occurring miRNA. In some embodiments, the 5' arm, 3' arm, or both, are derived from a naturally occurring miRNA is selected from the group consisting of: miR-155, miR-30, miR-17-92, miR-122, and miR-21. In illustrative embodiments, the 5' arm, 3' arm, or both are derived from miR-155. In some embodiments, the 5' arm, 3' arm, or both are derived from *Mus musculus* miR-155 or *Homo sapiens* miR-155. In some embodiments, the 5' arm has the sequence set forth in SEQ ID NO:256 or is a functional variant thereof, such as, for example, a sequence that is the same length as SEQ ID NO:256, or 95%, 90%, 85%, 80%,75%, or 50% as long as SEQ ID NO: 256 or is 100 nucleotides or less, 95 nucleotides or less, 90 nucleotides or less, 85 nucleotides or less, 80 nucleotides or less, 75 nucleotides or less, 70 nucleotides or less, 65 nucleotides or less, 60 nucleotides or less, 55 nucleotides or less, 50 nucleotides or less, 45 nucleotides or less, 40 nucleotides or less, 35 nucleotides or less, 30 nucleotides or less, or 25 nucleotides or less; and is at least 50%, 55%, 60%, 65%, 70%, 75%, 80%, 85%, 90%, or 95% identical to SEQ ID NO:256. In some embodiments, the 3' arm has the sequence set forth in SEQ ID NO:260 or is a functional variant thereof, such as, for example, the same length as SEQ ID NO:260, or 95%, 90%, 85%, 80%,75%, or 50% as long as SEQ ID NO: 260 or is a sequence that is 100 nucleotides or less, 95 nucleotides or less, 90 nucleotides or less, 85 nucleotides or less, 80 nucleotides or less, 75 nucleotides or less, 70 nucleotides or less, 65 nucleotides or less, 60 nucleotides or less, 55 nucleotides or less, 50 nucleotides or less, 45 nucleotides or less, 40 nucleotides or less, 35 nucleotides or less, 30 nucleotides or less, or 25 nucleotides or less; and is at least 50%, 55%, 60%, 65%, 70%, 75%, 80%, 85%, 90%, or 95% identical to SEQ ID NO:260. In some embodiments, the 3' arm comprises nucleotides 221-283 of the *Mus musculus* BIC.

In another aspect, provided herein is a method for making replication incompetent recombinant retroviral particles, including: culturing a population of packaging cells to accumulate a first transactivator, wherein the packaging cells include the first transactivator expressed from a constitutive promoter, wherein the first transactivator is capable of binding a first ligand and a first inducible promoter for affecting expression of a nucleic acid sequence operably linked thereto in the presence versus absence of the first ligand, and wherein expression of a second transactivator is regulated by the first transactivator; incubating the population of packaging cells including accumulated first transactivator in the presence of the first ligand to accumulate the second transactivator, wherein the second transactivator is capable of binding a second ligand and a second inducible promoter for affecting expression of a nucleic acid sequence operably linked thereto in the presence versus absence of the second ligand; and incubating the population of packaging cells including accumulated second transactivator in the presence of the second ligand thereby inducing expression of retroviral polypeptides involved in viral packaging, such as, for example, a gag polypeptide, a pol polypeptide, and/or a pseudotyping element, and optionally other polypeptides that are believed to inhibit mammalian cell proliferation or survival that will become incorporated in or on the replication incompetent recombinant retroviral particle, thereby making the replication incompetent recombinant retroviral particle. In illustrative embodiments, a packageable RNA genome is encoded by a polynucleotide operably linked to a promoter, sometimes referred to for convenience as a "third" promoter wherein said third promoter is either constitutively active or inducible by either the first transactivator or, in illustrative embodiments, the second transactivator, thereby making the replication incompetent recombinant retroviral particle. The pseudotyping elements are typically capable of binding to a cell membrane of a target cell and facilitating fusion of the target cell membrane to the replication incompetent recombinant retroviral particle membrane. The pseudotyping elements can be any envelope proteins known in the art. In some embodiments, the envelope protein can be vesicular stomatitis virus envelope protein (VSV-G), feline endogenous virus (RD114) envelope protein, oncoretroviral amphotropic envelope protein, and/or oncoretroviral ecotropic envelope protein. A skilled artisan will appreciate many different transactivators, ligands, and inducible promoters can be used in the method for making a replication incompetent recombinant retroviral particle. Suitable transactivators, ligands, and inducible promoters are disclosed elsewhere herein, including above. A skilled artisan will further appreciate that the teachings hereinabove related to a retroviral packaging system aspect provided herein, apply to method of making replication incompetent recombinant retroviral particles aspects as well, and the reverse.

In some embodiments, the first transactivator can regulate expression of an element to control the nuclear export of transcripts containing a consensus sequence, such as an HIV Rev and the consensus sequence can be the Rev Response Element (RRE). In illustrative embodiments, the target cell is typically a T cell. In some embodiments, the HIV RREs and the polynucleotide region encoding HIV Rev can be replaced with HIV-2 RREs and a polynucleotide region encoding the HIV-2 Rev, respectively. In some embodiments, the HIV RREs and the polynucleotide region encoding HIV Rev can be replaced with SIV RREs and a polynucleotide region encoding the SIV Rev, respectively. In some embodiments, the HIV RREs and the polynucleotide region encoding HIV Rev can be replaced with RemREs and a polynucleotide region encoding a betaretrovirus Rem, respectively. In some embodiments, the HIV RREs and the polynucleotide region encoding HIV Rev can be replaced with a deltaretrovirus RexRRE and a polynucleotide region encoding a deltaretrovirus Rex, respectively. In some embodiments, a Rev-like protein is not required and the RREs can be replaced with cis-acting RNA elements, such as the constitutive transport element (CTE).

In some embodiments, the pseudotyping element is a viral envelope protein. The pseudotyping element typically includes a binding polypeptide and a fusogenic polypeptide for binding to and facilitating membrane fusion of viral and target cell membranes. In some embodiments, the pseudotyping element can be the feline endogenous virus (RD114) envelope protein, the oncoretroviral amphotropic envelope protein, the oncoretroviral ecotropic envelope protein, and/or vesicular stomatitis virus envelope protein (VSV-G). In illustrative embodiments, the pseudotyping element includes a binding polypeptide and a fusogenic polypeptide derived from different proteins, as discussed in further detail herein. For example, in an illustrative embodiment, especially where the target cell is a T cell and/or NK cell, the binding polypeptide can be a cytoplasmic domain deletion variant of a Measles Virus H polypeptide and the fusogenic polypeptide can be the cytoplasmic domain deletion variant of a Measles Virus F polypeptide. In some embodiments, the fusogenic polypeptide can include multiple elements expressed as one polypeptide. In some embodiments, the binding polypeptide and fusogenic polypeptide can be translated from the same transcript and translated from separate ribosome binding sites, or the polypeptide can be cleaved after translation using a peptide cleavage signal or a ribosomal skip sequence, as disclosed elsewhere herein, to generate the binding polypeptide and the fusogenic polypeptide. In some embodiments, the translation of the binding polypeptide and fusogenic polypeptide from separate ribosome binding sites results in a higher amount of the fusogenic polypeptide as compared to the binding polypeptide. In some embodiments, the ratio of the fusogenic polypeptide to the binding polypeptide is at least 2:1, at least 3:1, at least 4:1, at least 5:1, at least 6:1, at least 7:1, or at least 8:1.

In some embodiments, the first transactivator can regulate the expression of an activation element capable of binding to and activating a target cell, such as a T cell. In these embodiments, the activation element can include: a.) aa membrane-bound polypeptide capable of binding to and activating CD3: and/or b.) a membrane-bound polypeptide capable of binding to and activating CD28. In some embodiments, the membrane-bound polypeptide capable of binding to and activating CD28 is CD80, CD86, or functional fragments thereof. In some embodiments, the replication incompetent recombinant retroviral particle can include the activation element on a retroviral membrane and the retroviral RNA within a nucleocapsid, thereby making a replication incompetent recombinant retroviral particles.

In some embodiments, the second transactivator can regulate the expression of an RNA including from 5' to 3': a 5' long terminal repeat, or active truncated fragment thereof; a nucleic acid sequence encoding a retroviral cis-acting RNA packaging element; a nucleic acid sequence encoding a first target polypeptide and optional second target polypeptide, as non-limiting example, one or two engineered signaling polypeptides; a promoter that is active in a target cell; and a 3' long terminal repeat, or active truncated fragment thereof. In some embodiments, the RNA can include a cPPT/CTS element. In some embodiments, the RNA can include a primer binding site. In some embodiments, the retroviral cis-acting RNA packaging element can be HIV Psi. In some embodiments, the retroviral cis-acting RNA packaging element can be the Rev Response Element. In any of the embodiments disclosed herein, retroviral components on the RNA, including RRE and Psi, can be located in any position, as a skilled artisan will understand. The engineered signaling polypeptide in illustrative embodiments, is one or more of the engineered signaling polypeptides disclosed herein.

In some embodiments, the engineered signaling polypeptide can include a first lymphoproliferative element. Suitable lymphoproliferative elements are disclosed in other sections herein. In some illustrative embodiments, the lymphoproliferative element is an IL-7 receptor mutant fused to a recognition domain, such as an eTag. In some embodiments, the packageable RNA genome can further include a nucleic acid sequence encoding a second engineered polypeptide including a chimeric antigen receptor, encoding any CAR embodiment provided herein. For example, the second engineered polypeptide can include a first antigen-specific targeting region, a first transmembrane domain, and a first intracellular activating domain. Examples of antigen-specific targeting regions, transmembrane domains, and intracellular activating domains are disclosed elsewhere herein. In some embodiments where the target cell is a T cell, the promoter that is active in a target cell is active in a T cell, as disclosed elsewhere herein.

In some embodiments, the packageable RNA genome can further include a riboswitch, as discussed in other sections herein. In some embodiments, the nucleic acid sequence encoding the engineered signaling polypeptide can be in reverse orientation. In further embodiments, the packageable RNA genome can further include a riboswitch and, optionally, the riboswitch can be in reverse orientation. In any of the embodiments disclosed herein, a polynucleotide including any of the elements can include a primer binding site. In illustrative embodiments, transcription blockers or polyA sequences can be placed near genes to prevent or reduce unregulated transcription. In any of the embodiments disclosed herein, a nucleic acid sequence encoding Vpx can be on the second or an optional third transcriptional unit, or on an additional transcriptional unit that is operably linked to the first inducible promoter.

In some embodiments of the packaging system or methods for making replication incompetent recombinant retroviral particles aspects, the encoded RNA can include an intron, which can be transcribed, for example, from the same promoter for expressing the target polypeptide(s). Such intron can encode 1, 2, 3, or 4 miRNAs, in certain illustrative embodiments. In these and other embodiments of the packaging system or methods for making replication incompetent recombinant retroviral particles aspects, the packageable RNA genome is 11,000 KB or less and in some instances 10,000 KB or less in size.

In some embodiments, the first transactivator can affect the expression of one or more polypeptides that are non-toxic. In some embodiments, the second transactivator can affect the expression of one or more polypeptides that are toxic. For example, the first transactivator can induce expression of the retroviral proteins Rev and Vpx in addition to polypeptides that will be transported to the cell membrane of the packaging cell and the second transactivator can induce expression of the retroviral proteins GAG, POL, MV(Ed)-FΔ30, and either MV(Ed)-HΔ18 or MV(Ed)-HΔ24 and expression of the lentiviral genome. In some embodiments, the first transactivator can affect the expression of one or more polypeptides that are toxic and/or the second transactivator can affect the expression of one or more polypeptides that are non-toxic.

In another aspect, provided herein is a mammalian packaging cell, including: a.) a first transcriptional unit in the genome of the mammalian packaging cell, including a nucleic acid sequence encoding a first transactivator, wherein said first transcriptional unit is operably linked to a constitutive promoter and wherein said transactivator is capable of binding a first inducible promoter and affecting expression of a nucleic acid sequence operably linked thereto in the presence versus absence of a first ligand, and wherein said first transactivator is capable of binding said first ligand; b.) a second and optional third transcriptional unit in the genome of the mammalian packaging cell, including a nucleic acid sequence encoding a retroviral REV protein and a nucleic acid sequence encoding a second transactivator capable of binding a second inducible promoter and affecting expression of a nucleic acid sequence operably linked thereto in the presence versus absence of a second ligand, wherein the second transactivator is capable of binding the second ligand, and wherein the second and optional third transcriptional units are operably linked to the first inducible promoter; c.) a fourth and optional fifth transcriptional unit in the genome of the mammalian packaging cell, including a nucleic acid sequence encoding a retroviral gag polypeptide and a retroviral pol polypeptide, and a binding polypeptide and a fusogenic polypeptide that are capable of binding to and facilitating fusion of a target cell membrane and the retroviral membrane, wherein the fourth and optional fifth transcriptional unit are operably linked to the second inducible promoter; and d) a sixth transcriptional unit in the genome of the mammalian packaging cell, including , from 5' to 3', a 5' LTR, or active truncated fragment thereof, a nucleic acid sequence encoding a retroviral cis-acting RNA packaging element, a cPPT/CTS element, a reverse complement of a nucleic acid sequence encoding an engineered signaling polypeptide, an intron, a promoter that is active in a target cell, and a 3' LTR, or active truncated fragment thereof, wherein the sixth transcriptional unit is operably linked to the second inducible promoter.

In another aspect, provided herein is a method for making a replication incompetent recombinant retroviral particle, including: 1.) culturing a population of packaging cells to accumulate a first transactivator, wherein the packaging cells include: a.) a first transcriptional unit in the genome of the mammalian packaging cell, including a nucleic acid sequence encoding a first transactivator, wherein said first transcriptional unit is operably linked to a constitutive promoter and wherein said transactivator is capable of binding a first inducible promoter and affecting expression of a nucleic acid sequence operably linked thereto in the presence versus absence of a first ligand, and wherein said first transactivator is capable of binding said first ligand; b.) a second and optional third transcriptional unit in the genome of the mammalian packaging cell, including a nucleic acid sequence encoding a retroviral REV protein and a nucleic acid sequence encoding a second transactivator capable of binding a second inducible promoter and affecting expression of a nucleic acid sequence operably linked thereto in the presence versus absence of a second ligand, wherein the second transactivator is capable of binding the second ligand, and wherein the second and optional third transcriptional units are operably linked to the first inducible promoter; c.) a fourth and optional fifth transcriptional unit in the genome of the mammalian packaging cell, including a nucleic acid sequence encoding a retroviral gag polypeptide and a retroviral pol polypeptide, and a binding polypeptide and a fusogenic polypeptide that are capable of binding to and facilitating fusion of the retroviral membrane with a target cell membrane, wherein the fourth and optional fifth transcriptional unit are operably linked to the second inducible promoter; and d.) a sixth transcriptional unit in the genome of the mammalian packaging cell, including from 5' to 3', a 5' LTR, or active truncated fragment thereof, a primer binding site (PBS), a nucleic acid sequence encoding a retroviral cis-acting RNA packaging element, a cPPT/CTS element, a reverse complement of a nucleic acid sequence encoding an engineered signaling polypeptide, an intron, a target cell promoter that is active in a target cell, a 3' LTR, or active truncated fragment thereof, wherein the fifth transcriptional unit is operably linked to the second inducible promoter; and 2.) incubating the population of packaging cells including the first transactivator in the presence of the first ligand to accumulate the second transactivator and the retroviral REV protein; and 3.) incubating the population of packaging cells including the second transactivator and the retroviral REV protein in the presence of the second ligand thereby inducing expression of the retroviral gag polypeptide, the retroviral pol polypeptide, the binding polypeptide, the fusogenic polypeptide, and a retroviral RNA including from 5' to 3', a 5' LTR, or active fragment thereof, the PBS, the retroviral cis-acting RNA packaging element, the reverse complement of the nucleic acid sequence encoding the engineered signaling polypeptide, the target cell promoter, and a 3' LTR, or active truncated fragment thereof, wherein replication incompetent recombinant retroviral particles are formed and release from the packaging cells, and wherein the replication incompetent recombinant retroviral particles include the binding polypeptide and/or the fusogenic polypeptide on a retroviral membrane and the retroviral RNA within a nucleocapsid, thereby making replication incompetent recombinant retroviral particles.

In one aspect provided herein, the retroviral packaging system can include a mammalian cell including: 1.) a first transactivator expressed from a constitutive promoter and capable of binding a first ligand and a first inducible promoter for affecting expression of a nucleic acid sequence operably linked thereto in the presence versus absence of the first ligand; 2.) a second transactivator capable of binding a second ligand and a second inducible promoter and affecting expression of a nucleic acid sequence operably linked thereto in the presence versus absence of a second ligand; and 3.) a packageable RNA genome for a retroviral particle, wherein the first transactivator regulates expression of the second transactivator, HIV REV, an IL7 GPI DAF, and an activation element, and wherein the second transactivator regulates expression of a gag polypeptide, a pol polypeptide, a retroviral cis-acting RNA packaging element, and one or more envelope polypeptides. In illustrative embodiments, the first transactivator can be an FRB domain fused to a p65 activation domain and one or more FKBP domains fused to a ZFHD1 DNA binding domain, the first ligand can be rapamycin, and the first inducible promoter can be one or more ZFHD1 binding sites. In illustrative embodiments, the second transactivator can be an rtTA protein, the second ligand can be tetracycline or doxycycline, and the second inducible promoter can be a TRE promoter or a bi-directional TRE promoter. In illustrative embodiments, the retroviral cis-acting RNA packaging element can be HIV Psi. In illustrative embodiments, the one or more envelope proteins include the cytoplasmic domain deletion variants of F and H polypeptides of a Measles Virus. In illustrative embodiments, transcription blockers or polyA sequences can be placed near genes to prevent or reduce unregulated transcription. In some embodiments, a rapamycin-doxycycline inducible lentiviral genome with riboswitch can be used (SEQ ID NO:83). In some embodiments, a rapamycin-doxycycline inducible GAG POL ENV can be used (SEQ ID NO:84). In some embodiments, a rapamycin-inducible TET activator can be used (SEQ ID NO:85). In some embodiments, a rapamycin inducer inducible REV srcVpx can be used (SEQ ID NO:86).

Some aspects of the present disclosure include or are cells, in illustrative examples, mammalian cells, that are used as packaging cells to make replication incompetent recombinant retroviral particles, such as lentiviruses, for transduction of T cells and/or NK cells. Any of a wide variety of cells can be selected for *in vitro* production of a virus or virus particle, such as a redirected recombinant retroviral particle. Eukaryotic cells are typically used, particularly mammalian cells including human, simian, canine, feline, equine and rodent cells. In illustrative examples, the cells are human cells. In further illustrative embodiments, the cells reproduce indefinitely, and are therefore immortal. Examples of cells that can be advantageously used in the present invention include NIH 3T3 cells, COS cells, Madin-Darby canine kidney cells, human embryonic 293T cells and any cells derived from such cells, such as gpnlslacZ φNX cells, which are derived from 293T cells. Highly transfectable cells, such as human embryonic kidney 293T cells, can be used. By "highly transfectable" it is meant that at least about 50%, more preferably at least about 70% and most preferably at least about 80% of the cells can express the genes of the introduced DNA.

Suitable mammalian cells include primary cells and immortalized cell lines. Suitable mammalian cell lines include human cell lines, non-human primate cell lines, rodent (e.g., mouse, rat) cell lines, and the like. Suitable mammalian cell lines include, but are not limited to, HeLa cells (e.g., American Type Culture Collection (ATCC) No. CCL-2), CHO cells (e.g., ATCC Nos. CRL9618, CCL61, CRL9096), 293 cells (e.g., ATCC No. CRL-1573), Vero cells, NIH 3T3 cells (e.g., ATCC No. CRL-1658), Huh-7 cells, BHK cells (e.g., ATCC No. CCLlO), PC12 cells (ATCC No. CRL1721), COS cells, COS-7 cells (ATCC No. CRL1651), RATl cells, mouse L cells (ATCC No. CCLI.3), human embryonic kidney (HEK) cells (ATCC No. CRL1573), HLHepG2 cells, Hut-78, Jurkat, HL-60, NK cell lines (e.g., NKL, NK92, and YTS), and the like.

In any of the embodiments disclosed herein, the methods of making a replication incompetent recombinant retroviral particle can include growing a mammalian packaging cells to 50%, 60%, 70%, 80%, 90% or 95% confluence or confluence or to 25%, 30%, 40%, 50%, 60%, 70%, 80%, 90% or 95% peak cell density or peak cell density and then splitting or diluting the cells. In some embodiments, a stirred tank reactor can be used to grow the cells. In some embodiments, the cells can be split at least about 1:2, 1:3, 1:4, 1:5, 1:6, 1:7, 1:8, 1:9, 1:10, 1:12, 1:15, or 1:20 using methods a skilled artisan will understand. In some embodiments, the cells can be diluted to 25%, 30%, 40%, 50%, 60%, 70%, 80%, 90% or 95% peak cell density. In some embodiments, after splitting or diluting the cells the cells can be grown for 1, 2, 3, 4, 5, 6, 7, 8, 10, or 16 hours or 1, 2, 3, 4, 5, 6, or 7 days before adding the first ligand. In some embodiments, the cells are grown in the presence of the first ligand for 1, 2, 3, 4, 5, 6, 7, 8, 9, 10, 11, 12, 13, 14, 21, or 28 days in the presence of the first ligand, which in illustrative embodiments can be rapamycin or a rapalog. In some embodiments, the second ligand can be added and the cells can be grown for at least 1, 2, 3, 4, 5, 6, 7, 8, 9, 10, 11, 12, 13, 14, 21, or 28 days which in illustrative embodiments can be tetracycline or doxycyline. Conditions for culturing will depend on the cells and ligands used and the methods are known in the art. A specific example of conditions for culturing and inducing HEK293S cells is shown in Example 8.

As disclosed herein, replication incompetent recombinant retroviral particles are a common tool for gene delivery (Miller, Nature (1992) 357:455-460). The ability of replication incompetent recombinant retroviral particles to deliver an unrearranged nucleic acid sequence into a broad range of rodent, primate and human somatic cells makes replication incompetent recombinant retroviral particles well suited for transferring genes to a cell. In some embodiments, the replication incompetent recombinant retroviral particles can be derived from the Alpharetrovirus genus, the Betaretrovirus genus, the Gammaretrovirus genus, the Deltaretrovirus genus, the Epsilonretrovirus genus, the Lentivirus genus, or the Spumavirus genus. There are many retroviruses suitable for use in the methods disclosed herein. For example, murine leukemia virus (MLV), human immunodeficiency virus (HIV), equine infectious anaemia virus (EIAV), mouse mammary tumor virus (MMTV), Rous sarcoma virus (RSV), Fujinami sarcoma virus (FuSV), Moloney murine leukemia virus (Mo-MLV), FBR murine osteosarcoma virus (FBR MSV), Moloney murine sarcoma virus (Mo-MSV), Abelson murine leukemia virus (A-MLV), Avian myelocytomatosis virus-29 (MC29), and Avian erythroblastosis virus (AEV) can be used. A detailed list of retroviruses may be found in Coffin et al ("Retroviruses" 1997 Cold Spring Harbor Laboratory Press Eds: J M Coffin, S M Hughes, H E Varmus pp 758-763). Details on the genomic structure of some retroviruses may be found in the art. By way of example, details on HIV may be found from the NCBI Genbank (i.e. Genome Accession No. AF033819).

In illustrative embodiments, the replication incompetent recombinant retroviral particles can be derived from the Lentivirus genus. In some embodiments, the replication incompetent recombinant retroviral particles can be derived from HIV, SIV, or FIV. In further illustrative embodiments, the replication incompetent recombinant retroviral particles can be derived from the human immunodeficiency virus (HIV) in the Lentivirus genus. Lentiviruses are complex retroviruses which, in addition to the common retroviral genes gag, pol and env, contain other genes with regulatory or structural function. The higher complexity enables the lentivirus to modulate the life cycle thereof, as in the course of latent infection. A typical lentivirus is the human immunodeficiency virus (HIV), the etiologic agent of AIDS. *In vivo,* HIV can infect terminally differentiated cells that rarely divide, such as lymphocytes and macrophages.

In illustrative embodiments, replication incompetent recombinant retroviral particles provided herein contain Vpx polypeptide. Vpx polypeptide can be expressed in a packaging cell line, after integration of a Vpx coding nucleic acid in its genome, for example as a cell membrane bound protein that gets incorporated into a retrovirus membrane (Durand et al., J. Virol. (2013) 87: 234-242). A retroviral membrane bound Vpx can be constructed with a processing sequence for a viral protease such that free Vpx is released once incorporated in a viral particle. Such an example of a Vpx fusion with this functionality is Src-Flag-Vpx, which includes a membrane-targeting domain (MGSSKSKPKDP) (SEQ ID NO:227) of the first 11 amino acids of c-Src followed by a viral protease cleavage domain KARVLAEA (SEQ ID NO:228) followed by Flag-tagged Vpx.

Not to be limited by theory, Vpx polypeptide aids in transduction of resting cells by stimulating the efficiency of the process of reverse transcription by degrading the restriction factor SAMHD1. Accordingly, it is believed that in the methods provided herein where Vpx is present in a replication incompetent recombinant retroviral particles used to transduce T cells and/or NK cells, Vpx is released into the cytoplasm of a resting T cell or a resting NK cell upon transduction of the cell by a replication incompetent recombinant retroviral particle that contains Vpx. Vpx then degrades SAMHD1, which causes an increase in free dNTPs, which in turn, stimulates reverse transcription of the retroviral genome.

In some embodiments, replication incompetent recombinant retroviral particles provided herein comprise and/or contain Vpu polypeptide. Vpu polypeptide can be expressed from a plasmid or in a packaging cell line, after integration of a Vpu coding nucleic acid in its genome, for example, as a viral membrane protein that gets incorporated into the retrovirus membrane. A recombinant form of Vpu, with its sequence codon optimized for expression in humans, can be constructed and expressed such that free Vpu can incorporate into the viral particles. Vpu is an accessory protein found in HIV-1 and some HIV-1-related simian immunodeficiency virus (SIV) isolates, such as SIVcpz, SIVgsn, and SIVmon, but not in HIV-2 or the majority of SIV isolates. The Vpu protein participates in the downregulation of CD4, and antagonism of tetherin for particle release. More importantly, Vpu shares structural similarities with viroporins, and particularly the viroporin protein M2 from Influenza A virus (IAV), As such, Vpu has been shown to form cation-selective ion channels and permeabilize membranes, in various models. It has been shown that IAV-M2 helps acidify the particles and thus favors an early release from the endosomal pathway used for entry, reducing the amount of dead-end transit products.

Example 20 herein demonstrates that the transduction of resting PBMCs by retroviral particles is enhanced by the presence of Vpu in the retroviral particles. Not to be limited by theory, Vpu polypeptide aids in transduction of resting cells by accelerating the acidification of the lentiviral pseudotypes, allowing more capsids to reach the cytoplasm faster. Acidification of virus interior, leads to weakening of electrostatic interaction between viral structural proteins and thus facilitates dissociation of the capsid and viral ribonucleoprotein (RNP) complexes. Accordingly, it is believed that in the methods provided herein where Vpu is present in a replication incompetent recombinant retroviral particle membrane used to transduce T cells and/or NK cells, Vpu accelerates the acidification of the lentiviral particles in the endosomal pathway, and favors the release of capsids into the cytoplasm of resting T cells or resting NK cells upon transduction of the cells by a replication incompetent recombinant retroviral particle that contains Vpu. Accordingly, in some embodiments therein a Vpu polypeptide is included that comprises or is a fragment of Vpu that retains the ability to promote transduction of resting PBMCs and in some embodiments, resting T cells. The fragment can comprise a fragment of 75, 80, 85, 90, 95, 96, 97, 98, 99 or 100% of Vpu that has at least 75, 80, 85, 90, 95, 98, 99, or 100% identity to a wildtype Vpu. In some embodiments, a Vpu polypeptide includes a Vpu fragment that retains the ability to accelerate acidification of a lentiviral particle in the endosomal pathway.

### RETROVIRAL GENOME SIZE

In the methods and compositions provided herein, the recombinant retroviral genomes, in non-limiting illustrative examples, lentiviral genomes, have a limitation to the number of polynucleotides that can be packaged into the viral particle. In some embodiments provided herein, the polypeptides encoded by the polynucleotide encoding region can be truncations or other deletions that retain a functional activity such that the polynucleotide encoding region is encoded by less nucleotides than the polynucleotide encoding region for the wild-type polypeptide. In some embodiments, the polypeptides encoded by the polynucleotide encoding region can be fusion polypeptides that can be expressed from one promoter. In some embodiments, the fusion polypeptide can have a cleavage signal to generate two or more functional polypeptides from one fusion polypeptide and one promoter. Furthermore, some functions that are not required after initial *ex vivo* transduction are not included in the retroviral genome, but rather are present on the surface of the replication incompetent recombinant retroviral particles via the packaging cell membrane. These various strategies are used herein to maximize the functional elements that are packaged within the replication incompetent recombinant retroviral particles.

In some embodiments, the recombinant retroviral genome to be packaged can be between 1,000, 2,000, 3,000, 4,000, 5,000, 6,000, 7,000, and 8,000 nucleotides on the low end of the range and 2,000, 3,000, 4,000, 5,000, 6,000, 7,000, 8,000, 9,000, 10,000, and 11,000 nucleotides on the high end of the range. The retroviral genome to be packaged includes one or more polynucleotide regions encoding a first and second engineering signaling polypeptide as disclosed in detail herein. In some embodiments, the recombinant retroviral genome to be packaged can be less than 5,000, 6,000, 7,000, 8,000, 9,000, 10,000, or 11,000 nucleotides. Functions discussed elsewhere herein that can be packaged include required retroviral sequences for retroviral assembly and packaging, such as a retroviral rev, gag, and pol coding regions, as well as a 5' LTR and a 3' LTR, or an active truncated fragment thereof, a nucleic acid sequence encoding a retroviral cis-acting RNA packaging element, and a cPPT/CTS element. Furthermore, in illustrative embodiments a replication incompetent recombinant retroviral particle herein can include any one or more or all of the following, in some embodiments in reverse orientation of these retroviral functional regions: one or more polynucleotide regions encoding a first and second engineering signaling polypeptide, at least one of which includes at least one lymphoproliferative element and can further include an ASTR; a second engineered signaling polypeptide that can include a chimeric antigen receptor; a control element, such as a riboswitch, which typically regulates expression of the first and/or the second engineering signaling polypeptide; a recognition domain, an intron, a promoter that is active in a target cell, such as a T cell, a 2A cleavage signal and/or an IRES.

### RECOMBINANT RETROVIRAL PARTICLES

Recombinant retroviral particles are disclosed in methods and compositions provided herein, for example, to transduce T cells and/or NK cells to make genetically modified T cells and/or NK cells. Typically, the recombinant retroviral particles included in aspects provided herein, are replication incompetent, meaning that a recombinant retroviral particle cannot replicate once it leaves the packaging cell. In illustrative embodiments, the recombinant retroviral particles are lentiviral particles.

Provided herein in some aspects are replication incompetent recombinant retroviral particles for use in transducing cells, typically lymphocytes and illustrative embodiments T cells and/or NK cells. The replication incompetent recombinant retroviral particles can include any of the pseudotyping elements discussed elsewhere herein. In some embodiments, the replication incompetent recombinant retroviral particles can include any of the activation elements discussed elsewhere herein. In one aspect, provided herein is a replication incompetent recombinant retroviral particle including a polynucleotide including: A. one or more transcriptional units operatively linked to a promoter active in T cells and/or NK cells, wherein the one or more transcriptional units encode a chimeric antigen receptor (CAR); and B. a pseudotyping element and a T cell activation element on its surface, wherein the T cell activation element is not encoded by a polynucleotide in the replication incompetent recombinant retroviral particle. In some embodiments, the T cell activation element can be any of the activation elements discussed elsewhere herein. In illustrative embodiments, the T cell activation element can be anti-CD3 scFvFc. In another aspect, provided herein is a replication incompetent recombinant retroviral particle, including a polynucleotide including one or more transcriptional units operatively linked to a promoter active in T cells and/or NK cells, wherein the one or more transcriptional units encode a first polypeptide including a chimeric antigen receptor (CAR) and a second polypeptide including a lymphoproliferative element. In some embodiments, the lymphoproliferative element can be a chimeric lymphoproliferative element. In illustrative embodiments, the lymphoproliferative element does not comprise IL-7 tethered to the IL-7 receptor alpha chain or a fragment thereof. In some embodiments the lymphoproliferative element does not comprise IL-15 tethered to the IL-2/IL-15 receptor beta chain.

In some aspects, provided herein is a replication incompetent recombinant retroviral particle, comprising a polynucleotide comprising one or more transcriptional units operatively linked to a promoter active in T cells and/or NK cells, wherein the one or more transcriptional units encode a first polypeptide comprising a chimeric antigen receptor (CAR) and a second polypeptide comprising a chimeric lymphoproliferative element, for example a constitutively active chimeric lymphoproliferative element. In illustrative embodiments, the chimeric lymphoproliferative element does not comprise a cytokine tethered to its cognate receptor or tethered to a fragment of its cognate receptor.

Provided herein in some aspects, is a recombinant retroviral particle that includes (i) a pseudotyping element capable of binding to a T cell and/or NK cell and facilitating membrane fusion of the recombinant retroviral particle thereto; (ii) a polynucleotide having one or more transcriptional units operatively linked to a promoter active in T cells and/or NK cells, wherein the one or more transcriptional units encode a first engineered signaling polypeptide having a chimeric antigen receptor that includes an antigen-specific targeting region, a transmembrane domain, and an intracellular activating domain, and a second engineered signaling polypeptide that includes at least one lymphoproliferative element; wherein expression of the first engineered signaling polypeptide and/or the second engineered signaling polypeptide are regulated by an *in vivo* control element; and (iii) an activation element on its surface, wherein the activation element is capable of binding to a T cell and/or NK cell and is not encoded by a polynucleotide in the recombinant retroviral particle. In some embodiments, the promoter active in T cells and/or NK cells is not active in the packaging cell line or is only active in the packaging cell line in an inducible manner. In any of the embodiments disclosed herein, either of the first and second engineered signaling polypeptides can have a chimeric antigen receptor and the other engineered signaling polypeptide can have at least one lymphoproliferative element.

Various elements and combinations of elements that are included in replication incompetent, recombinant retroviral particles are provided throughout this disclosure, such as, for example, pseudotyping elements, activation elements, and membrane bound cytokines, as well as nucleic acid sequences that are included in a genome of a replication incompetent, recombinant retroviral particle such as, but not limited to, a nucleic acid encoding a CAR; a nucleic acid encoding a lymphoproliferative element; a nucleic acid encoding a control element, such as a riboswitch; a promoter, especially a promoter that is constitutively active or inducible in a T cell; and a nucleic acid encoding an inhibitory RNA molecule. Furthermore, various aspects provided herein, such as methods of making recombinant retroviral particles, methods for performing adoptive cell therapy, and methods for transducing T cells, produce and/or include replication incompetent, recombinant retroviral particles. Replication incompetent recombinant retroviruses that are produced and/or included in such methods themselves form separate disclosures as replication incompetent, recombinant retroviral particle compositions, which can be in an isolated form. Such compositions can be in dried down (e.g. lyophilized) form or can be in a suitable solution or medium known in the art for storage and use of retroviral particles.

Accordingly, as a non-limiting example, provided herein in another aspect, is a replication incompetent recombinant retroviral particle having in its genome a polynucleotide having one or more nucleic acid sequences operatively linked to a promoter active in T cells and/or NK cells that in some instances, includes a first nucleic acid sequence that encodes one or more (e.g. two or more) inhibitory RNA molecules directed against one or more RNA targets and a second nucleic acid sequence that encodes a chimeric antigen receptor, or CAR, as described herein. In other embodiments, a third nucleic acid sequence is present that encodes at least one lymphoproliferative element described previously herein that is not an inhibitory RNA molecule. In certain embodiments, the polynucleotide incudes one or more riboswitches as presented herein, operably linked to the first nucleic acid sequence, the second nucleic acid sequence, and/or the third nucleic acid sequence, if present. In such a construct, expression of one or more inhibitory RNAs, the CAR, and/or one or more lymphoproliferative elements that are not inhibitory RNAs is controlled by the riboswitch. In some embodiments, two to 10 inhibitory RNA molecules are encoded by the first nucleic acid sequence. In further embodiments, two to six inhibitory RNA molecules are encoded by the first nucleic acid sequence. In illustrative embodiments, 4 inhibitory RNA molecules are encoded by the first nucleic acid sequence. In some embodiments, the first nucleic acid sequence encodes one or more inhibitory RNA molecules and is located within an intron. In certain embodiments, the intron includes all or a portion of a promoter. The promoter can be a Pol I, Pol II, or Pol III promoter. In some illustrative embodiments, the promoter is a Pol II promoter. In some embodiments, the intron is adjacent to and downstream of the promoter active in a T cell and/or NK cell. In some embodiments, the intron is EF1-α intron A.

Recombinant retroviral particle embodiments herein include those wherein the retroviral particle comprises a genome that includes one or more nucleic acids encoding one or more inhibitory RNA molecules. Various alternative embodiments of such nucleic acids that encode inhibitory RNA molecules that can be included in a genome of a retroviral particle, including combinations of such nucleic acids with other nucleic acids that encode a CAR or a lymphoproliferative element other than an inhibitory RNA molecule, are included for example, in the inhibitory RNA section provided herein, as well as in various other paragraphs that combine these embodiments. Furthermore, various alternatives of such replication incompetent recombinant retroviruses can be identified by exemplary nucleic acids that are disclosed within packaging cell line aspects disclosed herein. A skilled artisan will recognize that disclosure in this section of a recombinant retroviral particle that includes a genome that encodes one or more (e.g. two or more) inhibitory RNA molecules, can be combined with various alternatives for such nucleic acids encoding inhibitory RNA molecules provided in other sections herein. Furthermore, a skilled artisan will recognize that such nucleic acids encoding one or more inhibitory RNA molecules can be combined with various other functional nucleic acid elements provided herein, as for example, disclosed in the section herein that focuses on inhibitory RNA molecules and nucleic acid encoding these molecules. In addition, the various embodiments of specific inhibitory RNA molecules provided herein in other sections can be used in recombinant retroviral particle aspects of the present disclosure.

Necessary elements of recombinant retroviral vectors, such as lentiviral vectors, are known in the art. These elements are included in the packaging cell line section and in details for making replication incompetent, recombinant retroviral particles provided in the Examples section. For example, lentiviral particles typically include packaging elements REV, GAG and POL, which can be delivered to packaging cell lines via one or more packaging plasmids, a pseudotyping element, various examples which are provided herein, which can be delivered to a packaging cell line via a pseudotyping plasmid, and a genome, which is produced by a polynucleotide that is delivered to a host cell via a transfer plasmid. This polynucleotide typically includes the viral LTRs and a psi packaging signal. The 5' LTR can be a chimeric 5' LTR fused to a heterologous promoter, which includes 5' LTRs that are not dependent on Tat transactivation. The transfer plasmid can be self-inactivating, for example, by removing a U3 region of the 3' LTR. In some non-limiting embodiments, Vpu, such as a polypeptide comprising Vpu (sometimes called a "Vpu polypeptide" herein) including but not limted to, Src-FLAG-Vpu, is packaged within the retroviral particle for any composition or method aspect and embodiment provided herein that includes a retroviral particle. In some non-limiting embodiments, Vpx, such as Src-FLAG-Vpx, is packaged within the retroviral particle. Not to be limited by theory, upon transduction of a T cells, Vpx enters the cytosol of the cells and promotes the degradation of SAMHD1, resulting in an increased pool of cytoplasmic dNTPs available for reverse transcription. In some non-limiting embodiments, Vpu and Vpx is packaged within the retroviral particle for any composition or method aspect and embodiment that includes a retroviral particle provided herein.

Retroviral particles (e.g. lentiviral particles) as described herein are in illustrative embodiments, replication incompetent, especially for safety reasons for embodiments that include introducing cells transduced with such retroviral particles into a subject. When replication incompetent retroviral particles are used to transduce a cell, retroviral particles are not produced from the transduced cell. Modifications to the retroviral genome are known in the art to assure that retroviral particles that include the genome are replication incompetent. However, it will be understood that in some embodiments for any of the aspects provided herein, replication competent recombinant retroviral particles can be used.

A skilled artisan will recognize that the functional elements discussed herein can be delivered to packaging cells and/or to T cells using different types of vectors, such as expression vectors. Illustrative aspects as described herein utilize retroviral vectors, and in some particularly illustrative embodiments lentiviral vectors. Other suitable expression vectors can be used to achieve certain embodiments herein. Such expression vectors include, but are not limited to, viral vectors (e.g. viral vectors based on vaccinia virus; poliovirus; adenovirus (see, e.g., Li et al., Invest Opthalmol Vis Sci 35:2543 2549, 1994; Borras et al., Gene Ther 6:515 524, 1999; Li and Davidson, PNAS 92:7700 7704, 1995; Sakamoto et al., H Gene Ther 5:1088 1097, 1999; WO 94/12649, WO 93/03769; WO 93/19191; WO 94/28938; WO 95/11984 and WO 95/00655); adeno-associated virus (see, e.g., Ali et al., Hum Gene Ther 9:81 86, 1998, Flannery et al., PNAS 94:6916 6921, 1997; Bennett et al., Invest Opthalmol Vis Sci 38:2857 2863, 1997; Jomary et al., Gene Ther 4:683 690, 1997, Rolling et al., Hum Gene Ther 10:641 648, 1999; Ali et al., Hum Mol Genet 5:591 594, 1996; Srivastava in WO 93/09239, Samulski et al., J. Vir. (1989) 63:3822-3828; Mendelson et al., Virol. (1988) 166:154-165; and Flotte et al., PNAS (1993) 90: 10613-10617); SV40; herpes simplex virus; or a retroviral vector (e.g., Murine Leukemia Virus, spleen necrosis virus, and vectors derived from retroviruses such as Rous Sarcoma Virus, Harvey Sarcoma Virus, avian leukosis virus, human immunodeficiency virus, myeloproliferative sarcoma virus, and mammary tumor virus), for example a gamma retrovirus; or human immunodeficiency virus (see, e.g., Miyoshi *et al., PNAS* 94:10319 23, 1997; Takahashi *et al., J Virol* 73:7812 7816, 1999); and the like.

In illustrative embodiments, a retroviral particle is a lentiviral particle. Such retroviral particle typically includes a retroviral genome within a capsid which is located within a viral envelope.

In some embodiments, DNA-containing viral particles are utilized instead of recombinant retroviral particles. Such viral particles can be adenoviruses, adeno-associated viruses, herpesviruses, cytomegaloviruses, poxviruses, avipox viruses, influenza viruses, vesicular stomatitis virus (VSV), or Sindbis virus. A skilled artisan will appreciate how to modify the methods disclosed herein for use with different viruses and retroviruses, or retroviral particles. Where viral particles are used that include a DNA genome, a skilled artisan will appreciate that functional units can be included in such genomes to induce integration of all or a portion of the DNA genome of the viral particle into the genome of a T cell transduced with such virus.

In some embodiments, the HIV RREs and the polynucleotide region encoding HIV Rev can be replaced with N-terminal RGG box RNA binding motifs and a polynucleotide region encoding ICP27. In some embodiments, the polynucleotide region encoding HIV Rev can be replaced with one or more polynucleotide regions encoding adenovirus E1B 55-kDa and E4 Orf6.

Provided herein in one aspect is a container, such as a commercial container or package, or a kit comprising the same, comprising isolated replication incompetent recombinant retroviral particles according to any of the replication incompetent recombinant retroviral particle aspects provided herein. Furthermore, provided herein in another aspect is a container, such as a commercial container or package, or a kit comprising the same, comprising isolated packaging cells, in illustrative embodiments isolated packaging cells from a packaging cell line, according to any of the packaging cell and/or packaging cell line aspects provided herein. In some embodiments, the kit includes additional containers that include additional reagents such as buffers or reagents used in methods provided herein. Furthermore provided herein in certain aspects are use of any replication incompetent recombinant retroviral particle provided herein in any aspect, in the manufacture of a kit for genetically modifying a T cell or NK cell according to any aspect provided herein. Furthermore provided herein in certain aspects are use of any packaging cell or packaging cell line provided herein in any aspect, in the manufacture of a kit for producing the replication incompetent recombinant retroviral particles according to any aspect provided herein.

Provided herein in one aspect is a commercial container containing a replication incompetent recombinant retroviral particle and instructions for the use thereof to treat tumor growth in a subject, wherein the replication incompetent recombinant retroviral particle comprises in its genome a polynucleotide comprising one or more nucleic acid sequences operatively linked to a promoter active in T cells and/or NK cells. In some embodiments, a nucleic acid sequence of the one or more nucleic acid sequences can encode a chimeric antigen receptor (CAR) comprising an antigen-specific targeting region (ASTR), a transmembrane domain, and an intracellular activating domain. In some embodiments, a nucleic acid sequence of the one or more nucleic acid sequences can encode two or more inhibitory RNA molecules directed against one or more RNA targets.

The container that contains the recombinant retroviral particles can be a tube, vial, well of a plate, or other vessel for storage of a recombinant retroviral particle. The kit can include two or more containers wherein a second or other container can include, for example, a solution or media for transduction of T cells and/or NK cells, and/or a the second or other container can include a pH-modulating pharmacologic agent. Any of these containers can be of industrial strength and grade. The replication incompetent recombinant retroviral particle in such aspects that include a kit and a nucleic acid encoding an inhibitory RNA molecule, can be any of the embodiments for such replication incompetent recombinant retroviral particles provided herein, which include any of the embodiments for inhibitory RNA provided herein.

In another aspect, provided herein is the use of a replication incompetent recombinant retroviral particle in the manufacture of a kit for genetically modifying a T cell or NK cell, wherein the use of the kit includes: contacting the T cell or NK cell ex vivo with the replication incompetent recombinant retroviral particle, wherein the replication incompetent recombinant retroviral particle includes a pseudotyping element on a surface and a T cell activation element on the surface, wherein said contacting facilitates transduction of the T cell or NK cell by the replication incompetent recombinant retroviral particle, thereby producing a genetically modified T cell or NK cell. In some embodiments, the T cell or NK cell can be from a subject. In some embodiments, the T cell activation element can be membrane-bound. In some embodiments, the contacting can be performed for between 1, 2, 3, 4, 5, 6, 7, or 8 hours on the low end of the range and 4, 5, 6, 7, 8, 10, 12, 15, 18, 21, and 24 hours on the high end of the range, for example, between 1 and 12 hours. The replication incompetent recombinant retroviral particle for use in the manufacture of a kit can include any of the aspects, embodiments, or subembodiments discussed elsewhere herein.

### GENETICALLY MODIFIED T CELLS AND NK CELLS

In embodiments of the methods and compositions herein, genetically modified lymphocytes are produced. Such genetically modified lymphocytes can be transduced lymphocytes. In one aspect, provided herein is a genetically modified T cell or NK cell wherein the T cell or NK cell has been genetically modified to express a first engineered signaling polypeptide. In some embodiments, the first engineered signaling polypeptide can be a lymphoproliferative element or a CAR that includes an antigen-specific targeting region (ASTR), a transmembrane domain, and an intracellular activating domain. In some embodiments, the T cell or NK cell can further include a second engineered signaling polypeptide that can be a CAR or a lymphoproliferative element. In some embodiments, the lymphoproliferative element can be a chimeric lymphoproliferative element. In some embodiments, the T cell or NK cell can further include a pseudotyping element on a surface. In some embodiments, the T cell or NK cell can further include an activation element on a surface. The CAR, lymphoproliferative element, pseudotyping element, and activation element of the genetically modified T cell or NK cell can include any of the aspects, embodiments, or subembodiments disclosed herein. In illustrative embodiments, the activation element can be anti-CD3 scFvFc.

Some aspects provided herein include a genetically modified T cell or NK cell made by transducing resting T cells and/or resting NK cells according to a method comprising contacting the resting T cells and/or resting NK cells ex vivo, with replication incompetent recombinant retroviral particles, wherein the replication incompetent recombinant retroviral particles comprise a pseudotyping element on their surface and a membrane-bound T cell activation element on their surface, wherein said contacting facilitates transduction of the resting T cells and/or NK cells by the replication incompetent recombinant retroviral particles, thereby producing genetically modified T cells and/or NK cells, and wherein the contacting is performed for between 1, 2, 3, 4 or 6 hours on the low end of the range and 4, 6, 8, 10, 12, 18, 20, or 24 hours on the high end of the range, for example between 1 hour and 12 hours.

In some embodiments, genetically modified lymphocytes are lymphocytes such as T cells or NK cells that have been genetically modified to express a first engineered signaling polypeptide comprising at least one lymphoproliferative element and/or a second engineered signaling polypeptide comprising a chimeric antigen receptor, which includes an antigen-specific targeting region (ASTR), a transmembrane domain, and an intracellular activating domain. In some embodiments of any of the aspects herein, the NK cells are NKT cells. NKT cells are a subset of T cells that express CD3 and typically coexpress an αβ T-cell receptor, but also express a variety of molecular markers that are typically associated with NK cells (such as NK1.1 or CD56).

Genetically modified lymphocytes of the present disclosure possess a heterologous nucleic acid sequence that has been introduced into the lymphocyte by a recombinant DNA method. For example, the heterologous sequence in illustrative embodiments is inserted into the lymphocyte during a method for transducing the lymphocyte provided herein. The heterologous nucleic acid is found within the lymphocyte and in some embodiments is or is not integrated into the genome of the genetically modified lymphocyte.

In illustrative embodiments, the heterologous nucleic acid is integrated into the genome of the genetically modified lymphocyte. Such lymphocytes are produced, in illustrative embodiments, using a method for transducing lymphocytes provided herein, that utilizes a recombinant retroviral particle. Such recombinant retroviral particle can include a polynucleotide that encodes a chimeric antigen receptor that typically includes at least an antigen-specific targeting region (ASTR), a transmembrane domain, and an intracellular activating domain. Provided herein in other sections of this disclosure are various embodiments of replication incompetent recombinant retroviral particles and polynucleotides encoded in a genome of the replication incompetent retroviral particle, that can be used to produce genetically modified lymphocytes that themselves form another aspect of the present disclosure.

Genetically modified lymphocytes of the present disclosure can be isolated outside the body. For example, such lymphocytes can be found in media and other solutions that are used for ex vivo transduction as provided herein. The lymphocytes can be present in a genetically unmodified form in blood that is collected from a subject in methods provided herein, and then genetically modified during method of transduction. The genetically modified lymphocytes can be found inside a subject after they are introduced or reintroduced into the subject after they have been genetically modified. The genetically modified lymphocytes can be a resting T cell or a resting NK cell, or the genetically modified T cell or NK cell can be actively dividing, especially after it expresses some of the functional elements provided in nucleic acids that are inserted into the T cell or NK cell after transduction as disclosed herein.

Provided herein in one aspect is a transduced and/or genetically modified T cell or NK cell, comprising a recombinant polynucleotide comprising one or more transcriptional units operatively linked to a promoter active in T cells and/or NK cells, in its genome, that expresses one or more of the functional elements provided in any of the aspects and embodiments of the present disclosure. For example, the one or more transcriptional units can express a CAR, which can include any of the CAR elements provided herein such as an ASTR, as a non-limiting example a MBR-ASTR, a transmembrane domain, and an intracellular signaling domain, and can further include as non-limiting example, a modulatory domain. Furthermore, the functional element(s) expressed within the transduced and/or genetically modified T cell or NK cell, one or more of the lymphoproliferative elements provided herein, for example a constitutively active IL-7 receptor mutant or other lymphoproliferative element that is not an inhibitory RNA molecule (e.g. an miRNA or an shRNA), a recognition and/or elimination domain.

In one aspect, provided herein is a genetically modified T cell or NK cell comprising:
a. one or more (e.g. two or more) inhibitory RNA molecules directed against one or more RNA targets; and
b. a chimeric antigen receptor (CAR) comprising an antigen-specific targeting region (ASTR), a transmembrane domain, and an intracellular activating domain,
and/or nucleic acids encoding the inhibitory RNA molecules directed against one or more RNA targets and the CAR, wherein said one (e.g. two) or more inhibitory RNA molecules and the CAR, or the nucleic acids encoding the same are encoded by or are nucleic acid sequences that are genetic modifications of the T cell and/or NK cell.

The genetically modified T cell or NK cell can be a population of genetically modified T cells and/or NK cells that include the one (e.g. two) or more inhibitory RNA molecules directed against one or more RNA targets; and the CAR.

In some embodiments of the aspect immediately above where the T cell or NK cell comprises one or more (e.g. two or more) inhibitory RNA molecules and the CAR, or nucleic acids encoding the same, any of the specific embodiments provided herein for elements that can be included as part of the CAR or that can be expressed along with a lymphoproliferative element or used to control a lymphoproliferative element can be included.

In some embodiments of the aspect immediately above where the T cell or NK cell comprises one or more (e.g. two or more) inhibitory RNA molecules and the CAR, or nucleic acids encoding the same, the CAR is a microenvironment restricted biologic (MRB)-CAR and/or the genetically modified T cell or NK cell can further include at least one lymphoproliferative element that is not an inhibitory RNA molecule, and/or a nucleic acid encoding the lymphoproliferative element. In such embodiments, the lymphoproliferative element is encoded by nucleic acid sequences that are genetic modifications of the T cell and/or NK cell. Any of the lymphoproliferative elements disclosed herein can be used and/or encoded for, in such embodiments. For example, the at least one lymphoproliferative element can be a constitutively active IL-7 receptor.

In some embodiments of the aspect immediately above where the T cell or NK cell comprises one or more (e.g. two or more) inhibitory RNA molecules and the CAR, or nucleic acids encoding the same, the inhibitory RNA molecule is a precursor of a miRNA or an shRNA. In some embodiments of this aspect the one (e.g. two) or more inhibitory RNA molecules are polycistronic. In some embodiments of this aspect the one (e.g. two) or more inhibitory RNA molecules are directed against the same or in illustrative embodiments, different RNA targets. In some embodiments of this aspect, one, most or all of the one (e.g. two) or more inhibitory RNA molecules decreases expression of an endogenous TCR.

In some embodiments of the aspect immediately above where the T cell or NK cell comprises one or more (e.g. two or more) inhibitory RNA molecules and the CAR, or nucleic acids encoding the same, the RNA target is mRNA transcribed from a gene selected from the group consisting of: PD-1, CTLA4, TCR alpha, TCR beta, CD3 zeta, SOCS, SMAD2, a miR-155 target, IFN gamma, cCBL, TRAIL2, PP2A, and ABCG1. In some embodiments of this aspect at least one of the one (e.g. two) or more inhibitory RNA molecules is miR-155.

In some embodiments of the aspect immediately above where the T cell or NK cell comprises one or more (e.g. two or more) inhibitory RNA molecules and the CAR, or nucleic acids encoding the same, the ASTR of the CAR is an MRB ASTR and/or the ASTR of the CAR binds to a tumor associated antigen. Furthermore, in some embodiments of the above aspect, the first nucleic acid sequence is operably linked to a riboswitch, which for example is capable of binding a nucleoside analog, and in illustrative embodiments is an antiviral drug such as acyclovir.

In the methods and compositions disclosed herein, expression of engineered signaling polypeptides is regulated by a control element, and in some embodiments, the control element is a polynucleotide comprising a riboswitch. In certain embodiments, the riboswitch is capable of binding a nucleoside analog and when the nucleoside analog is present, one or both of the engineered signaling polypeptides are expressed.

The genetically modified lymphocytes disclosed herein can also have polypeptides on their surface that are remnants of fusion of a replication incompetent recombinant retroviral particle during a transduction method provided herein. Such polypeptides can include , an activation element, a pseudotyping element, and/or one or more fusion polypeptides that include a cytokine.

Provided herein in one aspect, is a genetically modified T cell and/or NK cell that expresses one or more (e.g. two or more) inhibitory RNA molecules directed against one or more RNA targets and a chimeric antigen receptor, or CAR, as disclosed herein. In some embodiments, the genetically modified T cell and/or NK cell further expresses at least one lymphoproliferative element as disclosed herein that is not an inhibitory RNA molecule. In certain embodiments, the genetically modified T cell and/or NK cell also expresses one or more riboswitches that control expression of the one or more inhibitory RNA molecules, the CAR, and/or the at least one lymphoproliferative element that is not an inhibitory RNA molecule. In some embodiments, the genetically modified T cell and/or NK cell expresses two to 10 inhibitory RNA molecules. In further embodiments, the genetically modified T cell and/or NK cell expresses two to six inhibitory RNA molecules. In illustrative embodiments, the genetically modified T cell and/or NK cell expresses four inhibitory RNA molecules.

### NUCLEIC ACIDS

The present disclosure provides nucleic acid encoding polypeptides of the present disclosure. A nucleic acid will in some embodiments be DNA, including, e.g., a recombinant expression vector. A nucleic acid will in some embodiments be RNA, e.g., *in vitro* synthesized RNA.

In some cases, a nucleic acid provides for production of a polypeptide of the present disclosure, e.g., in a mammalian cell. In other cases, a subject nucleic acid provides for amplification of the nucleic acid encoding a polypeptide of the present disclosure.

A nucleotide sequence encoding a polypeptide of the present disclosure can be operably linked to a transcriptional control element, e.g., a promoter, and enhancer, etc.

Suitable promoter and enhancer elements are known in the art. For expression in a bacterial cell, suitable promoters include, but are not limited to, lacl, lacZ, T3, T7, gpt, lambda P and trc. For expression in a eukaryotic cell, suitable promoters include, but are not limited to, light and/or heavy chain immunoglobulin gene promoter and enhancer elements; cytomegalovirus immediate early promoter; herpes simplex virus thymidine kinase promoter; early and late SV40 promoters; promoter present in long terminal repeats from a retrovirus; mouse metallothionein-I promoter; and various art-known tissue specific promoters.

Suitable reversible promoters, including reversible inducible promoters are known in the art. Such reversible promoters may be isolated and derived from many organisms, e.g., eukaryotes and prokaryotes. Modification of reversible promoters derived from a first organism for use in a second organism, e.g., a first prokaryote and a second a eukaryote, a first eukaryote and a second a prokaryote, etc., is well known in the art. Such reversible promoters, and systems based on such reversible promoters but also comprising additional control proteins, include, but are not limited to, alcohol regulated promoters (e.g., alcohol dehydrogenase I (alcA) gene promoter, promoters responsive to alcohol transactivator proteins (AlcR), etc.), tetracycline regulated promoters, (e.g., promoter systems including TetActivators, TetON, TetOFF, etc.), steroid regulated promoters (e.g., rat glucocorticoid receptor promoter systems, human estrogen receptor promoter systems, retinoid promoter systems, thyroid promoter systems, ecdysone promoter systems, mifepristone promoter systems, etc.), metal regulated promoters (e.g., metallothionein promoter systems, etc.), pathogenesis-related regulated promoters (e.g., salicylic acid regulated promoters, ethylene regulated promoters, benzothiadiazole regulated promoters, etc.), temperature regulated promoters (e.g., heat shock inducible promoters (e.g., HSP-70, HSP-90, soybean heat shock promoter, etc.), light regulated promoters, synthetic inducible promoters, and the like.

In some instances, the locus or construct or trans gene containing the suitable promoter is irreversibly switched through the induction of an inducible system. Suitable systems for induction of an irreversible switch are well known in the art, e.g., induction of an irreversible switch may make use of a Cre-lox-mediated recombination (see, e.g., Fuhrmann-Benzakein, et al., PNAS (2000) 28:e99, the disclosure of which can be referred to for further details). Any suitable combination of recombinase, endonuclease, ligase, recombination sites, etc. known to the art may be used in generating an irreversibly switchable promoter. Methods, mechanisms, and requirements for performing site-specific recombination, described elsewhere herein, find use in generating irreversibly switched promoters and are well known in the art, see, e.g., Grindley et al. (2006) Annual Review of Biochemistry, 567-605 and Tropp (2012) Molecular Biology (Jones & Bartlett Publishers, Sudbury, MA), the disclosures of which can be referred to for further details.

In some cases, the promoter is a CD8 cell-specific promoter, a CD4 cell-specific promoter, a neutrophil-specific promoter, or an NK-specific promoter. For example, a CD4 gene promoter can be used; see, e.g., Salmon et al. (1993) Proc. Natl. Acad. Sci. USA 90:7739; and Marodon et al. (2003) Blood 101:3416. As another example, a CD8 gene promoter can be used. NK cell-specific expression can be achieved by use of an *Neri (p46)* promoter; see, e.g., Eckelhart et al. (2011) Blood 117:1565.

In some embodiments, e.g., for expression in a yeast cell, a suitable promoter is a constitutive promoter such as an ADHl promoter, a PGKl promoter, an ENO promoter, a PYKl promoter and the like; or a regulatable promoter such as a GALI promoter, a GALlO promoter, an ADH2 promoter, a PH05 promoter, a CUPI promoter, a GAL7 promoter, a MET25 promoter, a MET3 promoter, a CYCl promoter, a HIS3 promoter, an ADHl promoter, a PGK promoter, a GAPDH promoter, an ADCl promoter, a TRPl promoter, a URA3 promoter, a LEU2 promoter, an ENO promoter, a TPl promoter, and AOXI (e.g., for use in *Pichia).* Selection of the appropriate vector and promoter is well within the level of ordinary skill in the art.

Suitable promoters for use in prokaryotic host cells include, but are not limited to, a bacteriophage T7 RNA polymerase promoter; a trp promoter; a lac operon promoter; a hybrid promoter, e.g., a lac/tac hybrid promoter, a tac/trc hybrid promoter, a trp/lac promoter, a T7/lac promoter; a trc promoter; a tac promoter, and the like; an araBAD promoter; *in vivo* regulated promoters, such as an *ssaG* promoter or a related promoter *(see,* e.g., U.S. Patent Publication No. 20040131637), a *pagC* promoter (Pulkkinen and Miller, J. Bacterial., 1991: 173(1): 86-93; Alpuche-Aranda et al., PNAS, 1992; 89(21): 10079-83), a *nirB* promoter (Harborne et al. (1992) Mal. Micro. 6:2805-2813), and the like *(see,* e.g., Dunstan et al. (1999) Infect. Immun. 67:5133-5141; McKelvie et al. (2004) Vaccine 22:3243-3255; and Chatfield et al. (1992) Biotechnol. 10:888-892); a sigma70 promoter, e.g., a consensus sigma70 promoter (see, e.g., GenBank Accession Nos. AX798980, AX798961, and AX798183); a stationary phase promoter, e.g., a *dps* promoter, an *spv* promoter, and the like; a promoter derived from the pathogenicity island SPI-2 *(see,* e.g., WO96/17951); an actA promoter *(see,* e.g., Shetron-Rama et al. (2002) Infect. Immun. 70:1087-1096); an rpsM promoter *(see,* e.g., Valdivia and Falkow (1996). Mal. Microbial. 22:367); a tet promoter *(see,* e.g., Hillen,W. and Wissmann,A. (1989) In Saenger,W. and Heinemann,U. (eds), Topics in Molecular and Structural Biology, Protein-Nucleic Acid Interaction. Macmillan, London, UK, Vol. 10, pp. 143-162); an SP6 promoter *(see,* e.g., Melton et al. (1984) Nucl. Acids Res. 12:7035); and the like. Suitable strong promoters for use in prokaryotes such as *Escherichia coli* include, but are not limited to Trc, Tac, T5, T7, and PLambda. Non-limiting examples of operators for use in bacterial host cells include a lactose promoter operator (Laci repressor protein changes conformation when contacted with lactose, thereby preventing the Laci repressor protein from binding to the operator), a tryptophan promoter operator (when complexed with tryptophan, TrpR repressor protein has a conformation that binds the operator; in the absence of tryptophan, the TrpR repressor protein has a conformation that does not bind to the operator), and a tac promoter operator (see, for example, deBoer et al. (1983) Proc. Natl. Acad. Sci. U.S.A. 80:21-25).

A nucleotide sequence encoding a polypeptide of the disclosure can be present in an expression vector and/or a cloning vector. Nucleotide sequences encoding two separate polypeptides can be cloned in the same or separate vectors. An expression vector can include a selectable marker, an origin of replication, and other features that provide for replication and/or maintenance of the vector. Suitable expression vectors include, e.g., plasmids, viral vectors, and the like.

Large numbers of suitable vectors and promoters are known to those of skill in the art; many are commercially available for generating a subject recombinant constructs. The following bacterial vectors are provided by way of example: pBs, phagescript, PsiXl74, pBluescript SK, pBs KS, pNH8a, pNH16a, pNH18a, pNH46a (Stratagene, La Jolla, CA, USA); pTrc99A, pKK223-3, pKK233-3, pDR540, and pRIT5 (Pharmacia, Uppsala, Sweden). The following eukaryotic vectors are provided by way of example: pWLneo, pSV2cat, pOG44, PXRl, pSG (Stratagene) pSVK3, pBPV, pMSG, and pSVL (Pharmacia).

Expression vectors generally have convenient restriction sites located near the promoter sequence to provide for the insertion of nucleic acid sequences encoding heterologous proteins. A selectable marker operative in the expression host may be present.

As noted above, in some embodiments, a nucleic acid encoding a polypeptide of the present disclosure will in some embodiments be RNA, e.g., *in vitro* synthesized RNA. Methods for *in vitro* synthesis of RNA are known in the art; any known method can be used to synthesize RNA including a nucleotide sequence encoding a polypeptide of the present disclosure. Methods for introducing RNA into a host cell are known in the art. See, e.g., Zhao et al. (2010) Cancer Res. 15:9053. Introducing RNA including a nucleotide sequence encoding a polypeptide of the present disclosure into a host cell can be carried out *in vitro* or *ex vivo* or *in vivo.* For example, a host cell (e.g., an NK cell, a cytotoxic T lymphocyte, etc.) can be electroporated *in vitro* or *ex vivo* with RNA comprising a nucleotide sequence encoding a polypeptide of the present disclosure.

Various aspects and embodiments that include a polynucleotide, a nucleic acid sequence, and/or a transcriptional unit, and/or a vector including the same, further include one or more of a Kozak-type sequence, a woodchuck hepatitis virus post-transcriptional regulatory element (WPRE), and a double stop codon or a triple stop codon, wherein one or more stop codons of the double stop codon or the triple stop codon define a termination of a reading from of at least one of the one or more transcriptional units. In certain embodiments, a polynucleotide a nucleic acid sequence, and/or a transcriptional unit, and/or a vector including the same, further includes a Kozak-type sequence having a 5' nucleotide within 10 nucleotides upstream of a start codon of at least one of the one or more transcriptional units. In one embodiment the Kozak-type sequence is or includes CCACCAT/UG(G) (SEQ ID NO:515), CCGCCAT/UG(G) (SEQ ID NO:516), GCCGCCGCCAT/UG(G) (SEQ ID NO:517), or GCCGCCACCAT/UG(G) (SEQ ID NO:515) (with nucleotides in parenthesis representing optional nucleotides and nucleotides separated by a slash indicated different possible nucleotides at that position, for example depending on whether the nucleic acid is DNA or RNA. In these embodiments that include the AU/TG start codon, the A can be considered position 0. In certain illustrative embodiments, the nucleotides at -3 and +4 are identical, for example the -3 and +4 nucleotides can be G. In another embodiment the Kozak-type sequence includes an A or G in the 3rd position upstream of ATG where ATG is the start codon. In another embodiment the Kozak-type sequence includes an A or G in the 3rd position upstream of AUG where AUG is the start codon. In an illustrative embodiment, the Kozak sequence is (GCC)GCCRCCATG, where R is a purine (A or G). In an illustrative embodiment, the Kozak-type sequence is GCCGCCACCAUG (SEQ ID NO:518). In another embodiment, which can be combined with the preceding embodiment that includes a Kozak-type sequence and/or the following embodiment that includes triple stop codon, the polynucleotide includes a WPRE element. WPREs have been characterized in the art (See e.g., (Higashimoto et al., Gene Ther. 2007; 14: 1298)) and exemplified in Examples 17 and 18 herein. In some embodiments, the WPRE element is located 3' of a stop codon of the one or more transcriptional units and 5' to a 3' LTR of the polynucleotide. In another embodiment, which can be combined with either or both of the preceding embodiments (i.e. an embodiment wherein the polynucleotide includes a Kozak-type sequence and/or an embodiment wherein the polynucleotide includes a WPRE), the one or more transcriptional units terminates with one or more stop codons of a double stop codon or a triple stop codon, wherein the double stop codon includes a first stop codon in a first reading frame and a second stop codon in a second reading frame, or a first stop codon in frame with a second stop codon, and wherein the triple stop codon includes a first stop codon in a first reading frame, a second stop codon in a second reading frame, and a third stop codon in a third reading frame, or a first stop codon in frame with a second stop codon and a third stop codon.

A triple stop codon herein includes three stop codons, one in each reading frame, within 10 nucleotides of each other, and preferably having overlapping sequence, or three stop codons in the same reading frame, preferably at consecutive codons. A double stop codon means two stop codons, each in a different reading frame, within 10 nucleotides of each other, and preferably having overlapping sequences, or two stop codons in the same reading frame, preferably at consecutive codons.

In some of the methods and compositions disclosed herein, the introduction of DNA into PBMCs, B cells, T cells and/or NK cells and optionally the incorporation of the DNA into the host cell genome, is performed using methods that do not utilize replication incompetent recombinant retroviral particles. For example, other viral vectors can be utilized, such as those derived from adenovirus, adeno-associated virus, or herpes simplex virus-1, as non-limiting examples.

In some embodiments, methods provided herein can include transfecting and/or transducing target cells with non-viral vectors. In any of the embodiments disclosed herein that utilize non-viral vectors to transfect target cells, the non-viral vectors, including naked DNA, can be introduced into the target cells, such as for example, PBMCs, B cells, T cells and/or NK cells using methods that include electroporation, nucleofection, liposomal formulations, lipids, dendrimers, cationic polymers such as poly(ethylenimine) (PEI) and poly(l-lysine) (PLL), nanoparticles, cell-penetrating peptides, microinjection, and/or non-integrating lentiviral vectors. In some embodiments, DNA can be introduced into target cells, such as PBMCs, B cells, T cells and/or NK cells in a complex with liposomes and protamine. Other methods for transfecting and/or transducing T cells and/or NK cells *ex vivo* that can be used in embodiments of methods provided herein, are known in the art (see e.g., Morgan and Boyerinas, Biomedicines. 2016 Apr 20;4(2). pii: E9, which can be referred to for further details).

In some embodiments of method provided herein, DNA can be integrated into the genome using transposon-based carrier systems by co-transfection, co-nucleofection or co-electroporation of target DNA as plasmid containing the transposon ITR fragments in 5' and 3' ends of the gene of interest and transposase carrier system as DNA or mRNA or protein or site specific serine recombinases such as phiC31 that integrates the gene of interest in pseudo attP sites in the human genome, in this instance the DNA vector contains a 34 to 40 bp attB site that is the recognition sequence for the recombinase enzyme (Bhaskar Thyagarajan et al. Site-Specific Genomic Integration in Mammalian Cells Mediated by Phage φC31 Integrase, Mol Cell Biol. 2001 Jun; 21(12): 3926-3934) and co transfected with the recombinase. For T cells and/or NK cells, transposon-based systems that can be used in certain methods provided herein utilize the *Sleeping Beauty* DNA carrier system (see e.g., U.S. Pat. No. 6,489,458 and U.S. Pat. Appl. No. 15/434,595, which can be referred to for further details), the *PiggyBac* DNA carrier system (see e.g., Manuri et al., Hum Gene Ther. 2010 Apr;21(4):427-37, which can be referred to for further details), or the *Tol2* transposon system (see e.g., Tsukahara et al., Gene Ther. 2015 Feb; 22(2): 209-215, which can be referred to for further details) in DNA, mRNA, or protein form. In some embodiments, the transposon and/or transposase of the transposon-based vector systems can be produced as a minicircle DNA vector before introduction into T cells and/or NK cells (see e.g., Hudecek et al., Recent Results Cancer Res. 2016;209:37-50 and Monjezi et al., Leukemia. 2017 Jan;31(1):186-194, which can be referred to for further details). The CAR or lymphoproliferative element can also be integrated into the defined and specific sites in the genome using CRISPR or TALEN mediated integration, by adding 50-1000 bp homology arms homologous to the integration 5' and 3' of the target site (Jae Seong Lee et al. Scientific Reports 5, Article number: 8572 (2015), Site-specific integration in CHO cells mediated by CRISPR/Cas9 and homology-directed DNA repair pathway). CRISPR or TALEN provide specificity and genomic-targeted cleavage and the construct will be integrated via homology-mediated end joining (Yao X at al. Cell Res. 2017 Jun;27(6):801-814. doi: 10.1038/cr.2017.76. Epub 2017 May 19). The CRISPR or TALEN can be co-transfected with target plasmid as DNA, mRNA, or protein.

### PACKAGING CELLS

The present disclosure provides packaging cells and mammalian cell lines that are packaging cell lines that produce replication incompetent recombinant retroviral particles that genetically modify target mammalian cells and the target mammalian cells themselves. In illustrative embodiments, the packaging cell comprises nucleic acid sequences encoding a packagable RNA genome of the replication incompetent retroviral particle, a REV protein, a gag polypeptide, a pol polypeptide, and a pseudotyping element.

Suitable mammalian cells include primary cells and immortalized cell lines. Suitable mammalian cell lines include human cell lines, non-human primate cell lines, rodent (e.g., mouse, rat) cell lines, and the like. Suitable mammalian cell lines include, but are not limited to, HeLa cells (e.g., American Type Culture Collection (ATCC) No. CCL-2), CHO cells (e.g., ATCC Nos. CRL9618, CCL61, CRL9096), HEK293 cells (e.g., ATCC No. CRL-1573), suspension-adapted HEK293 cells, Vero cells, NIH 3T3 cells (e.g., ATCC No. CRL-1658), Huh-7 cells, 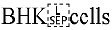 (e.g., ATCC No. CCLlO), PC12 cells (ATCC No. CRL1721), COS cells, COS-7 cells (ATCC No. CRL1651), RATl cells, mouse L cells (ATCC No. CCLI.3), human embryonic kidney (HEK) cells (ATCC No. CRL1573), HLHepG2 cells, Hut-78, Jurkat, HL-60, NK cell lines (e.g., NKL, NK92, and YTS), and the like.

In some instances, the cell is not an immortalized cell line, but is instead a cell (e.g., a primary cell) obtained from an individual or an *ex vivo* cell. For example, in some cases, the cell is an immune cell obtained from an individual. As another example, the cell is a stem cell or progenitor cell obtained from an individual. In any of the embodiments and aspects provided herein, that include a B cell, T cell, or NK cell, the cell can be an autologous cell or it can be an allogeneic cell (also called allogenic cell herein). In some embodiments the allogenic cell can be a genetically engineered allogenic cell. Allogeneic cells, such as allogenic T cells, and methods for genetically engineering allogeneic cells, are known in the art. In some embodiments, the allogeneic cell can be an immortalized cell. In some embodiments where the allogeneic cell is a T cell, the T cell has been genetically engineered such that at least one of its T cell receptor chains does not function and/or is at least partially deleted. In some embodiments the allogeneic cell can be modified such that it is missing all or part of the B2 microglobulin gene. In some embodiments where allogeneic cells are used in methods herein, lymphoproliferative elements and/or CLEs can function to reduce the number of cells that are necessary for practicing the invention claimed herein and can facilitate cell manufacturing of T cells, B cells, or NK cells from a subject.

### METHODS OF ACTIVATING AN IMMUNE CELL

The present disclosure provides methods of activating an immune cell, in illustrative embodiments a lymphocyte, *in vitro, in vivo,* or *ex vivo.* The methods generally involve contacting an immune cell *(in vitro, in vivo,* or *ex vivo)* with an activating element, or with one or more target antigens, where the immune cell has been genetically modified to produce a microenvironment restricted CAR of the present disclosure. In the presence of the one or more target antigens, the microenvironment restricted CAR activates the immune cell, thereby producing an activated immune cell. Immune cells include, e.g., a cytotoxic T lymphocyte, an NK cell, a CD4⁺ T cell, a T regulatory (Treg) cell, a γδ T cell, an NK-T cell, neutrophils, etc. In other embodiments, contacting a T cell with a T cell activating agent, activates the T cell. Such methods are provided herein and discussed in the Activation Element section herein.

Contacting the genetically modified immune cell (e.g., a T lymphocyte, an NK cell) with one or more target antigens can increase production of a cytokine by the immune cell by at least about 10%, at least about 15%, at least about 20%, at least about 25%, at least about 30%, at least about 40%, at least about 50%, at least about 75%, at least about 2-fold, at least about 2.5-fold, at least about 5-fold, at least about 10-fold, or more than 10-fold, compared with the amount of cytokine produced by the immune cell in the absence of the one or more target antigens. Cytokines whose production can be increased include, but are not limited to, IL-2 and IFN-γ.

Contacting a genetically modified cytotoxic cell (e.g., cytotoxic T lymphocyte) with AAR can increase cytotoxic activity of the cytotoxic cell by at least about 10%, at least about 15%, at least about 20%, at least about 25%, at least about 30%, at least about 40%, at least about 50%, at least about 75%, at least about 2-fold, at least about 2.5-fold, at least about 5-fold, at least about 10-fold, or more than 10-fold, compared to the cytotoxic activity of the cytotoxic cell in the absence of the one or more target antigens.

Contacting a genetically modified cytotoxic cell (e.g., cytotoxic T lymphocyte) with one or more target antigens can increase cytotoxic activity of the cytotoxic cell by at least about 10%, at least about 15%, at least about 20%, at least about 25%, at least about 30%, at least about 40%, at least about 50%, at least about 75%, at least about 2-fold, at least about 2.5-fold, at least about 5-fold, at least about 10-fold, or more than 10-fold, compared to the cytotoxic activity of the cytotoxic cell in the absence of the one or more target antigens.

In other embodiments, e.g., depending on the host immune cell, contacting a genetically modified host cell with an antigen can increase or decrease cell proliferation, cell survival, cell death, and the like.

### METHODS FOR MAKING A MICROENVIRONMENT RESTRICTED ANTIGEN-SPECIFIC TARGETING REGION

In some embodiments, antigen binding domains (also referred to herein as "antigen-specific target regions" or "ASTRs") of CARs constitutively bind their cognate antigens. In other embodiments, the ASTRs can be microenvironment restricted, preferentially or only binding their cognate antigen under certain aberrant conditions, such as those that exist in the tumor microenvironment, as disclosed in more detail herein. Microenvironment restricted ASTRs that bind preferentially or exclusively under aberrant conditions of a tumor microenvironment, can provide a reduction in on-target off-tumor effects as binding to the antigen in normal physiological conditions is reduced, in some situations to levels below detection by immunoassays. In certain aspects, CARs provided herein include a microenvironment restricted ASTR that specifically binds to a target protein, wherein the ASTR is an scFv fragment that includes a heavy chain variable region and a light chain variable region.

Certain illustrative embodiments of the aspects disclosed herein, for example the methods, cells, cells lines, replication incompetent recombinant retroviral particles, polynucleotides, or vectors disclosed herein, include CARs that include microenvironment restricted antigen-specific targeting regions.

Accordingly, in one aspect, provided herein is a chimeric antigen receptor for binding a target antigen, that includes:
a) a microenvironment restricted antigen-specific targeting region that exhibits an increase in binding to the target antigen in an aberrant condition compared to a normal physiological environment, wherein the antigen-specific targeting region binds to the target;
b) a transmembrane domain; and
c) an intracellular activating domain.

In another aspect, provided herein is a chimeric antigen receptor for binding a target antigen, that includes:
a) at least one microenvironment restricted antigen specific targeting region selected by panning a polypeptide library and having an increase in activity in a target antigen binding assay at an aberrant condition compared to a normal physiological condition;
b) a transmembrane domain; and
c). an intracellular activating domain.

In some embodiments of any aspect disclosed herein, any of the chimeric antigen receptors can be microenvironment restricted such that they exhibit an increase in binding activity at an aberrant condition compared to a normal physiological condition. In some illustrative embodiments of any aspect disclosed herein, the microenvironment restricted ASTR is identified from an initial polypeptide library without mutating/evolving members of the library before screening/evolving and/or without mutating during or between optional repeated rounds of screening. Exemplary transmembrane domains and intracellular activating domains can be any of those disclosed herein for CARs.

In one aspect, provided herein is a method for selecting a microenvironment restricted ASTR, comprising panning a polypeptide display library by:
a. subjecting polypeptides of the polypeptide display library to a target antigen binding assay under a normal physiological condition and a target antigen binding assay under an aberrant condition; and
b. selecting a polypeptide which exhibits an increase in target antigen binding activity at the aberrant condition compared to the physiological condition, thereby selecting the microenvironment restricted antigen specific targeting region.

In another aspect, provided herein is a method for isolating a microenvironment restricted ASTR, that includes panning a polypeptide library by:
contacting the polypeptide library under aberrant conditions with a target antigen bound to a solid support, wherein clones expressing polypeptides that bind the target antigen remain bound to the solid support through the target antigen;
incubating the solid supports with bound polypeptides under physiological conditions; and collecting clones that elute from the solid support under the physiological conditions, thereby isolating the microenvironment restricted antigen-specific targeting region.

In some illustrative embodiments of any aspect disclosed herein, the microenvironment restricted antigen-specific targeting region is identified from an initial polypeptide library screen without mutating/evolving members of the library before screening and/or without mutating/evolving during or between optional repeated rounds of screening or panning.

Normal physiological conditions can include those of temperature, pH, osmotic pressure, osmolality, oxidative stress, and electrolyte concentration that would be considered within a normal range at the site of administration, or at the tissue or organ at the site of action, to a subject. An aberrant condition is that which deviates from the normally acceptable range for that condition. In one aspect, a microenvironment restricted antigen-specific targeting region (i.e. polypeptide) is virtually inactive at normal conditions but is active at other than normal conditions at a level that is equal or better than at normal conditions. For example, in one aspect, the microenvironment restricted antigen-specific targeting region is virtually inactive at body temperature, but is active at lower temperatures. In another aspect, the microenvironment restricted antigen-specific targeting region is reversibly or irreversibly inactivated at the normal conditions. In a further aspect, the microenvironment restricted antigen-specific targeting region is a therapeutic protein. In another aspect, the microenvironment restricted antigen-specific targeting region is used as a drug, or therapeutic agent. In yet another aspect, the microenvironment restricted antigen-specific targeting region is more or less active in highly oxygenated blood, such as, for example, after passage through the lung or in the lower pH environments found in the kidney.

In some embodiments, a single round of selection is performed to obtain the microenvironment restricted antigen-specific targeting region. In certain embodiments, the screening or panning method is repeated after identifying free polypeptides that bound antigen under aberrant conditions and did not bind under physiological conditions, or cells expressing a test polypeptide that had these properties, or phage coated with a test polypeptide that has such properties in an initial or previous round. In some methods, phage that are collected are used to infect cells, which can be infected with helper phage as well, in order to amplify the collected phage. In other methods where polypeptides on the surface of cells are tested, collected cells can be grown to "amplify" the polypeptides expressed by the cells by amplifying polynucleotides in the cells that encode the polypeptides. In some embodiments, the amplifying is done by growing cells that express the identified polypeptides without performing a process to mutate the polynucleotides encoding the identified polypeptides between rounds. Thus, polypeptides that were collected in a previous round are enriched by amplifying cells that contain polynucleotides encoding these collected polypeptides.

The panning or screening method can be performed a single time, or repeated for 1 to 1000 times. In illustrative embodiments, the panning is repeated 1 to 20 times or 2 to 10 times or 2 to 5 times.

In other methods, microenvironment restricted ASTRs against an antigen of interest (i.e. target antigen) are performed using one or more rounds of mutation/evolution between rounds of panning. In one method, a wild-type protein is identified for example by generating a polypeptide or protein library and screening the polypeptide or protein library for a polypeptide or protein with a desired binding affinity to a target antigen. In some embodiments where the wild-type proteins are antibodies, the wild-type antibodies can be discovered by generating and screening polyclonal or monoclonal antibody libraries, including phage display antibody libraries, for example phage display humanized antibody libraries.

Evolved ASTRs can be generated by subjecting the wild-type protein, or a nucleic acid sequence encoding the wild-type protein, to a process of mutagenesis to produce a population of mutant polypeptides that can be screened to identify a mutant ASTR with an increased activity (e.g. enhanced binding affinity to the target antigen) in a tumor environment and/or in an *in vitro* tumor surrogate assay condition, compared to a normal physiological environment. Examples of such methods are provided in WO2016033331 ("CONDITIONALLY ACTIVE CHIMERIC ANTIGEN RECEPTORS FOR MODIFIED T CELLS") or U.S. Patent No. 8,709,755, which can be referred to for further details. This method of generating a microenvironment restricted antibody can for example be used.

In other embodiments, microenvironment restricted antigen-specific polypeptides (i.e. targeting regions, e.g. antibodies) can be identified by screening an initial polypeptide library under aberrant versus physiological conditions and identifying a test polypeptide from the initial polypeptide library, that binds preferentially or exclusively under aberrant vs. physiological conditions. In some examples, the identified and isolated microenvironment restricted antigen-specific polypeptides (i.e. targeting regions, e.g. antibodies) identified from an initial polypeptide library in an initial polypeptide library screen, bind their cognate antigen preferentially or exclusively under aberrant vs. physiological conditions. In such instances, no rounds of mutating/evolving are performed. Accordingly, the method in illustrative embodiments is performed without mutating polynucleotides encoding the isolated microenvironment restricted antigen-specific targeting region between rounds of screening (e.g. rounds of panning), or performed for only a single binding assay under aberrant versus physiological conditions to isolate and identify the microenvironment restricted antigen-specific polypeptide (i.e. targeting region, e.g. antibody). The method can be performed by culturing, high fidelity amplifying, and/or diluting polynucleotides encoding antigen-specific targeting regions, or host organisms including the same, between rounds of screening and/or panning, without any mutating/evolving. Furthermore, the method can be performed without repeating the screening and/or panning and can be performed without mutating/evolving a polynucleotide encoding the isolated microenvironment restricted antigen-specific targeting region, after the microenvironment restricted antigen-specific polypeptide (i.e. target region, e.g. antibody) is isolated.

Assays for use in the methods provided herein to detect binding of a polypeptide to a cognate binding partner include cell based assays, and in particular assays performed using cell surface display systems, such as mammalian cell surface display systems. In an exemplary method, nucleic acids encoding a polypeptide or a library of variant polypeptides, including a library of modified polypeptides, can be introduced into a vector suitable for expression in cells, such as mammalian cells. Cells are then transfected with the vector, and the polypeptide(s) is/are expressed by the cells. The library of cells containing surface-expressed polypeptides can be contacted with a solution containing a soluble or surface-bound cognate binding partner. Binding activity can be detected using any assay that can detect the binding to the surface of the cells. Activity also can be assessed by assessing a functional activity of the polypeptide or polypeptide. Any cell based assay known to the skilled artisan is contemplated for use in the methods provided herein, including cell proliferation assays, cell death assays, flow cytometry, cell separation techniques, fluorescence activated cell sorting (FACS), phase microscopy, fluorescence microscopy, receptor binding assays, cell signaling assays, immunocytochemistry and reporter gene assays. In some examples, the assays are fluorescence activated cell sorting (FACS) assays.

Polypeptides or proteins can be expressed by mammalian cells as secreted, soluble molecules, cell surface molecules, or intracellular antibodies. In an exemplary method, cells can be transfected with a library of proteins under conditions whereby most or all of the cells display a member of the protein library anchored on the cell surface. Optionally, an expression system can be used in which most of mammalian cell transfectants have only one plasmid integrated in their genome. Therefore, most (i.e., at least about 70% or about 80% or about 90%) of the transfectants express one or more molecules of one polypeptide. This can be verified, for example, by isolating and culturing individual transfectants; and amplifying and sequencing the expressed sequences to determine whether they have a single sequence.

In some examples of the methods provided herein, the polypeptides are antibodies displayed on the surface of mammalian cells. Any antibody described herein can be expressed on the surface of mammalian cells, including full length, bivalent, functional antibodies, such as IgG antibodies. The antibody can be a fragment, for example, Fab fragments or scFv fragments. Antibodies can include an Fc region, such as an scFv-Fc or a full length antibody, which comprises two heavy and two light chains. The skilled artisan can select a suitable antibody fragment. For example, an ScFv-Fcs and full length antibodies made in mammalian cells can have several advantages over scFv's or Fab fragments.

Solid supports that can be used in the binding assays provided herein include any carrier that is capable of being affixed with a binding partner of a polypeptide such as a ligand, receptor or antigen. Typically, to facilitate high throughput screening a cognate binding partner is affixed to the solid support. Examples of carriers for use as solid supports in the methods provided herein include, but are not limited to, glass, polystyrene, polypropylene, polyethylene, dextran, nylon, amyloses, natural and modified celluloses, polyacrylamides, agaroses and magnetic solid supports, such as solid supports that include magnetite. The solid support can be one or more beads or particles, microspheres, a surface of a tube or plate, a filter membrane, and other solid supports known in the art. Exemplary solid support systems include, but are not limited to, a flat surface constructed, for example, of glass, silicon, metal, nylon, cellulose, plastic or a composite, including multiwell plates or membranes; or can be in the form of a bead such as a silica gel, a controlled pore glass, a magnetic or cellulose bead. Further, such methods can be adapted for use in suspension or in the form of a column. In some embodiments, the microenvironment restricted antigen-specific polypeptide (i.e. target region, e.g. antibody) is identified and isolated by biopanning a phage display or yeast surface display (Colby et al., "Engineering Antibody Affinity by Yeast Surface Display," Meth. Enzym. 388, 26 (2004)) antibody (e.g. humanized antibody) library with an immobilized target antigen. For example, either a naive humanized antibody library or a synthetic humanized antibody library can be panned using the phage display or yeast surface display methods herein. In some embodiments, an initial phage display process, phage clones can be transferred to a mammalian vector and used to a mammalian cell surface screening method (See e.g., Yoon et al., BMC Biotechnology 12:62; 1472-6750 (2012)). An exemplary method for performing phage display to isolate a microenvironment restricted antigen-specific target region is provided in Example 2.

A microenvironment restricted ASTR identified using methods provided herein, can be an antibody, an antigen, a ligand, a receptor binding domain of a ligand, a receptor, a ligand binding domain of a receptor, or an affibody. In embodiments where the microenvironment restricted ASTR is an antibody, it can be a full-length antibody, a single-chain antibody, an Fab fragment, an Fab' fragment, an (Fab')2 fragment, an Fv fragment, and a divalent single-chain antibody or a diabody. wherein the antigen-specific targeting region comprises a heavy chain and a light chain from an antibody. In some embodiments, the microenvironment restricted ASTR is a single-chain variable fragment. Such single-chain variable fragment can have heavy and light chains separated by a linker, wherein the linker is between 6 and 100 amino acids in length. In some embodiments the heavy chain is positioned N-terminal to the light chain on the chimeric antigen receptor. In other embodiments, the light chain is positioned N-terminal to the heavy chain. The microenvironment restricted ASTR can be a bispecific ASTR.

Microenvironment restricted ASTRs identified using methods provided herein are typically polypeptides and more specifically polypeptide antibodies, and in illustrative embodiments, single chain antibodies. These polypeptides can bind to their cognate antigens with higher or lower affinity under aberrant conditions vs. normal conditions, but in illustrative embodiments, bind with higher affinity under aberrant conditions than normal conditions. In some embodiments, these polypeptides can bind to their cognate antigen with a 10%, 20%, 25%, 50%, 75%, 90%, 95% or 99% greater affinity under aberrant conditions than physiological (i.e. normal) conditions. In some embodiments, the ASTRs identifying using methods provided herein do not bind to their cognate antigens under normal physiological conditions to any detectable level above background levels obtained using negative controls, such as negative control antibodies.

The nucleotide sequence encoding a microenvironment restricted ASTR isolated by the method provided herein, can be determined by sequencing nucleotides of the collected cell expressing the microenvironment restricted antigen-specific targeting. This nucleotide sequence information can then be used to make a microenvironment restricted biologic chimeric antigen receptor (MRB-CAR) by generating a polynucleotide that encodes a polypeptide comprising the microenvironment restricted antigen-specific targeting region (MRB-ASTR), a transmembrane domain, and an intracellular activating domain. Microenvironment restricted antigen-specific targeting regions can be cloned into a CAR construct expression system, which can be used to generate recombinant lentiviruses that include the CAR in their genome, and then the recombinant lentiviruses can be used to transduce T cells for testing for CAR-mediated tumor antigen expressing target cell killing in a tumor-selective environment compared to physiologic conditions.

### CONDITIONS FOR CONDITIONAL ACTIVITY

In the methods provided herein, the activity of one or more polypeptides, such as, for example, single chain antibodies, is screened or tested under two different sets of conditions that simulate a condition or conditions in two different physiologic environments such as, for example, a diseased microenvironment and the normal physiologic condition of a non-diseased microenvironment. Typically, the conditions are conditions that can be simulated or replicated *in vitro.* A set of conditions can include one or more conditions to simulate a microenvironment associated with a disease. Disease can alter intracellular and extracellular homeostasis. For example, the diseased microenvironment can simulate one or more conditions in a tumor microenvironment or a cancer microenvironment. Typically, the difference or differences in activity under the two sets of conditions can result in the conditional activity of the molecule. Thus, a molecule that exhibits greater activity under the first set of conditions (e.g. simulating conditions in a tumor microenvironment) compared to the second set of conditions (e.g. simulating conditions in a normal or non-diseased environment) is identified as a candidate molecule that is microenvironment restricted.

The two sets of conditions can be selected to vary by one or more parameters that differ in two physiologic environments, such as described herein or known to one of skill in the art, including but not limited to chemical conditions, biological conditions, or physical conditions. Parameters that can be varied between the two sets of conditions can include one or more conditions selected from among pressure, temperature, pH, ionic strength, osmotic pressure, osmolality, oxidative stress, turbidity, exposure to light (including UV, infrared or visible light), concentration of one or more solutes, such as electrolytes, concentration of glucose, concentration of hyaluronan, concentration of lactic acid or lactate, concentration of albumin, levels of adenosine, levels of R-2-hydroxyglutarate, concentration of pyruvate, concentration of oxygen, and/or presence of oxidants, reductants, or co-factors. By varying the electrolyte and buffer systems in the calibration solutions, physiological conditions such as pH, buffer capacity, ionic environment, temperature, glucose concentration, and ionic strength can be adjusted to those of the biological environment to be simulated. The set of conditions that simulate a normal physiologic environment can be selected to be different from the set of conditions that simulate a diseased microenvironment, such as a tumor microenvironment, by one or more conditions described herein.

For example, as discussed below, various parameters of the tumor microenvironment differ compared to a non-tumor microenvironment, including, but not limited to, oxygen concentration, pressure, presence of co-factors, pH, hyaluronan concentration, lactate concentration, albumin concentration, levels of adenosine, levels of R-2-hydroxyglutarate, and pyruvate concentration. Any of these parameters can be replicated *in vitro* to simulate one or more conditions that exist in a tumor or cancer environment compared to conditions that exist in a non-tumor or a normal environment. The normal physiologic conditions that can be simulated include environments found in healthy or nondiseased tissue at any location of the body such as the GI tract, the skin, the vasculature, the blood, and extracellular matrix. Typically, in the assays herein, physiologic conditions can be simulated *in vitro* by the choice of buffer that is used to assess the activity of the protein. For example, any one or more conditions of a diseased microenvironment (such as a tumor microenvironment) and a non-diseased environment can be simulated by differences in the assay buffer used to assess activity in the assay. Hence, in the methods herein to identify a microenvironment restricted polypeptide, a component or components or characteristic or characteristics of an assay buffer are altered or made to be different in a first assay to test activity under a first condition and in a second assay to test activity under a second condition. For example, as discussed herein, various parameters of the tumor microenvironment are different compared to a non-tumor environment including, but not limited to, oxygen, pressure, presence of co-factors, pH, hyaluronan concentration (such as increased or decreased hyaluronan concentration), lactate concentration (such as increased or decreased lactate concentration), albumin concentration (such as increased or decreased albumin concentration), levels of adenosine (such as increased or decreased adenosine levels), levels of R-2-hydroxyglutarate (such as increased or decreased R-2-hydroxyglutarate levels) and pyruvate concentration (including increased or decreased pyruvate concentration). More specifically, conditions in a tumor microenvironment can include lower pH, higher concentrations of hyaluronan, higher concentrations of lactate and pyruvate, higher concentrations of albumin, increased levels of adenosine, increased levels of R-2-hydroxyglutarate, hypoxia, lower concentration of glucose, and slightly higher temperature in comparison with non-tumor microenvironment. For example, a microenvironment restricted ASTR is virtually inactive at normal body temperature, but is active at a higher temperature in a tumor microenvironment. In yet another aspect, the microenvironment restricted antibody is less active in normal oxygenated blood, but more active under a less oxygenated environment that exists in a tumor. In yet another aspect, the microenvironment restricted antibody is less active in normal physiological pH 7.2-7.8, but more active under an acidic pH 5.8-7.0, or 6.0-6.8 that exists in a tumor microenvironment. For example, the microenvironment restricted antibody is more active at a pH of 6.7 than at pH 7.4. There are other conditions in the tumor microenvironment known to a person skilled in the field that may also be used as the condition under which the conditionally active ASTRs have different binding affinities. *In vitro* assay conditions that mimic these *in vivo* tumor conditions are referred to herein as *in vitro* tumor surrogate assay conditions.

Any one or more of these conditions can be simulated *in vitro* by choice of the particular assay buffer. The composition of the assay buffer that simulates a diseased microenvironment can be selected to be identical to the composition of the assay buffer that simulate a normal environment, with the exception of one or more conditions known or described herein that is altered in the diseased microenvironment. Further, in screening or identifying the activity of one or more polypeptides under two different sets of conditions, generally the only conditions that are varied in the assay relate to the buffer conditions simulating the *in vivo* microenvironment. The other conditions of the assay, such as time, temperature and incubation conditions, can be the same for both sets of conditions. Typically, the same base buffer is used in the set of conditions that simulate a diseased microenvironment and conditions that simulate a normal microenvironment, but the design of the buffer composition can be made to differ in one or more parameters such as pH, oxygen, pressure, presence of co-factors, pH, hyaluronan concentration (such as increased or decreased hyaluronan concentration), lactate concentration (such as increased or decreased lactate concentration), albumin concentration (such as increased or decreased hyaluronan concentration) and/or pyruvate concentration (including increased or decreased pyruvate concentration). In the conditions that simulate a diseased microenvironment and the conditions that simulate a normal microenvironment, any base buffer known to one of skill in the art that can be used

### METHODS OF GENERATING A MICROENVIRONMENT RESTRICTED CELL

The present disclosure provides a method of generating a microenvironment restricted cell. The method generally involves genetically modifying a mammalian cell with an expression vector (e.g. a plasmid or a retroviral vector), or an RNA (e.g., *in vitro* transcribed RNA), including nucleotide sequences encoding microenvironment restricted CARs of the present disclosure. The genetically modified cell is microenvironment restricted in the presence of one or more target antigens. The genetic modification can be carried out *in vivo, in vitro,* or *ex vivo.* The cell can be an immune cell (e.g., a T lymphocyte, a T-helper cell, or an NK cell), a stem cell, a progenitor cell, etc.

In some cases, the genetic modification is carried out *ex vivo.* For example, a T lymphocyte, a stem cell, a T-helper cell, or an NK cell is obtained from an individual; and the cell obtained from the individual is genetically modified to express a CAR of the present disclosure. The genetically modified cell is microenvironment restrictable in the presence of one or more target antigens. In some cases, the genetically modified cell is activated *ex vivo.* In other cases, the genetically modified cell is introduced into an individual (e.g., the individual from whom the cell was obtained); and the genetically modified cell is activated *in vivo.* For example, where the one or more target antigens are present on the surface of a cell in the individual, there is no need to administer the antigen. The genetically modified cell comes into contact with the antigen present on the surface of a cell in the individual and the genetically modified cell is activated. For example, where the genetically modified cell is a T lymphocyte, the genetically modified cell can exhibit cytotoxicity toward a cell that expresses the one or more target antigens on its surface to which the CAR binds.

### METHODS FOR MODULATING MRB CAR-EXPRESSING T CELL AND/OR NK CELL ACTIVITY BY CHANGING pH

Provided herein in certain aspects, are methods for modulating binding and resulting lysis/killing of a target cell by an MRB CAR-expressing T cell and/or NK cell by causing a change or shift in pH within a microenvironment that includes a target cell either within a target tissue or within one or more non-target (e.g. healthy/normal) tissues, by modulating binding of the MRB-CAR to its cognate antigen on a target cell(s). Such methods typically include contacting a target cell, such as a mammalian cell (e.g. a human cell) with an MRB CAR-expressing T cell and/or NK cell in a microenvironment and then changing the pH of the microenvironment, either by decreasing or more typically increasing the pH. The microenvironment can be a target microenvironment, for example a tumor, or an off-target microenvironment, where off-target binding can cause side-effects. In some embodiments, such methods can provide a transient reduction of tumor microenvironment sensitive CAR-T target binding.

Accordingly, in one aspect, provided herein is a method for modulating binding of a microenvironment restricted biologic chimeric antigen receptor (MRB-CAR)-expressing T cell or NK cell to a cell expressing a cognate antigen of the MRB-CAR in a subject, that includes the following:
a. introducing a T cell and/or NK cell comprising a nucleic acid encoding the MRB-CAR into the subject, wherein after (and optionally and/or during) the introducing, the T cell and/or the NK cell comprising the nucleic acid encoding the MRB-CAR expresses the MRB-CAR and binds to the cell expressing the cognate antigen in the subject; and
b. administering a pharmacologic agent to the subject in sufficient amount to increase blood pH and/or pH of a tissue and/or pH of a microenvironment, wherein the administering is performed before, during, or after the introducing, and wherein the increased pH of the blood, the tissue, and/or the microenvironment modulates binding of the MRB-CAR expressing T cell and/or NK cell to the cell expressing the cognate antigen in the blood, the tissue, or the microenvironment with the increased pH.

The change/shift in pH in aspects that include a step of administering a pH-modulating pharmacologic agent of the present disclosure can be accomplished by exposing target or non-target cells/tissue to a pH-modulating pharmacologic agent, such as by administering the pH modulating pharmacologic agent to a subject. Non-limiting examples of pH-modulating pharmacologic agents are provided herein. In certain aspects, provided herein is a pharmacologic agent for use in a method for modulating binding of an MRB-CAR to its cognate antigen or for modulating binding of an MRB CAR-expressing T cell and/or NK cell to a cell that expresses its cognate antigen or for reducing or alleviating on target off tumor toxicity in a subject. Such aspects in certain embodiments, relate to treating tumor growth, cancer, hyperplasia, or cell proliferative disorders.

In other aspects, provided herein is a use of a pH-modulating pharmacologic agent for use in the manufacture of a medicament or a kit for controlling binding of a genetically engineered T cell and/or NK cell to a target mammalian cell in a subject *in vivo.* In other aspects, provided herein is a kit that includes a container containing a replication incompetent recombinant retroviral particle, and instructions for use thereof for performing a method for treating tumor growth, wherein the instructions instruct a method for controlling binding of a T cell and/or NK cell to a target mammalian cell by modulating pH. Such method can be any of the methods provided herein this section for modulating MRB CAR-expressing T cell and/or NK cell binding and/or activity by changing pH. The container that contains the recombinant retroviral particles can be a tube, vial, well of a plate, or other vessel for storage of a recombinant retroviral particle and/or a pH-modulating pharmacologic agent. Any of these can be of industrial strength and grade. The kit can include two or more containers in certain embodiments. One container/vessel can include the recombinant retroviral particles and another container/vessel can include a pH-modulating pharmacologic agent. In such methods the pharmacologic agent is delivered/administered in sufficient amount to increase blood pH and/or a tissue pH and/or a microenvironment pH to modulate binding of the MRB-CAR of a modified/recombinant T cell and/or NK cell expressing the MRB CAR, to its cognate antigen in the blood and/or the tissue with the increased pH. Non-limiting exemplary details are provided herein for administering a pH modulating pharmacologic agent in sufficient amount and for a sufficient time.

Target cells, whether on target or off target with respect to a tissue, can be contacted with a pH modulating agent, such as a pH modulating pharmacologic agent, after introducing the MRB-CAR into a subject. Accordingly, exemplary aspects provided herein for modulating binding and/or cytotoxic activity of an MRB CAR-expressing T cell, for example for alleviating on target off tumor activity and/or for inhibiting target cell proliferation, such as tumor cell proliferation, can include the following steps:
a. introducing a T cell and/or NK cell comprising a nucleic acid encoding an MRB-CAR into a subject wherein after the introducing, the T cell and/or the NK cell comprising the nucleic acid encoding the MRB-CAR expresses the MRB-CAR, and optionally binds to the cell expressing the cognate antigen in the subject; and
b. administering a pharmacologic agent to the subject in sufficient amount to increase blood pH and/or a tissue pH and/or a microenvironment pH to modulate binding of the MRB CAR-expressing T cell and/or NK cell to cells expressing the cognate antigen of the MRB CAR, in the blood, the tissue, or the microenvironment with the increased pH. It will be understood that depending on the specific method used to introduce the nucleic acid encoding the MRB-CAR into the T cell and/or NK cell, the T cell and/or NK cell may or may not express the MRB-CAR before it is introduced into the subject. However, at some timepoint after introduction into the subject, e.g. 2 hours, 4 hours, 8 hours, 12 hours, 1 day, 2 days, 4 days and/or 7 days, or longer, the T cell and/or NK cell that include the nucleic acid encoding the MRB-CAR, express the MRB-CAR. Then such cells typically bind to a target cell expressing the cognate antigen for the MRB-CAR.

Methods provided herein for genetically modifying and optionally expanding lymphocytes of a subject can be used to introduce a nucleic acid sequence that encodes an MRB-CAR into the genome of a T cell and/or NK cell of the subject to produce an T cell and/or NK cell capable of expressing the MRB CAR, and then to introduce the T cell and/or NK cell capable of expressing the MRB CAR into the subject, wherein after introducing the T cell and/or NK cell expresses the MRB CAR in order to contact the MRB-CAR with a target cells/tissue. The present disclosure provides details of how to perform such methods, along with various alternatives for modifying and expanding lymphocytes, any of which can be used in aspects of the disclosure that include changing pH to modulate binding of an MRB CAR-expressing T cell and/or NK cell to a target cell expressing a cognate antigen for the MRB-CAR.

Such methods for genetically modifying and expanding lymphocytes typically involve contacting T cells and/or NK cells, which can be resting cells in illustrative embodiments, with a replication incompetent recombinant retroviral particle to transduce the T cells and/or NK cells. Such contacting typically occurs *ex vivo* after removing the lymphocytes from the subject. The T cells and/or NK cells are then introduced/reintroduced into the subject, typically from whom they were removed. The replication incompetent recombinant retroviral particle includes a genome with a polynucleotide that encodes the MRB-CAR. Many alternative embodiments and further details regarding such a replication incompetent recombinant retroviral particle are provided in other sections herein and can be used in methods provided herein for regulating binding and resulting lysis/killing of MRB-CARs by modulating pH in a microenvironment of a cell expressing a cognate target polypeptide recognized by the MRB-CAR in a pH-dependent manner.

Particularly illustrative aspects that include such combinations can include other elements for regulating binding, cell killing activity, and/or survival of MRB CAR-expressing T cells that are provided herein in other sections. Such control elements that can be combined with MRB CAR-expression in methods that include a change in pH as well as other embodiments provided herein, include riboswitches and elimination domains, Thus, the combination of such methods and compositions provided herein, form a powerful multi-faceted approach to assuring safety of a subject after administration of CAR-expressing T cells, including MRB CAR-expressing T cells, to a subject.

Such methods for modulating binding of a target cell by an MRB CAR-expressing T cell and/or NK cell can be used, for example, to reduce on target, off-tumor toxicity by increasing the pH of blood and/or a non-tumor tissue(s) within the subject. For example, in a situation where a "normal" tissue pH within a subject becomes transiently lower, a pH modulating agent can be delivered in a manner where pH of the normal tissue is increased while pH of the tumor remains lower and still at a pH where the MRB-CAR-expressing T cell and/or NK cell binds a target tumor cell. In these embodiments, the pH modulating agent can be delivered at a lower concentration or in a targeted manner to the normal tissue.

In some embodiments, this can be accomplished while allowing the pH within the tumor microenvironment to remain low enough for an MRB-CAR T cell and/or NK cell to bind to its cognate target-expressing cells within the tumor. In illustrative aspects of methods provided herein, the pH of a tissue remains at a pH under which an MRB CAR-expressing T cell and/or NK cell binds its target for a period of time sufficient for a MRB CAR-expressing T cell and/or NK cell to contact and bind to a cell expressing its cognate antigen (e.g. 2, 4, 8, 12, or 24 hours, or 2, 4, 7, 14, 28, or 30 days, or 1, 2, 3, 4, 5, 6, 12, 24 months, or longer), and then the pH is shifted/changed, for example by increasing the pH of the tissue to such a magnitude as to affect binding of the MRB CAR-expressing T cell and/or NK cell to a target cell.

Accordingly, provided herein, in one aspect, is a method for transient reduction of tumor microenvironment sensitive CAR-T cell target binding through pharmacologic modification of vascular and tissue pH. The target binding portions of the tumor microenvironment sensitive CAR-T cell with different binding in different conditions are also referred to as microenvironment restricted biologic, microenvironment restricted, microenvironmentally controlled, or conditionally active and can refer to the entire CAR or any target binding domain thereof, for example, an ASTR, scFv, or scFvFc. These microenvironmentally controlled ASTRs in CAR-T cells provide an additional level of protection against on-target off tumor toxicity, requiring tumor local environmental conditions to enable T cell engagement. While attractive for some monoclonal antibody therapies, adoptive cellular therapy may create local environments that are transiently permissive for their CAR-T targets. For example, CAR-T cells activated in tissues with a low pH may further reduce the pH of the microenvironment, depending on cytoplasmic domains present in the CAR construct. In other instances, cytokine release syndrome and other morbidity associated with adoptive cellular therapy may result in loss of the bicarbonate buffering capacity of blood, leading to lactic acidosis. It has been established that adoptive cellular therapies administered by intravenous infusion result in temporary pulmonary entrapment. For some cellular therapies, infusion rate requires constant monitoring of dissolved oxygen (Fischer et al. Stem Cells Dev. 2009 Jun; 18(5): 683-691). The extent of pulmonary entrapment is dependent upon cell size, activation state, cell dose, and infusion rate. Cruz et al (Cytotherapy. 2010 Oct; 12(6): 743-749) report the adverse findings from over 300 T cell infusions, that low doses and slow infusion may reduce pulmonary entrapment. However, with certain high potency CAR-T cells, targets present even in low levels on lung endothelium, such as Her2 (Morgan et al. Mol Ther. 2010 Apr; 18(4): 843-851), can result in immediate toxicity that cannot be controlled, and results in rapid patient deterioration due to the initial high CAR-T cellular concentration in the lung following infusion and the presence of the T cell target in these tissues. In other cases, the presence of T cell targets in other off target tissues such as bile duct may create on target off tumor toxicities that cannot be controlled (Lamers Mol Ther. 2013 Apr;21(4):904-12) and result in severe organ toxicity before other agents such as steroids or cell elimination epitopes can be utilized. While venous and arterial plasma have strong buffering capacity against acidosis, conditions of respiratory acidosis, shock, metabolic acidosis and ischemic acidosis can occur in patients with cancer treated with adoptive cellular therapy.

In some aspects provided herein, the binding of an MRB-CAR in a subject can be modulated by administering a pharmacologic agent to the subject to increase or decrease the pH of the blood, a tissue and/or a microenvironment. In some aspects, on-target off tumor toxicity can be alleviated in a subject by administering a pharmacologic agent to the subject to increase or decrease the blood pH and/or the pH of a tissue and/or the pH of a microenvironment. In some aspects, the binding of a T cell and/or NK cell to a target mammalian cell can be controlled by introducing a pharmacologic agent to increase or decrease the blood pH and/or the pH of a tissue and/or the pH of a microenvironment. In some aspects, the binding of a genetically engineered T cell and/or NK cell to a target mammalian cell in a subject *in vivo* can be controlled by administering a pH-modulating pharmacologic agent to the subject. In illustrative embodiments, the pharmacologic agent can increase the blood pH and/or the pH of a tissue and/or the pH of a microenvironment. In some embodiments, the microenvironment can be an *in vivo* microenvironment. In illustrative embodiments, the microenvironment can be a tumor microenvironment. In some embodiments, the microenvironment can include a target mammalian cell, wherein the target mammalian cell expressed the target antigen on its surface. In some embodiments, administering a pharmacologic agent to a subject can increase the pH of blood, a tissue, and/or a microenvironment from a pH of less than 5.8, 5.9, 6.0, 6.1, 6.2, 6.3, 6.4, 6.5, 6.6, 6.7, 6.8, or 6.9 to a pH of at least 6.6, 6.7, 6.8, 6.9, 7.0, 7.1, 7.2, 7.3, 7.4, 7.5, or 7.6, wherein the pH of the blood, tissue, and/or microenvironment is lower before administering the pharmacologic agent than after administering the pharmacologic agent. In some embodiments, administering a pharmacologic agent to a subject can decrease the pH of blood, a tissue, or a microenvironment from a pH of more than 6.6, 6.7, 6.8, 6.9, 7.0, 7.1, 7.2, 7.3, 7.4, 7.5, or 7.6 to a pH of less than 5.8, 5.9, 6.0, 6.1, 6.2, 6.3, 6.4, 6.5, 6.6, 6.7, 6.8, 6.9, or 7.0, wherein the pH of the blood, tissue, and/or microenvironment is higher before administering the pharmacologic agent than after administering the pharmacologic agent. In some embodiments, administering a pharmacologic agent to a subject can cause a pH shift in the subject in the blood, a tissue, and/or a microenvironment. In some embodiments, the pH shift can be at least 0.1, 0.2, 0.3, 0.4, 0.5, 0.6, 0.7, 0.8, 0.9, 1.0, 1.1, 1.2, 1.3, 1.4, 1.5, 1.6, 1.7, or 1.8 pH units in either direction, i.e. an increase or decrease in pH after administering the pharmacologic agent relative to the pH before administering the pharmacologic agent. In illustrative embodiments, the pH shift is an increase in pH.

The MRB-CARs of the present disclosure can have reduced binding to its cognate antigen at one pH than at a different pH. In illustrative embodiments where illustrative pH values for differential binding of an MRB-CAR are not already provided in the broadest aspect and alternatively for other embodiments in place of those values for such aspects, the MRB-CAR can have reduced binding at a higher pH than at a lower pH. For example, the MRB-CAR can have reduced binding to its cognate antigen at a pH above 7.0, 7.1, 7.2, 7.3, 7.4, or 7.5 than at a pH below 6.4, 6.5, 6.6, 6.7, 6.8, 6.9, or 7.0. In other embodiments, the MRB-CAR can have reduced binding at a higher pH than at a lower pH. For example, the MRB-CAR can have reduced binding to its cognate antigen at a pH below 6.4, 6.5, 6.6, 6.7, 6.8, 6.9, or 7.0 than at a pH above 7.0, 7.1, 7.2, 7.3, 7.4, or 7.5. In some illustrative embodiments, the MRB-CAR exhibits increased binding at a pH of 6.5 to 6.7 compared to pH 7.4 to 7.6. In other illustrative embodiments, the MRB-CAR exhibits increased binding at a pH of 6.7 compared to a pH of 7.4. In other embodiments, the MRB-CAR exhibits increased binding in the pH of a tumor compared to the pH of blood. In some embodiments, the MRB-CAR can include an antigen-specific targeting region, a stalk, and an intracellular activating domain. In some embodiments, the MRB-CAR can also include a co-stimulatory domain. In some embodiments, the MRB-CAR can bind to a tumor associated antigen.

In methods that include modulating the pH of the blood, a tissue, or a microenvironment, the pH of the microenvironment can be increased from a pH below 7.0 to a pH above 7.0. For example, the pH can be increased from a pH below 6.4, 6.5, 6.6, 6.7, 6.8, 6.9, or 7.0 to a pH above 7.0, 7.1, 7.2, 7.3, or 7.4. In some embodiments, the MRB-CAR can bind to the cognate antigen at the increased pH but not a pH of the microenvironment before introducing the pharmacologic agent. In certain embodiments, the pH can be increased from below 7.0 to a pH of 7.1 to 8.0 or to a pH of 7.1 to 7.8 or to a pH of 7.2 to 7.8 or a pH of 7.2 to 7.6 or a pH of 7.3 to 7.6 or to a pH of 7.4 to 7.8 or to a pH of 7.4 to 7.6. Such an increase in pH can occur for less than 1, 2, 4, 6, 8, 12, or 24 hours or for more than 1, 2, 4, 6, 8, 12 or 24 hours depending on the type and dose of pharmacologic agent administered. In certain embodiments, the pharmacologic agent is administered such that the pH remains above 7.0, 7.1, 7.2, 7.3, 7.4, or 7.5; or between 7.0, 7.1, 7.2, 7.3 on the low end of the range and 7.4, 7.5, 7.6, 7.7, or 7.8 on the high end of the range, in the target tissue, such as a tumor, and for example in at least a surface of a target tissue (e.g. tumor) microenvironment, in at least a portion of a target tissue (e.g. tumor) microenvironment, and in illustrative embodiments throughout a target tissue (e.g. tumor) microenvironment. The microenvironment can be an in vivo microenvironment, such as a tumor, a tissue, a non-tumor tissue, a normal tissue, or a tissue that has undergone a transient shift in pH. For example, tissues that typically undergo transient shifts in pH include a muscle tissue in anaerobic conditions or muscle tissue undergoing exercise or an inflamed tissue or a tissue experiencing inflammation. In some embodiments that include a target mammalian cell, the target mammalian cell can be a tumor cell or a non-tumor or normal cell.

In some aspects, methods for transiently increasing vascular pH to reduce affinity of microenvironmentally controlled MRB-CARs for their antigens are provided. A 0.4U shift in blood pH can reduce the affinity of certain scFvs that form a portion of an MRB-CAR, for their cognate antigen by greater than 10-fold. In some embodiments, therapeutic pH control can be achieved via IV or oral administration routes of various pharmacologic agents. For example, in some embodiments, inactivation of binding affinity can be achieved with bicarbonate or sodium bicarbonate. In other embodiments, Tris-hydroxymethyl aminomethane (also known as tromethamine, trometamol, and THAM) and/or Carbicarb^{™} (an equimolar hypertonic solution of sodium bicarbonate and sodium carbonate) can be utilized to increase the pH of the blood in a sufficient amount to alleviate on-target off tumor toxicities. In still other embodiments, small molecule proton pump inhibitors can be utilized to increase blood pH and/or tissue pH in a sufficient amount to alleviate on-target off tumor toxicities. Proton pump inhibitors that can be used in methods that include modulating pH include, but are not limited to, esomeprazole (Nexium), esomeprazole and naproxen (Vimovo), lansoprazole (Prevacid), omeprazole (Prilosec and Zegerid), and rabeprazole (Aciphex). Administration of proton pump inhibitors can be used effectively over longer time periods to modulate the binding affinity of the antigen biding domain to its cognate antigen for days, weeks, months, or years. In other embodiments, the affinity of the antigen binding domain for its cognate antigen can be modulated by altering the blood pH and/or tissue pH by controlling the transcription, translation, membrane expression, and stability of transporters and pumps. Examples of such transporters and pumps whose altered expression can be to modulate pH include, but are not limited to, proton pumps, members of the sodium proton exchange family (NHE), bicarbonate transporter family (BCT), and monocarboxylate transporter family.

In certain embodiments, a pH-modulating pharmacologic agent, such as, for example, bicarbonate, THAM, or Caricarb^{™} are administered prior to or concurrent with infusion of a patient's CAR-T cells expressing microenvironment restricted biologic ASTRs (e.g. scFvs or scFvFcs). Such treatment will alleviate the immediate cytoxicity that is otherwise associated with the temporary pulmonary entrapment of CAR-T cell infusions. Accordingly, in certain aspects provided herein is a method for reducing cytotoxicity caused to normal, healthy tissue of a subject by administering a pharmacologic agent to the subject in sufficient amount to increase blood pH and/or a tissue pH and/or a microenvironment pH; and either concomitantly or subsequently (e.g. 1, 2, 4, 6, 8, 12, or 24 hours, or 1, 2, 3, 4, or 7 days later) introducing an MRB CAR-expressing T cell or NK cell into the subject. In certain embodiments, at a target time after such introducing (e.g. 1, 2, 4, 6, 8, 12, or 24 hours, or 1, 2, 3, 4, or 7 days later), administration of the pharmacologic agent is terminated for a period of time or indefinitely, in order to change the pH of the blood, a tissue, or a microenvironment of the subject and modulate binding/activity of the MRB CAR-expressing T cell.

Various effective dosing regimens for administering the pharmacologic agents capable of modulating pH (e.g. increasing blood pH and/or a tissue pH and/or the pH of a microenvironment in a subject) can be used, as will be understood by a skilled artisan. Herein, administering can refer to giving a pharmacologic agent to a subject including injecting a pharmacologic agent through an IV into a subject or providing an oral dose of a pharmacologic agent to a subject or a subject taking a pharmacologic agent. The pharmacologic agents can be administered to the subject or patient for various lengths of time, for example, at least 1, 2, 3, 4, 5, or 6 days; 1, 2, 3, 4, 5, 6, 7, 8, 9, 10, or 11 weeks; 3, 4, 5, 6, 7 ,8, 9, 10, 12, 15, or 18 months; or 2, 2.5, 3, 3.5, 4, 4.5, or 5 years or indefinitely. In some embodiments, the pharmacologic agent can be bicarbonate, sodium bicarbonate (NaHCO₃), or a solution of sodium bicarbonate and sodium carbonate and a parenteral or IV dosage can be: 0.2 x weight of subject (kg) x base deficit of the subject; HCO₃ (mEq) required = 0.5 x weight (kg) x [24 - serum HCO₃ (mEq/L)]; or 2 to 5 mEq/kg IV infusion over 4 to 8 hours. In some embodiments, standard dosing regimens of bicarbonate, sodium bicarbonate, or a solution of sodium bicarbonate can be used depending on the severity of the acidosis. For example, 50 to 150 mEq bicarbonate diluted in 1 L of 5% dextrose in water can be administered via IV at a rate of 1 to 1.5 L/hour. In another non-limiting example, 90 to 180 mEq bicarbonate diluted in 1 L of 5% dextrose in water can be administered via IV at a rate of 1 to 1.5 L/hour. In some embodiments where the pharmacologic agent is bicarbonate or sodium bicarbonate (NaHCO₃), an enteral or oral dosage can be, for example, 325 to 2000 mg sodium bicarbonate given to a subject 1 to 4 times/day.

In some embodiments, the pharmacologic agent can be tris-hydroxymethyl aminomethane (also known as tromethamine, trometamol, and THAM) and a parenteral or IV dosage can be estimated as: Tromethamine solution (mL of 0.3 M) required = Body Weight (kg) x Base Deficit (mEq/liter) x 1.1. In some embodiments, the IV dosage of tris-hydroxymethyl aminomethane can be estimated from the buffer base deficit of the extracellular fluid in mEq/L as determined by means of the Siggaard-Andersen nomogram. In some embodiments, the initial dose can be 500 ml (150 mEq) of tris-hydroxymethyl aminomethane injected by slow IV infusion with up to 1000 mL, wherein the maximum dose is 500 mg/kg (227 mg/lb) over a period of not less than one hour.

In some embodiments, the pharmacologic agent can be a small molecule proton pump inhibitor and can be administered for extended treatment lengths. For example, the small molecule proton pump inhibitor can be administered for at least 1, 2, 3, 4, 5, or 6 days; 1, 2, 3, 4, 5, 6, 7, 8, 9, 10, or 11 weeks; 3, 4, 5, 6, 7 ,8, 9, 10, 12, 15, or 18 months; or 2, 2.5, 3, 3.5, 4, 4.5, or 5 years or indefinitely. In some embodiments, the proton pump inhibitor can be esomeprazole (Nexium) and 20 mg or 40 mg esomeprazole can be administered orally once or twice daily. In some embodiments, the proton pump inhibitor can be a combination of esomeprazole and naproxen (Vimovo) and 20 mg esomeprazole with 375 or 500 mg naproxen can be administered orally twice daily. In some embodiments, the proton pump inhibitor can be lansoprazole (Prevacid) and 15, 30, or 60 mg lansoprazole can be administered orally once or twice daily. In some embodiments, lansoprazole can be administered by IV with 30 mg lansoprazole injected over 30 minutes once daily for up to 7 days. The subject can then switch to oral lansoprazole and continue treatment. In some embodiments, the proton pump inhibitor can be omeprazole (Prilosec and Zegerid) and 10, 20, or 40 mg omeprazole can be administered orally once or twice daily. In some embodiments, the proton pump inhibitor can be rabeprazole (Aciphex) and 20 or 60 mg rabeprazole can be administered orally once or twice daily or 100 mg rabeprazole can be administered orally once daily. In any of the embodiments disclosed herein, the pharmacologic agents can be used in combination with each other.

In any of the embodiments disclosed herein, the pH of the blood, a tissue, and/or a microenvironment of a subject can be measured before, during, or after the administration of a pharmacologic agent. In some embodiments, the decision to administer or to continue to administer, to a subject the pharmacologic agent to increase or decrease the pH can be based on the pH measurement of the blood, a tissue, and/or a microenvironment of the subject. Methods to measure the blood pH and/or bicarbonate levels of the blood of a subject are well-known in the art. In some embodiments, positron emission tomography (PET), magnetic resonance spectroscopy (MRS), magnetic resonance imaging (MRI), and optical imaging can be used to measure in vivo pH in microenvironments, for example, in tumors (for details of measuring tumor pH, see: Zhang X, Lin Y, Gillies RJ. Tumor pH and its measurement. J Nucl Med. 2010 Aug;51(8):1167-70).

In another aspect, provided herein is a method for alleviating on target off tumor toxicity in a subject, that includes the following:
a. introducing a polynucleotide encoding an microenvironment restricted biologic chimeric antigen receptor (MRB-CAR) into a T cell or NK cell of the subject to produce a T cell and/or NK cell capable of expressing the MRB-CAR;
b. introducing the T cell and/or NK cell capable of expressing the MRB-CAR into the subject, wherein the T cell and/or NK cell express the MRB-CAR in the subject; and
c. administering a pharmacologic agent to the subject in sufficient amount to increase blood pH and/or pH of a tissue and/or pH of a microenvironment to modulate binding of the MRB-CAR to its cognate antigen in the blood, the tissue, and/or the microenvironment with the increased pH, thereby alleviating on target off tumor toxicity in the subject.

In the introducing step, the T cell or NK cell is capable of expressing the MRB-CAR because it is genetically modified to contain the nucleic acid that encodes the MRB-CAR. This genetic modification can be the presence of the MRB-CAR coding sequence on a vector that has been introduced inside the T cell or NK cell by transfection or transduction. In illustrative embodiments the nucleic acid encoding the MRB-CAR is integrated into the genome of the T cell or NK cell.

It is envisioned that various methods known in the art for introducing a polynucleotide into a T cell and/or NK cell could be used with methods provided herein for aspects that include changing pH to affect binding of an MRB-CAR T cell or NK cell to its cognate antigen on a cell using an agent such as a pH-modulating pharmacologic agent (sometimes referred to herein as "pH Switch aspects"). Typically, a vector, in illustrative examples an expression vector, is used to deliver the polynucleotide. Such vectors can include various vectors known in the art for delivery nucleic acids to T cells and/or NK cells. Illustrative aspects of the claimed invention utilize retroviral vectors and retroviral particles, and in some particularly illustrative embodiments lentiviral vectors and in illustrative embodiments, recombinant lentiviral particles.

Other suitable expression vectors can be used in pH switch aspects provided herein. Such expression vectors include, but are not limited to, viral vectors (e.g. viral vectors based on vaccinia virus; poliovirus; adenovirus (see, e.g., Li et al., Invest Opthalmol Vis Sci 35:2543 2549, 1994; Borras et al., Gene Ther 6:515 524, 1999; Li and Davidson, PNAS 92:7700 7704, 1995; Sakamoto et al., H Gene Ther 5:1088 1097, 1999; WO 94/12649, WO 93/03769; WO 93/19191; WO 94/28938; WO 95/11984 and WO 95/00655); adeno-associated virus (see, e.g., Ali et al., Hum Gene Ther 9:81 86, 1998, Flannery et al., PNAS 94:6916 6921, 1997; Bennett et al., Invest Opthalmol Vis Sci 38:2857 2863, 1997; Jomary et al., Gene Ther 4:683 690, 1997, Rolling et al., Hum Gene Ther 10:641 648, 1999; Ali et al., Hum Mol Genet 5:591 594, 1996; Srivastava in WO 93/09239, Samulski et al., J. Vir. (1989) 63:3822-3828; Mendelson et al., Virol. (1988) 166:154-165; and Flotte et al., PNAS (1993) 90: 10613-10617); SV40; herpes simplex virus; or a retroviral vector (e.g., Murine Leukemia Virus, spleen necrosis virus, and vectors derived from retroviruses such as Rous Sarcoma Virus, Harvey Sarcoma Virus, avian leukosis virus, human immunodeficiency virus, myeloproliferative sarcoma virus, and mammary tumor virus), for example a gamma retrovirus; or human immunodeficiency virus (see, e.g., Miyoshi et al., PNAS 94:10319 23, 1997; Takahashi et al., J Virol 73:7812 7816, 1999); and the like.

In some embodiments, DNA-containing viral particles are utilized instead of recombinant retroviral particles. Such viral particles can be adenoviruses, adeno-associated viruses, herpesviruses, cytomegaloviruses, poxviruses, avipox viruses, influenza viruses, vesicular stomatitis virus (VSV), or Sindbis virus. A skilled artisan will appreciate how to modify the methods disclosed herein for use with different viruses and retroviruses, or retroviral particles. Where viral particles are used that include a DNA genome, a skilled artisan will appreciate that functional units can be included in such genomes to induce integration of all or a portion of the DNA genome of the viral particle into the genome of a T cell and/or NK cell transduced with such virus. Alternatively, functional DNA can be delivered to a T cell and/or NK cell that is expressed in the cell but is not integrated into the genome of the T cell and/or NK cell.

In illustrative embodiments, the vector used in a pH switch aspect of the present disclosure is a recombinant retroviral particle and in certain embodiments, a recombinant lentiviral particle. Such retroviral particle typically includes a retroviral genome within a capsid which is located within a viral envelope. The present disclosure in various sections herein, provide various embodiments of recombinant retroviral particles that disclose elements that can be included on the surface or within, and/or in the genome of a recombinant retroviral particle. Any of these recombinant retroviral particle embodiments can be used in the pH switch aspects provided herein.

### INHIBITORY RNA MOLECULES

In certain embodiments, methods provided herein for the present disclosure include inhibiting expression of one or more endogenous genes expressed in T cells and/or NK cells. Methods provided herein illustrate the ability to make recombinant retroviral particles that express one or more, and in illustrative embodiments two or more, inhibitory RNA molecules, such as for example, a miRNA or shRNA, that can be used for such methods. In fact, the methods provided herein illustrate that such inhibitory RNA molecules can be encoded within introns, including for example, an Ef1a intron. This takes advantage of the present teachings of methods to maximize the functional elements that can be included in a packageable retroviral genome to overcome shortcomings of prior teachings and maximize the effectiveness of such recombinant retroviral particles in adoptive T cell therapy.

In some embodiments, the inhibitory RNA molecule includes a 5' strand and a 3' strand (in some examples, sense strand and antisense strand) that are partially or fully complementary to one another such that the two strands are capable of forming a 18-25 nucleotide RNA duplex within a cellular environment. The 5' strand can be 18, 19, 20, 21, 22, 23, 24, or 25 nucleotides in length, and the 3' strand can be 18, 19, 20, 21, 22, 23, 24, or 25 nucleotides in length. The 5' strand and the 3' strand can be the same or different lengths, and the RNA duplex can include one or more mismatches. Alternatively, the RNA duplex has no mismatches.

The inhibitory RNA molecules included in compositions and methods provided herein, in certain illustrative examples, do not exist and/or are not expressed naturally in T cells into whose genome they are inserted. In some embodiments, the inhibitory RNA molecule is a miRNA or an shRNA. In some embodiments, where reference is made herein or in priority filings, to a nucleic acid encoding an siRNA, especially in a context where the nucleic acid is part of a genome, it will be understood that such nucleic acid is capable of forming an siRNA precursor such as miRNA or shRNA in a cell that is processed by DICER to form a double stranded RNA that typically interacts with, or becomes part of a RISK complex. In some embodiments, an inhibitory molecule of an embodiment of the present disclosure is a precursor of a miRNA, such as for example, a Pri-miRNA or a Pre-miRNA, or a precursor of an shRNA. In some embodiments, the miRNA or shRNA are artificially derived (i.e. artificial miRNAs or siRNAs). In other embodiments, the inhibitory RNA molecule is a dsRNA (either transcribed or artificially introduced) that is processed into an siRNA or the siRNA itself. In some embodiments, the miRNA or shRNA has a sequence that is not found in nature, or has at least one functional segment that is not found in nature, or has a combination of functional segments that are not found in nature.

In some embodiments, inhibitory RNA molecules are positioned in the first nucleic acid molecule in a series or multiplex arrangement such that multiple miRNA sequences are simultaneously expressed from a single polycistronic miRNA transcript. In some embodiments, the inhibitory RNA molecules can be adjoined to one another either directly or indirectly by non-functional linker sequence(s). The linker sequence in some embodiments, is between 5 and 120 nucleotides in length, and in some embodiments can be between 10 and 40 nucleotides in length, as non-limiting examples. In illustrative embodiments the first nucleic acid sequence encoding one or more (e.g. two or more) inhibitory RNAs and the second nucleic acid sequence encoding a CAR (e.g. an MRB-CAR) are operably linked to a promoter that is active constitutively or that can be induced in a T cell or NK cell. As such, the inhibitory RNA molecule(s) (e.g. miRNAs) as well as the CAR are expressed in a polycistronic manner. Additionally, functional sequences can be expressed from the same transcript. For example, any of the lymphoproliferative elements provided herein that are not inhibitory RNA molecules, can be expressed from the same transcript as the CAR and the one or more (e.g. two or more) inhibitory RNA molecules.

In some embodiments, the inhibitory RNA molecule is a naturally occurring miRNA such as but not limited to miR-155. Alternatively, artificial miRNAs can be produced in which sequences capable of forming a hybridizing/complementary stem structure and directed against a target RNA, are placed in a miRNA framework that includes microRNA flanking sequences for microRNA processing and a loop, which can optionally be derived from the same naturally occurring miRNA as the flanking sequences, between the stem sequences. Thus, in some embodiments, an inhibitory RNA molecule includes from 5' to 3' orientation: a 5' microRNA flanking sequence, a 5' stem, a loop, a 3' stem that is partially or fully complementary to said 5' stem, and a 3' microRNA flanking sequence. In some embodiments, the 5' stem (also called a 5' arm herein) is 18, 19, 20, 21, 22, 23, 24 or 25 nucleotides in length. In some embodiments, the 3' stem (also called a 3' arm herein) is 18, 19, 20, 21, 22, 23, 24, or 25 nucleotides in length. In some embodiments, the loop is 3 to 40, 10 to 40, 20 to 40, or 20 to 30 nucleotides in length, and in illustrative embodiments the loop can be 18, 19, 20, 21, or 22 nucleotides in length. In some embodiments, one stem is two nucleotides longer than the other stem. The longer stem can be the 5' or the 3' stem.

In some embodiments, the 5' microRNA flanking sequence, 3' microRNA flanking sequence, or both, are derived from a naturally occurring miRNA, such as but not limited to miR-155, miR-30, miR-17-92, miR-122, and miR-21. In certain embodiments, the 5' microRNA flanking sequence, 3' microRNA flanking sequence, or both, are derived from a miR-155, such as, e.g, the miR-155 from Mus musculus or Homo sapiens. Inserting a synthetic miRNA stem-loop into a miR-155 framework (i.e. the 5' microRNA flanking sequence, the 3' microRNA flanking sequence, and the loop between the miRNA 5' and 3' stems) is known to one of ordinary skill in the art (Chung, K. et al. 2006. Nucleic Acids Research. 34(7):e53; US 7,387,896). The SIBR (synthetic inhibitory BIC-derived RNA) sequence (Chung *et al.* 2006 supra), for example, has a 5' microRNA flanking sequence consisting of nucleotides 134-161 (SEQ ID NO:256) of the Mus musculus BIC noncoding mRNA (Genbank ID AY096003.1) and a 3' microRNA flanking sequence consisting of nucleotides 223-283 of the Mus musculus BIC noncoding mRNA (Genbank ID AY096003.1). In one study, the SIBR sequence was modified (eSIBR) to enhance expression of miRNAs (Fowler, D.K. et al. 2015. Nucleic acids Research 44(5):e48). In some embodiments of the present disclosure, miRNAs can be placed in the SIBR or eSIBR miR-155 framework. In illustrative embodiments herein, miRNAs are placed in a miR-155 framework that includes the 5' microRNA flanking sequence of miR-155 represented by SEQ ID NO:256, the 3' microRNA flanking sequence represented by SEQ ID NO:260 (nucleotides 221-265 of the *Mus musculus* BIC noncoding mRNA); and a modified miR-155 loop (SEQ ID NO:258). Thus, in some embodiments, the 5' microRNA flanking sequence of miR-155 is SEQ ID NO:256 or a functional variant thereof, such as, for example, a sequence that is the same length as SEQ ID NO:256, or 95%, 90%, 85%, 80%,75%, or 50% as long as SEQ ID NO: 256 or is 100 nucleotides or less, 95 nucleotides or less, 90 nucleotides or less, 85 nucleotides or less, 80 nucleotides or less, 75 nucleotides or less, 70 nucleotides or less, 65 nucleotides or less, 60 nucleotides or less, 55 nucleotides or less, 50 nucleotides or less, 45 nucleotides or less, 40 nucleotides or less, 35 nucleotides or less, 30 nucleotides or less, or 25 nucleotides or less; and is at least 50%, 55%, 60%, 65%, 70%, 75%, 80%, 85%, 90%, or 95% identical to SEQ ID NO:256. In some embodiments, the 3' microRNA flanking sequence of miR-155 is SEQ ID NO:260 or a functional variant thereof, such as, for example, the same length as SEQ ID NO:260, or 95%, 90%, 85%, 80%,75%, or 50% as long as SEQ ID NO: 260 or is a sequence that is 100 nucleotides or less, 95 nucleotides or less, 90 nucleotides or less, 85 nucleotides or less, 80 nucleotides or less, 75 nucleotides or less, 70 nucleotides or less, 65 nucleotides or less, 60 nucleotides or less, 55 nucleotides or less, 50 nucleotides or less, 45 nucleotides or less, 40 nucleotides or less, 35 nucleotides or less, 30 nucleotides or less, or 25 nucleotides or less; and is at least 50%, 55%, 60%, 65%, 70%, 75%, 80%, 85%, 90%, or 95% identical to SEQ ID NO:260. However, any known microRNA framework that is functional to provide proper processing within a cell of miRNAs inserted therein to form mature miRNA capable of inhibiting expression of a target mRNA to which they bind, is contemplated within the present disclosure.

In some embodiments, at least one, at least two, at least three, or at least four of the inhibitory RNA molecules encoded by a nucleic acid sequence in a polynucleotide of a replication incompetent recombinant retroviral particle has the following arrangement in the 5' to 3' orientation: a 5' microRNA flanking sequence, a 5' stem, a loop, a 3' stem that is partially or fully complementary to said 5' stem, and a 3' microRNA flanking sequence. In some embodiments, all of the inhibitory RNA molecules have the following arrangement in the 5' to 3' orientation: a 5' microRNA flanking sequence, a 5' stem, a loop, a 3' stem that is partially or fully complementary to said 5' stem, and a 3' microRNA flanking sequence. As disclosed herein, the inhibitory RNA molecules can be separated by one or more linker sequences, which in some embodiments have no function except to act as spacers between inhibitory RNA molecules.

In some embodiments, where two or more inhibitory RNA molecules (in some examples, 1, 2, 3, 4, 5, 6, 7, 8, 9, or 10 inhibitory RNA molecules) are included, these inhibitory RNA molecules are directed against the same or different RNA targets (such as e.g. mRNAs transcribed from genes of interest). In illustrative embodiments, between 2 and 10, 2 and 8, 2 and 6, 2 and 5, 3 and 5, or 3 and 6 inhibitory RNA molecules are included in the first nucleic acid sequence. In an illustrative embodiment, four inhibitory RNA molecules are included in the first nucleic acid sequence.

In some embodiments, the RNA targets are mRNAs transcribed from genes that are expressed by T cells such as but not limited to PD-1 (prevent inactivation); CTLA4 (prevent inactivation); TCRa (safety - prevent autoimmunity); TCRb (safety - prevent autoimmunity); CD3Z (safety - prevent autoimmunity); SOCS1 (prevent inactivation); SMAD2 (prevent inactivation); a miR-155 target (promote activation); IFN gamma (reduce CRS); cCBL (prolong signaling); TRAIL2 (prevent death); PP2A (prolong signaling); ABCG1 (increase cholesterol microdomain content by limiting clearance of cholesterol). In illustrative examples, miRNAs inserted into the genome of T cells in methods provided herein, are directed at targets such that proliferation of the T cells is induced and/or enhanced and/or apoptosis is suppressed.

In some embodiments, the RNA targets include mRNAs that encode components of the T cell receptor (TCR) complex. Such components can include components for generation and/or formation of a T cell receptor complex and/or components for proper functioning of a T cell receptor complex. Accordingly, in one embodiment at least one of the two or more of inhibitory RNA molecules causes a decrease in the formation and/or function of a TCR complex, in illustrative embodiments one or more endogenous TCR complexes of a T cell. The T cell receptor complex includes TCRa, TCRb, CD3d, CD3e, CD3g, and CD3z. It is known that there is a complex interdependency of these components such that a decrease in the expression of any one subunit will result in a decrease in the expression and function of the complex. Accordingly, in one embodiment the RNA target is an mRNA expressing one or more of TCRa, TCRb, CD3d, CD3e, CD3g, and CD3z endogenous to a transduced T cell. In certain embodiments, the RNA target is mRNA transcribed from the endogenous TCRα or TCRβ gene of the T cell whose genome comprises the first nucleic acid sequence encoding the one or more miRNAs. In illustrative embodiments, the RNA target is mRNA transcribed from the TCRα gene. In certain embodiments, inhibitory RNA molecules directed against mRNAs transcribed from target genes with similar expected utilities can be combined. In other embodiments, inhibitory RNA molecules directed against target mRNAs transcribed from target genes with complementary utilities can be combined. In some embodiments, the two or more inhibitory RNA molecules are directed against the mRNAs transcribed from the target genes CD3Z, PD1, SOCS1, and/or IFN gamma.

In some embodiments provided herein, the two or more inhibitory RNA molecules can be delivered in a single intron, such as but not limited to EF1-αa intron A. Intron sequences that can be used to harbor miRNAs for the present disclosure include any intron that is processed within a T cell. As indicated herein, one advantage of such an arrangement is that this helps to maximize the ability to include miRNA sequences within the size constraints of a retroviral genome used to deliver such sequences to a T cell in methods provided herein. This is especially true where an intron of the first nucleic acid sequence includes all or a portion of a promoter sequence used to express that intron, a CAR sequence, and other functional sequences provided herein, such as lymphoproliferative element(s) that are not inhibitory RNA molecules, in a polycistronic manner. Sequence requirements for introns are known in the art. In some embodiments, such intron processing is operably linked to a riboswitch, such as any riboswitch disclosed herein. Thus, the riboswitch can provide a regulatory element for control of expression of the one or more miRNA sequences on the first nucleic acid sequence. Accordingly, in illustrative embodiments provided herein is a combination of an miRNA directed against an endogenous T cell receptor subunit, wherein the expression of the miRNA is regulated by a riboswitch, which can be any of the riboswitches discussed herein.

In some embodiments, inhibitory RNA molecules can be provided on multiple nucleic acid sequences that can be included on the same or a different transcriptional unit. For example, a first nucleic acid sequence can encode one or more inhibitory RNA molecules and be expressed from a first promoter and a second nucleic acid sequence can encode one or more inhibitory RNA molecules and be expressed from a second promoter. In illustrative embodiments, two or more inhibitory RNA molecules are located on a first nucleic acid sequence that is expressed from a single promoter. The promoter used to express such miRNAs, are typically promoters that are inactive in a packaging cell used to express a retroviral particle that will deliver the miRNAs in its genome to a target T cell, but such promoter is active, either constitutively or in an inducible manner, within a T cell. The promoter can be a Pol I, Pol II, or Pol III promoter. In some illustrative embodiments, the promoter is a Pol II promoter.

### CHARACTERIZATION AND COMMERCIAL PRODUCTION METHODS

The present disclosure provides various methods and compositions that can be used in as research reagents in scientific experimentation and for commercial production. Such scientific experimentation can include methods for characterization of lymphocytes, such as NK cells and in illustrative embodiments, T cells using methods for genetically modifying, for example transducing lymphocytes provided herein. Such methods for example, can be used to study activation of lymphocytes and the detailed molecular mechanisms by which activation makes such cells transducible. Furthermore, provided herein are genetically modified lymphocytes that will have utility for example, as research tools to better understand factors that influence T cell proliferation and survival. Such genetically modified lymphocytes, such as NK cells and in illustrative embodiments T cells, can furthermore be used for commercial production, for example for the production of certain factors, such as growth factors and immunomodulatory agents, that can be harvested and tested or used in the production of commercial products.

The scientific experiments and/or the characterization of lymphocytes can include any of the aspects, embodiments, or subembodiments provided herein useful for analyzing or comparing lymphocytes. In some embodiments, T cells and/or NK cells can be transduced with the replication incompetent recombinant retroviral particles provided herein that include polynucleotides. In some embodiments, transduction of the T cells and/or NK cells can include polynucleotides that include polynucleotides encoding polypeptides of the present disclosure, for example, CARs, lymphoproliferative elements, and/or activation elements. In some embodiments, the polynucleotides can include inhibitory RNA molecules as discussed elsewhere herein. In some embodiments, the lymphoproliferative elements can be chimeric lymphoproliferative elements.

### TREATMENT METHODS

The present disclosure provides various treatment methods using a CAR. A CAR of the present disclosure, when present in a T lymphocyte or an NK cell, can mediate cytotoxicity toward a target cell. A CAR of the present disclosure binds to an antigen present on a target cell, thereby mediating killing of a target cell by a T lymphocyte or an NK cell genetically modified to produce the CAR. The ASTR of the CAR binds to an antigen present on the surface of a target cell.

The present disclosure provides methods of killing, or inhibiting the growth of, a target cell, the method involving contacting a cytotoxic immune effector cell (e.g., a cytotoxic T cell, or an NK cell) that is genetically modified to produce a subject CAR, such that the T lymphocyte or NK cell recognizes an antigen present on the surface of a target cell, and mediates killing of the target cell.

The present disclosure provides a method of treating a disease or disorder in an individual having the disease or disorder, the method including: a. introducing an expression vector including a polynucleotide sequence encoding a CAR into peripheral blood cells obtained from the subject to produce a genetically engineered cytotoxic cell; and b. administering the genetically engineered cytotoxic cell to the subject.

In methods provided herein that include administering genetically modified T cells and/or NK cells into a subject, especially where the subject has, or is suspected of having cancer, the methods can further include delivering an effective dose of an immune checkpoint inhibitor to the subject. This checkpoint inhibitor delivery can occur before, after, or at the same time as administering the genetically modified T cells and/or NK cells. Immune checkpoint inhibitors are known and various compounds are approved and in clinical development. Check point molecules, many of which are the target of checkpoint inhibitor compounds, include the following, with some checkpoint inhibitors noted:
CD27. This molecule supports antigen-specific expansion of naïve T cells and is vital for the generation of T cell memory. Celldex Therapeutics is working on CDX-1127, an agonistic anti-CD27 monoclonal antibody.
CD28. This molecule is constitutively expressed on almost all human CD4+ T cells and on around half of all CD8 T cells. CD28 was the target of the TGN1412 'superagonist'.
CD40. This molecule, found on a variety of immune system cells including antigen presenting cells has CD40L, otherwise known as CD154 and transiently expressed on the surface of activated CD4+ T cells, as its ligand. An anti-CD40 agonist monoclonal antibody was licensed to Roche.
CD122. This molecule, which is the Interleukin-2 receptor beta sub-unit, is known to increase proliferation of CD8+ effector T cells. Nektar Therapeutics has developed NKTR-214, a CD122-biased immune-stimulatory cytokine.
CD137. When this molecule, also called 4-1BB, is bound by CD137 ligand, the result is T-cell proliferation. Pieris Pharmaceuticals has developed an engineered lipocalin that is bi-specific for CD137 and HER2.
OX40. This molecule, also called CD134, has OX40L, or CD252, as its ligand. Like CD27, OX40 promotes the expansion of effector and memory T cells, however it is also noted for its ability to suppress the differentiation and activity of T-regulatory cells, and also for its regulation of cytokine production. AstraZeneca has three drugs in development targeting OX40: MEDI0562 is a humanised OX40 agonist; MEDI6469, murine OX4 agonist; and MEDI6383, an OX40 agonist.
GITR, short for Glucocorticoid-Induced TNFR family Related gene, prompts T cell expansion, including Treg expansion. TG Therapeutics is working on anti-GITR antibodies.
ICOS. This molecule, short for Inducible T-cell costimulator, and also called CD278, is expressed on activated T cells. Jounce Therapeutics is developing an ICOS agonist.
A2AR. The Adenosine A2A receptor is regarded as an important checkpoint in cancer therapy because adenosine in the immune microenvironment, leading to the activation of the A2a receptor, is negative immune feedback loop and the tumor microenvironment has relatively high concentrations of adenosine.
B7-H3, also called CD276, was originally understood to be a co-stimulatory molecule but is now regarded as co-inhibitory. MacroGenics is working on MGA271 is an Fc-optimized monoclonal antibody that targets B7-H3.
B7-H4, also called VTCN1, is expressed by tumor cells and tumor-associated macrophages and plays a role in tumour escape.
BTLA. This molecule, short for B and T Lymphocyte Attenuator and also called CD272, has HVEM (Herpesvirus Entry Mediator) as its ligand.
CTLA-4, short for Cytotoxic T-Lymphocyte-Associated protein 4 and also called CD152, is the target of Bristol-Myers Squibb's compound Yervoy.
IDO, short for Indoleamine 2,3-dioxygenase, is a tryptophan catabolic enzyme with immune-inhibitory properties. Another important molecule is TDO, tryptophan 2,3-dioxygenase. IDO. Newlink Genetics and Incyte have identified IDO pathway inhibitors.
KIR, short for Killer-cell Immunoglobulin-like Receptor, is a receptor for MHC Class I molecules on Natural Killer cells. Bristol-Myers Squibb has developed Lirilumab, a monoclonal antibody to KIR.
LAG3, short for Lymphocyte Activation Gene-3, works to suppress an immune response by action to Tregs as well as direct effects on CD8+ T cells. Bristol-Myers Squibb has developed an anti-LAG3 monoclonal antibody called BMS-986016.
PD-1, short for Programmed Death 1 (PD-1) receptor, has two ligands, PD-L1 and PD-L2. This checkpoint is the target of Merck & Co.'s melanoma drug Keytruda,
TIM-3, short for T-cell Immunoglobulin domain and Mucin domain 3, expresses on activated human CD4+ T cells and regulates Th1 and Th17 cytokines.
VISTA (protein), Short for V-domain Ig suppressor of T cell activation, VISTA is primarily expressed on hematopoietic cells.

### SUBJECTS SUITABLE FOR TREATMENT

A variety of subjects are suitable for treatment with the methods and compositions presented herein. Suitable subjects include any individual, e.g., a human or non-human animal who has a disease or disorder, who has been diagnosed with a disease or disorder, who is at risk for developing a disease or disorder, who has had a disease or disorder and is at risk for recurrence of the disease or disorder, who has been treated with an agent for the disease or disorder and failed to respond to such treatment, or who has been treated with an agent for the disease or disorder but relapsed after initial response to such treatment.

Subjects suitable for treatment with an immunomodulatory method include individuals who have an autoimmune disorder; individuals who are organ or tissue transplant recipients; and the like; individuals who are immunocompromised; and individuals who are infected with a pathogen.

### EXEMPLARY EMBODIMENTS

Provided in this Exemplary Embodiments section are exemplary aspects and embodiments provided herein and further discussed throughout this specification. For the sake of brevity and convenience, all of the disclosed aspects and embodiments and all of the possible combinations of the disclosed aspects and embodiments are not listed in this section. It will be understood that embodiments are provided that are specific embodiments for many aspects, as discussed in this entire disclosure. It is intended in view of the full disclosure herein, that any individual embodiment recited below or in this full disclosure can be combined with any aspect recited below or in this full disclosure where it is an additional element that can added to an aspect or because it is narrower element for an element already present in an aspect. Such combinations are discussed more specification in other sections of this detailed description.

In one aspect, provided herein is a replication incompetent recombinant retroviral particle including a polynucleotide including:
A. one or more transcriptional units operatively linked to a promoter active in T cells and/or NK cells, wherein the one or more transcriptional units encode a chimeric antigen receptor (CAR); and
B. a pseudotyping element and an activation element (e.g. an NK cell activation element or in illustrative embodiments a T cell activation element) on its surface, wherein the activation element is not encoded by a polynucleotide in the replication incompetent recombinant retroviral particle.

In another aspect, provided herein is a replication incompetent recombinant retroviral particle, including a polynucleotide including one or more transcriptional units operatively linked to a promoter active in T cells and/or NK cells, wherein the one or more transcriptional units encode a first polypeptide including a chimeric antigen receptor (CAR) and a second polypeptide including a lymphoproliferative element. Various embodiments for such particle, such as, but not limited to, lymphoproliferative elements, activation elements, pseudotyping elements, control elements, CARs, and other elements, are provided herein in this section and in this disclosure.

In another aspect, provided herein is a replication incompetent recombinant retroviral particle, comprising a polynucleotide comprising one or more transcriptional units operatively linked to a promoter active in T cells and/or NK cells, wherein the one or more transcriptional units encode a first polypeptide comprising a chimeric antigen receptor (CAR) and a second polypeptide comprising a chimeric lymphoproliferative element, for example a constitutively active chimeric lymphoproliferative element. In illustrative embodiments, the chimeric lymphoproliferative element does not comprise a cytokine tethered to its cognate receptor or tethered to a fragment of its cognate receptor. Various embodiments for such particle, such as, but not limited to, lymphoproliferative elements, activation elements, pseudotyping elements, control elements, CARs, and other elements, are provided herein in this section and in this disclosure.

In another aspect, provided herein is a replication incompetent recombinant retroviral particle, comprising:
A. a polynucleotide comprising one or more transcriptional units operatively linked to a promoter active in T cells and/or NK cells, wherein the one or more transcriptional units encode a chimeric antigen receptor (CAR); and
B. a pseudotyping element and a T cell activation element on its surface, wherein the T cell activation element is not encoded by a polynucleotide in the replication incompetent recombinant retroviral particle, and wherein the T cell activation element is an anti-CD3 scFvFc antibody. Various embodiments for such particle, such as, but not limited to, lymphoproliferative elements, activation elements, pseudotyping elements, control elements, CARs, and other elements, are provided herein in this section and in this disclosure.

In another aspect, provided herein are replication incompetent recombinant retroviral particles, comprising:
A. one or more pseudotyping elements capable of binding to a T cell and/or an NK cell and facilitating membrane fusion of the replication incompetent recombinant retroviral particles thereto;
B. a polynucleotide comprising one or more transcriptional units operatively linked to a promoter active in T cells and/or NK cells, wherein the one or more transcriptional units encode a first engineered signaling polypeptide comprising a chimeric antigen receptor comprising an antigen-specific targeting region, a transmembrane domain, and an intracellular activating domain, and a second engineered signaling polypeptide comprising at least one lymphoproliferative element; wherein expression of the first engineered signaling polypeptide and/or the second engineered signaling polypeptide are regulated by a control element; and
C. an activation element on its surface, wherein the activation element is capable of binding to a T cell and/or NK cell and is not encoded by a polynucleotide in the replication incompetent recombinant retroviral particles. Various embodiments for such particle, such as, but not limited to, lymphoproliferative elements, activation elements, pseudotyping elements, control elements, CARs, and other elements, are provided herein in this section and in this disclosure.

In some aspects provided herein is a genetically modified lymphocyte, such as a T cell or NK cell made by transducing resting T cells and/or resting NK cells according to a method comprising contacting the resting T cells and/or resting NK cells ex vivo, with any of the replication incompetent recombinant retroviral particles provided herein, wherein said contacting facilitates transduction of the resting T cells and/or NK cells by the replication incompetent recombinant retroviral particles, thereby producing genetically modified T cells and/or NK cells,. In one embodiment, the replication incompetent recombinant retroviral particles comprise a pseudotyping element on their surface and a membrane-bound T cell activation element on their surface. Various embodiments for such a cell, such as, but not limited to, contacting times, lymphoproliferative elements, activation elements, pseudotyping elements, control elements, and other elements, are provided herein in this section and in this disclosure.

In another aspect provided herein is the use of any replication incompetent recombinant retroviral particle in the manufacture of a kit for genetically modifying a T cell or NK cell, wherein the use of the kit includes the steps of any of the methods aspects and embodiments provided herein. For example, in another aspect, provided herein is the use of a replication incompetent recombinant retroviral particle in the manufacture of a kit for genetically modifying a T cell or NK cell, wherein the use of the kit includes: contacting the T cell or NK cell ex vivo with the replication incompetent recombinant retroviral particle, wherein the replication incompetent recombinant retroviral particle includes a pseudotyping element on a surface and typically a T cell activation element on the surface, wherein said contacting facilitates transduction of the T cell or NK cell by the replication incompetent recombinant retroviral particle, thereby producing a genetically modified T cell or NK cell. In some embodiments, the T cell activation element can be membrane-bound. In illustrative embodiments, the T cell activating element activates the T cell through the T cell receptor associated receptor complex. In some embodiments, the contacting can be performed for between 1, 2, 3, 4, 5, 6, 7, or 8 hours on the low end of the range and 4, 5, 6, 7, 8, 10, 12, 15, 18, 21, and 24 hours on the high end of the range, for example, between 1 and 12 hours or between 1 and 5 hours. In this use aspect and other use aspects herein, the replication incompetent recombinant retroviral particle for use in the manufacture of a kit can include any of the replication incompetent recombinant retroviral particle aspects and embodiments provided herein. Various other embodiments for such methods, such as but not limited to other contacting times, lymphoproliferative elements, activation elements, pseudotyping elements, percent transfected/genetically-modified cells, control elements and other elements are provided herein.

Provided herein in another aspect, is a method of transducing and/or genetically modifying a lymphocyte, typically a T cell and/or an NK cell, and typically a resting T cell and/or resting NK cell, that includes contacting the lymphocyte with a replication incompetent recombinant retroviral particle, wherein the replication incompetent recombinant retroviral particle typically comprises a pseudotyping element on its surface, wherein said contacting (which can also be considered incubating under contacting conditions) facilitates transduction of the resting T cell and/or NK cell by the replication incompetent recombinant retroviral particle, thereby producing the genetically modified T cell and/or NK cell. The pseudotyping element is typically capable of binding the resting T cell and/or NK cell and typically facilitating membrane fusion on its own or in conjunction with other protein(s) of the replication incompetent recombinant retroviral particles. In some embodiments of the method, the replication incompetent recombinant retroviral particle comprises an activation element, such as a T cell activation element that activates a T cell through T cell receptor associated complex. Such an activation element can be an anti-CD3 antibody, for example an anti-CD3 scFv or an antiCD3 scFvFc. Various exemplary and illustrative contacting times are provided herein. Various other embodiments for such methods, such as, but not limited to, contacting times, lymphoproliferative elements, activation elements, pseudotyping elements, percent transfected/genetically-modified cells, control elements and other elements are provided herein.

In another aspect, provided herein is a method for genetically modifying and/or transducing a lymphocyte, comprising contacting the lymphocyte ex vivo, with a replication incompetent recombinant retroviral particle, wherein the replication incompetent recombinant retroviral particle comprises a pseudotyping element on its surface and a membrane-bound T cell activation element on its surface, wherein said contacting facilitates transduction of the lymphocyte by the replication incompetent recombinant retroviral particles, thereby producing a genetically modified lymphocyte. In some embodiments, the membrane-bound T cell activation element is an anti-CD3 antibody, for example an anti-CD3 scFv or an antiCD3 scFvFc. Various other embodiments for such methods, such as, but not limited to, contacting times, lymphoproliferative elements, activation elements, pseudotyping elements, percent transfected/genetically-modified cells, control elements and other elements are provided herein.

Provided in another aspect herein, is a method of transducing resting lymphocytes of a subject, comprising contacting resting T cells and/or resting NK cells of a subject *ex vivo,* with replication incompetent recombinant retroviral particles, wherein the replication incompetent recombinant retroviral particles comprise a pseudotyping element on their surface that is capable of binding a resting T cell and/or resting NK cell and facilitating membrane fusion of the replication incompetent recombinant retroviral particles thereto, wherein said contacting facilitates transduction of the resting T cells and/or NK cells by the replication incompetent recombinant retroviral particles, thereby producing genetically modified T cells and/or NK cells. In illustrative embodiments of this aspect, at least 10%, 20%, or 25% of the resting T cells and/or NK cells, or between 10% and 70%, between 10% and 50%, or between 20% and 50% of T cells and/or NK cells are transduced as a result of the process. Various other embodiments for such methods, such as, but not limited to, contacting times, lymphoproliferative elements, activation elements, pseudotyping elements, percent transfected/genetically-modified cells, control elements and other elements are provided herein.

Provided in another aspect herein is a method for transducing resting T cells and/or resting NK cells from isolated blood, comprising:
A. collecting blood from a subject;
B. isolating peripheral blood mononuclear cells (PBMCs) comprising resting T cells and/or resting NK cells;
C. contacting the resting T cells and/or resting NK cells of the subject *ex vivo,* with replication incompetent recombinant retroviral particles, wherein the replication incompetent recombinant retroviral particles comprise a pseudotyping element on their surface that is capable of binding a resting T cell and/or resting NK cell and facilitating membrane fusion of the replication incompetent recombinant retroviral particles thereto, wherein said contacting facilitates transduction of at least 5% of the resting T cells and/or resting NK cells by the replication incompetent recombinant retroviral particles, thereby producing genetically modified T cells and/or NK cells, thereby transducing resting T cells and/or NK cells. Various other embodiments for such methods, such as, but not limited to, contacting times, lymphoproliferative elements, activation elements, pseudotyping elements, percent transfected/genetically-modified cells, control elements and other elements are provided herein.

Typically, a packaging cell line is used to produce the replication incompetent recombinant retroviral particle as discussed elsewhere herein. In some embodiments, the packaging cell line can be a suspension cell line. In illustrative embodiments, the packaging cell line can be grown in serum free media. In some embodiments, the lymphocyte can be from a subject. In illustrative embodiments, the lymphocyte can be from blood of a subject. Various other embodiments for such methods, such as, but not limited to, contacting times, lymphoproliferative elements, activation elements, pseudotyping elements, percent transfected/genetically-modified cells, control elements and other elements are provided herein.

In another aspect, provided herein is method of transducing a lymphocyte with a replication incompetent recombinant retroviral particle comprising:
A. transfecting a packaging cell with a group of vectors comprising components of the replication incompetent recombinant retroviral particle, wherein the packaging cell is grown in suspension in a serum-free media;
B. harvesting the replication incompetent recombinant retroviral particle from the serum-free media; and
C. contacting the lymphocyte with the replication incompetent recombinant retroviral particle. Various embodiments for such methods, such as, but not limited to, contacting times, lymphoproliferative elements, activation elements, pseudotyping elements, percent transfected/genetically-modified cells, control elements and other elements are provided herein.

In another aspect, provided herein is a method of transducing a lymphocyte with a replication incompetent recombinant retroviral particle comprising:
A. culturing a packaging cell in suspension in serum-free media, wherein the packaging cell comprises nucleic acid sequences encoding a packagable RNA genome of the replication incompetent retroviral particle, a REV protein, a gag polypeptide, a pol polypeptide, and a pseudotyping element;
B. harvesting the replication incompetent recombinant retroviral particle from the serum-free media; and
C. contacting the lymphocyte with the replication incompetent recombinant retroviral particle,
wherein the contacting is performed for less than 12 hours, thereby transducing the lymphocyte. Various other embodiments for such methods, such as, but not limited to, other contacting times, lymphoproliferative elements, activation elements, pseudotyping elements, percent transfected/genetically-modified cells, control elements and other elements are provided herein.

Unless incompatible with, or already stated in an aspect, in illustrative embodiments, the packaging cell is an immortalized cell comprising DNA nucleic acids stably integrated therein that encodes the packagable RNA genome. In some embodiments, the gag polypeptide and the pol polypeptide are expressed from one or more inducible promoters and wherein the method further comprises during the culturing, adding a transactivator to induce expression of the gag polypeptide and the pol polypeptide from the one or more inducible promoters. Various embodiments for such methods, such as, but not limited to, contacting times, lymphoproliferative elements, activation elements, control elements, and other elements are provided herein.

Unless incompatible with, or already stated in an aspect, in illustrative embodiments of any of the methods aspects for genetically modifying and/or transducing lymphocytes, for example T lymphocytes, for genetically modifying and expanding lymphocytes, for example T lymphocytes, or for performing cellular therapy herein, or similar methods the contacting can be carried out for between 15, 30 or 45 minutes or 1, 2, 3, 4, 5, 6, 7, or 8 hours on the low end of the range, and between 2, 4, 6, 8, 10, 12, 18, 24, 36, 48, and 72 hours on the high end of the range. For example, in illustrative embodiments, the contacting is carried out for between 2 and 24 hours, between 2 and 20 hours, between 2 and 6 hours, between 1 and 20 hours, between 1 and 12 hours, between 1 and 4 hours, between 4 and 12 hours, or between 4 and 8 hours. In some embodiments, the contacting of a PBMC, NK cell and in illustrative embodiments a T cell with a replication incompetent recombinant retroviral particle, in any of the methods, processes to produce products, or uses thereof unless stated, or stated otherwise in a broadest aspect, provided herein, can be performed for between 1 and 24 hours, for example, between 1 and 12 hours, between 1 and 6 hours. In some embodiments, the contacting can be performed for less than 24 hours, for example, less than 12 hours, less than 8 hours, or less than 4 hours. In illustrative embodiments, the contacting is performed for between 1 and 14 hours, between 1 and 12 hours, between 1 and 6 hours, between 1 and 4 hours, or between 2 and 14 hours. Such methods can be performed without prior activation.

Unless incompatible with, or already stated in an aspect, further embodiments of any of the above methods, use, or product by process aspects for transducing and/or genetically modifying a lymphocyte, for example an NK cell or in illustrative embodiments, a T cell, can include any of the embodiments of replication incompetent recombinant retroviral particles, lymphoproliferative elements, CARs, pseudotyping elements, riboswitches, activation elements, membrane-bound cytokines, miRNAs, and/or other elements disclosed herein. In certain illustrative embodiments, the retroviral particle is a lentiviral particle.

Unless incompatible with, or already stated in an aspect, in any of the methods, kits, uses, or compositions (e.g. polynucleotides, packing cells, or replication incompetent recombination retroviral particles) provided herein, he replication incompetent recombinant retroviral particle(s) comprises or further comprises an activation element on their surface that is capable of activating a resting T cell and/or a resting NK cell. Typically, the activation element such as a T cell activation element is not encoded by a polynucleotide in the replication incompetent recombinant retroviral particle.

Unless incompatible with, or already stated in an aspect, in any of the methods, kits, uses, or compositions (e.g. polynucleotides, packing cells, or replication incompetent recombination retroviral particles) provided herein that recite a T cell and/or a NK cell, or a resting T cell or a resting NK cell, in certain illustrative embodiments, the cell is a T cell.

Unless incompatible with, or already stated in an aspect, typically, the recombinant retroviral particle in any of the methods, kits, uses, or compositions (e.g. polynucleotides, packing cells, or replication incompetent recombination retroviral particles) provided herein, is replication incompetent, *i.e.* cannot replicate. In illustrative embodiments, the retrovirus is a lentivirus, such as a replication incompetent or replication defective HIV lentivirus. In illustrative embodiments, the retroviral particle is a lentiviral particle, such as a replication incompetent or replication defective HIV lentiviral particle. Thus, in illustrative embodiments of any of the methods, kits, uses, or compositions (e.g. polynucleotides, packing cells, or replication incompetent recombination retroviral particles) provided herein, if not already recited, or not already recited otherwise, the replication incompetent recombinant retroviral particle is a lentiviral particle and/or the genetically modified cell is a genetically modified T cell.

Unless incompatible with, or already stated in an aspect, in illustrative embodiments of any of the aspects provided herein, the lymphocytes can be T cells and/or NK cells. In further illustrative embodiments, the T cells and/or NK cells can be resting T cells and/or resting NK cells.

Unless incompatible with, or already stated in an aspect, in illustrative embodiments of any of the methods aspects for transducing, genetically modifying and expanding lymphocytes or for performing adoptive cellular therapy herein, or similar methods, between 10% and 75%, or 10% and 70%, or 10% and 60%, or 10% and 50%, or 10% and 25%, or 20% and 75%, or 20% and 50%, or at least 10%, 20%, or 25% of resting T cells are transduced and between 0% and 75% of NK cells are transduced. In other embodiments, between 5% and 80%, or 10% and 80%, or 10% and 70%, or 10% and 60%, or 10% and 50%, or 10% and 25%, or 10% and 20%, or 20% and 50% of resting NK cells are transduced.

Unless incompatible with, or already stated in an aspect,in illustrative embodiments of any of the methods aspects for genetically modifying and expanding lymphocytes or for performing adoptive cellular therapy herein, or similar methods or any compositions provided herein, expression of said second engineered signaling polypeptide is regulated by the control element.

Unless incompatible with, or already stated in an aspect, in any of the method, use, product by process, or composition aspects herein that include a genetically modified T cell or NK cell or a replication incompetent recombinant retroviral particle, such genetically modified T cell or NK cell or replication incompetent recombinant retroviral particle can include a polynucleotide comprising one or more transcriptional units operatively linked to a promoter active in T cells and/or NK cells, wherein the one or more transcriptional units encode a first polypeptide comprising a chimeric antigen receptor (CAR) and/or a second polypeptide comprising a T cell lymphoproliferative element. In some embodiments, expression of the CAR and/or the lymphoproliferative element are under the control of a control element or of different control elements for each. In some embodiments the CAR and the lymphoproliferative element can be expressed on the same polypeptide and in illustrative embodiments the CAR and the lymphoproliferative element are expressed as separate polypeptides. In some embodiments the CAR is an MRB CAR. In some embodiments, the chimeric lymphoproliferative element is constitutively active, for example a constitutively active chimeric cytokine receptor. In such embodiments, the constitutively active chimeric cytokine receptor can be under the control of a control element.

Unless incompatible with, or already stated in an aspect, in any of the aspects herein that include a polynucleotide comprising one or more transcriptional units operatively linked to a promoter active in T cells and/or NK cells, wherein the one or more transcriptional units encode a first polypeptide comprising a chimeric antigen receptor (CAR) and/or a second polypeptide comprising a T cell lymphoproliferative element, the polynucleotide can further comprise one or more of a Kozak-related sequence, a WPRE element, and a multiple stop sequence, such as a double stop codon or a triple stop codon, wherein one or more of the stop codons of the double stop codon or the triple stop codon define a termination of a reading from of at least one of the one or more transcriptional units. In certain embodiments, the polynucleotide comprises a Kozak-type sequence selected from CCACCAT/UG(G), CCGCCAT/UG(G), GCCGCCGCCAT/UG(G), or GCCGCCACCAT/U(G). In certain embodiments both the -3 and +4 nucleotides relative to a start codon of the first nucleic acid sequence comprise a G. In another embodiment, which can be combined with the preceding embodiment that comprises a Kozak-type sequence and/or the following embodiment that includes triple stop codon, the polynucleotide comprises a WPRE element. In some embodiments, the WPRE element is located 3' of a stop codon of the one or more transcriptional units and 5' to a 3' LTR of the polynucleotide. In another embodiment, which can be combined with either or both of the preceding embodiments (i.e. an embodiment wherein the polynucleotide comprises a Kozak-type sequence and/or an embodiment wherein the polynucleotide comprises a WPRE), the one or more transcriptional units terminates with one or more stop codons of a double stop codon or a triple stop codon.

Unless incompatible with, or already stated in an aspect, in illustrative embodiments of any of the methods, uses, and compositions provided herein that include a lymphoproliferative element, the lymphoproliferative element can be a chimeric lymphoproliferative element. In any of the embodiments herein that include a chimeric lymphoproliferative element, the chimeric lymphoproliferative element can be a chimeric cytokine receptor. In any of the embodiments herein that include a chimeric lymphoproliferative element, the chimeric lymphoproliferative element can be IL-7 tethered to IL-7 receptor alpha, or other than IL-7 tethered to IL-7 receptor alpha. In some embodiments, the chimeric cytokine receptor comprises IL-7 tethered to the IL-7 receptor alpha, or a fragment thereof that retains the ability to promote proliferation of T cells and/or NK cells, and wherein the chimeric cytokine receptor is constitutively active. In some embodiments, the chimeric cytokine receptor comprises IL-7 covalently attached to a functional extracellular fragment of the IL-7 receptor capable of binding to IL-7, an IL-7 transmembrane domain, and an IL-7 signaling domain.

Unless incompatible with, or already stated in an aspect, in any of the aspects or embodiments herein that include a chimeric lymphoproliferative element, the chimeric lymphoproliferative element can be other than a chimeric cytokine receptor. In illustrative embodiments of any of the methods and compositions provided herein that include a lymphoproliferative element, the chimeric lymphoproliferative element can be a chimeric cytokine receptor. In illustrative embodiments of any of the methods and compositions provided herein that include a chimeric cytokine receptor, the chimeric cytokine receptor comprises an intracellular signaling domain of an IL-7 receptor, an intracellular signaling domain of an IL-12 receptor, an intracellular signaling domain of an IL-15/IL-2 receptor, such an IL-15/IL-2 beta receptor, an intracellular signaling domain of an IL-21 receptor, an intracellular signaling domain of an IL-23 receptor, an intracellular signaling domain of an IL-27 receptor, an intracellular signaling domain of a transforming growth factor β (TGFβ) decoy receptor, or CD28. In some embodiments, the chimeric cytokine receptor comprises IL-7 fused to the IL-7 receptor.

Unless otherwise stated, or already stated in a broadest aspect, in illustrative embodiments of any of the methods and compositions provided herein that include a lymphoproliferative element, the lymphoproliferative element can comprise a T cell survival motif. The T cell survival motif can comprise all or a functional fragment of IL-7 receptor, IL-15 receptor, or CD28. In other embodiments, the lymphoproliferative element can include a cytokine or cytokine receptor polypeptide, or a fragment thereof comprising a signaling domain. For example, the lymphoproliferative element can comprise an interleukin polypeptide and the lymphoproliferative element can further comprise a portion of its cognate interleukin receptor polypeptide covalently attached via a linker. Typically, this portion of the cognate interleukin receptor includes a functional portion of the extracellular domain capable of binding the interleukin cytokine and the transmembrane domain. In some embodiments, the intracellular domain is an intracellular portion of the cognate interleukin receptor. In some embodiments, the intracellular domain is an intracellular portion of a different cytokine receptor that is capable of promoting lymphocyte proliferation and optionally survival *ex vivo* or *in vitro* in culture in the absence of exposure of the lymphocyte to exogenous cytiokines such as IL-15, IL-7, and in illustrative embodiments IL-2 and in the absence of the target for an ASTR of a CAR expressed by the lymphocytes during culturing for 6, 7, 14, 21, or 35 days, or longer.

Unless incompatible with, or already stated in an aspect, in some embodiments the lymphoproliferative element is an interleukin polypeptide covalently attached to its full length cognate interleukin receptor polypeptide via a linker. Alternatively, the lymphoproliferative element can be an intracellular signaling domain of an IL-7 receptor, an intracellular signaling domain of an IL-12 receptor, an intracellular signaling domain of IL-23, an intracellular signaling domain of IL-27, an intracellular signaling domain of an IL-15 receptor, an intracellular signaling domain of an IL-21 receptor, or an intracellular signaling domain of a transforming growth factor β (TGFβ) decoy receptor.

Unless incompatible with, or already stated in an aspect,,In some illustrative embodiments, the lymphoproliferative element is constitutively active. Furthermore, the lymphoproliferative element can include a mutated IL-7 receptor or a fragment thereof, which can further include a constitutively active mutated IL-7 receptor or a constitutively active fragment thereof. In some embodiments, the lymphoproliferative element or chimeric cytokine receptor comprises an IL7 InsPPCL mutant. Unless incompatible with, or already stated in an aspect, in any of the aspects herein that include a lymphoproliferative element, the lymphoproliferative element can be any of the CLEs recited in the Lymphoproliferative Element section herein. For example, in illustrative embodiments the CLEs comprise domains from genes specifically called out as especially noteworthy and/or Top Constructs with respect to the Examples herein.

In certain illustrative embodiments, the lymphoproliferative element comprises a cytokine receptor, or a fragment thereof that includes a signaling domain, that activates a Jak/STAT5 pathway. For example, such lymphoproliferative element can include an intracellular domain of IL21R, IL27R, IL31RA, LIFR, and OSMR. As shown in the experiments of Examples 17 and 18 and Tables 7 to 17, chimeric lymphoproliferative elements that include intracellular domains of these genes were found among the candidate chimeric polypeptides that induced the highest magnitude of proliferation in PBMCs cultured in the absence of exogenous cytokines such as IL-2.

In some embodiments, the lymphoproliferative element can comprise an intracellular domain that is an interleukin or an interleukin receptor and that was a part of a Top Construct identified in Libraries 1A, 1.1A, 2B, 2.1B, 3A, 3.1A, 3B, 3.1B, 4B, and/or 4.1B of Examples 17 and 18 (Tables 7 to 17). In some embodiments, the lymphoproliferative element can comprise an intracellular domain that is a cytokine receptor and that was a part of a Top Construct identified in Libraries 1A, 1.1A, 2B, 2.1B, 3A, 3.1A, 3B, 3.1B, 4B, and/or 4.1B of Examples 17 and 18 (Tables 7 to 17). In some embodiments, the lymphoproliferative element can comprise an intracellular domain that includes at least one ITAM motif and that was a part of a Top Construct identified in Libraries 1A, 1.1A, 2B, 2.1B, 3A, 3.1A, 3B, 3.1B, 4B, and/or 4.1B of Examples 17 and 18 (Tables 7 to 17).

In illustrative embodiments, the lymphoproliferative element can comprise an intracellular domain from IL7R, IL12RB1, IL15RA, or IL27RA, which were present in constructs that showed particularly noteworthy enrichments (i.e. elicited highest magnitude of proliferation) in an initial screen and a repeated screen as detailed in Examples 17 and 18 (Tables 19 to 23).

In illustrative embodiments, the lymphoproliferative element can comprise an intracellular domain from the cytokine receptors CD27, CD40, CRLF2, CSF2RA, CSF3R, EPOR, FCER1G, FCGR2A, FCGR2C, GHR, IFNAR1, IFNAR2, IFNGR2, IL1R1, IL1RL1, IL2RA, IL2RG, IL3RA, IL5RA, IL6R, IL7R, IL9R, IL10RB, IL11RA, IL12RB1, IL13RA1, IL13RA2, IL15RA, IL17RB, IL18R1, IL18RAP, IL20RB, IL22RA1, IL27RA, IL31RA, LEPR, MPL, OSMR, PRLR, TNFRSF4, TNFRSF8, TNFRSF9, TNFRSF14, or TNFRSF18, which were present in constructs that showed particularly noteworthy enrichments (i.e. elicited highest magnitude of proliferation) in an initial screen and a repeated screen as detailed in Examples 17 and 18 (Tables 19 to 23).

In illustrative embodiments, the lymphoproliferative element comprises an intracellular domain from CD3D, CD3E, CD3G, CD79A, CD79B, FCER1G, FCGR2A, or FCGR2C, which include at least one ITAM motif and were present in constructs that showed particularly noteworthy enrichments (i.e. elicited highest magnitude of proliferation) in an initial screen and a repeated screen as detailed in Examples 17 and 18 (Tables 19 to 23).

In some embodiments, the lymphoproliferative element in this paragraph, which were shown in examples 17 and 18 to be active in constructs with only a single intracellular domain, can be the intracellular domain of a lymphoproliferative element with either two or more intracellular domains, or in illustrative embodiments a single intracellular domain, i.e. the lymphoproliferative element does not comprise two or more intracellular domains. In illustrative embodiments, the intracellular domain in a lymphoproliferative element comprises a domain from CD40, CRLF2, CSF2RA, CSF3R, EPOR, FCGR2A, IFNAR2, IFNGR2, IL1R1, IL3RA, IL7R, IL10RB, IL11RA, IL12RB1, IL13RA2, IL18RAP, IL31RA, MPL, MYD88, TNFRSF14, or TNFRSF18, which were present in constructs that showed particularly noteworthy enrichments (i.e. elicited highest magnitude of proliferation) in an initial screen and a repeated screen as detailed in Example 18 as single intracellular signaling domains (Tables 22 and 23). In illustrative embodiments, the intracellular domain in a lymphoproliferative element comprises a domain from IL7R or IL12RB1, which were present in constructs that showed particularly noteworthy enrichments (i.e. elicited highest magnitude of proliferation) in an initial screen and a repeated screen as detailed in Example 18 (Tables 22 and 23). In some embodiments, the intracellular domain in a lymphoproliferative element with a single intracellular domain can be a cytokine receptor. In illustrative embodiments, the cytokine receptor in a lymphoproliferative element with a single intracellular domain comprises a domain from CD40, CRLF2, CSF2RA, CSF3R, EPOR, FCGR2A, IFNAR2, IFNGR2, IL1R1, IL3RA, IL7R, IL10RB, IL11RA, IL12RB1, IL13RA2, IL18RAP, IL31RA, MPL, TNFRSF14, or TNFRSF18, which were present in constructs that showed particularly noteworthy enrichments (i.e. elicited highest magnitude of proliferation) in an initial screen and a repeated screen as detailed in Example 18 (Tables 22 and 23). In some embodiments, the intracellular domain in a lymphoproliferative element can include at least one ITAM motif. In illustrative embodiments, the intracellular domain in a lymphoproliferative element that includes at least one ITAM motif comprises a domain from FCGR2A, which was present in constructs that showed particularly noteworthy enrichments (i.e. elicited highest magnitude of proliferation) in an initial screen and a repeated screen as detailed in Example 18 (Table 22).

In illustrative embodiments, the lymphoproliferative element can comprise a costimulatory domain from CD27, CD28, OX40 (also referred to as TNFRSF4), GITR (also referred to as TNFRSF18), or HVEM (also referred to as TNFRSF14), which were present in constructs that showed particularly noteworthy enrichments (i.e. elicited highest magnitude of proliferation) in an initial screen and a repeated screen as detailed in Examples 17 and 18 (Tables 19 to 23).

In some embodiments, the lymphoproliferative element can be one of the constructs M024-S190-S047, M025-5050-S197, M036-S170-S047, M012-5045-5048, M049-S194-S064, M025-S190-S050, M025-S190-S05, E013-T041-S186-S051, E013-T028-S186-S051, E014-T015-S186-S051, E011-T016-S186-S050, E011-T073-S186-S050, or E013-T011-S186-S211, all of which stimulated proliferation of resting lymphocytes after transduction as shown in Example 21 (FIGs. 35 and 36). In certain embodiments, lymphoproliferative elements comprise an extracellular domain from CSF3R, IL3RA, ICOS, CRLF, CSF2RA, LIFR, or CD40; a first intracellular domain from MyD88, CD40, or MPL, and/or a second intracellular domain from CD27 or MyD88.

In some embodiments, the lymphoproliferative element can be the construct E013-T041-S186-S051, which stimulated proliferation of resting lymphocytes after transduction with a replication incompetent recombinant retroviral particle displaying UCHT1scFvFc-GPI as shown and analyzed in Example 22 (FIGs. 38A and B). In other embodiments the lymphoproliferative element is IL7-IL7RAIL2RB, as shown and analyzed in Example 22.

As detailed in Example 17, for Libraries 1A and 1.1A, constructs with domains from the cytokine receptors CD27, IL1RL1, IL6R, IL31RA, TNFRSF4, or TNFRSF18, showed particularly noteworthy enrichments (i.e. elicited highest magnitude of proliferation) in both Libraries 1A and 1.1A (Table 19).

As detailed in Example 17, for Libraries 2B and 2.1B, constructs with domains from the cytokine receptors CD40, FCER1G, FCGR2C, IFNGR2, GHR, IL10RB, IL11RA, IL13RA2, IL17RB, IL22RA1, TNFRSF14, or TNFRSF9, showed particularly noteworthy enrichments (i.e. elicited highest magnitude of proliferation) in both Libraries 2B and 2.1B (Table 20).

As detailed in Example 18, for Libraries 3A and 3.1A, constructs with domains from the cytokine receptors CD27, CD40, CSF2RA, FCGR2A, MPL, OSMR, TNFRSF4, or TNFRSF18 showed particularly noteworthy enrichments (i.e. elicited highest magnitude of proliferation) in both Libraries 3A and 3.1A (Table 21).

As detailed in Example 18, for Libraries 3B and 3.1B, constructs with domains from the cytokine receptors CD27, CD40, CRLF2, CSF2RA, CSF3R, EPOR, FCER1G, FCGR2A, FCGR2C, IFNAR1, IFNAR2, IFNGR2, IL1RL1, IL2RG, IL3RA, IL5RA, IL6R, IL7R, IL9R, IL10RB, IL11RA, IL12RB1, IL13RA1, IL13RA2, IL15RA, IL18RAP, IL20RB, IL27RA, IL31RA, LEPR, MPL, OSMR, PRLR, TNFRSF4, TNFRSF8, TNFRSF9, TNFRSF14, or TNFRSF18, showed particularly noteworthy enrichments (i.e. elicited highest magnitude of proliferation) in both Libraries 3B and 3.1B (Table 22).

As detailed in Example 18, for Libraries 4B and 4.1B, constructs with domains from the cytokine receptors CD27, CD40, CRLF2, CSF2RA, CSF3R, EPOR, FCGR2A, FCGR2C, IFNAR2, IFNGR2, IL1R1, IL2RA, IL3RA, IL2RG, IL6R, IL7R, IL10RB, IL11RA, IL31RA, IL13RA1, IL13RA2, IL18R1, MPL, OSMR, TNFRSF4, TNFRSF9, or TNFRSF18, showed particularly noteworthy enrichments (i.e. elicited highest magnitude of proliferation) in both Libraries 4B and 4.1B (Table 23).

Unless incompatible with, or already stated in an aspect, in any of the aspects and embodiments disclosed herein such as method, use, compositions, and product by process aspects, that include a lymphoproliferative element, in illustrative embodiments the genetically modified PBMCs, lymphocytes, or genetically modified T cells and/or NK cells are capable of promoting lymphocyte proliferation/expansion and optionally survival *ex vivo* or *in vitro* in culture in the absence of exposure of the cells to cytokines such as IL-15, IL-7, and in illustrative embodiments IL-2 and the target for an ASTR of a CAR expressed by the cells during culturing for 6, 7, 14, 21, or 35 days, or longer. In any of the embodiments disclosed herein including a CAR and a lymphoproliferative element, the genetically modified T cells and/or NK cells can be capable of engraftment *in vivo* in mice without exposure to a target recognized by the CAR.

Unless incompatible with, or already stated in an aspect, in illustrative embodiments of any of the methods and compositions provided herein that include a replication incompetent recombinant retroviral particle(s), the replication incompetent recombinant retroviral particles can comprise on their surface an activation element. In illustrative embodiments, the activation element activates a T cell through T cell receptor associated complex. In some cases, the activation element can activate a T cell alone. In other cases, an activation element requires activation through the TCR receptor complex in order to further activate T cells. Accordingly, in some embodiments the activation element comprises:
A. a membrane-bound polypeptide capable of binding to CD3; and/or
B. a membrane-bound polypeptide capable of binding to CD28.

Furthermore, the membrane-bound polypeptide capable of binding to CD3 is a polypeptide capable of binding to CD3 that can be fused to a heterologous GPI anchor attachment sequence and the membrane-bound polypeptide capable of binding to CD28 can be a polypeptide capable of binding to CD28 that is fused to a heterologous GPI anchor attachment sequence. In some embodiments, the membrane-bound polypeptide capable of binding to CD28 is CD80, CD86, or a functional fragment thereof that is capable of inducing CD28-mediated activation of Akt, such as the extracellular domain of CD80.

Unless incompatible with, or already stated in an aspect, in illustrative embodiments of any of the methods and compositions provided herein that are or include a replication incompetent recombinant retroviral particle, the activation element can be a membrane-bound polypeptide capable of binding CD3, such as an anti-CD3 scFv or an anti-CD3 scFvFc. In illustrative embodiments of any of the methods and compositions provided herein that include a replication incompetent recombinant retroviral particle and an anti-CD3, the anti-CD3 can be an anti-CD3 scFvFc fused to a heterologous GPI anchor attachment sequence. In illustrative embodiments of any of the methods and compositions provided herein that include a replication incompetent recombinant retroviral particle, the membrane-bound polypeptide capable of binding CD3 can be an anti-CD3 scFv bound to a CD14 GPI anchor attachment sequence, and the membrane-bound polypeptide capable of binding to CD28 can be CD80, or the extracellular domain thereof, bound to a CD16B GPI anchor attachment sequence. In illustrative embodiments of any of the methods and compositions provided herein that include a replication incompetent recombinant retroviral particle, the replication incompetent recombinant retroviral particles can comprise on their surface, an anti-CD3 scFv bound to a CD14 GPI anchor attachment sequence, CD80, or the extracellular domain thereof, bound to a CD16B GPI anchor attachment sequence, and a fusion polypeptide of IL-7, or an active fragment thereof, and DAF comprising a GPI anchor attachment sequence. In illustrative embodiments of any of the methods and compositions provided herein that include a replication incompetent recombinant retroviral particle, the IL-7, or an active fragment thereof, and DAF fusion, the anti-CD3 scFV, and the CD80, or extracellular domain thereof each comprises a DAF signal sequence.

Unless otherwise stated, or already stated in a broadest aspect, in illustrative embodiments of any of the methods and compositions provided herein that are or include a replication incompetent recombinant retroviral particle, the replication incompetent recombinant retroviral particles can comprise on their surface a membrane-bound cytokine. The membrane-bound cytokine can be IL-7, IL-15, or an active fragment thereof. In other embodiments, the membrane-bound cytokine is a fusion polypeptide of IL-7, or an active fragment thereof, and DAF. For example, the fusion polypeptide can comprise the DAF signal sequence (nucleotides 1-34 of SEQ ID NO:286), IL-7 without its signal sequence (nucleotides 35-186 of SEQ ID NO:286), and a fragment of DAF that includes its GPI anchor attachment sequence (nucleotides 187-532 of SEQ ID NO:286).

Unless incompatible with, or already stated in an aspect, illustrative embodiments of any of the method and composition aspects provided herein the pseudotyping element can comprise one or more heterologous envelope proteins. In other examples, the pseudotyping element can include one or more viral polypeptides recognized by T cells. The one or more pseudotyping elements can comprise a Measles Virus F polypeptide, a Measles Virus H polypeptide, and/or a fragment thereof. The one or more pseudotyping elements can be cytoplasmic domain deletion variants of a measles virus F polypeptide and/or a measles virus H polypeptide.

Unless incompatible with, or already stated in an aspect, in illustrative embodiments of any of the methods and compositions provided herein that include the control element is the control element can regulate the lymphoproliferative element, wherein the lymphoproliferative element is inactive or less active at promoting proliferation of the T cells and/or NK cells in the absence of the compound, and wherein the compound is a molecular chaperone that binds the lymphoproliferative element and induces the activity of the lymphoproliferative element.

Unless incompatible with, or already stated in an aspect, in illustrative embodiments of any of the methods and compositions provided herein that include the control element, the control element can be a polynucleotide comprising a riboswitch. The riboswitch can be capable of binding a nucleoside analog and the compound that binds the control element is the nucleoside analog. The nucleoside analog can be an antiviral agent. The antiviral agent can be acyclovir or penciclovir.

Unless incompatible with, or already stated in an aspect, in illustrative embodiments of any of the methods and compositions provided herein that include an engineered signaling polypeptide, that includes an ASTR, the ASTR of either or both of the engineered signaling polypeptides can bind to a tumor associated antigen. In some illustrative embodiments, the antigen-specific targeting region of the second engineered polypeptide is a microenvironment restricted antigen-specific targeting region.

Unless incompatible with, or already stated in an aspect, in illustrative embodiments of any of the methods and compositions provided herein that include a replication incompetent recombinant retroviral particle(s), if not explicitly recited in the broadest aspect, the replication incompetent recombinant retroviral particles can encode a recognition domain for a monoclonal antibody approved biologic. In some embodiments, the recognition domain is expressed on the same transcript as the chimeric antigen receptor and wherein the recognition domain is separated from the chimeric antigen receptor by a ribosome skipping and/or cleavage signal. The ribosome skipping and/or cleavage signal can be 2A-1. The recognition domain can include a polypeptide that is recognized by an antibody that recognizes EGFR, or an epitope thereof. The recognition domain can be an EGFR mutant that is recognized by an EGFR antibody and expressed on the surface of transduced T cells and/or NK cells as another control mechanism provided herein. In related embodiments, the recognition domain can include a polypeptide that is recognized by an antibody that recognizes EGFR, or an epitope thereof.

Unless incompatible with, or already stated in an aspect, any of the method, use, product by process, the method can further include collecting blood from a subject. The method can further include after contacting the resting T cells and/or resting NK cells of the subject ex vivo, reintroducing the genetically modified T cells and/or NK cells into the subject. In certain embodiments, the resting T cells and/or resting NK cells that are contacted are from the blood of the subject, and wherein expansion of the genetically modified T cells and/or NK cells occurs in vivo within the subject. In certain embodiments, he steps between the collecting blood and the reintroducing the genetically modified T cells and/or NK cells are performed in no more than 24 hours, 18 hours, 12 hours, 8 hours, 6 hours, or 4 hours. Other times are provided herein. In certain embodiments, the subject is not exposed to a lymphodepleting agent within 7 days of performing the contacting, during the contacting, and/or within 7 days after the genetically modified T cells and/or NK cells are reintroduced into the subject.

Provided herein in one aspect, is a method of transducing and/or genetically modifying lymphocytes (e.g. T cell and/or NK cells), in illustrative embodiments resting lymphocytes (resting T cells and/or NK cells), of a subject, comprising contacting resting T cells and/or resting NK cells of a subject *ex vivo,* with replication incompetent recombinant retroviral particles, wherein the replication incompetent recombinant retroviral particles comprise a pseudotyping element on their surface and a membrane-bound anti-CD3 scFvFc antibody on their surface, that is capable of binding a resting T cell and/or resting NK cell and facilitating membrane fusion of the replication incompetent recombinant retroviral particle thereto, wherein said contacting facilitates transduction of the resting T cells and/or NK cells by the replication incompetent recombinant retroviral particles, thereby producing genetically modified T cells and/or NK cells.

Provided herein in one aspect, is a method for transducing and/or genetically modifying resting T cells and/or resting NK cells from isolated blood, comprising: collecting blood from a subject; isolating peripheral blood mononuclear cells (PBMCs) comprising resting T cells and/or resting NK cells; and contacting the resting T cells and/or resting NK cells of the subject *ex vivo* for an effective time, with replication incompetent recombinant retroviral particles, wherein the replication incompetent recombinant retroviral particle comprise a pseudotyping element on their surface and a membrane-bound anti-CD3 scFvFc antibody on their surface, thereby producing genetically modified T cells and/or NK cells, thereby transducing resting T cells and/or NK cells.

In the aspects herein for transducing and/or genetically modifying T lymphocytes (e.g. T cell and/or NK cells) that include a membrane-bound anti-CD3 scFvFc antibody, the pseudotyping element in certain embodiments is the vesicular stomatitis virus envelope protein (VSV-G). In some embodiments, the replication incompetent retroviral particles further comprise a membrane-bound polypeptide capable of binding to CD28, which can include, for example, an extracellular domain of CD80, CD86, or functional fragments thereof that retains the ability to bind CD28. In some embodiments, the anti-CD3 scFvFc antibody is fused to a heterologous GPI anchor attachment sequence. In some embodiments, the anti-CD3 scFvFc antibody is not encoded by a polynucleotide in the replication incompetent recombinant retroviral particle.

In the aspects herein for transducing and/or genetically modifying T lymphocytes (e.g. T cell and/or NK cells) that include a membrane-bound anti-CD3 scFvFc antibody, the recombinant retroviral particle can further include a polynucleotide comprising one or more transcriptional units operatively linked to a promoter active in T cells and/or NK cells, wherein the one or more transcriptional units encode a chimeric antigen receptor. In some embodiments, the membrane-bound polypeptide capable of binding to CD3 is not encoded by a polynucleotide in the replication incompetent recombinant retroviral particle. In some embodiments, the anti-CD3 scFvFc antibody is not encoded by a polynucleotide in the replication incompetent recombinant retroviral particle.

In another aspect provided herein is a method of transducing and/or genetically modifying resting lymphocytes of a subject, comprising contacting resting T cells and/or resting NK cells of a subject ex *vivo,* with replication incompetent recombinant retroviral particles, wherein the replication incompetent recombinant retroviral particles comprise a pseudotyping element on their surface and a membrane-bound polypeptide capable of binding to CD3 on their surface, but not a membrane-bound polypeptide capable of binding to and activating CD28 on their surface, wherein said contacting facilitates transduction of the resting T cells and/or NK cells by the replication incompetent recombinant retroviral particles, thereby producing genetically modified T cells and/or NK cells.

In another aspect, provided herein is a method for transducing and/or genetically modifying resting T cells and/or resting NK cells from isolated blood, comprising: collecting blood from a subject; isolating peripheral blood mononuclear cells (PBMCs) comprising resting T cells and/or resting NK cells; and contacting the resting T cells and/or resting NK cells of the subject *ex vivo* for an effective time, with replication incompetent recombinant retroviral particles, wherein the replication incompetent recombinant retroviral particles comprise a pseudotyping element on their surface and a membrane-bound polypeptide capable of binding to CD3 on their surface, but not a membrane-bound polypeptide capable of binding to and activating CD28 on their surface, thereby producing genetically modified T cells and/or NK cells, thereby transducing resting T cells and/or NK cells.

In the aspects herein for transducing and/or genetically modifying resting T lymphocytes that include a membrane-bound polypeptide capable of binding to CD3 on their surface, but not a membrane-bound polypeptide capable of binding to and activating CD28 on their surface, the pseudotyping element can be, for example, the vesicular stomatitis virus envelope protein (VSV-G). In illustrative embodiments, the membrane-bound polypeptide capable of binding to CD3 is an anti-CD3 scFvFc antibody, which in some embodiments is fused to a heterologous GPI anchor attachment sequence. In some embodiments of this aspect, the contacting is performed for at least 2 hours, or between 2 hours and 24 hours, or between 2 hours and 6 hours. In some embodiments, a detectable marker is encoded by the genome of the replication incompetent recombinant retroviral particle, and detected in the T cells and/or NK cells after the transduction. In some embodiments, the membrane-bound polypeptide capable of binding to CD3 is not encoded by a polynucleotide in the replication incompetent recombinant retroviral particle. In some embodiments, a detectable marker is encoded by the genome of the replication incompetent recombinant retroviral particles, and detected in the T cells and/or NK cells after the transduction.

In another aspect, provided herein is a replication incompetent recombinant retroviral particle, comprising: one or more pseudotyping elements; a polynucleotide comprising one or more transcriptional units operatively linked to a promoter active in T cells and/or NK cells, wherein the one or more transcriptional units encode a chimeric antigen receptor; and a pseudotyping element on its surface and an activation element on its surface, wherein the activation element is capable of binding to a T cell and/or NK cell and is not encoded by a polynucleotide in the replication incompetent recombinant retroviral particle, and wherein the activation element is an anti-CD3 scFvFc antibody.

In another aspect, provided herein is a replication incompetent recombinant retroviral particle, comprising: one or more pseudotyping elements capable of binding to a T cell and/or an NK cell and facilitating membrane fusion of the replication incompetent recombinant retroviral particle thereto;

a polynucleotide comprising one or more transcriptional units operatively linked to a promoter active in T cells and/or NK cells, wherein the one or more transcriptional units encode a chimeric antigen receptor; and a pseudotyping element on its surface and an activation element on its surface, wherein the activation element is capable of binding to a T cell and/or NK cell and is not encoded by a polynucleotide in the replication incompetent recombinant retroviral particle, and wherein the activation elements is a membrane-bound polypeptide capable of binding to CD3 on their surface, but not a membrane-bound polypeptide capable of binding to and activating CD28 on their surface.

In some embodiments of the replication incompetent recombinant retroviral particle aspects herein, the recombinant retroviral particle further comprises a polynucleotide comprising one or more transcriptional units operatively linked to a promoter active in T cells and/or NK cells, wherein the one or more transcriptional units encode a chimeric antigen receptor. In some embodiments in these aspects, the membrane-bound polypeptide capable of binding to CD3 is not encoded by a polynucleotide in the replication incompetent recombinant retroviral particle. In some embodiments of these aspects, the anti-CD3 scFvFc antibody is not encoded by a polynucleotide in the replication incompetent recombinant retroviral particle.

In any of the aspects and embodiments herein, such as those including a polypeptide or a nucleic acid encoding the same, that include an activation element, for example present on the surface of replication incompetent recombinant retroviral particles, the activation element can further include, for example as a fusion polypeptide with a polypeptide capable of binding CD28 or in illustrative embodiments, CD3, a dimerizing motif that can be active in the absence of a dimerizing agent. In some embodiments, the activation element can be an anti-CD3 single chain antibody and the dimerizing motif can be selected from the group consisting of: a leucine zipper polypeptide, CD69, CD71, CD72, CD96, Cd105, Cd161, Cd162, Cd249, CD271, and Cd324, as well as mutants and/or active fragments thereof that retain the ability to dimerize.

In any of the aspects and embodiments herein, such as those including a polypeptide or a nucleic acid encoding the same, that include an activation element, for example present on the surface of replication incompetent recombinant retroviral particles, the activation element can further include, for example as a fusion polypeptide with a polypeptide capable of binding CD28 or in illustrative embodiments, CD3, a dimerizing motif that can be active in the presence of a dimerizing agent. In some embodiments, the activation element can be an anti-CD3 single chain antibody and the dimerizing motif can be selected from the group consisting of: FKBP and rapamycin or analogs thereof, GyrB and coumermycin or analogs thereof, DHFR and methotrexate or analogs thereof, or DmrB and AP20187 or analogs thereof, as well as mutants and/or active fragments of the recited dimerizing proteins that retain the ability to dimerize.

In any of the aspects and embodiments herein, such as those including a polypeptide or a nucleic acid encoding the same, that include an activation element, for example present on the surface of replication incompetent recombinant retroviral particles, the activation element can further include, an antiCD3-scFvFc-GPI moiety (UCHT1) and optionally a VSV-G.

In one embodiment of the above aspect said polynucleotide further comprises one or more of a Kozak-type sequence, a WPRE element, and a double stop codon or a triple stop codon, wherein one or more of the stop codons of the double stop codon or the triple stop codon define a termination of a reading from of at least one of the one or more transcriptional units. In certain embodiments, the polynucleotide comprises a Kozak-type sequence selected from CCACCAT/UG(G), CCGCCAT/UG(G), GCCGCCGCCAT/UG(G), or GCCGCCACCAT/U(G). In certain embodiments both the -3 and +4 nucleotides relative to a start codon of the first nucleic acid sequence comprise a G. In another embodiment, which can be combined with the preceding embodiment that comprises a Kozak-type sequence and/or the following embodiment that includes triple stop codon, the polynucleotide comprises a WPRE element. In some embodiments, the WPRE element is located 3' of a stop codon of the one or more transcriptional units and 5' to a 3' LTR of the polynucleotide. In another embodiment, which can be combined with either or both of the preceding embodiments (i.e. an embodiment wherein the polynucleotide comprises a Kozak-type sequence and/or an embodiment wherein the polynucleotide comprises a WPRE), the one or more transcriptional units terminates with one or more stop codons of a double stop codon or a triple stop codon.

In certain embodiments of any of the aspects provided herein that include an ASTR, the ASTR can be an anti-tag ASTR. Such methods can further include, for example, a tag-conjugated antibody that binds to a target molecule, such as a target protein on a target cell.

In any of the embodiments and aspects provided herein, that include a B cell, T cell, or NK cell, the cell can be an allogeneic cell, or it can be other than an allogeneic cell.

In any of the aspects and embodiments disclosed herein, the introduction of DNA into PBMCs, B cells, T cells and/or NK cells and optionally the incorporation of the DNA into the host cell genome, can be performed using methods that do not utilize replication incompetent recombinant retroviral particles. For example, other viral vectors can be utilized, such as those derived from adenovirus, adeno-associated virus, or herpes simplex virus-1, as non-limiting examples. In other embodiments, these aspects and embodiments can include transfecting and/or transducing target cells with non-viral vectors. In any of these embodiments disclosed herein that utilize non-viral vectors to transfect target cells, the non-viral vectors, including naked DNA, can be introduced into the target cells, such as for example, PBMCs, B cells, T cells and/or NK cells using methods that include electroporation, nucleofection, liposomal formulations, lipids, dendrimers, cationic polymers such as poly(ethylenimine) (PEI) and poly(l-lysine) (PLL), nanoparticles, cell-penetrating peptides, microinjection, and/or non-integrating lentiviral vectors. In some embodiments of methods and compositions provided herein, DNA can be integrated into the genome using transposon-based carrier systems by co-transfection, co-nucleofection or co-electroporation of target DNA as plasmid containing the transposon ITR fragments in 5' and 3' ends of the gene of interest and transposase carrier system. A transposase or other enzyme, for example CRISPR or TALEN, used in these methods can be co-transfected with target plasmid as DNA, mRNA or protein.

In another aspect, provided herein are replication incompetent recombinant retroviral particles, each comprising:
A. a pseudotyping element on its surface that is capable of binding to a T cell and/or NK cell and facilitating membrane fusion of the replication incompetent recombinant retroviral particle thereto, wherein said pseudotyping element comprises cytoplasmic domain deletion variants of a measles virus F polypeptide and/or a measles virus H polypeptide;
B. a polynucleotide comprising one or more transcriptional units operatively linked to a promoter active in T cells and/or NK cells, wherein the one or more transcriptional units encode a first engineered signaling polypeptide comprising a chimeric antigen receptor comprising an antigen-specific targeting region, a transmembrane domain, and an intracellular activating domain, and a second engineered signaling polypeptide comprising a constitutively active IL-7 receptor mutant; wherein expression of the IL-7 receptor mutant is regulated by a riboswitch that binds a nucleoside analog antiviral drug; and
C. a polypeptide capable of binding to CD3 and a polypeptide capable of binding to CD28, wherein said polypeptides are expressed on the surface of a replication incompetent recombinant retroviral particle; are capable of binding to a T cell and/or NK cell; and are not encoded by a polynucleotide in the replication incompetent recombinant retroviral particle. In illustrative embodiments of this aspect, binding of the nucleoside analog antiviral drug to the riboswitch increases expression of the IL-7 receptor mutant.

In one aspect, provided herein is a method for genetically modifying and expanding lymphocytes of a subject, comprising:
A. contacting resting T cells and/or NK cells of the subject *ex vivo* without requiring prior *ex vivo* stimulation, with replication incompetent recombinant retroviral particles comprising:
   i. a pseudotyping element on its surface that is capable of binding to a T cell and/or NK cell and facilitating membrane fusion of the replication incompetent recombinant retroviral particles thereto; and
   ii. a polynucleotide comprising one or more transcriptional units operatively linked to a promoter active in T cells and/or NK cells, wherein the one or more transcriptional units encode a first engineered signaling polypeptide regulated by a control element, wherein said first engineered signaling polypeptide comprises at least one lymphoproliferative element,
   wherein said contacting facilitates transduction of at least some of the resting T cells and/or NK cells by the replication incompetent recombinant retroviral particles, thereby producing genetically modified T cells and/or NK cells;
B. introducing the genetically modified T cells and/or NK cells into the subject; and
C. exposing the genetically modified T cells and/or NK cells *in vivo* to a compound that binds the control element to affect expression of the first engineered signaling polypeptide and promote and/or potentiate expansion, engraftment, and/or persistence of the lymphocytes *in vivo,* thereby genetically modifying and expanding lymphocytes of the subject. In illustrative embodiments, the transduction is carried out without *ex vivo* stimulation.

In the above aspect and any of the method aspects for genetically modifying and expanding lymphocytes or for performing cellular therapy herein, if not recited in or incompatible with the aspect, in certain embodiments the polynucleotide further comprises a transcriptional unit that encodes a second engineered signaling polypeptide comprising a first chimeric antigen receptor comprising an antigen-specific targeting region (ASTR), a transmembrane domain, and an intracellular activating domain.

In another aspect, provided herein is a method for performing adoptive cell therapy on a subject, comprising:
A. collecting blood from the subject;
B. contacting resting T cells and/or NK cells from the blood of the subject *ex vivo* with replication incompetent recombinant retroviral particles, wherein the replication incompetent recombinant retroviral particles comprise
   i. a pseudotyping element on their surface that is capable of binding to a T cell and/or NK cell and facilitating membrane fusion of the replication incompetent recombinant retroviral particles thereto; and
   ii. a polynucleotide comprising one or more transcriptional units operatively linked to a promoter active in T cells and/or NK cells, wherein the one or more transcriptional units encode a first engineered signaling polypeptide comprising at least one lymphoproliferative element whose expression is regulated by a control element, and a second engineered signaling polypeptide comprising a chimeric antigen receptor comprising an antigen-specific targeting region (ASTR), a transmembrane domain, and an intracellular activating domain,
   wherein said contacting results in at least some of the resting T cells and/or NK cells becoming genetically modified; and
C. reintroducing the genetically modified T cells and/or NK cells into the subject, wherein expansion, engraftment, and/or persistence of the genetically modified T cells and/or NK cells occurs *in vivo* within the subject, and wherein the method between the collecting blood and the reintroducing the genetically modified T cells and/or NK cells is performed in no more than 24 hours, thereby performing adoptive cell therapy on the subject.

Provided in another aspect herein is a method for performing adoptive cell therapy on a subject, comprising:
A. collecting blood from a subject;
B. isolating peripheral blood mononuclear cells (PBMCs) comprising resting T cells and/or resting NK cells;
C. contacting the resting T cells and/or resting NK cells of the subject *ex vivo,* with replication incompetent recombinant retroviral particles, wherein the replication incompetent recombinant retroviral particles comprise a pseudotyping element on their surface that is capable of binding a resting T cell and/or NK cell and facilitating membrane fusion of the replication incompetent recombinant retroviral particles thereto, wherein said contacting facilitates transduction of the resting T cells and/or NK cells by the replication incompetent recombinant retroviral particles, thereby producing genetically modified T cells and/or NK cells; and
D. reintroducing the genetically modified cells into the subject within 24 hours of collecting blood from the subject, thereby performing adoptive cell therapy in the subject.

In illustrative embodiments of any of the methods aspects for genetically modifying and expanding lymphocytes or for performing adoptive cellular therapy herein, or similar methods, if not explicitly recited in the broadest aspect the method can further comprise exposing the genetically modified T cells and/or NK cells *in vivo* to a compound that binds the control element to affect expression of the first engineered signaling polypeptide and optionally the second engineered signaling polypeptide, and to promote expansion, engraftment, and/or persistence of the lymphocytes in vivo.

In illustrative embodiments of any of the methods aspects for genetically modifying and expanding lymphocytes or for performing adoptive cellular therapy herein, or similar methods, if not explicitly recited in the broadest aspect, the genetically modified T cells and/or NK cells undergo 8, 7, 6, 5, 4, 3 or fewer cell divisions *ex vivo* prior to being introduced or reintroduced into the subject.

In illustrative embodiments of any of the methods aspects for genetically modifying and expanding lymphocytes or for performing cellular therapy herein, or similar methods, if not explicitly recited in the broadest aspect, expansion, engraftment, and/or persistence of genetically modified T cells and/or NK cells *in vivo* is dependent on either the presence or absence of the compound that binds the control element, and in illustrative embodiments, is dependent on the presence of the compound that binds the control element.

In illustrative embodiments of any of the methods aspects for genetically modifying and expanding lymphocytes or for performing adoptive cellular therapy herein, or similar methods, if not explicitly recited in the broadest aspect, the subject is not exposed to a lymphodepleting agent within 7, 14, or 21 days of performing the contacting, during the contacting, and/or within 7, 14, or 21 days after the modified T cells and/or NK cells are introduced into the subject. In other embodiments, the subject is not exposed to a lymphodepleting agent during the contacting.

In illustrative embodiments of any of the methods aspects for genetically modifying and expanding lymphocytes or for performing cellular therapy herein, or similar methods, if not explicitly recited in the broadest aspect, the resting T cells and/or resting NK cells are in contact with the replication incompetent recombinant retroviral particles for between 15 minutes and 12 hours.

In illustrative embodiments of any of the methods aspects for genetically modifying and expanding lymphocytes or for performing adoptive cellular therapy herein, or similar methods, if not explicitly recited in the broadest aspect, the method further includes the step of separating the replication incompetent recombinant retroviral particles from the T cells and/or NK cells after the contacting. In methods that include an introducing or reintroducing, but the separating is performed before the introducing. In illustrative embodiments of any of the methods aspects for genetically modifying and expanding lymphocytes or for performing cellular therapy herein, or similar methods, if not explicitly recited in the broadest aspect, said exposing step comprises administering a dose of the compound to the subject prior to or during the contacting, and/or after the genetically modified T cells and/or NK cells have been introduced into the subject.

In illustrative embodiments of any of the methods aspects for genetically modifying and expanding lymphocytes or for performing adoptive cellular therapy herein, or similar methods, if not explicitly recited in the broadest aspect, the method comprises collecting blood comprising the T cells and/or the NK cells from the subject prior to contacting the T cells and/or NK cells *ex vivo* with the replication incompetent recombinant retroviral particles, and wherein the introducing is reintroducing. For example, between 20 and 250 ml of blood are withdrawn from the subject.

In illustrative embodiments of any of the methods aspects for genetically modifying and expanding lymphocytes or for performing cellular therapy herein, or similar methods, if not explicitly recited in the broadest aspect, no more than 8, 12, 24, or 48 hours pass between the time blood is collected from the subject and the time the modified T cells and/or NK cells are reintroduced into the subject.

In illustrative embodiments of any of the methods aspects for genetically modifying and expanding lymphocytes or for performing cellular therapy herein, or similar methods, if not explicitly recited in the broadest aspect, between 4 or 8 hours on the low end and 12. 24, 36, or 48 hours on the high end of the range pass between the time blood is collected from the subject and the time the modified T cells and/or NK cells are reintroduced into the subject.

In aspects and embodiments provided herein that include administering genetically modified T cells and/or NK cells into a subject, especially where the subject has, or is suspected of having cancer, the methods can further include delivering an effective amount of an immune checkpoint inhibitor to the subject.

In illustrative embodiments of any of the methods aspects for genetically modifying and expanding lymphocytes or for performing adoptive cellular therapy herein, or similar methods, if not explicitly recited in the broadest aspect, all steps after the blood is collected and before the blood is reintroduced, are performed in a closed system in which a person monitors the closed system throughout the processing. In another embodiment, after the blood is collected and before the blood is reintroduced, are performed in a closed system that remains in the same room with the subject.

In illustrative embodiments of any of the methods and compositions provided herein that include one or more transcriptional units or one or more engineered signaling polypeptides, if not recited in the broadest aspect, one of the transcriptional units or one of the engineered signaling polypeptides can encode or comprise or further comprise an antigen-specific targeting region (ASTR) and a transmembrane domain connecting the ASTR to the lymphoproliferative element. The ASTR of this engineered signaling polypeptide is capable of binding to a first tumor antigen and where present, the ASTR of the second engineered signaling polypeptide is capable of binding to a second tumor antigen. In illustrative embodiments, the first engineered signaling polypeptide and/or the second engineered signaling polypeptide further comprise a co-stimulatory domain. Furthermore, the first engineered signaling polypeptide and/or the second engineered signaling polypeptide further comprise a stalk. Furthermore, the first engineered signaling polypeptide further comprises an intracellular activating domain. The intracellular activating domain on the first engineered signaling polypeptide and/or the second engineered signaling polypeptide can be derived from CD3 zeta. In illustrative embodiments of any of the methods and compositions provided herein that include a lymphoproliferative element, the lymphoproliferative element can comprise MPL or can be MPL, or a variant and/or fragment thereof, including a variant and/or fragment that includes at least 75, 80, 85, 90, 95, 96, 97, 98, 99, or 100% of the intracellular domain of MPL, with or without a transmembrane and/or extracellular domain of MPL, and/or has at least 75, 80, 85, 90, 95, 96, 97, 98, 99, or 100% sequence identity to the intracellular domain of MPL, with or without a transmembrane and/or extracellular domain of MPL, wherein the variant and/or fragment retains the ability to promote cell proliferation of PBMCs, and in some embodiments T cells.

In any of the methods or compositions provided herein that include a lymphoproliferative element, the lymphoproliferative element can include an inhibitory RNA molecule, such as, *e.g.,* a miRNA or shRNA, that stimulates the STAT5 pathway or inhibits the SOCS pathway. For example, an inhibitory RNA molecule can bind to a nucleic acid encoding a protein selected from the group consisting of: ABCG1, SOCS, TGFbR2, SMAD2, cCBL, and PD1. In illustrative embodiments for any of the replication incompetent recombinant retroviral particles or transduced cells provided herein, or methods including the same, such replication incompetent recombinant retroviral particles or transduced cells can encode two or more inhibitory RNA molecules, such as, *e.g.,* a miRNA or shRNA, within an intron, in some embodiments, 1, 2, 3, or 4 inhibitory RNA molecules that bind nucleic acids encoding one or more of the following target endogenous T cell expressed genes: PD-1; CTLA4; TCR alpha; TCR beta; CD3 zeta; SOCS; SMAD2; miR-155; IFN gamma; cCBL; TRAIL2; PP2A; or ABCG1. For example, in one embodiment, a combination of miRNAs targeting any of the following can be included in a genome of a replication incompetent recombinant retroviral particle or transduced cell: TCR alpha, CD3 zeta, IFN gamma, and PD-1; and in another embodiment SOCS 1, IFN gamma, TCR alpha, and CD3 zeta.

In illustrative embodiments of any of the methods and compositions provided herein, the replication incompetent recombinant retroviral particles, mammalian cells, and/or packaging cells, can comprise a Vpu polypeptide. The Vpu polypeptide can be, for example, a fusion polypeptide, and in some examples, especially in packaging cells, a membrane bound Vpu polypeptide. In illustrative embodiments of any of the methods and compositions provided herein, the replication incompetent recombinant retroviral particles, mammalian cells, and/or packaging cells, can comprise both a Vpu polypeptide and a Vpx polypeptide.

In illustrative embodiments of any of the methods and compositions provided herein, the replication incompetent recombinant retroviral particles, mammalian cells, and/or packaging cells, can comprise a Vpx polypeptide. The Vpx polypeptide can be, for example, a fusion polypeptide, and in some examples, especially in packaging cells, a membrane bound Vpx polypeptide.

In any of the methods or compositions provided herein, the one or more pseudotyping elements can include a vesicular stomatitis virus envelope protein (VSV-G), a feline endogenous virus (RD114) envelope protein, an oncoretroviral amphotropic envelope protein, or an oncoretroviral ecotropic envelope protein, or functional fragments thereof.

In one aspect, provided herein is a genetically modified T cell or NK cell, wherein the T cell or NK cell has been genetically modified to express a first engineered signaling polypeptide comprising a lymphoproliferative element and a second engineered signaling polypeptide comprising a CAR that includes an antigen-specific targeting region (ASTR), a transmembrane domain, and an intracellular activating domain.

In another aspect, provided herein is a genetically modified T cell or NK cell comprising a pseudotyping element on a surface of the T cell or NK cell and an activation element on the surface of the T cell or NK cell, wherein the T cell or NK cell has been genetically modified to express a first engineered signaling polypeptide comprising a lymphoproliferative element and a second engineered signaling polypeptide comprising a chimeric antigen receptor that includes an antigen-specific targeting region (ASTR), a transmembrane domain, and an intracellular activating domain.

Provided herein in another aspect is a genetically modified T cell and/or NK cell comprising:
A. a first engineered signaling polypeptide comprising at least one lymphoproliferative element; and
B. a second engineered signaling polypeptide comprising a chimeric antigen receptor comprising an antigen-specific targeting region (ASTR), a transmembrane domain, and an intracellular activating domain.

In some embodiments, the genetically modified T cells and/or NK cells are capable of survival in *ex vivo* culture in the absence of IL-2. In some embodiments, the genetically modified T cells and/or NK cells comprise a chimeric cytokine receptor. In some embodiments, the genetically modified T cells and/or NK cells do not comprise a chimeric cytokine receptor.

In any of the methods provided herein that include a mammalian packaging cell, including a replication incompetent recombinant retroviral particle packaging system aspect, or a method for making a replication incompetent recombinant retroviral particle, for example, the packageable RNA genome is encoded by a polynucleotide operably linked to a promoter, wherein said promoter is either constitutively active or inducible by either the first transactivator or the second transactivator. The packageable RNA genome can be encoded by a polynucleotide operably linked to a promoter, wherein said promoter is inducible by the second transactivator. A promoter used herein to drive expression of the first and/or second engineered signaling polypeptide, is typically active in target cells, for example lymphocytes, PBLs, T-cells and/or NK cells, but in illustrative embodiments, is not active in the packaging cell line. The second transactivator can regulate the expression of an activation element capable of binding to and activating the target cell. In any of the methods provided herein that include a mammalian packaging cell, including a replication incompetent recombinant retroviral particle packaging system aspect, or a method for making a replication incompetent recombinant retroviral particle, for example, the packageable RNA genome in some embodiments, expression of the packageable RNA genome can be regulated by the second transactivator.

Furthermore, the packageable RNA genome can comprise, from 5' to 3':
1.) a 5' long terminal repeat, or active fragment thereof;
2.) a nucleic acid sequence encoding a retroviral cis-acting RNA packaging element;
3.) a nucleic acid sequence encoding a first target polypeptide and/or a nucleic acid sequence encoding one or more (e.g. two or more) inhibitory RNA molecules;
4.) a promoter that is active in the target cell; and
5.) a 3' long terminal repeat, or active fragment thereof.

In some embodiments, the nucleic acid sequence encoding the first target polypeptide is in reverse orientation to an RNA encoding retroviral components for packaging and assembly and the 5' LTR.

In any of the methods provided herein that include a mammalian packaging cell, including a replication incompetent recombinant retroviral particle packaging system aspect, or a method for making a replication incompetent recombinant retroviral particle, for example, the first target polypeptide comprises a first engineered signaling polypeptide and wherein said first engineered signaling polypeptide comprises at least one lymphoproliferative element. The packageable RNA genome can further comprises a nucleic acid sequence encoding a second target polypeptide. The second target polypeptide can comprise a second engineered signaling polypeptide including a chimeric antigen receptor comprising:
1.) a first antigen-specific targeting region;
2.) a first transmembrane domain; and
3.) a first intracellular activating domain.

In any of the methods provided herein that include a mammalian packaging cell, including a replication incompetent recombinant retroviral particle packaging system aspect, or a method for making a replication incompetent recombinant retroviral particle, for example, the mammalian cell, for example the packaging cell can include a nucleic acid sequence encoding Vpu polypeptide, for example on the second or an optional third transcriptional unit, or on an additional transcriptional unit that is operably linked to the first inducible promoter. In any of the methods provided herein that include a mammalian packaging cell, including a replication incompetent recombinant retroviral particle packaging system aspect, or a method for making a replication incompetent recombinant retroviral particle, for example, the mammalian cell, for example the packaging cell can include a nucleic acid sequence encoding both a Vpx polypeptide and a Vpu polypeptide, for example on the second or an optional third transcriptional unit, or on an additional transcriptional unit that is operably linked to the first inducible promoter. The mammalian cell, which can be a packaging cell, can be a 293 cell.

In any of the methods provided herein that include a mammalian packaging cell, including a replication incompetent recombinant retroviral particle packaging system aspect, or a method for making a replication incompetent recombinant retroviral particle, for example, the mammalian cell, for example the packaging cell can include a nucleic acid sequence encoding Vpx, for example on the second or an optional third transcriptional unit, or on an additional transcriptional unit that is operably linked to the first inducible promoter. The mammalian cell, which can be a packaging cell, can be a 293 cell.

In any of the methods provided herein that include a mammalian packaging cell, including a replication incompetent recombinant retroviral particle packaging system aspect, or a method for making a replication incompetent recombinant retroviral particle, a first ligand can be rapamycin and a second ligand can be tetracycline or doxorubicin or the first ligand can be tetracycline or doxorubicin and the second ligand can be rapamycin.

In some aspects, provided herein is a cell that has been transduced with any of the replication incompetent recombinant retroviral particles provided herein. The cell can be, for example, a lymphocyte, such as a T cell or NK cell. The cell in illustrative embodiments, is a human cell.

In one aspect provided herein, is a method of expanding modified T cells and/or NK cells in a subject, said method comprising:
A. contacting isolated resting T cells and/or resting NK cells obtained from said subject with the replication incompetent recombinant retroviral particle of any of the embodiments disclosed herein;
B. introducing the genetically modified T cells and/or NK cells into the subject; and
C. providing an effective amount of acyclovir, an acyclovir prodrug, penciclovir, or a penciclovir prodrug to said subject, wherein said modified T cells and/or NK cells proliferate in said subject upon administration of acyclovir, an acyclovir prodrug, penciclovir, or a penciclovir prodrug, thereby expanding the modified T cells and/or NK cells in the subject.

In another aspect, provided herein is a method of stopping the expansion, engraftment, and/or persistence of modified T cells and/or NK cells in a subject, said method comprising:
A. contacting isolated quiescent T cell and/or NK cells obtained from said subject with the replication incompetent recombinant retroviral particles of any of the embodiments disclosed herein;
B. introducing the modified T cell and/or NK cells into the subject;
C. administering an effective amount of acyclovir, an acyclovir prodrug, penciclovir, or a penciclovir prodrug to said subject to expand the modified T cell and/or NK cells in the subject, wherein said modified T cell and/or NK cells proliferate in said subject upon administration of acyclovir, an acyclovir prodrug, penciclovir, or a penciclovir prodrug, thereby expanding the modified PBLs in the subject; and
D. stopping administration of acyclovir, an acyclovir prodrug, penciclovir, or a penciclovir prodrug, wherein said modified T cell and/or NK cells stop proliferating in said subject upon stopping administration of acyclovir, an acyclovir prodrug, penciclovir, or a penciclovir prodrug, thereby controlling the expansion, engraftment, and/or persistence of the modified T cell and/or NK cells in the subject.

In another aspect, provided herein is a method of treating cancer in a subject, said method comprising:
A. contacting isolated quiescent T cells and/or NK cells obtained from said subject with the replication incompetent recombinant retroviral particles according to any of the embodiments disclosed herein;
B. introducing the genetically modified T cells and/or NK cells into the subject; and
C. administering an effective amount of acyclovir, an acyclovir prodrug, penciclovir, or a penciclovir prodrug to said subject to expand the modified T cell and/or NK cells in the subject, wherein said modified T cell and/or NK cells proliferate in said subject upon administration of acyclovir, an acyclovir prodrug, penciclovir, or a penciclovir prodrug, and wherein the chimeric antigen receptor in said modified T cell and/or NK cells binds cancer cells in said subject, thereby treating cancer in the subject.

In another aspect, provided herein is a transduced T cell and/or NK cell, comprising a recombinant polynucleotide comprising one or more transcriptional units operatively linked to a promoter active in T cells and/or NK cells, wherein the one or more transcriptional units encode a first engineered signaling polypeptide regulated by a control element, wherein said first engineered signaling polypeptide comprises a constitutively active IL-7 receptor mutant, and wherein the control element is capable of binding, and/or designed and/or configured to bind, to a compound *in vivo.*

In another aspect, provided herein is a retroviral packaging system, comprising:
a mammalian cell comprising:
   A. a first transactivator expressed from a constitutive promoter and capable of binding a first ligand and a first inducible promoter for affecting expression of a nucleic acid sequence operably linked thereto in the presence versus absence of the first ligand;
   B. a second transactivator capable of binding a second ligand and a second inducible promoter, and affecting expression of a nucleic acid sequence operably linked thereto in the presence versus absence of the second ligand; and
   C. a packageable RNA genome for a retroviral particle,
wherein the first transactivator regulates expression of the second transactivator and a retroviral REV protein, wherein the second transactivator regulates expression of a gag polypeptide, a pol polypeptide, and one or more pseudotyping elements capable of binding to a target cell and facilitating membrane fusion thereto, and wherein the retroviral proteins are derived from a retrovirus. Embodiments of this aspect, can include any of the embodiments provided herein for the recited elements in other aspects.

In another aspect, provided herein is a method for making a replication incompetent recombinant retroviral particle, comprising:
A. culturing a population of packaging cells to accumulate a first transactivator, wherein the packaging cells comprise the first transactivator expressed from a first constitutive promoter, wherein the first transactivator is capable of binding a first ligand and a first inducible promoter for affecting expression of a nucleic acid sequence operably linked thereto in the presence versus absence of the first ligand, and wherein expression of a second transactivator and a retroviral REV protein is regulated by the first transactivator;
B. incubating the population of packaging cells comprising accumulated first transactivator in the presence of the first ligand to accumulate the second transactivator and the retroviral REV protein, wherein the second transactivator is capable of binding a second ligand and a second inducible promoter for affecting expression of a nucleic acid sequence operably linked thereto in the presence versus absence of the second ligand; and
C. incubating the population of packaging cells comprising accumulated second transactivator and retroviral REV protein in the presence of the second ligand thereby inducing expression of a gag polypeptide, a pol polypeptide, and one or more pseudotyping elements, thereby making the replication incompetent recombinant retroviral particle, wherein a packageable RNA genome is encoded by a polynucleotide operably linked to a third promoter, wherein said third promoter is either constitutively active or inducible by either the first transactivator or the second transactivator, and wherein the one or more pseudotyping elements are capable of binding to a target cell and/or facilitating membrane fusion of the replication incompetent recombinant retroviral particle thereto.

In some embodiments of the retroviral packaging system and method for making a replication incompetent recombinant retroviral particles provided herein, the mammalian cell further comprises an activation element capable of binding to and activating a target cell, typically a lymphocyte, such as an NK cell, or in illustrative embodiments a T cell, and the first transactivator regulates the expression of the activation element. The activation element is on the surface of the replication incompetent recombinant retroviral particle and wherein the activation element can include: a membrane-bound polypeptide capable of binding to CD3; and/or a membrane-bound polypeptide capable of binding to CD28. The membrane-bound polypeptide capable of binding to CD3 is a polypeptide capable of binding to CD3 that is fused to a heterologous GPI anchor attachment sequence and the membrane-bound polypeptide capable of binding to CD28 is a polypeptide capable of binding to CD28 that is fused to a heterologous GPI anchor attachment sequence. The membrane-bound polypeptide capable of binding to CD28 in some embodiments comprises CD80, CD86, or a functional fragment thereof that is capable of inducing CD28-mediated activation of Akt, such as the extracullular domain of CD80. In other embodiments, membrane-bound polypeptide capable of binding CD3 is an anti-CD3 scFv or an anti-CD3 scFvFc bound to a CD14 GPI anchor attachment sequence, and wherein the membrane-bound polypeptide capable of binding to CD28 is CD80, or an extracellular fragment thereof, bound to a CD16B GPI anchor attachment sequence.

In some embodiments of the retroviral packaging system and method for making a replication incompetent recombinant retroviral particle aspects provided herein, the mammalian cell further comprises a membrane-bound cytokine, and the first transactivator regulates the expression of the membrane-bound cytokine. The membrane-bound cytokine can be, for example, IL-7, IL-15, or an active fragment thereof. The membrane-bound cytokine in embodiments can be a fusion polypeptide of IL-7, or an active fragment thereof, and DAF. For example, the fusion polypeptide can comprise the DAF signal sequence and IL-7 without its signal sequence, followed by residues 36-525 of DAF.

In some embodiments of the retroviral packaging system and method for making a replication incompetent recombinant retroviral particle aspects provided herein, the mammalian cell comprises associated with its membrane, an activation element comprising an anti-CD3 scFV or an anti-CD3 scFvFc bound to a CD14 GPI anchor attachment sequence and a CD80 bound, or an extracellular fragment thereof to a CD16B GPI anchor attachment sequence; and membrane-bound cytokine comprising a fusion polypeptide of IL-7, or an active fragment thereof, and DAF comprising a GPI anchor attachment sequence, and wherein the first transactivator regulates the expression of each of the activation element and membrane-bound cytokine. In some embodiments, the IL-7, or an active fragment thereof, and DAF fusion, the anti-CD3 scFV or an anti-CD3 scFvFc, and the CD80, or extracellular fragment thereof, each comprises a DAF signal sequence.

In some embodiments of the retroviral packaging system and method for making a replication incompetent recombinant retroviral particle aspects provided herein, the mammalian cell further comprises a Vpu polypeptide. In some embodiments of the retroviral packaging system and method for making a replication incompetent recombinant retroviral particle aspects provided herein, the mammalian cell further comprises a Vpu polypeptide and a Vpx polypeptide. In some embodiments of the retroviral packaging system and method for making a replication incompetent recombinant retroviral particle aspects provided herein, the mammalian cell further comprises a Vpx polypeptide. In these or other embodiments, the one or more pseudotyping elements comprise one or more viral polypeptides recognized by T cells. The one or more pseudotyping elements can comprise a Measles Virus F polypeptide, a Measles Virus H polypeptide, and/or a fragment thereof. In certain illustrative embodiments, the one or more pseudotyping elements are cytoplasmic domain deletion variants of a measles virus F polypeptide and/or a measles virus H polypeptide.

In some embodiments of the retroviral packaging system and method for making a replication incompetent recombinant retroviral particle aspects provided herein, the packageable RNA genome is encoded by a polynucleotide operably linked to a third promoter, wherein said third promoter is either constitutively active or inducible by either the first transactivator or the second transactivator. In illustrative embodiments, the packageable RNA genome is encoded by a polynucleotide operably linked to a third promoter, wherein said third promoter is inducible by the second transactivator.

In some embodiments of the retroviral packaging system and method for making a replication incompetent recombinant retroviral particle aspects provided herein, the packageable RNA genome further comprises, from 5' to 3':
a) a 5' long terminal repeat, or active fragment thereof;
b) a nucleic acid sequence encoding a retroviral cis-acting RNA packaging element;
c) a nucleic acid sequence encoding a first target polypeptide and an optional second target polypeptide;
d) a fourth promoter operably linked to the first target polypeptide and the optional second polypeptide, wherein said fourth promoter is active in the target cell but not active in the packaging cell line; and
e) a 3' long terminal repeat, or active fragment thereof.

In some embodiments of the retroviral packaging system and method for making a replication incompetent recombinant retroviral particle aspects provided herein including the construct immediately above, the third promoter promotes transcription or expression in the opposite direction from transcription or expression promoted from the fourth promoter.

In some embodiments of the retroviral packaging system and method for making a replication incompetent recombinant retroviral particle aspects provided herein, the packageable RNA genome encodes the replication incompetent recombinant retroviral particle of any embodiment disclosed in this disclosure, wherein the first target polypeptide and the second target polypeptide are the first engineered signaling polypeptide and the second engineered signaling polypeptide, respectively. In some embodiments, for example, the packageable RNA genome further comprises a control element operably linked to the nucleic acid encoding the first engineered signaling polypeptide or the second engineered signaling polypeptide. The control element in illustrative embodiments is a riboswitch. The riboswitch in illustrative embodiments is capable of binding a compound and the compound that binds the control element is a nucleoside analog, and the nucleoside analog can be an antiviral drug, for example acyclovir or penciclivir.

In some embodiments of the retroviral packaging system and method for making a replication incompetent recombinant retroviral particle aspects provided herein, the packageable RNA genome further comprises an intron comprising a polynucleotide encoding an inhibitory RNA molecules, such as, *e.g.,* a miRNA or shRNA. The intron can be adjacent to and downstream of the fourth promoter.

In some embodiments of the retroviral packaging system and method for making a replication incompetent recombinant retroviral particle aspects provided herein, the target cell can be a T cell and/or an NK cell.

In some embodiments of the retroviral packaging system and method for making a replication incompetent recombinant retroviral particle aspects provided herein, the one or more pseudotyping elements comprise a vesicular stomatitis virus envelope protein (VSV-G), a feline endogenous virus (RD114) envelope protein, an oncoretroviral amphotropic envelope protein, or an oncoretroviral ecotropic envelope protein, or functional fragments thereof.

In some embodiments of the retroviral packaging system and method for making a replication incompetent recombinant retroviral particle aspects provided herein, the packageable RNA genome is 11,000 KB or less or 10,000 KB or less in size. In some embodiments of the retroviral packaging system and method for making a replication incompetent recombinant retroviral particle aspects provided herein, the first target polypeptide comprises a first engineered signaling polypeptide and wherein said first engineered signaling polypeptide comprises at least one lymphoproliferative element, and the second target polypeptide comprises a second engineered signaling polypeptide including a CAR.

In one aspect, provided herein is an isolated polynucleotide for regulating expression of a target polynucleotide, comprising:
a polynucleotide encoding a target polynucleotide operably linked to a promoter and a riboswitch, wherein the riboswitch comprises:
a.) an aptamer domain capable of binding a nucleoside analogue antiviral drug and having reduced binding to guanine or 2'-deoxyguanosine relative to the nucleoside analogue antiviral drug; and
b.) a function switching domain capable of regulating expression of the target polynucleotide, wherein binding of the nucleoside analogue by the aptamer domain induces or suppresses the expression regulating activity of the function switching domain, thereby regulating expression of the target polynucleotide.

In illustrative embodiments of any of the methods and compositions provided herein that include the control element can be a polynucleotide comprising a riboswitch. The riboswitch can be capable of binding a nucleoside analog and the compound that binds the control element is the nucleoside analog. The nucleoside analog can be an antiviral agent. The antiviral agent can be acyclovir or penciclovir. The riboswitch can preferentially bind acyclovir over penciclovir or preferentially bind penciclovir over acyclovir. The riboswitch can have reduced binding to the nucleoside analogue antiviral drug at temperatures above 37 °C, 37.5 °C, 38 °C, 38.5 °C, or 39 °C, for example, above 39 °C. The riboswitch can be between 35, 40, 45, and 50 nucleotides in length on the low end of the range and 60, 65, 70, 75, 80, 85, 90, 95, and 100 nucleotides in length on the high end of the range, for example, between 45 and 80 nucleotides in length. In illustrative embodiments of any of the methods and compositions provided herein that include the riboswitch, the target polynucleotide that is regulated by the riboswitch can include a region encoding a miRNA, an shRNA, and/or a polypeptide. The target polynucleotide can encode a lymphoproliferative element. The target polynucleotide can be operably linked to a promoter. The target polynucleotide can include a region encoding a polypeptide and the polypeptide can include a chimeric antigen receptor comprising an antigen-specific targeting region, a transmembrane domain, and an intracellular activating domain. In illustrative embodiments of any of the methods and compositions provided herein that include the riboswitch, the function switching domain can regulate an internal ribosome entry site, pre-mRNA splice donor accessibility in the viral gene construct, translation, termination of transcription, transcript degradation, miRNA expression, or shRNA expression, thereby regulating expression of the target polynucleotide. The riboswitch can include a ribozyme. In illustrative embodiments of any of the methods and compositions provided herein that include the riboswitch, the isolated polynucleotide can be a molecular cloning vector or an expression vector. In illustrative embodiments of any of the methods and compositions provided herein that include the riboswitch, the isolated polynucleotide can be integrated into a retroviral genome or into a mammalian chromosome, or fragment thereof. In certain embodiments of this and any of the aspects and embodiments herein that comprise a riboswitch, the riboswitch regulates pre-mRNA splicing. For example, the riboswitch can comprise a branchpoint sequence, typically between two exons on a polynucleotide and/or transcriptional unit herein. As such, binding of a nucleoside analogue antiviral compound or drug to the riboswitch regulates exon splicing. Such embodiments can include a polynucleotide comprising a first exon, a second exon, and a riboswitch between the first exon and the second exon, wherein the riboswitch comprises a branchpoint sequence, and wherein binding of a nucleoside analogue antiviral compound or drug (e.g. acyclovir) to the riboswitch regulates exon splicing of the first and second exon. In other embodiments of this and any of the aspects and embodiments herein that comprise a riboswitch, the riboswitch can control gene expression by regulating pre-mRNA transplicing. In such embodiments, the riboswitch is located within a transplicing intron. For example, one aspect can include a polynucleotide that comprises a first exon, a second exon, and a riboswitch between the first exon and the second exon, wherein the riboswitch comprises a branchpoint sequence, and wherein binding of acyclovir to the riboswitch regulates exon splicing of the first and second exon.

Another aspect provided herein, is a method for genetically modifying and expanding lymphocytes of a subject, comprising:
A. collecting blood from the subject;
B. contacting T cells and/or NK cells from the blood of the subject ex vivo with replication incompetent recombinant retroviral particles comprising:
   i. a pseudotyping element on its surface that is capable of binding to a T cell and/or NK cell and facilitating membrane fusion of the replication incompetent recombinant retroviral particle thereto, wherein said pseudotyping element comprises cytoplasmic domain deletion variants of a measles virus F polypeptide and/or a measles virus H polypeptide;
   ii. a polypeptide capable of binding to CD3 and a polypeptide capable of binding to CD28, wherein said polypeptides are expressed on the surface of a replication incompetent recombinant retroviral particle and are capable of binding to a T cell and/or a NK cell and further wherein said polypeptides are not encoded by a polynucleotide in the replication incompetent recombinant retroviral particle; and
   iii. a polynucleotide comprising one or more transcriptional units operatively linked to a promoter active in T cells and/or NK cells,

   wherein the one or more transcriptional units encode a first engineered signaling polypeptide comprising a constitutively active IL-7 receptor mutant and a second engineered signaling polypeptide comprising a chimeric antigen receptor comprising an antigen-specific targeting region (ASTR), a transmembrane domain, and an intracellular activating domain,
   wherein expression of the IL-7 receptor mutant is regulated by a riboswitch that binds a nucleoside analog antiviral drug, wherein binding of the nucleoside analog antiviral drug to the riboswitch increases expression of the IL-7 receptor mutant, and
   wherein said contacting results in at least some of the resting T cells and/or NK cells becoming genetically modified;
C. reintroducing the genetically modified T cells and/or NK cells into the subject; and
D. exposing the genetically modified T cells and/or NK cells *in vivo* to the nucleoside analog antiviral drug to promote expansion of the T cells and/or NK cells, wherein the method between the collecting blood and the reintroducing the genetically modified T cells and/or NK cells is performed in no more than 24 hours and/or without requiring prior ex vivo stimulation, thereby genetically modifying and expanding lymphocytes of the subject.

In illustrative embodiments of this method aspect, the retroviral particle is a lentiviral particle. In another illustrative embodiment, the replication incompetent recombinant retroviral particle genetically modifies a T cell. In another illustrative embodiment, the polypeptide capable of binding to CD3 and the polypeptide capable of binding to CD28 are each fused to a heterologous GPI anchor attachment sequence. In some instances, the polypeptide capable of binding to CD3 can be anti-CD3 scFvFc or anti-CD3 scFv, and the polypeptide capable of binding to CD28 can be CD80. The anti-CD3 scFvFc or anti-CD3 scFv and CD80 can each be further fused to a DAF signal sequence. In another illustrative embodiment, the replication incompetent recombinant retroviral particles further comprise on their surface a fusion polypeptide comprising a cytokine covalently attached to DAF. In some instances, the cytokine can be IL-7 or IL-15, and the fusion polypeptide can comprise the DAF signal sequence, IL-7 without its signal sequence, and a fragment of DAF comprising a GPI anchor attachment sequence.

In another illustrative embodiment of this method aspect immediately above, the riboswitch further controls expression of the chimeric antigen receptor in a manner regulated by binding of the riboswitch to the nucleoside analog antiviral drug, which in some instances is acyclovir and/or penciclovir. In another embodiment, the constitutively active IL-7 can be replaced with a miRNA or shRNA or nucleic acids encoding an miRNA or shRNA and IL-7 can be present. In some instances, the miRNA or shRNA can be encoded by nucleic acids within an intron.

Another aspect provided herein is a replication incompetent recombinant retroviral particle, comprising:
A. a pseudotyping element on its surface that is capable of binding to a T cell and/or NK cell and facilitating membrane fusion of the replication incompetent recombinant retroviral particle thereto, wherein said pseudotyping element comprises cytoplasmic domain deletion variants of a measles virus F polypeptide and/or a measles virus H polypeptide;
B. a polynucleotide comprising one or more transcriptional units operatively linked to a promoter active in T cells and/or NK cells, wherein the one or more transcriptional units encode a first engineered signaling polypeptide comprising a chimeric antigen receptor comprising an antigen-specific targeting region, a transmembrane domain, and an intracellular activating domain, and a second engineered signaling polypeptide comprising a constitutively active IL-7 receptor mutant; wherein expression of the IL-7 receptor mutant is regulated by a riboswitch that binds a nucleoside analog antiviral drug, wherein binding of the nucleoside analog antiviral drug to the riboswitch increases expression of the IL-7 receptor mutant; and
C. a polypeptide capable of binding to CD3 and a polypeptide capable of binding to CD28,
wherein said polypeptides are expressed on the surface of a replication incompetent recombinant retroviral particle; are capable of binding to a T cell and/or NK cell; and are not encoded by a polynucleotide in the replication incompetent recombinant retroviral particle.

In illustrative embodiments of any replication incompetent recombinant retroviral particle aspect, the retroviral particle is a lentiviral particle. In other illustrative embodiments of the method, the polypeptide capable of binding to CD3 and the polypeptide capable of binding to CD28 are each fused to a heterologous GPI anchor attachment sequence. In some instances, the polypeptide capable of binding to CD3 can be anti-CD3 scFvFc or anti-CD3 scFv, and the polypeptide capable of binding to CD28 can be CD80. The anti-CD3 scFvFc or anti-CD3 scFv and CD80 can each be further fused to a DAF signal sequence. In another illustrative embodiment, the replication incompetent recombinant retroviral particles further comprise on their surface a fusion polypeptide comprising a cytokine covalently attached to DAF. In some instances, the cytokine can be IL-7 or IL-15, and the fusion polypeptide can comprise the DAF signal sequence, IL-7 without its signal sequence, and a fragment of DAF comprising a GPI anchor attachment sequence.

In another illustrative embodiment of any replication incompetent recombinant retroviral particle aspect herein, the riboswitch further controls expression of the chimeric antigen receptor in a manner regulated by binding of the riboswitch to the nucleoside analog antiviral drug, which in some instances is acyclovir and/or penciclovir. In another embodiment, the constitutively active IL-7 can be replaced with a miRNA or shRNA or nucleic acids encoding an miRNA or shRNA and IL-7 can be present. The miRNA or shRNA can be encoded by nucleic acids within an intron.

Another aspect provided herein is a method for making a replication incompetent recombinant retroviral particle, comprising:
A. culturing a population of packaging cells to accumulate a first transactivator, wherein the packaging cells comprise the first transactivator expressed from a constitutive promoter, wherein the first transactivator is capable of binding a first ligand and a first inducible promoter for affecting expression of a nucleic acid sequence operably linked thereto in the presence versus absence of the first ligand, and wherein expression of a second transactivator and a retroviral REV protein is regulated by the first transactivator;
B. incubating the population of packaging cells comprising accumulated first transactivator in the presence of the first ligand to accumulate the second transactivator and the retroviral REV protein and an activation element typically on their surface, comprising a polypeptide capable of binding to CD3 and a polypeptide capable of binding to CD28, wherein the second transactivator is capable of binding a second ligand and a second inducible promoter for affecting expression of a nucleic acid sequence operably linked thereto in the presence versus absence of the second ligand; and
C. incubating the population of packaging cells comprising accumulated second transactivator and retroviral REV protein in the presence of the second ligand thereby inducing expression of a gag polypeptide, a pol polypeptide and a pseudotyping element capable of binding to a T cell and/or an NK cell and facilitating membrane fusion of the replication incompetent recombinant retroviral particle thereto, wherein said pseudotyping element comprises cytoplasmic domain deletion variants of a measles virus F polypeptide and/or a measles virus H polypeptide,

wherein a packageable RNA genome is encoded by a polynucleotide operably linked to a third promoter and wherein said promoter is inducible by the second transactivator,
wherein the packageable RNA genome comprises, from 5' to 3':
   i. a 5' long terminal repeat, or active fragment thereof;
   ii. a nucleic acid sequence encoding a retroviral cis-acting RNA packaging element;
   iii. a nucleic acid sequence encoding a first engineered signaling polypeptide comprising a chimeric antigen receptor and a second engineered signaling polypeptide comprising a constitutively active IL-7 receptor mutant separated by a cleavage signal;
   iv. a fourth promoter that is active in the T cell and/or the NK cell; and
   v. a 3' long terminal repeat, or active fragment thereof, and
wherein the packageable RNA genome further comprises a riboswitch that binds a nucleoside analog antiviral drug, wherein binding of the riboswitch to the nucleoside analog antiviral drug increases expression of the IL-7 receptor mutant,
thereby making the replication incompetent recombinant retroviral particle.

In an illustrative embodiment of the method, the riboswitch further controls expression of the chimeric antigen receptor in a manner regulated by binding of the riboswitch to the nucleoside analog antiviral drug. In another illustrative embodiment, the nucleoside analog antiviral drug is acyclovir and/or penciclovir. In another illustrative embodiment, the packageable RNA genome further comprises a recognition domain, wherein the recognition domain comprises a polypeptide that is recognized by an antibody that recognizes EGFR or an epitope thereof. In another illustrative embodiment, the first ligand is rapamycin and the second ligand is tetracycline or doxorubicin or the first ligand is tetracycline or doxorubicin and the second ligand is rapamycin. In another illustrative embodiment, the packaging cell further comprises a nucleic acid sequence encoding Vpu on the second or an optional third transcriptional unit, or on an additional transcriptional unit that is operably linked to the first inducible promoter. In another illustrative embodiment, the packaging cell further comprises a nucleic acid sequence encoding a Vpu polypeptide and optionally a Vpx polypeptide on the second or an optional third transcriptional unit, or on an additional transcriptional unit that is operably linked to the first inducible promoter. In another illustrative embodiment, the packaging cell further comprises a nucleic acid sequence encoding Vpx on the second or an optional third transcriptional unit, or on an additional transcriptional unit that is operably linked to the first inducible promoter. In another illustrative embodiment, the polypeptide capable of binding to CD3 and the polypeptide capable of binding to CD28 are each fused to a heterologous GPI anchor attachment sequence. In some instances, the polypeptide capable of binding to CD3 can be anti-CD3 scFvFc or anti-CD3 scFv, and the polypeptide capable of binding to CD28 can be CD80. The anti-CD3 scFvFc or anti-CD3 scFv and CD80 can each be further fused to a DAF signal sequence. In another illustrative embodiment, expression of a fusion polypeptide comprising a cytokine covalently attached to DAF is also induced. In some instances, the cytokine can be IL-7 or IL-15, and the fusion polypeptide can comprise the DAF signal sequence, IL-7 without its signal sequence, and a fragment of DAF comprising a GPI anchor attachment sequence. In another illustrative embodiment, the riboswitch further controls expression of the chimeric antigen receptor in a manner regulated by binding of the riboswitch to the nucleoside analog antiviral drug, which in some instances is acyclovir and/or penciclovir. In another embodiment, the constitutively active IL-7 can be replaced with a miRNA or shRNA or nucleic acids encoding an miRNA or shRNA and IL-7 can be present. The miRNA or shRNA can be encoded by nucleic acids within an intron. In an illustrative embodiment, the retroviral particle is a lentiviral particle.

Provided in another aspect herein is a genetically modified lymphocyte comprising:
A. a first engineered signaling polypeptide comprising a constitutively active IL-7 receptor mutant; and
B. a second engineered signaling polypeptide comprising a chimeric antigen receptor comprising an antigen-specific targeting region (ASTR), a transmembrane domain, and an intracellular activating domain.

In illustrative embodiments of the genetically modified lymphocyte aspect above, the genetically modified lymphocyte is a T cell and/or an NK cell. In certain embodiments, the lymphocyte is a T cell. In another illustrative embodiment, expression of said first engineered signaling polypeptide and/or said second engineered signaling polypeptide is regulated by a riboswitch that binds a nucleoside analog antiviral drug, wherein binding of the nucleoside analog antiviral drug to the riboswitch increases expression of the IL-7 receptor mutant. In another embodiment, the genetically modified lymphocytes express at least one (e.g. two) inhibitory RNA molecules, such as, e.g. a miRNA or an shRNA. The inhibitory RNA molecules can further be encoded by nucleic acids within an intron.

Provided in another aspect herein is a genetically modified T cell and/or NK cell comprising:
a. a first engineered signaling polypeptide comprising at least one lymphoproliferative element; and
b. a second engineered signaling polypeptide comprising a chimeric antigen receptor comprising an antigen-specific targeting region (ASTR), a transmembrane domain, and an intracellular activating domain.

In illustrative embodiments of the genetically modified T cell and/or NK cell aspect, the lymphoproliferative element is constitutively active, and in some instances, is a constitutively active mutated IL-7 receptor or a fragment thereof. In another illustrative embodiment, expression of the first engineered signaling polypeptide and/or the second engineered signaling polypeptide is regulated by a control element. In some instances, the control element is a polynucleotide comprising a riboswitch. In some instances, the riboswitch is capable of binding a nucleoside analog and when the nucleoside analog is present, the first engineered signaling polypeptide and/or the second engineered polypeptide are expressed. In other illustrative embodiments, the genetically modified T cell and/or NK cell has on its surface an activation element, a pseudotyping element, and/or a membrane-bound cytokine. In some instances, the activation element comprises a membrane-bound polypeptide capable of binding to CD3; and/or a membrane-bound polypeptide capable of binding to CD28. In a certain embodiment, the activation element comprises anti-CD3 scFV or an anti-CD3 scFvFc fused to a heterologous GPI anchor attachment sequence and/or CD80 fused to a heterologous GPI anchor attachment sequence. In an illustrative embodiment, the pseudotyping element comprises a Measles Virus F polypeptide, a Measles Virus H polypeptide, and/or cytoplasmic domain deletion variants of a measles virus F polypeptide and/or a measles virus H polypeptide. In other embodiments, the membrane-bound cytokine is a fusion polypeptide comprising IL-7, or a fragment thereof, fused to DAF, or a fragment thereof comprising a GPI anchor attachment sequence.

In one aspect, provided herein is a method for genetically modifying and expanding lymphocytes of a subject, comprising:
A. contacting resting T cells and/or NK cells of the subject *ex vivo,* typically without requiring prior *ex vivo* stimulation, with replication incompetent recombinant retroviral particles comprising:
   i. a pseudotyping element on its surface that is capable of binding to a T cell and/or NK cell and facilitating membrane fusion of the replication incompetent recombinant retroviral particle thereto; and
   ii. a polynucleotide comprising one or more transcriptional units operatively linked to a promoter active in T cells and/or NK cells, wherein the one or more transcriptional units encode a first engineered signaling polypeptide regulated by a control element, wherein said first engineered signaling polypeptide comprises at least one lymphoproliferative element and optionally encode a second engineered signaling polypeptide optionally regulated by a control element, wherein the second engineered signaling polypeptide comprises an intracellular activating domain and optionally other components of a CAR, wherein said contacting facilitates transduction of at least some of the resting T cells and/or NK cells by the replication incompetent recombinant retroviral particles, thereby producing genetically modified T cells and/or NK cells;
B. introducing the genetically modified T cells and/or NK cells into the subject; and exposing the genetically modified T cells and/or NK cells *in vivo* to a compound that acts as the control element to affect expression of the first engineered signaling polypeptide and promote expansion, engraftment, and/or persistence of the lymphocytes *in vivo,* thereby genetically modifying and expanding lymphocytes of the subject.

In illustrative embodiments, the transduction is carried out without *ex vivo* stimulation. In illustrative embodiments, the compound is a molecular chaperone, such as a small molecular chaperone. In illustrative embodiments, binding of the molecular chaperone to the lymphoproliferative element increases the proliferative activity of the lymphoproliferative element. The molecular chaperone can be administered to the subject before the blood is collected, during the contacting, and/or after the T cells and/or NK cells are introduced into the subject. It will be understood with this aspect where the compound is the control element, that such compound typically is capable of binding to a lymphoproliferative element and/or a component of a CAR, and does bind to such lymphoproliferative element or CAR component during performance of the method. Other embodiments and teaches related to methods provided herein that include transfecting a T cell and/or an NK cell with a replication incompetent recombinant retroviral particle, apply to this aspect, including a molecular chaperone embodiment, as well.

In another aspect, provided herein is a method for selecting a microenvironment restricted antigen-specific targeting region, comprising panning a polypeptide display library by:
a. subjecting polypeptides of the polypeptide display library to a binding assay under a normal physiological condition and a binding assay under an aberrant condition; and
b. selecting a polypeptide which exhibits an increase in binding activity at the aberrant condition compared to the physiological condition, thereby selecting the microenvironment restricted antigen specific targeting region.

In another aspect, provided herein is a method for isolating a microenvironment restricted antigen-specific targeting region, comprising:
panning a polypeptide library by:
a) contacting the polypeptide library under aberrant conditions with a target antigen bound to a solid support, wherein clones expressing polypeptides that bind the target antigen remain bound to the solid support through the target antigen;
b) incubating the solid supports with bound polypeptides under physiological conditions; and
c) collecting clones that elute from the solid support under the physiological conditions, thereby isolating the microenvironment restricted antigen-specific targeting region.

In another aspect, provided herein is a chimeric antigen receptor for binding a target antigen, comprising:
a) at least one microenvironment restricted antigen specific targeting region selected by panning a polypeptide library and having an increase in activity in a binding assay at an aberrant condition compared to a normal physiological condition;
b) a transmembrane domain; and
c). an intracellular activating domain.

In another aspect, provided herein is a chimeric antigen receptor for binding a target antigen, comprising:
a) a microenvironment restricted antigen-specific targeting region that exhibits an increase in binding to the target antigen in an aberrant condition compared to a normal physiological environment, wherein the antigen-specific targeting region binds to the target;
b) a transmembrane domain; and
c) an intracellular activating domain.

In illustrative embodiments of any of the methods and compositions provided herein that include a microenvironment restricted antigen specific targeting region (ASTR), the ASTR can have at least a 5%, 10%, 15%, 20%, 25%, 30%, 40%, 50%, 60%, 70%, 80%, 90%, or 100% increase in binding affinity to the target antigen in the assay at the aberrant condition compared to the normal condition. The aberrant conditions can be hypoxia, an acidic pH, a higher concentration of lactic acid, a higher concentration of hyaluronan, a higher concentration of albumin, a higher concentration of adenosine, a higher concentration of R-2-hydroxyglutarate, a higher concentration of PAD enzymes, a higher pressure, a higher oxidation, and a lower nutrient availability. The microenvironment restricted ASTR can exhibit an increase in antigen binding at a pH of 6.7 as compared to a pH of 7.4. The microenvironment restricted ASTR can exhibit an increase in antigen binding in a tumor environment and/or in an *in vitro* tumor surrogate assay condition, relative to a corresponding physiological condition. The target can be 4-1BB,ST4, adenocarcinoma antigen, alpha- fetoprotein, AXL, BAFF, B-lymphoma cell, C242 antigen, CA-125, carbonic anhydrase 9 (CA- IX), C-MET, CCR4, CD 152, CD 19, CD20, CD200, CD22, CD221, CD23 (IgE receptor), CD28, CD30 (TNFRSF8), CD33, CD4, CD40, CD44 v6, CD51, CD52, CD56, CD74, CD80, CEA, CNT0888, CTLA-4, DRS, EGFR, EpCAM, CD3, FAP, fibronectin extra domain-B, folate receptor 1, GD2, GD3 ganglioside, glycoprotein 75, GPNMB, HER2/neu, HGF, human scatter factor receptor kinase, IGF-1 receptor, IGF -I, IgG1, Ll- CAM, IL-13, IL-6, insulin- like growth factor I receptor, integrin nSP1, integrin nvP3, MORAb-009, MS4A1, MUC1, mucin CanAg, Nglycolylneuraminic acid, NPC-1C, PDGF-R a, PDL192, phosphatidylserine, prostatic carcinoma cells, RANKL, RON, ROR1, ROR2 SCH 900105, SDC1, SLAMF7, TAG-72, tenascin C, TGF beta 2, TGF-P, TRAIL-R1, TRAIL-R2, tumor antigen CTAA16. 88, VEGF-A, VEGFR-1, VEGFR2, and vimentin. The ASTR can be an antibody, an antigen, a ligand, a receptor binding domain of a ligand, a receptor, a ligand binding domain of a receptor, or an affibody. The ASTR can be a full-length antibody, a single-chain antibody, an Fab fragment, an Fab' fragment, an (Fab')₂ fragment, an Fv fragment, and a divalent single-chain antibody or a diabody. The ASTR can include a heavy chain and a light chain from an antibody. The antibody can be a single-chain variable fragment. In some embodiments, the heavy and light chains can be separated by a linker, wherein the linker is between 6 and 100 amino acids in length. In some embodiments, the heavy chain can be positioned N-terminal to the light chain on the chimeric antigen receptor and in some embodiments the light chain can be positioned N-terminal to the heavy chain on the chimeric antigen receptor.

In illustrative embodiments of any of the methods that include a polypeptide display library, the polypeptide display library can be a phage display library or a yeast display library. The polypeptide display library can be an antibody display library. The antibody display library can be a human or humanized antibody display library. The antibody display library can be a naive library. The methods can include infecting bacterial cells with the collected phage to generate a refined phage display library, and repeating the contacting, incubating, and collecting for 1 to 1000 cycles, using the refined phage display library generated from a previous cycle.

In illustrative embodiments of any of the methods provided herein that include isolating or selecting a microenvironment restricted ASTR, the method can include determining the nucleotide sequence of a polynucleotide encoding the microenvironment restricted antigen-specific targeting region, thereby determining the polypeptide sequence of the microenvironment restricted ASTR. The methods can include making a microenvironment restricted biologic chimeric antigen receptor by generating a polynucleotide that encodes a polypeptide comprising the microenvironment restricted ASTR, a transmembrane domain, and an intracellular activating domain. The library can be a single chain antibody library.

The methods for isolating a microenvironment restricted ASTR can include the panning is repeated for between 1 and 1000 times. The methods for isolating a microenvironment restricted ASTR can be performed without mutating polynucleotides encoding the isolated microenvironment restricted antigen-specific targeting region between rounds of panning. The methods for isolating a microenvironment restricted ASTR can be performed by culturing, high fidelity amplifying, and/or diluting polynucleotides encoding antigen-specific targeting regions, or host organisms including the same, between rounds of panning. The methods can include, prior to repeating, mutagenizing the selected and/or isolated microenvironment restricted antigen-specific targeting region. The methods can include determining the sequence of the selected and/or isolated microenvironment restricted antigen-specific targeting region, and/or a polynucleotide encoding the same after one or more round of panning via long read DNA sequencing. The methods can include determining the sequence before and after expansion of the isolated microenvironment restricted ASTR. The methods for isolating a microenvironment restricted ASTR can be performed without repeating the panning. The methods for isolating a microenvironment restricted ASTR can be performed without mutating a polynucleotide encoding the isolated microenvironment restricted ASTR after the microenvironment restricted ASTR is isolated.

In illustrative embodiments of any of the compositions provided herein that include a chimeric antigen receptor with a microenvironment restricted ASTR, the microenvironment restricted ASTR can be identified by panning an antibody library. In some embodiments, the microenvironment restricted ASTR is identified by panning a phage display or a yeast display library. In some embodiments, the chimeric antigen receptor comprises a bispecific ASTR.

Provided herein in another aspect is a transduced T cell and/or NK cell, comprising a recombinant polynucleotide comprising one or more transcriptional units operatively linked to a promoter active in T cells and/or NK cells, wherein the one or more transcriptional units encode a first engineered signaling polypeptide regulated by a control element, wherein said first engineered signaling polypeptide comprises a constitutively active IL-7 receptor mutant, and wherein the control element is capable of binding to a compound *in vitro* or *in vivo* or is configured to bind a compound *in vivo.*

Provided herein in another aspect is a replication incompetent recombinant retroviral particle, comprising a recombinant polynucleotide comprising one or more transcriptional units operatively linked to a promoter active in T cells and/or NK cells, wherein the one or more transcriptional units encode a first engineered signaling polypeptide regulated by a control element, which can be an *in vivo* control element, wherein said first engineered signaling polypeptide comprises a constitutively active IL-7 receptor mutant, and wherein the control element is capable of binding to a compound *in vivo* or is configured to bind a compound *in vivo.*

Provided herein in another aspect is a method of transducing a T cell and/or NK cell, comprising contacting a T cell and/or NK cell, with a replication incompetent recombinant retroviral particle comprising a recombinant polynucleotide comprising one or more transcriptional units operatively linked to a promoter active in T cells and/or NK cells, wherein the one or more transcriptional units encode a first engineered signaling polypeptide regulated by a control element, wherein said first engineered signaling polypeptide comprises a constitutively active IL-7 receptor mutant, and wherein the *in vivo* control element is capable of binding to a compound *in vivo* or *in vitro,* under transduction conditions, thereby transducing the T cell and/or NK cell.

In illustrative embodiments of the transduced T cell and/or NK cell aspects, the replication incompetent recombinant retroviral particle aspects, and the method aspects, provided in the preceding paragraphs, the recombinant polynucleotide further comprises a transcriptional unit that encodes a second engineered signaling polypeptide comprising a first chimeric antigen receptor comprising an antigen-specific targeting region (ASTR), a transmembrane domain, and an intracellular activating domain. In other illustrative embodiments, the lymphoproliferative element comprises a mutated IL-7 receptor or a fragment thereof. In other illustrative embodiments, the control element is a polynucleotide comprising a riboswitch. In some instances, the riboswitch is capable of binding a nucleoside analog and the compound that binds the control element is the nucleoside analog. In some instances, the nucleoside analog is an antiviral agent such as for example acyclovir or penciclovir. In certain embodiments, the antiviral agent is acyclovir. In other illustrative embodiments, the constitutively active IL-7 receptor mutant is fused to EGFR or an epitope thereof. In other illustrative embodiments, the constitutively active IL-7 receptor mutant comprises an eTag. In other illustrative embodiments, the constitutively active IL-7 receptor mutant comprises a PPCL insertion. In other illustrative embodiments, the constitutively active IL-7 receptor mutant comprises a PPCL insertion at a position equivalent to position 243 in a wild-type human IL-8 receptor. In other illustrative embodiments, the transduced T cell or NK cell is a transduced T cell.

In another aspect, provided herein is a method for modulating binding of a microenvironment restricted biologic chimeric antigen receptor (MRB-CAR)-expressing T cell or NK cell to a cell expressing a cognate antigen of the MRB-CAR in a subject, including:
a. introducing a T cell and/or NK cell including a nucleic acid encoding the MRB-CAR into the subject, wherein after the introducing, the T cell and/or the NK cell including the nucleic acid encoding the MRB-CAR expresses the MRB-CAR and binds to the cell expressing the cognate antigen in the subject; and
b. administering a pharmacologic agent to the subject in sufficient amount to increase blood pH and/or pH of a tissue and/or pH of a microenvironment, wherein the administering is performed before, during, or after the introducing, and wherein the increased pH of the blood, the tissue, and/or the microenvironment modulates binding of the MRB-CAR expressing T cell and/or NK cell to the cell expressing the cognate antigen in the blood, the tissue, or the microenvironment with the increased pH.

In another aspect, provided herein is a method for alleviating on target off tumor toxicity in a subject, including:
a. introducing a nucleic acid encoding an microenvironment restricted biologic chimeric antigen receptor (MRB-CAR) into a T cell or NK cell of the subject to produce a T cell and/or NK cell including a nucleic acid encoding the MRB-CAR;
b. introducing the T cell and/or NK cell including the nucleic acid encoding the MRB-CAR into the subject, wherein after the introducing, the T cell and/or the NK cell including the nucleic acid encoding the MRB-CAR expresses the MRB-CAR and binds to the cell expressing the cognate antigen in the subject; and
c. administering a pharmacologic agent to the subject in sufficient amount to increase blood pH and/or pH of a tissue and/or pH of a microenvironment to modulate binding of the MRB-CAR to its cognate antigen in the blood, the tissue, and/or the microenvironment with the increased pH, thereby alleviating on target off tumor toxicity in the subject.

In some embodiments, the nucleic acid can be a vector. In illustrative embodiments, the vector is a retroviral particle.

In another aspect, provided herein is a method for controlling binding of a T cell and/or NK cell to a target mammalian cell, including:
a. contacting the target mammalian cell with the T cell and/or NK cell in a microenvironment,
   wherein the target mammalian cell expresses a cognate antigen, and the T cell and/or NK cell expresses a microenvironment restricted biologic chimeric antigen receptor (MRB-CAR) that binds to the cognate antigen differentially at pH 6.7 as compared to pH 7.4; and
b. increasing the pH of the microenvironment by introducing a pharmacologic agent to the microenvironment in sufficient amount, thereby controlling the binding of the T cell and/or NK cell to the target mammalian cell.

In another aspect, provided herein is a method for controlling the binding of a T cell and/or NK cell expressing a microenvironment restricted biologic chimeric antigen receptor (MRB-CAR) to a target mammalian cell in a subject in vivo, including administering a pH-modulating pharmacologic agent to the subject through an effective dosing regimen that increases the pH of a microenvironment within the subject, wherein the subject includes the T cell and/or the NK cell expressing the MRB-CAR, wherein the MRB-CAR binds to its cognate antigen differentially at pH 6.7 as compared to pH 7.4, wherein the microenvironment include the target mammalian cell, wherein the target mammalian cell expresses the cognate antigen on its surface, and wherein the T cell and/or NK cell binds to the target mammalian cell differentially before versus after the pH of the microenvironment is increased, thereby controlling the binding of the T cell and/or NK cell to the target mammalian cell in a subject in vivo.

In any of the aspects provided herein that include a pharmacologic agent and an MRB-CAR, the MRB-CAR can have reduced binding to its cognate antigen at one pH than at a different pH. In illustrative embodiments where illustrative pH values for differential binding of an MRB-CAR are not already provided in the broadest aspect and alternatively for other embodiments in place of those values for such aspects, the MRB-CAR can have reduced binding at a higher pH than at a lower pH. For example, the MRB-CAR can have reduced binding to its cognate antigen at a pH above 7.0, 7.1, 7.2, 7.3, 7.4, or 7.5 than at a pH below 6.4, 6.5, 6.6, 6.7, 6.8, 6.9, or 7.0. In other embodiments, the MRB-CAR can have reduced binding at a higher pH than at a lower pH. For example, the MRB-CAR can have reduced binding to its cognate antigen at a pH below 6.4, 6.5, 6.6, 6.7, 6.8, 6.9, or 7.0 than at a pH above 7.0, 7.1, 7.2, 7.3, 7.4, or 7.5. In some illustrative embodiments, the MRB-CAR exhibits increased binding at a pH of 6.5 to 6.7 compared to pH 7.4 to 7.6. In other illustrative embodiments, the MRB-CAR exhibits increased binding at a pH of 6.7 compared to a pH of 7.4. In other embodiments, the MRB-CAR exhibits increased binding in the pH of a tumor compared to the pH of blood. In some embodiments, the MRB-CAR can include an antigen-specific targeting region, a stalk, and an intracellular activating domain. In some embodiments, the MRB-CAR can also include a co-stimulatory domain. In some embodiments, the MRB-CAR can bind to a tumor associated antigen.

In any of the aspects provided herein that include a pharmacologic agent and an MRB-CAR, the pH of the microenvironment can be increased from a pH below 7.0 to a pH above 7.0. For example, the pH can be increased from a pH below 6.4, 6.5, 6.6, 6.7, 6.8, 6.9, or 7.0 to a pH above 7.0, 7.1, 7.2, 7.3, or 7.4. In some embodiments, the MRB-CAR can bind to the cognate antigen at the increased pH but not a pH of the microenvironment before introducing the pharmacologic agent. In certain embodiments, the pH can be increased from below 7.0 to a pH of 7.1 to 8.0 or to a pH of 7.1 to 7.8 or to a pH of 7.2 to 7.8 or a pH of 7.2 to 7.6 or a pH of 7.3 to 7.6 or to a pH of 7.4 to 7.8 or to a pH of 7.4 to 7.6. Such an increase in pH can occur for less than 1, 2, 4, 6, 8, 12, or 24 hours or for more than 1, 2, 4, 6, 8, 12 or 24 hours depending on the type and dose of pharmacologic agent administered. In certain embodiments, the pharmacologic agent is administered such that the pH remains above 7.0, 7.1, 7.2, 7.3, 7.4, or 7.5; or between 7.0, 7.1, 7.2, 7.3 on the low end of the range and 7.4, 7.5, 7.6, 7.7, or 7.8 on the high end of the range, in the target tissue, such as a tumor, and for example in at least a surface of a target tissue (e.g. tumor) microenvironment, in at least a portion of a target tissue (e.g. tumor) microenvironment, and in illustrative embodiments throughout a target tissue (e.g. tumor) microenvironment.

In any of the aspects provided herein that include a pharmacologic agent and an MRB-CAR, the microenvironment can be an in vivo microenvironment, such as a tumor, a tissue, a non-tumor tissue, a normal tissue, or a tissue that has undergone a transient shift in pH. For example, tissues that typically undergo transient shifts in pH include a muscle tissue in anaerobic conditions or muscle tissue undergoing exercise or an inflamed tissue or a tissue experiencing inflammation. In some embodiments that include a target mammalian cell, the target mammalian cell can be a tumor cell or a non-tumor or normal cell.

In any of the aspects provided herein that include a pharmacologic agent and an MRB-CAR, the pharmacologic agent can be sodium bicarbonate, tris-hydroxylmethyl aminomethane, an equimolar hypertonic solution of sodium bicarbonate and sodium carbonate, or proton pump inhibitors such esomeprazole, esomeprazole and naproxen, lansoprazole, omeprazole, and rabeprazole.

Nucleic acids encoding MRB-CARs of the present disclosure can be introduced through various means into T cells and/or NK cells. In any of the aspects provided herein that include a pharmacologic agent and an MRB-CAR, the introducing step or steps can be performed by
a. contacting resting T cells and/or NK cells of the subject ex vivo without requiring prior ex vivo stimulation, with a replication incompetent recombinant retroviral particle including:
   i. one or more pseudotyping elements on its surface that is capable of binding to a T cell and/or NK cell and facilitating membrane fusion of the replication incompetent recombinant retroviral particle thereto; and
   ii. a polynucleotide including a transcriptional unit operatively linked to a promoter active in T cells and/or NK cells, that encodes the MRB-CAR,
   wherein said contacting facilitates transduction of at least some of the resting T cells and/or NK cells by the replication incompetent recombinant retroviral particle, thereby producing T cells and/or NK cells capable of expressing the MRB-CAR, typically because they now include the polynucleotide that includes a transcriptional unit operatively linked to a promoter active in T cells and/or NK cells, that encodes the MRB-CAR; and
b. introducing the T cells and/or NK cells capable of expressing the MRB-CAR into the subject.

In some embodiments, the T cells and/or NK cells can undergo 2, 3, 4, 5, 6, 7, 8, 9, or 10 or fewer cells divisions ex vivo prior to being introduced. In some embodiments, the resting T cells and/or resting NK cells can be in contact with the replication incompetent recombinant retroviral particle for between 1, 2, 3, 4, 5, 6, 7, 8, 9, 10, 11, or 12 hours on the low end of the range and 6, 7, 8, 9, 10, 11, 12, 13, 14, 15, 16, 17, 18, 19, 20, 21, 22, 23 or 24 hours on the high end of the range, where for a given range a low value is below a high value. In some embodiments, the resting T cells and/or resting NK cells can be from blood which has been collected from the subject. In illustrative embodiments, no more than 12, 15. 16, 18, 21, 24, 30, 36, 42, or 48 hours can pass between the time the blood is collected from the subject and the time the T cells and/or resting NK cells capable of expressing the MRB-CAR are introduced into the subject. In some embodiments, all the steps after collecting the blood and before introducing the T cells and/or resting NK cells capable of expressing the MRB-CAR can be performed in a closed system.

In any embodiment provided herein that includes a replication incompetent recombinant retroviral particle in a method that includes an MRB-CAR and a pharmacologic agent, the polynucleotide that includes a transcriptional unit operatively linked to a promoter active in T cells and/or NK cells that encodes the MRB-CAR is taken up by the T cell(s) and/or NK cell(s) such that such the cell(s) is capable of expressing the MRB-CAR. In illustrative embodiments, the T cell(s) and/or NK cell(s) integrate the polynucleotide into their genome.

In any embodiment provided herein that includes a replication incompetent recombinant retroviral particle in a method that includes an MRB-CAR and a pharmacologic agent, the replication incompetent recombinant retroviral particle can further include an activation element on its surface, such as an activation element that is capable of activating a resting T cell and/or resting NK cell. In some embodiments, the activation element can include any activation element provided in this disclosure. In illustrative embodiments, the activation element can include a membrane-bound polypeptide capable of binding to CD3 and/or a membrane-bound polypeptide capable of binding to CD28. In any of the embodiments that includes an activation element on the surface of replication incompetent recombinant retroviral particle in a method that includes an MRB-CAR and a pharmacologic agent, one or more of the membrane-bound polypeptides can be fused to a heterologous GPI anchor attachment sequence. In some embodiments, the membrane-bound polypeptide capable of binding CD3 and/or the membrane-bound polypeptide capable of binding CD28 can be an scFv or scFvFc that binds CD3 or CD28, respectively. In illustrative embodiments, the membrane-bound polypeptide capable of binding CD3 can be an scFv or scFvFc that binds CD3. In some embodiments, the membrane-bound polypeptide capable of binding CD28 can be the extracellular domains of CD80, CD86, or a functional fragment thereof that is capable of inducing CD28-mediated activation of Akt.

In any embodiment provided herein that includes a replication incompetent recombinant retroviral particle in a method that includes an MRB-CAR and a pharmacologic agent, the polynucleotide encoding the MRB-CAR can be operably linked to a riboswitch. In some embodiments, the riboswitch can be capable of binding a nucleoside analog. In some embodiments, the nucleoside analog can be an antiviral drug, such as acyclovir or penciclovir.

In any embodiment provided herein that includes a replication incompetent recombinant retroviral particle in a method that includes an MRB-CAR and a pharmacologic agent, the replication incompetent recombinant retroviral particle can include on its surface a recognition domain of a monoclonal antibody approved biologic. For example, the recognition domain can include a polypeptide that is recognized by an antibody that recognizes EGFR, or an epitope thereof.

In any embodiment provided herein that includes a replication incompetent recombinant retroviral particle in a method that includes an MRB-CAR and a pharmacologic agent, the one or more pseudotyping elements can include a Measles Virus F polypeptide, a Measles Virus H polypeptide, and/or a fragment thereof that retains the ability to bind to resting T cells and/or resting NK cells. In some embodiments, the one or more pseudotyping elements can include a VSV-G polypeptide. In some embodiments, the replication incompetent recombinant retroviral particle can include on its surface a fusion polypeptide of IL-7, or an active fragment thereof, and DAF including a GPI anchor attachment sequence.

In any embodiment provided herein that includes a replication incompetent recombinant retroviral particle in a method that includes an MRB-CAR and a pharmacologic agent, the genome of the replication incompetent recombinant retroviral particle can encode one or more inhibitory RNA molecules, for example two or more, three or more, four or more, five or more, or six or more inhibitory RNA molecules. In some embodiments, the inhibitory RNA molecules can be directed against different RNA targets. In some embodiments, the inhibitory RNA molecules can be located within an intron. In some embodiments, the inhibitory RNA molecules are capable of forming a 5' stem and a 3' stem that form a 18-25 nucleotide RNA duplex. In some embodiments, at least one of the inhibitory RNA molecules can include from 5' to 3' orientation: a 5' microRNA flanking sequence, a 5' stem, a loop, a 3' stem, and a 3' microRNA flanking sequence, wherein the 5' stem or the 3' stem is capable of binding to an RNA target. In further embodiments, the 5' stem can 18 to 25 nucleotides in length, wherein said 3' stem is 18 to 25 nucleotides in length, wherein said loop is 3 to 40 nucleotides in length. In some embodiments, one or more of the 5' microRNA flanking sequence and the 3' microRNA flanking sequence can be derived from a naturally occurring miRNA, such as mIR-155.

In another aspect, provided herein is a pH-modulating pharmacologic agent for use in a method for controlling the binding of a T cell and/or NK cell to a target mammalian cell in a subject in vivo, including administering the pH-modulating pharmacologic agent to the subject through an effective dosing regimen that increases the pH of a microenvironment within the subject, wherein the subject includes the T cell and/or the NK cell, wherein the T cell and/or NK cell expresses a microenvironment restricted biologic chimeric antigen receptor (MRB-CAR) that binds to its cognate antigen differentially at pH 6.7 as compared to pH 7.4, wherein the T cell and/or NK cell expresses the MRB-CAR, wherein the microenvironment includes the target mammalian cell, wherein the target mammalian cell expresses the cognate antigen on their surface, and wherein the T cell and/or NK cell binds to the target mammalian cell differentially before versus after the pH of the microenvironment is increased by administering the pH-modulating pharmacologic agent thereby controlling the binding of the T cell and/or NK cell to the target mammalian cell in a subject in vivo.

In another aspect, provided herein is a pharmacologic agent for use in a method for modulating the binding of a microenvironment restricted biologic chimeric antigen receptor (MRB-CAR) expressing T cell or NK cell to a cell expressing a cognate antigen of the MRB-CAR in a subject, for treating tumor growth, wherein the method includes:
a. introducing a T cell and/or NK cell capable of expressing the MRB-CAR into the subject,
   wherein the MRB-CAR binds to the cell expressing the cognate antigen in the subject, wherein after the introducing, the T cell and/or the NK cell including the nucleic acid encoding the MRB-CAR expresses the MRB-CAR and binds to the cell expressing the cognate antigen in the subject; and
b. administering the pharmacologic agent to the subject in sufficient amount to increase blood pH and/or a tissue pH and/or a microenvironment pH to modulate binding of the MRB-CAR expressing T cell and/or NK cell to the cell expressing the cognate antigen in the blood, the tissue, or the microenvironment with the increased pH.

In another aspect, provided herein is a pharmacologic agent for use in a method for alleviating on target off tumor toxicity in a subject, wherein the method includes:
a. introducing a nucleic acid encoding a microenvironment restricted biologic chimeric antigen receptor (MRB-CAR) into a T cell or NK cell of the subject, to produce a T cell and/or NK cell capable of expressing the MRB-CAR;
b. introducing the T cell and/or NK cell capable of expressing the MRB-CAR into the subject, wherein after the introducing, the T cell and/or the NK cell including the nucleic acid encoding the MRB-CAR expresses the MRB-CAR and binds to the cell expressing the cognate antigen in the subject; and
c. administering the pharmacologic agent to the subject in sufficient amount to increase blood pH and/or a tissue pH and/or a microenvironment pH to modulate binding of the MRB-CAR to its cognate antigen in the blood, the tissue, and/or the microenvironment with the increased pH, thereby alleviating on target off tumor toxicity in the subject.

In another aspect, provided herein is a pharmacologic agent for use in a method for controlling the binding of a T cell and/or NK cell expressing a microenvironment restricted biologic chimeric antigen receptor (MRB-CAR) to a target mammalian cell, for treating tumor growth, wherein the method includes:
a. contacting the target mammalian cell with the T cell and/or NK cell expressing the MRB-CAR in a microenvironment, wherein the target mammalian cell expresses a cognate antigen, and the T cell and/or NK cell expresses the MRB-CAR that binds to the cognate antigen differentially at pH 6.7 as compared to pH 7.4; and
b. increasing the pH of the microenvironment by introducing the pharmacologic agent to the microenvironment in sufficient amount, thereby controlling the binding of the T cell and/or NK cell expressing the MRB-CAR to the target mammalian cell.

In another aspect, provided herein is a pharmacologic agent for use in a method for controlling the binding of a T cell and/or NK cell expressing a microenvironment restricted biologic chimeric antigen receptor (MRB-CAR) to a target mammalian cell in a subject in vivo, for treating tumor growth, wherein the pharmacologic agent is a pH-modulating pharmacologic agent, and wherein the method includes administering the pH-modulating pharmacologic agent to the subject through an effective dosing regimen that increases the pH of a microenvironment within the subject, wherein the subject includes the T cell and/or NK cell expressing the MRB-CAR, wherein the MRB-CAR binds to its cognate antigen differentially at pH 6.7 as compared to pH 7.4, wherein the microenvironment includes the target mammalian cell, wherein the target mammalian cell expresses the cognate antigen on its surface, and wherein the T cell and/or NK cell binds to the target mammalian cell differentially before versus after the pH of the microenvironment is increased.

In another aspect, provided herein is a pH-modulating pharmacologic agent for use in a method for controlling the binding of a T cell and/or NK cell expressing a microenvironment restricted biologic chimeric antigen receptor (MRB-CAR) to a target mammalian cell in a subject in vivo, for treating tumor growth, wherein the method includes administering the pH-modulating pharmacologic agent to the subject through an effective dosing regimen that increases the pH of a microenvironment within the subject, wherein the subject includes the T cell and/or NK cell expressing the MRB-CAR, wherein the MRB-CAR binds to its cognate antigen differentially at pH 6.7 as compared to pH 7.4, wherein the microenvironment includes the target mammalian cell, wherein the target mammalian cell expresses the cognate antigen on its surface, and wherein the T cell and/or NK cell binds to the target mammalian cell differentially before versus after the pH of the microenvironment is increased by administering the pH-modulating pharmacologic agent.

In another aspect, provided herein is a use of a pH-modulating pharmacologic agent for use in the manufacture of a medicament for controlling the binding of a T cell and/or NK cell expressing a microenvironment restricted biologic chimeric antigen receptor (MRB-CAR) to a target mammalian cell in a subject in vivo, wherein the pH-modulating pharmacologic agent is to be administered to the subject through an effective dosing regimen that increases the pH of a microenvironment within the subject, wherein the subject includes the T cell and/or NK cell expressing the MRB-CAR, wherein the MRB-CAR binds to its cognate antigen differentially at pH 6.7 as compared to pH 7.4, wherein the microenvironment includes the target mammalian cell, wherein the target mammalian cell expresses the cognate antigen on their surface, and wherein the T cell binds to the target mammalian cell differentially before versus after the pH of the microenvironment is increased by administering the pH-modulating pharmacologic agent.

In any of the aspects provided herein that include a pH-modulating pharmacologic agent or a pharmacologic agent for use in a method and an MRB-CAR or include the use of a pH-modulating pharmacologic agent and an MRB-CAR, the MRB-CAR can have reduced binding to its cognate antigen at one pH than at a different pH. In illustrative embodiments where illustrative pH values for differential binding of an MRB-CAR are not already provided in the broadest aspect and alternatively for other embodiments in place of those values for such aspects, the MRB-CAR can have reduced binding at a higher pH than at a lower pH. For example, the MRB-CAR can have reduced binding to its cognate antigen at a pH above 7.0, 7.1, 7.2, 7.3, 7.4, or 7.5 than at a pH below 6.4, 6.5, 6.6, 6.7, 6.8, 6.9, or 7.0. In other embodiments, the MRB-CAR can have reduced binding at a higher pH than at a lower pH. For example, the MRB-CAR can have reduced binding to its cognate antigen at a pH below 6.4, 6.5, 6.6, 6.7, 6.8, 6.9, or 7.0 than at a pH above 7.0, 7.1, 7.2, 7.3, 7.4, or 7.5. In some illustrative embodiments, the MRB-CAR exhibits increased binding at a pH of 6.5 to 6.7 compared to pH 7.4 to 7.6. In other illustrative embodiments, the MRB-CAR exhibits increased binding at a pH of 6.7 compared to a pH of 7.4. In other embodiments, the MRB-CAR exhibits increased binding in the pH of a tumor compared to the pH of blood. In some embodiments, the MRB-CAR can include an antigen-specific targeting region, a stalk, and an intracellular activating domain. In some embodiments, the MRB-CAR can also include a co-stimulatory domain. In some embodiments, the MRB-CAR can bind to a tumor associated antigen.

In any of the aspects provided herein that include a pH-modulating pharmacologic agent or a pharmacologic agent for use in a method and an MRB-CAR or include the use of a pH-modulating pharmacologic agent and an MRB-CAR, the pH of the microenvironment can be increased from a pH below 7.0 to a pH above 7.0. For example, the pH can be increased from a pH below 6.4, 6.5, 6.6, 6.7, 6.8, 6.9, or 7.0 to a pH above 7.0, 7.1, 7.2, 7.3, or 7.4. In some embodiments, the MRB-CAR can bind to the cognate antigen at the increased pH but not a pH of the microenvironment before introducing the pharmacologic agent. In certain embodiments, the pH can be increased from below 7.0 to a pH of 7.1 to 8.0 or to a pH of 7.1 to 7.8 or to a pH of 7.2 to 7.8 or a pH of 7.2 to 7.6 or a pH of 7.3 to 7.6 or to a pH of 7.4 to 7.8 or to a pH of 7.4 to 7.6. Such an increase in pH can occur for less than 1, 2, 4, 6, 8, 12, or 24 hours or for more than 1, 2, 4, 6, 8, 12 or 24 hours depending on the type and dose of pharmacologic agent administered. In certain embodiments, the pharmacologic agent is administered such that the pH remains above 7.0, 7.1, 7.2, 7.3, 7.4, or 7.5; or between 7.0, 7.1, 7.2, 7.3 on the low end of the range and 7.4, 7.5, 7.6, 7.7, or 7.8 on the high end of the range, in the target tissue, such as a tumor, and for example in at least a surface of a target tissue (e.g. tumor) microenvironment, in at least a portion of a target tissue (e.g. tumor) microenvironment, and in illustrative embodiments throughout a target tissue (e.g. tumor) microenvironment.

In any of the aspects provided herein that include a pH-modulating pharmacologic agent or a pharmacologic agent for use in a method and an MRB-CAR or include the use of a pH-modulating pharmacologic agent and an MRB-CAR, the microenvironment can be an in vivo microenvironment, such as a tumor, a tissue, a non-tumor tissue, a normal tissue, or a tissue that has undergone a transient shift in pH. For example, tissues that typically undergo transient shifts in pH include a muscle tissue in anaerobic conditions or muscle tissue undergoing exercise or an inflamed tissue or a tissue experiencing inflammation. In some embodiments that include a target mammalian cell, the target mammalian cell can be a tumor cell or a non-tumor or normal cell.

In any of the aspects provided herein that include a pH-modulating pharmacologic agent or a pharmacologic agent for use in a method and an MRB-CAR or include the use of a pH-modulating pharmacologic agent and an MRB-CAR, the pharmacologic agent can be sodium bicarbonate, tris-hydroxylmethyl aminomethane, an equimolar hypertonic solution of sodium bicarbonate and sodium carbonate, or proton pump inhibitors such esomeprazole, esomeprazole and naproxen, lansoprazole, omeprazole, and rabeprazole.

In any of the aspects provided herein that include a pH-modulating pharmacologic agent or a pharmacologic agent for use in a method and an MRB-CAR or include the use of a pH-modulating pharmacologic agent and an MRB-CAR, the pharmacologic agent can be used in a method for the treatment of cancer, tumors, tumor growth, or a cell proliferative disorder.

In another aspect, provided herein is a kit containing a container containing a replication incompetent recombinant retroviral particle, and instructions for use thereof for treating tumor growth, wherein the instructions instruct a method for controlling the binding of a T cell and/or NK cell to a target mammalian cell, in a method including:
a. transducing the T cell and/or NK cell with the replication incompetent recombinant retroviral particle including in its genome a microenvironment restricted biologic chimeric antigen receptor (MRB-CAR) that binds to the cognate antigen differentially at pH 6.7 as compared to pH 7.4 to produce a T cell and/or NK cell capable of expressing the MRB-CAR;
b. introducing the T cell and/or NK cell capable of expressing the MRB-CAR into the subject, wherein after the introducing, the T cell and/or the NK cell including the nucleic acid encoding the MRB-CAR expresses the MRB-CAR and binds to the cell expressing the cognate antigen in the subject;
c. contacting the target mammalian cell with the MRB-CAR expressing T cell and/or NK cell in a microenvironment, wherein the target mammalian cell expresses a cognate antigen of the MRB-CAR, and the T cell and/or NK cell expresses the MRB-CAR; and
d. increasing the pH of the microenvironment by introducing a pH-modulating pharmacologic agent to the microenvironment in sufficient amount, thereby affecting the binding of the target mammalian cell with the T cell and/or NK cell.
In some embodiments, the kit can further include a pH-modulating pharmacologic agent.

In some embodiments of the kit, the MRB-CAR can have reduced binding to its cognate antigen at one pH than at a different pH. In illustrative embodiments where illustrative pH values for differential binding of an MRB-CAR are not already provided in the broadest aspect and alternatively for other embodiments in place of those values for such aspects, the MRB-CAR can have reduced binding at a higher pH than at a lower pH. For example, the MRB-CAR can have reduced binding to its cognate antigen at a pH above 7.0, 7.1, 7.2, 7.3, 7.4, or 7.5 than at a pH below 6.4, 6.5, 6.6, 6.7, 6.8, 6.9, or 7.0. In other embodiments, the MRB-CAR can have reduced binding at a higher pH than at a lower pH. For example, the MRB-CAR can have reduced binding to its cognate antigen at a pH below 6.4, 6.5, 6.6, 6.7, 6.8, 6.9, or 7.0 than at a pH above 7.0, 7.1, 7.2, 7.3, 7.4, or 7.5. In some illustrative embodiments, the MRB-CAR exhibits increased binding at a pH of 6.5 to 6.7 compared to pH 7.4 to 7.6. In other illustrative embodiments, the MRB-CAR exhibits increased binding at a pH of 6.7 compared to a pH of 7.4. In other embodiments, the MRB-CAR exhibits increased binding in the pH of a tumor compared to the pH of blood. In some embodiments, the MRB-CAR can include an antigen-specific targeting region, a stalk, and an intracellular activating domain. In some embodiments, the MRB-CAR can also include a co-stimulatory domain. In some embodiments, the MRB-CAR can bind to a tumor associated antigen.

In some embodiments of the kit, the pH of the microenvironment can be increased from a pH below 7.0 to a pH above 7.0. For example, the pH can be increased from a pH below 6.4, 6.5, 6.6, 6.7, 6.8, 6.9, or 7.0 to a pH above 7.0, 7.1, 7.2, 7.3, or 7.4. In some embodiments, the MRB-CAR can bind to the cognate antigen at the increased pH but not a pH of the microenvironment before introducing the pharmacologic agent. In certain embodiments, the pH can be increased from below 7.0 to a pH of 7.1 to 8.0 or to a pH of 7.1 to 7.8 or to a pH of 7.2 to 7.8 or a pH of 7.2 to 7.6 or a pH of 7.3 to 7.6 or to a pH of 7.4 to 7.8 or to a pH of 7.4 to 7.6. Such an increase in pH can occur for less than 1, 2, 4, 6, 8, 12, or 24 hours or for more than 1, 2, 4, 6, 8, 12 or 24 hours depending on the type and dose of pharmacologic agent administered. In certain embodiments, the pharmacologic agent is administered such that the pH remains above 7.0, 7.1, 7.2, 7.3, 7.4, or 7.5; or between 7.0, 7.1, 7.2, 7.3 on the low end of the range and 7.4, 7.5, 7.6, 7.7, or 7.8 on the high end of the range, in the target tissue, such as a tumor, and for example in at least a surface of a target tissue (e.g. tumor) microenvironment, in at least a portion of a target tissue (e.g. tumor) microenvironment, and in illustrative embodiments throughout a target tissue (e.g. tumor) microenvironment. In some embodiments, the microenvironment can be an in vivo microenvironment, such as a tumor, a tissue, a non-tumor tissue, a normal tissue, or a tissue that has undergone a transient shift in pH. For example, tissues that typically undergo transient shifts in pH include a muscle tissue in anaerobic conditions or muscle tissue undergoing exercise or an inflamed tissue or a tissue experiencing inflammation. In some embodiments that include a target mammalian cell, the target mammalian cell can be a tumor cell or a non-tumor or normal cell.

In some embodiments of the kit, the pharmacologic agent can be sodium bicarbonate, tris-hydroxylmethyl aminomethane, an equimolar hypertonic solution of sodium bicarbonate and sodium carbonate, or proton pump inhibitors such esomeprazole, esomeprazole and naproxen, lansoprazole, omeprazole, and rabeprazole.

In one aspect, provided herein is a replication incompetent recombinant retroviral particle comprising in its genome a polynucleotide comprising one or more nucleic acid sequences operatively linked to a promoter active in T cells and/or NK cells, wherein:
a. a first nucleic acid sequence of the one or more nucleic acid sequences encodes one or more (e.g. two or more) inhibitory RNA molecules directed against one or more RNA targets, and
b. a second nucleic acid sequence of the one or more nucleic acid sequences encodes a chimeric antigen receptor (CAR) comprising an antigen-specific targeting region (ASTR), a transmembrane domain, and an intracellular activating domain.

Provided in another aspect herein is a mammalian packaging cell line comprising a packageable RNA genome for a replication incompetent retroviral particle, wherein said packageable RNA genome comprises:
a. a 5' long terminal repeat, or active fragment thereof;
b. a nucleic acid sequence encoding a retroviral cis-acting RNA packaging element;
c. a polynucleotide comprising one or more nucleic acid sequences operatively linked to a promoter active in T cells and/or NK cells, wherein a first nucleic acid sequence of the one or more nucleic acids encodes one or more (e.g. two or more) inhibitory RNA molecules directed against one or more RNA targets and a second nucleic acid sequence of the one or more nucleic acid sequences encodes a chimeric antigen receptor (CAR) comprising an antigen-specific targeting region (ASTR), a transmembrane domain, and an intracellular activating domain; and
d. a 3' long terminal repeat, or active fragment thereof.

In some embodiments of the mammalian packaging cell line aspect, the polynucleotide of (c) can be in reverse orientation to the nucleic acid sequence encoding the retroviral cis-acting RNA packaging element (b), the 5' long terminal repeat (a), and/or the 3' long terminal repeat (d).

In some embodiments of the mammalian packaging cell line aspect, expression of the packageable RNA genome is driven by an inducible promoter active in the mammalian packaging cell line.

In some embodiments of the mammalian packaging cell line aspect, the retroviral cis-acting RNA packaging element can comprise a central polypurine tract (cPPT)/central termination sequence, an HIV Psi, or a combination thereof.

Provided in another aspect herein is a retroviral vector comprising a packageable RNA genome for a replication incompetent retroviral particle, wherein said packageable RNA genome comprises:
a. a 5' long terminal repeat, or active fragment thereof;
b. a nucleic acid sequence encoding a retroviral cis-acting RNA packaging element;
c. a polynucleotide comprising one or more nucleic acid sequences operatively linked to a promoter active in T cells and/or NK cells, wherein a first nucleic acid sequence of the one or more nucleic acids encodes one or more (e.g. two or more) inhibitory RNA molecules directed against one or more RNA targets and a second nucleic acid sequence of the one or more nucleic acid sequences encodes a chimeric antigen receptor (CAR) comprising an antigen-specific targeting region (ASTR), a transmembrane domain, and an intracellular activating domain; and
d. a 3' long terminal repeat, or active fragment thereof.

In some embodiments of the retroviral vector aspect, the polynucleotide of (c) can be in reverse orientation to the nucleic acid sequence encoding the retroviral cis-acting RNA packaging element (b), the 5' long terminal repeat (a), and/or the 3' long terminal repeat (d).

In some embodiments of the retroviral vector aspect, expression of the packageable RNA genome is driven by an inducible promoter active in the mammalian packaging cell line.

In some embodiments of the retroviral vector aspect, the retroviral cis-acting RNA packaging element can comprise a central polypurine tract (cPPT)/central termination sequence, an HIV Psi, or a combination thereof. The retroviral vector can optionally include an antibiotic resistance gene and/or a detectable marker.

Provided herein in another aspect is a method for genetically modifying or transducing a lymphocyte (e.g. a T cell or an NK cell) or a population thereof, of a subject, comprising contacting the lymphocyte (e.g. the T cell or NK cell) or a population thereof, of the subject ex vivo, with a replication incompetent recombinant retroviral particle comprising in its genome a polynucleotide comprising one or more nucleic acid sequences operatively linked to a promoter active in lymphocytes (e.g. T cells and/or NK cells), wherein a first nucleic acid sequence of the one or more nucleic acid sequences encodes one or more (e.g. two or more) inhibitory RNA molecules directed against one or more RNA targets and a second nucleic acid sequence of the one or more nucleic acid sequences encodes a chimeric antigen receptor (CAR) comprising an antigen-specific targeting region (ASTR), a transmembrane domain, and an intracellular activating domain, wherein said contacting facilitates genetic modification and/or transduction of the lymphocyte (e.g. T cell or NK cell), or at least some of the lymphocytes (e.g. T cells and/or NK cells) by the replication incompetent recombinant retroviral particle, thereby producing a genetically modified and/or transduced lymphocyte (e.g. T cell and/or NK cell).

In some embodiments of the method provided immediately above, the genetically modified and/or transduced lymphocyte (e.g. T cell and/or NK cell) or population thereof, is introduced into the subject. In some embodiments, the genetically modified and/or transduced lymphocyte (e.g. T cell and/or NK cell) or population thereof, undergoes 4 or fewer cell divisions ex vivo prior to being introduced or reintroduced into the subject. In some embodiments, the lymphocyte(s) are resting T cells and/or resting NK cells that are in contact with the replication incompetent recombinant retroviral particles for between 1 hour and 12 hours. In some embodiments, no more than 8 hours pass between the time blood is collected from the subject and the time the genetically modified T cells and/or NK cells are reintroduced into the subject. In some embodiments, all steps after the blood is collected and before the blood is reintroduced, are performed in a closed system in which a person monitors the closed system throughout the processing.

Provided herein in another aspect is a genetically modified T cell and/or NK cell comprising:
a. one or more (e.g. two or more) inhibitory RNA molecules directed against one or more RNA targets; and
b. a chimeric antigen receptor (CAR) comprising an antigen-specific targeting region (ASTR), a transmembrane domain, and an intracellular activating domain, wherein said one or more (e.g. two or more) inhibitory RNA molecules and the CAR are encoded by nucleic acid sequences that are genetic modifications of the T cell and/or NK cell.

In some embodiments of the genetically modified T cell and/or NK cell aspect, the genetically modified T cell and/or NK cell also comprises at least one lymphoproliferative element that is not an inhibitory RNA molecule, wherein said lymphoproliferative element is encoded by a nucleic acid that is a genetic modification of the T cell and/or NK cell. In some embodiments, the inhibitory RNA molecules, the CAR, and/or the at least one lymphoproliferative element are expressed in a polycistronic matter. In illustrative embodiments, the inhibitory RNA molecules are expressed from a single polycistronic transcript.

Provided herein in another aspect is a replication incompetent recombinant retroviral particle for use in a method for genetically modifying a lymphocyte of a subject, for treating tumor growth, wherein the replication incompetent recombinant retroviral particle comprises in its genome a polynucleotide comprising one or more nucleic acid sequences operatively linked to a promoter active in T cells and/or NK cells, wherein a first nucleic acid sequence of the one or more nucleic acid sequences encodes one or more (e.g. two or more) inhibitory RNA molecules directed against one or more RNA targets and a second nucleic acid sequence of the one or more nucleic acid sequences encodes a chimeric antigen receptor (CAR) comprising an antigen-specific targeting region (ASTR), a transmembrane domain, and an intracellular activating domain, wherein the method comprises contacting a T cell and/or NK cell of the subject ex vivo, and said contacting facilitates transduction of at least some of the resting T cells and/or NK cells by the replication incompetent recombinant retroviral particles, thereby producing a genetically modified T cell and/or NK cell.

In the method for genetically modifying a lymphocyte of a subject aspect provided immediately above, in some embodiments, a pharmacologic agent is used in the method, which further includes introducing the genetically engineered T cell and/or an NK cell into the subject.

Provided herein in another aspect is a replication incompetent recombinant retroviral particle for use in a method for genetically modifying a T cell and/or NK cell of a subject, for treating tumor growth, wherein the method comprises:
a. contacting the T cell and/or NK cell of the subject ex vivo, with a replication incompetent recombinant retroviral particle comprising in its genome a polynucleotide comprising one or more nucleic acid sequences operatively linked to a promoter active in T cells and/or NK cells, wherein a first nucleic acid sequence of the one or more nucleic acid sequences encodes one or more (e.g. two or more) inhibitory RNA molecules directed against one or more RNA targets and a second nucleic acid sequence of the one or more nucleic acid sequences encodes a chimeric antigen receptor (CAR) comprising an antigen-specific targeting region (ASTR), a transmembrane domain, and an intracellular activating domain, wherein said contacting facilitates transduction of at least some of the resting T cells and/or NK cells by the replication incompetent recombinant retroviral particles, thereby producing a genetically modified T cell and/or NK cell; and
b. introducing the genetically modified T cell and/or NK cell into the subject, thereby genetically modifying the T cell and/or NK cell of the subject.

In the aspect provided immediately above, in some embodiments, a population of T cells and/or NK cells are contacted in the contacting step, and introduced into the subject in the introducing step.

Provided herein in another aspect is the use of a replication incompetent recombinant retroviral particle in the manufacture of a kit for genetically modifying a T cell and/or NK cell of a subject, wherein the use of the kit comprises:
1. contacting the T cell and/or NK cell of the subject ex vivo, with a replication incompetent recombinant retroviral particle comprising in its genome a polynucleotide comprising one or more nucleic acid sequences operatively linked to a promoter active in T cells and/or NK cells, wherein a first nucleic acid sequence of the one or more nucleic acid sequences encodes one or more (e.g. two or more) inhibitory RNA molecules directed against one or more target and a second nucleic acid sequence of the one or more nucleic acid sequences encodes a chimeric antigen receptor (CAR) comprising an antigen-specific targeting region (ASTR), a transmembrane domain, and an intracellular activating domain, wherein said contacting facilitates transduction of at least some of the resting T cells and/or NK cells by the replication incompetent recombinant retroviral particles, thereby producing a genetically modified T cell and/or NK cell; and
2. introducing the genetically modified T cell and/or NK cell into the subject, thereby genetically modifying the T cell and/or NK cell of the subject.

Provided herein in another aspect is the use of a replication incompetent recombinant retroviral particle in the manufacture of a medicament for genetically modifying a T cell and/or NK cell of a subject, wherein the use of the medicament comprises:
A) contacting the T cell and/or NK cell of the subject ex vivo, with a replication incompetent recombinant retroviral particle comprising in its genome a polynucleotide comprising one or more nucleic acid sequences operatively linked to a promoter active in T cells and/or NK cells, wherein a first nucleic acid sequence of the one or more nucleic acid sequences encodes one or more (e.g. two or more) inhibitory RNA molecules directed against one or more target and a second nucleic acid sequence of the one or more nucleic acid sequences encodes a chimeric antigen receptor (CAR) comprising an antigen-specific targeting region (ASTR), a transmembrane domain, and an intracellular activating domain, wherein said contacting facilitates transduction of at least some of the resting T cells and/or NK cells by the replication incompetent recombinant retroviral particles, thereby producing a genetically modified T cell and/or NK cell; and
B) introducing the genetically modified T cell and/or NK cell into the subject, thereby genetically modifying the T cell and/or NK cell of the subject.

Provided herein in another aspect is a commercial container containing a replication incompetent recombinant retroviral particle and instructions for the use thereof to treat tumor growth in a subject, wherein the replication incompetent recombinant retroviral particle comprises in its genome a polynucleotide comprising one or more nucleic acid sequences operatively linked to a promoter active in T cells and/or NK cells, wherein a first nucleic acid sequence of the one or more nucleic acid sequences encodes one or more (e.g. two or more) inhibitory RNA molecules directed against one or more RNA targets and a second nucleic acid sequence of the one or more nucleic acid sequences encodes a chimeric antigen receptor (CAR) comprising an antigen-specific targeting region (ASTR), a transmembrane domain, and an intracellular activating domain.

In some embodiments, in the aspects of the commercial container, the instructions instruct a user to contact a T cell and/or NK cell of the subject ex vivo, to facilitate transduction of at least one resting T cell and/or NK cell of the subject by the replication incompetent recombinant retroviral particles, thereby producing a genetically modified T cell and/or NK cell.

In any of the aspects provided herein that include a polynucleotide comprising one or more nucleic acid sequences operatively linked to a promoter active in T cells and/or NK cells, wherein a first nucleic acid sequence of the one or more nucleic acid sequences encodes one or more (e.g. two or more) inhibitory RNA molecules directed against one or more RNA targets, and a second nucleic acid sequence of the one or more nucleic acid sequences encodes a chimeric antigen receptor (CAR) comprising an antigen-specific targeting region (ASTR), a transmembrane domain, and an intracellular activating domain, the polynucleotide may further include a third nucleic acid sequence that encodes at least one lymphoproliferative element that is not an inhibitory RNA molecule. In some embodiments, the lymphoproliferative element can be a cytokine or cytokine receptor polypeptide, or a fragment thereof comprising a signaling domain. In some embodiments, the lymphoproliferative element is constitutively active. In certain embodiments, the lymphoproliferative element can be an IL-7 receptor or a fragment thereof. In illustrative embodiments, the lymphoproliferative element can be a constitutively active IL-7 receptor or a constitutively active fragment thereof.

In any of the aspects provided herein that include a polynucleotide comprising one or more nucleic acid sequences operatively linked to a promoter active in T cells and/or NK cells, wherein a first nucleic acid sequence of the one or more nucleic acid sequences encodes one or more (e.g. two or more) inhibitory RNA molecules directed against one or more RNA targets, an inhibitory RNA molecule can in some embodiments include a 5' strand and a 3' strand that are partially or fully complementary to one another, wherein said 5' strand and said 3' strand are capable of forming an 18-25 nucleotide RNA duplex. In some embodiments, the 5' strand can be 18, 19, 20, 21, 22, 23, 24, or 25 nucleotides in length, and the 3' strand can be 18, 19, 20, 21, 22, 23, 24, or 25 nucleotides in length. In some embodiments, the 5' strand and the 3' strand can be the same or different lengths. In some embodiments, the RNA duplex can include one or more mismatches. In alternate embodiments, the RNA duplex has no mismatches.

In any of the aspects provided herein that include a polynucleotide comprising one or more nucleic acid sequences operatively linked to a promoter active in T cells and/or NK cells, wherein a first nucleic acid sequence of the one or more nucleic acid sequences encodes one or more (e.g. two or more) inhibitory RNA molecules directed against one or more RNA targets, an inhibitory RNA molecule can be a miRNA or an shRNA. In some embodiments, the inhibitory molecule can be a precursor of a miRNA, such as for example, a Pri-miRNA or a Pre-miRNA, or a precursor of an shRNA. In some embodiments, the inhibitory molecule can be an artificially derived miRNA or shRNA. In other embodiments, the inhibitory RNA molecule can be a dsRNA (either transcribed or artificially introduced) that is processed into an siRNA or the siRNA itself. In some embodiments, the inhibitory RNA molecule can be a miRNA or shRNA that has a sequence that is not found in nature, or has at least one functional segment that is not found in nature, or has a combination of functional segments that are not found in nature. In illustrative embodiments, at least one or all of the inhibitory RNA molecules are miR-155.

In any of the aspects provided herein that include a polynucleotide comprising one or more nucleic acid sequences operatively linked to a promoter active in T cells and/or NK cells, wherein a first nucleic acid sequence of the one or more nucleic acid sequences encodes one or more (e.g. two or more) inhibitory RNA molecules directed against one or more RNA targets, an inhibitory RNA molecule, in some embodiments, can comprises from 5' to 3' orientation: a 5' arm, a 5' stem, a loop, a 3' stem that is partially or fully complementary to said 5' stem, and a 3' arm. In some embodiments, at least one of the two or more inhibitory RNA molecules has this arrangement. In other embodiments, all of the two or more inhibitory RNA molecules have this arrangement. In some embodiments, the 5' stem can be 18, 19, 20, 21, 22, 23, 24 or 25 nucleotides in length. In some embodiments, the 3' stem can be 18, 19, 20, 21, 22, 23, 24, or 25 nucleotides in length. In some embodiments, the loop can be 3, 4,5, 6, 7, 8, 9, 10, 11, 12, 13, 14, 15, 16, 17, 18, 19, 20, 21, 22, 23, 24,2 5, 26, 27, 28, 29, 30, 31, 32, 33, 34, 35, 36, 37, 38, 39, or 40 nucleotides in length. In some embodiments, the 5' arm, 3' arm, or both, are derived from a naturally occurring miRNA. In some embodiments, the 5' arm, 3' arm, or both, are derived from a naturally occurring miRNA is selected from the group consisting of: miR-155, miR-30, miR-17-92, miR-122, and miR-21. In illustrative embodiments, the 5' arm, 3' arm, or both are derived from miR-155. In some embodiments, the 5' arm, 3' arm, or both are derived from *Mus musculus* miR-155 or *Homo sapiens* miR-155. In some embodiments, the 5' arm has the sequence set forth in SEQ ID NO:256 or is a functional variant thereof, such as, for example, a sequence that is the same length as SEQ ID NO:256, or 95%, 90%, 85%, 80%,75%, or 50% as long as SEQ ID NO: 256 or is 100 nucleotides or less, 95 nucleotides or less, 90 nucleotides or less, 85 nucleotides or less, 80 nucleotides or less, 75 nucleotides or less, 70 nucleotides or less, 65 nucleotides or less, 60 nucleotides or less, 55 nucleotides or less, 50 nucleotides or less, 45 nucleotides or less, 40 nucleotides or less, 35 nucleotides or less, 30 nucleotides or less, or 25 nucleotides or less; and is at least 50%, 55%, 60%, 65%, 70%, 75%, 80%, 85%, 90%, or 95% identical to SEQ ID NO:256. In some embodiments, the 3' arm has the sequence set forth in SEQ ID NO:260 or is a functional variant thereof, such as, for example, the same length as SEQ ID NO:260, or 95%, 90%, 85%, 80%,75%, or 50% as long as SEQ ID NO: 260 or is a sequence that is 100 nucleotides or less, 95 nucleotides or less, 90 nucleotides or less, 85 nucleotides or less, 80 nucleotides or less, 75 nucleotides or less, 70 nucleotides or less, 65 nucleotides or less, 60 nucleotides or less, 55 nucleotides or less, 50 nucleotides or less, 45 nucleotides or less, 40 nucleotides or less, 35 nucleotides or less, 30 nucleotides or less, or 25 nucleotides or less; and is at least 50%, 55%, 60%, 65%, 70%, 75%, 80%, 85%, 90%, or 95% identical to SEQ ID NO:260. In some embodiments, the 3' arm comprises nucleotides 221-283 of the *Mus musculus* BIC.

In any of the aspects provided herein that include a polynucleotide comprising one or more nucleic acid sequences operatively linked to a promoter active in T cells and/or NK cells, wherein a first nucleic acid sequence of the one or more nucleic acid sequences encodes two or more inhibitory RNA molecules directed against one or more RNA targets, the two or more inhibitory RNA molecules, in some embodiments, can be positioned in the first nucleic acid sequence in series. In some embodiments, the inhibitory RNA molecules can be adjoined to one another either directly or indirectly by non-functional linker sequence(s). In some embodiments, the linker sequences can be between 5 and 120 nucleotides in length, or between 10 and 40 nucleotides in length.

In any of the aspects provided herein that include a polynucleotide comprising one or more nucleic acid sequences operatively linked to a promoter active in T cells and/or NK cells, wherein a first nucleic acid sequence of the one or more nucleic acid sequences encodes two or more inhibitory RNA molecules directed against one or more RNA targets, in some embodiments, the first nucleic acid sequence encodes two to four inhibitory RNA molecules. In illustrative embodiments, between 2 and 10, 2 and 8, 2 and 6, 2 and 5, 2 and 4, 3 and 5, or 3 and 6 inhibitory RNA molecules are included in the first nucleic acid sequence. In an illustrative embodiment, four inhibitory RNA molecules are included in the first nucleic acid sequence.

In any of the aspects provided herein that include a polynucleotide comprising one or more nucleic acid sequences operatively linked to a promoter active in T cells and/or NK cells, wherein a first nucleic acid sequence of the one or more nucleic acid sequences encodes one or more (e.g. two or more) inhibitory RNA molecules directed against one or more RNA targets, the one or more (e.g. two or more) inhibitory RNA molecules can be in an intron. In some embodiments, the intron is in a promoter. In illustrative embodiments, the intron is EF-1alpha intron A. In some embodiments, the intron is adjacent to and downstream of a promoter, which in illustrative embodiments, is inactive in a packaging cell used to produce the replication incompetent recombinant retroviral particle.

In any of the aspects provided herein that include a polynucleotide comprising one or more nucleic acid sequences operatively linked to a promoter active in T cells and/or NK cells, wherein a first nucleic acid sequence of the one or more nucleic acid sequences encodes two or more inhibitory RNA molecules directed against one or more RNA targets, the two or more inhibitory RNA molecules, in some embodiments, can be directed against different targets. In an alternate embodiment, the two or more inhibitory RNA molecules are directed against the same target. In some embodiments, the RNA targets are mRNAs transcribed from genes that are expressed by T cells such as but not limited to PD-1 (prevent inactivation); CTLA4 (prevent inactivation); TCRa (safety - prevent autoimmunity); TCRb (safety - prevent autoimmunity); CD3Z (safety - prevent autoimmunity); SOCS1 (prevent inactivation); SMAD2 (prevent inactivation); a miR-155 target (promote activation); IFN gamma (reduce CRS); cCBL (prolong signaling); TRAIL2 (prevent death); PP2A (prolong signaling); ABCG1 (increase cholesterol microdomain content by limiting clearance of cholesterol). In some embodiments, the RNA targets are mRNAs transcribed from genes that encode components of the T cell receptor (TCR) complex. In some embodiments, at least one of the two or more of inhibitory RNA molecules can decrease expression of T cell receptors, in illustrative embodiments, one or more endogenous T cell receptor(s) of a T cell. In certain embodiments, the RNA target can be mRNA transcribed from the endogenous TCRα or TCRβ gene of the T cell whose genome comprises the first nucleic acid sequence encoding the one or more miRNAs. In illustrative embodiments, the RNA target is mRNA transcribed from the TCRα gene.

In any of the aspects provided herein that include a polynucleotide comprising one or more nucleic acid sequences operatively linked to a promoter active in T cells and/or NK cells, wherein a first nucleic acid sequence of the one or more nucleic acid sequences encodes one or more (e.g. two or more) inhibitory RNA molecules directed against one or more RNA targets, and a second nucleic acid sequence of the one or more nucleic acid sequences encodes a chimeric antigen receptor (CAR) comprising an antigen-specific targeting region (ASTR), a transmembrane domain, and an intracellular activating domain, in some embodiments, the CAR is a microenvironment restricted biologic (MRB)-CAR. In other embodiments, the ASTR of the CAR binds to a tumor associated antigen. In other embodiments, the ASTR of the CAR is a microenvironment-restricted biologic (MRB)-ASTR.

In any of the aspects provided herein that include a polynucleotide comprising one or more nucleic acid sequences operatively linked to a promoter active in T cells and/or NK cells, wherein a first nucleic acid sequence of the one or more nucleic acid sequences encodes one or more (e.g. two or more) inhibitory RNA molecules directed against one or more RNA targets, and a second nucleic acid sequence of the one or more nucleic acid sequences encodes a chimeric antigen receptor (CAR) comprising an antigen-specific targeting region (ASTR), a transmembrane domain, and an intracellular activating domain, and in some instances a third nucleic acid sequence of the one or more nucleic acid sequences that encodes at least one lymphoproliferative element that is not an inhibitory RNA molecule, in some embodiments, any or all of the first nucleic acid sequence, second nucleic acid sequence, and third nucleic acid sequence is operably linked to a riboswitch. In some embodiments, the riboswitch is capable of binding a nucleoside analog. In some embodiments, the nucleoside analog is an antiviral drug.

In any of the aspects provided herein that include a replication incompetent recombinant retroviral particle, in some embodiments, the replication incompetent recombinant retroviral particle comprises a pseudotyping element on its surface that is capable of binding to a T cell and/or NK cell and facilitating membrane fusion of the replication incompetent recombinant retroviral particle thereto. In some embodiments, the pseudotyping element can be a Measles Virus F polypeptide, a Measles Virus H polypeptide, a VSV-G polypeptide, or a fragment of any thereof that retains the ability to bind to resting T cells and/or resting NK cells. In illustrative embodiments, the pseudotyping element is VSV-G.

In any of the aspects provided herein that include a replication incompetent recombinant retroviral particle, in some embodiments, the replication incompetent recombinant retroviral particle comprises an activation element on its surface that comprises a membrane-bound polypeptide capable of binding to CD3; and/or a membrane-bound polypeptide capable of binding to CD28. In some embodiments, the membrane-bound polypeptide capable of binding to CD3 is fused to a heterologous GPI anchor attachment sequence and/or the membrane-bound polypeptide capable of binding to CD28 is fused to a heterologous GPI anchor attachment sequence. In some embodiments, the membrane-bound polypeptide capable of binding CD3 is an anti-CD3 scFV or anti-CD3 scFvFc. In illustrative embodiments, the membrane-bound polypeptide capable of binding to CD3 is anti-CD3 scFvFc. In illustrative embodiments, the membrane-bound polypeptide capable of binding to CD28 is CD80, or an extra-cellular domain thereof, bound to a CD16B GPI anchor attachment sequence.

In any of the aspects provided herein that include a replication incompetent recombinant retroviral particle, in some embodiments, the replication incompetent recombinant retroviral particle comprises on its surface a nucleic acid encoding a domain recognized by a monoclonal antibody approved biologic.

Chimeric polypeptides in the aspects provided hereinbelow that include a chimeric polypeptide that promote cell proliferation of PBMCs, for example B cells, T cells, and/or NK cells, can be referred to herein as chimeric lymphoproliferative elements (CLEs). Embodiments provided hereinbelow that include nucleic acid sequences that encode a CLE and a CAR are referred to herein as CLE CAR polynucleotide embodiments.

In embodiments and subembodiments of any of the aspects and embodiments provided herein that include a lymphoproliferative element, including those in the paragraphs above in this section, the lymphoproliferative element can be any of the chimeric lymphoproliferative elements provided herein, including hereinbelow in this section. In other in embodiments and subembodiments of any of the aspects and embodiments herein that include a CAR, the lymphoproliferative element in certain illustrative embodiments is any of the chimeric lymphoproliferative elements encoded by nucleic acids in CLE CAR polynucleotide (and associated polypeptide) embodiments provided herein, for example as set out hereinbelow in this section.

In one aspect, provided herein is an isolated polynucleotide comprising one or more nucleic acid sequences, wherein:
a first nucleic acid sequence of the one or more nucleic acid sequences encodes a chimeric polypeptide comprising in amino to carboxy orientation,
a) an extracellular and transmembrane domain from a cytokine receptor or a hormone receptor, wherein at least one of the extracellular domain and the transmembrane domain comprise
   a. a mutation that is found on a constitutively active mutant of the cytokine receptor and wherein the extracellular sequence does not bind a ligand of the cytokine receptor, or
   b. an extracellular domain and a transmembrane domain from LMP1; and
b) a first intracellular domain selected from an intracellular domain of a gene having a first intracellular domain of a selected polypeptide identified in Tables 7-10, wherein said chimeric polypeptide promotes cell proliferation of B cells, T cells, and/or NK cells.

In one embodiment of the aspect in the paragraph immediately above, the first nucleic acid sequence further encodes a second intracellular domain, wherein the second intracellular domain can be upstream (i.e. 5') from the first intracellular domain, or in illustrative embodiments the second intracellular domain is downstream from the first intracellular domain. In a subembodiment of this embodiment, the first intracellular domain and the second intracellular domain combination comprise the first intracellular domain and the second intracellular domain combination of the selected polypeptide. Accordingly, for the sake of clarification, in a non-limiting exemplary embodiment, the selected polypeptide is M008-S212-S075. In such embodiment, the first intracellular domain of the chimeric polypeptide comprises or is TNFRSF8 transcript variant 1 (NM_001243_4) (S212) and the second polypeptide comprises or is FCGR2C (NM_201563_5) (S075).

In one embodiment of this aspect, the chimeric polypeptide comprises the transmembrane domain, the first intracellular domain, and the second intracellular domain of the selected polypeptide. In one embodiment of this aspect, the chimeric polypeptide comprises the extracellular and transmembrane domain, the first intracellular domain, and the second intracellular domain of the selected polypeptide. Accordingly, for the sake of clarification, in a non-limiting exemplary embodiment, the selected polypeptide is M008-S212-S075. In such embodiment, the extracllular and transmembrane domain of the chimeric polypeptide comprises or is Myc LMP1 (NC_007605_1) (M008), the first intracellular domain of the chimeric polypeptide comprises or is TNFRSF8 transcript variant 1 (NM_001243_4) (S212), and the second intracellular domain of the chimeric polypeptide comprises or is FCGR2C (NM_201563_5) (S075). In a related subembodiment, the chimeric polypeptide comprises the extracellular and transmembrane domain, the first intracellular domain, and the second intracellular domain of the selected polypeptide except that the extracellular and transmembrane domain optionally comprises a recognition or elimination domain, or does not comprise a recognition or elimination domain. In a related subembodiment, the chimeric polypeptide comprises the extracellular and transmembrane domain, the first intracellular domain, and the second intracellular domain of the selected polypeptide except that the extracellular and transmembrane domain comprises a recognition or elimination domain of the selected polypeptide, or another clearance tag. In another embodiment of the aspect provided in the paragraph above, the first intracellular domain and the second intracellular domain combination comprises a first intracellular domain of the selected polypeptide with any intracellular domain of any gene identified in the second intracellular domains of any candidate polypeptide in Tables 7-10.

In another embodiment of this aspect, the polynucleotide further comprises a second nucleic acid sequence that encodes a chimeric antigen receptor (CAR) comprising an antigen-specific targeting region (ASTR), a transmembrane domain, and an intracellular activating domain. In a subembodiment of this aspect, the first intracellular domain and the second intracellular domain combination are the first intracellular domain and the second intracellular domain combination of the selected polypeptide, and the selected polypeptide is identified in Tables 7-10. In another subembodiment of this embodiment, the chimeric polypeptide comprises the extracellular and transmembrane domain, the first intracellular domain, and the second intracellular domain of the selected polypeptide, and the selected polypeptide is identified in Tables 7-10. In a related subembodiment, the chimeric polypeptide comprises the extracellular and transmembrane domain, the first intracellular domain, and the second intracellular domain of the selected polypeptide except that the extracellular and transmembrane domain optionally comprises a recognition or elimination domain, and wherein the recognition or elimination domain is a recognition or elimination domain of the extracellular domain of the selected polypeptide, or another recognition or elimination domain, and wherein the selected polypeptide is identified in Tables 7-10.

In another aspect, provided herein is an isolated polynucleotide comprising one or more nucleic acid sequences, wherein:
a first nucleic acid sequence of the one or more nucleic acid sequences encodes a chimeric polypeptide comprising in amino to carboxy orientation,
a) an extracellular and transmembrane domain from a cytokine receptor or a hormone, wherein at least one of the extracellular domain and the transmembrane domain comprise
   c. a mutation that is found on a constitutively active mutant of the cytokine receptor and wherein the extracellular sequence does not bind a ligand of the cytokine receptor, or
   d. an extracellular domain and a transmembrane domain from LMP1; and
b) a first intracellular domain selected from an intracellular domain of CD3D, CD3E, CD8A, CD27, CD40, CD79B, IFNAR1, IL2RA, IL3RA, IL13RA2, TNFRSF8, TNFRSF9, IL31RA, or MyD88, wherein said chimeric polypeptide promotes cell proliferation of PBMCs, and in illustrative embodiments, of B cells, T cells, and/or NK cells.

In one embodiment of the aspect in the paragraph immediately above, the first nucleic acid sequence further encodes a second intracellular domain, wherein the second intracellular domain can be upstream (i.e. 5') from the first intracellular domain, or in illustrative embodiments the second intracellular domain is downstream from the first intracellular domain. In a subembodiment of this embodiment, the second intracellular domain is from a gene of a second intracellular domain in a selected polypeptide identified in any table of Example 17 as yielding an enrichment at day 35 versus day 7 above 0, 1 , or 2, wherein the first intracellular domain is from the gene of the first intracellular domain of the selected polypeptide. In a further subembodiment of this subembodiment or embodiment, the first intracellular domain and the second intracellular domain combination are the first intracellular domain and the second intracellular domain from a selected chimeric polypeptide identified in any table of Example 17 as yielding an enrichment at day 35 versus day 7 above 0, 1 , or 2. In another subembodiment of the embodiment or subembodiments in this paragraph, the first intracellular domain is from a first gene of a selected first intracellular domain of a selected polypeptide in Tables 7-10 and the second intracellular domain is from a second gene of a selected second intracellular domain of the selected polypeptide. In another subembodiment of the embodiment or subembodiments in this paragraph, the first intracellular domain is from a selected polypeptide in Tables 7-10 and the second intracellular domain is from the selected polypeptide in Tables 7-10.

In another subembodiment of the immediately above embodiments wherein the first nucleic acid sequence further encodes a second intracellular domain, the second intracellular domain is selected from a second intracellular domain identified in any of the tables of Example 17 as yielding an enrichment at day 35 versus day 7 above 0, 1, or 2, for the corresponding first intracellular domain. In subembodiments of these embodiments, the first intracellular domain is selected from an intracellular domain of CD27, CD40, or CD79B. In other subembodiments of this embodiment, the first intracellular domain is selected from an intracellular domain of CD3D, CD3E, CD8A, CD27, CD40, CD79B, IFNAR1, IL2RA, IL3RA, IL13RA2, TNFRSF8, TNFRSF9, IL31RA, or MyD88 identified in any of the tables of Example 17 as yielding an enrichment at day 35 versus day 7 above 0, 1, or 2. In another subembodiment of the embodiment or subembodiments in this paragraph, the first intracellular domain is from a first gene of a selected chimeric polypeptide in Tables 7-10 and the second intracellular domain is from a second gene of the selected chimeric polypeptide in Tables 7-10. In another subembodiment of the embodiment or subembodiments in this paragraph, the first intracellular domain is from a first gene of a selected chimeric polypeptide in Tables 7-10and the second intracellular domain is from an intracellular domain of CD3D, CD3G, CD8A, CD8B, CD27, CD40, CD79B, CRLF2, FCGR2C, ICOS, IL2RA, IL13RA1, IL13RA2, IL15RA, TNFRSF9, and TNFRSF18. In another subembodiment of the embodiment or subembodiments in this paragraph, the first intracellular domain is from a selected polypeptide identified in Tables 7-10 and the second intracellular domain is from the selected polypeptide identified in Tables 7-10. In a related subembodiment, the chimeric polypeptide comprises the extracellular and transmembrane domain, the first intracellular domain, and the second intracellular domain of the selected polypeptide except that the extracellular and transmembrane domain optionally comprises a clearance tag, or does not comprise a clearance tag, and wherein the selected polypeptide is identified in Tables 7-10. In a related subembodiment, the chimeric polypeptide comprises the extracellular and transmembrane domain, the first intracellular domain, and the second intracellular domain of the selected polypeptide except that the extracellular and transmembrane domain comprises a recognition or elimination domain of the selected polypeptide, or another recognition or elimination domain, and wherein the selected polypeptide is identified in Tables 7-10.

In another embodiment of this aspect, the first intracellular domain is selected from an intracellular domain of CD3D, CD3E, CD8A, CD27, CD40, CD79B, IFNAR1, IL2RA, IL3RA, IL13RA2, TNFRSF8, or TNFRSF9, the polynucleotide further comprises a second nucleic acid sequence that encodes a chimeric antigen receptor (CAR) comprising an antigen-specific targeting region (ASTR), a transmembrane domain, and an intracellular activating domain. In a subembodiment of this aspect, the first intracellular domain is from a gene of the first intracellular domain of the selected polypeptide and the second intracellular domain is from a gene of the second intracellular domain of the selected polypeptide, and the selected polypeptide is identified in Tables 7-10. In a subembodiment of this aspect, the first intracellular domain and the second intracellular domain combination are the first intracellular domain and the second intracellular domain combination of the selected polypeptide, and the selected polypeptide is identified in Tables 7-10. In another subembodiment of this embodiment, the chimeric polypeptide comprises the extracellular and transmembrane domain, the first intracellular domain, and the second intracellular domain of the selected polypeptide, and the selected polypeptide is identified in Tables 7-10. In a related subembodiment, the chimeric polypeptide comprises the extracellular and transmembrane domain, the first intracellular domain, and the second intracellular domain of the selected polypeptide except that the extracellular and transmembrane domain optionally comprises a recognition or elimination domain, and wherein the recognition or elimination domain is a recognition or elimination domain of the extracellular domain of the selected polypeptide, or another recognition or elimination domain, and wherein the selected polypeptide is identified in Tables 7-10.

In another aspect, provided herein is an isolated polynucleotide comprising one or more nucleic acid sequences, wherein:
a first nucleic acid sequence of the one or more nucleic acid sequences encodes a chimeric polypeptide comprising in amino to carboxy orientation,
(a) an extracellular and transmembrane domain from a cytokine receptor or a hormone, wherein at least one of the extracellular domain and the transmembrane domain comprise
   i. a mutation that is found on a constitutively active mutant of the cytokine receptor and wherein the extracellular sequence does not bind a ligand of the cytokine receptor, or
   ii. an extracellular domain and a transmembrane domain from LMP1; and
(b) a first intracellular domain selected from an intracellular domain of CD3D, CD3G, CD8A, CD8B, CD27, CD40, CD79B, CRLF2, FCGR2C, ICOS, IL2RA, IL13RA1, IL13RA2, IL15RA, TNFRSF9, and TNFRSF18, wherein said chimeric polypeptide promotes cell proliferation of PBMCs, and in illustrative embodiments, of B cells, T cells, and/or NK cells.

In Example 17, the intracellular domains recited in the immediately above aspect were identified as noteworthy second intracellular domains. However, it will be understood that in some embodiments, intracellular domains identified as noteworthy second intracellular domains are first intracellular domains of the chimeric polypeptides of these embodiments.

In another aspect, provided herein is an isolated polynucleotide comprising one or more nucleic acid sequences, wherein:
a first nucleic acid sequence of the one or more nucleic acid sequences encodes a chimeric polypeptide comprising in amino to carboxy orientation,
   a) an extracellular and transmembrane domain from a cytokine receptor or a hormone, wherein at least one of the extracellular domain and the transmembrane domain comprise
      a. a mutation that is found on a constitutively active mutant of the cytokine receptor and wherein the extracellular sequence does not bind a ligand of the cytokine receptor, or
      b. an extracellular domain and a transmembrane domain from LMP1; and
   b) a first intracellular domain selected from an intracellular domain of CD3D, CD3E, CD8A, CD27, CD40, CD79B, IFNAR1, IL2RA, IL3RA, IL13RA2, TNFRSF8, TNFRSF9, IL31RA, or MyD88,
wherein said chimeric polypeptide promotes cell proliferation of PBMCs, and in illustrative embodiments, of B cells, T cells, and/or NK cells, and wherein the extracellular and transmembrane domain is selected from the extracellular domain and transmembrane domain of IL7RA Ins PPCL, LMP1, CRLF2 F232C, CSF2RB V449E, CSF3R T640N, EPOR L251C I252C, GHR E260C I270C, IL27RA F523C, and MPL S505N.

In certain embodiments of any of the above aspects directed to a polynucleotide encoding a chimeric polypeptide wherein said chimeric polypeptide promotes cell proliferation of PBMCs, and in illustrative embodiments, of B cells, T cells, and/or NK cells, the extracellular domain and transmembrane domain are selected from CSF3R T640N and MPL S505N. In illustrative embodiments, the extracellular domain and transmembrane domain are from CSF3R T640N and in certain subembodiments, the isolated polynucleotide does not comprise a CAR. In certain subembodiments, the extracellular domain and transmembrane domain are CSF3R T640N provided in Table 6 except that the recognition and elimination domain is optional. In certain embodiments, the first intracellular domain and the second intracellular domain are from any of the polypeptides provided in Example 17, and the tables therein, that include a recited intracellular and extracellular domain and a recited first intracellular domain. In other subembodiments, the extracellular and transmembrane domain are from MPL S505N, and in certain embodiments, the extracellular domain and transmembrane domain of MPL S505N provided in Table 6 except that the recognition and elimination domain is optional.

In another aspect, provided herein is an isolated polynucleotide comprising one or more nucleic acid sequences, wherein:
a first nucleic acid sequence of the one or more nucleic acid sequences encodes a chimeric polypeptide comprising in amino to carboxy orientation,
a) a transmembrane domain from a type I transmembrane protein; and
b) a first intracellular domain selected from an intracellular domain of a gene having a first intracellular domain of a selected polypeptide identified in Tables 11-16 wherein said chimeric polypeptide promotes cell proliferation of B cells, T cells, and/or NK cells.
wherein said chimeric polypeptide promotes cell proliferation of PBMCs, and in illustrative embodiments, of B cells, T cells, and/or NK cells. As shown in the attached Example 18, such chimeric polypeptides are capable of promoting cell proliferation of PBMCs in the absence of exposure of the PBMCs to IL-2 during culturing, for example in the absence of adding IL-2 to culture media of PBMCs (e.g. B cells, T cells, or NK cells) that express a nucleic acid encoding the chimeric polypeptide.

In one embodiment of the aspect in the paragraph immediately above and any embodiment of this aspect herein below in this section, the first intracellular domain is an intracellular domain of a gene having a first intracellular domain in Tables 11-16, and in other illustrative embodiments one of the first 50, 25, or 10 constructs listed in Tables 11-16, and in further illustrative embodiments, one of the first 50, 25, or 10 constructs listed in two, three, four, or five of six tables of, or all of Tables 11-16. In one embodiment, the first intracellular domain is an intracellular domain identified in Table 17. Table 17 identifies constructs that promoted proliferation of PBMCs between day 7 and day 21 or day 35 where a second intracellular domain was not present on the construct. In some subembodiments of this embodiment, the chimeric polypeptide comprises a combination of transmembrane domain and intracellular domain, with or without an extracellular domain comprising a dimerizing motif, of Table 17.

In one embodiment of the aspect in the paragraph immediately above and any embodiment of this aspect herein below in this section, the first nucleic acid sequence further encodes an extracellular domain, and in illustrative embodiments, the extracellular domain comprises a dimerizing motif. In any aspects or embodiments wherein the extracellular domain of a CLE comprises a dimerizing motif, the dimerizing motif can be selected from the group consisting of: a leucine zipper motif-containing polypeptide, CD69, CD71, CD72, CD96, Cd105, Cd161, Cd162, Cd249, CD271, and Cd324, as well as mutants and/or active fragments thereof that retain the ability to dimerize. In any of the aspects and embodiments herein wherein the extracellular domain of a CLE comprises a dimerizing motif, the dimerizing motif can require a dimerizing agent, and the dimerizing motif and associated dimerizing agent can be selected from the group consisting of: FKBP and rapamycin or analogs thereof, GyrB and coumermycin or analogs thereof, DHFR and methotrexate or analogs thereof, or DmrB and AP20187 or analogs thereof, as well as mutants and/or active fragments of the recited dimerizing proteins that retain the ability to dimerize.

In illustrative embodiments of any aspects or embodiments herein wherein the extracellular domain of a CLE comprises a dimerizing motif, the extracellular domain can comprises a leucine zipper motif. In some embodiments, the leucine zipper motif is from a jun polypeptide, for example c-jun. In certain embodiments the c-jun polypeptide is the c-jun polypeptide region of ECD-11.

In one embodiment of this aspect, the first nucleic acid sequence further encodes a second intracellular domain, wherein the second intracellular domain can be upstream (i.e. 5') from the first intracellular domain, or in illustrative embodiments the second intracellular domain is downstream from the first intracellular domain. In a subembodiment of this embodiment, the first intracellular domain and the second intracellular domain combination comprises a first intracellular domain of the selected polypeptide with any intracellular domain of any gene identified in the second intracellular domains of any candidate polypeptide in Tables 11-16, and in illustrative embodiments in Tables 11-16, and in other illustrative embodiments one of the first 50, 25, or 10 constructs listed in Tables 11-16, and in further illustrative embodiments, one of the first 50, 25, or 10 constructs listed in two, three, four, or five of tables of, or all of Tables 11-16. In a subembodiment of this embodiment, the first intracellular domain and the second intracellular domain combination comprise the first intracellular domain and the second intracellular domain combination of the selected polypeptide.

In one embodiment of this aspect, the chimeric polypeptide comprises the transmembrane domain, the first intracellular domain, and the second intracellular domain of the selected polypeptide. In one embodiment of this aspect, the chimeric polypeptide comprises the extracellular and transmembrane domain, the first intracellular domain, and the second intracellular domain of the selected polypeptide, wherein the extracellular domain optionally comprises a recognition or elimination domain.

In a related subembodiment, the chimeric polypeptide comprises the extracellular and transmembrane domain, the first intracellular domain, and the second intracellular domain of the selected polypeptide except that the extracellular and transmembrane domain optionally comprises a recognition or elimination domain, or does not comprise a recognition or elimination domain. In a related subembodiment, the chimeric polypeptide comprises the extracellular and transmembrane domain, the first intracellular domain, and the second intracellular domain of the selected polypeptide except that the extracellular and transmembrane domain comprises a recognition or elimination domain of the selected polypeptide, or another recognition or elimination domain.

In another embodiment of this aspect, the polynucleotide further comprises a second nucleic acid sequence that encodes a chimeric antigen receptor (CAR) comprising an antigen-specific targeting region (ASTR), a transmembrane domain, and an intracellular activating domain. In a subembodiment of this aspect, the first intracellular domain and the second intracellular domain combination are the first intracellular domain and the second intracellular domain combination of the selected polypeptide, and the selected polypeptide is identified in Tables 11-16, and in other illustrative embodiments one of the first 50, 25, or 10 constructs listed in Tables 11-16, and in further illustrative embodiments, one of the first 50, 25, or 10 constructs listed in two, three, four, or five of tables of, or all of Tables 11-16. In another subembodiment of this embodiment, the chimeric polypeptide comprises the extracellular and transmembrane domain, the first intracellular domain, and the second intracellular domain of the selected polypeptide, and the selected polypeptide is identified in Tables 11-16, or in illustrative embodiments in one of the first 50, 25, or 10 constructs listed in Tables 11-16, an in further illustrative embodiments, one of the first 50, 25, or 10 constructs listed in two, three, four, or five of tables of, or all of Tables 11-16. In a related subembodiment, the chimeric polypeptide comprises the extracellular and transmembrane domain, the first intracellular domain, and the second intracellular domain of the selected polypeptide except that the extracellular and transmembrane domain optionally comprises a recognition and elimination domain, and wherein the recognition or elimination domain is a recognition or elimination domain of the extracellular domain of the selected polypeptide, or another reognition or elimination domain, and wherein the selected polypeptide is identified in Tables 11-16.

In another aspect, provided herein is an isolated polynucleotide comprising one or more nucleic acid sequences, wherein:
a first nucleic acid sequence of the one or more nucleic acid sequences encodes a chimeric polypeptide comprising in amino to carboxy orientation,
a) a transmembrane domain from a type I transmembrane protein; and
b) a first intracellular domain selected from an intracellular domain of LEPR, MYD88, IFNAR2, MPL, IL18R1, IL13RA2, IL10RB, IL23R, or CSF2RA,
wherein said chimeric polypeptide promotes cell proliferation of PBMCs, and in illustrative embodiments, of B cells, T cells, and/or NK cells.

In one embodiment of the aspect in the paragraph immediately above and any embodiment of this aspect herein below in this section, the first nucleic acid sequence further encodes an extracellular domain, and in illustrative embodiments, the extracellular domain comprises a dimerizing motif. In illustrative embodiments of this aspect, the extracellular domain comprises a leucine zipper. In some embodiments, the leucine zipper is from a jun polypeptide, for example c-jun. In certain embodiments the c-jun polypeptide is the c-jun polypeptide region of ECD-11.

In another embodiment of this aspect where the first intracellular domain is selected from an intracellular domain of LEPR, MYD88, IFNAR2, MPL, IL18R1, IL13RA2, IL10RB, IL23R, or CSF2RA, the first nucleic acid sequence further encodes a second intracellular domain, wherein the second intracellular domain can be upstream (i.e. 5') from the first intracellular domain, or in illustrative embodiments the second intracellular domain is downstream from the first intracellular domain. In a subembodiment of this embodiment, the second intracellular domain is from a gene of a second intracellular domain in a selected polypeptide identified in any table of Example 18 as yielding an enrichment at day 21 versus day 7 above 0, 1 , or 2, wherein the first intracellular domain is from the gene of the first intracellular domain of the selected polypeptide. In a further subembodiment of this subembodiment or embodiment, the first intracellular domain and the second intracellular domain combination are the first intracellular domain and the second intracellular domain from a selected chimeric polypeptide identified in any table of Example 18 as yielding an enrichment at day 21 versus day 7 above 0, 1 , or 2. In another subembodiment of the embodiment or subembodiments in this paragraph, the first intracellular domain is from a first gene of a selected first intracellular domain of a selected polypeptide in Tables 11-16 and the second intracellular domain is from a second gene of a selected second intracellular domain of the selected polypeptide. In another subembodiment of the embodiment or subembodiments in this paragraph, the first intracellular domain is from a selected polypeptide in Tables 11-16 and the second intracellular domain is from the selected polypeptide. In another embodiment of this aspect or any embodiment herein, the polynucleotide further comprises a second nucleic acid sequence that encodes a chimeric antigen receptor (CAR) comprising an antigen-specific targeting region (ASTR), a transmembrane domain, and an intracellular activating domain.

In one embodiment of the aspect and one subembodiment or further subembodiment of the embodiments and subembodiments in the immedatiately preceding paragraph that comprise a second intracellular domain, the first intracellular domain is an intracellular domain of LEPR, MYD88, IFNAR2, and MPL. In another embodiment, the first intracellular domain is an intracellular domain from LEPR, IFNAR2, and MPL. In another embodiment, the first intracellular domain is other than MyD88. In another embodiment, the first intracellular domain is other than MyD88 and the second intracellular domain is other than CD40. In a subembodiment of this the embodiments in this paragraph, the polynucleotide further comprises a second nucleic acid sequence that encodes a chimeric antigen receptor (CAR) comprising an antigen-specific targeting region (ASTR), a transmembrane domain, and an intracellular activating domain.

In one embodiment of this aspect as described herein, MPL is the first intracellular domain and the second intracellular domain is from a gene of a second intracellular domain in a selected polypeptide identified in Tables 11-16. In a further subembodiment of this embodiment, the first intracellular domain and the second intracellular domain combination comprise a first intracellular domain and a second intracellular domain from a selected chimeric polypeptide identified in Tables 11-16. In some subembodiments of the these embodiments, the orientation of the first intracellular domain and the second intracellular domain is reversed such that the carboxy terminal residue of the second domain is bound to the first domain or to a spacer between the second domain and the first domain.

In one embodiment of this aspect as described herein, LEPR is the first intracellular domain and the second intracellular domain is from a gene of a second intracellular domain in a selected polypeptide identified in Tables 11-16. In a further subembodiment of this embodiment, the first intracellular domain and the second intracellular domain combination comprise a first intracellular domain and a second intracellular domain from a selected chimeric polypeptide identified in Tables 11-16. In some subembodiments of the these embodiments, the orientation of the first intracellular domain and the second intracellular domain is reversed such that the carboxy terminal residue of the second domain is bound to the first domain or to a spacer between the second domain and the first domain.

In one embodiment of this aspect as described herein, IFNAR2 is the first intracellular domain and the second intracellular domain is from a gene of a second intracellular domain in a selected polypeptide identified in Tables 11-16. In a further subembodiment of this embodiment, the first intracellular domain and the second intracellular domain combination comprise a first intracellular domain and a second intracellular domain from a selected chimeric polypeptide identified in Tables 11-16. In some subembodiments of the these embodiments, the orientation of the first intracellular domain and the second intracellular domain is reversed such that the carboxy terminal residue of the second domain is bound to the first domain or to a spacer between the second domain and the first domain.

In one embodiment of this aspect as described herein, MyD88 is the first intracellular domain and the second intracellular domain is from a gene of a second intracellular domain in a selected polypeptide identified in Tables 11-16. In a further subembodiment of this embodiment, the first intracellular domain and the second intracellular domain combination comprise a first intracellular domain and a second intracellular domain from a selected chimeric polypeptide identified in Tables 11-16. In some subembodiments of the these embodiments, the orientation of the first intracellular domain and the second intracellular domain is reversed such that the carboxy terminal residue of the second domain is bound to the first domain or to a spacer between the second domain and the first domain. In some embodiments of isolated polynucleotide aspects above wherein the first intracellular domain is selected from an intracellular domain of LEPR, MYD88, IFNAR2, IL17RD, IL17RE, IL1RAP, IL23R, and MPL, the transmembrane domain is selected from a transmembrane domain of CD40, CD8B, CRLF2, CSF2RA, GCGR2C, ICOS, IFNAR1, IFNGR1, IL10RB, IL18R1, IL18RAP, IL3RA, and PRLR. In some illustrative embodiments, the transmembrane domain is a transmembrane domain of CD40 or ICOS. In some subembodiments, the first intracellular domain is from LEPR, MYD88, IFNAR2, and MPL. For example, in some embodiments the transmembrane domain is a transmembrane domain from CD40 and the first intracellular domain is an intracellular domain from MPL.

In some embodiments of isolated polynucleotide aspects above wherein the first intracellular domain is selected from an intracellular domain of LEPR, MYD88, IFNAR2, IL17RD, IL17RE, IL1RAP, IL23R, and MPL, the second intracellular domain is from CD40, CD79B, or CD27. In some subembodiments, the first intracellular domain is from LEPR, MYD88, IFNAR2, and MPL. For example, in some embodiments, the first intracellular domain is MPL and the second intracellular domain is CD40. In all embodiments in this paragraph, the order of the first intracellular domain and the second intracellular domain on the polypeptide can be reversed.

In another aspect, provided herein is an isolated polynucleotide comprising one or more nucleic acid sequences, wherein:
a first nucleic acid sequence of the one or more nucleic acid sequences encodes a chimeric polypeptide comprising in amino to carboxy orientation,
a) a transmembrane domain from a type I transmembrane protein; and
b) a first intracellular domain selected from an intracellular domain of a gene having a first intracellular domain of a selected polypeptide identified in Tables 11-16, wherein said chimeric polypeptide promotes cell proliferation of B cells, T cells, and/or NK cells.
wherein said chimeric polypeptide promotes cell proliferation of PBMCs, and in illustrative embodiments, of B cells, T cells, and/or NK cells. In some embodiments, the NK cells are NKT cells.

In one embodiment of the aspect in the paragraph immediately above and any embodiment of this aspect herein below in this section, the first nucleic acid sequence further encodes an extracellular domain, and in illustrative embodiments, the extracellular domain comprises a dimerizing motif. In any aspects or embodiments wherein the extracellular domain of a CLE comprises a dimerizing motif, the dimerizing motif can be selected from the group consisting of: a leucine zipper motif-containing polypeptide, CD69, CD71, CD72, CD96, Cd105, Cd161, Cd162, Cd249, CD271, and Cd324, as well as mutants and/or active fragments thereof that retain the ability to dimerize. In any of the aspects and embodiments herein wherein the extracellular domain of a CLE comprises a dimerizing motif, the dimerizing motif can require a dimerizing agent, and the dimerizing motif and associated dimerizing agent can be selected from the group consisting of: FKBP and rapamycin or analogs thereof, GyrB and coumermycin or analogs thereof, DHFR and methotrexate or analogs thereof, or DmrB and AP20187 or analogs thereof, as well as mutants and/or active fragments of the recited dimerizing proteins that retain the ability to dimerize.

In illustrative embodiments of any aspects or embodiments herein wherein the extracellular domain of a CLE comprises a dimerizing motif, the extracellular domain can comprises a leucine zipper motif. In some embodiments, the leucine zipper motif is from a jun polypeptide, for example c-jun. In certain embodiments the c-jun polypeptide is the c-jun polypeptide region of ECD-11.

In one embodiment of this aspect, the first nucleic acid sequence further encodes a second intracellular domain, wherein the second intracellular domain can be upstream (i.e. 5') from the first intracellular domain, or in illustrative embodiments the second intracellular domain is downstream from the first intracellular domain. In a subembodiment of this embodiment, the first intracellular domain and the second intracellular domain combination comprises a first intracellular domain of the selected polypeptide with any intracellular domain of any gene identified in the second intracellular domains of any candidate polypeptide in Tables 11-16. In a subembodiment of this embodiment, the first intracellular domain and the second intracellular domain combination comprise the first intracellular domain and the second intracellular domain combination of the selected polypeptide.

In one embodiment of this aspect, the chimeric polypeptide comprises the transmembrane domain, the first intracellular domain, and the second intracellular domain of the selected polypeptide. In one embodiment of this aspect, the chimeric polypeptide comprises the extracellular and transmembrane domain, the first intracellular domain, and the second intracellular domain of the selected polypeptide, wherein the extracellular domain optionally comprises a recognition or elimination domain.

In a related subembodiment, the chimeric polypeptide comprises the extracellular and transmembrane domain, the first intracellular domain, and the second intracellular domain of the selected polypeptide except that the extracellular and transmembrane domain optionally comprises a recognition or elimination domain, or does not comprise a recognition or elimination domain. In a related subembodiment, the chimeric polypeptide comprises the extracellular and transmembrane domain, the first intracellular domain, and the second intracellular domain of the selected polypeptide except that the extracellular and transmembrane domain comprises a recognition or elimination domain of the selected polypeptide, or another recognition or elimination domain.

In another embodiment of this aspect, the polynucleotide further comprises a second nucleic acid sequence that encodes a chimeric antigen receptor (CAR) comprising an antigen-specific targeting region (ASTR), a transmembrane domain, and an intracellular activating domain. In a subembodiment of this aspect, the first intracellular domain and the second intracellular domain combination are the first intracellular domain and the second intracellular domain combination of the selected polypeptide, and the selected polypeptide is identified in Tables 11-16. In another subembodiment of this embodiment, the chimeric polypeptide comprises the extracellular and transmembrane domain, the first intracellular domain, and the second intracellular domain of the selected polypeptide, and the selected polypeptide is identified in Tables 11-16. In a related subembodiment, the chimeric polypeptide comprises the extracellular and transmembrane domain, the first intracellular domain, and the second intracellular domain of the selected polypeptide except that the extracellular and transmembrane domain optionally comprises a recognition and elimination domain, and wherein the recognition or elimination domain is a recognition or elimination domain of the extracellular domain of the selected polypeptide, or another reognition or elimination domain, and wherein the selected polypeptide is identified in Tables 11-16.

In another aspect, provided herein is an isolated polynucleotide comprising one or more nucleic acid sequences, wherein:
a first nucleic acid sequence of the one or more nucleic acid sequences encodes a chimeric polypeptide comprising in amino to carboxy orientation,
c) a transmembrane domain from a type I transmembrane protein; and
d) a first intracellular domain selected from an intracellular domain of LEPR, MYD88, IFNAR2, IL17RD, IL17RE, IL1RAP, IL23R, and MPL,
wherein said chimeric polypeptide promotes cell proliferation of PBMCs, and in illustrative embodiments, of B cells, T cells, and/or NK cells.

In one embodiment of the aspect in the paragraph immediately above and any embodiment of this aspect herein below in this section, the first nucleic acid sequence further encodes an extracellular domain, and in illustrative embodiments, the extracellular domain comprises a dimerizing motif. In illustrative embodiments of this aspect, the extracellular domain comprises a leucine zipper. In some embodiments, the leucine zipper is from a jun polypeptide, for example c-jun. In certain embodiments the c-jun polypeptide is the c-jun polypeptide region of ECD-11.

In another embodiment of this aspect where the first intracellular domain is selected from an intracellular domain of LEPR, MYD88, IFNAR2, IL17RD, IL17RE, IL1RAP, IL23R, and MPL, the first nucleic acid sequence further encodes a second intracellular domain, wherein the second intracellular domain can be upstream (i.e. 5') from the first intracellular domain, or in illustrative embodiments the second intracellular domain is downstream from the first intracellular domain. In a subembodiment of this embodiment, the second intracellular domain is from a gene of a second intracellular domain in a selected polypeptide identified in any table of Example 18 as yielding an enrichment at day 21 versus day 7 above 0, 1 , or 2, wherein the first intracellular domain is from the gene of the first intracellular domain of the selected polypeptide. In a further subembodiment of this subembodiment or embodiment, the first intracellular domain and the second intracellular domain combination are the first intracellular domain and the second intracellular domain from a selected chimeric polypeptide identified in any table of Example 18 as yielding an enrichment at day 21 versus day 7 above 0, 1 , or 2. In another subembodiment of the embodiment or subembodiments in this paragraph, the first intracellular domain is from a first gene of a selected first intracellular domain of a selected polypeptide in Tables 11-16 and the second intracellular domain is from a second gene of a selected second intracellular domain of the selected polypeptide. In another subembodiment of the embodiment or subembodiments in this paragraph, the first intracellular domain is from a selected polypeptide in Tables 11-16 and the second intracellular domain is from the selected polypeptide. In another embodiment of this aspect or any embodiment herein, the polynucleotide further comprises a second nucleic acid sequence that encodes a chimeric antigen receptor (CAR) comprising an antigen-specific targeting region (ASTR), a transmembrane domain, and an intracellular activating domain.

In certain embodiments of any of the methods or isolated polynucleotide embodiments herein that include a chimeric polypeptide wherein said chimeric polypeptide promotes cell proliferation of PBMCs, and in illustrative embodiments, of B cells, T cells, and/or NK cells, the first and/or second intracellular domain, in illustrative embodiments the first intracellular domain, is an intracellular domain from LEPR, MYD88, IFNAR2, MPL, IL18R1, IL13RA2, IL10RB, IL23R, or CSF2RA. In some embodiments, the first intracellular domain is from MPL. In exemplary subembodiments, the chimeric polypeptide comprises an extracellular domain comprising a dimerizing motif provided herein.

In certain embodiments of any of the methods or isolated polynucleotide embodiments herein that include a chimeric polypeptide wherein said chimeric polypeptide promotes cell proliferation of PBMCs, and in illustrative embodiments, of B cells, T cells, and/or NK cells, the transmembrane domain is from CD40, ICOS, FCGR2C, PRLR, IL3RA, or IL6ST. In some illustrative embodiments, the transmembrane domain is from CD40 or ICOS. In exemplary subembodiments, the chimeric polypeptide comprises an extracellular domain comprising a dimerizing motif provided herein.

In certain embodiments of any of the methods or isolated polynucleotide embodiments herein that include a chimeric polypeptide wherein said chimeric polypeptide promotes cell proliferation of PBMCs, and in illustrative embodiments, of B cells, T cells, and/or NK cells, , the first and/or second intracellular domain, in illustrative embodiments the second intracellular domain, is an intracellular domain from CD40, CD79B, TNFRSF4, TNFRSF9, TNFRSF14, FCGRA2, CD3G, or CD27. In some illustrative embodiments, the second intracellular domain is from CD40, CD79B, or CD27. In exemplary subembodiments, the chimeric polypeptide comprises an extracellular domain comprising a dimerizing motif provided herein.

In certain embodiments of any of the methods or isolated polynucleotide embodiments herein that include a chimeric polypeptide wherein said chimeric polypeptide promotes cell proliferation of PBMCs, and in illustrative embodiments, of B cells, T cells, and/or NK cells, the first and/or second intracellular domain, is an intracellular domain (either a P3 or P4 element) of any of the chimeric polypeptides in Tables 11-16. In some embodiments the chimeric polypeptide comprises any P2, P3, and P4 combination of any of the chimeric polypeptides in Tables 11-16.

In an embodiment of any of the aspects and embodiments wherein the transmembrane domain is a type I transmembrane protein, the transmembrane domain can be a Type I cytokine receptor, a hormone receptor, a T cell receptor, or a TNF-family receptor. In an embodiment of any of the aspects and embodiments wherein the chimeric polypeptide comprises an extracellular domain and wherein the extracellular domain comprises a dimerizing motif, the transmembrane domain can a Type I cytokine receptor, a hormone receptor, a T cell receptor, or a TNF-family receptor. In illustrative subembodiments of any of these embodiments in this paragraph, the first intracellular domain is selected from an intracellular domain of LEPR, MYD88, IFNAR2, IL17RD, IL17RE, IL1RAP, IL23R, and MPL.

In an embodiment of any of the aspects and embodiments directed to polynucleotides comprising a nucleic acid sequence encoding a chimeric polypeptide that comprises an extracellular domain, the extracellular domain can comprise a 1, 2, 3, or 4 amino acid spacer within 20 amino acids of the carboxy terminus of the extracellular domain, and in illustrative embodiments, at the carboxy-terminus of the extracellular domain such that the spacer separates the extracellular domain and the transmembrane domain. In some embodiments, the 1, 2, 3, or 4 amino acid spacer comprises an alanine residue, and in some cases, only alanine residues. In illustrative subembodiments for embodiments that comprise said spacer, the extracellular domain comprises a dimerizing motif, for example a leucine zipper motif.

It will be understood that the first intracellular domain and the second intracellular domain for any aspect and embodiment provided herein that comprises a chimeric polypeptide that promotes cell proliferation of PBMCs, B cells, T cells, and/or NK cells, can include mutants and/or fragments of the recited genes provided that the chimeric polypeptide retains its ability to promote cell proliferation of PBMCs, B cells, T cells, and/or NK cells. These mutants and/or fragments typically retain signaling activity such that the chimeric polypeptide retains its survival and proliferation promoting activity. It will be understood that the first intracellular domain and the second intracellular domain can be reversed in order for any aspect and embodiment provided herein that comprises a chimeric polypeptide that promotes cell proliferation of PBMCs, B cells, T cells, and/or NK cells. Thus, it will be understood in these embodiment that the first intracellular domain is the second intracellular domain and vice versa.

In any of the aspects and embodiments provided herein that comprise polynucleotides that include a nucleic acid sequence encoding a chimeric polypeptide that promotes cell proliferation of PBMCs, B cells, T cells, and/or NK cells, the nucleic acid sequence can further encode a recognition or elimination domain. In some embodiments, the recognition or elimination domain is a recognition domain of a monoclonal antibody approved biologic. In some embodiments, the recognition or elimination domain comprises a polypeptide that is recognized by an antibody that recognizes EGFR, or an epitope thereof. In some embodiments, the recognition or elimination domain comprises a polypeptide that is recognized by an antibody that recognizes MYC, or an epitope thereof.

In any of the aspects and embodiments provided herein that comprises polynucleotides that include a nucleic acid sequence encoding a chimeric polypeptide that promotes cell proliferation of PBMCs, B cells, T cells, and/or NK cells, the first nucleic acid sequence, and/or the second nucleic acid sequence can be operatively linked to a first promoter active in B cells, T cells and/or NK cells. In some illustrative embodiments, the first promoter is active in T cell and/or NK cells.

In an embodiment of any of the isolated polynucleotide aspects and embodiments provided herein having a nucleic acid sequence that encode a chimeric polypeptide that promotes cell proliferation of PBMCs, B cells, T cells, and/or NK cells, a linker can be present between the transmembrane domain and the first intracellular domain and/or between the first intracellular domain and the second intracellular domain, for polynucleotide embodiments that comprise a second intracellular domain. In some embodiments, a linker of between 1 and 4 alanines is present between the extracellular domain and the transmembrane domain for embodiments comprising an extracellular domain.

In an embodiment of any of the isolated polynucleotide or vector aspects and embodiments provided herein that comprises a first nucleic acid sequence that encodes a chimeric polypeptide that promotes cell proliferation of PBMCs, B cells, T cells, and/or NK cells, and a second nucleic acid sequence that encodes a chimeric antigen receptor (CAR) comprising an antigen-specific targeting region (ASTR), a transmembrane domain, and an intracellular activating domain, the first nucleic acid sequence and the second nucleic acid sequence can be separated by a ribosomal skip sequence. In some embodiments, the ribosomal skip sequence is F2A, E2A, P2A, or T2A.

**In** an embodiment of any of the isolated polynucleotide aspects and embodiments provided herein that encode a chimeric polypeptide that promotes cell proliferation of PBMCs, B cells, T cells, and/or NK cells, said polynucleotide further comprises one or more of a Kozak-type sequence, a WPRE element, and a double stop codon or a triple stop codon, wherein one or more of the stop codons of the double stop codon or the triple stop codon define a termination of a reading from of at least one of the one or more transcriptional units. In certain embodiments, the polynucleotide further comprises a Kozak-type sequence selected from CCACCAUG(G), CCGCCAT/UG(G), GCCGCCGCCAT/UG(G), or GCCGCCGCCAT/UG. In certain embodiments both the -3 and +4 nucleotides relative to a start codon of the first nucleic acid sequence comprise a G. In another embodiment, which can be combined with the preceding embodiment that comprises a Kozak-type sequence and/or the following embodiment that includes triple stop codon, the polynucleotide comprises a WPRE element. In some embodiments, the WPRE element is located 3' of a stop codon of the one or more transcriptional units and 5' to a 3' LTR of the polynucleotide. In another embodiment, which can be combined with either or both of the preceding embodiments (i.e. an embodiment wherein the polynucleotide comprises a Kozak-type sequence and/or an embodiment wherein the polynucleotide comprises a WPRE), the one or more transcriptional units terminates with one or more stop codons of a double stop codon or a triple stop codon.

In one aspect, provided herein is a nucleic acid vector comprising an isolated polynucleotide of any of the isolated polynucleotide aspects and embodiments provided herein that encode a chimeric polypeptide that promotes cell proliferation of PBMCs, B cells, T cells, and/or NK cells,
a. an origin of replication;
b. an antibiotic resistance gene; and/or
c. one or more viral and/or retroviral sequences selected from a retroviral long terminal repeat (LTR), or active fragment thereof, a retroviral cis-acting RNA packaging element, and/or a retroviral central polypurine tract/central termination sequence (CTS) element.

In certain embodiments of this aspect, the vector is a plasmid. In other embodiments, the vector is a replication incompetent viral vector, for example, a replication incompetent retroviral vector or particle. In these embodiments, the replication incompetent retroviral vector or particle, can comprise a 5' retroviral LTR, or active fragment thereof, a retroviral cis-acting RNA packaging element, and a 3' retroviral LTR, or active fragment thereof, wherein the 5' retroviral LTR and the 3' retroviral LTR flank the isolated polynucleotide. In some embodiments, said isolated polynucleotide is in reverse orientation to the nucleic acid sequence encoding the retroviral cis-acting RNA packaging element, the 5' long terminal repeat, and/or the 3' long terminal repeat.

In some aspects, provided herein is a replication incompetent recombinant retroviral particle comprising a retroviral genome comprising any of the aspects and embodiments herein that comprise an isolated polynucleotide that comprises a first nucleic acid sequence that encodes a chimeric polypeptide that promotes cell proliferation of PBMCs, B cells, T cells, and/or NK cells, and further comprising a capsid and an envelope. The retroviral particle can additionally comprise any of the retroviral elements set out in this disclosure. In one embodiment, the retrovirus genome further comprises a second nucleic acid sequence that encodes a chimeric antigen receptor (CAR) comprising an antigen-specific targeting region (ASTR), a transmembrane domain, and an intracellular activating domain. In some embodiments, the first nucleic acid sequence and the second nucleic acid sequence can be separated by a ribosomal skip sequence. In some embodiments, the ribosomal skip sequence is F2A, E2A, P2A, or T2A. In these embodiments, the first nucleic acid sequence and the second nucleic acid sequence are typically on the same transcriptional unit.

In another aspect, provided herein is a mammalian cell wherein said mammalian cell, for example human cell, is a B cell, T cell or NK cell comprising an isolated polynucleotide, which optionally can be integrated into the genome of the cell, according to any of the aspects and embodiments herein that comprise an isolated polynucleotide that comprises a first nucleic acid sequence that encodes a chimeric polypeptide that promotes cell proliferation of PBMCs, B cells, T cells, and/or NK cells. In some embodiments, the isolated polynucleotide in the mammalian cell further comprises a second nucleic acid sequence that encodes a chimeric antigen receptor (CAR) comprising an antigen-specific targeting region (ASTR), a transmembrane domain, and an intracellular activating domain. In some embodiments, the first nucleic acid sequence and the second nucleic acid sequence can be separated by a ribosomal skip sequence. In some embodiments, the ribosomal skip sequence is F2A, E2A, P2A, or T2A. In these embodiments, the first nucleic acid sequence and the second nucleic acid sequence are typically on the same transcriptional unit. In some embodiments, the cell is a T cell or NK cell, and in certain illustrative embodiments, the cell is a human T cell.

In another aspect, provided herein is a packaging cell comprising an isolated polynucleotide according to any of the aspects and embodiments herein that comprise an isolated polynucleotide, which optionally can be integrated into the genome of the cell, that comprises a first nucleic acid sequence that encodes a chimeric polypeptide that promotes cell proliferation of PBMCs, B cells, T cells, and/or NK cells. In some embodiments, the isolated polynucleotide in the cell further comprises a second nucleic acid sequence that encodes a chimeric antigen receptor (CAR) comprising an antigen-specific targeting region (ASTR), a transmembrane domain, and an intracellular activating domain. In some embodiments, the first nucleic acid sequence and the second nucleic acid sequence can be separated by a ribosomal skip sequence. In some embodiments, the ribosomal skip sequence is F2A, E2A, P2A, or T2A. In these embodiments, the first nucleic acid sequence and the second nucleic acid sequence are typically on the same transcriptional unit. In some embodiments, the cell is cell provided herein for generation of retroviral particles.

In one aspect, provided herein is a method of promoting cell proliferation of a PBMC, for example a B cell, T cell, and/or NK cell, comprising transducing and/or genetically modifying the cell with a vector that encodes a lymphoproliferative element, and in illustrative embodiments a chimeric polypeptide according to any of the embodiments provided herein, wherein the chimeric polypeptide promotes PBMC proliferation. The method can include culturing the cell for at least 7, 14, 21, 28, 35, 42, or 60 days. Typically, this culturing is performed in the absence of IL-2, for example after transduction with media that can include IL-2 in some embodiments.

In one aspect, provided herein is a method for transfecting, transducing and/or genetically modifying a B cell, T cell, and/or NK cell, which in some non-limiting embodiments, can be an allogeneic cell, an autologous cell, or other than an allogeneic cell, comprising contacting the cell with an isolated polynucleotide according to any of the aspects and embodiments herein that comprise an isolated polynucleotide that comprises a first nucleic acid sequence that encodes a chimeric polypeptide that promotes cell proliferation of PBMCs, B cells, T cells, and/or NK cells (CLE aspects and embodiments). In some embodiments of this method aspect, the cell is a T cell and the isolated polynucleotide further comprises a second nucleic acid sequence that encodes a chimeric antigen receptor (CAR) comprising an antigen-specific targeting region (ASTR), a transmembrane domain, and an intracellular activating domain. In other words, the method for transfecting, transducing and/or genetically modifying a a B cell, T cell, and/or NK cell can include contacting the cell with isolated polynucleotides according to any of the CLE or CLE CAR embodiments herein. In some embodiments, the first nucleic acid sequence and the second nucleic acid sequence can be separated by a ribosomal skip sequence. In some embodiments, the ribosomal skip sequence is F2A, E2A, P2A, or T2A. In these embodiments, the first nucleic acid sequence and the second nucleic acid sequence are typically on the same transcriptional unit. In some embodiments, the cell is a T cell or NK cell, and in certain illustrative embodiments, the cell is a human T cell. The contacting is performed under effective conditions for transfecting, transducing and/or genetically modifying the cell. In some embodiments, the isolated polynucleotide at the time of contacting is within a vector according to any of the vector aspect and embodiments provided herein. In some embodiments, the isolated polynucleotide is in a non-replicative retroviral particle at the time of contacting.

In some embodiments of any of the aspects herein, the NK cells are NKT cells.

The following non-limiting examples are provided purely by way of illustration of exemplary embodiments, and in no way limit the scope of the present disclosure. Furthermore, it is to be understood that any subject matter disclosed or claimed herein encompass all variations, combinations, and permutations of any one or more features described herein. Any one or more features may be explicitly excluded from the claims even if the specific exclusion is not set forth explicitly herein. It should also be understood that disclosure of a reagent for use in a method is intended to be synonymous with (and provide support for) that method involving the use of that reagent, according either to the specific methods disclosed herein, or other methods known in the art unless one of ordinary skill in the art would understand otherwise. In addition, where the specification and/or claims disclose a method, any one or more of the reagents disclosed herein may be used in the method, unless one of ordinary skill in the art would understand otherwise.

### EXAMPLES

### Example 1. Generation of riboswitches that respond specifically to nucleoside analogue antiviral drugs.

This example provides a method to screen libraries based on natural structural riboswitches that bind guanosine and deoxyguanosine. These riboswitches were used as scaffolds to develop biased libraries for the selection of aptamers that bind specifically to a ligand nucleoside analogue. Previously, isothermal titration calorimetry has been used to show these natural riboswitches bind to their native ligands. Additional tests showed a deoxyguanosine switch also interacted weakly with the nucleoside analogues acyclovir and penciclovir, leading to the re-design of this sequence into a new library. The single-stranded regions of the riboswitch were targeted for mutation and variant sequences that specifically respond to acyclovir or penciclovir were selected for.

### Materials

Selection components guanine, guanosine, deoxyguanosine, acyclovir, and penciclovir were ordered from Sigma-Aldrich (St. Louis, MO). Acyclovir was the initial target while penciclovir was a special interest analyte used in latter rounds and guanine, guanosine, and deoxyguanosine were used as counter-targets. Graphene oxide (GrO), to be used as the partitioning medium, was purchased from Angstron Materials (Dayton, OH). HEPES (pH 7.3) and MgCl₂ were purchased from Amersco LLC. (Solon, OH). KCl was purchased from Teknova (Hollister, CA). Selection buffer was prepared at 5X (1X as 50 mM HEPES, 100 mM KCl, 20 mM MgCl₂, pH 7.3). Targets, counter-targets, and oligos were reconstituted in nuclease-free water for preliminary analysis and aptamer screening. Aliquots were prepared for all targets and stored at -20°C to maximize shelf life.

### Generation of the aptamer library

The initial aptamer library template was synthesized by IBA GmbH (Gottingen, Germany) as the reverse complement of the sequences in FIG. 14. In FIG. 14, the nucleotides in boxes are single-stranded in the known sequences, with "mutations" introduced during synthesis to allow for better binding to analogues of the original targets. For nucleotides within the boxes outlined with solid lines, substitution mutations were allowed; for nucleotides within the boxes outlined with dashed lines, substitution mutations as well as insertions or deletions were allowed. Primers were synthesized by IDT (Coralville, IA) as single-stranded DNA. T7 primer (SEQ ID NO:240) was combined with library template sequences for primer extension with Titanium Taq DNA polymerase (Clontech; Mountain View, CA). Primer-extended material was transcribed using the Ampliscribe T7 High Yield Transcription Kit (Epicentre; Madison, WI) and then purified on 10% denaturing polyacrylamide gel electrophoresis (PAGE) with 8 M urea before use in selection. During selection, the library was reverse-transcribed using SuperScript IV Reverse Transcriptase (Invitrogen; Carlsbad, CA) using reverse primer (SEQ ID NO:241) and amplified using Titanium Taq DNA polymerase (Clontech; Mountain View, CA). The aptamer with SEQ ID NO:248 had a J2-3 loop variation of -3 to -1 and a diversity of ~2.25x10¹⁰. The aptamer with SEQ ID NO:250 had a J2-3 loop variation of 0 (native) to +5 and a diversity of ~9.38x10¹⁴. The two oligonucleotides (SEQ ID NOs:249 and 250) were mixed at a ratio of 1:4160 to produce equimolar diversity in the combined library pool, with a total diversity of ~9.38x10¹³.

### Library screening

Library screening was conducted using a graphene oxide-Systematic Evolution of Ligands by EXponential enrichment (GO-SELEX) approach (FIG. 15) (Park *et al.,* 2012), taking advantage of the π-π interaction that grants graphene oxide a high affinity for single-stranded nucleic acids (Zeng *et al., 2015).* The goal was to select sequences that did not interact with the 1X selection buffer or with the counter-targets (guanine, guanosine, and deoxyguanosine) but did bind to the positive target acyclovir.

For each round, a given amount of library was first refolded in 1X selection buffer (5-minute denaturing at 90 °C, 5 minutes at 4 °C, then room temperature). The counter-targets were then added to refolded libraries and incubated for 30 minutes at 37 °C. The exceptions to this were rounds 1 and 2, where the counter-targets were only briefly (< 1 minute) included to help load the library onto the GrO. After allowing the library to interact with the counter-targets and buffer components, unbound library was loaded onto GrO (mass equal to 100 times the mass of the library at the start of the round) over the course of a 10-minute incubation at 37 °C. The solution was then centrifuged at 7,000 x g to sediment the GrO. The supernatant, which contained sequences bound to the counter-targets and/or to the buffer, was removed. The sediment was then washed twice with 200 µL 1X selection buffer, centrifuging at 7,000 x g and removing the supernatant after each wash. A positive target-containing solution was then added and allowed to elute library from the GrO under the conditions indicated in Table 1 for up to 60 minutes at 37 °C, essentially allowing the target to compete with graphene oxide for library binding. Sequences that bound more strongly to the target would desorb from graphene oxide and remain bound to the target at the end of the incubation. A final centrifugation step separated the released material, located in the supernatant, from the non-responsive library that remained bound to the graphene oxide.

After positive selection, the recovered RNA purified using 10% denaturing PAGE with 8 M Urea, was then quantified using a spectrophotometer reading (Table 1), reverse-transcribed with SuperScript IV, and amplified using PCR with Titanium Taq DNA polymerase. Amplification products were transcribed into RNA for the next round of selection.

Three tiers of stringency were implemented over the course of selection (Table 1). The first two rounds of selection did not include screening against counter-targets to maximize library loading onto GrO. Additionally, a large excess of acyclovir was used in positive incubations to maximize library recovery, thus the low-stringency designation. Counter-target incubations were introduced after library recovery was achieved, as middle-stringency conditions. The ratio of acyclovir to library was also reduced during these three rounds to increase library competition for binding to target. Once greater than 10% recovery was achieved, the final rounds of high-stringency selection were implemented. Counter-targets/library ratio remained high and positive target/library ratio was brought to 1:1 while positive incubation time was reduced, to select for faster binding sequences. Once library recovery was shown to remain over 10% after more than two rounds of the high-stringency conditions, parallel assessments were conducted.

**Table 1. Selection and Assessment Conditions. Conditions used for each round of selection or incubation, with recovery as the ratio between recovered sample and input library for each round. Library enrichment was monitored over the course of selection. *Counter-targets used for loading, not extended incubation. †Pre-loading incubation conducted with pooled counter-targets. ‡Pre-loading incubation conducted with positive target acyclovir. This was done to minimize the recovery of cross-reactive species. The following abbreviations are used in this table: "X-Targets" are counter-targets; "(+) Target" is acyclovir or penciclovir; "(+) Incubation Time (min)" is the time the "Library:(+) Target" solution was incubated on the GrO. G0 is Generation 0 and so on; R1 is Round 1 and so on. For the parallel assessment (parallel 1 and parallel 2) the incubations were performed with: (-) 1X selection buffer only, (X) counter-targets in 1X selection buffer, (+) acyclovir in 1X selection buffer, and (P) penciclovir in 1X selection buffer.**

| **Generation (Stringency)** | **Library:X-Targets (30-min inc.)** | **Library:(+) Target** | **(+) Incubation Time (min)** | **Recovery (%)** |
|---|---|---|---|---|
| G0/R1 (low) | 1:1000* | 1:1000 | 60 | 0.43 |
| G1/R2 (low) | 1:1000* | 1:1000 | 60 | 2.00 |
| G2/R3 (middle) | 1:1000 | 1:500 | 60 | 3.60 |
| G3/R4 (middle) | 1:1000 | 1:100 | 60 | 8.73 |
| G4/R5 (middle) | 1:1000 | 1:10 | 60 | 10.20 |
| G5/R6 (high) | 1:1000 | 1:1 | 60 | 12.00 |
| G6/R7 (high) | 1:1000 | 1:1 | 60 | 8.60 |
| G7/R8 (high) | 1:1000 | 1:1 | 60 | 9.72 |
| G8/R9 (high) | 1:1000 | 1:1 | 30 | 20.08 |
| G9/R10 (high) | 1:1000 | 1:1 | 30 | 10.62 |
| G10(-)^{†} (parallel 1) | - | - | 30 | 3.74 |
| G10(X)^{†} (parallel 1) | 1:40 | - | 30 | 3.60 |
| G10(+)^{†} (parallel 1) | - | 1:4 | 30 | 14.14 |
| G10(P)^{†} (parallel 1) | - | 1:4 | 30 | 5.46 |
| G11(-)^{‡} (parallel 2) | - | - | 30 | 4.60 |
| G11(X)^{‡} (parallel 2) | 1:40 | - | 30 | 5.26 |
| G11(+)^{†} (parallel 2) | - | 1:2 | 30 | 9.34 |
| G11(P)^{‡} (parallel 2) | - | 1:4 | 30 | 6.32 |

For the two parallel assessments, library to be assessed was divided into four equal amounts for preparation and refolding as above (FIG. 16). For each condition, 50 pmoles of library were combined with 1X selection buffer, refolded (90 °C for 5 minutes, 4 °C for 5 minutes), and then incubated with 200 µL of 10 µM combined counter-targets in 1X selection buffer for 30 minutes at 37 °C. These samples were then loaded onto an amount of graphene oxide equal to 100 times the mass of library in the sample and incubated for 10 minutes at 37 °C and then washed twice with 200 µL of 1X selection buffer as before. The loaded graphene oxide samples were then incubated in parallel with 200 µL of the appropriate assessment condition (1X selection buffer only, 10 µM pooled counter-targets, 1 µM penciclovir, 1 µM acyclovir for the first parallel assessment, or 0.5 µM acyclovir for the second parallel assessment; in Table 1 these conditions are shown as: (-); (X); (P); (+); and (+), respectively) in 1X selection buffer for 30 minutes at 37 °C. A final centrifugation step separated desorbed responsive library from non-responsive graphene oxide-bound library. The responsive libraries were quantified using spectrophotometric reading (Table 1), verified using 10% denaturing PAGE with 8 M urea, and prepared for a second parallel assessment. This follow-up assessment continued to use counter-targets for the positive sample's pre-loading incubation, but utilized positive target acyclovir for each other samples' pre-incubation. This was done to minimize representation of cross-reactive sequences in a given sample (i.e. responsive to counter-targets in the positive sample, responsive to acyclovir in the negative, counter-targets, or penciclovir samples). Material recovered from the second parallel assessment was quantified using spectrophotometric reading (Table 1), verified using 10% denaturing PAGE with 8 M urea, and prepared for sequencing by reverse transcription and PCR to generate double-stranded DNA.

### Sequencing

The initial library was subjected to over 10 rounds of GrO-based selection and parallel assessment (Table 1). The GO-SELEX process is designed to enrich for sequences over multiple rounds of selection that bind to the given targets of interest and remove sequences that bind to the non-target compounds or buffer components. As a result, the populations to be sequenced are expected to contain multiple copies of potential aptamer candidates.

The Illumina MiSeq system (San Diego, CA) was implemented to sequence the aptamer libraries after parallel assessment using a single-end read technique. Deep sequencing and subsequent data analysis reduces the large number of screening rounds traditional SELEX requires, which may introduce error and bias due to the screening process (Schütze *et al.,* 2011). Five samples were sequenced: the final generation library that responded to acyclovir, the final generation library that responded to the counter-targets, the final generation library that responded to 1X selection buffer (negative condition), the penultimate generation library that responded to acyclovir, and the final generation library that responded to the additional target of interest, penciclovir. From these sets of data, sequence families were constructed at 95% homology (sequence similarity considering mutations, deletions, and insertion) for aptamer candidate identification. There were 1,711,535 raw sequences (124,600 unique sequences) from the library that responded to acyclovir and 2,074,832 raw sequences (110,149 unique sequences) from the library that responded to penciclovir.

### Aptamer candidate selection

Sequence family construction focused primarily on sequence similarity. This means that a sequence's frequency in the positive target population was factored in, but greater emphasis was placed on the degree of variation between similar sequences, with 95% homology being the minimum requirement (100% match over the entire sequence is not necessary to join a family, up to 2 bases can be mismatched, inserted, or deleted). One would therefore expect families with the greatest number of members to rank highly as aptamer candidates. After families are constructed, consideration can be given to the relative presence of a family in a given population - families that occur frequently in the negative and counter-target populations are considered weaker candidates, as they demonstrate a degree on nonspecific interaction in binding to buffer or counter-target components. Additionally, families that demonstrate a high rate of enrichment (i.e. large ratio between the final positive population and penultimate positive population) improve their candidacy, as enrichment rate has been linked to the binding affinity of a candidate relative to the rest of the population (Levay et al., 2015; Wang et al., 2014). Under these conditions, several candidate families appeared to be strong candidates for binding acyclovir (Table 2) and penciclovir.

**Table 2. Candidates for binding acyclovir and penciclovir. DNA sequences corresponding to the non-stem regions of the acyclovir binding RNA riboswitches. Seven families were identified in the screen: 582, 769, 795, 935, 946, 961, and 996 with between 1 and 39 sequences in each family. The percent identity for each sequence in the family was compared to the most prevalent sequence within each family (582-1, 769-1, 795-1, 935-1, 946-1, 961-1, and 996-1). The percent identity for each sequence in the family was also compared to the wild-type sequence.**

| **Candidate Family-Sequence Number** | **SEQ ID NO:** | **Sequence** | **Length** | **% Identity** | |
|---|---|---|---|---|---|
| | | | | **Consensus** | **Wildtype** |
| 582-1 | 108 | ACAGCTTAGCGTAATGGCTACTGACG CCGTCCAAACCTATTTACAGACT | 49 | 100 | 80.77 |
| 582-2 | 109 | ACAGCTTAGGATAATGGCTACTGACG CCGTCCAAACCTATTTACAGACT | 49 | 95.92 | 80.77 |
| 582-3 | 110 | ACAGCTTAGCATAATGGCTACTGACG CCGTCCAAACCTATTCACAGACT | 49 | 95.92 | 80.77 |
| 582-4 | 111 | ACAGCTTAGCATAATGGCTACTGACG CCGTCCAAACCTATTGACAGACT | 49 | 95.92 | 80.77 |
| 582-5 | 112 | ACAGCATAGCATAATGGCTACTGAC GCCGTCCAAACCTATTTACAGACT | 49 | 95.92 | 82.69 |
| 582-6 | 113 | ACAGCTTAGCATAATGGCTACTGACG CCGTCCAAACCTATGTACAGACT | 49 | 95.92 | 80.77 |
| 582-7 | 114 | ACAGCTAGCGTAATGGCTACTGACGC CGTCCAAACCTATTTACAGACT | 48 | 97.96 | 80.77 |
| 582-8 | 115 | ACAGCTTAGCATTATGGCTACTGACG CCGTCCAAACCTATTTACAGACT | 49 | 95.92 | 80.77 |
| 582-9 | 116 | ACAGTTAGCATAATGGCTACTGACGC CGTCCAAACCTATTTACAGACT | 48 | 95.92 | 82.69 |
| 582-10 | 117 | ACAGCTTAGCATAATGGCTACTGACG CGGTCCAAACCTATTTACAGACT | 49 | 95.92 | 80.77 |
| 582-11 | 118 | ACAGCTTAGCTTAATGGCTACTGACG CCGTCCAAACCTATTTACAGACT | 49 | 97.96 | 80.77 |
| 582-12 | 119 | ACAGCTTAGCATAATGGCTACTGACG CCGTCCAAACCCATTTACAGACT | 49 | 95.92 | 80.77 |
| 582-13 | 120 | ACAGCTTAGCATAATGGCTACTGACG CCGTCCAAACCAATTTACAGACT | 49 | 95.92 | 80.77 |
| 582-14 | 121 | ACAGCTTAGCATAATGGATACTGACG CCGTCCAAACCTATTTACAGACT | 49 | 95.92 | 80.77 |
| 582-15 | 122 | ACAGCTTAGCATTGTGGCTACTGACG CCGTCCAAACCTATTTACAGACT | 49 | 93.88 | 78.85 |
| 582-16 | 123 | ACAGGTTAGCATAATGGCTACCGAC GCCGTCCAAACCTATTTACAGACT | 49 | 93.88 | 82.69 |
| 582-17 | 124 | ACAGCTTAGCGTAATGGCTACTGACG CCGCCCAAACCTATTTACAGACT | 49 | 97.96 | 82.69 |
| 582-18 | 125 | ACAGCTTAGCATAATGGCTACTGACG CCGTCCAAAACTATTTCCAGACT | 49 | 93.88 | 80.77 |
| 582-19 | 126 | ACAGCCTAGCATAAGGGCTACTGAC GCCGTCCAAACCTATTTACAGACT | 49 | 93.88 | 82.69 |
| 582-20 | 127 | ACAGCTTAGCATAATGGCTACTGAGG CCGTCCAAACCTATTTACAGACT | 49 | 95.92 | 80.77 |
| 582-21 | 128 | ACAGCTTACCTTAATGGCTACTGACG CCGTCCAAACCTATTTACAGACT | 49 | 95.92 | 78.85 |
| 582-22 | 129 | ACAGCTTAGCATAATGGCTACCGACG CTGTCCAAACCTATTTACAGACT | 49 | 93.88 | 78.85 |
| 582-23 | 130 | ACAGCTTAGCGTAATGGCTACTGGCG CCGTCCAAACCTATTTACAGACT | 49 | 97.96 | 78.85 |
| 582-24 | 131 | ACAGCTTAGCATACTGGCTACTGACG CCGCCCAAACCTATTTACAGACT | 49 | 93.88 | 82.69 |
| 582-25 | 132 | ACAGCTTAGCATAATGGCTACTGACG CCGTCCTAACCTATTTACAGACT | 49 | 95.92 | 80.77 |
| 582-26 | 133 | ACAGGTTAGCATAATGCCTACTGACG CCGTCCAAACCTATTTACAGACT | 49 | 93.88 | 82.69 |
| 582-27 | 134 | ACAGCTTAGCATAATTGCTACTGACG CCGTTCAAACCTATTTACAGACT | 49 | 93.88 | 82.69 |
| 582-28 | 135 | ACAGCTTAGCATAAAGGCTACTGAC GCCGTCCAAACCTATTTACAGACT | 49 | 95.92 | 80.77 |
| 582-29 | 136 | ACAGCTTAGCGTAATGGCTACTGACG CCGTCTAAACCTATTTCCAGACT | 49 | 95.92 | 80.77 |
| 582-30 | 137 | ACAGGTTAGCATAATGGCTACTGACG CCGTCCAAACCTATTTAGAGACT | 49 | 93.88 | 86.54 |
| 582-31 | 138 | ACAGGGTAGCGTAATGGCTACTGAC GCCGTCCAAACCTATTTACAGACT | 49 | 95.92 | 84.62 |
| 582-32 | 139 | ACAGCGTAGCATAATGGCTACTGAC GCCGTTCAAACCTATTTACAGACT | 49 | 93.88 | 86.54 |
| 582-33 | 140 | ACAGCTTAGCATAATGGCTACTGACG CCGTCCAAACTCATTTACAGACT | 49 | 93.88 | 78.85 |
| 582-34 | 141 | ACAGCGTAGCATAGTGGCTACTGAC GCCGTCCAAACCTATTTACAGACT | 49 | 93.88 | 82.69 |
| 582-35 | 142 | ACAGCTTAGTGTAATGGCTACTGACG CTGTCCAAACCTATTTACAGACT | 49 | 95.92 | 76.92 |
| 582-36 | 143 | ACAGCTTAGCATAATGGCTACTGACG GCGTTCAAACCTATTTACAGACT | 49 | 93.88 | 82.69 |
| 582-37 | 144 | ACAGGTTAGCATAATGGCTACTGACG CCGTCCAAACCTATTTATAGACT | 49 | 93.88 | 84.62 |
| 582-38 | 145 | ACAGCTTAGCATAATGGCTACTGACG CCGTCCAAACCTATTGTCGACT | 48 | 91.84 | 80.77 |
| 582-39 | 146 | ACAGCTTAGCATAATGGCTACTGACG CCGTCCAAACCTATTTACGACT | 48 | 95.92 | 80.77 |
| 582 Consensus Sequence | 222 | ACAGNNTASBDTWVDKSMTACYGRS GSBGYYYWAAMYHATKBHBNGACT Where the N at position 5 can be C, G, or no nucleotide, the N at position 6 can be A, C, G, T, or no nucleotide, and the N at position 45 can be A or no nucleotide. | 49 | - | - |
| 769-1 | 147 | ACAGGTCAGCATAATGTGCTAGTGCG CCTTCAAACCTATTTAGAGACT | 48 | 100 | 82.69 |
| 769-2 | 148 | ACAGGTCAGCATAATGTGCTAGTGCG CCCTCAAACCTATTTAGAGACT | 48 | 97.92 | 80.77 |
| 769-3 | 149 | ACAGGTTAGCATAATGTGCTATTGCG CCTTCAAACCTATTTAGAGACT | 48 | 95.83 | 84.62 |
| 769-4 | 150 | ACAGGTCAGCATAATGTGCTAGTGCG CATTCAAACCTATTTAGAGACT | 48 | 97.92 | 80.77 |
| 769-5 | 151 | ACAGGTTAGCATAATGTGCTAGTGCG CCTTCAAACCTATTTTGAGACT | 48 | 95.83 | 84.62 |
| 769-6 | 152 | ACAGGTTATCATAATGTGCTAGTGCG CCTTCAAACCTATTTAGAGACT | 48 | 95.83 | 82.69 |
| 769-7 | 153 | ACAGGTTAGCATGATGTGCTAGTGCG CCTTCAAACCTATTTAGAGACT | 48 | 95.83 | 82.69 |
| 769-8 | 154 | ACAGGTTAGCATAATGGGCTAGTGC GCCTTCAAACCTATTTAGAGACT | 48 | 95.83 | 86.54 |
| 769-9 | 155 | ACAGGTCAGCAAAATGTGCAAGTGC GCCTTCAAACCTATTTAGAGACT | 48 | 95.83 | 78.85 |
| 769-10 | 156 | ACAGGTCAGCATAATGTGCTAGTGCG CCTTCAAACCTATCTGGAGACT | 48 | 95.83 | 82.69 |
| 769-11 | 157 | ACAGCTTAGCATAATGTGCTAGTGCG CCTTCAAACCTATTTAGAGACT | 48 | 95.83 | 82.69 |
| 769-12 | 158 | ACAGGTCAGCATAATGTGCTAGTGCG CCTTCAAACCTATTTACAGACT | 48 | 97.92 | 80.77 |
| 769-13 | 159 | ACAGGTCAGCATAATGTGCTAGTGCG CCTTCAAACATATTTAGAGACT | 48 | 97.92 | 80.77 |
| 769-14 | 160 | ACAGGGTAGCATAATGTGCTAGTGC GCCTTCAAACCTATTTAGAGACT | 48 | 95.83 | 86.54 |
| 769-15 | 161 | ACAGGTTAGCATAATGTGCTAGTGCG CCCTCAAACCTATTTAGAGACT | 48 | 95.83 | 82.69 |
| 769-16 | 162 | ACAGGTTAGCATAATGTGCCAGTGCG CCTTCAAACCTATTTAGAGACT | 48 | 95.83 | 82.69 |
| 769-17 | 163 | ACAGGTCAGCATAATGGGCTAGTGC GCCTTCAAACCTATTTAGAGACT | 48 | 97.92 | 84.62 |
| 769 Consensus Sequence | 223 | ACAGSKYAKCAWRATGKGCHAKTGC GCMYTCAAACMTATYTDSAGACT | 48 | - | - |
| 795-1 | 164 | ACAGCGAAGCATAATGGCTACTGAC GCCCTCAAACCCTATTTGCAGACT | 49 | 100 | 83.02 |
| 795-2 | 165 | ACAGCGAAGCATAATGGCTACTGAC GCCCTCAAACCCTATTTACAGACT | 49 | 97.96 | 81.13 |
| 795-3 | 166 | ACAGCGAAGCATAATGGCTTCTGAC GCCCTCAAACCCTATTTGCAGACT | 49 | 97.96 | 81.13 |
| 795-4 | 167 | ACAGCCAAGCATACTGGCTACTGAC GCCCTCAAACCCTATTTGCAGACT | 49 | 95.92 | 79.25 |
| 795-5 | 168 | ACAGCGAAGCATAATGGCTACTGAC GCCCGCAAACCCTATTTGCAGACT | 49 | 97.96 | 81.13 |
| 795-6 | 169 | ACAGCGAAGCATAATGGCTACTGAC GGCCTCAAACCCTATTTGCAGACT | 49 | 97.96 | 80.77 |
| 795-7 | 170 | ACAGCGAGGCATAATGGCTACTGAC GCCCTCAAACCCTATTTGCAGACT | 49 | 97.96 | 81.13 |
| 795-8 | 171 | ACAGCGAAGCATAATGGCTACTGAC GCCTTCAAACCCTATTTGCAGACT | 49 | 97.96 | 84.91 |
| 795-9 | 172 | ACAGCGAAGCATAATGGCTACAGAC GCCCTCAAAACCTATTTGCAGACT | 49 | 95.92 | 80.77 |
| 795-10 | 173 | ACAGCGAAGCATAATGGCTACTGAC GCCCTCAAACCCTATTTGAGACT | 48 | 97.96 | 83.02 |
| 795-11 | 174 | ACAGCGAAGCATAATGGCTACTGAC GCCCTCAAACCCTATTGTCGACT | 48 | 93.88 | 76.92 |
| 795-12 | 175 | ACAGCCAAGCATAATGGCTACTGAC GCCCTCAAACCCTATTTGCAGACT | 49 | 97.96 | 81.13 |
| 795-13 | 176 | ACAGCGAAGCATAATGGCTACTGAC GCCCTCAAACCCTATTTGGCGACT | 49 | 95.92 | 83.02 |
| 795-14 | 177 | ACAGCGAAGCATAATGTCTACTGAC GCCCTCAAACCCTATTTGCAGACT | 49 | 97.96 | 81.13 |
| 795-15 | 178 | ACAGCGAAGCATAATGGCTACTGAC GCCGTCAAACCCTATTTGTAGACT | 49 | 95.92 | 83.02 |
| 795-16 | 179 | ACAGCGAAGCATAATGGCTACTGAC GCCCTCAAACCTTATTTGCAGACT | 49 | 97.96 | 83.02 |
| 795-17 | 180 | ACAGGTAGCATAATGGCTACTGACG CCCTCAAACCCTATTTGCAGACT | 48 | 95.92 | 84.91 |
| 795-18 | 181 | ACAGCGAAGCATAATGGCTACTGAC GCCCTCAAACCCTATTTCTAGACT | 49 | 95.92 | 81.13 |
| 795-19 | 182 | ACAGCGAAGCATAATGGCTACTGAC GCCCTCAAACCCTATTTGTAGACT | 49 | 97.96 | 83.02 |
| 795 Consensus Sequence | 224 | ACAGNSWRGCATAMTGKCTWCWGA CGSCBKCAAAMCYTANTTVNMGACT Where the N at position 5 can be C or no nucleotide, the N at position 40 can be T or no nucleotide, and the N at position 44 can be C, G, T, or no nucleotide | 49 | - | - |
| 935-1 | 183 | ACAGGGTAGCATAATGGGCTACTTG ACGCCTTCACCTATTTGTAGACT | 48 | 100 | 86.79 |
| 935-2 | 184 | ACAGGGTAGCATAATGGGCTACTTG ACGCCTTCACCTATTTGAGACT | 47 | 97.92 | 86.79 |
| 935-3 | 185 | ACAGGGTAGCATAATGGGCTACTTTA CGCCTTCACCTATTTGTAGACT | 48 | 97.92 | 84.62 |
| 935-4 | 186 | ACAGGGTAGCATAATGGGCTACTTG ACGCCTTCACCTATTTCTAGACT | 48 | 97.92 | 84.91 |
| 935-5 | 187 | ACAGGGTAGCATAATGGGCTACTTG ACGCCTTCACCTATTTGGAGACT | 48 | 97.92 | 88.68 |
| 935-6 | 188 | ACAGGGTAGCATAGTGGGCTACTTG ACGCCTTCACCTATTTGTAGACT | 48 | 97.92 | 84.91 |
| 935-7 | 189 | ACAGGGTAGCATGATGGGCTACTTG ACGCCTTCACCTATTTGTAGACT | 48 | 97.92 | 84.91 |
| 935-8 | 190 | ACAGGGTAGCATAATGGGCTACTTG ACGCCTTCACCTATTAGTAGACT | 48 | 97.92 | 84.91 |
| 935-9 | 191 | ACAGGGTAGCATAATGGGCTATTTGA CGCCTTCACCTATTTGTAGACT | 48 | 97.92 | 84.91 |
| 935-10 | 192 | ACAGGGTAGCATAATGGGCTACTTGC CGCCTTCACCTATTTGTAGACT | 48 | 97.92 | 86.54 |
| 935-11 | 193 | ACAGTGTAGCATAATTGGCTACTTGA CGCCTTCACCTATTTGTAGACT | 48 | 95.83 | 83.02 |
| 935-12 | 194 | ACAGGGTAGCATAATGGGCTACTTG ACGCTTTCACCTTTTTGTAGACT | 48 | 95.83 | 83.02 |
| 935-13 | 195 | ACAGGGTAGCATAAGGGGCTACTTG ACGCCTTCACCTATTTGTAGACT | 48 | 97.92 | 84.91 |
| 935-14 | 196 | ACAGGGTAGCATAATGGACTACTTG ACGCCTCCACCTATTTGTAGACT | 48 | 95.83 | 81.13 |
| 935-15 | 197 | ACAGGGTAGCATAATGGGCTACTTGT CGCCTTCACCTATTTGTAGACT | 48 | 97.92 | 84.62 |
| 935 Consensus Sequence | 225 | ACAGKGTCGCATRRKKGRCTAYTTKH CGCYTYCACCTWTTWSNAGACT Where the N at position 43 can be G, T, or no nucleotide. | 48 | - | - |
| 946-1 | 198 | ACAGCGTAGCATAATGGGCTGCAGA CGCCGTCAAACCTATTTGCAGACT | 49 | 100 | 84.62 |
| 946-2 | 199 | ACAGCGTAGCATAATGGGCTGCAGA CGCAGTCAAACCTATTTGCAGACT | 49 | 97.96 | 82.69 |
| 946-3 | 200 | ACATGTAGCATAATGGGCTACTGACG CCGTCAAACCTATTTGCAGACT | 48 | 91.84 | 86.54 |
| 946-4 | 201 | ACAGCGTAGCATAGTGGGCTGCAGA CGCCGTCAAACCTATTTGCAGACT | 49 | 97.96 | 82.69 |
| 946-5 | 202 | ACAGTGTAGCATAATGGGCTGCAGA CGCCTTCAAACCTATTTGGAGACT | 49 | 93.88 | 88.46 |
| 946-6 | 203 | ACAGTGTAGCATAATGGGCTGCTGAC GCCGTCAAACCTATTTGAAGACT | 49 | 93.88 | 86.54 |
| 946-7 | 204 | ACAGCGTAGCATAATGGGCTACAGG CGCCGTCAAACCTATTTGCAGACT | 49 | 95.92 | 84.62 |
| 946-8 | 205 | ACAGCGTAGCATAATGGGCTACTGG CGCCGTCAAACCTATTTGCAGACT | 49 | 93.88 | 86.54 |
| 946-9 | 206 | ACAGCGTAGCATAATGGGCTGCAGA CGCCGTCAAACCTATTTGAGACT | 48 | 97.96 | 84.62 |
| 946-10 | 207 | ACAGGTAGCATAATGGGCTGCAGAC GCCGTCAAACCTATTTGCAGACT | 48 | 97.96 | 84.62 |
| 946-11 | 208 | ACAGGTAGCATAATGGGCTGCTGAC GCCGTCAAACCTATTTACAGACT | 48 | 93.88 | 84.62 |
| 946-12 | 209 | ACAGCGTAGCATATTGGGCTGCAGA CGCCGTCAAACCTATTTGCAGACT | 49 | 97.96 | 82.69 |
| 946-13 | 210 | ACAGCGTAGCATAATGGGCTGCAGA CGCCTTCAAACCTATTTGGAGACT | 49 | 95.92 | 88.46 |
| 946-14 | 211 | ACAGTGTAGCATAATGGGCTGCAGA CGCCGTCAAACCTATTTGAGACT | 48 | 95.92 | 84.62 |
| 946-15 | 212 | ACAGCGTAGCATAATGGGCTGCTGA CGCCGTCAAACCTATTTGGAGACT | 49 | 95.92 | 88.46 |
| 946-16 | 213 | ACAGCGTAGCATAATGGGCTGCAGA CGCCGTCAAACCTATTTACAGACT | 49 | 97.96 | 82.69 |
| 946-17 | 214 | ACAGCGTAGCATAATGGGCTGCTGA CGCCGTCAAACCTATTTGCAGACT | 49 | 97.96 | 86.54 |
| 946-18 | 215 | ACAGGGTAGCATAATGGGCTGCAGA CGCCGTCAAACCTATTTGGAGACT | 49 | 95.92 | 88.46 |
| 946-19 | 216 | ACAGCGTAGCATAATGGGCTACAGA CGCCGTCAAACCTATTTGCAGACT | 49 | 97.96 | 86.54 |
| 946-20 | 217 | ACAGCGTCGCATAATGGGCTGCAGA CGCCGTCAAATCTATTTGCAGACT | 49 | 95.92 | 80.77 |
| 946-21 | 218 | ACAGCGTAGCATAATGGGCTTCAGA CGCCGTCAAACCTATTTGCAGACT | 49 | 97.96 | 84.62 |
| 946-22 | 219 | ACATGTAGCATAATGGGCTGCAGAC GCCGTCAAACCTATTTGGAGACT | 48 | 93.88 | 84.62 |
| 946 Consensus Sequence | 226 | ACANNGTMGCATADTGGGCTDCWGR CGCMKTCAAAYCTATTTRNAGACT Where the N at position 4 can be G or no nucleotide, the N at position 5 can be C, G, T, or no nucleotide, and the N at position 44 can be A, C, G, or no nucleotide. | 49 | - | - |
| 961-1 | 220 | ACACCGTAGCATAATGGGCTACTGCC GCCGTCGACCTTTTGGAGACT | 47 | 100% | 82.69 |
| 996-1 | 221 | ACAGGGTAGCATAATGGCTTAGGAC GCCTTCAAACCTATCAAGACT | 46 | 100% | 76.92 |

Positive target acyclovir produced seven strong candidates (SEQ ID NOs:87-93; RNA sequences including stem regions) corresponding to 582-1 (SEQ ID NO: 108), 769-1 (SEQ ID NO: 147), 795-1 (SEQ ID NO:164), 935-1 (SEQ ID NO:183), 946-1 (SEQ ID NO:198), 961-1 (SEQ ID NO:220), and 996-1 (SEQ ID NO:221), each designated F1A (FIG. 17). These sequences were the most prevalent sequences in each family (the DNA sequences of all the members of each family are: 582 (SEQ ID NOs: 108-146); 769 (SEQ ID NOs: 147-163); 795 (SEQ ID NOs: 164-182); 935 (SEQ ID NOs: 183-197); 946 (SEQ ID NOs: 198-219); 961 (SEQ ID NO:220); and 996 (SEQ ID NO:221)). The consensus sequences show all possible substitutions or gaps at each nucleotide position for each family (SEQ ID NOs:222-226). As the goal was to identify aptamers from a library based on RNA that is known to bind to deoxyguanosine, strong candidates needed to have minimal presence in the counter-targets population. Candidates F1A-795, F1A-935, and F1A-946 met this criterion very well, as they were not detected in the counter-target population. F1A-996 and F1A-961 are considered the next best candidates in this regard, although they do show up to a small degree in the counter-targets population. In addition, candidates should appear minimally in the negative population, as those sequences desorbed from GrO without the influence of acyclovir and could represent false positives. F1A-935 and F1A-946 performed ideally under this criterion as well, as they were not found in the negative population. Candidate F1A-769 was minimally detected in the negative population, with candidates F1A-961, F1A-795 and F1A-996 performing less well. Enrichment rate was the final condition to be considered, with F1A-935, F1A-946, and F1A-769 performing adequately. Candidate F1A-582 was included because it exhibited the greatest enrichment rate, although it did not perform well under the other criteria. The remaining candidates did not perform well relative to these four, but exhibited acceptable characteristics.

Additional target penciclovir produced seven strong candidates (SEQ ID NOs:94-100), each designated F1P (FIG. 18). As before, the goal was to identify aptamers from a library based on RNA that is known to bind to deoxyguanosine, diverging from libraries enriched for binding to acyclovir (acyclovir) after Round 10. Strong candidates needed to have minimal presence in both the acyclovir and the counter-targets populations to minimize cross-reactivity. Candidate F1P-923 met the first criterion, candidate F1P-710 met the second criterion, and candidate F1P-584 met both criteria to a degree. Candidate F1P-584 also demonstrated moderate favorability for penciclovir over the negative condition, as well as moderate enrichment relative to the previous generation's response to acyclovir. The remaining candidates demonstrated either minimal favoring of penciclovir over acyclovir or minimal favoring of penciclovir over counter-targets (F1P-837 and F1P-932; F1P-991 and F1P-718; respectively). These four candidates demonstrated some favorability for penciclovir over the negative condition which minimizes the chance of a false positive, although this criterion is not as significant if a candidate does not demonstrate selectivity for penciclovir over its analogues. Enrichment rate was the final condition to be considered, with F1P-923, F1P-932, and F1P-584 performing adequately.

Qualitative PAGE assessment of selected aptamers was performed. Individually synthesized and transcribed aptamers were subjected to selection on Graphene Oxide (GrO) under physiological Mg++ (0.5 mM) and elution with either acyclovir (+) or counter-targets (x). The specifically eluted aptamer fractions for each sample were subjected to PAGE for analysis.

100 pmoles of each aptamer candidate (per trial/lane) was resuspended in 1X modified selection buffer (50 mM HEPES, 100 mM KCl, 0.5 mM MgCl₂, pH 7.3) and refolded (90 °C for 5 min, then 4 °C for 5 min), then incubated at 37 °C for 30 minutes with 200 pmoles (each) of pooled counter-targets or target. Final library concentration was 0.5 µM, target/counter-targets concentration was 1 µM (incubation volume was 200 µl).

After target/counter-target incubation, 250 µg of GrO (Angstron Materials (Dayton, OH) was added to adsorb unbound candidate (10-minute incubation at 37 °C).

Samples were centrifuged for 5 minutes at 7,000 x g. Supernatant was recovered, denatured using 2X Formamide with 40 mM EDTA, and run on 10% denaturing PAGE with 8 M urea (supplier: American Bioanalytical; catalog #'s AB13021-01000. AB13022-01000). Running buffer was 1X TBE (supplier: Amresco/VWR; catalog # 0658-20L, diluted using DI water). DNA ladder was 20/100 DNA ladder (IDT). Gels stained with Gel Star (Lonza, 50535) and imaged on a blue light transilluminator.

Candidates F1A-769, F1A-795, F1A-946, and F1A-996 appear to exhibit selective positive response in this qualitative PAGE assessment (good elution of the Aptamer from GrO with Acyclovir target and relatively lower or minimal elution with counter-targets).

### Conclusion

Strong candidates for acyclovir were identified after twelve rounds of iterative screening and parallel assessment; reasonable candidates for penciclovir were identified after two rounds of screening and parallel assessment.

### Example 2. Isolation of conditional scFv's

Potential splice site liabilities are removed and tumor antigen specific scFv's are synthesized by overlapping oligo synthesis and cloned into the CAR shuttle construct containing the acyclovir responsive element and the primate CD3ζ promoter. As an initial prototype, anti-ECD of EPCAM or ERBB2scFv with a CD8-alpha signal peptide, stalk, and transmembrane domain is utilized. Solid tumor microenvironment restricted CAR products are generated either using methods as described in US Patent No. 8,709,755 and PCT Publication No. WO/2016/033331A1 or by direct selection from human phage libraries under permissive and non-permissive conditions. Briefly, a human V_{H} x V_{L} library from Creative Biolabs (Shirley, NY) is panned in the following tumor permissive conditions: 100 µg/ml hyaluronan, 100 kDa fraction (Lifecore Biomedical, Chaska, MN), 20 mg/ml recombinant HSA (Cyagen, Santa Clara, CA), 200 ng/ml recombinant human VEGF in 25 mM sodium bicarbonate buffer, 2 µM adenosine, 10 mM sodium lactate pH 6.7, following clearance with streptavidin magnetic beads (ThermoFisher, Carlsbad, CA) bound to biotinylated human IgG. Binding to biotinylated-target receptor ECD of EPCAM and ERBB2 conjugated beads at 37 °C is performed under permissive conditions followed by serial washes in permissive conditions. Phage are released with physiologic conditions (1 µg/ml hyaluronan, 20 mg/ml HSA, 25 mM bicarbonate, 1 mM sodium lactate pH 7.2) followed by elution of tight variants with acid elution and rapid neutralization with 1 M Tris. Phage are expanded and genomic DNA is split for deep sequence analysis of V_{H} x V_{L} chains using long read sequencing (PacBio, Menlo Park, CA). Panning can be repeated for enrichment. V_{H} x V_{L} sequences showing preferential amplification of reads during the phage culturing process over enrichment to target are excluded for further analysis. Phage with selective binding to the target that are enriched under tumor permissive conditions but released under physiologic conditions are chosen for further characterization by cloning into the CAR construct expression system, generation of lentivirus, and transduction into T cells for testing CAR-mediated tumor antigen expressing target cell killing in a tumor-selective environment compared to physiologic conditions.

### Example 3. Generation of MRB-CARs Using Microenvironment restricted scFv's.

Microenvironment restricted ASTRs were obtained that were made by subjecting V_{H} and V_{L} sequences with low selectivity for the low pH microenvironment by evolution as described in application WO/2016/033331A1. Chimeric antigen receptors (CARs) for binding either of two cognate tumor antigens, Target 1 or Target 2, with increased activity at the reduced pH of a tumor microenvironment compared to the microenvironment of normal tissue MRB-CARs were made by incorporating the heavy chains and light chains of the microenvironment restricted single-chain antibodies into lentiviral expression vectors along with other CAR domains to generate a series of candidate MRB-CARs. These MRB-CARs included various combinations of modules. The MRB-CARs included, from amino to carboxy terminus, in positions 1 through 9, a CD8 signal peptide (sp) (P1) (SEQ ID NO:74); a microenvironment restricted anti-Target 1 ASTR or anti-Target 2 ASTR (P2-P4); a stalk and transmembrane (TM) domain from CD8 (SEQ ID NO:75) (P5) and a co-stimulatory domain from CD137 (P6) (SEQ ID NO:1) in the cases of T2A and T2B or a stalk and transmembrane (TM) domain from CD28 (SEQ ID NO:76) (P5) and a co-stimulatory domain from ICΔ (SEQ ID NO:3) (P6) in the case of T1A; an activation domain from CD3Z (SEQ ID NO: 13) (P7); a 2A-1 ribosomal skip sequence (SEQ ID NO:77) (P8); and an exemplary eTAG (SEQ ID NO:78) (P9).

Pan T cells (AllCells, Alameda, CA) were transduced with the recombinant lentiviral particles to express the series of candidate MRB-CARs and the percent transduced cells was calculated by determining the percent of cells expressing the eTag using FACS. Pan T cells were successfully transduced with the recombinant lentiviral particles encoding the candidate MRB-CARs.

The cytotoxic activity of the candidate MRB-CARs against target cells expressing either Target 1 or Target 2 (CHO-Target 1 and CHO-Target 2, respectively) was analyzed at a pH of 7.4 (normal tissue) or a pH of 6.7 (reduced pH of a tumor microenvironment) by xCELLigence System (ACEA). Briefly, target cells expressing Target 1 or Target 2 were seeded to a 96-well E-plate at 20,000 cells/well with tumor conditional or normal medium one day before the experiment. Effector cells were rested for two days in human T cell medium containing 100 IU/mL of IL-2 and added into experimental wells containing Target cells at effector cell/target cell ratios (E/T) of 3:1, 1:1, and 0.3:1.

Impedance readings were taken every 5 minutes for approximately 40 hours after effector cell addition and impedance was reported as the Cell Index (CI). Percentage of specific cytolysis was calculated as follows ((CI Target + Control virus transduced effector T cells) - (CI Target + effector T cells transduced with CARs directed to Target 1 or Target 2)) / (CI Target + Control virus transduced effector T cells) ×100.

### Results

Many of the candidate MRB-CARs had higher cytotoxic activity on the target cells at a pH of 6.7 than at a pH of 7.4. Exemplary MRB-CARs that were more effective at lysing target cells at a pH of 6.7 than at a pH of 7.4 included MRB-CAR T1A, MRB-CAR T2A, and MRB-CAR T2B. The ASTR of MRB-CAR T1A comprised, from 5' to 3', Target 1 MRB VH (SEQ ID NO:281) and Target 1 MRB VL (SEQ ID NO:282) separated by Linker 1 (SEQ ID NO:283). The ASTR of MRB-CAR T2A comprised, from 5' to 3', Target 2 MRB VH (SEQ ID NO:289) and Target 2 MRB VL (SEQ ID NO:290) separated by Linker 2 (SEQ ID NO:291). The ASTR of MRB-CAR T2B was the same as that for MRB-CAR T2A except that the positions of VH and VL were swapped.

### Example 4. Construction of ligand-inducible riboswitches.

Deoxyguanosine riboswitch aptamer and guanine riboswitch aptamers (Pikovskaya, 2014; Kim, 2007) or other purine riboswitch aptamers are synthesized as oligonucleotides. In one example, the deoxyguanosine IA riboswitch from *Mesoplasma florum* (underlined and in bold in FIG. 6; FIG. 7) is selected for evolution to generate an acyclovir-responsive riboswitch. In another example, the guanine xpt riboswitch from *Bacillus subtilis* (underlined and in bold in FIG. 10; FIG. 11) is selected for evolution to generate an acyclovir-responsive riboswitch. For each of these two examples, a random RNA library is generated with alternate nucleotides at targeted sequence positions in the P2, P3, J1-2, and J2-3 segments (FIGs. 7 and 11). Each segment allows for 3 alternate nucleic acids at each targeted sequence position, or alternatively base deletion and insertion of 4 nucleotides in the +1 site at each targeted sequence position for saturation mutagenesis as indicated in FIGs. 8A-8B and 9 (*M. florum* IA) and FIGs. 12A-12B and 13 *(B. subtilis* xpt). Primer extension and reagent preparation is followed by RNA transcription. The resultant RNA library is negatively selected on graphene oxide in the presence of guanine, guanosine, and deoxyguanosine followed by positive selection with acyclovir or penciclovir. During the negative and positive selection processes, human cell physiologic magnesium levels (0.5 mM to 1.2 mM) are used and the temperature is kept at 37 °C. Recovered aptamers are reverse transcribed and PCR amplified followed by transcription and subsequent screening for at least 8 successive rounds of selection. In a parallel approach, aptamers are screened with an additional negative screen at 40 °C. Resultant positive pools are examined by NextGen sequencing and analysis. Individual aptamers are synthesized and examined for affinity by isothermal calorimetry at 35-40 °C in human cell physiologic magnesium levels. Following selection for positive acyclovir and penciclovir specific aptamers, aptamers are integrated with ribozyme hammerhead and pistol ribozymes. Positive acyclovir selective aptamers are combined with pistol ribozymes to identify acyclovir regulated ribozymes. (Harris KA RNA. 2015 Nov;21(11):1852-8. doi: 10.1261/rna). Variants are subjected to gel shift based PAGE purification in the presence of acyclovir and absence of penciclovir. Additionally, the acycloguanosine selective riboswitch is placed immediately 3' in a loop to a splice acceptor upstream of the CAR/IL-7 construct. In the absence of acyclovir, the splice site position is bound in the riboswitch complex but in the presence of acyclovir becomes accessible, generating a functional CAR transcript.

### Example 5. Construction of in vivo propagation domains.

A series of constitutively active IL7 receptor (IL7R) transmembrane mutants from T cell lymphoblastic leukemias (243 InsPPCL (SEQ ID NO:82); 246 InsKCH (SEQ ID NO:101); 241 InsFSCGP (SEQ ID NO:102); 244 InsCHL (SEQ ID NO:103); and 244 InsPPVCSVT (SEQ ID NO:104); all from Shochat et al 2011, J. Exp. Med. Vol. 208 No. 5 901-908) are synthesized by overlapping oligo nucleotide synthesis (DNA2.0, Newark, California). The synthesized constitutively active IL7R transmembrane mutants are inserted into a constitutively expressing lentiviral vector backbone immediately behind a 2A ribosomal skip sequence followed by an anti-CD19 CD3ζ expression cassette, which includes a CD8A stalk (SEQ ID NO:79) and a leader peptide (SEQ ID NO:74). HEK293 packaging cells are transfected with the IL7R transmembrane mutant lentiviral vectors and lentiviral packaging constructs, grown, and viral supernatants are harvested using methods known in the art. CD3/CD28-stimulated T cells are transduced with the viral supernatants and grown in IL2 deficient AIM V, CTS OpTmizer T Cell Expansion SFM, or X-VIVO 15 media for 4 weeks, supplemented weekly with frozen PBMCs from the same donor. The resulting expanded transduced T cells expressing IL7R variants are cloned by FACS sorting and the sequences of the IL7R constructs are identified by sequencing RT-PCR products. The 243 InsPPCL variant (PPCL) (SEQ ID NO:82) is selected for further evolution to generate a conditionally active CAR.

### Example 6. Screening of accessory components for CAR-T activation and propagation.

A series of protein-encoding domains (ABCG1, SOCS1, SMAD2, TGFBR2, cCBL, and PD1) and miRNA sequences are constructed for incorporation into a synthetic intron on the reverse strand of a CD3-promoter driven CAR cassette. Each construct containing the CD3-promoter driven CAR cassette and a protein-encoding domain or miRNA sequence includes a unique bar code for deep sequencing and is assembled using Gibson assembly followed by transformation and library expansion in E. *coli.* Viral stocks are produced and used to transduce CD3/CD28-stimulated T cells in AIM V, CTS OpTmizer T Cell Expansion SFM, or X-VIVO 15 media without IL2 and allowed to grow for 4 weeks in culture with serial sampling of DNA for amplification and deep sequencing for code identification. The library is also subject to PACBio full length sequencing to determine library diversity and to decode the bar code components. The miRNA sequences and protein-encoding domains are tested for synergistic activation of CAR CD3ζ domains.

### Example 7. Engineering a retroviral packaging and transducing system to target resting T cells for selective T cell integration and expression from PBMCs.

Although producing high-titer lentiviral vectors by transient transfection is possible, this method carries the risk of generating replication competent retroviruses (RCRs) and is not scalable for clinical applications. Herein, a stable retroviral packaging cell line is generated by the simultaneous introduction of multiple constructs encoding inducible promoters and their regulators into HEK293 suspension-adapted cells (HEK293S) to stably produce the viral components, CAR genes, and their regulatory components. Two distinct inducible systems can be used to temporally control the expression of genes. One system is based on rapamycin- or rapalog-induced dimerization of two transcription factors. One transcription factor consists of three copies of the FKPB protein fused to a ZFHD1 DNA binding domain and the other transcription factor consists of a FRB protein fused to a p65 activation domain. Rapamycin or a rapalog dimerizes the transcription factors to form ZFHD1/p65 AD and can activate gene transcription at 12xZFHD1 binding sites.

A series of vectors as shown in FIGs. 3A-3E are generated with flanking transposon sequences for integration into the HEK293S genome. Once integrated into the genome of a cell, these sequences function as regulatory components and lox and/or FRT sites for subsequent integration using Cre and/or flp recombinases, herein referred to as landing pads. The initial 5 constructs contain polynucleotide sequences encoding puromycin resistance, GFP, RFP, and an extracellular MYC tag that is targeted to the cell membrane through an N-terminal PLss (bovine prolactin signal peptide) and anchored to the cell membrane through a platelet-derived growth factor receptor (PDGFR) C-terminal transmembrane anchoring domain. The initial 5 constructs can also include constitutive minimal CMV and minimal IL-2 promoters, a rapamycin-regulated ZFHD1-based promoter, a tetracycline-responsive element (TRE) promoter, or a bidirectional TRE (BiTRE) promoter. The construct in FIG. 3A contains a polynucleotide sequence encoding FRB domain fused to the NFκB p65 activator domain (p65 AD) and ZFHD1 DNA binding domain fused to three FKBP repeats that is constitutively expressed. The construct in FIG. 3A also includes HIV1 REV and HSV VP65 domain SrcFlagVpx under the rapamycin-inducible ZFHD1/p65 AD promoter. The construct in FIG. 3B includes a polynucleotide encoding an rtTA sequence under the control of the ZFHD1/p65 AD promoter. The construct in FIG. 3C includes a polynucleotide encoding a puromycin resistance gene flanked by loxP sites and the extracellular MYC tag flanked by lox2272 sites. Both of these selectable markers are under the control of a BiTRE promoter, which is flanked by FRT sites. The construct in FIG. 3D includes a polynucleotide encoding GFP flanked by loxP sites that is under the control of a TRE promoter. The construct in FIG. 3D also includes a single FRT site between the TRE promoter and the 5' loxP site of GFP. The construct in FIG. 3E includes a polynucleotide encoding RFP flanked by loxP sites that is under the control of the ZFHD1/p65 AD promoter. The construct in FIG. 3E also includes a single FRT site between the ZFHD1/p65 AD promoter and the 5' loxP site of RFP The constructs in FIGs. 3C-3E function as landing pads for other polynucleotide sequences to insert into the genome of the packaging cell line. The polynucleotide sequences to be inserted can be flanked by lox sites and inserted into the genome using Cre recombinase and the loxP sites. This results in insertion and simultaneous removal of the genomic regions encoding puromycin resistance, the extracellular MYC tag, GFP, and RFP. Alternatively, the polynucleotide sequences can be flanked by FRT sites and inserted into the genome using flp recombinase and the FRT sites followed by removal of the polynucleotide sequences encoding puromycin resistance, the extracellular MYC tag, GFP, and RFP using Cre recombinase.

To generate the packaging cell line with landing pads integrated into the genome, HEK293S cells are co-transfected with equimolar concentrations of the 5 plasmids (FIGs. 3A-3E) plus 5 µg of *in vitro-*transcribed piggybac transposase mRNA or 5 µg of a plasmid with a promoter for expressing piggybac transposase in the presence of PEI at a ratio of 2:1 or 3:1 PEI to DNA (w/w) or 2-5 µg piggybac transposase protein using a cationic peptide mixture. The transfected cells are selected with puromycin in the presence of 100 nm rapamycin and 1ug/mL doxycycline for 2-5 days followed by fluorescence-activated cell sorting to collect cells expressing GFP and RFP. The sorted cells are grown 5 days in the absence of puromycin, rapamycin, and doxycycline and cells expressing GFP and RFP are removed also myc positive cells are removed with myc beads. Individual clones from negatively sorted cells are then screened for induction of GFP and RFP by rapamycin and doxycycline and single cell cloned. The DNA from clones is harvested and sequenced for integration analysis. Clones positive for strong inducible expression of GFP and RFP in the presence of rapamycin and doxycycline with limited background expression in the absence of rapamycin and doxycycline are expanded and banked.

The HEK293S cells with the constructs from FIGs. 3A-3E integrated into the genome are then transfected with a construct containing a tricistronic polynucleotide encoding a DAF signal sequence/anti-CD3 scFvFc (UCHT1)/CD14 GPI anchor attachment site (SEQ ID NO:287), a DAF signal sequence/CD80 extra-cellular domain capable of binding CD28/CD16B GPI anchor attachment site (SEQ ID NO:286), and a DAF signal sequence/IL-7 /DAF (SEQ ID NO:286) and transposon sequences flanking the polynucleotide region for integration into the HEK293S genome (FIG. 4A). After transfection, cells are expanded for 2 days in the absence of rapamycin and doxycycline and colonies that are constitutively red are selected. Positive colonies are then transiently transfected with a construct for expressing Cre recombinase to remove remaining genomic DNA, and the RFP encoding region. Another construct (FIG. 4B) containing a polynucleotide with a BiTRE promoter and a polynucleotide region encoding the gag and pol polypeptides in one direction and a polynucleotide region encoding the measles virus F and H proteins in the other direction is transfected at the same time. The Cre recombinase integrates the construct into the genome to generate the integrated sequence shown in FIG. 4B. Resultant colonies are evaluated for protein expression in the presence of doxycycline and rapamycin and analyzed by deep sequencing for genomic integration. The remaining TRE responsive GFP site is retained for the lentiviral genome insertion.

### Example 8. Generation of lentivirus vector and retroviral packaging.

The retroviral packaging stable cell line generated in Example 7 is transfected with a construct (FIG. 4C) for expressing Flp recombinase and a construct containing a polynucleotide sequence encoding a CAR and the lymphoproliferative element IL7Rα-insPPCL under the control of a CD3Z promoter that is not active in HEK293S cells, wherein the CAR and IL7Rα-insPPCL are separated by a polynucleotide sequence encoding a T2A ribosomal skip sequence and the IL7Rα-insPPCL has an acyclovir riboswitch controlled ribozyme. The CAR-containing construct further includes cPPT/CTS and RRE sequences and a polynucleotide sequence encoding HIV-1 Psi. The entire polynucleotide sequence on the CAR-containing construct to be integrated into the genome is flanked by FRT sites. Successful integration of the CAR-containing construct causes constitutive expression of GFP that is consequently removed by transient transfection with a construct for expressing Cre recombinase. The HEK293S line is grown in serum free media. Following growth to peak cell density in a stirred tank reactor, the cells are diluted to 70% peak cell density and treated with 100 nM rapamycin for 2 days to induce expression of early genes REV, Vpx, and aCD3 scFv CD16B GPI, aCD28 scFv CD16B GPI, and IL-7 SD GPI DAF followed by the addition of 1 ug/ mL doxycycline in the media to induce expression of structural elements like Gag Pol, MV(Ed)-FΔ30, MV(Ed)-HΔ18, and lentiviral genome including the therapeutic target. Levels of virus production are examined by qPCR of the packaging sequence and p24 ELISA. Virus is harvested by depth filtration of cells, and concentration/diafiltration using a TFF cartridge followed by flash freezing for vialing.

### Example 9. Peripheral blood mononuclear cell (PBMC) isolation, transduction, and expansion.

The following example illustrates the use of a closed system for *ex vivo* processing of PBMCs before *in vivo* expansion. As an example, 30 to 200 ml of human blood is drawn from a subject with Acid Citrate Dextrose Solution (ACD) as an anticoagulant into a blood collection bag. Alternatively, blood is drawn into Vacutainer tubes, a syringe, or an equivalent and is transferred to an empty blood collection or IV bag. The whole blood is processed using a Neat Cell kit (Cat # CS-900.2, Omniamed) on a Sepax 2 cell processing system (BioSafe) according to the manufacturers' instructions. The peripheral blood mononuclear cells (PBMCs) are collected either into a culture bag, or alternatively a syringe. An aliquot is taken aseptically for cell counting to determine the number of viable cells. The PBMCs are transferred to a G-Rex100MCS Gas Permeable Cell Culture System device (Wilson Wolf) at a final concentration of 0.1 - 1.0 x 10⁶ viable cells/ml in X-VIVO 15 (Cat # 08-879H, Lonza) or CTS OpTmizer Cell Expansion SFM (Cat # A1048501, Thermo Fisher Scientific) media with 10-300 IU/ml IL-2 (Cat # 202-IL-010, R&D Systems) in up to 200 ml final volume. In addition to IL-2, CTS Immune Cell SR (Cat # A2596101, Thermo Fisher Scientific) can be added to the media. The closed G-Rex Gas Permeable Cell Culture System device can be pre-coated with Retronectin (Cat # CH-296, Takara), or a similar fibronectin-derived equivalent, according to the manufacturer's instructions.

The PBMCs isolated from peripheral blood are loaded onto a PALL PBMC filter, washed once through the filter with 10 ml of AIM V (Thermo Fisher Scientific) or X-VIVO 15 media followed by perfusion with 10-60 ml of lentivirus stock (as prepared in Example 8) at 37 °C at 5 ml/hr. The PBMCs are then washed again with AIM V, CTS OpTmizer T Cell Expansion SFM, or X-VIVO 15 media containing recombinant human DNase (Pulmozyme, Genentech) followed by a wash with DNase-free Lactated Ringers (Cat # L7500, Braun). The PBMCs are then reverse perfused through the filter into a syringe. The cells (target levels of cells are 5 x 10⁵ to 1 x 10⁶ cells/kg) are then reinfused into the subject through intravenous infusion.

Depending upon the riboswitch contained within the retroviral genome, the subject is given the respective nucleoside analogue antiviral drug or nucleoside analogue antiviral prodrug (acyclovir, valaciclovir, penciclovir, or famciclovir). Subjects can be given any therapeutically effective dose, such as 500 mg of the nucleoside analogue antiviral drug or prodrug orally three times/day. Treatment with the nucleoside analogue antiviral drug or prodrug preferably begins before reinfusion, such as 2 hours before, and can also begin at the time of reinfusion or at some time after reinfusion. The treatment can continue for at least 1, 2, 3, 4, 5, 7, 10, 14, 21, 28, 30, 60, 90, 120 days or 5, 6, 9, 12, 24, 36, or 48 months or longer. The treatment can include administration of the nucleoside analogue antiviral drug or prodrug once, twice, three, or four times daily. After reinfusion and treatment is begun, the number of infected cells is determined through blood counts on days 2, 5, 7, 11, 13, 18, 28, and 56 post-reinfusion using qPCR to quantitate the amount of viral genome. A subject experiencing fever or cytokine release syndrome may have the dose or frequency of the nucleoside analogue antiviral drug or prodrug reduced or halted. If the infected T cells fail to amplify 10,000-100,000 fold by day 18, the dose or frequency of the nucleoside analogue antiviral drug or prodrug may be increased. The clinical response of the subject can be measured through FDG PET imaging and serial CT scan. Oral dosing of the nucleoside analogue antiviral drug or prodrug can be reduced or halted following prolonged remission or in the event of excessive T cell propagation beyond 30% of total peripheral T cell counts.

### Example 10. Therapeutic intervention to raise vascular or tissue pH.

To reduce the binding of an antigen binding domain to its cognate antigen, NaHCO₃ is administered as an IV bolus or by IV infusion. The standard dosage is 1 mg/kg of body weight as the initial dose followed by 0.5 mg/kg every 10 minutes. A 50-milliliter bolus of NaHCO₃ will raise the serum pH approximately 0.1 of a pH unit. If the pH is 7.0, it requires four 50 mEq ampules of NaHCO₃ to correct the pH to 7.40

### Example 11. Testing activity of IL-7 receptor lymphoproliferative/survival elements in PBMCs.

To test IL-7Rα variants for their ability to mediate cytokine-independent survival of T cells, thirty milliliters of human blood were drawn with acid citrate dextrose (ACD) as an anticoagulant into Vacutainer tubes. The whole blood was processed using density gradient centrifugation with Ficoll-Pacque^{™} (General Electric) following manufacturer's instruction, to obtain peripheral blood mononuclear cells (PBMCs). Aliquots of the PBMCs were transferred aseptically to wells of a 12 well tissue culture plate, along with X-Vivo^{™} 15 media (Lonza) to a final concentration of 0.5 million viable cells / mL in a final volume of 1mL. Recombinant human interleukin-2 (IL-2) (Novoprotein) was also added to a concentration of 100IU/ml in some samples. Activating anti-CD3 Ab (OKT3, Novoprotein) was added at a concentration of 50ng/ml, to activate the PBMC for viral transduction. The plates were incubated overnight in a standard humidified tissue culture incubator at 37 degrees C and 5% Carbon Dioxide. After overnight incubation, lentivirus particle preparations containing the desired test constructs (FIG. 19A) were added to individual wells at a multiplicity of infection (MOI) of 5. The plate was incubated overnight in a standard humidified tissue culture incubator at 37 degrees C and 5% Carbon Dioxide. Following the overnight incubation, the contents of each of the wells of the 12 well plate were collected and centrifuged to obtain a pellet. The samples were washed once with D-PBS + 2% Human Serum Albumin (HSA), resuspended in X-Vivo 15^{™} media, and transferred to wells of G-Rex^{®} 6-well gas permeable cell culture devices (Wilson Wolf). Additional X-Vivo^{™} 15 media was added to bring the final volume of each well to 30ml. Matching control samples for each of the constructs were transferred to wells of G-Rex^{®} 6-well gas permeable cell culture devices (Wilson Wolf) and additional media was added to bring the final volume to 30ml with 100IU/ml IL-2 for some control samples. The G-Rex^{®} device was incubated in a standard humidified tissue culture incubator at 37 degrees C and 5% Carbon Dioxide for 7 days. Fresh IL-2 was added to the control samples containing IL-2 during the culture every 2-3days. Matched test samples without IL-2 were not supplemented. Samples were removed for tracking cell numbers and viability during expansion (Countess, Thermo Fisher) at day 7.

FIG. 19A provides a schematic of the IL7Rα constructs that were tested. These constructs were inserted into a recombinant lentiviral genome. The replication incompetent recombinant retroviral particles were used to transduce PBMCs. FIG. 19A shows a schematic of wild-type IL7Rα (SEQ ID NO:229), which consists of a signal sequence (SS), an extracellular domain (ECD), a transmembrane (TM), and an intracellular domain (ICD). "1" indicates the site of a fibronectin type III domain; "2" indicates the site of a WSXWS motif"; "3" indicates a Box 1 site, "4" indicates the site of a protein kinase C (PKC) phosphorylation site, and "5" indicates a Box 2 site.

Variant "A" is the IL-7Rα with an InsPPCL at position 243 (Shochat et al 2011, J. Exp. Med. Vol. 208 No. 5 901-908) but without the S185C mutation, expressed on a transcript with a GFP polypeptide, a GSG linker, and a P2A ribosomal skip sequence fused to its N-terminus. Variant "B" is the IL-7Rα InsPPCL with a GFP polypeptide, a GSG linker, and a P2A ribosomal skip sequence fused to its N-terminus as well as a Myc Tag between the signal sequence and the extracellular domain. Variant "C" is similar to variant "B" except its intracellular domain is truncated at position 292. Variant "D" is similar to variant "A" except its intracellular domain is truncated at position 292. Variant "E" is the IL-7Rα InsPPCL variant truncated at its N terminus such that the signal sequence and most of the extracellular domain (residues 1-228) are not present; variant "E" also has a GFP polypeptide, a GSG linker, a P2A ribosomal skip sequence, and an eTag fused to the N terminus, in that order from the amino terminus. Numbering of the amino acid residues is based on IL7Rα (NCBI GI No. 002176.2). T cells containing each of the variants were tested for viability in the presence or absence of IL-2 using Trypan Blue exclusion.

As shown in FIG. 19B, PBMCs require IL-2 for survival *in vitro.* As illustrated in FIG. 19B, untransfected PBMCs have about 80% viability in the presence of IL-2 and 0% viability in the absence of IL-2 at 7 days after transduction. PBMCs having the full-length versions of IL-7Rα InsPPCL (IL-7Rα variants A and B in FIG. 19A) had over 20% viability in the absence of IL-2 7 days after transduction, indicating that expression of the constitutively active IL-7Rα InsPPCL receptor has survival activity in these cells. Furthermore, T cells expressing the IL-7Rα InsPPCL variants with a truncated intracellular domain (ICD) (IL-7Rα variants C and D in FIG. 19A) had increased viability at 7 days after transduction compared to the wild-type IL-7 receptor. Finally, the N-terminal IL-7 receptor mutant (IL-7Rα variant E in FIG. 19A) as shown in FIG. 19B had survival activity in these cells. Accordingly, this example illustrates that IL-7 receptor has survival activity when expressed in PBMCs.

### Example 12. Transduction efficiency of freshly isolated and unstimulated human T cells by retroviral particles pseudotyped with VSV-G and expressing anti-CD3 scFvFc on their surfaces.

Recombinant lentiviral particles were produced by transient transfection of 293T cells (Lenti-X^{™} 293T, Clontech) with separate lentiviral packaging plasmids encoding gag/pol and rev, and a pseudotyping plasmid encoding VSV-G. A third generation lentiviral expression vector (containing a deletion in the 3'LTR leading to self-inactivation) encoding GFP, an anti-CD19 chimeric antigen receptor, and an eTAG referred to herein as F1-0-03 (FIG. 20) was co-transfected with the packaging plasmids. The cells were adapted to suspension culture by serial growth in Freestyle^{™} 293 Expression Medium (ThermoFisher Scientific). The cells in suspension were seeded at 1 × 10⁶ cells/mL (30 mL) in a 125 mL Erlenmeyer flask, and immediately transfected using polyethylenimine (PEI) (Polysciences) dissolved in weak acid.

Plasmid DNA was diluted in 1.5 ml Gibco^{™} Opti-MEM^{™} media for 30 mL of cells. To obtain lentiviral particles pseudotyped with VSV-G, the total DNA (1 µg/mL of culture volume) used was a mixture of 4 plasmids with the following molar ratios: 2x genomic plasmid (F1-0-03), 1x Rev-containing plasmid, 1x VSV-G-containing plasmid, and 1x gag/pol-containing plasmid. To obtain lentiviral particles pseudotyped with VSV-G and expressing an antiCD3-scFvFc-GPI on their surfaces, the total DNA (1 µg/mL of culture volume) used was a mixture of 5 plasmids with the following molar ratios: 2x genomic plasmid (F1-0-03), 1x Rev-containing plasmid, 1x VSV-G-containing plasmid, 1x anti-CD3-scFvFc-GPI-containing plasmid, and 1x gag/pol-containing plasmid. To obtain lentiviral particles pseudotyped with VSV-G and expressing anti-CD3-scFvFc-GPI and CD80-GPI on their surfaces, the total DNA (1 µg/mL of culture volume) used was a mixture of 6 plasmids with the following molar ratios: 2x genomic plasmid (F1-0-03), 1x Rev-containing plasmid, 1x VSV-G-containing plasmid, 1x anti-CD3-scFvFc containing plasmid, 1x CD80-containing plasmid, and 1x gag/pol-containing plasmid. Separately, the PEI was diluted in 1.5 ml Gibco^{™} Opti-MEM^{™} to 2 µg/mL (culture volume, 2:1 ratio to DNA). After a 5-minute room temperature incubation, the two solutions were mixed together thoroughly, and incubated at room temperature for 20 more minutes. The final volume (3 ml) was added to the cells. The cells were then incubated at 37 °C for 72 hours with rotation at 125 rpm and with 8% CO₂. The antiCD3-scFvFc-GPI containing plasmids included scFvs derived from either OKT3 or UCHT1, and a GPI anchor attachment sequence. The UCHT1scFvFc-GPI vector encodes a peptide (SEQ ID NO:278) that includes human Ig Kappa signal peptide (amino acids 1-22 of NCBI GI No. CAA45494.1) fused to the UCHT1 scFv (amino acids 21-264 of NCBI GI No. CAH69219.1), fused to human IgG1 Fc (amino acids 1-231 of NCBI GI No. AEV43323.1) with an A to T substitution at position 115, fused to the human DAF GPI anchor attachment sequence (amino acids 345-381 of NCBI GI No. NP_000565). The OKT3scFvFc-GPI vector encodes a peptide (SEQ ID NO:279) that includes a human Ig Kappa signal peptide (amino acids 1-22 of NCBI GI No. CAA45494.1) fused to the OKT3 scFv (SEQ ID NO:285) fused to human IgG1 Fc (amino acids 1-231 of NCBI GI No. AEV43323.1) fused to the human DAF GPI anchor attachment sequence (amino acids 345-381 of NCBI GI No. NP_000565). The CD80-GPI vector encodes a peptide (SEQ ID NO:280) that includes the human CD80 signal peptide and extracellular domain (amino acids 1-242 of NCBI GI No. NP_005182) fused to the human CD16b GPI anchor attachment sequence (amino acids 196-233 of NCBI GI No. NP_000561).

After 72 hours, the supernatants were harvested and clarified by centrifugation at 1,200g for 10 minutes. The clarified supernatants were decanted to a new tube. The lentiviral particles were precipitated by overnight centrifugation at 3,300g, at 4 °C. The supernatant was discarded, and the lentiviral particle pellets were resuspended in 1:100 of initial volume of X-Vivo^{™} 15 medium (Lonza). Lentiviral particles were titered by serial dilution and analysis of GFP expression, in 293T and Jurkat cells, 72 hours post-transduction, by flow cytometry.

Peripheral blood mononuclear cells (PBMCs) were first isolated from either fresh blood in ACD (acid citrate dextrose) tubes, for Donors 12F and 12M, or from a buffy coat for Donor 13F, collected and distributed by the San Diego Blood Bank, CA. SepMate^{™} 50 (Stemcell^{™})-based gradient density separation of PBMCs on Ficoll-Paque PLUS^{®} (GE Healthcare Life Sciences) was performed per manufacturers' instructions. 30 mL of blood or buffy-coat diluted in PBS-2%HIFCS (heat inactivated fetal calf serum) were layered per each SepMate^{™} tube. After centrifugation at room temperature, at 1,200g, for 20 min, the PBMC layers were collected, pooled and washed three times with 45 mL of PBS-2%HIFCS and centrifugation at 400g for 10 min at room temperature. The pellets were then incubated at room temperature for 10 min in 10 mL of RBC lysis buffer (Alfa Aesar) and washed an additional two times with 45 mL of PBS-2%HIFCS, and centrifugation at 400g for 10 min at room temperature. A final wash was performed in the transduction and culture media: X-Vivo^{™} 15 for Donor 13F, or RPMI-1640+10%HIFCS for Donors 12F and 12M. No additional steps were taken to remove monocytes. After isolation, fresh and unstimulated PBMCs were resuspended to a final concentration of 1 x 10⁶/mL in their respective medium, and were transduced, in duplicates or triplicates, with the lentiviral particles disclosed previously. The transductions were conducted for 14h, at 37°C, 5% CO₂, in X-Vivo^{™} 15 medium for Donor 13F, or in RPMI-1640+10%HIFCS for Donors 12F and 12M. Transductions were usually conducted at MOI 1 in a 12 wells plate format, 1 mL/well. For the kinetic experiment, 0.5 x 10⁶ PBMCs/mL were transduced in 7 mL final, at MOI 1, in a 125 mL shake flask incubated at 37 °C for 2-20h hours with rotation at 125 rpm and with 8% CO₂. After incubation with the retroviral particles for the selected time, the cells were washed three times with X-Vivo^{™} 15 medium for Donor 13F, or PBS+2%HIFCS for Donors 12F and 12M, and finally incubated at a cell density of 1x 10⁶/mL in X-Vivo^{™} 15 medium for Donor 13F, or RPMI-1640+10%HIFCS for Donors 12F and 12M, at 37°C, 5% CO₂. Starting at day 3 post transduction, and every 2-3 days from then on, the volume of the medium was doubled and supplemented with IL-2 to a final concentration of 100 U/mL. Samples were collected at various days post-transduction (Day 3-17) to evaluate, by GFP expression levels, the transduction efficiencies of each type of lentivirus that was generated.

At various days post-transduction, for lentiviral particles pseudotyped with VSV-G, with or without OKT3 antibody (Biolegend) at 1 µg/ml; lentiviral particles pseudotyped with VSV-G and expressing anti-CD3 scFvFc-GPI on their surface; or lentiviral particles pseudotyped with VSV-G and expressing anti-CD3 scFvFc-GPI and CD80-GPI on their surface; 100 µL of cells were collected and analyzed by flow cytometry for expression of GFP in the CD3+ cell population.

FIGs. 21A and FIG. 21B show a histogram of the percentage (%) CD3+GFP+ cells in the total CD3+ population and a histogram of the absolute cell count per well of the CD3+GFP+ population, respectively, at 3, 6, 9, 13 and 17 days after transduction of freshly isolated and unstimulated PBMCs from Donor 12M, for 14h with the indicated lentiviral particles. Each bar represents the mean +/- SD of duplicates. FIGs. 21A and 21B show that pseudotyping lentiviral particles with VSV-G and expressing antiCD3-scFvFc on the surface of the lentiviral particles effectively transduces freshly isolated and unstimulated PBMCs. Anti-CD3 scFv's derived from either OKT3 or UCHT1, when in the form of an scFvFc-GPI, were effective.

FIGs. 22A and FIG. 22B show a histogram of the percentage (%) CD3+GFP+ cells in the total CD3+ population and a histogram of the absolute cell count per well of the CD3+GFP+ population, respectively, at 3 and 6 days after transduction of freshly isolated and unstimulated PBMCs from Donor 13F, for 14h, with the indicated lentiviral particles. Please note that "A" are results using VSV-G pseudotyped lentiviral particles (triplicate experiments); "B" are results using VSV-G pseudotyped lentiviral particles with OKT3 antibody (1ug/mL) added to the transduction medium (duplicate experiments); "C" are results using VSV-G pseudotyped lentiviral particles expressing GPI-anchored UCHT1scFvFc on their surface (triplicate experiments); and "D" are results using VSV-G pseudotyped lentiviral particles expressing GPI anchored UCHT1scFvFc and GPI-anchored CD80 on their surface (duplicate experiments). Each bar represents the mean +/- SD of duplicates or triplicates, as indicated in FIG. 22A. FIGs. 22A and 22B show that pseudotyping lentiviral particles with VSV-G and expressing antiCD3-scFvFc-GPI and CD80-GPI on their surfaces also effectively transduces freshly isolated and unstimulated PBMCs when the transduction is performed for 14 hours.

FIGs. 23A and 23B show a histogram of percentage (%) CD3+GFP+ cells in the total CD3+ population and a histogram of the absolute cell count per well of the CD3+GFP+ population, respectively, at 3, 6 and 9 days after transduction of freshly isolated and unstimulated PBMCs from Donor 12M for the indicated time of exposure (2-20h) (i.e contacting of cells with lentiviral particles in transduction reaction mixture), with the indicated lentiviral particles. Transduction was performed in a plate or a shaker flask as indicated. Each bar represents the mean +/- SD of duplicates for lentiviral particles pseudotyped with VSV-G ("[VSV-G]"); the other experiments did not have replicates. FIGs. 23A and 23B show that freshly isolated and unstimulated PMBCs can be effectively transduced in as few as 2 hours with lentivirus particles pseudotyped with VSV-G and expressing anti-CD3 scFvFc and CD80 on their surfaces.

It is noteworthy that subsequent experiments were performed using lentiviral particles produced using only Rev, VSV-G, gag/pol, and F1-0-03 (and not a vector encoding an antiCD3 or CD80) to transduce freshly isolated resting PBMCs in the presence of soluble anti-CD3 antibodies (100ng/ml) for 2.5 hours. Transduction was achieved using 3 different soluble anti-CD3 antibody clones. Transduction efficiencies as measured by CD3+GFP+ cells at Day 6 were 4.31%, 3.27%, and 0.52% for CD3 antibody clones HIT3A, OKT3, and UCHT1, respectively. Background transduction efficiencies in the absence of soluble antibodies was 0.06%.

### Example 13. Functionality of miRNAs inserted into the EF-1alpha promoter intron.

Four separate gBlocks^{®} Gene Fragments were designed, each containing a miR-155 framework, including a miR-155 5' flanking sequence or "5' arm" (SEQ ID NO:256) and a miR-155 3' flanking sequence or "3' arm" (SEQ ID NO:260). For each gBlock^{®}, a unique miRNA fragment targeting the CD3zeta mRNA transcript was used to replace the miR-155 stem-loop precursor. Each gBlock^{®} contained a 40bp overlap sequence designed to facilitate assembly of all four gBlocks^{®} as a single chain into the EF-1alpha promoter intron. The gBlocks^{®} were assembled using a commercial kit for performing Gibson^{®} assembly ultra (NEBuilder, New England Biolabs, Inc.).

The synthetic EF-1alpha promoter and intron A containing the miRNAs (in SEQ ID NO:255) was part of a transgene expression cassette driving expression of GFP and eTag contained in a lentivirus vector backbone (the lentivirus vector backbone with the GFP and exemplary eTag recognized by cetuximab is referred to herein as F1-0-02; FIGs. 24A and 24B). The nucleotide positions of each gBlock^{®} and its respective components in SEQ ID NO:255 are denoted in Table 3 as are the positions of each "Feature" in FIG. 24B. Proper assembly of four miRNA into the lentivirus vector backbone was confirmed by comprehensive sequencing of the modified EF-1alpha promoter and intron region.

**Table 3. Nucleotide positions of features in SEQ ID NO:255**

| **Feature** | **Nucleotid e positions in SEQ ID NO:255** | **SEQ ID NO:** | **Featur e in** **FIG. 24B** |
|---|---|---|---|
| **gBlock^{®} 1** | 927-1138 | | |
| EF1alpha overlap | 927-966 | | 1 |
| miR155 - 5' arm | 967-994 | SEQ ID NO:256 CTGGAGGCTTGCTGAAGGCTGTATGCTG | 2 |
| miRNA1 - 5' Stem | 995-1015 | SEQ ID NO:257 ACATGGTACAGTTCAATGGTG | 3 |
| miR loop | 1016-1034 | SEQ ID NO:258 GTTTTGGCCACTGACTGAC | 4 |
| miRNA1 - 3' Stem | 1035-1053 | SEQ ID NO:259 CACCATTGCTGTACCATGT | 5 |
| miR155 - 3' arm | 1054-1098 | SEQ ID NO:260 | 6 |
| | | CAGGACACAAGGCCTGTTACTAGCACTCACATGG AACAAATGGCC | |
| **gBlock^{®} 2** | 1099-1310 | | |
| 40bp 50% GC Linker 1 | 1099-1138 | | 7 |
| miR155 - 5' arm | 1139-1166 | SEQ ID NO:256 | 2 |
| miRNA2 - 5' Stem | 1167-1187 | SEQ ID NO:261 TCAGTCTGTTCATCTTCTGGC | 8 |
| miR loop | 1188-1206 | SEQ ID NO:258 | 4 |
| miRNA2 - 3' Stem | 1207-1225 | SEQ ID NO:262 GCCAGAAGGAACAGACTGA | 9 |
| miR155 - 3' arm | 1226-1270 | SEQ ID NO:260 | 6 |
| **gBlock^{®} 3** | 1271-1482 | | |
| 40bp 50% GC Linker 2 | 1271-1310 | | 7 |
| miR155 - 5' arm | 1311-1338 | SEQ ID NO:256 | 2 |
| miRNA3 - 5' Stem | 1339-1359 | SEQ ID NO:263 AAGCGTGAAGTGAATCAACGG | 10 |
| miR loop | 1360-1378 | SEQ ID NO:258 | 4 |
| miRNA3 - 3' Stem | 1379-1397 | SEQ ID NO:264 CCGTTGATACTTCACGCTT | 11 |
| miR155 - 3' arm | 1398-1442 | SEQ ID NO:260 | 6 |
| **gBlock^{®} 4** | 1443-1654 | | |
| 40bp 50% GC Linker 4 | 1443-1482 | | 7 |
| miR155 - 5' arm | 1483-1510 | SEQ ID NO:256 | 2 |
| miRNA4 - 5' Stem | 1511-1531 | SEQ ID NO:265 GCAGTATCCTAGTACATTGAC | 12 |
| miR loop | 1532-1550 | SEQ ID NO:258 | 4 |
| miRNA4 - 3' Stem | 1551-1569 | SEQ ID NO:266 GTCAATGTTAGGATACTGC | 13 |
| miR155 - 3' arm | 1570-1614 | SEQ ID NO:260 | 6 |
| EF-1alpha overlap | 1615-1654 | | |

Replication incompetent lentiviral particles containing a nucleic acid encoding the four miRNAs directed against CD3zeta in their genome were produced by transient co-transfection of four plasmids into suspension HEK293 cells: a plasmid containing the nucleic acid encoding F1-0-02 modified to include the four miRNAs targeting the CD3zeta mRNA transcript, a plasmid encoding VSV-G, a plasmid encoding REV, and a plasmid encoding GAG-POL. Lentiviral particle supernatant was harvested after 48 hours and PEG-precipitated for 24 hours. Supernatants were centrifuged, and pelleted lentivirus particles were resuspended in complete PBMC growth media without IL-2. Lentivirus particle titers were calculated by 48 hour transduction of Jurkat cells.

For transduction, PBMCs were thawed on Day 0 and incubated for 24 hours with 100U/mL of hrIL-2. On Day 1, PBMCs were activated via CD3/CD28 conjugated beads. On Day 2, activated PBMCs were transduced with the lentiviral particles containing a genome with a nucleic acid sequence encoding the miRNAs at an MOI of 10. Cells were expanded until Day 11, with fresh hrIL-2 added every two days. On days 7, 9, and 11, 1 million cells were harvested for FACS analysis.

Cells were stained for CD3 Epsilon surface expression, using PE conjugated OKT-3 antibody (Biolegend). Expression levels were determined by the mean fluorescence intensity (MF) of PE in the GFP positive population *(transduced cells).* Expression levels of transduced cells were compared between retroviral particles derived from F 1-0-02 and retroviral particles derived from F 1-0-02 in which the nucleic acid sequence encoding the CD3z miRNAs positioned in series were inserted into the EF-1alpha promoter and intron A.

Results are shown in FIG. 25. This data shows that serial miRNAs targeting CD3zeta encoded by a nucleic acid sequence within the EF-1alpha promoter intron A, are effective at knocking down expression of the CD3 complex.

### Example 14, Positional independence of serial inhibitory RNAs inserted into the EF-1alpha promoter intron.

### Cloning

Four miRNA-expressing lentiviral vector constructs were designed to test the processing of individual miRNA precursors in a structure comprising 4 miRNA precursors in series. Table 4 shows the names of the individual constructs and the position of the miR-TCRα in each construct.

**Table 4. Constructs containing polycistronic miRNAs**

| **Construct** | **Position 1** | **Position 2** | **Position 3** | **Position 4** |
|---|---|---|---|---|
| TCRa-P1 | miR-TCRa | miR-155 | miR-PD-1 | miR-CTLA-4 |
| TCRa-P2 | miR-155 | miR-TCRa | miR-PD-1 | miR-CTLA-4 |
| TCRa-P3 | miR-155 | miR-PD-1 | miR-TCRa | miR-CTLA-4 |
| TCRa-P4 | miR-155 | miR-CTLA-4 | miR-PD-1 | miR-TCRa |

Each miRNA contained the miR-155 framework used in Example 13, i.e. a miR-155 5' arm (SEQ ID NO:256), a miR-155 3' arm (SEQ ID NO:260), a loop (SEQ ID NO:258), and a specific order of stem sequences as shown in Table 5. The type IIs assembly method was used to achieve assembly of the four miRNA fragments into their appropriate positions within the EF-1alpha intron of the lentivirus vector construct (F1-0-02; provided in Example 13 and shown in FIG. 24A).

**Table 5: Sequences in miRNA constructs**

| **TCRa-P1 (SEQ ID NO:278)** | **TCRa-P2 (SEQ ID NO:279)** | **TCRa-P3 (SEQ ID NO:280)** | **TCRa-P4 (SEQ ID NO:281)** | |
|---|---|---|---|---|
| 5' arm | SEQ ID NO:256 | SEQ ID NO:256 | SEQ ID NO:256 | SEQ ID NO:256 |
| miRNA1 - 5' Stem | SEQ ID NO:267 | SEQ ID NO:270 | SEQ ID NO:270 | SEQ ID NO:270 |
| miR loop | SEQ ID NO:258 | SEQ ID NO:258 | SEQ ID NO:258 | SEQ ID NO:258 |
| miRNA1 - 3' Stem | SEQ ID NO:268 | SEQ ID NO:271 | SEQ ID NO:271 | SEQ ID NO:271 |
| 3' arm | SEQ ID NO:260 | SEQ ID NO:260 | SEQ ID NO:260 | SEQ ID NO:260 |
| Linker 1 | SEQ ID NO:269 | SEQ ID NO:269 | SEQ ID NO:269 | SEQ ID NO:269 |
| 5' arm | SEQ ID NO:256 | SEQ ID NO:256 | SEQ ID NO:256 | SEQ ID NO:256 |
| miRNA2 - 5' Stem | SEQ ID NO:270 | SEQ ID NO:267 | SEQ ID NO:273 | SEQ ID NO:276 |
| miR loop | SEQ ID NO:258 | SEQ ID NO:258 | SEQ ID NO:258 | SEQ ID NO:258 |
| miRNA2 - 3' Stem | SEQ ID NO:271 | SEQ ID NO:268 | SEQ ID NO:274 | SEQ ID NO:277 |
| 3' arm | SEQ ID NO:260 | SEQ ID NO:260 | SEQ ID NO:260 | SEQ ID NO:260 |
| Linker 2 | SEQ ID NO:272 | SEQ ID NO:272 | SEQ ID NO:272 | SEQ ID NO:272 |
| 5' arm | SEQ ID NO:256 | SEQ ID NO:256 | SEQ ID NO:256 | SEQ ID NO:256 |
| miRNA3 - 5' Stem | SEQ ID NO:273 | SEQ ID NO:273 | SEQ ID NO:267 | SEQ ID NO:273 |
| miR loop | SEQ ID NO:258 | SEQ ID NO:258 | SEQ ID NO:258 | SEQ ID NO:258 |
| miRNA3 - 3' Stem | SEQ ID NO:274 | SEQ ID NO:274 | SEQ ID NO:268 | SEQ ID NO:274 |
| 3' arm | SEQ ID NO:260 | SEQ ID NO:260 | SEQ ID NO:260 | SEQ ID NO:260 |
| Linker 3 | SEQ ID NO:275 | SEQ ID NO:275 | SEQ ID NO:275 | SEQ ID NO:275 |
| 5' arm | SEQ ID NO:256 | SEQ ID NO:256 | SEQ ID NO:256 | SEQ ID NO:256 |
| miRNA4 - 5' Stem | SEQ ID NO:276 | SEQ ID NO:276 | SEQ ID NO:276 | SEQ ID NO:267 |
| miR loop | SEQ ID NO:258 | SEQ ID NO:258 | SEQ ID NO:258 | SEQ ID NO:258 |
| miRNA4 - 3' Stem | SEQ ID NO:277 | SEQ ID NO:277 | SEQ ID NO:277 | SEQ ID NO:268 |
| 3' arm | SEQ ID NO:260 | SEQ ID NO:260 | SEQ ID NO:260 | SEQ ID NO:260 |
| where: | | | | |
| SEQ ID NO:267 = TCRalpha miRNA Stem 1; ATATGTACTTGGCTGGACAGC | | | | |
| SEQ ID NO:268 = TCRalpha miRNA Stem 2; GCTGTCCACAAGTACATAT | | | | |
| SEQ ID NO:269 = Linker1; CACATTGGTGCCGGATGAAGCTCTTATGTTGCCGGTCAT | | | | |
| SEQ ID NO:270 = mir-155 Stem 1; CTGTTAATGCTAATCGTGATA | | | | |
| SEQ ID NO:271 = mir-155 Stem 2; TATCACGATTATTAACAG | | | | |
| SEQ ID NO:272 = Linker2; GTTGCCGGAGTCTTGGCAGCGAGAGATCACTATCAACTAA | | | | |
| SEQ ID NO:273 = PD-1 miRNA Stem 1; TACCAGTTTAGCACGAAGCTC | | | | |
| SEQ ID NO:274 = PD-1 miRNA Stem 2; GAGCTTCGCTAAACTGGTA | | | | |
| SEQ ID NO:275 = Linker3; GTGTTAATTGTCCATGTAGCGAGGCATCCTTATGGCGTGG | | | | |
| SEQ ID NO:276 = CTLA-4 miRNA Stem 1; TGCCGCTGAAATCCAAGGCAA | | | | |
| SEQ ID NO:277 = CTLA-4 miRNA Stem 2; TTGCCTTGTTTCAGCGGCA | | | | |

### Lentiviral Particle Production

The four constructs and the control F1-0-02, which includes no nucleic acid sequence encoding a miRNA, were used to produce lentiviral particles in 30mL suspension cultures of 293T cells. The lentiviral particles were harvested and concentrated by PEG precipitation. Functional lentiviral particle titers were obtained by transducing Jurkat cells at multiple dilutions (1: 1000, 1: 10000, 1:100000), incubating the lentiviral particles and cells for 2 days at 37°C, washing the cells 2X with FACS buffer, and analyzing for GFP by flow cytometry. Other details regarding lentiviral particle production are provided in Example 13 herein.

### Transduction

For transduction, PBMCs were thawed and recovered overnight in complete media containing 100U/mL hrIL-2. 1 x 10⁵ PBMCs were activated via exposure to CD3/CD28 conjugated beads for 24 hours. Cells were transduced in duplicate wells with each of the four miRNA constructs or with control retroviral particle F1-0-02 at MOI 10. The cells were supplied with 100U/mL hrIL-2 every 3 days and expanded until day 10.

### FACS

Cells were harvested for FACS analysis which confirmed that the cells were transduced with the replication incompetent lentiviral vectors. The results showed that approximately equivalent amounts of miRNA containing virus were delivered to each well in the experiment.

### Cells-to-ct miRNA RT-qPCR and analysis

An RT-qPCR assay was designed to detect expression and processing of the miRNA precursors into mature processed miRs. Analysis was done by first normalizing all miR-TCRa ct values to the RNU48 internal control to produce ΔCt values. Next, the ΔCt values of each transduced sample were subtracted from the ΔCt of the non-transduced control to produce ΔΔCt. This value is representative of the amount of processed miR-TCRa miRNA in each transduced sample, relative to the non-transduced control.

As shown in FIG. 26, the RT-qPCR assay successfully detected processed miR-TCRα in samples transduced with miR-TCRα containing replication incompetent lentiviral particles. Furthermore, the results clearly indicate that there is no remarkable difference in miRNA TCRα processing at any of the four positions tested.

### Example 15. Cytotoxic activity of Microenvironment Restricted Biologic CAR-expressing T cells can be controlled by changing pH.

The following example illustrates how the cytotoxic activity of transduced T cells (also referred to as effector cells) expressing MRB-CARs can be modulated by changes in the pH of the microenvironment. In this example, nucleic acids encoding an MRB-CAR capable of binding the cognate antigen Target 1 (anti-Target 1) were used to generate replication incompetent recombinant lentiviral particles. Pan T cells were transduced with the lentiviral particles and the cytotoxic activity of the effector cells were compared using Real-Time Cell Analysis (RTCA) before and after changing the pH of the media.

### Production of replication incompetent recombinant lentiviral particles

A nucleic acid that encoded T1B, an anti-Target 1 MRB-CAR from the series of candidate MRB-CARs in Example 3, was tested. Replication incompetent recombinant lentiviral particles were produced by transient transfection of Lenti-X 293T cells (Clontech, Mountain View, CA) with lentiviral expression vectors and nucleic acids that included segments encoding either the MRB-CAR or a control, C1, that contained a GMCFsp and an eTAG (SEQ ID NO:284), but did not include an anti-Target 1 MRB-CAR. The cells were adapted to suspension culture by serial growth in Freestyle 293 Expression Medium (ThermoFisher Scientific, Waltham, MA). The cells in suspension were transfected using PEI (Polysciences, Warminster, PA) dissolved in weak acid. Cells (30 mL) were grown to 1 × 10⁶ cells/mL in a 125 mL Erlenmeyer flask.

Total DNA was diluted in 1.5 ml Optimem media for 30 mL of cells. Total DNA (1 µg/mL of culture volume) was a mixture of 4 plasmids with the following molar ratios: 2x genomic plasmid that included lentiviral packaging elements, LTRs and the nucleic acid encoding T1B, 1x Rev-encoding plasmid, 1x VSVg-encoding plasmid, and 1x Gagpol-encoding plasmid. Separately, the PEI was diluted in 1.5 ml Optimem to 2 µg/mL (culture volume, 2:1 ratio to DNA). After a 5 minute room temperature incubation, the two solutions were mixed together well and incubated at room temperature for 20 minutes. The final volume (3 ml) was added to the cells. The cells were then incubated at 37 °C for 72 hours with rotation at 120 rpm and with 5-8% CO2.

After 72 hours, the supernatant was harvested by centrifugation at 1,000g for 10 minutes. The supernatant was decanted to a fresh tube and ¼ of the supernatant volume in PEG solution (PEG-IT, System Biosciences) was added. The replication incompetent recombinant lentiviral particles were precipitated by incubation overnight at 4 °C followed by centrifugation at 1,500g for 20 minutes at 4 °C. The supernatant was removed, and the virus was resuspended in 1:100 volume of X-VIVO 15 media. Viruses were titered by eTAG expression in Jurkat cells.

### T cell transduction/expansion

Pan T cells were obtained from AllCells. Anti-Target 1 MRB-CAR replication incompetent recombinant lentiviral particles were made as discussed above. Two days prior to lentiviral transduction, cells were thawed and cultured in X-VIVO 15 media (Lonza, Basel, Switzerland) with 5% human AB serum (Valley Biomedical Inc., Winchester, VA) and 10 mM N-acetyl L-Cysteine (Sigma-Aldrich, St. Louis, MO). Recombinant human IL-2 (R&D Systems, Minneapolis, MN) was added to a final concentration of 100 IU/mL. Twenty-four hours prior to viral transduction, primary human T cells were seeded into a 12-well plate at 0.5 x 10⁶ cells/well and activated using Dynabeads Human T-Activator CD3/CD28 (ThermoFisher Scientific) at a 1:3 cell:bead ratio. On the day of transduction, the lentiviral particle solution was added to the wells at an MOI of 5. Transduced Pan T cells were maintained at ~10⁶/mL in X-VIVO 15 media for 3 days, then transferred into a 6-well G-Rex plate with 30 mL/well of X-VIVO 15 media with 100 IU/mL IL-2. Cells were cultured for at least 10 days before experiments were conducted and IL-2 was added every other day.

### pH shift cytotoxicity assay

The cytotoxic activity of transduced T cells before and after pH change by addition of NaHCO₃ or NaOH was measured using the xCELLigence System. Briefly, one day before the experiment, target cells (CHO cells stably transfected with a construct to express Target 1 on the cell surface (CHO-Target 1 cells)), were seeded into a 96-well E-plate (ACEA; San Diego, CA) at 10,000 cells/well with X-VIVO 15 media containing 40 mM HEPES and 40 mM PIPES, pH 6.7. Cryopreserved effector cells previously transduced with either lentiviral particles containing the nucleic acid encoding T1B or C1 (T1BVP and C1VP, respectively) produced as discussed above, were thawed and cultured for two days in X-VIVO 15 media containing 100 IU/mL of IL-2 (R&D Systems, Minneapolis, MN). On the day of the experiment, cells transduced with T1BVP or C1VP were washed and resuspended in X-VIVO 15 media containing 40 mM HEPES and 40 mM PIPES, pH 6.7 and then added into the experimental wells at effector cell/target cell ratios (E/T) of 1:1.

Impedance readings measured on the xCELLigence System (ACEA) were taken every 5 minutes and reported as the Cell Index (CI) to quantitate cell confluency as a measure of cell proliferation / cell lysis. Approximately 3 hours after effector cell addition, 8 µl of 7.5% NaHCO₃ or 14 µl of 0.5 M NaOH was added into the wells with X-VIVO 15 media containing 40 mM HEPES and 40 mM PIPES, pH 6.7 to increase the pH from 6.7 to 7.4. Impedance readings were continued for approximately 20 hours after effector cell addition. Percentage of specific cytolysis was calculated as follows ((CI Target + C1VP transduced effector T cells) - (CI Target + T1BVP transduced effector T cells)) / (CI Target + C1VP transduced effector T cells) × 100.

### HCl switch on RTCA killing assay

The cytotoxic activity of transduced T cells before and after pH change by addition of HCl was measured using the xCELLigence System. Briefly, one day before the experiment, CHO-Target 1 cells were seeded into a 96 well E-plate at 10,000 cells/well with X-VIVO 15 media containing 40 mM HEPES and 40 mM PIPES, pH 7.4. Cryopreserved effector cells previously transduced with either C1VP or T1BVP, were thawed and cultured for two days in X-VIVO 15 media containing 100 IU/mL of IL-2. On the day of the experiment, cells transduced with T1BVP or C1VP were washed and resuspended in X-VIVO 15 media containing 40 mM HEPES and 40 mM PIPES, pH 7.4 and then added into experimental wells at effector cell/target cell ratios (E/T) of 1:1.

Impedance readings were taken every 5 minutes and reported as the Cell Index (CI). Approximately 3 hours after effector cell addition, 8 µl of 1 M HCl was added into the wells with X-VIVO 15 media containing 40 mM HEPES and 40 mM PIPES, pH 7.4 to switch the pH from 7.4 to 6.7. Impedance readings were continued for approximately 20 hours after effector cell addition. Percentage of specific cytolysis was calculated as follows ((CI Target + C1VP transduced effector T cells) - (CI Target + T1BVP transduced effector T cells)) / (CI Target C1VP) ×100.

### Results

The cytotoxic activity of an MRB-CAR capable of binding cognate antigen Target 1 with increased activity at a reduced pH was compared in pH 6.7 and pH 7.4. T cells that were transduced with lentiviral particles encoding an anti-Target 1 MRB-CAR were used to kill CHO cells expressing Target 1, and then the pH was increased to determine whether the cytotoxic activity could be inhibited by a pH shift. As shown in FIGs. 27A and 27B, the addition of either NaHCO₃ or NaOH to the microenvironment of active CAR-T cells to increase the pH of the media inhibited the cytotoxic activity of the T cells expressing the MRB-CAR. These results show that active MRB-CAR expressing T cells can kill target-expressing cells and then this killing activity can be inhibited by increasing the pH of the microenvironment.

The ability of the cytotoxic activity of T cells expressing the MRB-CAR to be activated by a pH change was also determined. As shown in FIG. 27C, the cytotoxic activity of anti-Target 1 MRB-CAR expressing T cells on CHO-Target 1 cells was low at a pH of 7.4 and was increased by the addition of HCl to reduce the pH of the microenvironment. Cumulatively, these results demonstrate the cytotoxic activity of T cells expressing MRB-CARs can be modulated by a shift in pH within the microenvironment, both by reducing cytotoxic activity after an increase in pH and increasing cytotoxic activity after a decrease in pH. In this non-limiting example, pH was increased from pH 6.7 and decreased from 7.4.

### Example 16. Bicarbonate administration can increase pH of the tumor microenvironment in mice.

The following example demonstrates the pH of an *in vivo* tumor microenvironment can be modulated by administering a pharmacologic agent. In this example, the pharmacologic agent is sodium bicarbonate and the tumor microenvironment is a CHO xenograft tumor in mice. The example includes two methods of measuring the pH of a tumor microenvironment, both *in vivo* and *ex vivo.*

The extracellular microenvironment of most solid tumors is acidic, with a pH typically between 6.5 and 6.9. On the contrary, normal tissue pH is basic, with a pH typically between 7.2 and 7.5. However, directly measuring the *in vivo* pH of a tumor microenvironment can be difficult. Fortunately, the relative protease activity of cathepsin is higher at lower pH and lower at higher pH. Therefore, the measurement of intratumoral cathepsin activity can serve as a surrogate measure of the pH of the tumor microenviroment. To measure *in vivo* activities of cathepsin B, L, S, K, V, and D, the near-infrared ProSense 750 FAST probe (PerkinElmer) was used. To further confirm modulation of the pH in the tumor microenvironment by administration of sodium bicarbonate, excised tumors were treated with phenol red and the color was noted. Phenol red is a pH indicator which undergoes a pH-dependent color transition. The sodium salt of phenol red is widely used in cell culture media to identify pH values. A solution of phenol red has a yellow color at a pH of 6.4 or below, an orange color around pH 7.0, a red color around pH 7.4, and a purple color above pH 7.8.

Mice were handled in accordance with Institutional Animal Care and Use Committee approved protocols. Subcutaneous (sc) Chinese Hamster Ovary (CHO) tumor xenografts were established in the hind flank of 12-14 week old female B-NSG mice (NOD-PrkdcscidIl2rgtm1/Bcgen (Beijing Biocytogen Co. Ltd.). Briefly, cultured CHO cells (ATCC, Manassas, VA) were washed in DPBS (ThermoFisher), counted, resuspended in cold DPBS and mixed with an appropriate volume of Matrigel ECM (Corning; final concentration 5 mg/mL) at a concentration of 1.5 x 10⁶ cells/200 µl on ice.

Animals were prepared for injection using standard approved anesthesia with hair removal (Nair) prior to injection. 200 µl of the cell suspension in ECM was injected sc into the rear flanks of the mice. Once tumors were palpable, the tumors were measured using calipers 2 times/week. Tumor volume was calculated using the following equation: (longest diameter * shortest diameter²)/2. When average tumor volume reached 200 mm³, mice were randomly assigned to the respective treatment groups.

Two days before the administration of bicarbonate, the drinking water for the B-NSG mice was changed from acidic to regular pH autoclaved purified water. The following day, the 750 ProSense FAST probe was administered to 6 CHO-xenograft tumor bearing mice via 100 µl tail vein injections (4 nmol ProSense 750 FAST probe/100 µl PBS). A separate group of CHO-xenograft tumor bearing mice was left untreated. The following day, sodium bicarbonate was administered and imaging of the mice treated with the ProSense 750 FAST probe was performed using a Caliper IVIS Lumina XR. Briefly, mice were anesthetized using 3% O₂ 2 L/min isoflurane in O₂ carrier gas at 2 L/min and then placed with nose cones supplying 1.5% isoflurane to anesthetized mice during imaging. Image acquisitions consisted of a 5 sec exposure for near-infrared probes (745/810 nm excitation/emission wavelength). Fluorescence images were overlaid on normal light images of the mice. Time 0 (pretreatment) images were acquired before administration of either PBS (control) or sodium bicarbonate. The mice were then administered either 1 ml/mouse PBS (control, ThermoFisher) or 1 ml/mouse 1 M sodium bicarbonate (Shanghai Experiment Reagent Co., LTD) via intraperitoneal injection (ip). Mice were then imaged at 30 min post administration of PBS or bicarbonate. The collected fluorescence images were adjusted to have identical minimums, maximums, and threshold values. The photon counts were defined in this study as relative fluorescence units (RFU). RFU was calculated by normalizing the photon counts from the 30 min time point to the pretreatment time point (time 0; 100%) in each mouse. Due to variability between fluorescence values in each mouse at the time 0 pretreatment value, the observed fluorescence intensity values at different time points were normalized only to the individual mouse and not to a mean pretreatment value.

In a separate arm of the experiment, the 6 mice that did not receive the NIR cathepsin probe were euthanized by cervical dislocation at 1.5 hours post ip administration of PBS or sodium bicarbonate. The CHO xenograft tumor was excised from each mouse. The xenograft tumors were split into two halves with a scalpel and placed on a petri dish. The tumor tissue halves were then cut/sliced repeatedly using the scalpel. Water or 0.05% phenol red solution (50 mg phenol red/100 ml water) was added dropwise to each tumor half, respectively. The color was noted and images were taken of the treated tumor xenografts and of the phenol red solution remaining on the petri plate once the tumor xenograft samples were removed.

### Results

FIG. 29 shows the RFU results (mean with SEM) from imaging intratumoral cathepsin activity in CHO-xenograft tumor bearing mice before and after administration of PBS (control; n=3) or bicarbonate (n=3). These results suggest that sodium bicarbonate administration can increase the pH of the tumor microenvironment *in vivo* as evidenced by the decreased cathepsin activity observed following ip sodium bicarbonate administration.

A color change of the phenol red indicator from yellow/orange to red was observed using the tumor tissue excised from sodium bicarbonate-treated mice (n=3) relative to the PBS-treated mice (n=3). These results suggest that sodium bicarbonate administration increased the pH of the tumor microenvironment *in vivo* following ip administration as evidenced by the color change of the phenol red indicator from yellow/orange to red.

### Example 17. Identification of candidate chimeric polypeptide lymphoproliferative elements.

In this example, two chimeric polypeptide libraries (Library 1 and Library 2) of candidate (putative) chimeric lymphoproliferative elements were assembled into viral vectors from pools of extracellular-transmembrane block sequences, intracellular block sequences, and a barcode library according to the chimeric polypeptide-encoding constructs provided in FIGs. 30 and 31. The proliferation of library-transduced PBMCs was followed over time: genomic DNA was extracted from PBMCs at one-week intervals and the barcode region amplified for DNA sequencing to estimate the number of cells in these mixed cultures of PBMCs containing each of the test candidate chimeric lymphoproliferative elements. Thus, the screen tested the ability of the candidate chimeric lymphoproliferative elements to promote PBMC cell proliferation in the absence of IL-2 or any other exogenous cytokine.

Two libraries were made and analyzed in this study: the constructs of Library 1 (which was used in the screens identified as Libraries 1A, 1.1A, and 1.1B) was flanked with a CAR at the 5' end of the candidate chimeric polypeptide, while the constructs of Library 2 (which was used in the screens identified as Libraries 2B and 2.1B) did not include a CAR (FIGs. 30 and 31). FIG. 30 provides a schematic of a non-limiting, exemplary transgene expression cassette containing a polynucleotide sequence encoding a CAR and a candidate chimeric lymphoproliferative element (CLE) of Library 1 driven by an EF-1 alpha promoter and a Kozak-type sequence (GCCGCCACC (SEQ ID NO:519)), in a lentiviral vector backbone. The CAR was FLAG-tagged and contained an ASTR directed to CD19, a stalk and transmembrane portion of CD8, and an intracellular activating domain from CD3z. Each candidate lymphoproliferative element of Library 1 included 3 modules; an extracellular/transmembrane module (P1-2) and 2 intracellular modules (P3 and P4). The P1-2 module also encoded a recognition and/or elimination domain (TAG). A triple stop sequence (TAATAGTGA (SEQ ID NO:520)) separated P4 from a DNA barcode (P5). A WPRE (GTCCTTTCCATGGCTGCTCGCCTGTGTTGCCACCTGGATTCTGCGCGGGACGTCCTTCTGCTA CGTCCCTTCGGCCCTCAATCCAGCGGACCTTCCTTCCCGCGGCCTGCTGCCGGCTCTGCGGCC TCTTCCGCGTCTTCGCCTTCGCCCTCAGACGAGTCGGATCTCCCTTTGGGCCGCCTCCCCGCC TG (SEQ ID NO:521)) was present between the last stop codon (starting 4 bp after the last nucleotide of the last stop codon) and the 3' LTR (which started 83 nucleotides after the last nucleotide of the WPRE).

The CAR and P1-2 of Library 1 were separated by a polynucleotide sequence encoding a T2A ribosomal skip sequence.

FIG. 31 provides a schematic of a non-limiting, exemplary transgene expression cassette containing a polynucleotide sequence encoding a candidate CLE of Library 2 driven by an EF-1 alpha promoter and a Kozak-type sequence (GCCGCCACC(SEQ ID NO:519)), in a lentiviral vector backbone. Each candidate lymphoproliferative element of Library 2 included 3 modules; an extracellular/transmembrane module (P1-2), and 2 intracellular modules (P3 and P4). The P1-2 module also encoded a recognition and/or elimination domain (TAG). A triple stop sequence (TAATAGTGA(SEQ ID NO:520)) separated P4 from a DNA barcode (P5). A WPRE (GTCCTTTCCATGGCTGCTCGCCTGTGTTGCCACCTGGATTCTGCGCGGGACGTCCTTCTGCTA CGTCCCTTCGGCCCTCAATCCAGCGGACCTTCCTTCCCGCGGCCTGCTGCCGGCTCTGCGGCC TCTTCCGCGTCTTCGCCTTCGCCCTCAGACGAGTCGGATCTCCCTTTGGGCCGCCTCCCCGCC TG (SEQ ID NO:521)) was present between the last stop codon (starting 4 bp after the last nucleotide of the last stop codon) and the 3' LTR (which started 83 nucleotides after the last nucleotide of the WPRE).

The assembled candidate chimeric polypeptide part of both libraries followed the exact same design with 3 positions (1 extracellular/transmembrane module (P1-2) and 2 intracellular modules (P3 and P4), one of which could be an early termination signal). GGS linkers encoded by nucleic acid sequences between each of the domains were included as a product of construct assembly. Nucleic acids encoding full or variant versions of either a Myc or an eTAG recognition and/or elimination domain (referred to in Table 6 as "eTAG" or "Myc Tag") were inserted 5' to the extracellular domain of each truncated cytokine receptor (or select LMP1 constructs) as part of the P1-2 modules, such that the encoded candidate chimeric polypeptides included a recognition and/or elimination domain in frame with the extracellular domain. A signal sequence was inserted at the 5' terminus of the CAR-encoding sequences in Library 1 and at the 5' terminus of the recognition and/or elimination domain-encoding sequences of Library 2. A Kozak-type sequence (GCCGCCACC (SEQ ID NO:519)), which is also as an enhanced Kozak-type sequence, was inserted within 10 nucleotides before the ATG start codon, which was at the 5' terminus of the signal-sequence encoding sequences in Library 1 and at the 5' terminus the recognition and/or elimination domain-encoding sequences (or select LMP1 constructs) in Library 2. A 4th module contained a random barcode generated by random PCR assembly of ~10,000 x ~10,000 sub-barcodes (~100 million combinations). The coding sequences of the CARs for Library 1 constructs encoded a signal sequence, a FLAG tag, an anti-CD19 ASTR scFv, a CD8a stalk, a CD8a transmembrane domain, and a CD3zeta intracellular activating domain. Expression of the CAR (Library 1 only) and CLEs (Library 1 and Library 2) was driven by an EF1 alpha promoter. In the constructs of Library 1, a T2A site was positioned between the CAR and the chimeric polypeptide encoding sequences. In libraries 1 and 2, a triple stop codon was present at the 3' end of P4.

### Synthesis of viral vectors

Individual parts for the library assembly were designed and synthesized as DNA blocks with compatible Type IIs enzyme (AarI) overhangs and connectors encoding a Glycine-Glycine-Serine sequence on the 5' and 3' ends of each part. These parts were then individually cloned into universal plasmid vectors. The assembly for each part was done as a one tube assembly in which 0.04 pmol of the part, 0.04 pmol barcode mix, and 0.04 pmol plasmid vector backbone were added into a 10 ul reaction followed by 1 unit AarI enzyme, 10 units T4 DNA ligase, and 1X ligase buffer (50mM Tris-HCl, 10mM MgCl₂, 1mM ATP, 10mM DTT) for optimal ligase activity. Digestion and ligation cycles were performed using a thermocycler as follows: 55 cycles of 37 °C for 2 mins and 16 °C for 5 mins; a 5 min incubation at 50 °C for final digestion; a 10 min incubation at 80 °C for AarI inactivation; and a final hold at 4 °C. Then the assembled vectors were purified by conventional ethanol precipitation. The resulting purified vectors were electroporated (Electro Micropulser, Bio-rad) into Top10 electro competent cells (Thermo Fisher Scientific) and directly recovered in liquid cultures containing Kanamycin antibiotic for selection and expansion. Cultures were incubated at 37 °C for 10-12 hours and then harvested and plasmids purified using ZymoPURE-EndoZero^{™} Plasmid Gigaprep Kit.

### Lentiviral particle production

Recombinant lentiviral particles were produced by transient transfection of 293T cells (Lenti-X^{™} 293T, Clontech) with separate lentiviral packaging plasmids encoding gag/pol and rev, and a pseudotyping plasmid encoding VSV-G. The synthesized viral vectors encoding candidate chimeric polypeptides with or without a co-expressed chimeric antigen receptor were co-transfected with the packaging plasmids. The cells were adapted to suspension culture by serial growth in Freestyle^{™} 293 Expression Medium (ThermoFisher Scientific). The cells in suspension were seeded at 1 × 10⁶ cells/mL (200 mL) in a 1 L Erlenmeyer flask and immediately transfected using polyethylenimine (PEI) (Polysciences) dissolved in weak acid.

Plasmid DNA was diluted in 10 ml Gibco^{™} Opti-MEM^{™} media for 200 mL of cells. To obtain lentiviral particles pseudotyped with VSV-G, the total DNA (1 µg/mL of culture volume) used was a mixture of 4 plasmids with the following molar ratios: 2x combined viral vectors, 1x Rev-containing plasmid, 1x VSV-G-containing plasmid, and 1x gag/pol-containing plasmid. Separately, the PEI was diluted in 10 ml Gibco^{™} Opti-MEM^{™} to 2 µg/mL (culture volume, 2:1 ratio to DNA). After a 5-minute room temperature incubation, the two solutions were mixed together thoroughly and incubated at room temperature for 20 minutes. The final volume (20 ml) was added to the cells. The cells were then incubated at 37 °C for 48 hours with rotation at 125 rpm and with 8% CO₂.

After 48 hours, the supernatants were harvested and clarified by centrifugation at 1,000g for 10 minutes. Viral supernatant was then filtered through low protein-binding Millex-HV 0.45 µm PVDF filters (Millipore, Catalog no. SLHVR25LS). The clarified supernatants were decanted to a new tube, 1 volume of Lenti-X PEG (Takara ,4x) was mixed with 3 volumes of clarified supernatant, and incubated at 4 °C overnight. The lentiviral particles were then precipitated by 90 min centrifugation at 1,500g and 4 °C. The supernatant was discarded, and the lentiviral particle pellets were resuspended in 1:100 of initial volume of complete PBMC growth media without IL-2. Lentiviral particles were titered using a p24 assay ELISA kit from Takara (#632200).

### Transduction and culturing of PBMCs

Whole human blood from a healthy donor (101ml) was collected into a 200ml blood bag containing CDP anticoagulant (Nanger; S-200). The blood was processed using density gradient centrifugation with Ficoll-Pacque^{™} (General Electric) using a Sepax 2 S-100 device (Biosafe; 14000) following manufacturer's instruction and kit CS900.2 (BioSafe; 1008), to obtain peripheral blood mononuclear cells (PBMCs). Yield of viable PBMC was 7.3 x 10⁷ cells. 1.5 x 10⁷ PBMC were added each to two G-Rex 100M cell culture devices (Wilson Wolf, 81100S) to a final concentration of 0.5 x 10⁶ viable cells / ml in a final volume of 30ml of Complete OpTmizer^{™} CTS^{™} T-Cell Expansion SFM (OpTmizer^{™} CTS^{™} T-Cell Expansion Basal Medium 1 L (Thermo Fisher, A10221) supplemented with 26 ml OpTmizer^{™} CTS^{™} T-Cell Expansion Supplement (Thermo Fisher, A10484-02), 25 ml CTS^{™} Immune Cell SR (Thermo Fisher, A2596101), and 10 ml CTS^{™} GlutaMAX^{™}-I Supplement (Thermo Fisher, A1286001) according to manufacturer's instructions). Recombinant human interleukin-2 (IL-2) (Novoprotein) was also added to a concentration of 100IU/ml along with activating anti-CD3 Ab (OKT3, Novoprotein) at a concentration of 50ng/ml, to activate the PBMCs for viral transduction. The G-Rex devices were incubated overnight in a standard humidified tissue culture incubator at 37 °C and 5% CO₂. After overnight incubation, lentivirus particle preparations containing the desired test chimeric polypeptide-encoding constructs (Library 1 or Library 2) were added to individual G-Rex devices at a multiplicity of infection (MOI) of 5. The G-Rex devices (Library 1 and Library 2) were incubated overnight in a standard humidified tissue culture incubator at 37 °C and 5% CO₂. Following the overnight incubation, additional Complete OpTmizer^{™} CTS^{™} T-Cell Expansion SFM was added to bring the final volume of each G-Rex device to 500ml. No additional IL-2 or other exogenous cytokine was added at this or following cell culture steps. The G-Rex^{®} device was incubated in a standard humidified tissue culture incubator at 37 °C and 5% CO₂ for 7 days from PBMC isolation. On day 7, the cells from the two G-Rex devices transduced with Library 1 or Library 2 were collected by centrifugation and resuspended into fresh Complete OpTmizer^{™} CTS^{™} T-Cell Expansion SFM without IL-2. 1.25 x 10⁷ of Library 1 cells and 2.54 10⁷ cells of Library 2 were placed in a new G-Rex 100M devices containing 500ml Complete OpTmizer^{™} CTS^{™} T-Cell Expansion SFM without IL-2. One vial of thawed Day 0 frozen donor matched PBMCs (1.46 x 10⁷ PBMC) was added to the G-Rex devices containing cells transduced with Library 1 to provide CD19+ B-cell activation of the CD19 ASTR. In these screens, cells fed with PBMCs were designated with an A after the library number, for example screens performed with cells transduced with Library 1 and fed with PBMCs are referred to as Library 1A herein. Library 2 received no Day 0 PBMCs. In these screens, cells not fed with PBMCs were designated with a B after the library number, for example screens performed with cells transduced with Library 2 and not fed with PBMCs are referred to as Library 2B herein. The G-Rex devices were incubated overnight in a standard humidified tissue culture incubator at 37 °C and 5% CO₂. On day 14, the cells from the two G-Rex devices (Library 1A and Library 2B) were collected by centrifugation and resuspended into fresh Complete OpTmizer^{™} CTS^{™} T-Cell Expansion SFM without IL-2. These were each placed into individual wells of 6-well G-rex plates (Wilson-Wolf; 80240M) containing 30ml Complete OpTmizer^{™} CTS^{™} T-Cell Expansion SFM without IL-2. One vial of thawed Day 0 frozen donor matched PBMCs was added to the well containing Library 1A cells to provide CD19+ B-cell activation of the CD19 ASTR. The G-Rex devices were incubated overnight in a standard humidified tissue culture incubator at 37 °C and 5% CO₂ On day 21, the cells were collected from the wells of the 6-well G-rex plates, and the process performed on day 14 was repeated. Samples (1 x 10⁵ to ~4-5 x 10⁶ cells) were collected at various days post-transduction (Days 7, 21, 28, and/or 35) and genomic DNA was purified using GenElute^{™} Mammalian Genomic DNA Miniprep following the kits protocol. In some cases, samples were purified using density gradient centrifugation with Ficoll-Pacque^{™} (General Electric) to remove dead cells prior to genomic DNA extraction. Purified genomic DNA was sequenced using an Illumina HiSeq, generating paired-end 150 bp reads. Usually, a subset of 10 million reads was extracted from each indexed fastq file and processed for analysis using barcode_reader, a custom R script engineered to extract barcode sequences based on the presence of a constant region. Purified genomic DNA was also sequenced on a PacBio sequencing system to obtain longer read lengths to associate barcodes with constructs. These screens were repeated and the Libraries were designated 1.1A, 1.1B, and 2.1B, where the A and B indicated whether or not the cells were fed with PBMCs as discussed above.

### Data analysis

Once HiSeq data was obtained for all time points, barcode count tables were merged based on barcode ID. Counts were normalized to 'Count per million' using the formula: 1 x 10⁶ * raw count / sum (raw counts for all barcodes in sample), which is referred to as prevalence values. Barcodes with an average prevalence of 0.33 cpm per time point or less were discarded. Full length construct-barcode associations obtained by SMRT sequencing (Pacific Biosciences) at select time points were used to identify assemblies of interest. Incomplete constructs not having 3 parts or ambiguous constructs having more than one construct call were filtered out. Enrichment values for each assembly were calculated as (Normalized count at final time point + 1) / (Normalized count at initial time point + 1). Candidate chimeric polypeptide genes were identified as constructs with an enrichment above a threshold value specific for each library.

### Results

Chimeric polypeptide candidates were designed to have 3 test domains, which included an extracellular and transmembrane domain (P1-2), a first intracellular domain (P3), and a second intracellular domain (P4) (FIGs. 30 and 31). Furthermore, the constructs included a DNA barcode to aid in analysis and identification of the construct using next-generation sequencing. Additionally, most constructs as shown in Table 6 included nucleic acid sequences encoding a recognition and/or elimination domain in frame with the extracellular and transmembrane domain. However, the recognition and/or elimination domains of those constructs encoding an extracellular or transmembrane fragment of LMP1 were intracellular for those constructs that contained such a recognition and/or elimination domain. Library 1 constructs encoded a CAR upstream of the chimeric polypeptide candidate. A total of 226,840 conceptualized possible combinations were designed based on 20 different extracellular-transmembrane domains, 106 possible P3 domains and 107 possible P4 domains, one of which was a STOP codon. Not all of the conceptualized constructs were detected following viral vector assembly, lentiviral particle production, transduction of PMBC, and 7 days of culture. For Library 1A, 57,815 constructs were present after transduction of PBMCs and 7 days of culture. For Library 2B, 69,578 constructs were present after transduction of PBMCs and 7 days of culture. Detailed information about the candidates analyzed, as well as detailed results, are provided in Tables 7 to 11. Detailed information about the various candidate parts is provided in Table 6. The headers for Tables 7-11 are as follows: BlockSequence is the code of P1-P2, P3, and P4 domains from Table 6 used in each construct; Norm_D7 is the normalized counts at day 7; Enrich_DXX is the enrichment at day XX versus day 7, where XX is the day indicated on the table, for example, Enrich _D21 is the enrichment at day 21 versus day 7 and Enrich_D35 is the enrichment at day 35 versus day 7. Data for a construct is shown to the immediate right columns for the construct. Data for two constructs are shown in each row. For example, the first candidate identified in Table 7is M008-S212-S075. For this candidate, the extracellular and transmembrane domain (P1-2) is M008, the first intracellular domain (P3) is S212, and the second intracellular domain (P4) is S075. Detailed information about the identity of these modules, for example M008, is found in Table 6. Table 6 discloses that M008 is LMP1 and that the construct includes a Myc tag. The amino acid sequences of the candidate domains are provided in Table 6. For clarification, the GenBank identifier in Table 6 provides a nucleic acid sequence that encodes a polypeptide domain. In some cases the nucleic acid sequence was modified to assist with cloning, or for codon optimization, but encodes the same polypeptide as the GenBank sequence. In some cases, as will be apparent by the amino acid sequence provided in Table 6, a portion of the polypeptide encoded by the GenBank sequence was used.

Candidate extracellular and transmembrane domains were selected from known mutant receptors such as cytokine or hormone receptors that have been reported to be constitutively active when expressed at least in some cell types. Ligand binding regions were not included in the candidate extracellular and transmembrane domains. Mutations in such receptor mutants occur in the transmembrane region or juxtamembrane region. Exemplary candidate extracellular and transmembrane domains included IL7RA Ins PPCL, LMP1, CRLF2 F232C, CSF2RB V449E, CSF3R T640N, EPOR L251C I252C, GHR E260C I270C, IL27RA F523C, and MPL S505N. The candidate extracellular and transmembrane domains included the wild type viral protein LMP1, which is a multispan transmembrane protein that is known to activate cell signaling independent of ligand when targeted to lipid rafts or when fused to CD40 (Kaykas et al. EMBO J. 20: 2641 (2001)).

The identities of potential polypeptides chosen to occupy the first and second intracellular domains of the candidate chimeric polypeptides were identical. These candidate intracellular domains were intracellular signaling domains of genes that are known in at least some cell types, to promote proliferation, survival (anti-apoptotic), and/or provide a co-stimulatory signal that enhances a differentiation state, proliferative potential or resistance to cell death. Some of the intracellular domains of candidate chimeric polypeptides are known to activate JAK1/JAK2 signaling and STATS. Intracellular domains from some of the genes listed in Table 6 were included in the library. Exemplary intracellular domains from these genes are provided in the P3 and P4 locations of Tables 7 to 11. Detailed information about these P3 and P4 domains are provided in Table 6.

For Library 1A, which included constructs that encoded the chimeric polypeptides and a CAR, 172 top candidate chimeric polypeptides were identified (See Table 7, in which the enrichment was calculated as Log2(Normalized count at the last day indicated on the table + 1) - log2(Normalized count at day 7 + 1)) that promoted PBMC proliferation to the greatest magnitude (and likely survival too) between day 7 and the last day indicated on the table when cultured in the absence of IL-2. It is noteworthy that this and all of the screening experiments of Examples 17 and 18 are competitive experiments. Thus, a negative result does not mean that a construct does not promote proliferation of T cells in the absence of growth factors such as IL-2. It only means that relative to other constructs tested, it was outperformed with respect to proliferation.

For Library 1A, some of the chimeric polypeptide constructs promoted cell proliferation between day 7 and the last day indicated on the table for every extracellular and transmembrane mutant domain tested in the P1-2 position. Furthermore, when all results from all constructs derived from the same position P1-2 mutant were combined, all extracellular and transmembrane domains tested yielded cell enrichment of greater than 1 (i.e. promoted cell proliferation) between day 7 and the last day indicated on the table. Examples of extracellular and/or transmembrane domains or portions and/or mutants thereof, that were present in constructs that promoted cell proliferation are provided in Table 7. Examples of extracellular and/or transmembrane domains or portions and/or mutants thereof, that were present in constructs in the repeated screen referred to as Library 1.1A that promoted cell proliferation are provided in Table 9. Examples of extracellular and/or transmembrane domains or portions and/or mutants thereof, that were present in constructs in the repeated screen referred to as Library 1.1B, which included cells transduced with Library 1 that were not fed PBMCs, that promoted cell proliferation are provided in Table 10.

For Library 1A, intracellular domains from CD3D, CD3E, CD8A, CD27, CD40, CD79B, IFNAR1, IL2RA, IL3RA, IL13RA2, TNFRSF8, and TNFRSF9, or mutants thereof that are known to have signaling activity if such mutants are present in the constructs provided in the tables, when found at the first intracellular domain position (P3) of candidate chimeric polypeptides promoted proliferation of PBMCs between day 7 and the last day indicated on the table, when data was considered in a combined manner for all constructs with first intracellular domains (P3) derived from that gene. This conclusion is based on enrichment of sequence counts of constructs in mixed cultured PBMC cell populations, such that at least 3 times more counts for such domain were present at the last day indicated on the table than day 7 for Library 1A, when results for all constructs for a gene are combined. Examples of first intracellular domains or portions and/or mutants thereof, that were present in constructs that promoted the highest magnitude of cell proliferation are provided in Table 7. Examples of first intracellular domains or portions and/or mutants thereof, that were present in constructs in the repeated screen referred to as Library 1.1A that promoted the highest magnitude of cell proliferation are provided in Table 9. Examples of first intracellular domains or portions and/or mutants thereof, that were present in constructs in the repeated screen referred to as Library 1.1B, which included cells transduced with Library 1 that were not fed PBMCs, that promoted cell proliferation are provided in Table 10.

For Library 1A, intracellular domains from CD3D, CD3G, CD8A, CD8B, CD27, CD40, CD79B, CRLF2, FCGR2C, ICOS, IL2RA, IL13RA1, IL13RA2, IL15RA, TNFRSF9, and TNFRSF18, or mutants thereof that are known to have signaling activity if such mutants are present in the tables, when found at the second intracellular domain position (P4) of candidate chimeric polypeptides promoted proliferation of PBMCs between day 7 and the last day indicated on the table, when data was considered in a combined manner for all constructs with a second intracellular domains (P4) derived from that gene. This conclusion is based on enrichment of sequence counts of constructs in mixed cultured PBMC cell populations, such that at least 2.9 times more counts for such domain were present at day 35 than day 7 for Library 1A, when results for all constructs for a gene are combined. Examples of second intracellular domains or portions and/or mutants thereof, that were present in constructs that promoted the highest magnitude of cell proliferation are provided in Table 7. Examples of second intracellular domains or portions and/or mutants thereof, that were present in constructs in the repeated screen referred to as Library 1.1A that promoted cell proliferation are provided in Table 9. Examples of second intracellular domains or portions and/or mutants thereof, that were present in constructs in the repeated screen referred to as Library 1.1B, which included cells transduced with Library 1 that were not fed PBMCs, that promoted cell proliferation are provided in Table 10.

More specifically for Library 1A, of the 5,996 constructs with MPL S505N (MPL proto-oncogene, thrombopoietin receptor with serine 505 mutated to asparagine) as the P1-P2 module, 165 were positive, which was defined as constructs having an enrichment above two for Library 1A. Of the 436 constructs with CD3D (CD3d molecule) as the P3 module, 24 were positive. Of the 528 constructs with CD3E (CD3e molecule) as the P3 module, 17 were positive. Of the 261 constructs with CD8A (CD8a molecule) as the P3 module, 14 were positive. Of the 1,022 constructs with CD40 (CD40 molecule) as the P3 module, 50 were positive. Of the 568 constructs with IL3RA (interleukin 3 receptor subunit alpha) as the P3 module, 26 were positive. Of the 556 constructs with CD79B (CD79b molecule) as the P3 module, 27 were positive. Of the 131 constructs with IFNAR1 (interferon alpha and beta receptor subunit 1) as the P3 module, 7 were positive. Of the 519 constructs with IL13RA2 (interleukin 13 receptor subunit alpha 2) as the P3 module, 33 were positive. Of the 571 constructs with IL2RA (interleukin 2 receptor subunit alpha) as the P3 module, 28 were positive. Of the 618 constructs with CD27 (CD27 molecule) as the P3 module, 30 were positive. Of the 573 constructs with TNFRSF8 (TNF receptor superfamily member 8) as the P3 module, 15 were positive. Of the 573 constructs with TNFRSF9 (TNF receptor superfamily member 9) as the P3 module, 25 were positive. Of the 360 constructs with IL13RA2 (interleukin 13 receptor subunit alpha 2) as the P4 module, 25 were positive. Of the 490 constructs with IL2RA (interleukin 2 receptor subunit alpha) as the P4 module, 28 were positive. Of the 489 constructs with IL15RA (interleukin 15 receptor subunit alpha) as the P4 module, 22 were positive. Of the 590 constructs with CD8A (CD8a molecule) as the P4 module, 25 were positive. Of the 619 constructs with IL13RA1 (interleukin 13 receptor subunit alpha 1) as the P4 module, 25 were positive. Of the 637 constructs with CD3G (CD3g molecule) as the P4 module, 30 were positive. Of the 618 constructs with CD3D (CD3d molecule) as the P4 module, 37 were positive. Of the 673 constructs with CD27 (CD27 molecule) as the P4 module, 29 were positive. Of the 654 constructs with CD79B (CD79b molecule) as the P4 module, 33 were positive. Of the 728 constructs with TNFRSF9 (TNF receptor superfamily member 9) as the P4 module, 30 were positive. Of the 1,169 constructs with CD40 (CD40 molecule) as the P4 module, 53 were positive. Of the 1,324 constructs with TNFRSF18 (TNF receptor superfamily member 18) as the P4 module, 54 were positive. Of the 1,739 constructs with CD8B as the P4 module, 73 were positive. Of the 286 constructs with CRLF2 as the P4 module, 8 were positive. Of the 725 constructs with FCGR2C as the P4 module, 29 were positive. Of the 602 constructs with ICOS as the P4 module, 24 were positive.

For Libraries 1A and 1.1A, the constructs M001-S116-S044, M024-5192-5045, M001-5047-S102, M048-S195-S043, M012-S216-S211, and M030-S170-S194 had particularly noteworthy enrichments in both screens. For the purposes of the repeated screens, constructs with particularly noteworthy enrichments were those that had a log₂((normalized count data on the last day + 1)/(normalized count data on day 7 + 1)) value above 2.

Further information about the first and second intracellular domains in constructs with particularly noteworthy enrichments in screens of both Library 1A and Library 1.1A is provided in Table 19, including the gene(s) the first and second intracellular domain are derived from, whether the first and/or second intracellular domain are cytokine receptors, and whether the first and/or second intracellular domain have at least one ITAM motif. Constructs with intracellular domains from IL6R, MYD88, CD27, MYD88, TNFRSF18, or IL31RA, when present at the first intracellular domain (P3), and constructs with intracellular domains from CD8B, IL1RL1, CD8A, TNFRSF4, or MYD88, when present at the second intracellular domain (P4), showed particularly noteworthy enrichments in both Libraries 1A and 1.1A (Table 19). Constructs with domains from the cytokine receptors IL6R, CD27, TNFRSF18, or IL31RA, when present at the first intracellular domain (P3), and constructs with domains from the cytokine receptors IL1RL1 or TNFRSF4, when present at the second intracellular domain (P4), showed particularly noteworthy enrichments in both Libraries 1A and 1.1A (Table 19).

In Library 2B, which included constructs that encoded the chimeric polypeptides and did not encode a CAR, 167 top candidates were identified (See Table 8, in which the enrichment was calculated as Log2(Normalized count at the last day indicated on the table + 1) - log2(Normalized count at day 7 + 1)) that promoted PBMC proliferation between day 7 and the last day indicated on the table when cultured in the absence of IL-2. It is noteworthy that, in general enrichment was less than that found for Library 1A constructs, which included a CAR.

For Library 2B, CSF3R T640N in the P1-2 position, promoted cell proliferation between day 7 and the last day indicated on the table better than other extracellular and transmembrane (P1-2) domains tested, when all results from all constructs derived from an extracellular and transmembrane domain were combined, yielding an enrichment factor of greater than 2.5. Examples of extracellular and/or transmembrane domains or portions and/or mutants thereof, that were present in constructs that promoted the highest magnitude of cell proliferation are provided in Table 8. Examples of extracellular and/or transmembrane domains or portions and/or mutants thereof, that were present in constructs in the repeated screen referred to as Library 2.1B that promoted the highest magnitude of cell proliferation are provided in Table 11.

For Library 2B, intracellular domains from CD8A, CD40, IFNAR1, IL31RA, and MyD88, or mutants thereof that are known to have signaling activity if such mutants are present in the tables, when found at the first intracellular domain position (P3) of candidate chimeric polypeptides promoted cell proliferation of PBMCs between day 7 and the last day indicated on the table, when data is considered in a combined manner for all constructs with first intracellular domains (P3) derived from that gene. This conclusion is based on enrichment of sequence counts of constructs in mixed cultured PBMC cell populations, such that at least 2 times more counts for such domain were present at day 28 than day 7 for Library 2B, when results for all constructs for a gene are combined. Examples of first intracellular domains or portions and/or mutants thereof, that were present in constructs that promoted the largest magnitude of cell proliferation are provided in Table 8. Examples of first intracellular domains or portions and/or mutants thereof, that were present in constructs in the repeated screen referred to as Library 2.1B that promoted the highest magnitude of cell proliferation are provided in Table 11.

For Library 2B, intracellular domains from CD27, or mutants thereof that are known to have signaling activity if such mutants are present in the constructs provided in the tables, when found at the second intracellular domain position (P4) of chimeric polypeptide candidates, when data is considered in a combined manner for all constructs with a second intracellular domains (P4) derived from that gene, promoted proliferation of PBMCs between day 7 and the last day indicated on the table. This conclusion is based on enrichment of sequence counts of constructs in mixed cultured PBMC cell populations, such that 11 times more counts for such domain were present at day 28 than day 7 for Library 2B, when results for all constructs for a gene are combined. Examples of second intracellular domains or portions and/or mutants thereof, that were present in constructs that promoted the largest magnitude of cell proliferation are provided in Table 8. Examples of second intracellular domains or portions and/or mutants thereof, that were present in constructs in the repeated screen referred to as Library 2.1B that promoted the largest magnitude of cell proliferation are provided in Table 11.

More specifically, for Library 2B, of the 6,909 constructs with CSF3R T640N (colony stimulating factor 3 receptor with threonine 640 mutated to asparagine) as the P1/P2 module for Library 2B, 59 were positive, which was defined as constructs having an enrichment above two for Library 2B. Of the 184 constructs with IFNAR1 (interferon alpha and beta receptor subunit 1) as the P3 module for Library 2B, 3 were positive. Of the 1,258 constructs with CD40 (CD40 molecule) as the P3 module for Library 2B, 17 were positive. Of the 851 constructs with CD27 (CD27 molecule) as the P4 module for Library 2B, 11 were positive. Of the 418 constructs with CD8A as the P3 module for Library 2B, 10 were positive. Of the 1,385 constructs with IL31RA as the P3 module for Library 2B, 12 were positive. Of the 5,757 constructs with MyD88 as the P3 module for Library 2B, 56 were positive.

For Libraries 2B and 2.1B, the constructs M007-S049-S051, M007-S050-S039, M012-S050-S043, M012-S161-S213, M030-S142-S049, M001-S145-S130, M018-5085-5039, M018-5075-5053, M012-5135-S074, and M007-S214-S077 had particularly noteworthy enrichments in both screens. For the purposes of the repeated screens, constructs with particularly noteworthy enrichments were those that had a log₂((normalized count data on the last day + 1)/(normalized count data on day 7 + 1)) value above 2.

Further information about the first and second intracellular domains in constructs with particularly noteworthy enrichments in screens of both Library 2B and Library 2.1B is provided in Table 20, including the gene(s) the first and second intracellular domain are derived from, whether the first and/or second intracellular domain are cytokine receptors, and whether the first and/or second intracellular domain have at least one ITAM motif. Constructs with intracellular domains from CD28, CD40, IL22RA1, IL13RA2, IL17RB, IFNGR2, FCGR2C, IL11RA, or TNFRSF14, when present at the first intracellular domain (P3), and constructs with intracellular domains from CD40, CD3G, CD8A, TNFRSF9, CD28, IL10RB, CD79B, FCER1G, or GHR, when present at the second intracellular domain (P4), showed particularly noteworthy enrichments in both Libraries 2B and 2.1B (Table 20). Constructs with domains from the cytokine receptors CD40, IL22RA1, IL13RA2, IL17RB, IFNGR2, FCGR2C, IL11RA, or TNFRSF14, when present at the first intracellular domain (P3), and constructs with domains from the cytokine receptors TNFRSF9, IL10RB, FCER1G, GHR, or CD40, when present at the second intracellular domain (P4), showed particularly noteworthy enrichments in both Libraries 2B and 2.1B (Table 20). Constructs with domains containing ITAM motifs from FCGR2C, when present at the first intracellular domain (P3), and constructs with domains containing ITAM motifs from CD3G, CD79B, or FCER1G, when present at the second intracellular domain (P4), showed particularly noteworthy enrichments in both Libraries 2B and 2.1B (Table 20).

### Example 18. Identification of candidate chimeric polypeptide lymphoproliferative elements.

In this example, two chimeric polypeptide libraries (Library 3 and Library 4) of candidate (putative) chimeric lymphoproliferative elements were assembled into viral vectors from pools of extracellular-transmembrane block sequences, intracellular block sequences, and a barcode library according to the chimeric polypeptide-encoding construct provided in FIGs. 32 and 33. The proliferation of library-transduced PBMCs was followed over time: genomic DNA was extracted from PBMCs at one-week intervals and the barcode region amplified for DNA sequencing to estimate the number of cells in these mixed cultures of PBMCs containing each of the test candidate chimeric polypeptides . Thus, the screen tested the ability of the candidate chimeric polypeptides to promote PBMC cell proliferation in the absence of exogenously added IL-2 or any other exogenous cytokine.

Two libraries were made and analyzed in this study: Library 3 (which was used in the screens identified as Libraries 3A, 3B, 3.1A, and 3.1B) was flanked with a CAR at the 5' end of the chimeric polypeptide, while Library 4 (which was used in the screens identified as Libraries 4B and 4.1B) did not include a CAR. FIG. 32 provides a schematic of a non-limiting, exemplary transgene expression cassette containing a polynucleotide sequence encoding a CAR and a candidate CLE of Library 3 driven by an EF-1 alpha promoter and a Kozak-type sequence (GCCGCCACC(SEQ ID NO:519)), in a lentiviral vector backbone. The CAR was FLAG tagged and contained an ASTR directed to CD19, a stalk and transmembrane portion of CD8, and an intracellular activating domain from CD3z. Each candidate lymphoproliferative element of Library 3 included 4 modules; an extracellular module (P1), a transmembrane module (P2), and 2 intracellular modules (P3 and P4). The P1 module also encoded a Myc recognition and/or elimination domain. A triple stop sequence (TAATAGTGA (SEQ ID NO:520)) separated P4 from a DNA barcode (P5). A WPRE (GTCCTTTCCATGGCTGCTCGCCTGTGTTGCCACCTGGATTCTGCGCGGGACGTCCTTCTGCTA CGTCCCTTCGGCCCTCAATCCAGCGGACCTTCCTTCCCGCGGCCTGCTGCCGGCTCTGCGGCC TCTTCCGCGTCTTCGCCTTCGCCCTCAGACGAGTCGGATCTCCCTTTGGGCCGCCTCCCCGCC TG (SEQ ID NO:521)) was present between the last stop codon (starting 4 bp after the last nucleotide of the last stop codon) and the 3' LTR (which started 83 nucleotides after the last nucleotide of the WPRE).

The CAR and P1 of Library 3 were separated by a polynucleotide sequence encoding a T2A ribosomal skip sequence.

FIG. 33 provides a schematic of a non-limiting, exemplary transgene expression cassette containing a polynucleotide sequence a candidate CLE of Library 4 driven by an EF-1 alpha promoter and a Kozak-type sequence (GCCGCCACC(SEQ ID NO:519)), in a lentiviral vector backbone. Each candidate lymphoproliferative element of Library 4 included 4 modules; an extracellular module (P1), a transmembrane module (P2), and 2 intracellular modules (P3 and P4). The P1 module also encoded a Myc recognition and/or elimination domain. A triple stop sequence (TAATAGTGA (SEQ ID NO:520)) separated P4 from a DNA barcode (P5). A WPRE (GTCCTTTCCATGGCTGCTCGCCTGTGTTGCCACCTGGATTCTGCGCGGGACGTCCTTCTGCTA CGTCCCTTCGGCCCTCAATCCAGCGGACCTTCCTTCCCGCGGCCTGCTGCCGGCTCTGCGGCC TCTTCCGCGTCTTCGCCTTCGCCCTCAGACGAGTCGGATCTCCCTTTGGGCCGCCTCCCCGCC TG (SEQ ID NO:521)) was present between the last stop codon (starting 4 bp after the last nucleotide of the last stop codon) and the 3' LTR (which started 83 nucleotides after the last nucleotide of the WPRE).

The assembled chimeric polypeptide parts of both libraries followed the exact same design with 4 positions (1 extracellular module (P1), 1 transmembrane module (P2), and 2 intracellular parts (P3 and P4), one of which could be an early termination signal) (FIGs. 32 and 33). Nucleic acids encoding full or variant versions of either a Myc tag or an eTAG recognition and/or elimination domain (referred to in Table 6 as "eTAG" or "Myc Tag") was inserted at the 5' terminus of the CAR-encoding sequences in Library 3 and at the 5' terminus of the c-Jun domain in P1 in Library 4. A signal sequence was inserted at the 5' terminus of the CAR-encoding sequences in Library 3 and at the 5' terminus of the recognition and/or elimination domain-encoding sequences of Library 4. The general design and construction of the library, including the barcode, was as disclosed in Example 17 herein, except for the P1 and P2 domains, as set out in more detail later in this Example. Furthermore, the clearance domain was on some constructs, as indicated in Table 6, and when present was an eTag variant or a Myc tag.

### Synthesis of viral vectors and lentiviral production

Vectors were synthesized and lentiviral particles were produced for each library as disclosed in Example 17.

### Transduction and culturing of PBMCs

For Library 3A, whole human blood from a healthy donor (89.1ml) was collected into a 200ml blood bag containing CDP anticoagulant (Nanger; S-200). The blood was processed using density gradient centrifugation with Ficoll-Pacque^{™} (General Electric) using a Sepax 2 S-100 device (Biosafe; 14000) following manufacturer's instruction and kit CS900.2 (BioSafe; 1008), to obtain peripheral blood mononuclear cells (PBMCs). Yield of viable PBMC was 8.4 x 10⁷ cells. Ten vials of cells at 5 x 10⁶ PBMC per vial were frozen for later use in feeding CD19+ B cells for CD19-CAR containing Library 3A samples. 3 x 10⁷ PBMC were added to one G-Rex 100M cell culture device (Wilson Wolf, 81100S) to a final concentration of 0.5 x 10⁶ viable cells / ml in a final volume of 60ml of Complete OpTmizer^{™} CTS^{™} T-Cell Expansion SFM (OpTmizer^{™} CTS^{™} T-Cell Expansion Basal Medium 1 L (Thermo Fisher, A10221) supplemented with 26 ml OpTmizer^{™} CTS^{™} T-Cell Expansion Supplement (Thermo Fisher, A10484-02), 25 ml CTS^{™} Immune Cell SR (Thermo Fisher, A2596101), and 10 ml CTS^{™} GlutaMAX^{™}-I Supplement (Thermo Fisher, A 1286001)according to manufacturer's instructions. Recombinant human interleukin-2 (IL-2) (Novoprotein) was also added to a concentration of 100IU/ml along with activating anti-CD3 Ab (OKT3, Novoprotein) added at a concentration of 50ng/ml, to activate the PBMCs for viral transduction. The G-Rex device was incubated overnight in a standard humidified tissue culture incubator at 37 °C and 5% CO₂. After overnight incubation, lentivirus particle preparations containing the desired test chimeric polypeptide-encoding constructs (Library 3) were added to the G-Rex device at a multiplicity of infection (MOI) of 5. The G-Rex device was incubated overnight in a standard humidified tissue culture incubator at 37 °C and 5% CO₂. Following the overnight incubation, additional Complete OpTmizer^{™} CTS^{™} T-Cell Expansion SFM was added to bring the final volume to 500ml. No additional IL-2 or other exogenous cytokine was added at this or following cell culture steps. The G-Rex^{®} device was incubated in a standard humidified tissue culture incubator at 37 °C and 5% CO₂ for 7 days from PBMC isolation. On day 7, the cells from the G-Rex device transduced with Library 3 were collected by centrifugation and resuspended into fresh Complete OpTmizer^{™} CTS^{™} T-Cell Expansion SFM without IL-2. 5 x 10⁷ of Library 3 cells were placed in a new G-Rex 100M device containing 500ml Complete OpTmizer^{™} CTS^{™} T-Cell Expansion SFM without IL-2 and labelled as Library 3A. 1.1 x 10⁸ of Library 3 cells were placed in a new G-Rex 100M device containing 500ml Complete OpTmizer^{™} CTS^{™} T-Cell Expansion SFM without IL-2 and labelled as Library 3B. In these screens, cells fed with PBMCs were designated with an A after the library number, for example screens performed with cells transduced with Library 3 and fed with PBMCs are referred to as Library 3A herein. Four vials of thawed Day 0 frozen donor matched PBMC (2 x 10⁷ PBMC) was added to the G-Rex devices containing cells transduced with library 3A to provide CD19+ B-cell activation of the CD19 ASTR. Library 3B received no Day 0 PBMC. In these screens, cells not fed with PBMCs were designated with a B after the library number. For example screens performed with cells transduced with Library 3 and not fed with PBMCs are referred to as Library 3B herein. The G-Rex devices were incubated in a standard humidified tissue culture incubator at 37 °C and 5% CO₂. On day 14, the cells from the two G-Rex devices (library 3A, library 3B) were collected by centrifugation and resuspended into fresh Complete OpTmizer^{™} CTS^{™} T-Cell Expansion SFM without IL-2. These were each placed into individual wells of 6-well G-Rex plates (Wilson-Wolf; 80240M) containing 30ml Complete OpTmizer^{™} CTS^{™} T-Cell Expansion SFM without IL-2. One vial of thawed Day 0 frozen donor matched PBMC (5 x 10⁶ PBMC) was added to the library 3A G-Rex to provide CD19+ B-cell activation of the CD19 ASTR. The G-Rex devices were incubated in a standard humidified tissue culture incubator at 37 °C and 5% CO₂. This process was repeated on Day 21, Day 28, Day 35, and Day 42 except two vials of frozen Day 0 PBMC were added to Library 3A at day 21, and one vial on Days 28, 35, and 42. Library3B was not cultured past day 21. These screens were repeated, except the Myc tag on the P1 part was replaced with an eTag as shown in Tables 6, 14, and 15, and designated as Libraries 3.1A and 3.1B, where the A and B indicated whether or not the cells were fed with PBMCs as discussed above.

Cells from Library 3A were prepared for analysis by flow cytometry on Day 49. Cells were harvested by centrifugation (400g, 5minutes) and layered over Ficoll (Ficoll-Paque Premium (GE, cat# 17-5442-02) according to manufacturer's directions. 1.7 x 10⁶ cells were resuspended in 450µl FACs Staining Buffer (554656, BD). Resuspended cells were aliquoted into 9 eppendorf tubes, each containing 50µl or 1.8 x 10⁵ cells. 2.5µl human Fc Block (564220, BD) was added into each tube and incubated at room temperature for 10 minutes. The following antibody recipe was added into the 50µl samples to stain for CD3, CD4, CD8, CD56, CD19, FLAG Tag (CAR+): 2.5µl anti-CD3-BV421 antibody (clone OKT3,317344, Biolegend), 2.5µl anti-CD8-BV510 antibody (clone RPA-T8 (301048, Biolegend), 2.5µl anti-CD4-PE-Cy7 antibody (clone OKT4 (317412, Biolegend), 2.5µl anti-CD56-BV785(362550, Biolegend) and 0.5µl PE anti-FLAG Tag (anti-DYKDDDDK, Clone L5, 637310, Biolegend) for 5 color stained samples. 2.5µl BB515 CD19 antibody (clone HIB19, 564456, BD) and 0.5ul PE anti-FLAG Tag antibody were added for 2 color staining samples. Samples single stained for CD3, CD4, CD8, CD56, or FLAG Tag were also prepared using the antibodies and volumes above. 2.5µl BV421 IgG (mouse IgG2a,400259, Biolegend), 2.5µl PE/CY5 IgG (mouse IgG2b,400317, Biolegend), 2.5µl BV510 IgG (mouse IgG1,400171, Biolegend), 2.5µl BV785 IgG (mouse IgG1, 400169, Biolegend) and 10µl PE IgG (mouse IgG1, 555749, BD) were added for IgG control stained samples. No antibody was added for a non-stained control. Samples were incubated 30 minutes, on ice. The samples were centrifuged (400g, 5minutes) and supernatant was removed. The samples were washed with 0.5ml FACS Staining Buffer twice. The stained cell pellets were resuspended in 125µl FACS Staining Buffer and then fixed by adding 125ul BD Fixative buffer (554655 BD). The samples were incubated with fixation buffer for 15 minutes at 4°C. The samples were washed twice with 500µl FACS Staining Buffer and resuspended with 0.4ml FACS Staining Buffer. FACs analysis of samples was performed on a Novocyte flow cytometer (ACEA Biosciences) and analyzed using a lymphocyte gate based on forward and side scatter.

For Library 4B, whole human blood from a healthy donor (70ml) was collected into a 200ml blood bag containing CDP anticoagulant (Nanger; S-200). The blood was processed using density gradient centrifugation with Ficoll-Pacque^{™} (General Electric) using a Sepax 2 S-100 device (Biosafe; 14000) following manufacturer's instruction and kit CS900.2 (BioSafe; 1008), to obtain peripheral blood mononuclear cells (PBMCs). Yield of viable PBMC was 4.9 x 10⁷ cells. 4.9 x 10⁷ PBMC were added to one G-Rex 100M cell culture device (Wilson Wolf, 81100S) to a final concentration of 0.5 x 10⁶ viable cells / ml in a final volume of 98ml of Complete OpTmizer^{™} CTS^{™} T-Cell Expansion SFM. Recombinant human interleukin-2 (IL-2) (Novoprotein) was also added to a concentration of 100IU/ml along with activating anti-CD3 Ab (OKT3, Novoprotein) added at a concentration of 50ng/ml, to activate the PBMC for viral transduction. The G-Rex device was incubated overnight in a standard humidified tissue culture incubator at 37 °C and 5% CO₂. After overnight incubation, lentivirus particle preparations containing the desired test chimeric polypeptide-encoding constructs (Library 4) were added to the G-Rex device at a multiplicity of infection (MOI) of 5. The G-Rex device was incubated overnight in a standard humidified tissue culture incubator at 37 °C and 5% CO₂. Following the overnight incubation, additional Complete OpTmizer^{™} CTS^{™} T-Cell Expansion SFM was added to bring the final volume to 500ml. No additional IL-2 was added at this or following cell culture steps. The G-Rex^{®} device was incubated in a standard humidified tissue culture incubator at 37 °C and 5% CO₂ for 7 days from PBMC isolation. On day 7, the cells from the G-Rex device transduced with library 4 were collected by centrifugation and resuspended into fresh Complete OpTmizer^{™} CTS^{™} T-Cell Expansion SFM without IL-2. 1.63 x 10⁸ of Library 4 cells were placed in a new G-Rex 100M device containing 500ml Complete OpTmizer^{™} CTS^{™} T-Cell Expansion SFM without IL-2 and labelled as Library 4. Library 4 received no Day 0 PBMC. Thus, In these screens, the libraries were designated with a B after the library number, for example screens performed with cells transduced with Library 4 were not fed with PBMCs and are referred to as Library 4B herein. The G-Rex device was incubated in a standard humidified tissue culture incubator at 37 °C and 5% CO₂. On day 14, the cells from the G-Rex device (Library 4) were collected by centrifugation and resuspended into fresh Complete OpTmizer^{™} CTS^{™} T-Cell Expansion SFM without IL-2. These cells were each placed into an individual well of 6-well G-rex plates (Wilson-Wolf; 80240M) containing 30ml Complete OpTmizer^{™} CTS^{™} T-Cell Expansion SFM without IL-2. The G-Rex device was incubated in a standard humidified tissue culture incubator at 37 °C and 5% CO₂. Library 4 samples were not cultured past Day 21. These screens were repeated, except the Myc tag on the P1 part was replaced with an eTag as shown in Tables 6 and 16, and designated as Library 4.1B, where the B indicated the cells were not fed with PBMCs as discussed above.

Samples (7 x 10⁴ to ~4-8 x 10⁶ cells) were collected at various days post-transduction (Days 7 and 21) and genomic DNA was purified using GenElute^{™} Mammalian Genomic DNA Miniprep following the kits protocol. In some cases, samples were purified using density gradient centrifugation with Ficoll-Pacque^{™} (General Electric) to remove dead cells prior to genomic DNA extraction. Purified genomic DNA was sequenced using an Illumina HiSeq, generating paired-end 150 bp reads. Usually, a subset of 10 million reads was extracted from each indexed fastq file and processed for analysis using barcode_reader, a custom R script engineered to extract barcode sequences based on the presence of a constant region. Purified genomic DNA was also sequenced on a PacBio sequencing system to associate barcodes with constructs.

### Data analysis

Data analysis was performed as disclosed in Example 17 except the time points differed.

### Results

In this experiment, chimeric polypeptide candidates were designed to have 4 test domains, which included an extracellular domain (P1), a transmembrane domain (P2), a first intracellular domain (P3), and a second intracellular domain (P4) (FIGs. 32 and 33). As explained in Example 17, the constructs included a DNA barcode to aid in analysis and identification of the construct using next-generation sequencing. Additionally, all of the constructs included nucleic acid sequences encoding a recognition and/or elimination domain in frame with the extracellular domain. Constructs in Library 3, but not Library 4. encoded a CAR upstream of the chimeric polypeptide candidate, which was identical in structure to the CAR used in Library 1 (Example 17).

The extracellular domain used for Library 3 and Library 4 was a variant of c-Jun that included the leucine zipper motif (NM_002228_3) known to be a dimerizing motif (see, e.g. Chinenov and Kerppola, Oncogene. 2001 Apr 30;20(19):2438-52). A spacer of between 0 and 4 alanine residues was included in the candidate chimeric polypeptides between the c-Jun extracellular domain and the transmembrane domain. Not to be limited by theory, it is believed that the alanine spacer could affect signaling of intracellular domains connected to the leucine zipper extracellular region via the transmembrane domain, by changing the orientation of the connected transmembrane domains and/or the intracellular domains.

Candidate transmembrane domains were selected from known single spanning transmembrane domains from wild-type and mutant Type I receptors such as cytokine, hormone, costimulatory, or activating receptors that have been reported to be constitutively active when expressed at least in some cell types. Mutations in such receptor mutants selected for Library 3 and Library 4 occur in the transmembrane region. Exemplary transmembrane domains for Libraries 3A, 3B, 3.1A, 3.1B, 4B, and 4.1B can be identified in Tables 12 to 17. The headers for Tables 12-17 are as follows: BlockSequence is the code of P1, P2, P3, and P4 domains from Table 6 used in each construct; Norm_D7 is the normalized counts at day 7; Enrich_DXX is the enrichment at day XX versus day 7, where XX is the day indicated on the table, for example, Enrich _D21 is the enrichment at day 21 versus day 7 and Enrich_D35 is the enrichment at day 35 versus day 7. Data for a construct is shown to the immediate right columns for the construct. Data for two constructs are shown in each row. For example, the first construct that is identified in Table 12 is E013-T047-S158-S080, which sets out P1-P2-P3-P4. In that construct, the transmembrane domain (P2) is T047, which is the transmembrane domain of IL7RA Ins PPCL (interleukin 7 receptor), as provided in Table 6, along with the amino acid sequence of this domain that was included in this construct. The same first intracellular domains (P3) and second intracellular domains (P4) included in Example 17 were included in the libraries in this example except that one of the P3 parts was a spacer (see below).

A total of 5 conceptualized possible combinations were designed based on a Jun leucine zipper extracellular domain with a 0-4 alanine spacer separating it from 82 different transmembrane domains, 81 possible P3 domains, one of which was a 23 amino acid spacer, and 21 possible P4 domains, one of which was a STOP codon. Not all of the conceptualized constructs were detected following viral vector assembly, lentiviral particle production, transduction of PMBC, and 7 days of culture. For Libraries 3A and 3B, 68,951 constructs were present after transduction of PBMCs and 7 days of culture. For Library 4B, 50,652 constructs were present after transduction of PBMCs and 7 days of culture. Detailed information about the candidates analyzed can be determined from Tables 6 and 12 to 17. The coding system for constructs is the same as explained for Example 17, as set out in the preceding paragraph.

For Libraries 3A, 3B, 3.1A, 3.1B, 4B, and 4,1B, constructs were identified that promoted PBMC cell proliferation between day 7 and the last day indicated on the table. Certain polypeptides in specific modules of the CLEs were among the top hits across the libraries 3A, 3B, and 4. These CLEs promoted proliferation to the the largest magnitude. For example, exemplary constructs containing CD40 or ICOS in the P2 position; MPL, MyD88, LEPR, or IFNAR2 in the P3 position; and/or CD40, CD79B, or CD27 in the P4 position were among the top 5 most frequent chimeric polypeptides in at least 2 of the 3 Libraries 3A, 3B, and 4B (see Tables 12, 13, and 16). Strikingly, MPL was the most frequent chimeric polypeptide at P3 by a large margin for all 3 libraries. Note the top hits from P3 (MPL, MyD88, LEPR, and IFNAR2) were not included in the P4 position for these constructs.

In Libraries 3A and 3B, which included constructs that encoded the chimeric polypeptides and a CAR and transduced PBMCs that were supplemented with (Library 3A) or without (Library 3B) fresh untransduced PBMCs, 126 and 127 top candidates were identified, respectively (See Tables 12 and 13) that promoted PBMC proliferation between day 7 and the last day indicated on the table when cultured in the absence of IL-2. The top candidate lists of Tables 12 and 13 were generated by combining the top 100 most prevalent candidates at day 21 based on initial interim data analysis, and the top 100 most enriched candidate constructs between day 21 and day 7 based on the initial interim data analysis. The initial interim data was generated by decoding part of the coded library such that 66 of the top 100 constructs, and 538 out of the top 1000 constructs had been decoded for library 3A; and 77 out of the top 100 constructs, and 464 out of the top 1000 constructs had been decoded for library 3B.

For Library 3A, transmembrane domains (P2) from CD40, CD8B, CRLF2, CSF2RA, FCGR2C, ICOS, IFNAR1, IFNGR1, IL10RB, IL18R1, IL18RAP, IL3RA, LEPR, and PRLR, or mutants thereof that are known to promote signaling activity in certain cell types if such mutants are present in the constructs provided in the tables, promoted proliferation of PBMCs between day 7 and day 21, when data is considered in a combined manner for all constructs with a transmembrane domain derived from that gene (data not shown). This conclusion is based on enrichment of sequence counts of constructs in mixed cultured PBMC cell populations, such that enrichment, calculated as the logarithm in base 2 of the ratio between normalized count at day 21 plus one and normalized count at day 7 plus one, was at least 2 for Library 3A, when results for all constructs for a gene are combined. For Library 3B, transmembrane domains from CD40, ICOS, CSF2RA, IL18R1, IL3RA, and TNFRSF14, were the best performers when analyzed in this way. Examples of transmembrane domains or portions and/or mutants thereof, that were present in constructs that promoted cell proliferation for Libraries 3A and 3B on day 35 and 21, respectively, are provided in Tables 12 and 13. Examples of transmembrane domains or portions and/or mutants thereof, that were present in constructs in the repeated screens referred to as Libraries 3.1A and 3.1B that promoted cell proliferation are provided in Tables 14 and 15.

For Library 3A, first intracellular (P3) domains from IL17RD, IL17RE, IL1RAP, IL23R, and MPL, or mutants thereof that are known to promote signaling activity in certain cell types if such mutants are present in the constructs provided in the tables, when found at the first intracellular domain (P3) of candidate chimeric polypeptides elements herein, promoted proliferation of PBMCs between day 7 and day 21, when data is considered in a combined manner for all constructs with a first intracellular domain derived from that gene (data not shown). This conclusion is based on enrichment of sequence counts of constructs in mixed cultured PBMC cell populations, such that enrichment, calculated as the logarithm in base 2 of the ratio between normalized count at day 21 plus one and normalized count at day 7 plus one, was at least 2 for Library 3A, when results for all constructs for a gene are combined. For Library 3B, first intracellular domains from IL21R, MPL, and IL2RB, or mutants thereof that are known to promote signaling activity in certain cell types if such mutants are present in the tables, were the best performers when analyzed in this way. Examples of first intracellular domains or portions and/or mutants thereof that were present in constructs that promoted the largest magnitude of cell proliferation for Libraries 3A and 3B for days 35 and 21, respectively, are provided in Tables 12 and 13. Examples of first intracellular domains or portions and/or mutants thereof, that were present in constructs in the repeated screens referred to as Libraries 3.1A and 3.1B that promoted cell proliferation are provided in Tables 14 and 15.

For Library 3A, second intracellular (P4) domains from CD27, CD3G, CD40, and CD79B, or mutants thereof that are known to promote signaling activity in certain cell types if such mutants are present in the tables, when found at the second intracellular domain (P4) of candidate chimeric polypeptides elements herein, promoted cell proliferation of PBMCs between day 7 and day 21, when data is considered in a combined manner for all constructs with a second intracellular domain derived from that gene (data not shown). This conclusion is based on enrichment of sequence counts of constructs in mixed cultured PBMC cell populations, such that enrichment, calculated as the logarithm in base 2 of the ratio between normalized count at day 21 plus one and normalized count at day 7 plus one, was at least 2 for Library 3A, when results for all constructs for a gene are combined. For Library 3B, second intracellular domains from CD40, or mutants thereof that are known to promote signaling activity in certain cell types if such mutants are present in the tables, were the best performers when analyzed in this way. Examples of second intracellular domains or portions and/or mutants thereof that were present in constructs that promoted the largest magnitude of cell proliferation for Libraries 3A and 3B for days 35 and 21, respectively, are provided in Tables 12 and 13. Examples of second intracellular domains or portions and/or mutants thereof, that were present in constructs in the repeated screens referred to as Libraries 3.1A and 3.1B that promoted the largest magnitude of cell proliferation are provided in Tables 14 and 15.

With respect to specific genes, of the 798 constructs with CD8B (CD8b molecule) as the P2 part, 111 were positive, which was defined as constructs having an enrichment above two (log2) for Libraries 3A or 3B on day 21. Of the 1,032 constructs with CD40 (CD40 molecule) as the P2 part, 159 were positive. Of the 1,339 constructs with CRLF2 (cytokine receptor like factor 2) as the P2 part, 175 were positive. Of the 593 constructs with CSF2RA (colony stimulating factor 2 receptor alpha subunit) as the P2 part, 108 were positive. Of the 714 constructs with FCGR2C (Fc fragment of IgG receptor IIc (gene/pseudogene)) as the P2 part, 88 were positive. Of the 788 constructs with ICOS (inducible T-cell costimulator) as the P2 part, 108 were positive. Of the 882 constructs with IFNAR1 (interferon alpha and beta receptor subunit 1) as the P2 part, 106 were positive. Of the 806 constructs with IFNGR1 (interferon gamma receptor 1) as the P2 part, 104 were positive. Of the 938 constructs with IL3RA (interleukin 3 receptor subunit alpha) as the P2 part, 132 were positive. Of the 746 constructs with IL10RB (interleukin 10 receptor subunit beta) as the P2 part, 99 were positive. Of the 814 constructs with IL18R1 (interleukin 18 receptor 1) as the P2 part, 131 were positive. Of the 552 constructs with IL18RAP (interleukin 18 receptor accessory protein) as the P2 part, 83 were positive. Of the 1,390 constructs with LEPR (leptin receptor) as the P2 part, 184 were positive. Of the 795 constructs with PRLR (prolactin receptor) as the P2 part, 123 were positive.

Of the 1,259 constructs with IL1RAP (interleukin 1 receptor accessory protein) as the P3 part, 158 were positive, which was defined as constructs having an enrichment above two for Libraries 3A or 3B on day 21. Of the 200 constructs with IL17RD (interleukin 17 receptor D) as the P3 part, 31 were positive. Of the 11 constructs with IL17RE (interleukin 17 receptor E) as the P3 part, 3 were positive. Of the 538 constructs with IL23R (interleukin 23 receptor) as the P3 part, 86 were positive. Of the 1,531 constructs with MPL (MPL proto-oncogene, thrombopoietin receptor) as the P3 part, 509 were positive.

Of the 3,124 constructs with CD3G (CD3g molecule) as the P4 part, 458 were positive, which was defined as constructs having an enrichment above two for Libraries 3A or 3B on day 21. Of the 3,293 constructs with CD27 (CD27 molecule) as the P4 part, 443 were positive. Of the 5,163 constructs with CD40 (CD40 molecule) as the P4 part, 724 were positive. Of the 4,486 constructs with CD79B (CD79b molecule) as the P4 part, 663 were positive. It is noteworthy for any construct that since this is a competitive assay, "negative" means that a construct was out competed with respect to promoting proliferation, but not that a construct is not capable of promoting proliferation.

For Libraries 3A and 3.1A, the constructs E008/E013-T041-S186-S050, E006/E011-T077-S186-S211, E007/E012-T021-S186-S051, E009/E014-T041-S186-S053, E007/E012-T073-S186-S053, E006/E011-T017-S186-S051, E006/E011-T031-S186-S211, E006/E011-T011-S186-S050, E006/E011-T011-S186-S047, E007/E012-T001-S186-S050, E006/E011-T041-S186-S051, E008/E013-T028-S186-S076, E009/E014-T029-S199-S053, E009/E014-T062-S186-S216, E007/E012-T006-S058-S051, E009/E014-T076-S186-S211, and E007/E012-T001-S186-S047 had particularly noteworthy enrichments in both screens, where the P1 parts separated by a slash here included different tags for Libraries 3A and 3A.1 as shown in Tables 6, 12, and 14. For the purposes of the repeated screens, constructs with particularly noteworthy enrichments were those that had a log₂((normalized count data on the last day + 1)/(normalized count data on day 7 + 1)) value above 2.

Further information about the first and second intracellular domains in constructs with particularly noteworthy enrichments in screens of both Library 3A and Library 3.1A is provided in Table 21, including the gene(s) the first and second intracellular domain are derived from, whether the first and/or second intracellular domain are cytokine receptors, and whether the first and/or second intracellular domain have at least one ITAM motif. Constructs with intracellular domains from MPL, OSMR, or CSF2RA, when present at the first intracellular domain (P3), and constructs with intracellular domains from CD40, TNFRSF4, CD79B, CD27, FCGR2A, or TNFRSF18, when present at the second intracellular domain (P4), showed particularly noteworthy enrichments in both Libraries 3A and 3.1A (Table 21). Constructs with domains from the cytokine receptors MPL, OSMR, or CSF2RA, when present at the first intracellular domain (P3), and constructs with domains from the cytokine receptors CD40, TNFRSF4, CD27, FCGR2A, or TNFRSF18, when present at the second intracellular domain (P4), showed particularly noteworthy enrichments in both Libraries 3A and 3.1A (Table 21). Constructs with domains containing ITAM motifs from MPL or OSMR, when present at the second intracellular domain (P4), showed particularly noteworthy enrichments in both Libraries 3A and 3.1A (Table 21).

For Libraries 3B and 3.1B, the constructs E007/E012-T017-S186-S051, E007/E012-T073-S 186-S053, E008/E013-T028-S186-S047, E006/E011-T011-S186-5047, E007/E012-T082-S176-S214, E006/E011-T046-S186-S052, E008/E013-T029-S186-S052, E009/E014-T011-S186-S053, E008/E013-T032-S186-S039, E007/E012-T034-S186-S051, E007/E012-T041-S192-S213, E006/E011-T014-S069-S213, E006/E011-T022-S186-S053, E006/E011-T023-S115-S075, E006/E011-T029-S106-S213, E006/E011-T032-S155-S080, E006/E011-T041-S186-S216, E006/E011-T057-S135-S080, E006/E011-T072-S191-X002, E006/E011-T077-S186-5216, E006/E011-T080-S141-5080, E007/E012-T001-X001-S214, E007/E012-T007-S059-S211, E007/E012-T016-S186-S052, E007/E012-T031-S186-S053, E007/E012-T044-S102-S052, E007/E012-T044-S142-X002, E007/E012-T055-S069-S053, E007/E012-T063-S176-S216, E007/E012-T065-S157-S075, E008/E013-T008-5085-X002, E008/E013-T011-S085-S048, E008/E013-T021-S109-X002, E008/E013-T021-S168-S211, E008/E013-T032-S064-S214, E008/E013-T037-S170-S215, E008/E013-T038-S176-S048, E008/E013-T039-S137-S216, E008/E013-T041-S141-S053, E008/E013-T045-S177-S048, E008/E013-T048-S109-S074, E008/E013-T073-S199-S075, E009/E014-T001-S157-S074, E009/E014-T005-S196-S049, E009/E014-T011-S130-X002, E009/E014-T013-S155-X002, E009/E014-T017-S186-S076, E009/E014-T021-S142-S080, E009/E014-T023-S082-S076, E009/E014-T038-S196-S037, EO09/E014-T055-S186-S052, E009/E014-T060-S175-S053, E009/E014-T070-S085-S212, E008/E013-T026-S054-S213, E009/E014-T007-S120-S053, E007/E012-T045-S186-S211, E008/E013-T073-S186-X002, E008/E013-T074-S186-X002, E007/E012-T055-S186-S053, E008/E013-T036-S186-S053, E007/E012-T017-S058-S053, E008/E013-T030-S189-S080, E006/E011- T029-S081-S047, E009/E014-T044-S194-S050, E006/E011-T028-S121-X002, E008/E013-T028-S186-S053, E009/E014-T078-S142-S213, E009/E014-T041-S186-S051, E008/E013-T006-S186-S050, E006/E011-T028-S186-S075, E006/E011-T040-S120-S038, E007/E012-T044-S115-S211, E009/E014-T039-S176-S075, E007/E012-T028-S186-S050, E008/E013-T031-S202-S050, E007/E012-T072-S192-S053, E006/E011-T065-X001-S051, E007/E012-T030-S062-X002, E007/E012-T073-S186-X002, E009/E014-T056-S186-S053, E008/E013-T046-S137-X002, E006/E011-T016-S136-S076, E007/E012-T032-S142-S037, E007/E012-T065-S120-S215, E009/E014-T077-S186-S047, E009/E014-T001-S126-S051, E006/E011-T030-S121-S039, E008/E013-T006-S176-S213, E009/E014-T032-S130-S215, E008/E013-T041-S186-S039, E009/E014-T021-S186-S047, E008/E013-T026-S137-S214, E007/E012-T029-S116-S075, E008/E013-T026-S106-S049, and E007/E012-T032-S168-S075 had particularly noteworthy enrichments in both screens, where the P1 parts separated by a slash here included different tags for Libraries 3B and 3.1B as shown in Tables 6, 13, and 15. For the purposes of the repeated screens, constructs with particularly noteworthy enrichments were those that had a log2₂((normalized count data on the last day + 1)/(normalized count data on day 7 + 1)) value above 2.

Further information about the first and second intracellular domains in constructs with particularly noteworthy enrichments in screens of both Library 3B and Library 3.1B is provided in Table 22, including the gene(s) the first and second intracellular domain are derived from, whether the first and/or second intracellular domain are cytokine receptors, and whether the first and/or second intracellular domain have at least one ITAM motif. Constructs with intracellular domains from MPL, LEPR, MYD88, EPOR, IL5RA, IL2RG, IL18RAP, IL11RA, IL13RA1, CSF2RA, IL1RL1, IL13RA2, IL20RB, IFNGR2, IL3RA, IL27RA, CSF3R, IL31RA, IL12RB1, OSMR, IL10RB, IFNAR2, CRLF2, IL7R, IFNAR1, PRLR, IL9R, IL6R, or IL15RA, when present at the first intracellular domain (P3), and constructs with intracellular domains from CD40, CD79B, CD27, TNFRSF14, CD79A, CD3G, TNFRSF9, FCGR2C, ICOS, TNFRSF18, TNFRSF4, CD28, FCER1G, FCGR2A, CD3D, TNFRSF8, or CD3E, when present at the second intracellular domain (P4), showed particularly noteworthy enrichments in both Libraries 3B and 3.1B (Table 22). Constructs with domains from the cytokine receptors MPL, LEPR, EPOR, IL5RA, IL2RG, IL18RAP, IL11RA, IL13RA1, CSF2RA, IL1RL1, IL13RA2, IL20RB, IFNGR2, IL3RA, IL27RA, CSF3R, IL31RA, IL12RB1, OSMR, IL10RB, IFNAR2, CRLF2, IL7R, IFNAR1, PRLR, IL9R, IL6R, or IL15RA, when present at the first intracellular domain (P3), and constructs with domains from the cytokine receptors CD40, CD27, TNFRSF14, TNFRSF9, FCGR2C, TNFRSF18, TNFRSF4, FCER1G, FCGR2A, or TNFRSF8, when present at the second intracellular domain (P4), showed particularly noteworthy enrichments in both Libraries 3B and 3.1B (Table 22). Constructs with domains containing ITAM motifs from CD79B, CD79A, CD3G, FCGR2C, FCER1G, FCGR2A, CD3D, or CD3E, when present at the second intracellular domain (P4), showed particularly noteworthy enrichments in both Libraries 3B and 3.1B (Table 22).

FACs analysis revealed that the Day 49 Library 3A expanded cells were primarily CD3+, CD8+, CD56+, and FLAG-Tag+. The table immediately below shows the percentage of lymphocytes that stained positive for the indicated marker and marker combinations. This data shows that the drivers identified from this library screen were primarily expanding the CD3+ T cell component.

| | **% of Lymphocytes** |
|---|---|
| **CD3+** | 98.11% |
| **CD3+CD4+** | 8.96% |
| **CD3+CD8+** | 87.00% |
| **CD3-CD56+** | 0.22% |
| **CD3+CD56+** | 79.13% |
| | |
| **CD3+FLAG+** | 79.93% |
| **CD3+CD4+FLAG+** | 0.77% |
| **CD3+CD8+FLAG+** | 79.66% |
| **CD3-CD56+FLAG+** | 3.33% |
| **CD3+CD56+FLAG+** | 78.14% |
| | |
| **CD19+FLAG+** | 0.03% |

For Library 4B, which included constructs that encoded the chimeric polypeptides but no CAR, and transduced PBMCs that were not supplemented with fresh untransduced PBMCs, 154 top candidates were identified (See Table 16) that promoted the largest magnitude of PBMC proliferation between day 7 and the last day indicated on the table when cultured in the absence of IL-2.

For Library 4B, transmembrane domains (P2) from CD40, CD8B, CSF2RA, IL18R1, and IL3RA, or mutants thereof that are known to promote constitutive signaling activity in certain cell types if such mutants are present in the constructs provided in the tables, when found at the transmembrane domain position (P2) of candidate chimeric polypeptides elements herein, promoted cell proliferation of PBMCs between day 7 and the last day indicated on the table, when data is considered in a combined manner for all constructs with a transmembrane domain derived from that gene. This conclusion is based on enrichment of sequence counts of constructs in mixed cultured PBMC cell populations, such that enrichment, calculated as the logarithm in base 2 of the ratio between normalized count at the last day indicated on the table plus one and normalized count at day 7 plus one, was at least 2 for Library 4B, when results for all constructs for a gene are combined. Examples of transmembrane domains or portions and/or mutants thereof, that were present in Library 4B in constructs that promoted cell proliferation are provided in Tables 16. Examples of transmembrane domains or portions and/or mutants thereof, that were present in constructs in the repeated screen referred to as Library 4.1B that promoted cell proliferation are provided in Table 17.

For Library 4B, first intracellular (P3) domains from CRLF2, CSF2RA, IFNGR2, IL4R, MPL, and OSMR or mutants thereof that are known to promote signaling activity in certain cell types if such mutants are present in the constructs provided in the tables, when found at the first intracellular domain (P3) of candidate chimeric polypeptides elements herein, promoted cell proliferation of PBMCs between day 7 and the last day indicated on the table, when data is considered in a combined manner for all constructs with a first intracellular domain derived from that gene. This conclusion is based on enrichment of sequence counts of constructs in mixed cultured PBMC cell populations, such that enrichment, calculated as the logarithm in base 2 of the ratio between normalized count at the last day indicated on the table plus one and normalized count at day 7 plus one, was above 0 for Library 4B, when results for all constructs for a gene are combined. Examples of first intracellular domains or portions and/or mutants thereof, that were present in constructs in Library 4B that promoted cell proliferation are provided in Table 16. Examples of first intracellular domains or portions and/or mutants thereof, that were present in constructs in the repeated screen referred to as Library 4.1B that promoted cell proliferation are provided in Table 17.

For Library 4, second intracellular (P4) domains from CD27, CD40, and CD79B, or mutants thereof that are known to promote signaling activity in certain cell types if such mutants are present in the constructs provided in the tables, when found at the second intracellular domain (P4) of candidate chimeric polypeptides elements herein, promoted proliferation of PBMCs between day 7 and the last day indicated on the table, when data is considered in a combined manner for all constructs with a second intracellular domain derived from that gene. This conclusion is based on enrichment of sequence counts of constructs in mixed cultured PBMC cell populations, such that more counts for such domains were present at the last day than day 7 for Library 4B, when results for all constructs for a gene are combined. Examples of second intracellular domains or portions and/or mutants thereof, that were present in constructs in Library 4B that promoted cell proliferation are provided in Table 16. Examples of second intracellular domains or portions and/or mutants thereof, that were present in constructs in the repeated screen referred to as Library 4.1B that promoted cell proliferation are provided in Table 17.

For Libraries 4B and 4.1B, the constructs E007/E012-T078-S154-S047, E008/E013-T062-S186-X002, E008/E013-T055-S186-S050, E009/E014-T057-S186-S050, E007/E012-T077-S054-S053, E007/E012- T034-S135-S211, E009/E014-T071-X001-S216, E009/E014-T011-S141-S037, E008/E013-T041-S186-S037, E006/E011-T038-S106-S039, E006/E011-T011-S121-X002, E007/E012-T007-5085-S215, E006/E011- T041-S 186-S050, E007/E012-T008-S064-S051, E006/E011-T041-S186-S047, E008/E013-T045-S186-S051, E008/E013-T003-S104-S216, E006/E011-T019-S186-S053, E008/E013-T071-S064-S080, E006/E011-T021-S054-X002, E006/E011-T003-S135-X002, E009/E014-T020-S199-S213, E008/E013-T027-S121-S211, E009/E014-T032-S195-X002, E009/E014-T050-S171-X002, E008/E013-T069-S083-X002, E008/E013-T026-S116-S038, E009/E014-T072-S195-X002, E007/E012-T047-S058-S211, E008/E013-T046-S142-5080, E006/E011-T065-S186-S076, E006/E011-T062-S069-X002, E007/E012-T047-S098-X002, E009/E014-T069-S099-S048, E008/E013-T039-S141-S050, E006/E011-T052-S130-S052, E008/E013-T041-S186-X002, E007/E012-T019-S120-X002, E008/E013-T045-S186-S053, E006/E011-T003-S170-5039, E007/E012-T047-S058-S051, E007/E012-T069-S109-X002, E009/E014-T019-S130-S075, and E006/E011-T047-S054-S053 had particularly noteworthy enrichments in both screens, where the P1 parts separated by a slash here included different tags for Libraries 4B and 4.1B as shown in Tables 6, 16, and 17. For the purposes of the repeated screens, constructs with particularly noteworthy enrichments were those that had a log₂((normalized count data on the last day + 1)/(normalized count data on day 7 + 1)) value above 2.

Further information about the first and second intracellular domains in constructs with particularly noteworthy enrichments in screens of both Library 4B and Library 4.1B is provided in Table 23, including the gene(s) the first and second intracellular domain are derived from, whether the first and/or second intracellular domain are cytokine receptors, and whether the first and/or second intracellular domain have at least one ITAM motif. Constructs with intracellular domains from IL18R1, MPL, CRLF2, IL11RA, IL13RA1, IL2RG, IL7R, IFNGR2, CSF3R, IL2RA, OSMR, MYD88, IL31RA, IFNAR2, IL6R, CSF2RA, IL13RA2, EPOR, IL1R1, IL10RB, or IL3RA, when present at the first intracellular domain (P3), and constructs with intracellular domains from CD27, CD40, CD79B, TNFRSF4, TNFRSF18, CD3D, CD3G, CD27, ICOS, TNFRSF9, CD3E, FCGR2A, CD28, CD79A, or FCGR2C, when present at the second intracellular domain (P4), showed particularly noteworthy enrichments in both Libraries 4B and 4.1B (Table 23). Constructs with domains from the cytokine receptors IL18R1, MPL, CRLF2, IL11RA, IL13RA1, IL2RG, IL7R, IFNGR2, CSF3R, IL2RA, OSMR, IL31RA, IFNAR2, IL6R, CSF2RA, IL13RA2, EPOR, IL1R1, IL10RB, or IL3RA, when present at the first intracellular domain (P3), and constructs with domains from the cytokine receptors CD27, CD40, TNFRSF4, TNFRSF18, TNFRSF9, FCGR2A, or FCGR2C, when present at the second intracellular domain (P4), showed particularly noteworthy enrichments in both Libraries 4B and 4.1B (Table 23). Constructs with domains containing ITAM motifs from CD79B, CD3D, CD3G, CD3E, FCGR2A, CD79A, or FCGR2C, when present at the second intracellular domain (P4), showed particularly noteworthy enrichments in both Libraries 4B and 4.1B (Table 23).

For Library 4B, of the 572 constructs with CD8B (CD8b molecule) as the P2 part, 21 were positive, which was defined as constructs having an enrichment above two for Library 4B. Of the 897 constructs with CD40 (CD40 molecule) as the P2 part, 50 were positive. Of the 551 constructs with CSF2RA (colony stimulating factor 2 receptor alpha subunit) as the P2 part, 25 were positive. Of the 732 constructs with IL3RA (interleukin 3 receptor subunit alpha) as the P2 part, 47 were positive. Of the 738 constructs with IL18R1 (interleukin 18 receptor 1) as the P2 part, 44 were positive. Of the 1,683 constructs with MPL (MPL proto-oncogene, thrombopoietin receptor) as the P3 part, 305 were positive.

In the initial interim analysis for Library 3A at day 21, 66 of the top 100 hits and 538 of the top 1000 hits had been decoded. Further decoding provided deep decoding and the identity of 99 of the top 100 hits and 992 of the top 1000 hits of Library 3A and data was collected for day 35 and analysis was updated at day 21. In the initial analysis for Library 3B at day 21, 77 of the top 100 hits and 464 of the top 1000 hits had been decoded in the initial interim analysis. Further decoding provided deep decoding and the identity of 100 of the top 100 hits and 708 of the top 1000 hits of Library 3B. The top constructs for Libraries 3A and 3B with day 35 data for Library 3A and day 21 data for Library 3B are shown in Tables 12 and 13, respectively.

In Libraries 3A and 3B, which included constructs that encoded the chimeric polypeptides and a CAR and transduced PBMCs that were supplemented with (Library 3A) or without (Library 3B) fresh untransduced PBMCs, after deep decoding 124 top candidates were identified for Library 3A at day 35, and 131 top candidates were identified for library 3B at day 21 (See Tables 12 and 13, respectively) that promoted the largest magnitude of PBMC proliferation between day 7 and the last day indicated on the table when cultured in the absence of IL-2 (i.e. IL-2 was not added to the culture medium during culturing after the initial transduction). The top candidate lists of Tables 12 (Library 3A day 35) and 13 (Library 3B day 21) were generated by combining the top 100 most prevalent candidates at day 21 (Library 3B) or day 21 or 35 (Library 3A) and the top 100 most enriched candidate constructs between day 21 and day 7 (Library 3B) or between day 21 and day 7 or day 35 and day 7 (Library 3A) after deep decoding.

From the top hits tables using results with the deep decoding certain polypeptides in specific modules of the CLEs were among the top hits across at least one of Libraries 3A and 3B and for at least one of the time points of day 21 and day 35 (Library 3A) or day 21 (Library 3B). For example, exemplary constructs containing CD40 (specifically for example the transmembrane domain with the code T011), ICOS (specifically for example the transmembrane domain with the code T028), FCGR2C (specifically for example transmembrane domain with the code T024), PRLR (specifically for example the transmembrane domain with the code T077), IL3RA (specifically for example the transmembrane domain with the code T041), IL6ST (specifically for example the transmembrane domain with the code T045) in the P2 position, IL18R1 (specifically for example the transmembrane domain with the code T062) in the P2 position;
MPL (specifically for example the intracellular domain with the code S186), IL18R1 (specifically for example the intracellular domain with the code S154), IL13RA2 (specifically for example the intracellular domain with the code S142), IL10RB (specifically for example the intracellular domain with the code S130), LEPR (specifically for example intracellular domains with the code S176), IFNAR2 (specifically for example the intracellular domain with the code S083), IL23R (specifically for example the intracellular domain with the code S165), MyD88 (specifically for example the intracellular domain with the code S194), and CSF2RA (specifically for example the intracellular domain with the code S058), in the P3 position; and/or CD40 (specifically for example an intracellular domain with the code S050 or S051), CD79B (specifically for example the intracellular domain with the code S053), TNFRSF4 specifically for example the intracellular domain with the code S211), TNFRSF9 (specifically for example the intracellular domain with the code S213), TNFRSF14 (specifically for example the intracellular domain with the code S214), FCGRA2 (specifically for example the intracellular domains with the code S076), CD3G (specifically for example the intracellular domain with the code S039), or CD27 (specifically for example the intracellular domain with the code S047) in the P4 position were among the top 5 most frequent chimeric polypeptides (see Tables 12 and 13). Strikingly, MPL was the most frequent chimeric polypeptide at P3 by a large margin for both Libraries 3A and 3B. Note the top hits parts from P3 were not included in the P4 position for these constructs in Library 3A or 3B.

The following additional observations are noteworthy: P2_CD40, P2_ICOS, P3_MPL, P4_CD40, and P4_CD79B came up as most frequent parts in the top hits in each of Tables 12 and 13. P4_CD27 came up as a most frequent part in Tables 12 and 13. CSF2RB came up 4 times in top 123 most enriched and most rep at P2, but mutant V449E mutant did not occur in these top hits (Table 12). Accordingly, in some embodiments herein a chimeric polypeptide comprises a transmembrane domain identified in any of the constructs shown in Tables 12 and 13 other than a transmembrane domain mutant V449E of CSF2RB. Eight MyD88 variants occurred at the P3 position at least once in the Library 3A top 123 (Table 12). Both variants of CD40 tested in the library occurred at least once at the P4 position in the top 123 list for Library 3A (Table 12). The combination of MPL at P3 and CD40 at P4 showed up in 26 constructs in the list of the top 123 constructs of Library 3A (Table 12).

For gene by gene analysis, for Library 3A, transmembrane domains (P2) from CD4, CD8B, CD40, CRLF2, CSF2RA, CSF3R, EPOR, FCGR2C, GHR, ICOS, IFNAR1, IFNGR1, IFNGR2, IL1R1, IL1RAP, IL2RG, IL3RA, IL5RA, IL6ST, IL7RA, IL10RB, IL11RA, IL13RA2, IL17RA, IL17RB, IL17RC, IL17RE, IL18R1, IL18RAP, IL20RA, IL22RA1, IL31RA, LEPR, PRLR, and TNFRSF8, or mutants thereof that are known to promote signaling activity in certain cell types if such mutants are present in the constructs provided in the tables, promoted proliferation of PBMCs between day 7 and the last day indicated on the table, when data is considered in a combined manner for all constructs with a transmembrane domain derived from that gene (Table 12). This conclusion is based on enrichment of sequence counts of constructs in mixed cultured PBMC cell populations, such that enrichment, calculated as the logarithm in base 2 of the ratio between normalized count at the last day indicated on the table plus one and normalized count at day 7 plus one, was at least 2 for Library 3A, when results for all constructs for a gene are combined. For Library 3B (Table 13), transmembrane domains from CD40, ICOS, and IL18R1, or mutants thereof that are known to promote signaling activity in certain cell types if such mutants are present in the constructs provided in the tables, were the best performers when analyzed in this way. Examples of transmembrane domains or portions and/or mutants thereof, that were present in constructs that promoted the largest magnitude of cell proliferation for Library 3A and 3B are provided in Tables 12 and 13. Examples of transmembrane domains or portions and/or mutants thereof, that were present in constructs in the repeated screens referred to as Libraries 3.1A and 3.1B that promoted cell proliferation are provided in Tables 14 and 15.

For Library 3A, first intracellular (P3) domains from CSF2RA, IFNAR1, IL1RAP, IL4R, IL6ST, IL11RA, IL12RB2, IL17RA, IL17RD, IL17RE, IL18R1, IL21R, IL23R, MPL, and MyD88, or mutants thereof that are known to promote signaling activity in certain cell types if such mutants are present in the constructs provided in the tables, when found at the first intracellular domain (P3) of candidate chimeric polypeptides elements herein, promoted cell proliferation of PBMCs between day 7 and the last day indicated on the table, when data is considered in a combined manner for all constructs with a first intracellular domain derived from that gene. This conclusion is based on enrichment of sequence counts of constructs in mixed cultured PBMC cell populations, such that enrichment, calculated as the logarithm in base 2 of the ratio between normalized count at the last day indicated on the table plus one and normalized count at day 7 plus one, was at least 2 for Library 3A, when results for all constructs for a gene are combined. For Library 3B, first intracellular domains from CSF2RB, IL4R, and MPL, or mutants thereof that are known to promote signaling activity in certain cell types if such mutants are present in the constructs provided in the tables, were the best performers when analyzed in this way. Examples of first intracellular domains or portions and/or mutants thereof that were present in constructs that promoted the largest magnitude of cell proliferation for Libraries 3A and 3B are provided in Tables 12 and 13. Examples of first intracellular domains or portions and/or mutants thereof, that were present in constructs in the repeated screens referred to as Libraries 3.1A and 3.1B that promoted cell proliferation are provided in Table 14 and 15.

For Library 3A, second intracellular (P4) domains from CD3D, CD3G, CD27, CD40, CD79A, CD79B, FCER1G, FCGRA2, ICOS, TNFRSF4, and TNFRSF8, or mutants thereof that are known to promote signaling activity in certain cell types if such mutants are present in the constructs provided in the tables, when found at the second intracellular domain (P4) of candidate chimeric polypeptides elements herein, promoted proliferation of PBMCs between day 7 and day 21 or between day 7 and the last day indicated on the table, when data is considered in a combined manner for all constructs with a second intracellular domain derived from that gene. This conclusion is based on enrichment of sequence counts of constructs in mixed cultured PBMC cell populations, such that enrichment, calculated as the logarithm in base 2 of the ratio between normalized count at the last day indicated on the table plus one and normalized count at day 7 plus one, was at least 2 for Library 3A, when results for all constructs for a gene are combined. For Library 3B, second intracellular domains from CD40, or mutants thereof that are known to promote signaling activity in certain cell types if such mutants are present in the tables, although not achieving a 2-fold log2 enrichment, were the best performers when analyzed in this way. Examples of second intracellular domains or portions and/or mutants thereof that were present in constructs that promoted the largest magnitude of cell proliferation for Libraries 3A and 3B are provided in Tables 12 and 13. Examples of second intracellular domains or portions and/or mutants thereof, that were present in constructs in the repeated screens referred to as Libraries 3.1A and 3.1B that promoted cell proliferation are provided in Tables 14 and 15.

### Example 19. Thermal Denaturation of F1A-795 in the Absence and Presence of Acyclovir by Differential Scanning Calorimetry (DSC).

In this example, the binding of a nucleoside analogue antiviral drug to an aptamer domain of a riboswitch (SEQ ID NO:87) was demonstrated by comparing the thermal denaturation of the aptamer domain in the absence versus presence of the nucleoside analogue antiviral drug acyclovir.

### Aptamer preparation

The T7 primer (SEQ ID NO:246) as well as the template (reverse complement) of the identified candidate sequence was synthesized by IDT (Coralville, IA) as single-stranded DNA. Candidates were primer-extended by combining components to 1 µM template, 2 µM T7 primer, 200 µM dNTPs, 1X Titanium Taq DNA Polymerase, and 1X Titanium Taq buffer (Clontech Laboratories; Mountain View, CA), then heating for 3 minutes at 95 °C, 1 minute at 55 °C, and 2 minutes at 68 °C (no cycling). 42 pmoles of double-stranded DNA were used for twelve 20-µl reactions with the Ampliscribe T7 High Yield Transcription Kit (Lucigen; Middleton, WI) according to standard kit directions (1X reaction buffer, 7.5 mM each NTP, 10 mM DTT, 1X RiboGuard RNase inhibitor, 1X Ampliscribe T7 enzyme solution). Reactions were incubated at 42 °C overnight, then stopped by adding 1 µl of DNase I and incubating for 15 minutes at 37 °C. Transcription products were purified on 10% denaturing PAGE with 8 M urea. At least 10 nmole of each candidate was lyophilized, and resuspended in 1X Binding Buffer (50 mM HEPES, 100 mM KCl, 0.5 mM MgCl₂, pH 7.3) on the day of binding assessment.

Briefly, DSC was used to analyze F1A-795 (7.2 µM) in the absence of acyclovir or F1A-795 (2.77 µM) in the presence of acyclovir (29.4 µM). All analyses were conducted in 1X Binding Buffer and all water used was DEPC-treated. All analytes were resuspended first in DEPC-treated water then diluted to their final listed concentration in 1X Binding Buffer. Prior to loading of the DSC (GE Healthcare MicroCal VP-DSC), the samples were degassed at 25 °C for 10 minutes. The sample without acyclovir was scanned from 10 °C to 115 °C at a rate of 1 °C per minute. The sample with acyclovir was scanned from 10 °C to 105 °C at a rate of 1 °C per minute.

### Results

The thermal denaturation of F1A-795 in the absence of acyclovir as measured by DSC has a transition centered at about 60 °C (FIG. 28). This transition suggests the aptamer domain is structured in the absence of acyclovir. In the presence of acyclovir, F1A-795 has a transition centered at about 75 °C (FIG. 28). This stabilizing effect indicates the nucleoside analogue antiviral drug acyclovir binds to the aptamer domain F1A-795. Thus, this experiment confirmed that F1A-795 is bound by acyclovir.

### Example 20. Transduction efficiency of freshly isolated and unstimulated human T cells by retroviral particles containing a VSV-G pseudotyping element, and a membrane-bound activation element, and optionally containing Vpu protein.

Recombinant lentiviral particles were produced by transient transfection of 293T cells (Lenti-X^{™} 293T, Clontech) with separate lentiviral packaging plasmids encoding gag/pol and rev, and a pseudotyping plasmid encoding VSV-G. A third generation lentiviral expression vector encoding GFP, an anti-CD19 chimeric antigen receptor, and an eTAG referred to herein as F1-0-03 (FIG. 20) was co-transfected with the packaging plasmids. The cells were adapted to suspension culture by serial growth in Freestyle^{™} 293 Expression Medium (ThermoFisher Scientific). The cells in suspension were seeded at 1 × 10⁶ cells/mL (30 mL) in a 125 mL Erlenmeyer flask, and immediately transfected using polyethylenimine (PEI) (Polysciences) dissolved in weak acid.

Plasmid DNA was diluted in 1.5 ml Gibco^{™} Opti-MEM^{™} media for 30 mL of cells. To obtain lentiviral particles pseudotyped with VSV-G, the total DNA (1 µg/mL of culture volume) used was a mixture of 4 plasmids with the following molar ratios: 2x genomic plasmid (F1-0-03), 1x Rev-containing plasmid, 1x VSV-G-containing plasmid, and 1x gag/pol-containing plasmid. To obtain lentiviral particles pseudotyped with VSV-G and displaying anti-CD3-scFvFc-GPI on their surfaces, the total DNA (1 µg/mL of culture volume) used was a mixture of 5 plasmids with the following molar ratios: 2x genomic plasmid (F1-0-03), 1x Rev-containing plasmid, 1x VSV-G-containing plasmid, 1x anti-CD3-scFvFc-GPI-containing plasmid, and 1x gag/pol-containing plasmid. To obtain lentiviral particles pseudotyped with VSV-G, displaying anti-CD3-scFvFc-GPI, and containing the accessory protein Vpu, the total DNA (1 µg/mL of culture volume) used was a mixture of 6 plasmids with the following molar ratios: 2x genomic plasmid (F1-0-03), 1x Rev-containing plasmid, 1x VSV-G-containing plasmid, 1x anti-CD3-scFvFc-GPI containing plasmid, 1x Vpu-CH--containing plasmid, and 1x gag/pol-containing plasmid. Separately, the PEI was diluted in 1.5 ml Gibco^{™} Opti-MEM^{™} to 2 µg/mL (culture volume, 2:1 ratio to DNA). After a 5-minute room temperature incubation, the two solutions were mixed together thoroughly, and incubated at room temperature for 20 more minutes. The final volume (3 ml) was added to the cells. The cells were then incubated at 37 °C for 72 hours with rotation at 125 rpm and with 8% CO₂. The antiCD3-scFvFc-GPI containing plasmid includes an scFv derived from UCHT1, and a GPI anchor attachment sequence derived from DAF (CD55). The UCHT1scFvFc-GPI vector encodes a peptide (SEQ ID NO:278) that includes human Ig Kappa signal peptide (amino acids 1-22 of NCBI GI No. CAA45494.1) fused to the UCHT1 scFv (amino acids 21-264 of NCBI GI No. CAH69219.1), fused to human IgG1 Fc (amino acids 1-231 of NCBI GI No. AEV43323.1), fused to the human DAF GPI anchor attachment sequence (amino acids 345-381 of NCBI GI No. NP_000565). The Vpu-CH containing plasmid encodes a peptide (MFDFIARVDYRVGVVALVVALIIAIIVWSIVYIEYRKLLKQRKIDWLIKRIRERAEDSGNESDGEI EELSTMVDMEHLRLLDDL*) that includes the Vpu sequence from the HIV-1 M CH167-CC isolate (amino acids 1-84, GenBank: AGF30946.1).

After 72 hours, the supernatants were harvested and clarified by centrifugation at 1,200g for 10 minutes. The clarified supernatants were decanted to a new tube. The lentiviral particles were precipitated by overnight centrifugation at 3,300g, at 4 °C. The supernatant was discarded, and the lentiviral particle pellets were resuspended in 1:100 of initial volume of X-Vivo^{™} 15 medium (Lonza). Lentiviral particles were titered by serial dilution and analysis of GFP expression, in 293T and Jurkat cells, 72 hours post-transduction, by flow cytometry.

Peripheral blood mononuclear cells (PBMCs) were isolated from a buffy coat, collected and distributed by the San Diego Blood Bank, CA. SepMate^{™} 50 (Stemcell^{™})-based gradient density separation of PBMCs on Ficoll-Paque PLUS^{®} (GE Healthcare Life Sciences) was performed per manufacturers' instructions. 30 mL of buffy-coat diluted in X-Vivo^{™} 15 medium were layered per each SepMate^{™} tube. After centrifugation at room temperature, at 1,200g, for 20 min, the PBMC layers were collected, pooled and washed three times with 45 mL of X-Vivo^{™} 15 medium and centrifugation at 400g for 10 min at room temperature. The pellets were then incubated at room temperature for 10 min in 10 mL of RBC lysis buffer (Alfa Aesar) and washed an additional two times with 45 mL of X-Vivo^{™} 15 medium, and centrifugation at 400g for 10 min at room temperature. No additional steps were taken to remove monocytes. After isolation, fresh and unstimulated PBMCs were resuspended to a final concentration of 1 x 10⁶/mL in X-Vivo^{™} 15 medium, and were transduced, in duplicates, with the lentiviral particles disclosed previously. The transductions were conducted for 3 days, at 37°C, 5% CO₂, in X-Vivo^{™} 15 medium. Transductions were conducted at MOI 10 for [VSV-g]pp and MOI 1 for [VSV-G + UCHT1]pp and [VSV-G + UCHT1 + Vpu-CH]pp, in a 12 wells plate format, 1 mL/well, in duplicates. After 3 days of transduction, samples were collected and analyzed by FACS for absolute cell count and GFP expression levels in the CD3+ population.

FIG. 34A and FIG. 34B show histograms of the percentage (%)CD3+GFP+ cells in the total CD3+ population (FIG. 34A) and a histogram of the absolute cell count per well of the Live CD3+ population (FIG 34B), respectively, 3 days after transduction of freshly isolated and unstimulated PBMCs with the indicated lentiviral particles. Each bar represents the mean +/- SD of duplicates. FIG. 34A shows that adding an antiCD3-scFvFc-GPI moiety (UCHT1) to VSV-G pseudotyped lentiviral particles results in higher transduction efficiencies of freshly isolated and unstimulated PBMCs compared to [VSV-G]pp alone. FIG. 34A also shows that the addition of Vpu-CH can further improve the transduction efficiency of the dually pseudotyped vector [VSV-G + UCHT1]pp. FIG. 34B shows that the UCHT1 moiety enhances cell stimulation by absolute cell count per uL as compared to VSV-G only. The addition of Vpu and UCHT1 similarly enhances cell stimulation by absolute cell count as compared to VSV-G only.

### Example 21. Characterization of individual lymphoproliferative elements.

In this example, select chimeric lymphoproliferative elements (CLEs) identified in the library screens described in Example 17 and Example 18 were assessed individually. To further analyze CLEs identified in the screens of Library 2B, activated PBMCs were transduced overnight with lentiviral particles encoding a CLE alone and cultured in the absence of exogenous cytokines. To further analyze CLEs identified in the screens of Library 3A, activated PBMCs were transduced overnight with lentiviral particles encoding a CLE flanked with an anti-CD19 CAR at the 5' end, and cultured in the presence of donor-matched CD19+ B cells added to the culture every 7 days, but in the absence of exogenous cytokines. The cells were cultured for up to 35 days to assess the ability of the CLEs to promote PBMC proliferation. This example further provides methods by which to characterize any individual putative lymphoproliferative element and further confirms the identities of several highly active CLEs.

Recombinant lentiviral particles were produced in 293T cells (Lenti-X^{™} 293T, Clontech) that were adapted to suspension culture in Freestyle^{™} 293 Expression Medium (Thermo Fisher Scientific) serum-free chemically defined medium. The cells were transiently transfected using PEI with a genomic plasmid and 3 separate lentiviral packaging plasmids encoding gag/pol, rev, and a pseudotyping plasmid encoding VSV-G as explained in Example 20. Selected CLEs identified in the library screens described in Examples 17 and 18 were regenerated individually from their parts (P1-2, P3, and P4 or P1, P2, P3, and P4) and inserted into the same transgene expression cassettes as that of the library from which they were first identified. The module parts of each of the selected CLEs are shown in FIGs. 35 and 36 and the gene names and amino acids sequences are shown in Table 6. FIGs. 31 and 32 show the cassettes for Libraries 2 and 3, respectively. Cells transduced with lentiviral particles containing constructs identified through library screens are referred to as "DL" followed by the library number. For example, cells transduced with lentiviral particles containing constructs from Library 2 are referred to as DL2. Thus, CLEs with the prefix "DL2" and "DL3" were constructed in the cassette shown in FIGs. 31 and 32, respectively. Similarly, "DC1-5" and "DC1-6" comprise CLEs inserted into the transgene expression cassette used for Library 1 as shown in FIG. 30. The chimeric lymphoproliferative element of DC1-5 encoded from amino to carboxy terminus, human IL-7 (SEQ ID NO:511), a flexible linker (SEQ ID NO:53), and residues 21-458 of the human IL-7Rα (SEQ ID NO:229) which is the full length IL-7Rα without its signal peptide. The CLE of DC1-6 encoded from amino to carboxy terminus, IL-7 (SEQ ID NO:511), a flexible linker (SEQ ID NO:53), the extracellular and transmembrane portions of the IL-7Rα (CD127) (SEQ ID NO:513), and the intracellular domain of the IL-2Rβ (CD122) (SEQ ID NO:514). The chimeric proteins IL-7 - IL-7Rα and IL-7 - IL-7Rα - IL-2Rβ have been described previously (Hunter et al. Molecular Immunology 56(2013):1-11). The IL-7 "DC2-5" and "DC2-6" comprise CLEs inserted into the transgene expression cassette used for Library 2 as shown in FIG. 31. The CLEs in DC2-5 and DC2-6 were the same CLEs as DC1-5 and DC1-6, respectively. Untransduced PBMCs (NT), PMBCs transduced with a vector that encoded an eTag but not a CLE (F1-0-01), and PMBCs transduced with a vector that encoded an anti-CD19 CAR and an eTag but not a CLE (F1-3-23) were included as negative controls.

The constructs from Library 2B used in this Example were: DL2-1 (M024-S190-S047), DL2-2 (M025-S050-S197), DL2-3 (M036-S170-S047), DL2-4 (M012-S045-S048), DL2-5 (M049-S194-S064), DL2-6 (M025-S190-S050), and DL2-7 (M025-S190-S051).

The constructs from Library 3A used in this Example were: DL3A-1 (E013-T047-S158-S080), DL3A-2 (E011-T024-S194-S039), DL3A-3 (E014-T040-S135-S076), DL3A-4 (E013-T041-S186-S051), DL3A-5 (E013-T064-S058-S212), DL3A-6 (E013-T028-S186-S051), DL3A-7 (E014-T015-S186-S051), DL3A-8 (E011-T016-S186-S050), DL3A-9 (E011-T073-S186-S050), and DL3A-10 (E013-T011-S186-S211).

On Day 0, PBMCs were enriched from buffy coats (San Diego Blood Bank) by density gradient centrifugation with Ficoll-Paque PREMIUM^{®} (GE Healthcare Life Sciences) according to the manufacturer's instructions followed by lysis of red blood cells. 1.5 x 10⁶ viable PBMCs were seeded in the wells of G-Rex 6 Well Plates (Wilson Wolf, 80240M) in 3ml Complete OpTmizer^{™} CTS^{™} T-Cell Expansion SFM supplemented with 100 IU/ml (IL-2), and 50 ng/ml anti-CD3 antibody (317326, Biolegend) to activate the PBMCs for viral transduction. After incubation overnight at 37 °C and 5% CO₂, lentiviral particles encoding the constructs described above were added directly to the activated PBMCs at an MOI of 5 and incubated overnight at 37°C and 5% CO2. The following day, the media volume in each well was brought to 30ml with Complete OpTmizer^{™} CTS^{™} T-Cell Expansion SFM and the plates were returned to the incubator. No IL-2, IL-7, or other exogenous cytokine was added at this or subsequent cell culture steps. The cells from each well were collected on Day 7 to determine cell numbers, percent viability, and percent transduced cells, which was defined as the percent of FLAG-Tag+ or E-Tag+ cells by FACS analysis. The cells were then centrifuged, resuspended in fresh Complete OpTmizer^{™} CTS^{™} T-Cell Expansion SFM, and 0.5 x 10⁶ cells for each sample were re-seeded into the well of a G-Rex 6 Well Plate in 30ml of Complete OpTmizer^{™} CTS^{™} T-Cell Expansion SFM. Previously cryopreserved Day 0 donor matched PBMCs containing 0.5 x 10⁶ CD19+ B cells (as determined by FACS on Day 0) were added to cultures of "DL3A-" to provide CD19+ B-cell activation of the CD19 ASTR. This process was repeated on days 14, 20, and 28 before the cells were harvested on Day 35.

### RESULTS

PBMCs transduced with lentiviral particles encoding CLEs were cultured in the absence of exogenous cytokines for 35 days. Proliferation is represented as fold expansion which was calculated by dividing the total number of cells by the number of cells seeded for that time point. PBMCs transduced with each individual selected CLE shown in FIG. 35 proliferated more than the negative controls, which included untransduced PBMCs and PMBCs transduced with a vector that encoded an eTag but not a CLE (FIG. 35). Furthermore, the following CLEs exhibited a fold expansion greater than 23 at days 14, 21, 28, and 35 while the fold expansion of the negative controls was near 0 for these timepoints: DL2-7, DC2-6, DL2-6, DL2-2, DL2-1, and DC2-5. PBMCs transduced with constructs encoding an anti-CD19 CAR and various CLEs (shown in FIG. 36) and cultured in the presence of fresh donor matched PBMCs added every 7 days, but in the absence of exogenous cytokines, proliferated more than the negative controls, which included untransduced PBMCs and PMBCs transduced with a vector that encoded an anti-CD19 CAR and an eTag but not a CLE. All of the CLEs shown in FIG. 36 exhibited a fold expansion greater than 23 at day 35 while the fold expansion of the negative controls was near 0 at day 35. The proliferation of PBMCs transduced with lentiviral particles containing polynucleotides encoding DL3A-1 (E013-T047-S158-S080), DL3A-2 (E011-T024-S194-S039), DL3A-3 (E014-T040-S135-S076), and DL3A-5 (E013-T064-S058-S212) were comparable to the negative controls and are not shown on FIG. 36.

### Example 22. Proof of Concept for various illustrative methods provided herein, including in vivo expansion of genetically modified Lymphocytes.

This example provides exemplary methods for transducing PBMCs, including T cells, ex vivo and expanding those transduced PBMCs, including T cells, in vivo. Such methods include an illustrative 4 hour transduction method with illustrative recombinant lentiviral vectors that express certain illustrative chimeric lymphoproliferative elements. Furthermore, such methods provide additional exemplary methods for transducing PBMCs, which in illustrative embodiments are typically T cells, with replication incompetent recombinant retroviral particles in 4 hours wherein the replication incompetent recombinant retroviral particles were produced by transfecting packaging cells with vectors encoding various components of the replication incompetent recombinant retroviral particles in suspension in serum-free, chemically defined media.

### Materials and Methods

Recombinant lentiviral particles were produced in 293T cells (Lenti-X^{™} 293T, Clontech) that were adapted to suspension culture in Freestyle^{™} 293 Expression Medium (Thermo Fisher Scientific) serum-free chemically defined medium. The cells were transiently transfected using PEI with 1 of 3 genomic plasmids (detailed below) and 3 separate lentiviral packaging plasmids encoding gag/pol, rev, and a pseudotyping plasmid encoding VSV-G as explained in Example 20. To produce retroviral particles that also display a membrane-bound activation element, a fourth packing plasmid encoding a membrane-bound polypeptide capable of binding to CD3 (UCHT1scFvFc-GPI) was co-transfected as described in Example 20. The genomic plasmids were third generation lentiviral expression vectors containing a deletion in the 3'LTR leading to self-inactivation wherein the plasmids encoded the following:
(1) Anti-ROR2 MRB-CAR:T2A:eTag (F1-1-27): This genomic plasmid is identical to that shown in FIG. 20 except that the sequence encoding anti-CD19 CAR was replaced with an MRB-ASTR that has an scFv that recognizes human ROR2, a CD8 stalk and transmembrane sequence (SEQ ID NO:75), CD137 (SEQ ID NO: 1), and CD3z (SEQ ID NO: 13). Recombinant lentiviral particles encoding this construct are called F1-1-27;
(2) Flag-Anti-ROR2 MRB-CAR:T2A:CLE (F1-1-228 and F1-1-228U): This genomic plasmid is identical to that shown in FIG. 20 except that the sequence encoding anti-CD19 was replaced with a Flag-tag covalently linked to the anti-ROR2 MRB-CAR described in (1) above, and the GMCSFR ss:eTag was replaced with a CLE. The CLE was a type I transmembrane protein comprising an extracellular dimerization module (P1), a transmembrane module (P2), and 2 intracellular modules (P3 and P4). The genes in positions P1-P2-P3-P4 were MycTag 2A Jun-IL13RA-MPL-CD40. The codes for these modules are E013-T041-S186-S051 (See Table 6). This CLE was first identified in libraries 3A, 3B, and 4B and was the 6^{th} most enriched CLE between days 7 and 35 in library 3A. Recombinant lentiviral particles encoding this construct are called F1-1-228 and recombinant lentiviral particles encoding this construct and displaying UCHT1scFvFc-GPI are called F1-1-228U; or
(3) Flag-Anti-CD19 CAR:T2A:CLE (F1-3-219 and F1-3-219U): This genomic plasmid is identical to the anti-human CD19 CAR shown in FIG. 20 except that a Flag-tag was inserted between the CD8ss and the CAR, and the sequence encoding the GMCSFRss:eTag was replaced with a CLE. The CLE was a type I transmembrane protein comprising an interleukin polypeptide covalently linked via a flexible linker to the extracellular and transmembrane domains of its cognate interleukin receptor and covalently linked to the intracellular domain of a dissimilar cytokine receptor. In particular, from amino to carboxy terminus the plasmid encoded IL-7 (SEQ ID NO:511), a flexible linker (SEQ ID NO:53), the extracellular and transmembrane portions of the IL-7Rα (CD127) (SEQ ID NO:513), and the intracellular domain of the IL-2Rβ (CD122) (SEQ ID NO: 514). Recombinant lentiviral particles encoding this construct are called F1-3-219 and recombinant lentiviral particles encoding this construct and displaying UCHT1scFvFc-GPI are called F1-3-219U.

### PBMC isolation, overnight transduction of PBMCs after ex vivo stimulation, followed by 15 day ex vivo expansion of engineered lymphocytes

On Day 0, PBMCs were isolated from ACD peripheral blood from a healthy volunteer with informed consent by density gradient centrifugation with Ficoll-Pacque^{™} (General Electric) using a CS-900.2 kit (BioSafe; 1008) on a Sepax 2 S-100 device (Biosafe; 14000) according to the manufacturer's instructions. 3.0 x 10⁷ viable PBMCs were seeded in a 1 L G-Rex (Wilson-Wolf ) and the volume was brought to 60ml with Complete OpTmizer^{™} CTS^{™} T-Cell Expansion SFM supplemented with 100 IU/ml IL-2 (Novoprotein, GMP-CD66), 10 ng/ml IL-7 (Novoprotein, GMP-CD47), and 50 ng/ml anti-CD3 antibody (OKT3, Novoprotein) to activate the PBMCs, which included T cells and NK cells, for viral transduction. After incubation overnight at 37 °C and 5% CO₂, lentiviral particles encoding the anti-ROR2 MRB-CAR, F1-1-27, were added directly to the activated PBMCs at an MOI of 5 (440ul) and incubated overnight at 37 °C and 5% CO₂. Following the overnight incubation, the cells were fed by bringing the total volume of media in the G-Rex to 100ml with Complete OpTmizer^{™} CTS^{™} T-Cell Expansion SFM supplemented with NAC (Sigma) to increase the final concentration by 10mM along with 100 IU/ml recombinant human IL-2 and 10 ng/ml recombinant human IL-7. The G-Rex device was incubated in a standard humidified tissue culture incubator at 37 °C and 5% CO₂ with additions of 100 IU/ml recombinant human IL-2 and 10 ng/ml recombinant human IL-7 solution every 48 hours. The cells were expanded through Day 15 before being harvested. These transduced cells were washed in freezing media (70% RPMI 1640, 20% heat-inactivated FBS, 10% DMSO) and cryopreserved in 1 ml aliquots at 5.0 x 10⁷ cells/ml for later use. Two days prior to use in Group A of the experiments in this example below, 8 vials (4.0 x 10⁸) of these cryopreserved F1-1-27 transduced PBMCs were thawed and rested for 2 days in a G-Rex100M containing 377ml Complete OpTmizer^{™} CTS^{™} T-Cell Expansion SFM (OpTmizer^{™} CTS^{™} T-Cell Expansion Basal Medium 1 L (Thermo Fisher, A10221) supplemented with 26 ml OpTmizer^{™} CTS^{™} T-Cell Expansion Supplement (Thermo Fisher, A10484-02), 25 ml CTS^{™} Immune Cell SR (Thermo Fisher, A2596101), and 10 ml CTS^{™} GlutaMAX^{™}-I Supplement (Thermo Fisher, A1286001) according to manufacturer's instructions) supplemented with 100 IU/ml of IL-2 (Novoprotein), 10 ng/ml IL-7 (Novoprotein), and sufficient NAC to increase the final concentration by 10mM.

### PBMC isolation and advantageously fast transduction of resting lymphocytes without prior ex vivo stimulation and without ex vivo cell expansion

Whole human blood from 2 healthy volunteers with informed consent was collected into multiple 100 mm Vacutainer tubes (Becton Dickenson; 364606) containing 1.5 ml of Acid Citrate Dextrose Solution A anticoagulant (ACD peripheral blood). For each volunteer, blood from the Vacutainer tubes was pooled (185.2ml for Group B, 182.5ml for Group C) and distributed to 2 standard 500 ml blood collection bags. The following steps of PBMCs enrichment through transduction were performed in a closed system.

The blood in the 2 blood bags from each volunteer was processed sequentially using density gradient centrifugation with Ficoll-Paque^{™} (General Electric) using a CS-900.2 kit (BioSafe; 1008) on a Sepax 2 S-100 device (Biosafe; 14000) using 2 wash cycles according to the manufacturer's instructions, to obtain 45 ml of isolated PBMCs from each run. The wash solution used in the Sepax 2 process was Normal Saline (Chenixin Pharm) + 2% human serum albumin (HSA) (Sichuan Yuanda Shuyang Pharmaceutical). The final cell resuspension solution was 45 ml Complete OpTmizer^{™} CTS^{™} T-Cell Expansion SFM (OpTmizer^{™} CTS^{™} T-Cell Expansion Basal Medium 1 L (Thermo Fisher, A10221-03) supplemented with 26 ml OpTmizer^{™} CTS^{™} T-Cell Expansion Supplement (Thermo Fisher, A10484-02), 25 ml CTS^{™} Immune Cell SR (Thermo Fisher, A2596101), and 10 ml CTS^{™} GlutaMAX^{™}-I Supplement (Thermo Fisher, A1286001)). Each processing step on the Sepax 2 machine was approximately 1 hour and 12 minutes. Enriched PBMCs obtained from the 2 processing runs were pooled separately for Group B and Group C, and the cells counted.

5.5 x 10⁷ freshly enriched, viable PBMCs were seeded into each of 4 standard blood collection bags for Group B and the volume was brought to 55ml with Complete OpTmizer^{™} CTS^{™} T-Cell Expansion SFM such that the cells were at a density of 1.0 x 10⁶ /ml. 1.12 x 10⁸ freshly enriched, viable PBMCs were seeded into each of 2 standard blood collection bags for Group C and the volume was brought to 110ml with Complete OpTmizer^{™} CTS^{™} T-Cell Expansion SFM such that the cells were at a density of 1.0 x 10⁶ /ml. No anti-CD3, anti-CD28, IL-2, IL-7, or other exogenous cytokine was added to activate or otherwise stimulate the lymphocyte ex vivo prior to transduction. Lentiviral particles were added directly to the non-stimulated PBMCs in blood collection bags at an MOI of 1 as follows: 0.779ml of F1-1-228 was added to one bag and 3.11ml of F 1-1-228U was added to the other bag of Group B PBMCs; 0.362ml of F1-3-219 was added to one bag and 3.52ml of F1-3-219U was added to the other bag of Group C PBMCs. The transduction reaction mixtures were gently massaged to mix the contents then incubated for four (4) hours in the blood collection bags in a standard humidified tissue culture incubator at 37 °C and 5% CO₂. The PBMCs from each bag were then transferred to a 50ml Conical tube (thus removing the cells from the closed system in this proof of concept experiment) and washed 3 times in DPBS + 2% HSA before being resuspended in 5mls DPBS + 2% HSA and counted. The table below shows the duration of each step in the process and the total time elapsed. No further processing was performed on these PBMCs prior to their use in the experiments in this example.

**Table 24. Elapsed times for steps after blood draw and pooling.**

| | Group B | | Group C | |
|---|---|---|---|---|
| | Time | Total Elapsed | Time | Total Elapsed |
| Blood draw & pooling | 9:00- | | 10:00 | |
| 1^{st} Sepax run | 10:08-11:16 | 2hr 16min | 10:30-11:45 | 1hr 45min |
| 2^{nd} Sepax run | 11:21-12:30 | 3hr 30min | 11:48-13:04 | 3hr 4min |
| Cell counting | 12:40-13:00 | 4hr 0min | 13:11-13:35 | 3hr 35min |
| Dispense into bags | 13:55-14:03 | 5hr 3min | 13:37-13:44 | 3hr 44min |
| Media addition | 14:06-14:20 | 5hr 20min | 13:45-13:58 | 3hr 58min |
| Virus addition | 14:55-15:01 | 6hr 1min | 14:00-14:30 | 4hr 30min |
| Transduction | 15:10-19:10 | 10hr 10min | 14:30-18:30 | 8hr 30min |
| Transfer & Pellet | 19:10-19:36 | 10hr 36min | 18:30-19:04 | 8hr 4min |
| 1^{st} wash | 19:55-20:10 | 11hr 10min | 19:14-19:30 | 9hr 30min |
| 2^{nd} wash | 20:21-20:36 | 11hr 36min | 19:35-19:51 | 9hr 51min |
| 3^{rd} wash | 20:47-21:02 | 12hr 2min | 19:56-20:02 | 9hr 2min |
| Cell counting | 21:12-22:10 | 13hr 10min | 20:03-20:50 | 10hr 50min |
| Transportation | 21:58-23:10 | 14hr 10min | 21:50-21:25 | 11hr 25min |
| Dosing mice | 23:12-23:30 | 14hr 30min | 21:25-21:30 | 11hr 30min |

### Transduction efficiency and cytokine-independent survival/proliferation in vitro of PBMCs transduced by the methods above

2.0 x 10⁶ PBMCs that included T cells and NK cells were seeded in duplicate or triplicate into the wells of a 6 well tissue culture plate for each sample. The plates were centrifuged and each sample was resuspended in 2ml of Complete OpTmizer^{™} CTS^{™} T-Cell Expansion SFM. No cytokines were added. The plates were incubated in a standard humidified tissue culture incubator at 37 °C and 5% CO₂ for 6 days. Half (1ml) of the cell suspension from each well was removed on day 3 and the remaining cells removed on day 6 for to determine cell numbers, percent viability, and percent transduced cells, which was defined as the percent of FLAG-Tag+ cells by FACS analysis. Total cell counts on day 6 were doubled to account for removing half of the cells on day 3.

### Proliferation/survival and target killing of tumors in vivo by effector PBMCs transduced by the methods above

A xenograft model using NSG, or NOD Scid Gamma mice was chosen to probe the ability of human PBMCs transduced with F1-1-27, F1-1-228, F1-1-228U, F1-3-219, and F1-3-219U to survive, proliferate, and kill cognate antigen-expressing tumors in vivo. NSG is a strain of mice that lack mature T cells, NK cells, and B cells and is among the most immunodeficient mouse strain described to date. Removal of these cellular components of the immune system is typically performed to enable human PBMCs to engraft without innate, humoral or adaptive immune reactions from the host. Concentrations of homeostatic cytokines normally present only after radiation or lymphodepleting chemotherapy in humans is achieved due to the absence of the murine extracellular common gamma chain, which enables adoptively transferred human cells to receive such cytokines. At the same time, these animals can also be utilized to engraft tumor xenograft targets to examine the efficacy of CARs to kill target-expressing tumors. While the presence of xenoreactive T cell receptor antigens in the effector cellular product will eventually give rise to graft versus host disease, these models enable short term evaluation of animal pharmacology and acute tolerability.

Raji cells (ATCC, Manassas, VA) which express endogenous human CD19, and CHO cells (ATCC, Manassas, VA) transfected to stably express human ROR2 (CHO-ROR2) were utilized to provide antigen to stimulate the CAR effector cells and to generate uniform target tumors to determine the efficacy of CAR effector cells to kill cognate antigen-expressing tumors. The Raji cells and transgenic CHO variants grew rapidly with disseminated malignancy after subcutaneous administration into NSG mice in combination with Matrigel artificial basement membrane.

Mice were handled in accordance with Institutional Animal Care and Use Committee approved protocols. Subcutaneous (sc) tumor xenografts were established in the hind flank of female NOD-Prkdc^{scid}Il2rg^{tm1}/Begen (B-NSG) mice (Beijing Biocytogen Co. Ltd.). Briefly, cultured Raji cells and cultured CHO-ROR2 cells were separately washed in DPBS (Thermo Fisher), counted, resuspended in cold DPBS and mixed with an appropriate volume of Matrigel ECM (Corning; final concentration 5 mg/mL) at a concentration of 0.5 x 10⁶ cells/200 µl on ice. Animals were prepared for injection using standard approved anesthesia with hair removal (Nair) prior to injection. 200 µl of either cell suspension in ECM was injected sc into the rear flanks of 9 or 10 week old mice for Raji and CHO-ROR2 cells, respectively.

5 days after tumor inoculation, mice bearing CHO-ROR2 tumors, which averaged 77 mm³ in volume, were dosed intravenously (IV) by tail vein injection as follows: NSG mice in Group A received 1 x 10⁷ PBMCs transduced with F1-1-27 lentiviral particles in 200µl DPBS (n=4), or 200µl DPBS alone (n=2); NSG mice in Group B received 0.85 x 10⁷ PBMCs transduced with F1-1-228 lentiviral particles in 200µl DPBS (n=2), 0.85 x 10⁷ PBMCs transduced with F1-1-228U lentiviral particles in 200µl DPBS (n=2), or 200µl DPBS alone (n=2). Similarly, 5 days after tumor inoculation, mice in Group C bearing Raji tumors, which averaged 76 mm³ in volume, were dosed intravenously (IV) by tail vein injection with 200µl DPBS alone (n=4) or 1 x 10⁷ PBMCs transduced with either F1-3-219 lentiviral particles in 200µl DPBS (n=3) or F1-3-219U lentiviral particles in 200µl DPBS (n=3). Note that the protocol for the advantageously fast transduction of resting lymphocytes prior to ex vivo activation and without ex vivo cell expansion was used to transduce the PBMCs with F1-1-228, F1-1-228U, F1-3-219, and F1-3-219U used for dosing these mice. The total time elapsed from whole human blood collection to IV dosing of the mice with transduced PBMCs was 14.5 hours for F1-1-228 and F1-1-228U, and 11.5 hours for F1-3-219 and F1-3-219U.

Tumors were measured using calipers 2 times a week and tumor volume was calculated using the following equation: (longest diameter * shortest diameter²)/2. Approximately 100µl of blood was collected from each mouse on days 7, **14,** and 21 for analysis by FACS and qPCR. Blood, spleen, and tumor was also collected when the mice were euthanized consistent with necropsy guidelines from tumor burden.

### Flow Cytometry

For cells harvested from in vitro culture - cells were spun down and resuspended in 0.5ml FACS Staining Buffer (554656, BD). 2.5µl human Fc Block (BD, 564220) was added to each sample and incubated at room temperature for 10 minutes. Cells were stained with 0.5µl anti-FLAG Tag PE ((anti-DYKDDDDK) 637310, Biolegend) and 0.5µl Live/Dead Fixable Green Dead cell stain (L34970, Thermo Fisher) for 30 mins on ice. Cells were washed twice in FACS buffer, fixed in a 1:1 mixture of the FACS buffer and BD Cytofix (554655, BD), processed with Novocyte (ACEA), and the resulting data was analyzed with NovoExpress software (ACEA) using live gates based on forward and side scatter and the live dead stain. Transduced lymphocytes were measured as FLAG Tag+ cells.

For cells obtained from blood - red blood cells in the freshly collected blood were lysed using Lysing Buffer (555899, BD) and the remaining cells resuspended in 100µl of FACS Staining Buffer. 2.5µl human Fc Block (BD, 564220) was added to each sample and incubated at room temperature for 10 minutes. Cells were stained with biotinylated-cetuximab for 30 mins on ice. Stained cells were washed with FACS buffer and further stained with 5µl anti-human CD45-PE-Cy7 and 0.5µl anti-mouse CD45-FITC. 0.4µl SA-PE was added to samples from mice dosed with cells transduced with F1-1-27, F1-1-228, F1-1-228U, and PBS controls for these groups. 1 µl anti-FLAG Tag PE ((anti-DYKDDDDK) 637310, Biolegend) was added to samples from mice dosed with cells transduced with F1-3-219 and F1-3-219U and PBS controls for this group. Cells were incubated for 30 mins on ice, washed twice in FACS buffer, and resuspended in 100µl FACS Staining Buffer with 1µl 7-AAD (420404, Biolegend). Freshly stained samples were processed with Novocyte (ACEA), and the resulting data was analyzed with NovoExpress software (ACEA) using live gates based on forward and side scatter, a live gate based on 7-AAD, human CD45+ and examined for the expression of FLAG or eTag.

### qPCR

Genomic DNA (gDNA) isolated from blood samples were evaluated for the presence of transduced lymphocytes by bioanalytical qPCR. Genomic DNA was isolated from 50µl blood samples using the QIAamp DNA Blood Mini kit (Qiagen 51106) and the DNA was further cleaned using the QIAamp DNA Micro Kit (56304). A TaqMan assay (Thermo Fisher) was performed on the isolated genomic DNA using a primer and probe set specific for the 5' LTR of lentivirus to detect transduced cells.

### RESULTS

Lentiviral particles pseudotyped with VSV-G and encoding a CAR and a lymphoproliferative element were used in this experiment. Lentiviral particles F1-1-228 and F1-1-228U encoded a MRB-CAR to ROR2 and lentiviral particles F1-2-219 and F1-3-219U encoded a CAR to CD19. Lentiviral particles F1-1-228U and F1-3-219U also displayed UCHT1scFvFc-GPI on their surface. PBMCs were isolated from human blood by density gradient centrifugation with Ficoll-Paque^{™}. The fresh PBMCs were transduced with the lentiviral particles in standard blood bags without prior activation of the cells ex vivo. After a 4 hour transduction, the cells were washed and used in the experiments below. A skilled artisan will understand that the entire process from blood collection to washed cells could be performed in a closed system. As a control, PBMCs were activated overnight, transduced with lentiviral particles encoding a CAR without a lymphoproliferative element or UCHT1scFvFc-GPI, and expanded ex vivo for 15 days (F1-1-27).

Transduced PBMCs were cultured in vitro in the absence of cytokines. FIG. 37 shows transduction efficiencies at Day 6. UCHT1scFvFc-GPI increased the transduction efficiency of both F1-1-228 and F1-3-219. Resting PBMCs, when exposed for 4 hours to F1-1-228U or F1-3-219U had transduction efficiencies of 34% and 73%, respectively. FIG. 38 shows the total number of viable cells in these cultures between days 3 and 6. Resting PBMCs transduced with either F1-1-228U or F1-3-219U survived and even proliferated between day 3 and day 6 in the absence of exogenous cytokines while PBMCs transduced with either F1-1-228 or F1-3-219 did not. These results demonstrate that retroviral particles displaying an activation element and encoding a lymphoproliferative element can transduce resting PBMCs in 4 hours and that these transduced PMBCs can proliferate and survive *in vitro* when cultured for 6 days in the absence of exogenous cytokines.

Immunodeficient mice bearing ROR2 or CD19 tumors were dosed intravenously with 1 x 10⁷ PBMCs that express a CAR to ROR2 or CD19, respectively. The ability of the transduced PBMCs to survive and proliferate in vivo was examined over time. FIGs. 39A-C show the lentiviral copies per µg of genomic DNA by qPCR as a readout for transduced cells. FIG. 39A shows that lentiviral copies per µg of genomic DNA for F1-1-27, which does not encode for a lymphoproliferative element, decreased from an average of 884 on day 7 to below the lower limit of quantitation (LLOQ) by day 21. FIG. 39B shows that the lentiviral copies for F1-1-228U was below the LLOQ on days 7 and 14, but increased above the LLOQ by day 21 and increased to an average of 1,939 on day 25. FIG. 39C shows that the lentiviral copies for F1-3-219U increased from below the LLOQ on day 7 to 20,430 on day 14 when the mice were euthanized. Lentiviral copies in the control samples, F1-1-228 and F1-3-219, remained below the LLOQ. FIG. 40 shows the number of transduced cells per 200ul of blood at the time the mice were euthanized (the last time points are shown in FIG. 39) as determined by FACS analysis for the eTag or FLAG-Tag of the CAR construct. Significant numbers of CAR+ cells were detected per 200ul of blood from mice dosed with cells transduced with F1-1-228U (5,857) and F1-3-219U (121,324). Lymphocytes transduced with F1-1-228U or F1-3-219U killed tumors expressing their target antigen in vivo (FIGs. 41A and B). In both cases the lymphocytes reduced the tumor volume in a delayed manner. Not to be limited by theory, but this delay is consistent with a need for the injected cells to expand, which took time as seen by qPCR. Later time points could not be studied in this experiment because the mice harboring ROR2 tumors reach euthanasia guidelines for tumor burden and the mice harboring Raji tumors developed graft-versus-host disease caused by the significant number of transduced and expanded lymphocytes in the mice. Together these data show that retroviral particles displaying an activation element and encoding a lymphoproliferative element can transduce resting PBMCs in 4 hours and that these transduced PMBCs can proliferate and survive in vivo. Both lymphoproliferative elements tested in this experiment, MycTag 2A Jun-IL13Ra-MPL-CD40 and IL-7- IL-7Rα - IL-2Rβ, displayed the ability to promote survival and proliferation in vivo. Furthermore, these lymphocytes transduced in this manner expressing a MRB-CAR (F1-1-228U) or a traditional CAR (F1-3-219U) were able to recognize and kill tumor cells in vivo.

The disclosed embodiments, examples and experiments are not intended to limit the scope of the disclosure or to represent that the experiments below are all or the only experiments performed. Efforts have been made to ensure accuracy with respect to numbers used (e.g., amounts, temperature, etc.) but some experimental errors and deviations should be accounted for. It should be understood that variations in the methods as described may be made without changing the fundamental aspects that the experiments are meant to illustrate.

Those skilled in the art can devise many modifications and other embodiments within the scope of the present disclosure. Indeed, variations in the materials, methods, drawings, experiments, examples, and embodiments described may be made by skilled artisans without changing the fundamental aspects of the present disclosure. Any of the disclosed embodiments can be used in combination with any other disclosed embodiment.

In some instances, some concepts have been described with reference to specific embodiments. However, one of ordinary skill in the art appreciates that various modifications and changes can be made without departing from the scope of the invention as set forth in the claims below. Accordingly, the specification and figures are to be regarded in an illustrative rather than a restrictive sense, and all such modifications are intended to be included within the scope of invention.

**Table 6. Codes, names, cytokine receptors, ITAM motif, and amino acid sequences for parts P1-P2, P1, P2, P3, and P4.**

| Code | Name | Amino Acid Sequence |
|---|---|---|
| M001 | eTAG IL7RA Ins PPCL (interleukin 7 receptor) | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQVCHALCSPEGCWGPEPRDCVSCRNVSRGRECVDK CNLLEGEPREFVENSECIQCHPECLPQAMNITCTGRGPDNCIQCAHYIDGPHCVKTCPAGVMGENNTLVWK YADAGHVCHLCHPNCTYGCTGPGLEGCPTNGPEINNSSGEMDPILLPPCLTISILSFFSVALLVILACVL (SEQ ID NO:292) |
| M002 | eTAG IL7RA Ins PPCL (interleukin 7 receptor) | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQPEINNSSGEMDPILLPPCLTISILSFFSVALLVILACVL (SEQ ID NO:293) |
| M007 | Myc Tag LMP1 NC_007605_1 | MEQKLISEEDLEHDLERGPPGPRRPPRGPPLSSSLGLALLLLLLALLFWLYIVMSDWTGGALLVLYSFALMLIIIIL IIFIFRRDLLCPLGALCILLLMITLLLIALWNLHGQALFLGIVLFIFGCLLVLGIWIYLLEMLWRLGATIWQLLAFFLA FFLDLILLIIALYLQQNWWTLLVDLLWLLLFLAILIWM (SEQ ID NO:294) |
| M008 | Myc LMP1 NC_007605_1 | MEQKLISEEDLSSSLGLALLLLLLALLFWLYIVMSDWTGGALLVLYSFALMLIIIILIIFIFRRDLLCPLGALCILLLMI TLLLIALWNLHGQALFLGIVLFIFGCLLVLGIWIYLLEMLWRLGATIWQLLAFFLAFFLDLILLIIALYLQQNWWT LLVDLLWLLLFLAILIWM (SEQ ID NO:295) |
| M009 | LMP1 NC_007605_1 | MEHDLERGPPGPRRPPRGPPLSSSLGLALLLLLLLALLFWLYIVMSDWTGGALLVLYSFALMLIIIILIIFIFRRDLLC PLGALCILLLMITLLLIALWNLHGQALFLGIVLFIFGCLLVLGIWIYLLEMLWRLGATIWQLLAFFLAFFLDLILLIIA LYLQQNWWTLLVDLLWLLLFLAILIWM (SEQ ID NO:296) |
| M010 | LMP1 NC_007605_1 | MSLGLALLLLLLALLFWLYIVMSDWTGGALLVLYSFALMLIIIILIIFIFRRDLLCPLGALCILLLMITLLLIALWNLHG QALFLGIVLFIFGCLLVLGIWIYLLEMLWRLGATIWQLLAFFLAFFLDLILLIIALYLQQNWWTLLVDLLWLLLFLA ILIWM (SEQ ID NO:297) |
| M012 | eTAG CRLF2 transcript variant 1 NM_022148_3 | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQVCHALCSPEGCWGPEPRDCVSCRNVSRGRECVDK CNLLEGEPREFVENSECIQCHPECLPQAMNITCTGRGPDNCIQCAHYIDGPHCVKTCPAGVMGENNTLVWK YADAGHVCHLCHPNCTYGCTGPGLEGCPTNGAETPTPPKPKLSKCILISSLAILLMVSLLLLSLW (SEQ ID NO:298) |
| M013 | eTAG CRLF2 transcript variant 1 NM_022148_3 | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQAETPTPPKPKLSKCILISSLAILLMVSLLLLSLW (SEQ ID NO:299) |
| M018 | eTAG CSF2RB NM_000395_2 | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQVCHALCSPEGCWGPEPRDCVSCRNVSRGRECVDK CNLLEGEPREFVENSECIQCHPECLPQAMNITCTGRGPDNCIQCAHYIDGPHCVKTCPAGVMGENNTLVWK YADAGHVCHLCHPNCTYGCTGPGLEGCPTNGTESVLPMWVLALIEIFLTIAVLLAL (SEQ ID NO:300) |
| M019 | eTAG CSF2RB NM_000395_2 | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQTESVLPMWVLALIEIFLTIAVLLAL (SEQ ID NO:301) |
| M024 | eTAG CSF3R transcript variant 1 NM_000760_3 | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQVCHALCSPEGCWGPEPRDCVSCRNVSRGRECVDK CNLLEGEPREFVENSECIQCHPECLPQAMNITCTGRGPDNCIQCAHYIDGPHCVKTCPAGVMGENNTLVWK YADAGHVCHLCHPNCTYGCTGPGLEGCPTNGTPEGSELHIILGLFGLLLLLNCLCGTAWLCC (SEQ ID NO:302) |
| M025 | eTAG CSF3R transcript variant 1 NM_000760_3 | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQTPEGSELHIILGLFGLLLLLNCLCGTAWLCC (SEQ ID NO:303) |
| M030 | eTAG EPOR transcript variant 1 NM_000121_3 | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQVCHALCSPEGCWGPEPRDCVSCRNVSRGRECVDK CNLLEGEPREFVENSECIQCHPECLPQAMNITCTGRGPDNCIQCAHYIDGPHCVKTCPAGVMGENNTLVWK YADAGHVCHLCHPNCTYGCTGPGLEGCPTNGTPSDLDPCCLTLSLILVVILVLLTVLALLS (SEQ ID NO:304) |
| M031 | eTAG EPOR transcript variant 1 NM_000121_3 | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQTPSDLDPCCLTLSLILVVILVLLTVLALLS (SEQ ID NO:305) |
| M036 | eTAG GHR transcript variant 1 NM_000163_4 | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQVCHALCSPEGCWGPEPRDCVSCRNVSRGRECVDK CNLLEGEPREFVENSECIQCHPECLPQAMNITCTGRGPDNCIQCAHYIDGPHCVKTCPAGVMGENNTLVWK YADAGHVCHLCHPNCTYGCTGPGLEGCPTNGTLPQMSQFTCCEDFYFPWLLCIIFGIFGLTVMLFVFLFS (SEQ ID NO:306) |
| M037 | eTAG GHR transcript variant 1 NM_000163_4 | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQTLPQMSQFTCCEDFYFPWLLCIIFGIFGLTVMLFVF LFS (SEQ ID NO:307) |
| M042 | eTAG truncated after Fn F523C IL27RA NM_004843_3 | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQVCHALCSPEGCWGPEPRDCVSCRNVSRGRECVDK CNLLEGEPREFVENSECIQCHPECLPQAMNITCTGRGPDNCIQCAHYIDGPHCVKTCPAGVMGENNTLVWK YADAGHVCHLCHPNCTYGCTGPGLEGCPTNGHLPDNTLRWKVLPGILCLWGLFLLGCGLSLA (SEQ ID NO:308) |
| M043 | eTAG truncated after Fn F523C IL27RA NM_004843_3 | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQHLPDNTLRWKVLPGILCLWGLFLLGCGLSLA (SEQ ID NO:309) |
| M048 | eTAG truncated after Fn S505N MPL NM_005373_2 | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQVCHALCSPEGCWGPEPRDCVSCRNVSRGRECVDK CNLLEGEPREFVENSECIQCHPECLPQAMNITCTGRGPDNCIQCAHYIDGPHCVKTCPAGVMGENNTLVWK YADAGHVCHLCHPNCTYGCTGPGLEGCPTNGETATETAWISLVTALHLVLGLNAVLGLLLL (SEQ ID NO:310) |
| M049 | eTAG truncated after Fn S505N MPL NM_005373_2 | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQETATETAWISLVTALHLVLGLNAVLGLLLL (SEQ ID NO:311) |
| E006 | eTag 0A JUN NM_002228_3 | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQVCHALCSPEGCWGPEPRDCVSCRNVSRGRECVDK CNLLEGEPREFVENSECIQCHPECLPQAMNITCTGRGPDNCIQCAHYIDGPHCVKTCPAGVMGENNTLVWK YADAGHVCHLCHPNCTYGCTGPGLEGCPTNGLERIARLEEKVKTLKAQNSELASTANMLREQVAQLKQKV (SEQ ID NO:312) |
| E007 | eTag 1A JUN NM_002228_3 | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQVCHALCSPEGCWGPEPRDCVSCRNVSRGRECVDK CNLLEGEPREFVENSECIQCHPECLPQAMNITCTGRGPDNCIQCAHYIDGPHCVKTCPAGVMGENNTLVWK YADAGHVCHLCHPNCTYGCTGPGLEGCPTNGLERIARLEEKVKTLKAQNSELASTANMLREQVAQLKQKVA (SEQ ID NO:313) |
| E008 | eTag 2A JUN NM_002228_3 | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQVCHALCSPEGCWGPEPRDCVSCRNVSRGRECVDK CNLLEGEPREFVENSECIQCHPECLPQAMNITCTGRGPDNCIQCAHYIDGPHCVKTCPAGVMGENNTLVWK YADAGHVCHLCHPNCTYGCTGPGLEGCPTNGLERIARLEEKVKTLKAQNSELASTANMLREQVAQLKQKVA A (SEQ ID NO:314) |
| E009 | eTag 3A JUN NM_002228_3 | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQVCHALCSPEGCWGPEPRDCVSCRNVSRGRECVDK CNLLEGEPREFVENSECIQCHPECLPQAMNITCTGRGPDNCIQCAHYIDGPHCVKTCPAGVMGENNTLVWK YADAGHVCHLCHPNCTYGCTGPGLEGCPTNGLERIARLEEKVKTLKAQNSELASTANMLREQVAQLKQKVA AA (SEQ ID NO:315) |
| E010 | eTag 4A JUN NM_002228_3 | MLLLVTSLLLCELPHPAFLLIPRKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNITSLGLRSLKEISDGDVIISGN KNLCYANTINWKKLFGTSGQKTKIISNRGENSCKATGQVCHALCSPEGCWGPEPRDCVSCRNVSRGRECVDK CNLLEGEPREFVENSECIQCHPECLPQAMNITCTGRGPDNCIQCAHYIDGPHCVKTCPAGVMGENNTLVWK YADAGHVCHLCHPNCTYGCTGPGLEGCPTNGLERIARLEEKVKTLKAQNSELASTANMLREQVAQLKQKVA AAA (SEQ ID NO:316) |
| E011 | Myc Tag 0A JUN NM_002228_3 | MTILGTTFGMVFSLLQVVSGEQKLISEEDLLERIARLEEKVKTLKAQNSELASTANMLREQVAQLKQKV (SEQ ID NO:317) |
| E012 | Myc Tag 1A JUN NM_002228_3 | MTILGTTFGMVFSLLQVVSGEQKLISEEDLLERIARLEEKVKTLKAQNSELASTANMLREQVAQLKQKVA (SEQ ID NO:318) |
| E013 | Myc Tag 2A JUN NM_002228_3 | MTILGTTFGMVFSLLQVVSGEQKLISEEDLLERIARLEEKVKTLKAQNSELASTANMLREQVAQLKQKVAA (SEQ ID NO:319) |
| E014 | Myc Tag 3A JUN NM_002228_3 | MTILGTTFGMVFSLLQVVSGEQKLISEEDLLERIARLEEKVKTLKAQNSELASTANMLREQVAQLKQKVAAA (SEQ ID NO:320) |
| E015 | Myc Tag 4A JUN NM_002228_3 | MTILGTTFGMVFSLLQVVSGEQKLISEEDLLERIARLEEKVKTLKAQNSELASTANMLREQVAQLKQKVAAAA (SEQ ID NO:321) |
| T001 | CD2 transcript variant 1 NM_001328609_1 | LIIGICGGGSLLMVFVALLVFYI (SEQ ID NO:322) |
| T002 | CD3D transcript variant 1 NM_000732_4 | GIIVTDVIATLLLALGVFCFA (SEQ ID NO:323) |
| T003 | CD3E NM_000733_3 | VMSVATIVIVDICITGGLLLLVYYWS (SEQ ID NO:324) |
| T004 | CD3G NM_000073_2 | GFLFAEIVSIFVLAVGVYFIA (SEQ ID NO:325) |
| T005 | CD3Z CD247 transcript variant 1 NM_198053_2 | LCYLLDGILFIYGVILTALFL (SEQ ID NO:326) |
| T006 | CD4 transcript variant 1 and 2 NM_000616_4 | MALIVLGGVAGLLLFIGLGIFF (SEQ ID NO:327) |
| T007 | CD8A transcript variant 1 NM_001768_6 | IYIWAPLAGTCGVLLLSLVIT (SEQ ID NO:328) |
| T008 | CD8B transcript variant 2 NM_172213_3 | LGLLVAGVLVLLVSLGVAIHLCC (SEQ ID NO:329) |
| T009 | CD27 NM_001242_4 | ILVIFSGMFLVFTLAGALFLH (SEQ ID NO:330) |
| T010 | CD28 transcript variant 1 NM_006139_3 | FWVLVVVGGVLACYSLLVTVAFIIFWV (SEQ ID NO:331) |
| T011 | CD40 transcript variant 1 and 6 NM_001250_5 | ALVVIPIIFGILFAILLVLVFI (SEQ ID NO:332) |
| T012 | CD79A transcript variant 1 NM_001783_3 | IITAEGIILLFCAVVPGTLLLF (SEQ ID NO:333) |
| T013 | CD79B transcript variant 3 NM_001039933_2 | GIIMIQTLLIILFIIVPIFLLL (SEQ ID NO:334) |
| T014 | CRLF2 transcript variant 1 NM_022148_3 | FILISSLAILLMVSLLLLSLW (SEQ ID NO:335) |
| T015 | CRLF2 transcript variant 1 NM_022148_3 | CILISSLAILLMVSLLLLSLW (SEQ ID NO:336) |
| T016 | CSF2RA transcript variant 7 and 8 NM_001161529_1 | NLGSVYIYVLLIVGTLVCGIVLGFLF (SEQ ID NO:337) |
| T017 | CFS2RB NM_000395_2 | MWVLALIVIFLTIAVLLAL (SEQ ID NO:338) |
| T018 | CFS2RB NM_000395_2 | MWVLALIEIFLTIAVLLAL (SEQ ID NO:339) |
| T019 | CSF3R transcript variant 1 NM_000760_3 | IILGLFGLLLLLTCLCGTAWLCC (SEQ ID NO:340) |
| T020 | CSF3R transcript variant 1 NM_000760_3 | IILGLFGLLLLLNCLCGTAWLCC (SEQ ID NO:341) |
| T021 | EPOR transcript variant 1 NM_000121_3 | LILTLSLILVVILVLLTVLALLS (SEQ ID NO:342) |
| T022 | EPOR transcript variant 1 NM_000121_3 | CCLTLSLILVVILVLLTVLALLS (SEQ ID NO:343) |
| T023 | FCER1G NM_004106_1 | LCYILDAILFLYGIVLTLLYC (SEQ ID NO:344) |
| T024 | FCGR2C NM_201563_5 | IIVAVVTGIAVAAIVAAVVALIY (SEQ ID NO:345) |
| T025 | FCGRA2 transcript variant 1 NM_001136219_1 | IIVAVVIATAVAAIVAAVVALIY (SEQ ID NO:346) |
| T026 | GHR transcript variant 1 NM_000163_4 | FPWLLIIIFGIFGLTVMLFVFLFS (SEQ ID NO:347) |
| T027 | GHR transcript variant 1 NM_000163_4 | FPWLLCIIFGIFGLTVMLFVFLFS (SEQ ID NO:348) |
| T028 | ICOS NM_012092.3 | FWLPIGCAAFVVVCILGCILI (SEQ ID NO:349) |
| T029 | IFNAR1 NM_000629_2 | IWLIVGICIALFALPFVIYAA (SEQ ID NO:350) |
| T030 | IFNAR2 transcript variant 1 NM_207585_2 | IGGIITVFLIALVLTSTIVTL (SEQ ID NO:351) |
| T031 | IFNGR1 NM_000416_2 | SLWIPVVAALLLFLVLSLVFI (SEQ ID NO:352) |
| T032 | IFNGR2 transcript variant 1 NM_001329128_1 | VILISVGTFSLLSVLAGACFF (SEQ ID NO:353) |
| T033 | IFNLR1 NM_170743_3 | FLVLPSLLILLLVIAAGGVIW (SEQ ID NO:354) |
| T034 | IL1R1 transcript variant 2 NM_001288706_1 | HMIGICVTLTVIIVCSVFIYKIF (SEQ ID NO:355) |
| T035 | IL1RAP transcript variant 1 NM_002182_3 | VLLVVILIVVYHVYWLEMVLF (SEQ ID NO:356) |
| T036 | IL1RL1 transcript variant 1 NM_016232.4 | IYCIIAVCSVFLMLINVLVII (SEQ ID NO:357) |
| T037 | IL1RL2 NM_003854.2 | AYLIGGLIALVAVAVSVVYIY (SEQ ID NO:358) |
| T038 | IL2RA transcript variant 1 NM_000417_2 | VAVAGCVFLLISVLLLSGL (SEQ ID NO:359) |
| T039 | IL2RB transcript variant 1 NM_000878_4 | IPWLGHLLVGLSGAFGFIILVYLLI (SEQ ID NO:360) |
| T040 | IL2RG NM_000206_2 | VVISVGSMGLIISLLCVYFWL (SEQ ID NO:361) |
| T041 | IL3RA transcript variant 1 and 2 NM_002183_3 | TSLLIALGTLLALVCVFVIC (SEQ ID NO:362) |
| T042 | IL4R transcript variant 1 NM_000418_3 | LLLGVSVSCIVILAVCLLCYVSIT (SEQ ID NO:363) |
| T043 | IL5RA transcript variant 1 NM_000564_4 | FVIVIMATICFILLILSLIC (SEQ ID NO:364) |
| T044 | IL6R transcript variant 1 NM_000565_3 | TFLVAGGSLAFGTLLCIAIVL (SEQ ID NO:365) |
| T045 | IL6ST **transcript** variant 1 and 3 NM_002184_3 | AIVVPVCLAFLLTTLLGVLFCF (SEQ ID NO:366) |
| T046 | IL7RA NM_002185_3 | ILLTISILSFFSVALLVILACVL (SEQ ID NO:367) |
| T047 | IL7RA Ins PPCL (interleukin 7 receptor) | ILLPPCLTISILSFFSVALLVILACVL (SEQ ID NO:368) |
| T048 | IL9R transcript variant 1 NM_002186_2 | GNTLVAVSIFLLLTGPTYLLF (SEQ ID NO:369) |
| T049 | IL10RA transcript variant 1 NM_001558_3 | VIIFFAFVLLLSGALAYCLAL (SEQ ID NO:370) |
| T050 | IL10RB NM_000628_4 | WMVAVILMASVFMVCLALLGCF (SEQ ID NO:371) |
| T051 | IL11RA NM_001142784_2 | SLGILSFLGLVAGALALGLWL (SEQ ID NO:372) |
| T052 | IL12RB1 transcript variant 1 and 4 NM_005535_2 | WLIFFASLGSFLSILLVGVLGYLGL (SEQ ID NO:373) |
| T053 | IL12RB2 transcript variant 1 and 3 NM_001559_2 | WMAFVAPSICIAIIMVGIFST (SEQ ID NO:374) |
| T054 | IL13RA1 NM_001560_2 | LYITMLLIVPVIVAGAIIVLLLYL (SEQ ID NO:375) |
| T055 | IL13RA2 NM_000640_2 | FWLPFGFILILVIFVTGLLL (SEQ ID NO:376) |
| T056 | IL15RA transcript variant 4 NM_001256765_1 | VAISTSTVLLCGLSAVSLLACYL (SEQ ID NO:377) |
| T057 | IL17RA NM_014339_6 | VYWFITGISILLVGSVILLIV (SEQ ID NO:378) |
| T058 | IL17RB NM_018725_3 | LLLLSLLVATWVLVAGIYLMW (SEQ ID NO:379) |
| T059 | IL17RC transcript variant 1 NM_153460_3 | WALVWLACLLFAAALSLILLL (SEQ ID NO:380) |
| T060 | IL17RD transcript variant 2 NM_017563_4 | AVAITVPLVVISAFATLFTVM (SEQ ID NO:381) |
| T061 | IL17RE transcript variant 1 NM_153480_1 | LGLLILALLALLTLLGVVLAL (SEQ ID NO:382) |
| T062 | IL18R1 transcript variant 1 NM_003855_3 | GMIIAVLILVAVVCLVTVCVI (SEQ ID NO:383) |
| T063 | IL18RAP NM_003853_3 | GVVLLYILLGTIGTLVAVLAA (SEQ ID NO:384) |
| T064 | IL20RA transcript variant 1 NM_014432_3 | IIFWYVLPISITVFLFSVMGY (SEQ ID NO:385) |
| T065 | IL20RB NM_144717_3 | VLALFAFVGFMLILVVVPLFV (SEQ ID NO:386) |
| T066 | IL21R transcript variant 2 NM_181078_2 | GWNPHLLLLLLLVIVFIPAFW (SEQ ID NO:387) |
| T067 | IL22RA1 NM_021258_3 | YSFSGAFLFSMGFLVAVLCYL (SEQ ID NO:388) |
| T068 | IL23R NM_144701_2 | LLLGMIVFAVMLSILSLIGIF (SEQ ID NO:389) |
| T069 | IL27RA NM_004843_3 | VLPGILFLWGLFLLGCGLSLA (SEQ ID NO:390) |
| T070 | IL27RA NM_004843_3 | VLPGILCLWGLFLLGCGLSLA (SEQ ID NO:391) |
| T071 | IL31RA transcript variant 1 NM_139017 5 | IILITSLIGGGLLILIILTVAYGL (SEQ ID NO:392) |
| T072 | LEPR transcript variant 1 NM_002303_5 | AGLYVIVPVIISSSILLLGTLLI (SEQ ID NO:393) |
| T073 | LIFR NM_001127671_1 | VGLIIAILIPVAVAVIVGVVRSILC (SEQ ID NO:394) |
| T074 | MPL NM_005373_2 | ISLVTALHLVLGLSAVLGLLLL (SEQ ID NO:395) |
| T075 | MPL NM_005373_2 | ISLVTALHLVLGLNAVLGLLLL (SEQ ID NO:396) |
| T076 | OSMR transcript variant 4 NM_001323505_1 | LIHILLPMVFCVLLIMVMCYL (SEQ ID NO:397) |
| T077 | PRLR transcript variant 1 NM_000949_6 | TTVWISVAVLSAVICLIIVWAVAL (SEQ ID NO:398) |
| T078 | TNFRSF4 NM_003327_3 | VAAILGLGLVLGLLGPLAILL (SEQ ID NO:399) |
| T079 | TNFRSF8 transcript variant 1 NM_001243_4 | PVLDAGPVLFWVILVLVVVVGSSAFLLC (SEQ ID NO:400) |
| T080 | TNFRSF9 NM_001561_5 | IISFFLALTSTALLFLLFFLTLRFSVV (SEQ ID NO:401) |
| T081 | TNFRSF14 transcript variant 1 NM_003820_3 | WWFLSGSLVIVIVCSTVGLII (SEQ ID NO:402) |
| T082 | TNFRSF18 transcript variant 1 NM_004195_2 | LGWLTVVLLAVAACVLLLTSA (SEQ ID NO:403) |
| 5036 | CD2 transcript variant 1 NM_001328609_1 | TKRKKQRSRRNDEELETRAHRVATEERGRKPHQIPASTPQNPATSQHPPPPPGHRSQAPSHRPPPPGHRVQ HQPQKRPPAPSGTQVHQQKGPPLPRPRVQPKPPHGAAENSLSPSSN (SEQ ID NO:404) |
| 5037 | CD3D transcript variant 1 NM_000732_4 | GHETGRLSGAADTQALLRNDQVYQPLRDRDDAQYSHLGGNWARNK (SEQ ID NO:405) |
| 5038 | CD3E NM_000733_3 | KNRKAKAKPVTRGAGAGGRQRGQNKERPPPVPNPOYEPIRKGQROLYSGLNQRRI (SEQ ID NO:406) |
| 5039 | CD3G NM_000073_2 | GQDGVRQSRASDKQTLLPNDQLYQPLKDREDDQYSHLQGNQLRRN (SEQ ID NO:407) |
| 5042 | CD4 transcript variant 1 and 2 NM_000616_4 | CVRCRHRRRQAERMSQIKRLLSEKKTCQCPHRFQKTCSPI (SEQ ID NO:408) |
| 5043 | CD8A transcript variant 1 NM_001768_6 | LYCNHRNRRRVCKCPRPVVKSGDKPSLSARYV (SEQ ID NO:409) |
| 5044 | CD8B transcript variant 2 NM_172213_3 | RRRRARLRFMKQPQGEGISGTFVPQCLHGYYSNTTTSQKLLNPWILKT (SEQ ID NO:410) |
| 5045 | CD8B transcript variant 3 NM 172101 3 | RRRRARLRFMKQLRLHPLEKCSRMDY (SEQ ID NO:411) |
| 5046 | CD8B transcript variant 5 NM_004931_4 | RRRRARLRFMKQFYK (SEQ ID NO:412) |
| 5047 | CD27 NM_001242_4 | QRRKYRSNKGESPVEPAEPCRYSCPREEEGSTIPIQEDYRKPEPACSP (SEQ ID NO:413) |
| 5048 | mutated Delta Lck CD28 transcript variant 1 NM_006139_3 | RSKRSRLLHSDYMNMTPRRPGPTRKHYQAYAAARDFAAYRS (SEQ ID NO:414) |
| 5049 | CD28 transcript variant 1 NM_006139_3 NM_006139_3 | RSKRSRLLHSDYMNMTPRRPGPTRKHYQPYAPPRDFAAYRS (SEQ ID NO:415) |
| 5050 | CD40 transcript variant 1 and 6 NM_001250_5 | KKVAKKPTNKAPHPKQEPQEINFPDDLPGSNTAAPVQETLHGCQPVTQEDGKESRISVQERQ (SEQ ID NO:416) |
| 5051 | CD40 transcript variant 5 NM_001322421_1 | SESSEKVAKKPTNKAPHPKQEPQEINFPDDLPGSNTAAPVQETLHGCQPVTQEDGKESRISVQERQ (SEQ ID NO:417) |
| 5052 | CD79A transcript variant 1 NM_001783_3 | RKRWQNEKLGLDAGDEYEDENLYEGLNLDDCSMYEDISRGLQGTYQDVGSLNIGDVQLEKP (SEQ ID NO:418) |
| 5053 | CD79B transcript variant 3 NM_001039933_2 | LDKDDSKAGMEEDHTYEGLDIDQTATYEDIVTLRTGEVKWSVGEHPGQE (SEQ ID NO:419) |
| 5054 | CRLF2 transcript variant 1 NM_022148_3 | KLWRVKKFLIPSVPDPKSIFPGLFEIHQGNFQEWITDTQNVAHLHKMAGAEQESGPEEPLVVQLAKTEAESPR MLDPQTEEKEASGGSLQLPHQPLQGGDVVTIGGFTFVMNDRSYVAL (SEQ ID NO:420) |
| 5057 | CFS2RB NM_000395_2 | KLWRVKKFLIPSVPDPKSIFPGLFEIHQGNFQEWITDTQNVAHLHKMAGAEQESGPEEPLVVQLAKTEAESPR MLDPQTEEKEASGGSLQLPHQPLQGGDVVTIGGFTFVMNDRSYVAL (SEQ ID NO:420) RFCGIYGYRLRRKWEEKIPNPSKSHLFQNGSAELWPPGSMSAFTSGSPPHQGPWGSRFPELEGVFPVGFGDS EVSPLTIEDPKHVCDPPSGPDTTPAASDLPTEQPPSPQPGPPAASHTPEKQASSFDFNGPYLGPPHSRSLPDIL GQPEPPQEGGSQKSPPPGSLEYLCLPAGGQVQLVPLAQAMGPGQAVEVERRPSQGAAGSPSLESGGGPAP PALGPRVGGQDQKDSPVAIPMSSGDTEDPGVASGYVSSADLVFTPNSGASSVSLVPSLGLPSDQTPSLCPGL ASGPPGAPGPVKSGFEGYVELPPIEGRSPRSPRNNPVPPEAKSPVLNPGERPADVSPTSPQPEGLLVLQQVGD YCFLPGLGPGPLSLRSKPSSPGPGPEIKNLDQAFQVKKPPGQAVPQVPVIQLFKALKQQDYLSLPPWEVNKPG EVC (SEQ ID NO:421) |
| S058 | CSF2RA transcript variant 7 and 8 NM_0011615 29_1 | KRFLRIQRLFPPVPQIKDKLNDNHEVEDEIIWEEFTPEEGKGYREEVLTVKEIT (SEQ ID NO:422) |
| 5059 | CSF2RA transcript variant 9 NM_001161531_1 | KRFLRIQRLFPPVPQIKDKLNDNHEVEDEMGPQRHHRCGWNLYPTPGPSPGSGSSPRLGSESSL (SEQ ID NO:423) |
| 5062 | CSF3R transcript variant 1 NM_000760_3 | SPNRKNPLWPSVPDPAHSSLGSWVPTIMEEDAFQLPGLGTPPITKLTVLEEDEKKPVPWESHNSSETCGLPTL VQTYVLQGDPRAVSTQPQSQSGTSDQVLYGQLLGSPTSPGPGHYLRCDSTQPLLAGLTPSPKSYENLWFQAS PLGTLVTPAPSQEDDCVFGPLLNFPLLQGIRVHGMEALGSF (SEQ ID NO:424) |
| 5063 | CSF3R transcript variant 3 NM_156039_3 | SPNRKNPLWPSVPDPAHSSLGSWVPTIMEELPGPRQGQWLGQTSEMSRALTPHPCVQDAFQLPGLGTPPI TKLTVLEEDEKKPVPWESHNSSETCGLPTLVQTYVLQGDPRAVSTQPQSQSGTSDQVLYGQLLGSPTSPGPG HYLRCDSTQPLLAGLTPSPKSYENLWFQASPLGTLVTPAPSQEDDCVFGPLLNFPLLQGIRVHGMEALGSF (SEQ ID NO:425) |
| 5064 | CSF3R transcript variant 4 NM_172313_2 | SPNRKNPLWPSVPDPAHSSLGSWVPTIMEEDAFQLPGLGTPPITKLTVLEEDEKKPVPWESHNSSETCGLPTL VQTYVLQGDPRAVSTQPQSQSGTSDQVLYGQLLGSPTSPGPGHYLRCDSTQPLLAGLTPSPKSYENLWFQAS PLGTLVTPAPSQEDDCVFGPLLNFPLLQGIRVHGMEALGSF (SEQ ID NO:424) SPNRKNPLWPSVPDPAHSSLGSWVPTIMEELPGPRQGQWLGQTSEMSRALTPHPCVQDAFQLPGLGTPPI TKLTVLEEDEKKPVPWESHNSSETCGLPTLVQTYVLQGDPRAVSTQPQSQSGTSDQVLYGQLLGSPTSPGPG HYLRCDSTQPLLAGLTPSPKSYENLWFQASPLGTLVTPAPSQEDDCVFGPLLNFPLLQGIRVHGMEALGSF (SEQ ID NO:425) SPNRKNPLWPSVPDPAHSSLGSWVPTIMEEDAFQLPGLGTPPITKLTVLEEDEKKPVPWESHNSSETCGLPTL VQTYVLQGDPRAVSTQPQSQSGTSDQAGPPRRSAYFKDQIMLHPAPPNGLLCLFPITSVL (SEQ ID NO:426) HRRALKQKIWPGIPSPESEFEGLFTTHKGNFQLWLYQNDGCLWWSPCTPFTEDPPASLEVLSERCWGTMQA VEPGTDDEGPLLEPVGSEHAQDTYLVLDKWLLPRNPPSEDLPGPGGSVDIVAMDEGSEASSCSSALASKPSPE GASAASFEYTILDPSSQLLRPWTLCPELPPTPPHLKYLYLVVSDSGISTDYSSGDSQGAQGGLSDGPYSNPYENS LIPAAEPLPPSYVACS (SEQ ID NO:427) HRRALKQKIWPGIPSPESEFEGLFTTHKGNFQLWLYQNDGCLWWSPCTPFTEDPPASLEVLSERCWGTMQA VEPGTDDEGPLLEPVGSEHAQDTYLVLDKWLLPRNPPSEDLPGPGGSVDIVAMDEGSEASSCSSALASKPSPE GASAASFEYTILDPSSQLLRPWTLCPELPPTPPHLKFLFLVVSDSGISTDYSSGDSQGAQGGLSDGPYSNPYENS LIPAAEPLPPSYVACS (SEQ ID NO:428) |
| 5069 | EPOR transcript variant 1 NM_000121_3 | HRRALKQKIWPGIPSPESEFEGLFTTHKGNFQLWLYQNDGCLWWSPCTPFTEDPPASLEVLSERCWGTMQA VEPGTDDEGPLLEPVGSEHAQDTYLVLDKWLLPRNPPSEDLPGPGGSVDIVAMDEGSEASSCSSALASKPSPE GASAASFEYTILDPSSQLLRPWTLCPELPPTPPHLKYLYLVVSDSGISTDYSSGDSQGAQGGLSDGPYSNPYENS LIPAAEPLPPSYVACS (SEQ ID NO:427) |
| 5072 | EPOR transcript variant 1 NM_000121_3 | HRRALKQKIWPGIPSPESEFEGLFTTHKGNFQLWLYQNDGCLWWSPCTPFTEDPPASLEVLSERCWGTMQA VEPGTDDEGPLLEPVGSEHAQDTYLVLDKWLLPRNPPSEDLPGPGGSVDIVAMDEGSEASSCSSALASKPSPE GASAASFEYTILDPSSQLLRPWTLCPELPPTPPHLKFLFLVVSDSGISTDYSSGDSQGAQGGLSDGPYSNPYENS LIPAAEPLPPSYVACS (SEQ ID NO:428) |
| 5074 | FCER1G NM_004106_1 | RLKIQVRKAAITSYEKSDGVYTGLSTRNQETYETLKHEKPPQ (SEQ ID NO:429) |
| 5075 | FCGR2C NM_201563_5 | CRKKRISANSTDPVKAAQFEPPGRQMIAIRKRQPEETNNDYETADGGYMTLNPRAPTDDDKNIYLTLPPNDH VNSNN (SEQ ID NO:430) |
| 5076 | FCGRA2 transcript variant 1 NM_001136219_1 | CRKKRISANSTDPVKAAQFEPPGRQMIAIRKRQLEETNNDYETADGGYMTLNPRAPTDDDKNIYLTLPPNDH VNSNN (SEQ ID NO:431) |
| S077 | NM_001136219_1 GHR transcript variant 1 NM_000163_4 | CRKKRISANSTDPVKAAQFEPPGRQMIAIRKRQPEETNNDYETADGGYMTLNPRAPTDDDKNIYLTLPPNDH VNSNN (SEQ ID NO:430) CRKKRISANSTDPVKAAQFEPPGRQMIAIRKRQLEETNNDYETADGGYMTLNPRAPTDDDKNIYLTLPPNDH VNSNN (SEQ ID NO:431) KQQRIKMLILPPVPVPKIKGIDPDLLKEGKLEEVNTILAIHDSYKPEFHSDDSWVEFIELDIDEPDEKTEESDTDRL LSSDHEKSHSNLGVKDGDSGRTSCCEPDILETDFNANDIHEGTSEVAQPQRLKGEADLLCLDQKNQNNSPYH DACPATQQPSVIQAEKNKPQPLPTEGAESTHQAAHIQLSNPSSLSNIDFYAQVSDITPAGSVVLSPGQKNKAG MSQCDMHPEMVSLCQENFLMDNAYFCEADAKKCIPVAPHIKVESHIQPSLNQEDIYITTESLTTAAGRPGTG EHVPGSEMPVPDYTSIHIVQSPQGLILNATALPLPDKEFLSSCGYVSTDQLNKIMP (SEQ ID NO:432) |
| 5080 | ICOS NM_012092.3 | CWLTKKKYSSSVHDPNGEYMFMRAVNTAKKSRLTDVTL (SEQ ID NO:433) |
| 5081 | IFNAR1 NM_000629_2 | KVFLRCINYVFFPSLKPSSSIDEYFSEQPLKNLLLSTSEEQIEKCFIIENISTIATVEETNQTDEDHKKYSSQTSQDSG NYSNEDESESKTSEELQQDFV (SEQ ID NO:434) |
| 5082 | IFNAR2 transcript variant 1 NM_207585_2 | KWIGYICLRNSLPKVLNFHNFLAWPFPNLPPLEAMDMVEVIYINRKKKVWDYNYDDESDSDTEAAPRTSGG GYTMHGLTVRPLGQASATSTESQLIDPESEEEPDLPEVDVELPTMPKDSPQQLELLSGPCERRKSPLQDPFPEE DYSSTEGSGGRITFNVDLNSVFLRVLDDEDSDDLEAPLMLSSHLEEMVDPEDPDNVQSNHLLASGEGTQPTF PSPSSEGLWSEDAPSDQSDTSESDVDLGDGYIMR (SEQ ID NO:435) |
| 5083 | IFNAR2 transcript variant 2 NM 000874 4 | KWIGYICLRNSLPKVLRQGLAKGWNAVAIHRCSHNALQSETPELKQSSCLSFPSSWDYKRASLCPSD (SEQ ID NO:436) |
| 5084 | IFNGR1 NM_000416_2 | CFYIKKINPLKEKSIILPKSLISVVRSATLETKPESKYVSLITSYQPFSLEKEVVCEEPLSPATVPGMHTEDNPGKVE HTEELSSITEVVTTEENIPDVVPGSHLTPIERESSSPLSSNQSEPGSIALNSYHSRNCSESDHSRNGFDTDSSCLES HSSLSDSEFPPNNKGEIKTEGQELITVIKAPTSFGYDKPHVLVDLLVDDSGKESLIGYRPTEDSKEFS (SEQ ID NO:437) |
| 5085 | IFNGR2 transcript variant 1 NM_001329128_1 | LVLKYRGLIKYWFHTPPSIPLQIEEYLKDPTQPILEALDKDSSPKDDVWDSVSIISFPEKEQEDVLQTL (SEQ ID NO:438) |
| 5086 | IFNLR1 NM_170743_3 | KTLMGNPWFQRAKMPRALDFSGHTHPVATFQPSRPESVNDLFLCPQKELTRGVRPTPRVRAPATQQTRWK KDLAEDEEEEDEEDTEDGVSFQPYIEPPSFLGQEHQAPGHSEAGGVDSGRPRAPLVPSEGSSAWDSSDRSW ASTVDSSWDRAGSSGYLAEKGPGQGPGGDGHQESLPPPEFSKDSGFLEELPEDNLSSWATWGTLPPEPNLV PGGPPVSLQTLTFCWESSPEEEEEARESEIEDSDAGSWGAESTQRTEDRGRTLGHYMAR (SEQ ID NO:439) |
| 5087 | IFNLR1 transcript variant 2 NM_173064_2 | KTLMGNPWFQRAKMPRALELTRGVRPTPRVRAPATQQTRWKKDLAEDEEEEDEEDTEDGVSFQPYIEPPSF LGQEHQAPGHSEAGGVDSGRPRAPLVPSEGSSAWDSSDRSWASTVDSSWDRAGSSGYLAEKGPGQGPGG DGHQESLPPPEFSKDSGFLEELPEDNLSSWATWGTLPPEPNLVPGGPPVSLQTLTFCWESSPEEEEEARESEIE DSDAGSWGAESTQRTEDRGRTLGHYMAR (SEQ ID NO:440) |
| 5098 | IL1R1 transcript variant 2 NM_001288706_1 | KIDIVLWYRDSCYDFLPIKVLPEVLEKQCGYKLFIYGRDDYVGEDIVEVINENVKKSRRLIIILVRETSGFSWLGGS SEEQIAMYNALVQDGIKVVLLELEKIQDYEKMPESIKFIKQKHGAIRWSGDFTQGPQSAKTRFWKNVRYHMP VQRRSPSSKHQLLSPATKEKLQREAHVPLG (SEQ ID NO:441) |
| 5099 | IL1R1 transcript variant 3 NM_001320978_1 | KIDIVLWYRDSCYDFLPIKASDGKTYDAYILYPKTVGEGSTSDCDIFVFKVLPEVLEKQCGYKLFIYGRDDYVGED IVEVINENVKKSRRLIIILVRETSGFSWLGGSSEEQIAMYNALVQDGIKVVLLELEKIQDYEKMPESIKFIKQKHG AIRWSGDFTQGPQSAKTRFWKNVRYHMPVQRRSPSSKHQLLSPATKEKLQREAHVPLG (SEQ ID NO:442) |
| S100 | IL1RAP transcript variant 1 NM_002182_3 | YRAHFGTDETILDGKEYDIYVSYARNAEEEEFVLLTLRGVLENEFGYKLCIFDRDSLPGGIVTDETLSFIQKSRRLL VVLSPNYVLQGTQALLELKAGLENMASRGNINVILVQYKAVKETKVKELKRAKTVLTVIKWKGEKSKYPQGRF WKQLQVAMPVKKSPRRSSSDEQGLSYSSLKNV (SEQ ID NO:443) |
| S101 | IL1RAP transcript variant 6 NM_001167931_1 | YRAHFGTDETILDGKEYDIYVSYARNAEEEEFVLLTLRGVLENEFGYKLCIFDRDSLPGGNTVEAVFDFIQRSRR MIVVLSPDYVTEKSISMLEFKLGVMCQNSIATKLIVVEYRPLEHPHPGILQLKESVSFVSWKGEKSKHSGSKFW KALRLALPLRSLSASSGWNESCSSQSDISLDHVQRRRSRLKEPPELQSSERAAGSPPAPGTMSKHRGKSSATCR CCVTYCEGENHLRNKSRAEIHNQPQWETHLCKPVPQESETQWIQNGTRLEPPAPQISALALHHFTDLSNNN DFYIL (SEQ ID NO:444) |
| S102 | IL1RL1 transcript variant 1 NM_016232.4 | LKMFWIEATLLWRDIAKPYKTRNDGKLYDAYVVYPRNYKSSTDGASRVEHFVHQILPDVLENKCGYTLCIYGR DMLPGEDVVTAVETNIRKSRRHIFILTPQITHNKEFAYEQEVALHCALIQNDAKVILIEMEALSELDMLQAEAL QDSLQHLMKVQGTIKWREDHIANKRSLNSKFWKHVRYQMPVPSKIPRKASSLTPLAAQKQ (SEQ ID NO:445) |
| S103 | IL1RL2 NM_003854.2 | NIFKIDIVLWYRSAFHSTETIVDGKLYDAYVLYPKPHKESQRHAVDALVLNILPEVLERQCGYKLFIFGRDEFPG QAVANVIDENVKLCRRLIVIVVPESLGFGLLKNLSEEQIAVYSALIQDGMKVILIELEKIEDYTVMPESIQYIKQKH GAIRWHGDFTEQSQCMKTKFWKTVRYHMPPRRCRPFPPVQLLQHTPCYRTAGPELGSRRKKCTLTTG (SEQ ID NO:446) |
| S104 | IL2RA transcript variant 1 NM 000417 2 | TWQRRQRKSRRTI (SEQ ID NO:447) |
| S105 | IL2RB transcript variant 1 NM_000878_4 | NCRNTGPWLKKVLKCNTPDPSKFFSQLSSEHGGDVQKWLSSPFPSSSFSPGGLAPEISPLEVLERDKVTQLLLQ QDKVPEPASLSSNHSLTSCFTNQGYFFFHLPDALEIEACQVYFTYDPYSEEDPDEGVAGAPTGSSPQPLQPLSG EDDAYCTFPSRDDLLLFSPSLLGGPSPPSTAPGGSGAGEERMPPSLQERVPRDWDPQPLGPPTPGVPDLVDF QPPPELVLREAGEEVPDAGPREGVSFPWSRPPGQGEFRALNARLPLNTDAYLSLQELQGQDPTHLV (SEQ ID NO:448) |
| S106 | IL2RG NM_000206_2 | ERTMPRIPTLKNLEDLVTEYHGNFSAWSGVSKGLAESLQPDYSERLCLVSEIPPKGGALGEGPGASPCNQHSP YWAPPCYTLKPET (SEQ ID NO:449) |
| S109 | IL3RA transcript variant 1 and 2 NM_002183_3 | RRYLVMQRLFPRIPHMKDPIGDSFQNDKLVVWEAGKAGLEECLVTEVQVVQKT (SEQ ID NO:450) |
| S110 | IL4R transcript variant 1 NM_000418_3 | KIKKEWWDQIPNPARSRLVAIIIQDAQGSQWEKRSRGQEPAKCPHWKNCLTKLLPCFLEHNMKRDEDPHKA AKEMPFQGSGKSAWCPVEISKTVLWPESISVVRCVELFEAPVECEEEEEVEEEKGSFCASPESSRDDFQEGRE GIVARLTESLFLDLLGEENGGFCQQDMGESCLLPPSGSTSAHMPWDEFPSAGPKEAPPWGKEQPLHLEPSPP ASPTQSPDNLTCTETPLVIAGNPAYRSFSNSLSQSPCPRELGPDPLLARHLEEVEPEMPCVPQLSEPTTVPQPE PETWEQILRRNVLQHGAAAAPVSAPTSGYQEFVHAVEQGGTQASAVVGLGPPGEAGYKAFSSLLASSAVSPE KCGFGASSGEEGYKPFQDLIPGCPGDPAPVPVPLFTFGLDREPPRSPQSSHLPSSSPEHLGLEPGEKVEDMPKP PLPQEQATDPLVDSLGSGIVYSALTCHLCGHLKQCHGQEDGGQTPVMASPCCGCCCGDRSSPPTTPLRAPDP SPGGVPLEASLCPASLAPSGISEKSKSSSSFHPAPGNAQSSSQTPKIVNFVSVGPTYMRVS (SEQ ID NO:451) |
| S113 | IL4R transcript variant 1 NM_000418_3 | KIKKEWWDQIPNPARSRLVAIIIQDAQGSQWEKRSRGQEPAKCPHWKNCLTKLLPCFLEHNMKRDEDPHKA AKEMPFQGSGKSAWCPVEISKTVLWPESISVVRCVELFEAPVECEEEEEVEEEKGSFCASPESSRDDFQEGRE GIVARLTESLFLDLLGEENGGFCQQDMGESCLLPPSGSTSAHMPWDEFPSAGPKEAPPWGKEQPLHLEPSPP ASPTQSPDNLTCTETPLVIAGNPAYRSFSNSLSQSPCPRELGPDPLLARHLEEVEPEMPCVPQLSEPTTVPQPE PETWEQILRRNVLQHGAAAAPVSAPTSGYQEFVHAVEQGGTQASAVVGLGPPGEAGYKAFSSLLASSAVSPE KCGFGASSGEEGYKPFQDLIPGCPGDPAPVPVPLFTFGLDREPPRSPQSSHLPSSSPEHLGLEPGEKVEDMPKP PLPQEQATDPLVDSLGSGIVFSALTCHLCGHLKQCHGQEDGGQTPVMASPCCGCCCGDRSSPPTTPLRAPDP SPGGVPLEASLCPASLAPSGISEKSKSSSSFHPAPGNAQSSSQTPKIVNFVSVGPTYMRVS (SEQ ID NO:452) |
| S115 | IL5A transcript variant 1 NM_000564_4 | KICHLWIKLFPPIPAPKSNIKDLFVTTNYEKAGSSETEIEVICYIEKPGVETLEDSVF (SEQ ID NO:453) |
| S116 | IL6R transcript variant 1 NM_000565_3 | RFKKTWKLRALKEGKTSMHPPYSLGQLVPERPRPTPVLVPLISPPVSPSSLGSDNTSSHNRPDARDPRSPYDIS NTDYFFPR (SEQ ID NO:454) |
| S117 | IL6ST transcript variant 1 and 3 NM_002184_3 | NKRDLIKKHIWPNVPDPSKSHIAQWSPHTPPRHNFNSKDQMYSDGNFTDVSVVEIEANDKKPFPEDLKSLDL FKKEKINTEGHSSGIGGSSCMSSSRPSISSSDENESSQNTSSTVQYSTVVHSGYRHQVPSVQVFSRSESTQPLLD SEERPEDLQLVDHVDGGDGILPRQQYFKQNCSQHESSPDISHFERSKQVSSVNEEDFVRLKQQISDHISQSCG SGQMKMFQEVSAADAFGPGTEGQVERFETVGMEAATDEGMPKSYLPQTVRQGGYMPQ (SEQ ID NO:455) WKKRIKPIVWPSLPDHKKTLEHLCKKPRKNLNVSFNPESFLDCQIHRVDDIQARDEVEGFLQDTFPQQLEESE KQRLGGDVQSPNCPSEDVVITPESFGRDSSLTCLAGNVSACDAPILSSSRSLDCRESGKNGPHVYQDLLLSLGT TNSTLPPPFSLQSGILTLNPVAQGQPILTSLGSNQEEAYVTMSSFYQNQ (SEQ ID NO:456) |
| S120 | IL7RA Isoform 1 NM_002185.4 | WKKRIKPIVWPSLPDHKKTLEHLCKKPRKNLNVSFNPESFLDCQIHRVDDIQARDEVEGFLQDTFPQQLEESE KQRLGGDVQSPNCPSEDVVITPESFGRDSSLTCLAGNVSACDAPILSSSRSLDCRESGKNGPHVYQDLLLSLGT TNSTLPPPFSLQSGILTLNPVAQGQPILTSLGSNQEEAYVTMSSFYQNQ (SEQ ID NO:456) |
| S121 | IL7RA Isoform 3 (C-term deletion) (interleukin 7 receptor) | WKKRIKPIVWPSLPDHKKTLEHLCKKPRKVSVFGA (SEQ ID NO:457) |
| 5126 | IL9R transcript variant 1 NM_002186_2 | KLSPRVKRIFYQNVPSPAMFFQPLYSVHNGFQTWMGAHGAGVLLSQDCAGTPQGALEPCVQEATALLTC GPARPWKSVALEEEQEGPGTRLPGNLSSEDVLPAGCTEWRVQTLAYLPQEDWAPTSLTRPAPPDSEGSRSSS SSSSSNNNNYCALGCYGGWHLSALPGNTQSSGPIPALACGLSCDHQGLETQQGVAWVLAGHCQRPGLHED LQGMLLPSVLSKARSWTF (SEQ ID NO:458) |
| S129 | IL10RA transcript variant 1 NM_001558_3 | KLSPRVKRIFYQNVPSPAMFFQPLYSVHNGNFQTWMGAHGAGVLLSQDCAGTPQGALEPCVQEATALLTC GPARPWKSVALEEEQEGPGTRLPGNLSSEDVLPAGCTEWRVQTLAYLPQEDWAPTSLTRPAPPDSEGSRSSS SSSSSNNNNYCALGCYGGWHLSALPGNTQSSGPIPALACGLSCDHQGLETQQGVAWVLAGHCQRPGLHED LQGMLLPSVLSKARSWTF (SEQ ID NO:458) QLYVRRRKKLPSVLLFKKPSPFIFISQRPSPETQDTIHPLDEEAFLKVSPELKNLDLHGSTDSGFGSTKPSLQTEEP QFLLPDPHPQADRTLGNREPPVLGDSCSSGSSNSTDSGICLQEPSLSPSTGPTWEQQVGSNSRGQDDSGIDL VQNSEGRAGDTQGGSALGHHSPPEPEVPGEEDPAAVAFQGYLRQTRCAEEKATKTGCLEEESPLTDGLGPKF GRCLVDEAGLHPPALAKGYLKQDPLEMTLASSGAPTGQWNQPTEEWSLLALSSCSDLGISDWSFAHDLAPL GCVAAPGGLLGSFNSDLVTLPLISSLQSSE (SEQ ID NO:459) ALLWCVYKKTKYAFSPRNSLPQHLKEFLGHPHHNTLLFFSFPLSDENDVFDKLSVIAEDSESGKQNPGDSCSLG TPPGQGPQS (SEQ ID NO:460) |
| S130 | IL10RB NM 000628 4 | ALLWCVYKKTKYAFSPRNSLPQHLKEFLGHPHHNTLLFFSFPLSDENDVFDKLSVIAEDSESGKQNPGDSCSLG TPPGQGPQS (SEQ ID NO:460) |
| 5135 | IL11RA NM_001142784_2 | RLRRGGKDGSPKPGFLASVIPVDRRPGAPNL (SEQ ID NO:461) |
| S136 | IL12RB1 transcript variant 1 and 4 NM_005535_2 | NRAARHLCPPLPTPCASSAIEFPGGKETWQWINPVDFQEEASLQEALVVEMSWDKGERTEPLEKTELPEGAP ELALDTELSLEDGDRCKAKM (SEQ ID NO:462) |
| S137 | IL12RB1 transcript variant 3 NM_001290023_1 | NRAARHLCPPLPTPCASSAIEFPGGKETWQWINPVDFQEEASLQEALVVEMSWDKGERTEPLEKTELPEGAP ELALDTELSLEDGDRCKAKM (SEQ ID NO:462) NRAARHLCPPLPTPCASSAIEFPGGKETWQWINPVDFQEEASLQEALVVEMSWDKGERTEPLEKTELPEGAP ELALDTELSLEDGDRCDR (SEQ ID NO:463) HYFQQKVFVLLAALRPQWCSREIPDPANSTCAKKYPIAEEKTQLPLDRLLIDWPTPEDPEPLVISEVLHQVTPVF RHPPCSNWPQREKGIQGHQASEKDMMHSASSPPPPRALQAESRQLVDLYKVLESRGSDPKPENPACPWTV LPAGDLPTHDGYLPSNIDDLPSHEAPLADSLEELEPQHISLSVFPSSSLHPLTFSCGDKLTLDQLKMRCDSLML (SEQ ID NO:464) |
| S138 | IL12RB2 transcript variant 1 and 3 NM_001559_2 | HYFQQKVFVLLAALRPQWCSREIPDPANSTCAKKYPIAEEKTQLPLDRLLIDWPTPEDPEPLVISEVLHQVTPVF RHPPCSNWPQREKGIQGHQASEKDMMHSASSPPPPRALQAESRQLVDLYKVLESRGSDPKPENPACPWTV LPAGDLPTHDGYLPSNIDDLPSHEAPLADSLEELEPQHISLSVFPSSSLHPLTFSCGDKLTLDQLKMRCDSLML (SEQ ID NO:464) |
| S141 | IL13RA1 NM_001560_2 | KRLKIIIFPPIPDPGKIFKEMFGDQNDDTLHWKKYDIYEKQTKEETDSVVLIENLKKASQ (SEQ ID NO:465) |
| S142 | IL13RA2 NM_000640_2 | RKPNTYPKMIPEFFCDT (SEQ ID NO:466) |
| S143 | IL15RA transcript variant 4 NM_001256765_1 | KSRQTPPLASVEMEAMEALPVTWGTSSRDEDLENCSHHL (SEQ ID NO:467) |
| S144 | IL17RA NM_014339_6 | CMTWRLAGPGSEKYSDDTKYTDGLPAADLIPPPLKPRKVWIIYSADHPLYVDVVLKFAQFLLTACGTEVALDLL EEQAISEAGVMTWVGRQKQEMVESNSKIIVLCSRGTRAKWQALLGRGAPVRLRCDHGKPVGDLFTAAMN MILPDFKRPACFGTYVVCYFSEVSCDGDVPDLFGAAPRYPLMDRFEEVYFRIQDLEMFQPGRMHRVGELSG DNYLRSPGGRQLRAALDRFRDWQVRCPDWFECENLYSADDQDAPSLDEEVFEEPLLPPGTGIVKRAPLVREP GSQACLAIDPLVGEEGGAAVAKLEPHLQPRGQPAPQPLHTLVLAAEEGALVAAVEPGPLADGAAVRLALAGE GEACPLLGSPGAGRNSVLFLPVDPEDSPLGSSTPMASPDLLPEDVREHLEGLMLSLFEQSLSCQAQGGCSRPA MVLTDPHTPYEEEQRQSVQSDQGYISRSSPQPPEGLTEMEEEEEEEQDPGKPALPLSPEDLESLRSLQRQLLFR QLQKNSGWDTMGSESEGPSA (SEQ ID NO:468) |
| 5145 | IL17RB NM_018725_3 | RHERIKKTSFSTTTLLPPIKVLVVYPSEICFHHTICYFTEFLQNHCRSEVILEKWQKKKIAEMGPVQWLATQKKA ADKVVFLLSNDVNSVCDGTCGKSEGSPSENSQDLFPLAFNLFCSDLRSQIHLHKYVVVYFREIDTKDDYNALSV CPKYHLMKDATAFCAELLHVKQQVSAGKRSQACHDGCCSL (SEQ ID NO:469) |
| S146 | IL17RC transcript variant 1 NM_153460_3 | KKDHAKGWLRLLKQDVRSGAAARGRAALLLYSADDSGFERLVGALASALCQLPLRVAVDLWSRRELSAQGP VAWFHAQRRQTLQEGGVVVLLFSPGAVALCSEWLQDGVSGPGAHGPHDAFRASLSCVLPDFLQGRAPGSY VGACFDRLLHPDAVPALFRTVPVFTLPSQLPDFLGALQQPRAPRSGRLQERAEQVSRALQPALDSYFHPPGTP APGRGVGPGAGPGAGDGT (SEQ ID NO:470) |
| S147 | IL17RC transcript variant 4 NM_001203263_1 | KKDHAKAAARGRAALLLYSADDSGFERLVGALASALCQLPLRVAVDLWSRRELSAQGPVAWFHAQRRQTLQ EGGVVVLLFSPGAVALCSEWLQDGVSGPGAHGPHDAFRASLSCVLPDFLQGRAPGSYVGACFDRLLHPDAV PALFRTVPVFTLPSQLPDFLGALQQPRAPRSGRLQERAEQVSRALQPALDSYFHPPGTPAPGRGVGPGAGPG AGDGT (SEQ ID NO:471) |
| S148 | IL17RD transcript variant 2 NM_017563_4 | RHERIKKTSFSTTTLLPPIKVLVVYPSEICFHHTICYFTEFLQNHCRSEVILEKWQKKKIAEMGPVQWLATQKKA ADKVVFLLSNDVNSVCDGTCGKSEGSPSENSQDLFPLAFNLFCSDLRSQIHLHKYVVVYFREIDTKDDYNALSV CPKYHLMKDATAFCAELLHVKQQVSAGKRSQACHDGCCSL (SEQ ID NO:469) KKDHAKGWLRLLKQDVRSGAAARGRAALLLYSADDSGFERLVGALASALCQLPLRVAVDLWSRRELSAQGP VAWFHAQRRQTLQEGGVVVLLFSPGAVALCSEWLQDGVSGPGAHGPHDAFRASLSCVLPDFLQGRAPGSY VGACFDRLLHPDAVPALFRTVPVFTLPSQLPDFLGALQQPRAPRSGRLQERAEQVSRALQPALDSYFHPPGTP APGRGVGPGAGPGAGDGT (SEQ ID NO:470) KKDHAKAAARGRAALLLYSADDSGFERLVGALASALCQLPLRVAVDLWSRRELSAQGPVAWFHAQRRQTLQ EGGVVVLLFSPGAVALCSEWLQDGVSGPGAHGPHDAFRASLSCVLPDFLQGRAPGSYVGACFDRLLHPDAV PALFRTVPVFTLPSQLPDFLGALQQPRAPRSGRLQERAEQVSRALQPALDSYFHPPGTPAPGRGVGPGAGPG AGDGT (SEQ ID NO:471) CRKKQQENIYSHLDEESSESSTYTAALPRERLRPRPKVFLCYSSKDGQNHMNVVQCFAYFLQDFCGCEVALDL WEDFSLCREGQREWVIQKIHESQFIIVVCSKGMKYFVDKKNYKHKGGGRGSGKGELFLVAVSAIAEKLRQAK QSSSAALSKFIAVYFDYSCEGDVPGILDLSTKYRLMDNLPQLCSHLHSRDHGLQEPGQHTRQGSRRNYFRSKS GRSLYVAICNMHQFIDEEPDWFEKQFVPFHPPPLRYREPVLEKFDSGLVLNDVMCKPGPESDFCLKVEAAVL GATGPADSQHESQHGGLDQDGEARPALDGSAALQPLLHTVKAGSPSDMPRDSGIYDSSVPSSELSLPLMEG LSTDQTETSSLTESVSSSSGLGEEEPPALPSKLLSSGSCKADLGCRSYTDELHAVAPL (SEQ ID NO:472) TCRRPQSGPGPARPVLLLHAADSEAQRRLVGALAELLRAALGGGRDVIVDLWEGRHVARVGPLPWLWAAR TRVAREQGTVLLLWSGADLRPVSGPDPRAAPLLALLHAAPRPLLLLAYFSRLCAKGDIPPPLRALPRYRLLRDLP RLLRALDARPFAEATSWGRLGARQRRQSRLELCSRLEREAARLADLG (SEQ ID NO:473) YRVDLVLFYRHLTRRDETLTDGKTYDAFVSYLKECRPENGEEHTFAVEILPRVLEKHFGYKLCIFERDVVPGGAV VDEIHSLIEKSRRLIIVLSKSYMSNEVRYELESGLHEALVERKIKIILIEFTPVTDFTFLPQSLKLLKSHRVLKWKADK SLSYNSRFWKNLLYLMPAKTVKPGRDEPEVLPVLSES (SEQ ID NO:474) SALLYRHWIEIVLLYRTYQSKDQTLGDKKDFDAFVSYAKWSSFPSEATSSLSEEHLALSLFPDVLENKYGYSLCLL ERDVAPGGVYAEDIVSIIKRSRRGIFILSPNYVNGPSIFELQAAVNLALDDQTLKLILIKFCYFQEPESLPHLVKKAL RVLPTVTWRGLKSVPPNSRFWAKMRYHMPVKNSQGFTWNQLRITSRIFQWKGLSRTETTGRSSQPKEW (SEQ ID NO:475) SIYRYIHVGKEKHPANLILIYGNEFDKRFFVPAEKIVINFITLNISDDSKISHQDMSLLGKSSDVSSLNDPQPSGNL RPPQEEEEVKHLGYASHLMEIFCDSEENTEGTSLTQQESLSRTIPPDKTVIEYEYDVRTTDICAGPEEQELSLQEE VSTQGTLLESQAALAVLGPQTLQYSYTPQLQDLDPLAQEHTDSEEGPEEEPSTTLVDWDPQTGRLCIPSLSSFD QDSEGCEPSEGDGLGEEGLLSRLYEEPAPDRPPGENETYLMQFMEEWGLYVQMEN (SEQ ID NO:476) |
| S149 | IL17RE transcript variant 1 NM_153480_1 | LSTDQTETSSLTESVSSSSGLGEEEPPALPSKLLSSGSCKADLGCRSYTDELHAVAPL (SEQ ID NO:472) TCRRPQSGPGPARPVLLLHAADSEAQRRLVGALAELLRAALGGGRDVIVDLWEGRHVARVGPLPWLWAAR TRVAREQGTVLLLWSGADLRPVSGPDPRAAPLLALLHAAPRPLLLLAYFSRLCAKGDIPPPLRALPRYRLLRDLP RLLRALDARPFAEATSWGRLGARQRRQSRLELCSRLEREAARLADLG (SEQ ID NO:473) |
| 5154 | IL18R1 transcript variant 1 NM_003855_3 | YRVDLVLFYRHLTRRDETLTDGKTYDAFVSYLKECRPENGEEHTFAVEILPRVLEKHFGYKLCIFERDVVPGGAV VDEIHSLIEKSRRLIIVLSKSYMSNEVRYELESGLHEALVERKIKIILIEFTPVTDFTFLPQSLKLLKSHRVLKWKADK SLSYNSRFWKNLLYLMPAKTVKPGRDEPEVLPVLSES (SEQ ID NO:474) |
| 5155 | IL18RAP NM_003853_3 | SALLYRHWIEIVLLYRTYQSKDQTLGDKKDFDAFVSYAKWSSFPSEATSSLSEEHLALSLFPDVLENKYGYSLCLL ERDVAPGGVYAEDIVSIIKRSRRGIFILSPNYVNGPSIFELQAAVNLALDDQTLKLILIKFCYFQEPESLPHLVKKAL RVLPTVTWRGLKSVPPNSRFWAKMRYHMPVKNSQGFTWNQLRITSRIFQWKGLSRTETTGRSSQPKEW (SEQ ID NO:475) |
| 5156 | IL20RA transcript variant 1 NM_014432_3 | (SEQ ID NO:475) SIYRYIHVGKEKHPANLILIYGNEFDKRFFVPAEKIVINFITLNISDDSKISHQDMSLLGKSSDVSSLNDPQPSGNL RPPQEEEEVKHLGYASHLMEIFCDSEENTEGTSLTQQESLSRTIPPDKTVIEYEYDVRTTDICAGPEEQELSLQEE VSTQGTLLESQAALAVLGPQTLQYSYTPQLQDLDPLAQEHTDSEEGPEEEPSTTLVDWDPQTGRLCIPSLSSFD QDSEGCEPSEGDGLGEEGLLSRLYEEPAPDRPPGENETYLMQFMEEWGLYVQMEN (SEQ ID NO:476) |
| 5157 | IL20RB NM_144717_3 | WKMGRLLQYSCCPVVVLPDTLKITNSPQKLISCRREEVDACATAVMSPEELLRAWIS (SEQ ID NO:477) |
| 5158 | IL21R transcript variant 2 NM_181078_2 | SLKTHPLWRLWKKIWAVPSPERFFMPLYKGCSGDFKKWVGAPFTGSSLELGPWSPEVPSTLEVYSCHPPRSP AKRLQLTELQEPAELVESDGVPKPSFWPTAQNSGGSAYSEERDRPYGLVSIDTVTVLDAEGPCTWPCSCEDD GYPALDLDAGLEPSPGLEDPLLDAGTTVLSCGCVSAGSPGLGGPLGSLLDRLKPPLADGEDWAGGLPWGGRS PGGVSESEAGSPLAGLDMDTFDSGFVGSDCSSPVECDFTSPGDEGPPRSYLRQWVVIPPPLSSPGPQAS (SEQ ID NO:478) |
| S161 | IL22RA1 NM_021258_3 | SYRYVTKPPAPPNSLNVQRVLTFQPLRFIQEHVLIPVFDLSGPSSLAQPVQYSQIRVSGPREPAGAPQRHSLSEI TYLGQPDISILQPSNVPPPQILSPLSYAPNAAPEVGPPSYAPQVTPEAQFPFYAPQAISKVQPSSYAPQATPDS WPPSYGVCMEGSGKDSPTGTLSSPKHLRPKGQLQKEPPAGSCMLGGLSLQEVTSLAMEESQEAKSLHQPLGI CTDRTSDPNVLHSGEEGTPQYLKGQLPLLSSVQIEGHPMSLPLQPPSRPCSPSDQGPSPWGLLESLVCPKDEA KSPAPETSDLEQPTELDSLFRGLALTVQWES (SEQ ID NO:479) |
| 5165 | IL23R NM_144701_2 | NRSFRTGIKRRILLLIPKWLYEDIPNMKNSNVVKMLQENSELMNNNSSEQVLYVDPMITEIKEIFIPEHKPTDYK KENTGPLETRDYPQNSLFDNTTVVYIPDLNTGYKPQISNFLPEGSHLSNNNEITSLTLKPPVDSLDSGNNPRLQ KHPNFAFSVSSVNSLSNTIFLGELSLILNQGECSSPDIQNSVEEETTMLLENDSPSETIPEQTLLPDEFVSCLGIVN EELPSINTYFPQNILESHFNRISLLEK (SEQ ID NO:480) [?] |
| S168 | IL27RA NM_004843_3 | SLKTHPLWRLWKKIWAVPSPERFFMPLYKGCSGDFKKWVGAPFTGSSLELGPWSPEVPSTLEVYSCHPPRSP AKRLQLTELQEPAELVESDGVPKPSFWPTAQNSGGSAYSEERDRPYGLVSIDTVTVLDAEGPCTWPCSCEDD GYPALDLDAGLEPSPGLEDPLLDAGTTVLSCGCVSAGSPGLGGPLGSLLDRLKPPLADGEDWAGGLPWGGRS PGGVSESEAGSPLAGLDMDTFDSGFVGSDCSSPVECDFTSPGDEGPPRSYLRQWVVIPPPLSSPGPQAS (SEQ ID NO:478) SYRYVTKPPAPPNSLNVQRVLTFQPLRFIQEHVLIPVFDLSGPSSLAQPVQYSQIRVSGPREPAGAPQRHSLSEI TYLGQPDISILQPSNVPPPQILSPLSYAPNAAPEVGPPSYAPQVTPEAQFPFYAPQAISKVQPSSYAPQATPDS WPPSYGVCMEGSGKDSPTGTLSSPKHLRPKGQLQKEPPAGSCMLGGLSLQEVTSLAMEESQEAKSLHQPLGI CTDRTSDPNVLHSGEEGTPQYLKGQLPLLSSVQIEGHPMSLPLQPPSRPCSPSDQGPSPWGLLESLVCPKDEA KSPAPETSDLEQPTELDSLFRGLALTVQWES (SEQ ID NO:479) NRSFRTGIKRRILLLIPKWLYEDIPNMKNSNVVKMLQENSELMNNNSSEQVLYVDPMITEIKEIFIPEHKPTDYK KENTGPLETRDYPQNSLFDNTTVVYIPDLNTGYKPQISNFLPEGSHLSNNNEITSLTLKPPVDSLDSGNNPRLQ KHPNFAFSVSSVNSLSNTIFLGELSLILNQGECSSPDIQNSVEEETTMLLENDSPSETIPEQTLLPDEFVSCLGIVN EELPSINTYFPQNILESHFNRISLLEK (SEQ ID NO:480) TSGRCYHLRHKVLPRWVWEKVPDPANSSSGQPHMEQVPEAQPLGDLPILEVEEMEPPPVMESSQPAQATA PLDSGYEKHFLPTPEELGLLGPPRPQVLA (SEQ ID NO:481) TSWVWEKVPDPANSSSGQPHMEQVPEAQPLGDLPILEVEEMEPPPVMESSQPAQATAPLDSGYEKHFLPT PEELGLLGPPRPQVLA (SEQ ID NO:482) KKPNKLTHLCWPTVPNPAESSIATWHGDDFKDKLNLKESDDSVNTEDRILKPCSTPSDKLVIDKLVVNFGNVL QEIFTDEARTGQENNLGGEKNGYVTCPFRPDCPLGKSFEELPVSPEIPPRKSQYLRSRMPEGTRPEAKEQLLFS GQSLVPDHLCEEGAPNPYLKNSVTAREFLVSEKLPEHTKGEV (SEQ ID NO:483) KKPNKLTHLCWPTVPNPAESSIATWHGDDFKDKLNLKESDDSVNTEDRILKPCSTPSDKLVIDKLVVNFGNVL QEIFTDEARTGQENNLGGEKNGTRILSSCPTSI (SEQ ID NO:484) SHQRMKKLFWEDVPNPKNCSWAQGLNFQKPETFEHLFIKHTASVTCGPLLLEPETISEDISVDTSWKNKDEM MPTTVVSLLSTTDLEKGSVCISDQFNSVNFSEAEGTEVTYEDESQRQPFVKYATLISNSKPSETGEEQGLINSSV TKCFSSKNSPLKDSFSNSSWEIEAQAFFILSDQHPNIISPHLTFSEGLDELLKLEGNFPEENNDKKSIYYLGVTSIK KRESGVLLTDKSRVSCPFPAPCLFTDIRVLQDSCSHFVENNINLGTSSKKTFASYMPQFQTCSTQTHKIMENK MCDLTV (SEQ ID NO:485) SHQRMKKLFWEDVPNPKNCSWAQGLNFQKMLEGSMFVKSHHHSLISSTQGHKHCGRPQGPLHRKTRDLC SLVYLLTLPPLLSYDPAKSPSVRNTQE (SEQ ID NO:486) |
| S169 | IL27RA NM_004843_3 | TSWVWEKVPDPANSSSGQPHMEQVPEAQPLGDLPILEVEEMEPPPVMESSQPAQATAPLDSGYEKHFLPT PEELGLLGPPPPQVLA (SEQ ID NO:482) |
| S170 | IL31RA transcript variant 1 NM_139017_5 | FLLLGLLGPPRPQVLA (SEQ ID NO:482) KKPNKLTHLCWPTVPNPAESSIATWHGDDFKDKLNLKESDDSVNTEDRILKPCSTPSDKLVIDKLVVNFGNVL QEIFTDEARTGQENNLGGEKNGYVTCPFRPDCPLGKSFEELPVSPEIPPRKSQYLRSRMPEGTRPEAKEQLLFS GQSLVPDHLCEEGAPNPYLKNSVTAREFLVSEKLPEHTKGEV (SEQ ID NO:483) |
| S171 | IL31RA transcript variant 4 NM_001242638_1 | KKPNKLTHLCWPTVPNPAESSIATWHGDDFKDKLNLKESDDSVNTEDRILKPCSTPSDKLVIDKLVVNFGNVL QEIFTDEARTGQENNLGGEKNGTRILSSCPTSI (SEQ ID NO:484) |
| S174 | LEPR transcript variant 1 NM_002303_5 | SHQRMKKLFWEDVPNPKNCSWAQGLNFQKPETFEHLFIKHTASVTCGPLLLEPETISEDISVDTSWKNKDEM MPTTVVSLLSTTDLEKGSVCISDQFNSVNFSEAEGTEVTYEDESQRQPFVKYATLISNSKPSETGEEQGLINSSV TKCFSSKNSPLKDSFSNSSWEIEAQAFFILSDQHPNIISPHLTFSEGLDELLKLEGNFPEENNDKKSIYYLGVTSIK KRESGVLLTDKSRVSCPFPAPCLFTDIRVLQDSCSHFVENNINLGTSSKKTFASYMPQFQTCSTQTHKIMENK MCDLTV (SEQ ID NO:485) |
| 5175 | LEPR transcript variant 2 NM_001003680_3 | SHQRMKKLFWEDVPNPKNCSWAQGLNFQKMLEGSMFVKSHHHSLISSTQGHKHCGRPQGPLHRKTRDLC SLVYLLTLPPLLSYDPAKSPSVRNTQE (SEQ ID NO:486) |
| S176 | LEPR transcript variant 3 NM_001003679_3 | SHQRMKKLFWEDVPNPKNCSWAQGLNFQKRTDIL (SEQ ID NO:487) |
| S177 | LEPR transcript variant 5 NM 001198688 1 | SHQRMKKLFWEDVPNPKNCSWAQGLNFQKKMPGTKELLGGGWLT (SEQ ID NO:488) |
| 5180 | LIFR NM_001127671_1 | YRKREWIKETFYPDIPNPENCKALQFQKSVCEGSSALKTLEMNPCTPNNVEVLETRSAFPKIEDTEIISPVAERP EDRSDAEPENHVVVSYCPPIIEEEIPNPAADEAGGTAQVIYIDVQSMYQPQAKPEEEQENDPVGGAGYKPQ MHLPINSTVEDIAAEEDLDKTAGYRPQANVNTWNLVSPDSPRSIDSNSEIVSFGSPCSINSRQFLIPPKDEDSPK SNGGGWSFTNFFQNKPND (SEQ ID NO:489) |
| S183 | LMP1 NC_007605_1 | YYHGQRHSDEHHHDDSLPHPQQATDDSGHESDSNSNEGRHHLLVSGAGDGPPLCSQNLGAPGGGPDNGP QDPDNTDDNGPQDPDNTDDNGPHDPLPQDPDNTDDNGPQDPDNTDDNGPHDPLPHSPSDSAGNDGGP PQLTEEVENKGGDQGPPLMTDGGGGHSHDSGHGGGDPHLPTLLLGSSGSGGDDDDPHGPVQLSYYD (SEQ ID NO:490) |
| S186 | MPL NM_005373 2 | RWQFPAHYRRLRHALWPSLPDLHRVLGQYLRDTAALSPPKATVSDTCEEVEPSLLEILPKSSERTPLPLCSSQA QMDYRRLQPSCLGTMPLSVCPPMAESGSCCTTHIANHSYLPLSYWQQP (SEQ ID NO:491) |
| S189 | MYD88 transcript variant 1 NM_001172567_1 | MAAGGPGAGSAAPVSSTSSLPLAALNMRVRRRLSLFLNVRTQVAADWTALAEEMDFEYLEIRQLETQADPT GRLLDAWQGRPGASVGRLLELLTKLGRDDVLLELGPSIEEDCQKYILKQQQEEAEKPLQVAAVDSSVPRTAEL AGITTLDDPLGHMPERFDAFICYCPSDIQFVQEMIRQLEQTNYRLKLCVSDRDVLPGTCVWSIASELIEKRLAR RPRGGCRRMVVVVSDDYLQSKECDFQTKFALSLSPGAHQKRLIPIKYKAMKKEFPSILRFITVCDYTNPCTKSW FWTRLAKALSLP (SEQ ID NO:492) |
| S190 | MYD88 transcript variant 2 NM_002468_4 | MAAGGPGAGSAAPVSSTSSLPLAALNMRVRRRLSLFLNVRTQVAADWTALAEEMDFEYLEIRQLETQADPT GRLLDAWQGRPGASVGRLLELLTKLGRDDVLLELGPSIEEDCQKYILKQQQEEAEKPLQVAAVDSSVPRTAEL AGITTLDDPLGHMPERFDAFICYCPSDIQFVQEMIRQLEQTNYRLKLCVSDRDVLPGTCVWSIASELIEKRCRR MVVVVSDDYLQSKECDFQTKFALSLSPGAHQKRLIPIKYKAMKKEFPSILRFITVCDYTNPCTKSWFWTRLAKA LSLP (SEQ ID NO:493) |
| S191 | MYD88 transcript variant 3 NM_001172568_1 | MAAGGPGAGSAAPVSSTSSLPLAALNMRVRRRLSLFLNVRTQVAADWTALAEEMDFEYLEIRQLETQADPT GRLLDAWQGRPGASVGRLLELLTKLGRDDVLLELGPSIGHMPERFDAFICYCPSDIQFVQEMIRQLEQTNYRL KLCVSDRDVLPGTCVWSIASELIEKRCRRMVVVVSDDYLQSKECDFQTKFALSLSPGAHQKRLIPIKYKAMKKE FPSILRFITVCDYTNPCTKSWFWTRLAKALSLP (SEQ ID NO:494) |
| S192 | MYD88 transcript variant 4 NM_01172569_1 | MAAGGPGAGSAAPVSSTSSLPLAALNMRVRRRLSLFLNVRTQVAADWTALAEEMDFEYLEIRQLETQADPT GRLLDAWQGRPGASVGRLLELLTKLGRDDVLLELGPSIEEDCQKYILKQQQEEAEKPLQVAAVDSSVPRTAEL AGITTLDDPLGAAGWWWLSLMITCRARNVTSRPNLHSASLQVPIRSD (SEQ ID NO:495) |
| S193 | MYD88 transcript variant 5 NM_001172566_1 | MAAGGPGAGSAAPVSSTSSLPLAALNMRVRRRLSLFLNVRTQVAADWTALAEEMDFEYLEIRQLETQADPT GRLLDAWQGRPGASVGRLLELLTKLGRDDVLLELGPSIGAAGWWWLSLMITCRARNVTSRPNLHSASLQVPI RSD (SEQ ID NO:496) |
| S194 | MYD88 transcript variant 1 NM_001172567_1 | MAAGGPGAGSAAPVSSTSSLPLAALNMRVRRRLSLFLNVRTQVAADWTALAEEMDFEYLEIRQLETQADPT GRLLDAWQGRPGASVGRLLELLTKLGRDDVLLELGPSIEEDCQKYILKQQQEEAEKPLQVAAVDSSVPRTAEL AGITTLDDPLGHMPERFDAFICYCPSDI (SEQ ID NO:497) |
| 5195 | MYD88 transcript variant 3 NM_001172568_1 | MAAGGPGAGSAAPVSSTSSLPLAALNMRVRRRLSLFLNVRTQVAADWTALAEEMDFEYLEIRQLETQADPT GRLLDAWQGRPGASVGRLLELLTKLGRDDVLLELGPSIGHMPERFDAFICYCPSDI (SEQ ID NO:498) |
| S196 | MYD88 transcript variant 1 NM_001172567_1 | MAAGGPGAGSAAPVSSTSSLPLAALNMRVRRRLSLFLNVRTQVAADWTALAEEMDFEYLEIRQLETQADPT GRLLDAWQGRPGASVGRLLELLTKLGRDDVLLELGPSIEEDCQKYILKQQQEEAEKPLQVAAVDSSVPRTAEL AGITTLDDPLGHMPERFDAFICYCPSDIQFVQEMIRQLEQTNYRLKLCVSDRDVLPGTCVWSIASELIEKRLAR RPRGGCRRMVVVVSDDYLQSKECDFQTKFALSLSPGAHQKRPIPIKYKAMKKEFPSILRFITVCDYTNPCTKSW FWTRLAKALSLP (SEQ ID NO:499) |
| S197 | MYD88 transcript variant 2 NM_002468_4 | MAAGGPGAGSAAPVSSTSSLPLAALNMRVRRRLSLFLNVRTQVAADWTALAEEMDFEYLEIRQLETQADPT GRLLDAWQGRPGASVGRLLELLTKLGRDDVLLELGPSIEEDCQKYILKQQQEEAEKPLQVAAVDSSVPRTAEL AGITTLDDPLGHMPERFDAFICYCPSDIQFVQEMIRQLEQTNYRLKLCVSDRDVLPGTCVWSIASELIEKRCRR MVVVVSDDYLQSKECDFQTKFALSLSPGAHQKRPIPIKYKAMKKEFPSILRFITVCDYTNPCTKSWFWTRLAKA LSLP (SEQ ID NO:500) |
| S198 | MYD88 transcript variant 3 NM_001172568_1 | MAAGGPGAGSAAPVSSTSSLPLAALNMRVRRRLSLFLNVRTQVAADWTALAEEMDFEYLEIRQLETQADPT GRLLDAWQGRPGASVGRLLELLTKLGRDDVLLELGPSIGHMPERFDAFICYCPSDIQFVQEMIRQLEQTNYRL KLCVSDRDVLPGTCVWSIASELIEKRCRRMVVVVSDDYLQSKECDFQTKFALSLSPGAHQKRPIPIKYKAMKKE FPSILRFITVCDYTNPCTKSWFWTRLAKALSLP (SEQ ID NO:501) |
| S199 | OSMR transcript variant 4 NM_001323505_1 | KSQWIKETCYPDIPDPYKSSILSLIKFKENPHLIIMNVSDCIPDAIEVVSKPEGTKIQFLGTRKSLTETELTKPNYLYL LPTEKNHSGPGPCICFENLTYNQAASDSGSCGHVPVSPKAPSMLGLMTSPENVLKALEKNYMNSLGEIPAGE TSLNYVSQLASPMFGDKDSLPTNPVEAPHCSEYKMQMAVSLRLALPPPTENSSLSSITLLDPGEHYC (SEQ ID NO:502) |
| S202 | PRLR transcript variant 1 NM_000949_6 | KGYSMVTCIFPPVPGPKIKGFDAHLLEKGKSEELLSALGCQDFPPTSDYEDLLVEYLEVDDSEDQHLMSVHSKE HPSQGMKPTYLDPDTDSGRGSCDSPSLLSEKCEEPQANPSTFYDPEVIEKPENPETTHTWDPQCISMEGKIPY FHAGGSKCSTWPLPQPSQHNPRSSYHNITDVCELAVGPAGAPATLLNEAGKDALKSSQTIKSREEGKATQQR EVESFHSETDQDTPWLLPQEKTPFGSAKPLDYVEIHKVNKDGALSLLPKQRENSGKPKKPGTPENNKEYAKVS |
| | | GVMDNNILVLYPDPHAKNVACFEESAKEAPPSLEQNQAEKALANFTATSSKCRLQLGGLDYLDPACFTHSFH (SEQ ID NO:503) |
| 5211 | TNFRSF4 NM_003327_3 | ALYLLRRDQRLPPDAHKPPGGGSFRTPIQEEQADAHSTLAKI (SEQ ID NO:504) |
| S212 | TNFRSF8 transcript variant 1 NM_001243_4 | HRRACRKRIRQKLHLCYPVQTSQPKLELVDSRPRRSSTQLRSGASVTEPVAEERGLMSQPLMETCHSVGAAYL ESLPLQDASPAGGPSSPRDLPEPRVSTEHTNNKIEKIYIMKADTVIVGTVKAELPEGRGLAGPAEPELEEEELEAD HTPHYPEQETEPPLGSCSDVMLSVEEEGKEDPLPTAASGK (SEQ ID NO:505) |
| 5213 | TNFRSF9 NM_001561_5 | KRGRKKLLYIFKQPFMRPVQTTQEEDGCSCRFPEEEEGGCEL (SEQ ID NO:506) |
| 5214 | TNFRSF14 transcript variant 1 NM_003820_3 | CVKRRKPRGDVVKVIVSVQRKRQEAEGEATVIEALQAPPDVTTVAVEETIPSFTGRSPNH (SEQ ID NO:507) |
| 5215 | TNFRSF18 transcript variant 1 NM_0041952 | QLGLHIWQLRSQCMWPRETQLLLEVPPSTEDARSCQFPEEERGERSAEEKGRLGDLWV (SEQ ID NO:508) |
| 5216 | TNFRSF18 transcript variant 3_NM_148902_1 | QLGLHIWQLRKTQLLLEVPPSTEDARSCQFPEEERGERSAEEKGRLGDLWV (SEQ ID NO:509) |
| X001 | Linker | GSGGSEGGGSEGGAATAGSGSGS (SEQ ID NO:510) |

**Table 7. Library 1A Top Constructs.**

| BlockSequence | Norm_D7 | Enrich_D35 | BlockSequence | Norm_D7 | Enrich_D35 |
|---|---|---|---|---|---|
| M008-5212-5075 | 1.42 | 10.58 | M008-S039-S212 | 2.44 | 8.05 |
| M025-S94-S45 | 22.50 | 7.23 | M049-S195-S213 | 2.46 | 8.03 |
| M018-S195-S104 | 2.75 | 9.75 | M049-S045-S142 | 4.08 | 7.47 |
| M030-S076-S104 | 2.60 | 9.49 | M007-S109-S076 | 7.95 | 6.65 |
| M049-S211-S076 | 7.09 | 8.24 | M043-5087-5038 | 1.42 | 8.53 |
| M013-S165-S194 | 2.96 | 9.15 | M049-5081-5074 | 2.34 | 8.06 |
| M013-S038-S059 | 14.06 | 7.15 | M008-S045-S126 | 1.62 | 8.41 |
| M019-S059-S043 | 0.00 | 10.85 | M008-S044-S039 | 10.46 | 6.28 |
| M008-5047-5053 | 7.27 | 7.78 | M037-S180-S157 | 0.00 | 9.79 |
| M030-S104-S176 | 2.31 | 9.10 | M007-S099-S059 | 17.07 | 5.61 |
| M013-S121-S053 | 19.93 | 6.41 | M024-S197-S037 | 3.32 | 7.68 |
| M025-S103-S141 | 2.73 | 8.90 | M036-S104-S154 | 13.42 | 5.94 |
| M031-5074-5038 | 2.71 | 8.86 | M042-S135-S120 | 0.00 | 9.78 |
| M013-5084-5059 | 9.91 | 7.30 | M037-5059-5045 | 3.19 | 7.70 |
| M007-S137-S047 | 13.29 | 6.87 | M024-S038-S213 | 0.00 | 9.72 |
| M043-S169-S213 | 2.53 | 8.88 | M009-5051-5216 | 0.00 | 9.64 |
| M013-S190-S047 | 0.00 | 10.68 | M049-S116-S043 | 0.00 | 9.64 |
| M007-S116-S050 | 10.42 | 7.14 | M025-S183-S189 | 0.00 | 9.61 |
| M043-S106-S176 | 6.06 | 7.82 | M019-S083-S050 | 0.00 | 9.59 |
| M031-S190-S141 | 3.01 | 8.62 | M007-S053-S189 | 0.00 | 9.58 |
| M002-S143-S212 | 9.08 | 7.24 | M049-5054-5216 | 0.00 | 9.52 |
| M018-S157-S216 | 24.51 | 5.90 | M049-S154-S143 | 0.00 | 9.47 |
| M019-S136-S214 | 2.65 | 8.68 | M002-S170-S043 | 0.00 | 9.46 |
| M002-S135-S104 | 11.35 | 6.85 | M025-S049-S168 | 0.00 | 9.38 |
| M030-S100-S064 | 7.68 | 7.35 | M049-5086-5045 | 0.00 | 9.38 |
| M043-S109-S176 | 3.71 | 8.22 | M036-S045-S043 | 0.00 | 9.35 |
| M049-S083-S190 | 25.27 | 5.71 | M043-S104-S213 | 0.00 | 9.32 |
| M001-S064-S036 | 1.30 | 9.21 | M031-S192-S047 | 0.00 | 9.32 |
| M043-S106-S211 | 13.93 | 6.49 | M043-S044-S136 | 0.00 | 9.30 |
| M009-S038-S212 | 5.54 | 7.66 | M048-S171-S075 | 0.00 | 9.30 |
| M007-5216-5052 | 0.50 | 9.75 | M008-S050-S104 | 0.00 | 9.29 |
| M048-S109-S054 | 16.67 | 6.19 | M007-S051-S109 | 0.00 | 9.28 |
| M049-S121-S169 | 12.36 | 6.58 | M002-S130-S037 | 0.00 | 9.18 |
| M010-S194-S214 | 2.92 | 8.34 | M002-S106-S141 | 0.00 | 9.16 |
| M019-S169-S038 | 2.93 | 8.32 | M018-S053-S051 | 0.00 | 9.09 |
| M019-S142-S053 | 14.15 | 6.38 | M018-S216-S104 | 0.00 | 9.00 |
| M043-S109-S215 | 0.00 | 10.29 | M008-S050-S048 | 0.00 | 9.00 |
| M013-S214-S197 | 24.02 | 5.64 | M007-5051-5085 | 0.00 | 8.98 |
| M019-S104-S157 | 0.00 | 10.28 | M031-5212-5042 | 0.00 | 8.97 |
| M049-S142-S043 | 16.10 | 6.18 | M012-S170-S135 | 0.00 | 8.94 |
| M012-S213-S080 | 0.00 | 10.27 | M024-S211-S141 | 0.00 | 8.91 |
| M007-S213-S213 | 34.94 | 5.09 | M008-S039-S158 | 0.00 | 8.89 |
| M007-S115-S189 | 1.92 | 8.70 | M012-5049-5072 | 0.00 | 8.86 |
| M049-S135-S142 | 7.42 | 7.17 | M007-S171-S175 | 0.00 | 8.85 |
| M002-S052-S050 | 20.15 | 5.84 | M024-S142-S053 | 0.00 | 8.80 |
| M013-S101-S157 | 1.82 | 8.72 | M042-5044-5075 | 0.00 | 8.77 |
| M031-S142-S051 | 6.29 | 7.34 | M036-S136-S104 | 0.00 | 8.77 |
| M042-5083-5037 | 5.91 | 7.38 | M001-S104-S142 | 0.00 | 8.76 |
| M024-S194-S053 | 0.00 | 10.16 | M036-S191-S135 | 0.00 | 8.75 |
| M002-S169-S042 | 14.27 | 6.22 | M043-S214-S043 | 0.00 | 8.73 |
| M010-S192-S136 | 16.88 | 5.99 | M037-S175-S077 | 0.00 | 8.70 |
| M013-S121-S039 | 9.20 | 6.75 | M013-S039-S169 | 0.00 | 8.66 |
| M002-S199-S042 | 16.48 | 5.97 | M007-S115-S046 | 0.00 | 8.65 |
| M001-S051-S142 | 20.08 | 5.70 | M001-S202-S085 | 0.00 | 8.63 |
| M030-S052-S121 | 27.94 | 5.19 | M049-S175-S202 | 0.00 | 8.58 |
| M025-S120-S039 | 4.61 | 7.55 | M030-S047-S142 | 0.00 | 8.56 |
| M013-S050-S135 | 28.40 | 5.13 | M019-S126-S116 | 0.00 | 8.55 |
| M010-S157-S050 | 2.61 | 8.14 | M019-S213-S058 | 0.00 | 8.55 |
| M042-S109-S116 | 2.48 | 8.18 | M002-S051-S037 | 0.00 | 8.52 |
| M018-S116-S176 | 23.75 | 5.35 | M019-S177-S116 | 0.01 | 8.51 |
| M037-S142-S043 | 7.79 | 6.84 | M030-S136-S104 | 1.03 | 8.49 |
| M037-S175-S186 | 2.73 | 8.07 | M002-S043-S109 | 0.00 | 8.49 |
| M009-S059-S069 | 0.00 | 9.97 | M025-S136-S198 | 0.00 | 8.48 |
| M001-S175-S048 | 3.93 | 7.65 | M037-5043-5216 | 0.00 | 8.48 |
| M013-S051-S197 | 4.74 | 7.42 | M018-S053-S169 | 0.00 | 8.47 |
| M025-S213-S215 | 22.18 | 5.41 | M049-S155-S104 | 0.00 | 8.46 |
| M049-S080-S046 | 2.12 | 8.30 | M007-S199-S135 | 0.00 | 8.46 |
| M007-S121-S050 | 1.94 | 8.37 | M018-S175-S058 | 0.00 | 8.45 |
| M019-S135-S216 | 27.12 | 5.11 | M048-S191-S142 | 0.00 | 8.45 |
| M031-S116-S052 | 14.89 | 5.92 | M007-S171-S051 | 0.00 | 8.44 |
| M043-S183-S038 | 1.31 | 8.70 | M037-S137-Z001 | 0.00 | 8.44 |
| M025-S080-S102 | 18.66 | 5.60 | M009-S183-S183 | 0.75 | 8.44 |
| M007-S158-S084 | 13.62 | 6.03 | M002-S143-S075 | 0.00 | 8.43 |
| M009-S042-S047 | 15.99 | 5.81 | M025-S036-S215 | 0.00 | 8.43 |
| M008-S050-S062 | 14.05 | 5.98 | M049-S183-S135 | 0.00 | 8.43 |
| M043-S195-S048 | 30.26 | 4.93 | M008-S080-S145 | 0.00 | 8.41 |
| M037-S044-S058 | 13.82 | 6.00 | M009-S052-S039 | 0.00 | 8.39 |
| M030-S212-S045 | 14.24 | 5.95 | M002-5212-5049 | 0.00 | 8.39 |
| M031-S053-S143 | 5.48 | 7.18 | M007-S137-S202 | 0.00 | 8.39 |
| M019-S168-S216 | 5.39 | 7.19 | M025-S037-S050 | 0.00 | 8.39 |
| M013-S047-S046 | 6.89 | 6.89 | M024-S189-S076 | 0.01 | 8.38 |
| M007-S042-S215 | 5.27 | 7.22 | M019-S083-S155 | 0.00 | 8.38 |
| M049-S143-S053 | 2.99 | 7.85 | M049-5214-5054 | 0.00 | 8.38 |
| M037-S076-S115 | 0.00 | 9.85 | M043-S126-S037 | 0.00 | 8.38 |
| M043-S081-S098 | 10.07 | 6.38 | M012-S054-S194 | 0.00 | 8.38 |
| M025-S142-S048 | 3.67 | 7.62 | M018-S038-S169 | 0.95 | 8.37 |

**Table 8. Library 2B Top Constructs.**

| BlockSequence | Norm_D7 | Enrich_D28 | BlockSequence | Norm_D7 | Enrich_D28 |
|---|---|---|---|---|---|
| M024-S190-S047 | 30.96 | 10.78 | M018-S199-S083 | 6.87 | 6.78 |
| M025-S050-S197 | 2.95 | 13.25 | M008-5074-5072 | 7.98 | 6.59 |
| M036-S170-S047 | 0.00 | 13.79 | M043-5053-5039 | 20.99 | 5.30 |
| M012-S045-S048 | 1.35 | 12.24 | M018-S115-S213 | 7.37 | 6.69 |
| M049-S194-S064 | 10.08 | 9.04 | M002-S069-S051 | 10.80 | 6.19 |
| M019-S161-S215 | 10.44 | 8.59 | M037-5046-5053 | 18.41 | 5.47 |
| M025-S190-S050 | 25.07 | 7.20 | M049-S138-S191 | 7.49 | 6.67 |
| M013-S141-S045 | 2.58 | 9.78 | M010-S103-S197 | 8.34 | 6.53 |
| M036-S104-S062 | 10.56 | 8.06 | M030-S198-S043 | 13.25 | 5.92 |
| M001-S099-S080 | 7.86 | 8.42 | M013-S215-S215 | 15.21 | 5.73 |
| M018-S053-S130 | 4.30 | 8.84 | M019-S130-S135 | 4.30 | 7.34 |
| M037-S081-S142 | 4.67 | 8.67 | M001-S190-S047 | 8.72 | 6.47 |
| M030-S170-S168 | 8.60 | 7.83 | M024-S176-S046 | 4.42 | 7.31 |
| M001-S174-S044 | 8.59 | 7.81 | M030-S064-S121 | 7.24 | 6.70 |
| M002-S190-S183 | 9.34 | 7.63 | M049-S064-S100 | 1.60 | 8.37 |
| M049-S105-S084 | 12.28 | 7.22 | M008-S176-S074 | 10.31 | 6.24 |
| M019-S046-S197 | 2.45 | 9.08 | M002-S051-S102 | 0.00 | 9.69 |
| M036-S085-S044 | 8.23 | 7.61 | M001-S063-S039 | 0.00 | 9.68 |
| M030-S059-S106 | 1.47 | 9.50 | M007-S050-S039 | 0.00 | 9.67 |
| M048-S157-S154 | 10.32 | 7.30 | M030-S141-S045 | 0.00 | 9.66 |
| M025-S177-S194 | 13.51 | 6.88 | M018-S197-S137 | 0.00 | 9.63 |
| M019-S214-S138 | 17.44 | 6.51 | M019-S098-S126 | 0.00 | 9.62 |
| M037-S120-S085 | 8.35 | 7.47 | M037-S141-S101 | 0.00 | 9.61 |
| M037-5043-5074 | 12.77 | 6.90 | M008-S202-S191 | 0.00 | 9.61 |
| M009-S213-S037 | 0.00 | 10.68 | M036-S144-S103 | 0.00 | 9.61 |
| M049-S192-S212 | 3.93 | 8.37 | M013-S143-S194 | 0.00 | 9.60 |
| M049-S098-S177 | 13.39 | 6.82 | M019-S039-S069 | 0.00 | 9.58 |
| M043-S069-S177 | 0.74 | 9.86 | M036-S126-S103 | 0.00 | 9.57 |
| M025-5072-5047 | 38.55 | 5.34 | M018-S190-S048 | 0.00 | 9.56 |
| M030-S043-S194 | 9.21 | 7.29 | M024-S072-S198 | 0.00 | 9.52 |
| M037-S083-S109 | 2.82 | 8.70 | M036-S175-S064 | 0.00 | 9.49 |
| M025-S154-S216 | 0.00 | 10.63 | M010-S130-S170 | 0.00 | 9.49 |
| M049-S138-S047 | 27.13 | 5.71 | M018-S130-S080 | 0.00 | 9.41 |
| M002-S103-S158 | 8.72 | 7.23 | M048-S051-S129 | 0.00 | 9.24 |
| M012-S050-S043 | 3.19 | 8.42 | M042-S109-S103 | 0.73 | 8.87 |
| M002-S036-S121 | 19.90 | 6.05 | M036-S174-S195 | 0.73 | 8.85 |
| M031-S083-S130 | 5.28 | 7.72 | M009-S101-S115 | 0.86 | 8.83 |
| M043-S186-S087 | 13.75 | 6.48 | M049-5042-5051 | 0.00 | 8.75 |
| M012-S216-S039 | 9.45 | 6.97 | M013-S190-S099 | 0.00 | 8.74 |
| M043-S214-S104 | 15.84 | 6.24 | M036-S156-S135 | 0.86 | 8.72 |
| M049-S076-S157 | 4.79 | 7.78 | M025-S050-S043 | 0.00 | 8.71 |
| M008-S186-S076 | 7.37 | 7.24 | M037-S042-S195 | 0.86 | 8.67 |
| M031-S038-S212 | 3.80 | 8.00 | M002-S174-S049 | 0.98 | 8.65 |
| M030-S130-S216 | 3.56 | 8.04 | M009-S049-S038 | 0.98 | 8.59 |
| M007-5049-5051 | 0.00 | 10.22 | M010-S190-S050 | 0.00 | 8.58 |
| M042-S082-S158 | 12.40 | 6.47 | M018-5053-5156 | 1.23 | 8.57 |
| M037-S194-S194 | 10.07 | 6.75 | M024-S084-S157 | 1.23 | 8.56 |
| M031-S135-S183 | 6.75 | 7.23 | M007-S189-S069 | 0.86 | 8.55 |
| M043-S156-S099 | 5.77 | 7.41 | M030-S197-S130 | 1.10 | 8.53 |
| M012-S186-S169 | 3.81 | 7.89 | M018-5130-5059 | 0.00 | 8.51 |
| M048-5038-5076 | 10.55 | 6.59 | M042-S155-S195 | 1.47 | 8.43 |
| M042-S059-S106 | 8.34 | 6.82 | M010-S052-S038 | 1.10 | 8.42 |
| M037-S074-S175 | 1.47 | 8.69 | M042-S087-S142 | 1.35 | 8.42 |
| M012-S121-S106 | 14.24 | 6.05 | M018-5062-5105 | 0.00 | 8.39 |
| M013-S075-S212 | 17.18 | 5.74 | M010-S099-S202 | 1.47 | 8.30 |
| M013-S193-S168 | 5.16 | 7.28 | M030-S059-S213 | 1.35 | 8.26 |
| M002-S142-S072 | 36.59 | 4.65 | M019-S176-S084 | 1.35 | 8.22 |
| M001-S199-S102 | 5.77 | 7.11 | M037-S058-S194 | 1.84 | 8.21 |
| M010-S062-S192 | 14.00 | 5.95 | M002-S171-S080 | 1.84 | 8.21 |
| M031-S075-S169 | 11.66 | 6.19 | M002-S130-S196 | 1.84 | 8.20 |
| M049-S216-S193 | 10.55 | 6.31 | M036-S183-S136 | 1.72 | 8.19 |
| M030-S130-S064 | 5.27 | 7.19 | M048-S106-S053 | 1.59 | 8.18 |
| M031-S1S8-S046 | 7.24 | 6.79 | M036-5045-5086 | 2.09 | 8.08 |
| M049-S193-S044 | 16.94 | 5.67 | M049-S129-S044 | 1.72 | 8.08 |
| M043-S102-S080 | 4.91 | 7.25 | M008-S146-S154 | 1.96 | 8.03 |
| M042-S191-S047 | 1.47 | 8.51 | M030-S051-S199 | 1.84 | 7.95 |
| M008-S156-S161 | 19.26 | 5.47 | M043-S051-S211 | 2.33 | 7.92 |
| M007-S050-S110 | 5.52 | 7.10 | M024-S190-S171 | 2.33 | 7.88 |
| M031-S109-S192 | 5.27 | 7.15 | M010-S177-S196 | 1.35 | 7.88 |
| M024-S064-S168 | 14.48 | 5.83 | M049-S050-S212 | 2.45 | 7.87 |
| M018-S215-S213 | 20.62 | 5.35 | M008-S143-S050 | 2.33 | 7.86 |
| M025-5084-5039 | 8.47 | 6.54 | M002-S039-S104 | 1.72 | 7.84 |
| M042-S101-S171 | 5.89 | 6.99 | M019-S138-S075 | 2.70 | 7.82 |
| M013-S148-S085 | 14.36 | 5.83 | M019-S116-S177 | 2.33 | 7.82 |
| M031-S054-S138 | 10.18 | 6.29 | M013-S076-S197 | 1.59 | 7.80 |
| M030-S161-S213 | 6.99 | 6.77 | M024-5042-5216 | 2.57 | 7.80 |
| M042-S180-S042 | 10.43 | 6.26 | M007-S202-S102 | 2.33 | 7.78 |
| M024-S049-S189 | 1.23 | 8.61 | M002-S043-S104 | 2.94 | 7.76 |
| M025-S121-S052 | 5.27 | 7.12 | M030-S215-S064 | 2.58 | 7.75 |
| M012-S072-Z001 | 5.15 | 7.14 | M001-S098-S126 | 1.84 | 7.74 |
| M048-S104-S180 | 12.27 | 6.03 | M013-S085-S046 | 2.70 | 7.73 |
| M048-5045-5045 | 0.00 | 9.76 | M043-S046-S189 | 2.82 | 7.73 |
| M031-S189-S050 | 7.61 | 6.66 | M009-S059-S050 | 2.70 | 7.70 |
| M037-S109-S135 | 2.70 | 7.87 | | | |

**Table 9. Library 1.1A Top Constructs.**

| BlockSequence | Norm_D7 | Enrich_D42 | BlockSequence | Norm_D7 | Enrich_D42 |
|---|---|---|---|---|---|
| M007-S120-S214 | 0.00 | 16.52 | M030-S054-S169 | 0.97 | 10.42 |
| M001-S117-S105 | 2.25 | 15.48 | M012-S117-S142 | 4.31 | 10.40 |
| M024-S062-S137 | 0.77 | 14.07 | M042-S038-S053 | 1.54 | 10.24 |
| M012-S117-S194 | 4.25 | 14.05 | M001-S186-S084 | 3.02 | 10.17 |
| M012-S117-S126 | 0.00 | 13.58 | M024-S130-S171 | 0.00 | 10.05 |
| M001-S117-S169 | 0.84 | 13.18 | M042-S190-S042 | 3.41 | 9.93 |
| M012-S062-S046 | 2.45 | 12.77 | M036-S143-S194 | 0.39 | 9.92 |
| M012-S117-S058 | 2.96 | 12.75 | M030-S168-S137 | 7.34 | 9.81 |
| M048-5054-5076 | 5.15 | 12.73 | M012-S062-S136 | 1.29 | 9.77 |
| M036-S080-S195 | 3.09 | 12.44 | M030-S084-S143 | 7.53 | 9.60 |
| M042-S120-S199 | 3.09 | 12.29 | M012-S054-S169 | 2.70 | 9.51 |
| M030-S080-S197 | 0.00 | 12.21 | M012-S062-S170 | 14.22 | 9.34 |
| M001-S155-S048 | 1.29 | 11.96 | M030-S062-S143 | 0.45 | 9.34 |
| M012-S171-S074 | 1.09 | 11.86 | M030-S154-S194 | 3.09 | 9.28 |
| M030-S117-S049 | 5.21 | 11.74 | M012-S064-S063 | 3.35 | 9.25 |
| M030-S170-S080 | 6.18 | 11.63 | M030-S170-S194 | 7.27 | 9.19 |
| M030-S136-S101 | 4.38 | 11.59 | M030-S170-S168 | 3.67 | 9.14 |
| M024-S130-S063 | 8.30 | 11.51 | M012-S106-S169 | 1.35 | 9.10 |
| M024-S211-S154 | 4.83 | 11.45 | M001-S117-S052 | 2.64 | 9.10 |
| M012-S062-S138 | 0.90 | 11.06 | M030-S198-S169 | 1.03 | 9.04 |
| M030-S102-S191 | 1.42 | 10.86 | M030-S214-S050 | 2.32 | 9.02 |
| M036-S197-S076 | 0.00 | 10.82 | M036-S199-S038 | 1.61 | 9.01 |
| M030-S190-S047 | 0.00 | 10.75 | M018-S044-S177 | 4.38 | 8.85 |
| M012-S062-S169 | 11.00 | 10.59 | M001-S190-S042 | 0.00 | 8.79 |
| M012-S037-S157 | 0.90 | 10.52 | M036-S175-S059 | 10.04 | 8.78 |

**Table 10. Library 1.1B Top Constructs.**

| BlockSequence | Norm_D7 | Enrich_D28 | BlockSequence | Norm_D7 | Enrich_D28 |
|---|---|---|---|---|---|
| M001-S126-S170 | 2.57 | 7.68 | M042-S141-S120 | 0.84 | 6.17 |
| M012-S106-S169 | 1.35 | 7.65 | M042-S130-S177 | 0.84 | 6.17 |
| M007-S194-S100 | 0.00 | 7.19 | M036-S176-S116 | 0.58 | 6.16 |
| M036-S101-S183 | 0.00 | 7.11 | M007-S109-S212 | 0.64 | 6.14 |
| M024-S135-S098 | 0.00 | 7.08 | M010-S195-S100 | 0.84 | 6.10 |
| M024-S038-S098 | 0.00 | 7.04 | M012-S168-S175 | 0.00 | 6.07 |
| M007-S109-S135 | 0.00 | 7.02 | M018-5102-5117 | 0.00 | 6.01 |
| M024-S191-S038 | 0.00 | 6.94 | M018-5211-5083 | 0.00 | 6.00 |
| M012-S064-S116 | 0.00 | 6.93 | M018-S192-S058 | 0.00 | 6.00 |
| M009-S100-S074 | 0.00 | 6.84 | M042-S143-S168 | 0.00 | 5.95 |
| M001-S120-S039 | 0.00 | 6.84 | M012-S186-S120 | 0.00 | 5.95 |
| M018-S154-S085 | 0.00 | 6.83 | M030-S087-S052 | 0.00 | 5.95 |
| M030-S104-S048 | 0.00 | 6.79 | M008-S186-S085 | 0.00 | 5.94 |
| M030-S168-S121 | 0.00 | 6.78 | M042-S202-S050 | 0.00 | 5.93 |
| M042-S120-S171 | 1.16 | 6.75 | M001-S171-S214 | 0.00 | 5.91 |
| M036-S176-S195 | 0.90 | 6.62 | M001-S138-S037 | 0.00 | 5.91 |
| M010-S171-S169 | 0.00 | 6.53 | M012-S143-S176 | 0.00 | 5.91 |
| M030-S175-S120 | 1.48 | 6.52 | M042-S186-S050 | 0.00 | 5.91 |
| M048-S051-S115 | 0.84 | 6.52 | M012-S192-S076 | 3.09 | 5.91 |
| M024-S047-S080 | 0.00 | 6.50 | M048-S098-S074 | 0.00 | 5.90 |
| M009-S199-S141 | 0.77 | 6.39 | M042-S186-S215 | 0.00 | 5.90 |
| M018-S136-S183 | 0.39 | 6.34 | M048-S050-S211 | 0.00 | 5.90 |
| M036-S062-S212 | 1.03 | 6.30 | M010-S074-S169 | 1.35 | 5.88 |
| M012-S211-S137 | 0.71 | 6.21 | M008-S212-S099 | 3.22 | 5.88 |
| M010-S064-S171 | 0.71 | 6.19 | M001-S074-S197 | 0.00 | 5.88 |

**Table 11. Library 2.1B Top Constructs.**

| BlockSequence | Norm_D7 | Enich_D28 | BlockSequence | Norm_D7 | Enich_D28 |
|---|---|---|---|---|---|
| M042-S186-S044 | 0.78 | 8.65 | M010-S177-S215 | 1.63 | 5.85 |
| M048-S100-S045 | 0.98 | 8.51 | M024-S193-S193 | 0.33 | 5.83 |
| M001-S186-S170 | 2.21 | 8.24 | M012-S049-S192 | 0.20 | 5.80 |
| M024-S039-S052 | 6.24 | 7.53 | M048-S130-S170 | 0.39 | 5.80 |
| M001-S214-S058 | 0.26 | 7.27 | M018-S175-S171 | 1.56 | 5.80 |
| M042-S080-S074 | 2.02 | 7.04 | M030-S051-S080 | 2.28 | 5.79 |
| M030-S170-S115 | 2.60 | 6.96 | M012-S121-S072 | 0.13 | 5.76 |
| M009-S083-S138 | 1.04 | 6.44 | M009-S183-S161 | 1.50 | 5.75 |
| M042-S154-S075 | 2.73 | 6.44 | M007-5077-5059 | 0.33 | 5.75 |
| M030-S214-S143 | 4.49 | 6.33 | M018-S058-S062 | 1.76 | 5.73 |
| M012-S213-S211 | 1.63 | 6.23 | M008-S170-S168 | 0.33 | 5.73 |
| M036-S212-S214 | 0.78 | 6.17 | M018-S050-S043 | 0.33 | 5.73 |
| M030-S036-S106 | 2.86 | 6.05 | M012-S215-S126 | 1.17 | 5.69 |
| M001-S169-S083 | 1.30 | 6.04 | M024-5083-5047 | 3.58 | 5.68 |
| M007-S105-S064 | 0.13 | 5.95 | M048-S052-S084 | 0.52 | 5.68 |
| M009-S039-S183 | 1.56 | 5.95 | M042-S052-S130 | 1.56 | 5.67 |
| M009-S074-S050 | 1.63 | 5.94 | M036-S064-S047 | 1.95 | 5.65 |
| M012-S076-S051 | 1.43 | 5.94 | M008-S148-S137 | 0.52 | 5.65 |
| M010-S072-S186 | 1.30 | 5.92 | M036-S198-S135 | 0.52 | 5.64 |
| M008-S142-S075 | 0.13 | 5.91 | M018-S043-S051 | 1.11 | 5.64 |
| M001-S175-S168 | 0.33 | 5.87 | M042-S105-S104 | 0.46 | 5.62 |
| M036-S170-S175 | 0.13 | 5.87 | M030-S074-S082 | 0.39 | 5.61 |
| M018-S194-S116 | 1.43 | 5.87 | M036-S083-S130 | 0.52 | 5.59 |
| M042-S115-S121 | 0.33 | 5.85 | M001-S137-S157 | 0.46 | 5.58 |
| M018-5043-5086 | 0.00 | 5.85 | M008-S083-S104 | 0.72 | 5.57 |

**Table 12. Library 3A Top Constructs.**

| BlockSequence | Norm_D7 | Enrich_D35 | BlockSequence | Norm_D7 | Enrich_D35 |
|---|---|---|---|---|---|
| E013-T047-S158-S080 | 0.00 | 17.08 | E014-T054-S186-S053 | 0.00 | 8.77 |
| E011-T024-S194-S039 | 0.00 | 16.99 | E012-T062-S186-S047 | 0.92 | 8.74 |
| E014-T040-S135-S076 | 0.61 | 15.47 | E012-T066-S197-S076 | 1.34 | 8.72 |
| E013-T041-S186-S051 | 2.54 | 15.08 | E012-T054-S102-S214 | 1.18 | 8.71 |
| E013-T064-S058-S212 | 3.91 | 13.86 | E013-T010-S106-S076 | 1.03 | 8.68 |
| E013-T028-S186-S051 | 12.36 | 12.80 | E011-T046-S186-S050 | 2.26 | 8.65 |
| E014-T015-S186-S051 | 0.00 | 12.78 | E014-T071-S100-S049 | 3.43 | 8.63 |
| E011-T016-S186-S050 | 2.31 | 11.97 | E014-T045-S186-S051 | 2.26 | 8.62 |
| E011-T073-S186-S050 | 0.00 | 11.96 | E013-T032-S145-X002 | 0.00 | 8.61 |
| E013-T011-S186-S211 | 1.75 | 11.90 | E013-T049-5149-5037 | 0.00 | 8.60 |
| E012-T011-S186-S047 | 3.85 | 11.85 | E012-T046-S186-S050 | 7.48 | 8.60 |
| E012-T020-S117-S212 | 0.54 | 11.33 | E013-T061-S121-S074 | 1.50 | 8.59 |
| E012-T017-S186-S051 | 0.00 | 11.31 | E014-T017-S198-S211 | 0.94 | 8.57 |
| E012-T028-S186-S039 | 0.00 | 11.22 | E014-T059-S059-S050 | 1.08 | 8.57 |
| E014-T034-S069-S053 | 0.00 | 11.19 | E012-T077-S196-S080 | 0.00 | 8.56 |
| E012-T059-S175-S213 | 3.20 | 11.05 | E014-T005-S137-S214 | 1.09 | 8.54 |
| E014-T035-S154-S076 | 2.56 | 11.04 | E013-T052-S148-S050 | 2.08 | 8.53 |
| E013-T045-S169-S215 | 4.85 | 11.02 | E011-T051-S083-S076 | 0.00 | 8.52 |
| E014-T031-S186-S211 | 2.18 | 10.86 | E014-T072-S085-S213 | 1.13 | 8.52 |
| E012-T063-S191-S052 | 0.00 | 10.75 | E013-T006-S177-S075 | 1.63 | 8.51 |
| E011-T064-S083-S048 | 2.15 | 10.67 | E012-T050-S099-S214 | 0.00 | 8.51 |
| E013-T055-S099-S215 | 0.00 | 10.65 | E014-T056-S058-S216 | 1.10 | 8.50 |
| E014-T029-S154-S047 | 0.00 | 10.64 | E013-T045-S186-S053 | 4.11 | 8.50 |
| E013-T050-5186-5047 | 0.00 | 10.62 | E014-T045-S186-S053 | 3.15 | 8.50 |
| E013-T017-S106-S080 | 1.75 | 10.54 | E013-T021-S186-S211 | 0.00 | 8.49 |
| E012-T049-S197-S053 | 1.12 | 10.44 | E013-T073-S054-S075 | 0.00 | 8.49 |
| E011-T011-S186-S211 | 5.44 | 10.40 | E014-T072-S101-X002 | 3.23 | 8.49 |
| E014-T041-S186-S053 | 3.26 | 10.35 | E013-T036-S102-S053 | 0.00 | 8.48 |
| E011-T007-S154-S216 | 0.00 | 10.33 | E011-T047-S145-S052 | 0.00 | 8.46 |
| E013-T041-S186-S050 | 11.95 | 10.28 | E012-T006-S186-S039 | 0.00 | 8.45 |
| E012-T072-S189-S212 | 0.92 | 10.18 | E012-T031-S186-S050 | 0.95 | 8.41 |
| E011-T077-S186-S211 | 5.16 | 10.13 | E014-T060-S 109-5076 | 2.22 | 8.41 |
| E013-T045-S143-S038 | 0.00 | 10.11 | E011-T005-S098-S074 | 1.12 | 8.39 |
| E014-T041-S186-X002 | 3.72 | 10.09 | E011-T017-S186-X002 | 1.05 | 8.38 |
| E014-T057-S186-S050 | 3.07 | 10.09 | E011-T043-S054-S049 | 1.04 | 8.37 |
| E013-T068-S170-S050 | 1.45 | 9.91 | E014-T080-S193-S050 | 1.01 | 8.35 |
| E014-T055-5186-5053 | 0.00 | 9.90 | E014-T007-5190-5053 | 2.44 | 8.33 |
| E014-T028-S186-S051 | 2.10 | 9.86 | E013-T023-S105-X002 | 1.09 | 8.29 |
| E013-T032-S136-S050 | 0.00 | 9.83 | E013-T057-S186-S051 | 6.29 | 8.16 |
| E012-T064-S135-S080 | 1.11 | 9.80 | E014-T031-S186-S053 | 11.27 | 6.86 |
| E014-T050-S186-S050 | 1.53 | 9.80 | E012-T062-S186-S211 | 3.99 | 8.15 |
| E011-T001-S177-S074 | 6.99 | 9.74 | E012-T006-5186-5047 | 3.85 | 8.07 |
| E013-T040-S171-S038 | 2.08 | 9.73 | E014-T028-S186-S053 | 3.49 | 8.13 |
| E013-T034-S186-S050 | 0.00 | 9.72 | E014-T041-S186-S211 | 5.72 | 7.54 |
| E013-T055-5104-5052 | 0.00 | 9.50 | E011-T047-S085-S211 | 3.66 | 8.06 |
| E014-T051-S058-S211 | 3.87 | 9.48 | E014-T038-S186-X002 | 6.13 | 7.42 |
| E013-T012-S194-X002 | 0.79 | 9.42 | E013-T028-S186-S076 | 8.76 | 6.60 |
| E013-T019-S186-S050 | 3.33 | 9.42 | E011-T045-S186-S051 | 2.25 | 8.16 |
| E012-T071-S069-S075 | 0.00 | 9.42 | E011-T022-S186-S050 | 1.93 | 8.29 |
| E011-T026-S186-S051 | 1.06 | 9.34 | E012-T004-S202-S211 | 1.91 | 8.26 |
| E013-T050-S186-S050 | 0.00 | 9.29 | E011-T022-S194-S074 | 2.04 | 8.17 |
| E012-T024-S186-S051 | 0.90 | 9.27 | E011-T011-S186-S047 | 2.19 | 8.10 |
| E012-T073-S186-S053 | 0.00 | 9.12 | E012-T031-S186-S052 | 3.62 | 7.55 |
| E013-T071-S115-S213 | 1.04 | 9.09 | E013-T030-S138-S216 | 1.95 | 8.20 |
| E014-T062-S186-S211 | 2.15 | 8.99 | E013-T016-S186-S050 | 2.98 | 7.74 |
| E011-T062-S186-S211 | 0.00 | 8.92 | E013-T034-S186-S053 | 3.51 | 7.54 |
| E014-T011-S186-S039 | 0.46 | 8.86 | E011-T012-S193-S052 | 4.66 | 7.19 |
| E013-T069-S109-S074 | 0.00 | 8.84 | E012-T047-5186-5047 | 3.51 | 7.43 |
| E011-T058-S156-X002 | 0.92 | 8.83 | E011-T055-5186-5053 | 8.63 | 6.29 |
| E014-T021-S171-S053 | 0.00 | 8.83 | E013-T077-S186-S051 | 2.48 | 7.73 |
| E014-T073-S161-S050 | 0.00 | 8.81 | E014-T041-S186-S052 | 7.19 | 6.47 |
| E011-T011-S186-S051 | 1.71 | 8.79 | E013-T029-S186-S051 | 5.53 | 6.76 |

**Table 13. Library 3B Top Constructs.**

| BlockSequence | Norm_D7 | Enrich_D21 | BlockSequence | Norm_D7 | Enrich_D21 |
|---|---|---|---|---|---|
| E014-T011-S186-S039 | 0.46 | 9.67 | E011-T007-S064-S075 | 0.00 | 6.58 |
| E013-T057-S115-S214 | 0.00 | 9.40 | E013-T059-S136-S050 | 1.83 | 6.58 |
| E013-T028-S186-S051 | 12.36 | 9.28 | E013-T034-S186-S053 | 3.51 | 6.56 |
| E013-T019-S142-S212 | 0.00 | 9.04 | E014-T022-S087-S075 | 0.00 | 6.55 |
| E011-T020-S104-S080 | 0.99 | 8.92 | E012-T055-S142-X002 | 0.03 | 6.55 |
| E011-T011-S186-S039 | 0.00 | 8.75 | E011-T067-S186-S051 | 1.79 | 6.53 |
| E013-T031-S169-S214 | 0.00 | 8.74 | E012-T073-S083-S037 | 4.04 | 6.48 |
| E013-T062-S158-S051 | 1.30 | 8.51 | E011-T028-S171-S038 | 1.19 | 6.47 |
| E011-T041-S186-S047 | 2.62 | 8.43 | E012-T016-S186-S039 | 0.00 | 6.45 |
| E012-T024-S142-S211 | 1.17 | 8.31 | E012-T047-S104-S053 | 1.60 | 6.44 |
| E013-T017-S143-S216 | 0.00 | 8.26 | E014-T016-S186-S039 | 2.60 | 6.37 |
| E013-T058-S183-S051 | 1.00 | 8.19 | E013-T042-S191-S074 | 1.11 | 6.37 |
| E011-T030-S176-S050 | 0.60 | 8.16 | E014-T017-S186-S053 | 0.96 | 6.33 |
| E012-T062-S186-S037 | 3.05 | 8.10 | E013-T048-S154-S038 | 0.10 | 6.33 |
| E014-T041-S085-S037 | 0.91 | 8.02 | E013-T045-S186-S053 | 4.11 | 6.32 |
| E011-T080-S115-S211 | 0.00 | 7.86 | E013-T016-S186-S050 | 2.98 | 6.27 |
| E013-T041-S186-S039 | 1.07 | 7.81 | E013-T062-S186-S051 | 1.11 | 6.23 |
| E014-T028-S186-S053 | 3.49 | 7.78 | E011-T001-S177-S074 | 6.99 | 6.23 |
| E014-T019-5057-5074 | 1.16 | 7.76 | E012-T027-S116-S215 | 0.00 | 6.22 |
| E012-T044-S130-S074 | 1.09 | 7.75 | E011-T046-S186-S052 | 0.00 | 6.21 |
| E013-T081-S085-S211 | 3.54 | 7.74 | E013-T031-S186-S047 | 3.33 | 6.10 |
| E014-T028-S186-S051 | 2.10 | 7.68 | E014-T012-S121-S213 | 1.72 | 6.09 |
| E012-T027-S083-S080 | 0.00 | 7.60 | E012-T033-S130-X002 | 0.00 | 6.09 |
| E013-T052-S126-S213 | 0.00 | 7.58 | E014-T055-S109-S211 | 0.67 | 6.06 |
| E012-T039-S176-S213 | 4.06 | 7.53 | E013-T014-S197-S214 | 0.00 | 6.02 |
| E014-T072-S101-X002 | 3.23 | 7.45 | E014-T041-S186-S047 | 0.00 | 6.02 |
| E012-T011-S186-S047 | 3.85 | 7.43 | E012-T021-S186-S051 | 1.47 | 6.01 |
| E014-T041-S186-S053 | 3.26 | 7.41 | E014-T073-S059-S048 | 0.93 | 5.99 |
| E013-T002-S135-S038 | 0.00 | 7.40 | E013-T050-5186-5047 | 0.00 | 5.98 |
| E011-T015-S170-S047 | 4.71 | 7.39 | E013-T022-S189-S215 | 1.69 | 5.97 |
| E014-T072-S198-S076 | 2.28 | 7.35 | E014-T042-S177-X002 | 6.13 | 5.95 |
| E014-T028-S186-S039 | 1.44 | 7.31 | E014-T076-S186-S050 | 8.73 | 5.95 |
| E013-T027-S186-S051 | 3.03 | 7.30 | E012-T011-S186-S216 | 0.60 | 5.93 |
| E014-T011-S186-X002 | 4.22 | 7.29 | E012-T034-S186-S053 | 3.99 | 5.92 |
| E014-T015-S186-S051 | 0.00 | 7.25 | E014-T031-S186-S053 | 11.27 | 5.70 |
| E012-T010-S063-S039 | 3.22 | 7.23 | E013-T020-S083-X002 | 7.87 | 5.69 |
| E011-T011-S186-S053 | 8.27 | 7.23 | E012-T072-S176-S211 | 6.58 | 5.74 |
| E012-T067-S072-S075 | 0.00 | 7.21 | E014-T062-S186-X002 | 11.85 | 4.83 |
| E012-T043-S186-S051 | 1.16 | 7.21 | E013-T011-S186-S039 | 5.19 | 5.75 |
| E012-T030-S059-X002 | 2.08 | 7.15 | E013-T021-5069-5216 | 4.36 | 5.82 |
| E011-T011-S186-S051 | 1.71 | 7.14 | E012-T077-S186-S052 | 5.77 | 5.42 |
| E012-T059-S175-S213 | 3.20 | 7.08 | E012-T077-S195-S080 | 4.48 | 5.64 |
| E011-T038-S116-S039 | 2.16 | 7.07 | E012-T043-S186-S053 | 8.59 | 4.72 |
| E012-T028-S186-S039 | 0.00 | 7.03 | E012-T011-S186-S052 | 4.03 | 5.63 |
| E012-T024-S186-S053 | 4.74 | 6.98 | E012-T016-S186-S075 | 7.32 | 4.87 |
| E012-T033-S157-S053 | 0.00 | 6.96 | E012-T043-S186-X002 | 3.44 | 5.73 |
| E013-T071-S072-S213 | 0.00 | 6.95 | E014-T071-S121-X002 | 3.30 | 5.77 |
| E012-T050-S099-S214 | 0.00 | 6.94 | E014-T028-S186-X002 | 5.96 | 5.03 |
| E011-T062-S186-S053 | 0.00 | 6.90 | E014-T062-S186-S051 | 4.96 | 5.24 |
| E013-T044-S143-S050 | 1.31 | 6.85 | E012-T021-S186-S053 | 17.53 | 3.59 |
| E014-T008-S186-S053 | 1.59 | 6.82 | E014-T062-S102-S215 | 5.11 | 5.13 |
| E014-T065-S142-S050 | 2.28 | 6.79 | E014-T011-S186-S053 | 10.09 | 4.26 |
| E013-T055-S195-S076 | 0.00 | 6.78 | E012-T062-S186-S076 | 3.01 | 5.70 |
| E013-T028-S186-S047 | 0.52 | 6.77 | E013-T028-S186-S076 | 8.76 | 4.42 |
| E014-I077-S130-S038 | 0.00 | 6.74 | E013-T062-S186-S075 | 2.89 | 5.73 |
| E014-T016-S186-S051 | 1.17 | 6.73 | E011-T030-S082-X002 | 8.98 | 4.37 |
| E013-T039-S186-S214 | 1.55 | 6.72 | E013-T019-S186-S050 | 3.33 | 5.51 |
| E012-T012-S103-S213 | 0.00 | 6.69 | E013-T011-S186-S037 | 3.85 | 5.33 |
| E013-T032-S142-S074 | 1.03 | 6.67 | E012-T002-S136-S080 | 6.27 | 4.74 |
| E013-T057-S186-S051 | 6.29 | 6.66 | E013-I062-S186-S047 | 2.87 | 5.61 |
| E012-T078-S183-S212 | 0.00 | 6.65 | E012-T034-S186-S051 | 6.25 | 4.70 |
| E013-T046-S135-S037 | 0.00 | 6.64 | E012-T046-S186-S050 | 7.48 | 4.37 |
| E013-T008-S101-S080 | 1.08 | 6.62 | E011-T067-S165-S038 | 6.40 | 4.56 |
| E012-T044-S105-S214 | 1.16 | 6.61 | E012-T031-S186-X002 | 4.42 | 4.95 |
| E014-T001-S186-S053 | 0.00 | 6.59 | E014-T019-S170-X002 | 3.05 | 5.35 |
| E013-T028-S186-S050 | 0.00 | 6.58 | | | |

**Table 14. Library 3.1A Top Constructs.**

| BlockSequence | Norm_D7 | Enrich_D35 | BlockSequence | Norm_D7 | Enrich_D35 |
|---|---|---|---|---|---|
| E006-T074-S194-S211 | 0.00 | 15.37 | E007-T065-S106-S053 | 1.23 | 11.32 |
| E010-T073-S186-S211 | 3.27 | 14.82 | E008-T001-5190-5053 | 0.00 | 11.30 |
| E009-T049-S106-S037 | 0.00 | 13.34 | E007-T032-S138-S052 | 0.00 | 11.24 |
| E009-T073-S190-S215 | 2.10 | 13.00 | E010-T044-S190-S080 | 2.28 | 11.20 |
| E009-T009-S165-S052 | 0.80 | 12.98 | E009-T044-5129-5053 | 1.23 | 11.18 |
| E009-T066-S168-S0S3 | 2.10 | 12.85 | E008-T073-S186-S080 | 0.00 | 11.17 |
| E010-T024-S197-S214 | 0.00 | 12.59 | E007-T020-S082-S211 | 0.00 | 11.16 |
| E009-T072-S186-S039 | 0.00 | 12.42 | E007-T006-S195-S052 | 0.00 | 11.11 |
| E009-T038-S105-S050 | 0.00 | 12.38 | E008-T027-5190-5050 | 1.61 | 11.08 |
| E006-T057-S105-S039 | 0.00 | 12.38 | E007-T056-S143-S050 | 0.68 | 10.99 |
| E007-T017-S197-S047 | 1.79 | 12.25 | E010-T042-S177-S049 | 1.85 | 10.96 |
| E010-T045-S138-S074 | 0.00 | 12.20 | E008-T034-5064-5039 | 0.00 | 10.95 |
| E008-T024-S197-S039 | 0.00 | 12.16 | E007-T040-S054-S052 | 1.85 | 10.83 |
| E006-T077-S202-S0S3 | 0.00 | 12.09 | E010-T019-S054-S051 | 1.48 | 10.80 |
| E009-I032-S105-S048 | 1.85 | 12.05 | E007-T058-S086-S052 | 1.48 | 10.78 |
| E008-T056-S197-S050 | 0.00 | 12.02 | E009-T019-S192-S214 | 0.86 | 10.69 |
| E006-T008-S170-S075 | 1.36 | 12.01 | E010-T066-S192-S037 | 1.91 | 10.67 |
| E010-T017-S084-S052 | 0.00 | 11.96 | E006-T048-5194-5074 | 0.00 | 10.57 |
| E009-T032-S129-S047 | 0.00 | 11.69 | E006-T078-S102-S075 | 1.05 | 10.55 |
| E010-T053-S194-S211 | 1.30 | 11.69 | E010-T017-5197-5053 | 1.17 | 10.55 |
| E008-T010-S064-S037 | 0.00 | 11.68 | E010-T041-S169-S074 | 0.00 | 10.50 |
| E009-T060-S175-S053 | 0.00 | 11.50 | E006-T038-S192-S0S3 | 1.79 | 10.47 |
| E008-T073-S169-X002 | 0.00 | 11.39 | E007-T032-S129-S212 | 0.49 | 10.46 |
| E009-T071-S186-S211 | 4.08 | 11.38 | E007-T035-S189-S051 | 4.08 | 10.45 |
| E006-T077-S069-S050 | 4.94 | 11.35 | E006-T006-S116-S214 | 0.00 | 10.44 |

**Table 15. Library 3.1B Top Constructs.**

| BlockSequence | Norm_D7 | Enrich_D35 | BlockSequence | Norm_D7 | Enrich_D35 |
|---|---|---|---|---|---|
| E010-T044-S171-S050 | 0.00 | 15.69 | E006-T034-5192-5049 | 0.99 | 9.11 |
| E007-T061-S169-S211 | 0.00 | 15.11 | E010-T040-S102-S051 | 0.00 | 9.11 |
| E010-T072-S183-S212 | 3.89 | 14.56 | E009-T041-5129-5049 | 0.93 | 9.03 |
| E008-T013-S192-S037 | 5.19 | 14.19 | E008-T003-S176-S215 | 0.00 | 8.92 |
| E008-T058-S176-S212 | 6.11 | 13.64 | E006-T055-S105-S214 | 0.00 | 8.87 |
| E006-T045-S130-S039 | 3.64 | 13.62 | E007-T039-S058-S211 | 0.00 | 8.79 |
| E008-T006-S169-X002 | 5.93 | 13.43 | E010-T020-S104-X002 | 1.54 | 8.67 |
| E008-T041-S141-S053 | 6.54 | 13.29 | E010-T030-5197-5047 | 0.00 | 8.65 |
| E007-T024-S115-S074 | 8.40 | 12.97 | E007-T038-S099-X002 | 0.00 | 8.62 |
| E009-T021-S138-S037 | 7.16 | 12.91 | E010-T024-S197-S214 | 0.00 | 8.59 |
| E009-T044-S197-S051 | 7.78 | 11.62 | E008-T041-S058-S216 | 0.00 | 8.59 |
| E009-T019-S192-S214 | 0.86 | 11.28 | E010-T013-S130-S080 | 1.11 | 8.56 |
| E008-T065-S101-X002 | 1.42 | 10.48 | E008-T079-5168-5038 | 0.00 | 8.48 |
| E008-T082-S102-S048 | 0.00 | 10.15 | E008-T021-S126-S0S3 | 0.00 | 8.39 |
| E010-T072-S186-S211 | 2.59 | 10.14 | E010-T045-S102-S213 | 1.17 | 8.38 |
| E007-T006-S141-S050 | 0.00 | 10.08 | E009-T076-S186-S211 | 0.68 | 8.36 |
| E009-T073-5064-5037 | 0.00 | 9.96 | E009-T005-5196-5049 | 1.54 | 8.33 |
| E010-T072-S197-S051 | 0.00 | 9.80 | E008-T020-S100-S053 | 0.00 | 8.25 |
| E007-T054-S194-S214 | 0.00 | 9.72 | E009-T075-S102-S039 | 9.88 | 8.23 |
| E010-T044-S137-S052 | 0.00 | 9.51 | E008-T006-S064-S051 | 0.00 | 8.20 |
| E010-T078-S197-S051 | 0.31 | 9.40 | E007-T046-5154-5075 | 0.00 | 8.18 |
| E007-T003-S120-S075 | 0.00 | 9.24 | E008-T071-5138-5047 | 0.00 | 8.17 |
| E010-T030-S197-S037 | 0.00 | 9.22 | E006-T008-5170-5075 | 1.36 | 8.15 |
| E007-T017-S058-S211 | 0.74 | 9.20 | E007-T073-5154-5048 | 0.00 | 8.13 |

**Table 16. Library 4B Top Constructs.**

| BlockSequence | Norm_D7 | Enrich_D21 | BlockSequence | Norm_D7 | Enrich_D21 |
|---|---|---|---|---|---|
| E014-T046-S186- | 0.000 | 11.939 | E013-T062-S058- | 6.678 | 7.463 |
| E014-T041-S058- | 0.000 | 10.739 | E013-T008-S072- | 2.701 | 7.451 |
| E012-T067-S142- | 0.000 | 10.684 | E012-T030-S130- | 0.954 | 7.443 |
| E013-T053-S101- | 0.000 | 10.439 | E012-T065-S057- | 0.000 | 7.436 |
| E011-T041-S186- | 0.000 | 10.295 | E011-T015-S186- | 0.000 | 7.430 |
| E012-T013-S158- | 0.000 | 10.040 | E014-T028-S186- | 0.000 | 7.417 |
| E013-T029-S058- | 4.437 | 9.654 | E011-T071-S098- | 4.692 | 7.401 |
| E011-T044-S145- | 0.000 | 9.625 | E013-T079-X001- | 0.000 | 7.392 |
| E012-T008-S085- | 4.537 | 9.592 | E011-T040-S143- | 0.000 | 7.372 |
| E014-T065-S064- | 1.253 | 9.559 | E012-T006-S186- | 0.000 | 7.362 |
| E011-T047-S189- | 0.000 | 9.513 | E014-T082-S109- | 0.000 | 7.351 |
| E012-T078-S154- | 0.000 | 9.471 | E014-T004-X001- | 0.000 | 7.344 |
| E013-T024-S176- | 2.385 | 9.354 | E014-T053-S174- | 0.000 | 7.338 |
| E012-T022-S186- | 0.000 | 9.344 | E012-T023-S135- | 0.000 | 7.320 |
| E012-T016-5186- | 1.875 | 9.244 | E011-T038-X001- | 0.000 | 7.307 |
| E013-T030-S175- | 2.252 | 9.223 | E012-T011-S186- | 0.000 | 7.287 |
| E013-T077-S186- | 0.000 | 9.217 | E011-T057-S169- | 0.000 | 7.284 |
| E012-T048-S157- | 0.000 | 9.153 | E012-T015-S186- | 0.000 | 7.275 |
| E013-T016-S186- | 2.036 | 9.085 | E011-T049-S189- | 0.000 | 7.263 |
| E011-T041-S199- | 2.779 | 9.018 | E011-T031-S186- | 5.596 | 7.257 |
| E011-T041-S186- | 0.000 | 8.856 | E013-T029-S104- | 0.000 | 7.234 |
| E012-T022-S186- | 0.000 | 8.856 | E013-T041-S186- | 0.000 | 7.226 |
| E014-T016-S142- | 0.000 | 8.830 | E013-T073-S186- | 0.000 | 7.214 |
| E011-T077-S186- | 0.000 | 8.615 | E011-T021-S157- | 170.218 | 4.017 |
| E013-T011-S186- | 5.158 | 8.540 | E014-T027-S176- | 174.583 | 3.959 |
| E012-T031-S085- | 0.000 | 8.537 | E011-T021-S083- | 151.189 | 3.611 |
| E013-T041-S143- | 4.731 | 8.536 | E012-T050-S145- | 68.520 | 4.545 |
| E013-T023-S105- | 0.000 | 8.515 | E011-T067-S146- | 49.063 | 4.896 |
| E012-T011-S186- | 2.024 | 8.510 | E011-T052-S143- | 70.467 | 4.322 |
| E012-T031-S085- | 0.000 | 8.471 | E014-T074-S138- | 66.035 | 4.290 |
| E014-T016-S186- | 1.137 | 8.452 | E013-T012-S058- | 92.824 | 3.772 |
| E013-T069-S115- | 0.000 | 8.437 | E012-T015-S069- | 86.934 | 3.776 |
| E011-T008-S177- | 0.000 | 8.330 | E013-T023-S157- | 85.531 | 3.689 |
| E014-T017-S141- | 0.971 | 8.328 | E012-T006-S136- | 7.798 | 6.983 |
| E014-T028-S186- | 1.897 | 8.305 | E012-T024-S062- | 69.784 | 3.940 |
| E013-T016-S186- | 0.000 | 8.292 | E012-T025-S085- | 108.753 | 3.034 |
| E014-T044-S099- | 2.202 | 8.285 | E011-T016-S186- | 5.447 | 7.123 |
| E014-T020-S168- | 2.235 | 8.284 | E011-T015-S186- | 9.296 | 6.412 |
| E012-T030-S115- | 0.000 | 8.257 | E012-T004-S195- | 44.560 | 4.178 |
| E014-T002-S169- | 0.000 | 8.239 | E011-T032-S102- | 5.635 | 6.948 |
| E013-T020-S194- | 0.000 | 8.226 | E011-T014-S117- | 10.350 | 6.156 |
| E011-T018-S117- | 0.000 | 8.212 | E014-T015-S186- | 6.839 | 6.688 |
| E013-T062-S186- | 7.937 | 8.203 | E013-T048-S177- | 60.727 | 3.700 |
| E014-T062-S186- | 0.000 | 8.155 | E012-T028-S186- | 17.510 | 5.428 |
| E012-T078-S129- | 0.000 | 8.111 | E012-T059-S135- | 6.206 | 6.655 |
| E011-T017-S077- | 0.000 | 8.105 | E012-T044-S081- | 45.663 | 3.895 |
| E011-T006-S186- | 0.000 | 8.095 | E014-T011-S186- | 4.298 | 7.022 |
| E013-T050-S186- | 4.359 | 8.015 | E011-T017-S058- | 7.582 | 6.323 |
| E011-T041-S054- | 0.000 | 7.984 | E013-T027-S175- | 27.643 | 4.558 |
| E011-T036-S138- | 0.000 | 7.978 | E014-T056-S136- | 4.099 | 7.044 |
| E014-T080-S197- | 0.000 | 7.899 | E014-T065-S196- | 4.875 | 6.823 |
| E014-T036-S106- | 0.000 | 7.886 | E013-T028-S186- | 4.215 | 6.893 |
| E014-T017-S058- | 2.263 | 7.837 | 1011-T023-S058- | 37.543 | 3.995 |
| E012-T055-S186- | 0.932 | 7.824 | E011-T028-S186- | 5.524 | 6.499 |
| E011-T015-S186- | 0.000 | 7.806 | E014-T072-S069- | 48.963 | 3.487 |
| E014-T030-S058- | 0.000 | 7.798 | E011-T032-S130- | 25.169 | 4.402 |
| E011-T017-S054- | 1.764 | 7.786 | E012-T052-S169- | 4.099 | 6.662 |
| E012-T028-S186- | 1.198 | 7.779 | E012-T076-S121- | 38.253 | 3.711 |
| E013-T062-S186- | 0.000 | 7.749 | E012-T062-S186- | 10.250 | 5.469 |
| E012-T011-S186- | 0.000 | 7.707 | E013-T011-S186- | 9.972 | 5.499 |
| E013-T035-S145- | 0.000 | 7.704 | E013-T029-S186- | 4.215 | 6.526 |
| E012-T066-S130- | 0.000 | 7.701 | E013-T011-S186- | 4.398 | 6.474 |
| E012-T017-S192- | 0.000 | 7.624 | E011-T075-S130- | 60.189 | 2.941 |
| E012-T002-S084- | 0.998 | 7.614 | E014-T044-S193- | 2.302 | 7.150 |
| E013-T050-S138- | 0.000 | 7.603 | E013-T045-S186- | 5.175 | 6.203 |
| E013-T043-S186- | 0.000 | 7.595 | E011-T065-S143- | 28.447 | 3.947 |
| E011-T034-S064- | 0.000 | 7.589 | E013-T077-S099- | 2.191 | 7.134 |
| E012-T017-S186- | 0.072 | 7.567 | E014-T057-S186- | 3.627 | 6.594 |
| E011-T030-S186- | 0.000 | 7.564 | E013-T011-S186- | 6.678 | 5.862 |
| E012-T011-S170- | 0.000 | 7.560 | E011-T073-S069- | 4.104 | 6.445 |
| E012-T039-S177- | 0.000 | 7.540 | E012-T046-S186- | 7.621 | 5.686 |
| E014-T041-S186- | 2.158 | 7.534 | E014-T062-S186- | 5.940 | 5.989 |
| E014-T012-S183- | 0.000 | 7.513 | E014-T028-S186- | 2.074 | 7.146 |
| E013-T055-S186- | 0.000 | 7.509 | E014-T055-S186- | 7.565 | 5.614 |
| E012-T045-S170- | 0.000 | 7.487 | E014-T011-S186- | 8.885 | 5.336 |
| E012-T024-S186- | 0.000 | 7.469 | E013-T055-S186- | 2.202 | 6.852 |
| E013-T010-S183- | 0.000 | 7.463 | E011-T019-S072- | 26.351 | 3.742 |

**Table 17. Library 4.1B Top Constructs.**

| BlockSequence | Norm_D7 | Enrich_D42 | BlockSequence | Norm_D7 | Enrich_D42 |
|---|---|---|---|---|---|
| E009-T061-S170-S050 | 0.56 | 9.29 | E008-T079-S142-S211 | 0.00 | 5.84 |
| E008-T001-S190-S047 | 0.00 | 8.97 | E008-T082-S126-S0S3 | 0.21 | 5.82 |
| E008-T020-S083-S080 | 0.77 | 8.64 | E010-T022-S143-S213 | 0.70 | 5.78 |
| E009-T074-S085-X002 | 1.39 | 8.43 | E009-T006-5194-5038 | 0.28 | 5.76 |
| E010-T034-S058-S212 | 0.70 | 8.13 | E006-T077-5199-5075 | 0.21 | 5.76 |
| E006-T010-S064-S051 | 0.42 | 7.90 | E006-T002-S198-X002 | 0.70 | 5.74 |
| E009-I055-S064-S053 | 1.05 | 7.73 | E010-T002-5165-5052 | 0.70 | 5.72 |
| E006-T039-S193-S075 | 0.70 | 7.38 | E010-T079-S194-S053 | 0.14 | 5.68 |
| E008-T071-S109-S212 | 1.12 | 7.37 | E006-T019-5199-5076 | 0.07 | 5.68 |
| E010-T019-S168-X002 | 1.81 | 7.31 | E008-T053-S102-S037 | 0.35 | 5.65 |
| E009-T072-S170-S211 | 0.77 | 6.56 | E006-T069-S193-S038 | 0.07 | 5.65 |
| E006-T049-S145-S052 | 0.28 | 6.52 | E008-T072-S171-S212 | 0.91 | 5.63 |
| E009-T010-S064-S051 | 2.02 | 6.49 | E007-T062-S137-S214 | 0.28 | 5.60 |
| E007-T010-S064-S047 | 1.26 | 6.41 | E007-T060-5176-5047 | 0.21 | 5.60 |
| E007-T056-S180-S213 | 1.32 | 6.33 | E009-T034-S064-S051 | 1.19 | 5.58 |
| E007-T008-S154-S074 | 0.28 | 6.31 | E008-T078-S103-S211 | 0.07 | 5.57 |
| E010-T027-S199-S075 | 0.07 | 6.31 | E008-T010-S062-S051 | 0.56 | 5.56 |
| E010-T017-S136-S050 | 0.14 | 6.26 | E010-T057-S154-S213 | 0.28 | 5.55 |
| E008-T043-S109-S047 | 0.35 | 6.20 | E010-T051-S138-S075 | 0.28 | 5.54 |
| E008-T038-S102-S051 | 0.14 | 6.13 | E010-T031-S102-S039 | 0.07 | 5.48 |
| E008-T017-S058-X002 | 0.70 | 6.13 | E009-T071-5180-5074 | 0.14 | 5.48 |
| E007-T065-S102-S049 | 1.32 | 6.06 | E009-T027-S17S-S213 | 0.00 | 5.46 |
| E006-T030-5084-5074 | 1.53 | 6.03 | E007-T052-S193-S214 | 1.46 | 5.45 |
| E010-T036-S192-S050 | 0.70 | 5.88 | E009-T030-5087-5039 | 0.56 | 5.45 |
| E009-T017-S168-S216 | 0.49 | 5.87 | E007-T006-S059-S047 | 0.35 | 5.44 |

**Table 18. Lib3 P4 STOP.**

| BlockSequence | N D7 | N D21 3B | E D21 3B | N D21 3A | E D21 3A | N D35 3A | E D35 3A |
|---|---|---|---|---|---|---|---|
| E014-T041-S186- | 3.72 | 146.55 | 4.97 | 4927.54 | 10.03 | 5163.05 | 10.09 |
| E013-T012-S194- | 0.79 | 16.46 | 3.29 | 268.84 | 7.24 | 1221.60 | 9.42 |
| E011-T058-S156- | 0.92 | 41.10 | 4.45 | 275.82 | 7.17 | 874.92 | 8.83 |
| E013-T032-S145- | 0.00 | 0.00 | 0.00 | 613.68 | 9.26 | 390.18 | 8.61 |
| E014-T072-S101- | 3.23 | 740.94 | 7.45 | 14542.93 | 11.75 | 1520.08 | 8.49 |
| E011-T017-S186- | 1.05 | 0.00 | -1.04 | 1960.69 | 9.90 | 680.44 | 8.38 |
| E013-T023-S105- | 1.09 | 0.00 | -1.07 | 151.63 | 6.19 | 655.49 | 8.29 |
| E011-T039-S176- | 1.12 | 20.25 | 3.32 | 742.40 | 8.45 | 504.52 | 7.89 |
| E013-T055-S194- | 0.00 | 16.40 | 4.12 | 1736.80 | 10.76 | 191.90 | 7.59 |
| E014-T038-S186- | 6.13 | 0.00 | -2.83 | 1147.62 | 7.33 | 1218.74 | 7.42 |
| E014-T019-S170- | 3.05 | 164.40 | 5.35 | 950.31 | 7.87 | 665.15 | 7.36 |
| E014-T014-S110- | 0.00 | 0.00 | 0.00 | 269.39 | 8.08 | 133.71 | 7.07 |
| E013-T045-S135- | 2.82 | 0.00 | -1.94 | 39.44 | 3.40 | 460.88 | 6.92 |
| E013-T035-S176- | 0.00 | 34.31 | 5.14 | 216.60 | 7.77 | 105.91 | 6.74 |
| E014-T067-S106- | 2.21 | 118.19 | 5.21 | 541.72 | 7.40 | 322.27 | 6.65 |
| E012-T020-S186- | 3.03 | 26.98 | 2.80 | 5712.32 | 10.47 | 326.42 | 6.35 |
| E014-T074-S199- | 1.17 | 0.00 | -1.12 | 90.02 | 5.39 | 146.76 | 6.09 |
| E013-T066-S194- | 0.00 | 9.85 | 3.44 | 9.12 | 3.34 | 52.14 | 5.73 |
| E012-T030-S059- | 2.08 | 437.19 | 7.15 | 1336.66 | 8.76 | 142.82 | 5.54 |
| E011-T057-S102- | 1.18 | 9.13 | 2.22 | 0.00 | -1.12 | 99.52 | 5.53 |
| E013-T075-S116- | 0.95 | 0.00 | -0.96 | 551.34 | 8.14 | 86.06 | 5.48 |
| E011-T063-S158- | 1.45 | 0.00 | -1.29 | 218.81 | 6.49 | 106.86 | 5.46 |
| E013-T057-S120- | 0.00 | 17.18 | 4.18 | 33.01 | 5.09 | 42.82 | 5.45 |
| E012-T033-S130- | 0.00 | 67.18 | 6.09 | 142.99 | 7.17 | 41.19 | 5.40 |
| E014-T067-S194- | 0.86 | 79.68 | 5.44 | 342.51 | 7.53 | 67.16 | 5.20 |
| E014-T032-S197- | 0.45 | 10.58 | 2.99 | 166.33 | 6.85 | 48.53 | 5.09 |
| E012-T017-S059- | 2.72 | 15.38 | 2.14 | 66.93 | 4.19 | 125.01 | 5.08 |
| E014-T022-S130- | 0.00 | 19.29 | 4.34 | 132.58 | 7.06 | 29.71 | 4.94 |
| E012-T003-S130- | 0.00 | 16.46 | 4.13 | 316.91 | 8.31 | 27.19 | 4.82 |
| E011-T030-S082- | 8.98 | 204.84 | 4.37 | 1073.77 | 6.75 | 277.27 | 4.80 |
| E012-T043-S116- | 2.15 | 0.00 | -1.66 | 9.74 | 1.77 | 79.53 | 4.68 |
| E014-T028-S186- | 5.96 | 226.89 | 5.03 | 2380.18 | 8.42 | 153.76 | 4.47 |
| E012-T064-S168- | 2.07 | 6.61 | 1.31 | 263.63 | 6.43 | 58.60 | 4.28 |
| E013-T028-S117- | 0.00 | 0.00 | 0.00 | 147.22 | 7.21 | 17.67 | 4.22 |
| E014-T014-S106- | 1.39 | 9.07 | 2.07 | 0.00 | -1.26 | 42.21 | 4.17 |
| E012-T064-S072- | 0.96 | 0.00 | -0.97 | 36.87 | 4.27 | 33.92 | 4.15 |
| E013-T071-S117- | 0.00 | 0.00 | 0.00 | 68.83 | 6.13 | 16.18 | 4.10 |
| E013-T020-S083- | 7.87 | 458.11 | 5.69 | 1612.42 | 7.51 | 148.53 | 4.08 |
| E013-T009-S195- | 1.08 | 8.65 | 2.22 | 0.00 | -1.05 | 33.58 | 4.06 |
| E012-T074-S143- | 0.73 | 8.35 | 2.44 | 89.35 | 5.71 | 26.65 | 4.00 |
| E013-T007-S084- | 0.00 | 0.00 | 0.00 | 13.23 | 3.83 | 14.89 | 3.99 |
| E013-T031-S165- | 2.94 | 10.58 | 1.55 | 45.56 | 3.56 | 60.02 | 3.95 |
| E013-T016-S186- | 4.90 | 113.99 | 4.29 | 584.77 | 6.63 | 87.76 | 3.91 |
| E011-T021-S104- | 0.00 | 9.55 | 3.40 | 97.43 | 6.62 | 14.00 | 3.91 |
| E012-T001-X001- | 5.69 | 8.65 | 0.53 | 645.09 | 6.59 | 92.65 | 3.81 |
| E013-T041-S186- | 15.80 | 41.16 | 1.33 | 286.42 | 4.10 | 234.04 | 3.81 |
| E014-T011-S186- | 4.22 | 814.84 | 7.29 | 2615.15 | 8.97 | 71.51 | 3.80 |
| E013-T007-S121- | 2.12 | 10.34 | 1.86 | 7.29 | 1.41 | 39.90 | 3.71 |
| E012-T037-X001- | 1.28 | 0.00 | -1.19 | 15.49 | 2.85 | 28.82 | 3.71 |
| E014-T071-S121- | 3.30 | 233.32 | 5.77 | 469.83 | 6.77 | 55.13 | 3.71 |
| E013-T057-S176- | 8.49 | 10.03 | 0.22 | 131.66 | 3.81 | 114.27 | 3.60 |
| E014-T011-S170- | 2.73 | 0.00 | -1.90 | 484.03 | 7.02 | 43.91 | 3.59 |
| E014-T044-S190- | 1.73 | 9.97 | 2.00 | 149.91 | 5.79 | 31.00 | 3.55 |
| E012-T055-S168- | 1.19 | 0.00 | -1.13 | 154.51 | 6.15 | 24.54 | 3.54 |
| E014-T068-S121- | 0.00 | 19.53 | 4.36 | 115.93 | 6.87 | 10.40 | 3.51 |
| E012-T043-S186- | 3.44 | 234.64 | 5.73 | 434.80 | 6.62 | 49.42 | 3.50 |
| E012-T060-S195- | 4.58 | 26.26 | 2.29 | 150.89 | 4.77 | 60.91 | 3.47 |
| E014-T017-S099- | 2.26 | 12.50 | 2.05 | 193.70 | 5.90 | 33.92 | 3.42 |
| E014-T047-S189- | 0.55 | 4.09 | 1.71 | 13.47 | 3.22 | 15.50 | 3.41 |
| E014-T062-S186- | 11.85 | 364.97 | 4.83 | 1056.62 | 6.36 | 135.27 | 3.41 |
| E014-T069-S103- | 1.86 | 0.00 | -1.52 | 136.56 | 5.59 | 28.89 | 3.39 |
| E012-T041-S190- | 3.43 | 10.52 | 1.38 | 105.39 | 4.58 | 43.84 | 3.34 |
| E014-T046-S186- | 3.96 | 0.00 | -2.31 | 471.60 | 6.57 | 46.29 | 3.25 |
| E014-T079-S085- | 0.97 | 9.97 | 2.48 | 107.90 | 5.79 | 17.40 | 3.22 |
| E014-T032-S142- | 2.26 | 6.13 | 1.13 | 164.98 | 5.67 | 29.03 | 3.20 |
| E013-T021-S157- | 5.66 | 0.00 | -2.74 | 130.01 | 4.30 | 60.16 | 3.20 |
| E012-T031-S186- | 4.42 | 166.56 | 4.95 | 882.03 | 7.35 | 47.86 | 3.17 |
| E014-T035-S117- | 0.00 | 0.00 | 0.00 | 51.75 | 5.72 | 7.34 | 3.06 |
| E011-T043-S183- | 1.84 | 0.00 | -1.50 | 0.00 | -1.50 | 19.85 | 2.88 |
| E011-T045-S147- | 0.19 | 5.17 | 2.37 | 12.43 | 3.50 | 7.27 | 2.80 |
| E013-T003-S104- | 3.48 | 101.07 | 4.51 | 391.99 | 6.46 | 29.71 | 2.78 |
| E014-T006-S054- | 1.13 | 0.00 | -1.09 | 0.00 | -1.09 | 11.76 | 2.58 |
| E013-T073-S126- | 0.00 | 7.51 | 3.09 | 7.78 | 3.13 | 4.96 | 2.58 |
| E012-T057-S186- | 2.73 | 29.74 | 3.04 | 210.23 | 5.82 | 20.66 | 2.54 |
| E014-T015-S165- | 2.19 | 0.00 | -1.67 | 0.00 | -1.67 | 17.33 | 2.52 |
| E014-T010-S147- | 0.57 | 0.00 | -0.65 | 36.25 | 4.57 | 7.89 | 2.50 |
| E011-T012-S176- | 1.95 | 7.75 | 1.57 | 0.00 | -1.56 | 15.43 | 2.48 |
| E014-T078-S116- | 0.93 | 4.03 | 1.38 | 92.72 | 5.60 | 9.58 | 2.46 |
| E013-T051-S120- | 5.29 | 11.30 | 0.97 | 89.23 | 3.84 | 33.04 | 2.44 |
| E014-T072-S129- | 2.37 | 20.07 | 2.65 | 88.06 | 4.73 | 15.16 | 2.26 |
| E013-T001-S101- | 0.93 | 0.00 | -0.95 | 0.00 | -0.95 | 7.48 | 2.13 |
| E012-T019-S062- | 1.19 | 0.00 | -1.13 | 89.35 | 5.37 | 8.56 | 2.13 |
| E012-T015-S186- | 2.88 | 0.00 | -1.96 | 0.00 | -1.96 | 14.61 | 2.01 |
| E011-T055-S186- | 3.65 | 41.34 | 3.19 | 27.99 | 2.64 | 17.54 | 2.00 |
| E013-T021-S165- | 2.06 | 0.00 | -1.61 | 410.18 | 7.07 | 10.20 | 1.87 |
| E012-T056-S143- | 2.34 | 0.00 | -1.74 | 78.02 | 4.56 | 10.94 | 1.84 |
| E014-T016-S186- | 8.46 | 36.29 | 1.98 | 483.36 | 5.68 | 32.02 | 1.80 |
| E012-T069-S165- | 2.12 | 0.00 | -1.64 | 309.01 | 6.64 | 9.65 | 1.77 |
| E013-T034-S186- | 4.33 | 0.00 | -2.41 | 64.55 | 3.62 | 15.84 | 1.66 |
| E012-T057-S183- | 5.58 | 25.90 | 2.03 | 18.25 | 1.55 | 17.06 | 1.46 |
| E014-T057-S126- | 1.87 | 0.00 | -1.52 | 30.07 | 3.43 | 6.12 | 1.31 |
| E013-T010-S054- | 6.44 | 40.50 | 2.48 | 122.11 | 4.05 | 16.11 | 1.20 |
| E012-T011-S186- | 10.02 | 87.67 | 3.01 | 424.02 | 5.27 | 22.09 | 1.07 |
| E014-T050-S186- | 7.72 | 31.31 | 1.89 | 59.95 | 2.81 | 15.63 | 0.93 |
| E013-T027-S194- | 5.15 | 42.12 | 2.81 | 167.98 | 4.78 | 9.31 | 0.75 |
| E014-T028-S191- | 1.72 | 46.09 | 4.12 | 68.40 | 4.67 | 2.99 | 0.55 |
| E011-T062-S083- | 0.00 | 0.00 | 0.00 | 45.26 | 5.53 | 0.07 | 0.09 |
| E011-T001-S081- | 0.00 | 8.17 | 3.20 | 0.00 | 0.00 | 0.00 | 0.00 |
| E011-T001-S171- | 0.00 | 0.00 | 0.00 | 6.74 | 2.95 | 0.00 | 0.00 |
| E011-T001-S183- | 0.00 | 0.00 | 0.00 | 6.92 | 2.99 | 0.00 | 0.00 |
| E011-T004-S082- | 0.00 | 9.37 | 3.37 | 0.00 | 0.00 | 0.00 | 0.00 |
| E011-T004-S126- | 0.00 | 0.00 | 0.00 | 5.33 | 2.66 | 0.00 | 0.00 |
| E011-T004-S169- | 0.00 | 0.00 | 0.00 | 8.82 | 3.30 | 0.00 | 0.00 |
| E011-T005-S086- | 0.00 | 8.41 | 3.23 | 0.00 | 0.00 | 0.00 | 0.00 |
| E011-T005-S121- | 0.00 | 6.91 | 2.98 | 0.00 | 0.00 | 0.00 | 0.00 |
| E011-T005-S129- | 0.00 | 3.55 | 2.18 | 0.00 | 0.00 | 0.00 | 0.00 |
| E011-T006-S168- | 0.00 | 19.77 | 4.38 | 42.38 | 5.44 | 0.00 | 0.00 |
| E011-T006-S189- | 0.00 | 9.61 | 3.41 | 0.00 | 0.00 | 0.00 | 0.00 |
| E011-T008-S077- | 0.00 | 9.07 | 3.33 | 0.00 | 0.00 | 0.00 | 0.00 |
| E011-T008-S104- | 0.00 | 0.00 | 0.00 | 5.45 | 2.69 | 0.00 | 0.00 |
| E011-T008-S117- | 0.00 | 10.03 | 3.46 | 0.00 | 0.00 | 0.00 | 0.00 |
| E011-T009-S077- | 0.00 | 8.89 | 3.31 | 0.00 | 0.00 | 0.00 | 0.00 |
| E011-T009-S145- | 0.00 | 7.45 | 3.08 | 0.00 | 0.00 | 0.00 | 0.00 |
| E011-T011-S058- | 0.00 | 9.55 | 3.40 | 0.00 | 0.00 | 0.00 | 0.00 |
| E011-T011-S186- | 0.00 | 21.69 | 4.50 | 142.75 | 7.17 | 0.00 | 0.00 |
| E011-T012-S085- | 0.00 | 8.59 | 3.26 | 0.00 | 0.00 | 0.00 | 0.00 |
| E011-T012-S149- | 0.00 | 0.00 | 0.00 | 28.72 | 4.89 | 0.00 | 0.00 |
| E011-T012-S186- | 0.00 | 9.55 | 3.40 | 0.00 | 0.00 | 0.00 | 0.00 |
| E011-T014-S104- | 0.00 | 0.00 | 0.00 | 8.21 | 3.20 | 0.00 | 0.00 |
| E011-T014-S180- | 0.00 | 10.46 | 3.52 | 0.00 | 0.00 | 0.00 | 0.00 |
| E011-T016-S157- | 0.00 | 9.13 | 3.34 | 0.00 | 0.00 | 0.00 | 0.00 |
| E011-T016-S175- | 0.00 | 5.89 | 2.78 | 0.00 | 0.00 | 0.00 | 0.00 |
| E011-T018-S129- | 0.00 | 10.88 | 3.57 | 0.00 | 0.00 | 0.00 | 0.00 |
| E011-T018-S186- | 0.00 | 8.29 | 3.22 | 0.00 | 0.00 | 0.00 | 0.00 |
| E011-T019-S137- | 0.00 | 9.61 | 3.41 | 0.00 | 0.00 | 0.00 | 0.00 |
| E011-T019-S143- | 0.00 | 9.79 | 3.43 | 0.00 | 0.00 | 0.00 | 0.00 |
| E011-T019-S168- | 0.00 | 4.45 | 2.45 | 0.00 | 0.00 | 0.00 | 0.00 |
| E011-T019-S175- | 0.00 | 9.43 | 3.38 | 0.00 | 0.00 | 0.00 | 0.00 |
| E011-T020-S156- | 0.00 | 9.73 | 3.42 | 0.00 | 0.00 | 0.00 | 0.00 |
| E011-T021-S103- | 0.00 | 4.27 | 2.40 | 0.00 | 0.00 | 0.00 | 0.00 |
| E011-T022-S069- | 0.00 | 9.31 | 3.37 | 0.00 | 0.00 | 0.00 | 0.00 |
| E011-T023-S084- | 0.00 | 5.11 | 2.61 | 0.00 | 0.00 | 0.00 | 0.00 |
| E011-T023-S143- | 0.00 | 9.91 | 3.45 | 0.00 | 0.00 | 0.00 | 0.00 |
| E011-T027-S058- | 0.00 | 4.81 | 2.54 | 0.00 | 0.00 | 0.00 | 0.00 |
| E011-T027-S186- | 0.00 | 0.00 | 0.00 | 19.11 | 4.33 | 0.00 | 0.00 |
| E011-T028-S186- | 0.00 | 3.06 | 2.02 | 143.48 | 7.17 | 0.00 | 0.00 |
| E011-T029-S101- | 0.00 | 7.93 | 3.16 | 0.00 | 0.00 | 0.00 | 0.00 |
| E011-T029-S136- | 0.00 | 10.09 | 3.47 | 0.00 | 0.00 | 0.00 | 0.00 |
| E011-T030-S109- | 0.00 | 8.83 | 3.30 | 0.00 | 0.00 | 0.00 | 0.00 |
| E011-T030-S176- | 0.00 | 9.01 | 3.32 | 0.00 | 0.00 | 0.00 | 0.00 |
| E011-T031-S191- | 0.00 | 9.55 | 3.40 | 7.41 | 3.07 | 0.00 | 0.00 |
| E011-T032-S175- | 0.00 | 8.71 | 3.28 | 0.00 | 0.00 | 0.00 | 0.00 |
| E011-T033-S059- | 0.00 | 7.09 | 3.02 | 0.00 | 0.00 | 0.00 | 0.00 |
| E011-T033-S117- | 0.00 | 6.97 | 2.99 | 0.00 | 0.00 | 0.00 | 0.00 |
| E011-T033-X001- | 0.00 | 8.29 | 3.22 | 0.00 | 0.00 | 0.00 | 0.00 |
| E011-T034-S137- | 0.00 | 8.77 | 3.29 | 0.00 | 0.00 | 0.00 | 0.00 |
| E011-T034-S170- | 0.00 | 0.00 | 0.00 | 7.10 | 3.02 | 0.00 | 0.00 |
| E011-T035-S176- | 0.00 | 9.49 | 3.39 | 0.00 | 0.00 | 0.00 | 0.00 |
| E011-T037-S106- | 0.00 | 0.00 | 0.00 | 6.98 | 3.00 | 0.00 | 0.00 |
| E011-T038-S077- | 0.00 | 9.01 | 3.32 | 0.00 | 0.00 | 0.00 | 0.00 |
| E011-T038-S103- | 0.00 | 8.41 | 3.23 | 0.00 | 0.00 | 0.00 | 0.00 |
| E011-T039-S054- | 0.00 | 8.83 | 3.30 | 0.00 | 0.00 | 0.00 | 0.00 |
| E011-T039-S137- | 0.00 | 10.52 | 3.53 | 0.00 | 0.00 | 0.00 | 0.00 |
| E011-T040-S190- | 0.00 | 9.37 | 3.37 | 0.00 | 0.00 | 0.00 | 0.00 |
| E011-T041-S117- | 0.00 | 28.90 | 4.90 | 49.97 | 5.67 | 0.00 | 0.00 |
| E011-T041-S169- | 0.00 | 7.33 | 3.06 | 0.00 | 0.00 | 0.00 | 0.00 |
| E011-T041-S171- | 0.00 | 8.95 | 3.32 | 0.00 | 0.00 | 0.00 | 0.00 |
| E011-T041-S189- | 0.00 | 9.43 | 3.38 | 0.00 | 0.00 | 0.00 | 0.00 |
| E011-T041-X001- | 0.00 | 9.07 | 3.33 | 3.49 | 2.17 | 0.00 | 0.00 |
| E011-T042-S105- | 0.00 | 8.89 | 3.31 | 0.00 | 0.00 | 0.00 | 0.00 |
| E011-T042-S117- | 0.00 | 9.49 | 3.39 | 0.00 | 0.00 | 0.00 | 0.00 |
| E011-T042-S126- | 0.00 | 9.37 | 3.37 | 0.00 | 0.00 | 0.00 | 0.00 |
| E011-T044-S106- | 0.00 | 9.85 | 3.44 | 0.00 | 0.00 | 0.00 | 0.00 |
| E011-T044-S165- | 0.00 | 0.00 | 0.00 | 6.61 | 2.93 | 0.00 | 0.00 |
| E011-T045-S120- | 0.00 | 9.25 | 3.36 | 0.00 | 0.00 | 0.00 | 0.00 |
| E011-T045-S143- | 0.00 | 9.25 | 3.36 | 6.61 | 2.93 | 0.00 | 0.00 |
| E011-T046-S102- | 0.00 | 0.00 | 0.00 | 21.37 | 4.48 | 0.00 | 0.00 |
| E011-T047-S146- | 0.00 | 4.51 | 2.46 | 0.00 | 0.00 | 0.00 | 0.00 |
| E011-T047-S192- | 0.00 | 0.00 | 0.00 | 29.33 | 4.92 | 0.00 | 0.00 |
| E011-T048-S058- | 0.00 | 8.11 | 3.19 | 0.00 | 0.00 | 0.00 | 0.00 |
| E011-T048-S104- | 0.00 | 7.87 | 3.15 | 0.00 | 0.00 | 0.00 | 0.00 |
| E011-T050-S156- | 0.00 | 17.55 | 4.21 | 0.00 | 0.00 | 0.00 | 0.00 |
| E011-T050-S161- | 0.00 | 8.05 | 3.18 | 0.00 | 0.00 | 0.00 | 0.00 |
| E011-T050-S186- | 0.00 | 0.00 | 0.00 | 20.45 | 4.42 | 0.00 | 0.00 |
| E011-T050-S197- | 0.00 | 8.47 | 3.24 | 0.00 | 0.00 | 0.00 | 0.00 |
| E011-T051-S130- | 0.00 | 9.19 | 3.35 | 0.00 | 0.00 | 0.00 | 0.00 |
| E011-T052-S143- | 0.00 | 10.58 | 3.53 | 0.00 | 0.00 | 0.00 | 0.00 |
| E011-T053-S102- | 0.00 | 10.40 | 3.51 | 0.00 | 0.00 | 0.00 | 0.00 |
| E011-T054-S058- | 0.00 | 9.73 | 3.42 | 0.00 | 0.00 | 0.00 | 0.00 |
| E011-T054-S129- | 0.00 | 0.00 | 0.00 | 4.10 | 2.35 | 0.00 | 0.00 |
| E011-T055-S168- | 0.00 | 9.67 | 3.42 | 0.00 | 0.00 | 0.00 | 0.00 |
| E011-T058-S145- | 0.00 | 0.00 | 0.00 | 28.97 | 4.91 | 0.00 | 0.00 |
| E011-T061-S158- | 0.00 | 0.00 | 0.00 | 16.72 | 4.15 | 0.00 | 0.00 |
| E011-T061-S177- | 0.00 | 6.85 | 2.97 | 0.00 | 0.00 | 0.00 | 0.00 |
| E011-T061-S193- | 0.00 | 8.53 | 3.25 | 0.00 | 0.00 | 0.00 | 0.00 |
| E011-T063-S171- | 0.00 | 9.85 | 3.44 | 0.00 | 0.00 | 0.00 | 0.00 |
| E011-T065-S064- | 0.00 | 11.72 | 3.67 | 0.00 | 0.00 | 0.00 | 0.00 |
| E011-T065-S100- | 0.00 | 8.29 | 3.22 | 0.00 | 0.00 | 0.00 | 0.00 |
| E011-T065-S102- | 0.00 | 9.19 | 3.35 | 0.00 | 0.00 | 0.00 | 0.00 |
| E011-T066-X001- | 0.00 | 9.07 | 3.33 | 0.00 | 0.00 | 0.00 | 0.00 |
| E011-T067-S148- | 0.00 | 0.00 | 0.00 | 50.15 | 5.68 | 0.00 | 0.00 |
| E011-T068-S102- | 0.00 | 9.85 | 3.44 | 0.00 | 0.00 | 0.00 | 0.00 |
| E011-T068-S176- | 0.00 | 9.01 | 3.32 | 0.00 | 0.00 | 0.00 | 0.00 |
| E011-T069-S103- | 0.00 | 9.67 | 3.42 | 0.00 | 0.00 | 0.00 | 0.00 |
| E011-T069-S171- | 0.00 | 0.00 | 0.00 | 7.72 | 3.12 | 0.00 | 0.00 |
| E011-T070-S069- | 0.00 | 8.05 | 3.18 | 0.00 | 0.00 | 0.00 | 0.00 |
| E011-T072-S191- | 0.00 | 9.85 | 3.44 | 0.00 | 0.00 | 0.00 | 0.00 |
| E011-T073-S120- | 0.00 | 5.71 | 2.75 | 0.00 | 0.00 | 0.00 | 0.00 |
| E011-T074-S062- | 0.00 | 8.59 | 3.26 | 0.00 | 0.00 | 0.00 | 0.00 |
| E011-T075-S195- | 0.00 | 7.27 | 3.05 | 4.04 | 2.33 | 0.00 | 0.00 |
| E011-T079-S135- | 0.00 | 9.19 | 3.35 | 0.00 | 0.00 | 0.00 | 0.00 |
| E011-T080-S069- | 0.00 | 8.89 | 3.31 | 0.00 | 0.00 | 0.00 | 0.00 |
| E011-T080-S135- | 0.00 | 22.23 | 4.54 | 0.00 | 0.00 | 0.00 | 0.00 |
| E011-T081-S102- | 0.00 | 7.03 | 3.01 | 0.00 | 0.00 | 0.00 | 0.00 |
| E012-T001-S155- | 0.00 | 0.00 | 0.00 | 6.00 | 2.81 | 0.00 | 0.00 |
| E012-T001-S157- | 0.00 | 8.71 | 3.28 | 0.00 | 0.00 | 0.00 | 0.00 |
| E012-T002-S069- | 0.00 | 0.00 | 0.00 | 3.18 | 2.07 | 0.00 | 0.00 |
| E012-T002-S101- | 0 | 7.390728973 | 3.068796155 | 0 | 0 | 0 | 0 |
| E012-T002-S137- | 0 | 0 | 0 | 3.551864665 | 2.186457664 | 0 | 0 |
| E012-T003-X001- | 0 | 0 | 0 | 4.22549417 | 2.385567478 | 0 | 0 |
| E012-T004-S154- | 0 | 3.7254081 | 2.24043893 | 0 | 0 | 0 | 0 |
| E012-T004-S196- | 0 | 10.09465421 | 3.471792798 | 0 | 0 | 0 | 0 |
| E012-T006-5054- | 0 | 0 | 0 | 15.61595672 | 4.054497458 | 0 | 0 |
| E012-T007-S137- | 0 | 0 | 0 | 198.7819431 | 7.642282384 | 0 | 0 |
| E012-T007-S183- | 0 | 0 | 0 | 5.572753181 | 2.71649781 | 0 | 0 |
| E012-T008-S059- | 0 | 8.832822432 | 3.29760559 | 0 | 0 | 0 | 0 |
| E012-T008-S165- | 0 | 0 | 0 | 6.552577916 | 2.916969163 | 0 | 0 |
| E012-T009-S126- | 0 | 4.686803739 | 2.507618015 | 0 | 0 | 0 | 0 |
| E012-T009-S199- | 0 | 10.09465421 | 3.471792798 | 0 | 0 | 0 | 0 |
| E012-T010-S105- | 0 | 7.330641746 | 3.058427637 | 0 | 0 | 0 | 0 |
| E012-T010-S180- | 0.00 | 8.65 | 3.27 | 0.00 | 0.00 | 0.00 | 0.00 |
| E012-T011-S059- | 0.00 | 0.00 | 0.00 | 77.53 | 6.30 | 0.00 | 0.00 |
| E012-T011-S062- | 0.00 | 9.07 | 3.33 | 0.00 | 0.00 | 0.00 | 0.00 |
| E012-T011-S100- | 0 | 8.892909659 | 3.306394903 | 0 | 0 | 0 | 0 |
| E012-T011-S157- | 0 | 7.030205609 | 3.005436928 | 0 | 0 | 0 | 0 |
| E012-T011-S158- | 0 | 3.96575701 | 2.312013668 | 0 | 0 | 0 | 0 |
| E012-T012-S116- | 0 | 10.03456698 | 3.463958111 | 0 | 0 | 0 | 0 |
| E012-T012-S137- | 0 | 4.746890967 | 2.522781678 | 0 | 0 | 0 | 0 |
| E012-T012-S146- | 0 | 9.613956388 | 3.407890621 | 0 | 0 | 0 | 0 |
| E012-T013-S115- | 0 | 0 | 0 | 6.246382686 | 2.857260997 | 0 | 0 |
| E012-T014-S082- | 0 | 8.352124612 | 3.225294153 | 0 | 0 | 0 | 0 |
| E012-T014-S194- | 0 | 0 | 0 | 6.307621732 | 2.869401957 | 0 | 0 |
| E012-T016-S146- | 0 | 7.751252338 | 3.129489487 | 0 | 0 | 0 | 0 |
| E012-T016-S155- | 0 | 5.287676014 | 2.652526881 | 0 | 0 | 0 | 0 |
| E012-T017-S198- | 0 | 8.292037385 | 3.215994959 | 0 | 0 | 0 | 0 |
| E012-T019-S145- | 0 | 8.111775703 | 3.187732234 | 0 | 0 | 0 | 0 |
| E012-T019-S165- | 0 | 0 | 0 | 8.022315018 | 3.173497659 | 0 | 0 |
| E012-T019-S176- | 0 | 8.352124612 | 3.225294153 | 7.716119789 | 3.123686024 | 0 | 0 |
| E012-T020-S168- | 0 | 9.613956388 | 3.407890621 | 5.021601767 | 2.5901473 | 0 | 0 |
| E012-T020-S195- | 0 | 7.751252338 | 3.129489487 | 0 | 0 | 0 | 0 |
| E012-T021-S105- | 0 | 10.57535203 | 3.532984164 | 0 | 0 | 0 | 0 |
| E012-T021-S116- | 0 | 9.133258569 | 3.341026273 | 0 | 0 | 0 | 0 |
| E012-T022-S116- | 0 | 8.772735204 | 3.288762402 | 0 | 0 | 0 | 0 |
| E012-T022-S120- | 0 | 11.05604985 | 3.591685382 | 0 | 0 | 0 | 0 |
| E012-T022-S186- | 0 | 0 | 0 | 28.41491732 | 4.878476076 | 0 | 0 |
| E012-T023-S109- | 0 | 0 | 0 | 6.552577916 | 2.916969163 | 0 | 0 |
| E012-T023-S189- | 0 | 10.57535203 | 3.532984164 | 0 | 0 | 0 | 0 |
| E012-T024-S054- | 0 | 8.352124612 | 3.225294153 | 0 | 0 | 0 | 0 |
| E012-T024-S186- | 0 | 0 | 0 | 24.9242917 | 4.696232667 | 0 | 0 |
| E012-T025-S059- | 0 | 8.352124612 | 3.225294153 | 0 | 0 | 0 | 0 |
| E012-T026-S069- | 0 | 0 | 0 | 6.49133887 | 2.905223584 | 0 | 0 |
| E012-T026-S130- | 0 | 9.253433024 | 3.358035124 | 0 | 0 | 0 | 0 |
| E012-T026-S155- | 0 | 0 | 0 | 6.736295054 | 2.951642819 | 0 | 0 |
| E012-T026-S169- | 0 | 4.987239876 | 2.581891072 | 0 | 0 | 0 | 0 |
| E012-T029-S169- | 0 | 10.39509034 | 3.510340458 | 0 | 0 | 0 | 0 |
| E012-T030-S099- | 0 | 0 | 0 | 12.7989606 | 3.786487696 | 0 | 0 |
| E012-T030-S183- | 0 | 4.20610592 | 2.380204664 | 0 | 0 | 0 | 0 |
| E012-T030-S193- | 0 | 5.167501559 | 2.624686175 | 0 | 0 | 0 | 0 |
| E012-T031-S098- | 0 | 0 | 0 | 7.103729329 | 3.018585988 | 0 | 0 |
| E012-T031-S176- | 0 | 9.373607479 | 3.374845781 | 0 | 0 | 0 | 0 |
| E012-T032-S109- | 0 | 7.93151402 | 3.158904754 | 0 | 0 | 0 | 0 |
| E012-T032-S130- | 0 | 7.871426793 | 3.149166152 | 0 | 0 | 0 | 0 |
| E012-T032-S137- | 0 | 4.146018692 | 2.363456698 | 0 | 0 | 0 | 0 |
| E012-T032-S174- | 0 | 10.27491589 | 3.495044766 | 0 | 0 | 0 | 0 |
| E012-T032-S176- | 0 | 9.974479753 | 3.456080645 | 0 | 0 | 0 | 0 |
| E012-T034-S098- | 0 | 8.892909659 | 3.306394903 | 0 | 0 | 0 | 0 |
| E012-T034-S142- | 0 | 11.1762243 | 3.605994935 | 0 | 0 | 0 | 0 |
| E012-T034-S143- | 0 | 10.39509034 | 3.510340458 | 0 | 0 | 0 | 0 |
| E012-T036-S168- | 0 | 8.472299067 | 3.243714632 | 18.55543092 | 4.289497422 | 0 | 0 |
| E012-T038-S175- | 0 | 9.013084114 | 3.3238145 | 0 | 0 | 0 | 0 |
| E012-T041-S121- | 0 | 9.313520251 | 3.366464938 | 0 | 0 | 0 | 0 |
| E012-T042-S174- | 0 | 3.845582555 | 2.276670123 | 0 | 0 | 0 | 0 |
| E012-T042-S198- | 0 | 7.93151402 | 3.158904754 | 0 | 0 | 0 | 0 |
| E012-T043-S142- | 0 | 10.03456698 | 3.463958111 | 0 | 0 | 0 | 0 |
| E012-T043-S192- | 0 | 3.845582555 | 2.276670123 | 0 | 0 | 0 | 0 |
| E012-T043-S194- | 0 | 8.472299067 | 3.243714632 | 0 | 0 | 0 | 0 |
| E012-T044-S082- | 0 | 10.09465421 | 3.471792798 | 0 | 0 | 0 | 0 |
| E012-T045-S190- | 0 | 9.734130843 | 3.424133474 | 0 | 0 | 0 | 0 |
| E012-T046-S083- | 0 | 5.528024923 | 2.7066465 65 | 0 | 0 | 0 | 0 |
| E012-T046-S126- | 0 | 0 | 0 | 7.164968375 | 3.029447298 | 0 | 0 |
| E012-T047-S155- | 0 | 5.347763241 | 2.666248319 | 0 | 0 | 0 | 0 |
| E012-T048-S165- | 0 | 8.472299067 | 3.243714632 | 0 | 0 | 0 | 0 |
| E012-T049-S084- | 0 | 0 | 0 | 14.02374152 | 3.909172242 | 0 | 0 |
| E012-T049-S177- | 0 | 6.669682244 | 2.939166808 | 0 | 0 | 0 | 0 |
| E012-T050-S136- | 0 | 0 | 0 | 6.552577916 | 2.916969163 | 0 | 0 |
| E012-T051-S054- | 0 | 9.073171341 | 3.332446054 | 0 | 0 | 0 | 0 |
| E012-T051-S138- | 0 | 10.03456698 | 3.463958111 | 0 | 0 | 0 | 0 |
| E012-T052-S085- | 0 | 8.892909659 | 3.306394903 | 0 | 0 | 0 | 0 |
| E012-T052-S103- | 0 | 0 | 0 | 15.73843481 | 4.065092724 | 0 | 0 |
| E012-T052-S141- | 0 | 11.1762243 | 3.605994935 | 0 | 0 | 0 | 0 |
| E012-T053-S054- | 0 | 12.55823053 | 3.761097001 | 0 | 0 | 0 | 0 |
| E012-T053-S143- | 0 | 9.974479753 | 3.456080645 | 0 | 0 | 0 | 0 |
| E012-T053-S175- | 0 | 7.691165111 | 3.1195495 93 | 0 | 0 | 0 | 0 |
| E012-T054-S176- | 0 | 9.373607479 | 3.374845781 | 0 | 0 | 0 | 0 |
| E012-T055-S059- | 0 | 9.433694706 | 3.38317822 | 0 | 0 | 0 | 0 |
| E012-T055-S087- | 0 | 7.390728973 | 3.068796155 | 0 | 0 | 0 | 0 |
| E012-T055-S104- | 0 | 8.532386295 | 3.252837417 | 0 | 0 | 0 | 0 |
| E012-T055-S190- | 0 | 6.609595017 | 2.927819675 | 0 | 0 | 0 | 0 |
| E012-T056-S100- | 0 | 0 | 0 | 14.14621961 | 3.920885847 | 0 | 0 |
| E012-T056-S157- | 0 | 0 | 0 | 7.287446467 | 3.050927646 | 0 | 0 |
| E012-T057-S100- | 0 | 5.167501559 | 2.624686175 | 0 | 0 | 0 | 0 |
| E012-T057-S121- | 0 | 8.352124612 | 3.225294153 | 0 | 0 | 0 | 0 |
| E012-T057-S126- | 0 | 7.390728973 | 3.068796155 | 0 | 0 | 0 | 0 |
| E012-T057-S197- | 0 | 11.05604985 | 3.591685382 | 0 | 0 | 0 | 0 |
| E012-T057-S199- | 0 | 10.09465421 | 3.471792798 | 0 | 0 | 0 | 0 |
| E012-T058-S145- | 0 | 0 | 0 | 5.695231273 | 2.743133891 | 0 | 0 |
| E012-T059-S077- | 0 | 9.79421807 | 3.432186834 | 0 | 0 | 0 | 0 |
| E012-T059-S102- | 0 | 0 | 0 | 7.716119789 | 3.123686024 | 0 | 0 |
| E012-T060-S130- | 0 | 8.952996887 | 3.315130992 | 7.348685513 | 3.061549065 | 0 | 0 |
| E012-T061-S084- | 0 | 7.390728973 | 3.068796155 | 0 | 0 | 0 | 0 |
| E012-T061-S104- | 0 | 0 | 0 | 18.73914806 | 4.302987819 | 0 | 0 |
| E012-T061-S175- | 0 | 11.1762243 | 3.605994935 | 0 | 0 | 0 | 0 |
| E012-T062-S138- | 0 | 8.652560749 | 3.270911729 | 0 | 0 | 0 | 0 |
| E012-T062-S154- | 0 | 3.96575701 | 2.312013668 | 0 | 0 | 0 | 0 |
| E012-T062-S180- | 0 | 10.63543925 | 3.54045377 | 0 | 0 | 0 | 0 |
| E012-T063-S145- | 0 | 9.313520251 | 3.366464938 | 0 | 0 | 0 | 0 |
| E012-T063-S183- | 0 | 10.87578816 | 3.569951359 | 0 | 0 | 0 | 0 |
| E012-T063-S198- | 0 | 8.832822432 | 3.29760559 | 0 | 0 | 0 | 0 |
| E012-T065-S085- | 0 | 8.832822432 | 3.29760559 | 0 | 0 | 0 | 0 |
| E012-T066-S102- | 0 | 9.013084114 | 3.3238145 | 0 | 0 | 0 | 0 |
| E012-T066-S120- | 0 | 7.210467291 | 3.037464334 | 0 | 0 | 0 | 0 |
| E012-T066-S135- | 0 | 9.133258569 | 3.341026273 | 0 | 0 | 0 | 0 |
| E012-T066-S198- | 0 | 9.373607479 | 3.374845781 | 0 | 0 | 0 | 0 |
| E012-T068-S103- | 0 | 9.674043616 | 3.416034906 | 0 | 0 | 0 | 0 |
| E012-T068-S106- | 0 | 10.39509034 | 3.510340458 | 0 | 0 | 0 | 0 |
| E012-T068-S186- | 0 | 8.712647977 | 3.279864674 | 0 | 0 | 0 | 0 |
| E012-T068-S202- | 0 | 0 | 0 | 21.31118799 | 4.479695429 | 0 | 0 |
| E012-T069-5121- | 0 | 7.991601248 | 3.168578057 | 0 | 0 | 0 | 0 |
| E012-T071-S115- | 0 | 7.751252338 | 3.129489487 | 0 | 0 | 0 | 0 |
| E012-T071-S142- | 0 | 6.369246107 | 2.881517035 | 0 | 0 | 0 | 0 |
| E012-T072-5086- | 0 | 7.691165111 | 3.1195495 93 | 0 | 0 | 0 | 0 |
| E012-T073-S143- | 0 | 0 | 0 | 15.37100053 | 4.033070591 | 0 | 0 |
| E012-T073-S168- | 0 | 3.905669783 | 2.294450124 | 0 | 0 | 0 | 0 |
| E012-T074-S147- | 0 | 5.227588786 | 2.638673685 | 0 | 0 | 0 | 0 |
| E012-T074-S171- | 0 | 9.073171341 | 3.332446054 | 0 | 0 | 0 | 0 |
| E012-T075-S154- | 0 | 11.8371838 | 3.682256836 | 0 | 0 | 0 | 0 |
| E012-T075-S156- | 0 | 9.674043616 | 3.416034906 | 0 | 0 | 0 | 0 |
| E012-T076-S072- | 0 | 6.369246107 | 2.881517035 | 0 | 0 | 0 | 0 |
| E012-T076-S146- | 0 | 4.686803739 | 2.507618015 | 0 | 0 | 0 | 0 |
| E012-T077-S165- | 0 | 9.493781933 | 3.39146281 | 0.183717138 | 0.243324374 | 0 | 0 |
| E012-T078-S102- | 0 | 10.21482866 | 3.487335674 | 0 | 0 | 0 | 0 |
| E012-T079-S176- | 0 | 19.64852337 | 4.367966709 | 0 | 0 | 0 | 0 |
| E012-T080-S109- | 0 | 21.0305296 | 4.461432272 | 0 | 0 | 0 | 0 |
| E012-T080-S138- | 0 | 0 | 0 | 4.470450354 | 2.451659608 | 0 | 0 |
| E012-T080-S143- | 0 | 0 | 0 | 14.57489293 | 3.961150341 | 0 | 0 |
| E012-T081-S135- | 0 | 10.15474144 | 3.479585167 | 0 | 0 | 0 | 0 |
| E012-T081-S143- | 0 | 0 | 0 | 7.838597881 | 3.143817524 | 0 | 0 |
| E012-T082-S057- | 0 | 8.652560749 | 3.270911729 | 0 | 0 | 0 | 0 |
| E012-T082-S138- | 0 | 5.167501559 | 2.624686175 | 0 | 0 | 0 | 0 |
| E013-T001-S100- | 0 | 0 | 0 | 7.287446467 | 3.050927646 | 0 | 0 |
| E013-T001-S120- | 0 | 10.63543925 | 3.54045377 | 0 | 0 | 0 | 0 |
| E013-T001-S141- | 0 | 10.39509034 | 3.510340458 | 0 | 0 | 0 | 0 |
| E013-T001-S193- | 0 | 4.266193147 | 2.396760436 | 0 | 0 | 0 | 0 |
| E013-T003-S103- | 0 | 0 | 0 | 7.348685513 | 3.061549065 | 0 | 0 |
| E013-T003-S170- | 0 | 0 | 0 | 7.287446467 | 3.050927646 | 0 | 0 |
| E013-T003-S175- | 0 | 8.472299067 | 3.243714632 | 0 | 0 | 0 | 0 |
| E013-T003-S195- | 0 | 9.253433024 | 3.358035124 | 0 | 0 | 0 | 0 |
| E013-T003-X001- | 0 | 7.390728973 | 3.068796155 | 0 | 0 | 0 | 0 |
| E013-T004-S116- | 0 | 9.313520251 | 3.366464938 | 0 | 0 | 0 | 0 |
| E013-T005-S126- | 0 | 8.952996887 | 3.315130992 | 0 | 0 | 0 | 0 |
| E013-T005-S168- | 0 | 0 | 0 | 25.84287739 | 4.746467423 | 0 | 0 |
| E013-T006-S126- | 0 | 4.085931465 | 2.346512021 | 0 | 0 | 0 | 0 |
| E013-T006-S138- | 0 | 10.33500312 | 3.502712883 | 0 | 0 | 0 | 0 |
| E013-T006-S147- | 0 | 4.20610592 | 2.380204664 | 0 | 0 | 0 | 0 |
| E013-T008-S085- | 0 | 8.41221184 | 3.234533792 | 0 | 0 | 0 | 0 |
| E013-T008-S180- | 0 | 9.433694706 | 3.38317822 | 0 | 0 | 0 | 0 |
| E013-T008-S193- | 0 | 0 | 0 | 6.858773146 | 2.974304107 | 0 | 0 |
| E013-T008-X001- | 0 | 9.914392525 | 3.44815993 | 0 | 0 | 0 | 0 |
| E013-T009-S121- | 0 | 10.03456698 | 3.463958111 | 0 | 0 | 0 | 0 |
| E013-T009-S137- | 0 | 8.532386295 | 3.252837417 | 0 | 0 | 0 | 0 |
| E013-T010-S104- | 0 | 0 | 0 | 6.981251237 | 2.996614938 | 0 | 0 |
| E013-T010-S120- | 0 | 9.253433024 | 3.358035124 | 0 | 0 | 0 | 0 |
| E013-T010-S136- | 0 | 14.66128349 | 3.9691305 46 | 0 | 0 | 0 | 0 |
| E013-T011-S186- | 0 | 0 | 0 | 79.73323782 | 6.335090847 | 0 | 0 |
| E013-T012-S059- | 0 | 0 | 0 | 3.674342757 | 2.224763527 | 0 | 0 |
| E013-T012-S099- | 0 | 0 | 0 | 6.981251237 | 2.996614938 | 0 | 0 |
| E013-T012-S169- | 0 | 8.051688475 | 3.178186933 | 0 | 0 | 0 | 0 |
| E013-T013-S130- | 0 | 10.81570094 | 3.562633311 | 0 | 0 | 0 | 0 |
| E013-T013-S147- | 0 | 0 | 0 | 3.368147527 | 2.127021582 | 0 | 0 |
| E013-T013-S154- | 0 | 8.051688475 | 3.178186933 | 0 | 0 | 0 | 0 |
| E013-T014-S116- | 0 | 5.287676014 | 2.652526881 | 0 | 0 | 0 | 0 |
| E013-T015-S136- | 0 | 7.631077883 | 3.10954074 | 0 | 0 | 0 | 0 |
| E013-T016-S064- | 0 | 18.32660437 | 4.272516277 | 0 | 0 | 0 | 0 |
| E013-T016-S117- | 0 | 8.592473522 | 3.261902878 | 0 | 0 | 0 | 0 |
| E013-T016-S143- | 0 | 9.253433024 | 3.358035124 | 0 | 0 | 0 | 0 |
| E013-T017-S193- | 0 | 13.69988785 | 3.877733244 | 0 | 0 | 0 | 0 |
| E013-T018-S137- | 0 | 9.734130843 | 3.424133474 | 0 | 0 | 0 | 0 |
| E013-T019-S106- | 0 | 16.16346418 | 4.101268863 | 0 | 0 | 0 | 0 |
| E013-T019-S130- | 0 | 9.974479753 | 3.456080645 | 0 | 0 | 0 | 0 |
| E013-T019-S191- | 0 | 0 | 0 | 5.940187456 | 2.794974631 | 0 | 0 |
| E013-T020-S062- | 0 | 9.674043616 | 3.416034906 | 0 | 0 | 0 | 0 |
| E013-T020-S098- | 0 | 6.970118381 | 2.994601153 | 0 | 0 | 0 | 0 |
| E013-T020-S099- | 0 | 19.7086106 | 4.372158857 | 0 | 0 | 0 | 0 |
| E013-T020-S145- | 0 | 0 | 0 | 6.981251237 | 2.996614938 | 0 | 0 |
| E013-T020-S189- | 0 | 9.974479753 | 3.456080645 | 0 | 0 | 0 | 0 |
| E013-T020-S195- | 0 | 6.729769472 | 2.950425389 | 0 | 0 | 0 | 0 |
| E013-T020-S198- | 0 | 10.99596262 | 3.584477027 | 0 | 0 | 0 | 0 |
| E013-T021-S109- | 0 | 18.32660437 | 4.272516277 | 0 | 0 | 0 | 0 |
| E013-T021-S144- | 0 | 10.87578816 | 3.569951359 | 0 | 0 | 0 | 0 |
| E013-T022-S064- | 0 | 5.047327104 | 2.596297617 | 0 | 0 | 0 | 0 |
| E013-T022-S154- | 0 | 9.433694706 | 3.38317822 | 0 | 0 | 0 | 0 |
| E013-T023-S183- | 0 | 5.047327104 | 2.596297617 | 0 | 0 | 0 | 0 |
| E013-T024-S085- | 0 | 11.95735826 | 3.695699707 | 0 | 0 | 0 | 0 |
| E013-T025-S116- | 0 | 9.013084114 | 3.3238145 | 0 | 0 | 0 | 0 |
| E013-T025-S170- | 0 | 9.253433024 | 3.358035124 | 0 | 0 | 0 | 0 |
| E013-T025-S197- | 0 | 0 | 0 | 27.06765831 | 4.810836799 | 0 | 0 |
| E013-T026-S115- | 0 | 6.369246107 | 2.881517035 | 0 | 0 | 0 | 0 |
| E013-T027-S175- | 0 | 9.79421807 | 3.432186834 | 0 | 0 | 0 | 0 |
| E013-T028-S126- | 0 | 6.188984425 | 2.845787978 | 0 | 0 | 0 | 0 |
| E013-T028-S138- | 0 | 9.073171341 | 3.332446054 | 0 | 0 | 0 | 0 |
| E013-T030-S130- | 0.00 | 9.01 | 3.32 | 0.00 | 0.00 | 0.00 | 0.00 |
| E013-T031-S186- | 0.00 | 19.89 | 4.38 | 28.84 | 4.90 | 0.00 | 0.00 |
| E013-T032-S190- | 0.00 | 0.00 | 0.00 | 6.55 | 2.92 | 0.00 | 0.00 |
| E013-T033-S145- | 0.00 | 8.95 | 3.32 | 0.00 | 0.00 | 0.00 | 0.00 |
| E013-T034-S136- | 0.00 | 0.00 | 0.00 | 79.24 | 6.33 | 0.00 | 0.00 |
| E013-T034-S158- | 0 | 9.373607479 | 3.374845781 | 0 | 0 | 0 | 0 |
| E013-T034-S169- | 0 | 9.974479753 | 3.456080645 | 0 | 0 | 0 | 0 |
| E013-T036-S156- | 0 | 3.785495328 | 2.25866826 | 0 | 0 | 0 | 0 |
| E013-T036-S177- | 0 | 9.433694706 | 3.38317822 | 0 | 0 | 0 | 0 |
| E013-T037-S101- | 0 | 9.854305298 | 3.440195488 | 0 | 0 | 0 | 0 |
| E013-T037-S193- | 0 | 3.905669783 | 2.294450124 | 0 | 0 | 0 | 0 |
| E013-T037-S196- | 0 | 7.751252338 | 3.129489487 | 3.368147527 | 2.127021582 | 0 | 0 |
| E013-T038-S168- | 0 | 17.66564486 | 4.222313447 | 0 | 0 | 0 | 0 |
| E013-T038-X001- | 0 | 9.854305298 | 3.440195488 | 0 | 0 | 0 | 0 |
| E013-T039-S083- | 0 | 7.450816201 | 3.079090687 | 7.777358835 | 3.133786889 | 0 | 0 |
| E013-T039-S145- | 0 | 18.98756387 | 4.321030738 | 0 | 0 | 0 | 0 |
| E013-T040-S154- | 0 | 9.553869161 | 3.399700098 | 0 | 0 | 0 | 0 |
| E013-T041-S105- | 0 | 9.193345796 | 3.349555765 | 33.55899718 | 5.110989449 | 0 | 0 |
| E013-T041-S169- | 0 | 10.27491589 | 3.495044766 | 0 | 0 | 0 | 0 |
| E013-T042-S186- | 0 | 9.553869161 | 3.399700098 | 0 | 0 | 0 | 0 |
| E013-T043-S121- | 0 | 8.472299067 | 3.243714632 | 0 | 0 | 0 | 0 |
| E013-T043-S136- | 0 | 8.231950157 | 3.206635435 | 0 | 0 | 0 | 0 |
| E013-T045-S058- | 0 | 0 | 0 | 6.062665548 | 2.820212779 | 0 | 0 |
| E013-T045-S102- | 0 | 9.193345796 | 3.349555765 | 0 | 0 | 0 | 0 |
| E013-T045-S157- | 0 | 7.811339565 | 3.139361365 | 0 | 0 | 0 | 0 |
| E013-T045-S194- | 0 | 9.914392525 | 3.44815993 | 0 | 0 | 0 | 0 |
| E013-T046-S165- | 0 | 0 | 0 | 15.30976149 | 4.027663779 | 0 | 0 |
| E013-T046-S186- | 0 | 8.712647977 | 3.279864674 | 0 | 0 | 0 | 0 |
| E013-T048-S195- | 0 | 7.811339565 | 3.139361365 | 0 | 0 | 0 | 0 |
| E013-T049-S085- | 0 | 9.79421807 | 3.432186834 | 0 | 0 | 0 | 0 |
| E013-T049-S101- | 0 | 10.57535203 | 3.532984164 | 58.60576697 | 5.897380016 | 0 | 0 |
| E013-T049-S168- | 0 | 7.030205609 | 3.005436928 | 0 | 0 | 0 | 0 |
| E013-T050-S146- | 0 | 5.167501559 | 2.624686175 | 0 | 0 | 0 | 0 |
| E013-T052-S058- | 0 | 9.193345796 | 3.349555765 | 0 | 0 | 0 | 0 |
| E013-T053-S146- | 0 | 4.085931465 | 2.346512021 | 0 | 0 | 0 | 0 |
| E013-T053-S177- | 0 | 19.04765109 | 4.325361306 | 0 | 0 | 0 | 0 |
| E013-T054-S129- | 0 | 7.691165111 | 3.119549593 | 25.10800884 | 4.706420528 | 0 | 0 |
| E013-T054-S136- | 0 | 10.33500312 | 3.502712883 | 0 | 0 | 0 | 0 |
| E013-T054-S157- | 0 | 18.56695327 | 4.29034723 | 0 | 0 | 0 | 0 |
| E013-T054-S175- | 0 | 9.734130843 | 3.424133474 | 0 | 0 | 0 | 0 |
| E013-T054-S183- | 0 | 0 | 0 | 3.980537986 | 2.316301587 | 0 | 0 |
| E013-T055-S138- | 0 | 8.592473522 | 3.261902878 | 0 | 0 | 0 | 0 |
| E013-T055-S155- | 0 | 10.57535203 | 3.532984164 | 0 | 0 | 0 | 0 |
| E013-T056-S082- | 0 | 0 | 0 | 7.409924559 | 3.072092859 | 0 | 0 |
| E013-T056-S120- | 0 | 9.854305298 | 3.440195488 | 0 | 0 | 0 | 0 |
| E013-T056-S143- | 0 | 6.910031154 | 2.983683377 | 0 | 0 | 0 | 0 |
| E013-T056-S155- | 0 | 8.892909659 | 3.306394903 | 0 | 0 | 0 | 0 |
| E013-T057-S082- | 0 | 8.532386295 | 3.252837417 | 0 | 0 | 0 | 0 |
| E013-T057-S086- | 0 | 7.570990656 | 3.099461964 | 0 | 0 | 0 | 0 |
| E013-T057-S135- | 0 | 8.532386295 | 3.252837417 | 0 | 0 | 0 | 0 |
| E013-T057-S155- | 0 | 0 | 0 | 7.471163605 | 3.082560153 | 0 | 0 |
| E013-T057-S175- | 0 | 8.352124612 | 3.225294153 | 0 | 0 | 0 | 0 |
| E013-T059-S103- | 0 | 0 | 0 | 6.368860778 | 2.881441597 | 0 | 0 |
| E013-T059-S168- | 0 | 9.734130843 | 3.424133474 | 0 | 0 | 0 | 0 |
| E013-T059-S175- | 0 | 10.09465421 | 3.471792798 | 0 | 0 | 0 | 0 |
| E013-T060-S129- | 0 | 3.96575701 | 2.312013668 | 0 | 0 | 0 | 0 |
| E013-T061-S054- | 0.00 | 0.00 | 0.00 | 5.88 | 2.78 | 0.00 | 0.00 |
| E013-T062-S105- | 0.00 | 16.58 | 4.14 | 0.00 | 0.00 | 0.00 | 0.00 |
| E013-T062-S109- | 0.00 | 0.00 | 0.00 | 19.66 | 4.37 | 0.00 | 0.00 |
| E013-T062-S117- | 0.00 | 0.00 | 0.00 | 48.87 | 5.64 | 0.00 | 0.00 |
| E013-T063-S083- | 0.00 | 0.00 | 0.00 | 11.21 | 3.61 | 0.00 | 0.00 |
| E013-T063-S099- | 0.00 | 0.00 | 0.00 | 8.33 | 3.22 | 0.00 | 0.00 |
| E013-T063-S101- | 0.00 | 4.09 | 2.35 | 0.00 | 0.00 | 0.00 | 0.00 |
| E013-T063-S120- | 0.00 | 0.00 | 0.00 | 471.54 | 8.88 | 0.00 | 0.00 |
| E013-T063-S121- | 0.00 | 0.00 | 0.00 | 6.74 | 2.95 | 0.00 | 0.00 |
| E013-T063-S198- | 0.00 | 0.00 | 0.00 | 28.78 | 4.90 | 0.00 | 0.00 |
| E013-T064-S138- | 0.00 | 4.87 | 2.55 | 0.00 | 0.00 | 0.00 | 0.00 |
| E013-T066-S198- | 0.00 | 0.00 | 0.00 | 14.15 | 3.92 | 0.00 | 0.00 |
| E013-T066-X001- | 0.00 | 8.77 | 3.29 | 0.00 | 0.00 | 0.00 | 0.00 |
| E013-T067-S180- | 0 | 8.17186293 | 3.197214795 | 0 | 0 | 0 | 0 |
| E013-T068-S136- | 0 | 7.631077883 | 3.10954074 | 0 | 0 | 0 | 0 |
| E013-T068-S145- | 0 | 10.09465421 | 3.471792798 | 0 | 0 | 0 | 0 |
| E013-T069-S083- | 0 | 8.832822432 | 3.29760559 | 0 | 0 | 0 | 0 |
| E013-T069-S120- | 0 | 4.987239876 | 2.581891072 | 0 | 0 | 0 | 0 |
| E013-T069-S121- | 0 | 9.674043616 | 3.416034906 | 0 | 0 | 0 | 0 |
| E013-T069-S135- | 0 | 9.914392525 | 3.44815993 | 0 | 0 | 0 | 0 |
| E013-T070-S147- | 0 | 0 | 0 | 6.246382686 | 2.857260997 | 0 | 0 |
| E013-T071-S121- | 0 | 24.81602493 | 4.690194971 | 35.76360283 | 5.200206252 | 0 | 0 |
| E013-T071-S126- | 0 | 8.952996887 | 3.315130992 | 0 | 0 | 0 | 0 |
| E013-T071-S197- | 0 | 7.871426793 | 3.149166152 | 0 | 0 | 0 | 0 |
| E013-T072-S126- | 0 | 0 | 0 | 8.022315018 | 3.173497659 | 0 | 0 |
| E013-T072-S135- | 0 | 4.987239876 | 2.581891072 | 0 | 0 | 0 | 0 |
| E013-T073-S082- | 0 | 9.013084114 | 3.3238145 | 0 | 0 | 0 | 0 |
| E013-T073-S102- | 0 | 9.613956388 | 3.407890621 | 0 | 0 | 0 | 0 |
| E013-T073-S168- | 0 | 7.751252338 | 3.129489487 | 5.940187456 | 2.794974631 | 0 | 0 |
| E013-T074-S138- | 0 | 10.15474144 | 3.479585167 | 0 | 0 | 0 | 0 |
| E013-T074-S193- | 0 | 8.472299067 | 3.243714632 | 0 | 0 | 0 | 0 |
| E013-T076-S062- | 0 | 0 | 0 | 4.22549417 | 2.385567478 | 0 | 0 |
| E013-T076-S171- | 0 | 8.772735204 | 3.288762402 | 0 | 0 | 0 | 0 |
| E013-T077-S098- | 0 | 0 | 0 | 11.51294064 | 3.645348969 | 0 | 0 |
| E013-T077-S102- | 0 | 4.326280375 | 2.413128374 | 0 | 0 | 0 | 0 |
| E013-T077-S137- | 0 | 9.433694706 | 3.38317822 | 0 | 0 | 0 | 0 |
| E013-T077-S156- | 0 | 10.33500312 | 3.502712883 | 0 | 0 | 0 | 0 |
| E013-T077-S193- | 0 | 8.712647977 | 3.279864674 | 0 | 0 | 0 | 0 |
| E013-T078-S082- | 0 | 4.867065422 | 2.552639079 | 5.87894841 | 2.782188036 | 0 | 0 |
| E013-T078-S197- | 0 | 12.4981433 | 3.75468907 | 78.32473976 | 6.309698978 | 0 | 0 |
| E013-T080-S081- | 0 | 0 | 0 | 13.41135106 | 3.849133689 | 0 | 0 |
| E013-T080-S175- | 0 | 8.652560749 | 3.270911729 | 0 | 0 | 0 | 0 |
| E013-T082-S137- | 0 | 8.532386295 | 3.252837417 | 0 | 0 | 0 | 0 |
| E013-T082-S186- | 0 | 10.03456698 | 3.463958111 | 0 | 0 | 0 | 0 |
| E014-T001-S120- | 0 | 8.892909659 | 3.306394903 | 0 | 0 | 0 | 0 |
| E014-T001-S186- | 0 | 0 | 0 | 7.164968375 | 3.029447298 | 0 | 0 |
| E014-T003-S121- | 0 | 8.051688475 | 3.178186933 | 0 | 0 | 0 | 0 |
| E014-T003-S192- | 0 | 7.631077883 | 3.10954074 | 0 | 0 | 0 | 0 |
| E014-T003-S193- | 0 | 0 | 0 | 6.981251237 | 2.996614938 | 0 | 0 |
| E014-T006-S138- | 0 | 0 | 0 | 6.18514364 | 2.845016998 | 0 | 0 |
| E014-T006-S156- | 0 | 11.1762243 | 3.605994935 | 0 | 0 | 0 | 0 |
| E014-T006-S176- | 0 | 8.832822432 | 3.29760559 | 0 | 0 | 0 | 0 |
| E014-T007-S194- | 0 | 9.613956388 | 3.407890621 | 0 | 0 | 0 | 0 |
| E014-T008-S103- | 0 | 0 | 0 | 3.245669435 | 2.085992048 | 0 | 0 |
| E014-T010-S054- | 0 | 9.553869161 | 3.399700098 | 0 | 0 | 0 | 0 |
| E014-T010-S100- | 0 | 0 | 0 | 9.981964489 | 3.457064246 | 0 | 0 |
| E014-T010-S104- | 0 | 4.566629284 | 2.476804009 | 0 | 0 | 0 | 0 |
| E014-T011-S130- | 0 | 8.472299067 | 3.243714632 | 0 | 0 | 0 | 0 |
| E014-T012-S104- | 0 | 8.832822432 | 3.29760559 | 0 | 0 | 0 | 0 |
| E014-T012-S138- | 0 | 9.674043616 | 3.416034906 | 0 | 0 | 0 | 0 |
| E014-T013-S117- | 0 | 7.691165111 | 3.1195495 93 | 0 | 0 | 0 | 0 |
| E014-T013-S155- | 0 | 8.41221184 | 3.234533792 | 0 | 0 | 0 | 0 |
| E014-T014-S183- | 0 | 4.806978194 | 2.537787617 | 0 | 0 | 0 | 0 |
| E014-T015-S062- | 0 | 9.674043616 | 3.416034906 | 0 | 0 | 0 | 0 |
| E014-T016-S085- | 0 | 8.292037385 | 3.215994959 | 0 | 0 | 0 | 0 |
| E014-T017-S058- | 0 | 0 | 0 | 7.348685513 | 3.061549065 | 0 | 0 |
| E014-T017-S069- | 0 | 17.48538318 | 4.208313044 | 0 | 0 | 0 | 0 |
| E014-T017-S104- | 0 | 0 | 0 | 3.735581802 | 2243541683 | 0 | 0 |
| E014-T017-S183- | 0 | 0 | 0 | 7.348685513 | 3.061549065 | 0 | 0 |
| E014-T017-S193- | 0 | 48.73074144 | 5.636066036 | 12.49276537 | 3.754114157 | 0 | 0 |
| E014-T018-S136- | 0 | 0 | 0 | 6.981251237 | 2.996614938 | 0 | 0 |
| E014-T018-S186- | 0 | 8.712647977 | 3.279864674 | 0 | 0 | 0 | 0 |
| E014-T018-X001- | 0 | 9.433694706 | 3.38317822 | 0 | 0 | 0 | 0 |
| E014-T019-S130- | 0 | 15.14198131 | 4.012745765 | 0 | 0 | 0 | 0 |
| E014-T019-S176- | 0 | 10.75561371 | 3.555277953 | 0 | 0 | 0 | 0 |
| E014-T019-S199- | 0 | 4.386367602 | 2.429312692 | 0 | 0 | 0 | 0 |
| E014-T021-S135- | 0 | 0 | 0 | 7.532402651 | 3.092952049 | 0 | 0 |
| E014-T021-S147- | 0 | 8.472299067 | 3.243714632 | 0 | 0 | 0 | 0 |
| E014-T024-S121- | 0 | 9.253433024 | 3.358035124 | 0 | 0 | 0 | 0 |
| E014-T026-S104- | 0 | 10.39509034 | 3.510340458 | 0 | 0 | 0 | 0 |
| E014-T026-S137- | 0 | 11.59683489 | 3.65498938 | 0 | 0 | 0 | 0 |
| E014-T026-S143- | 0 | 0 | 0 | 8.695944524 | 3.277381444 | 0 | 0 |
| E014-T027-S126- | 0 | 9.433694706 | 3.38317822 | 0 | 0 | 0 | 0 |
| E014-T028-S146- | 0 | 5.528024923 | 2.706646565 | 0 | 0 | 0 | 0 |
| E014-T029-S054- | 0 | 11.59683489 | 3.65498938 | 57.13602986 | 5.861360647 | 0 | 0 |
| E014-T030-S062- | 0 | 7.871426793 | 3.149166152 | 0 | 0 | 0 | 0 |
| E014-T030-S177- | 0 | 0 | 0 | 6.123904594 | 2.832668196 | 0 | 0 |
| E014-T030-S189- | 0 | 0 | 0 | 121.1308329 | 6.932283656 | 0 | 0 |
| E014-T031-S115- | 0 | 6.970118381 | 2.994601153 | 0 | 0 | 0 | 0 |
| E014-T032-S082- | 0.00 | 0.00 | 0.00 | 6.49 | 2.91 | 0.00 | 000 |
| E014-T032-S098- | 0.00 | 8.05 | 3.18 | 000 | 0.00 | 0.00 | 000 |
| E014-T032-S157- | 0.00 | 6.37 | 2.88 | 000 | 0.00 | 0.00 | 000 |
| E014-T033-S115- | 0.00 | 8.05 | 3.18 | 000 | 0.00 | 0.00 | 000 |
| E014-T033-S170- | 0 | 11.77709658 | 3.675488135 | 0 | 0 | 0 | 0 |
| E014-T034-S143- | 0 | 8.772735204 | 3.288762402 | 0 | 0 | 0 | 0 |
| E014-T035-S058- | 0 | 9.974479753 | 3.456080645 | 0 | 0 | 0 | 0 |
| E014-T036-S058- | 0 | 8.051688475 | 3.178186933 | 0 | 0 | 0 | 0 |
| E014-T036-S104- | 0 | 4.566629284 | 2.476804009 | 0 | 0 | 0 | 0 |
| E014-T036-S137- | 0 | 8.111775703 | 3.187732234 | 0 | 0 | 0 | 0 |
| E014-T037-S196- | 0 | 0 | 0 | 7.348685513 | 3.061549065 | 0 | 0 |
| E014-T038-S063- | 0 | 6.489420562 | 2.904854105 | 0 | 0 | 0 | 0 |
| E014-T038-S069- | 0 | 17.00468536 | 4.170300483 | 0 | 0 | 0 | 0 |
| E014-T038-S154- | 0 | 9.493781933 | 3.39146281 | 0 | 0 | 0 | 0 |
| E014-T038-S170- | 0 | 11.71700935 | 3.668687528 | 0 | 0 | 0 | 0 |
| E014-T039-S099- | 0 | 0 | 0 | 6.49133887 | 2.905223584 | 0 | 0 |
| E014-T040-S142- | 0 | 0 | 0 | 6.675056008 | 2.940177278 | 0 | 0 |
| E014-T041-S069- | 0 | 5.407850468 | 2.679840482 | 0 | 0 | 0 | 0 |
| E014-T041-S199- | 0 | 5.047327104 | 2.596297617 | 0 | 0 | 0 | 0 |
| E014-T042-S062- | 0 | 9.073171341 | 3.3324460 54 | 0 | 0 | 0 | 0 |
| E014-T042-S100- | 0 | 9.854305298 | 3.440195488 | 0 | 0 | 0 | 0 |
| E014-T042-S120- | 0 | 9.854305298 | 3.440195488 | 0 | 0 | 0 | 0 |
| E014-T042-S199- | 0 | 0 | 0 | 5.817709365 | 2.769287099 | 0 | 0 |
| E014-T042-S202- | 0 | 5.467937696 | 2.693305782 | 0 | 0 | 0 | 0 |
| E014-T044-S077- | 0 | 5.467937696 | 2.693305782 | 0 | 0 | 0 | 0 |
| E014-T044-S104- | 0 | 10.99596262 | 3.584477027 | 0 | 0 | 0 | 0 |
| E014-T044-S169- | 0 | 9.674043616 | 3.416034906 | 0 | 0 | 0 | 0 |
| E014-T045-S099- | 0 | 14.36084735 | 3.941185897 | 0 | 0 | 0 | 0 |
| E014-T045-S137- | 0 | 10.87578816 | 3.569951359 | 0 | 0 | 0 | 0 |
| E014-T045-S143- | 0 | 10.57535203 | 3.532984164 | 0 | 0 | 0 | 0 |
| E014-T045-S158- | 0 | 8.652560749 | 3.270911729 | 0 | 0 | 0 | 0 |
| E014-T046-S084- | 0 | 7.390728973 | 3.068796155 | 0 | 0 | 0 | 0 |
| E014-T046-S194- | 0 | 10.33500312 | 3.502712883 | 0 | 0 | 0 | 0 |
| E014-T047-S137- | 0 | 9.193345796 | 3.349555765 | 0 | 0 | 0 | 0 |
| E014-T048-S063- | 0 | 9.373607479 | 3.374845781 | 0 | 0 | 0 | 0 |
| E014-T048-S104- | 0 | 10.27491589 | 3.495044766 | 0 | 0 | 0 | 0 |
| E014-T049-S103- | 0 | 8.532386295 | 3.252837417 | 0 | 0 | 0 | 0 |
| E014-T049-S161- | 0 | 0 | 0 | 3.796820848 | 2.262078559 | 0 | 0 |
| E014-T050-S084- | 0 | 10.33500312 | 3.502712883 | 0 | 0 | 0 | 0 |
| E014-T051-S058- | 0 | 8.772735204 | 3.288762402 | 0 | 0 | 0 | 0 |
| E014-T051-S069- | 0 | 10.63543925 | 3.54045377 | 0 | 0 | 0 | 0 |
| E014-T052-S130- | 0 | 9.373607479 | 3.374845781 | 0 | 0 | 0 | 0 |
| E014-T052-S142- | 0 | 7.330641746 | 3.058427637 | 0 | 0 | 0 | 0 |
| E014-T052-S171- | 0 | 9.073171341 | 3.332446054 | 0 | 0 | 0 | 0 |
| E014-T052-S183- | 0 | 0 | 0 | 21.55614417 | 4.495448564 | 0 | 0 |
| E014-T053-S102- | 0 | 9.013084114 | 3.3238145 | 30.19084965 | 4.963050948 | 0 | 0 |
| E014-T053-S109- | 0 | 0 | 0 | 6.246382686 | 2.857260997 | 0 | 0 |
| E014-T055-S197- | 0 | 8.292037385 | 3.215994959 | 0 | 0 | 0 | 0 |
| E014-T056-S193- | 0 | 3.485059191 | 2.165127025 | 0 | 0 | 0 | 0 |
| E014-T057-S202- | 0 | 5.948635515 | 2.796729708 | 0 | 0 | 0 | 0 |
| E014-T058-S194- | 0 | 11.53674767 | 3.648091222 | 0 | 0 | 0 | 0 |
| E014-T059-S115- | 0 | 14.66128349 | 3.9691305 46 | 0 | 0 | 0 | 0 |
| E014-T060-S081- | 0 | 8.292037385 | 3.215994959 | 0 | 0 | 0 | 0 |
| E014-T060-S084- | 0 | 6.609595017 | 2.927819675 | 0 | 0 | 0 | 0 |
| E014-T060-S157- | 0 | 8.111775703 | 3.187732234 | 0 | 0 | 0 | 0 |
| E014-T060-S170- | 0 | 0 | 0 | 14.3911758 | 3.944031544 | 0 | 0 |
| E014-T061-S072- | 0 | 10.63543925 | 3.54045377 | 0 | 0 | 0 | 0 |
| E014-T061-S130- | 0 | 8.892909659 | 3.306394903 | 0 | 0 | 0 | 0 |
| E014-T061-S192- | 0 | 0 | 0 | 3.490625619 | 2.16691645 | 0 | 0 |
| E014-T061-S198- | 0 | 0 | 0 | 8.083554064 | 3.183256882 | 0 | 0 |
| E014-T062-S109- | 0 | 10.63543925 | 3.54045377 | 0 | 0 | 0 | 0 |
| E014-T063-S064- | 0 | 5.287676014 | 2.652526881 | 0 | 0 | 0 | 0 |
| E014-T065-S109- | 0 | 5.107414331 | 2.610561722 | 0 | 0 | 0 | 0 |
| E014-T065-S137- | 0 | 0 | 0 | 6.981251237 | 2.996614938 | 0 | 0 |
| E014-T065-S176- | 0 | 0 | 0 | 3.061952297 | 2.022173297 | 0 | 0 |
| E014-T066-S175- | 0 | 0 | 0 | 5.633992227 | 2.729877322 | 0 | 0 |
| E014-T066-S177- | 0 | 8.532386295 | 3.252837417 | 0 | 0 | 0 | 0 |
| E014-T066-S194- | 0 | 8.292037385 | 3.215994959 | 0 | 0 | 0 | 0 |
| E014-T067-S198- | 0 | 0 | 0 | 8.022315018 | 3.173497659 | 0 | 0 |
| E014-T068-S069- | 0 | 9.553869161 | 3.399700098 | 0 | 0 | 0 | 0 |
| E014-T068-S199- | 0 | 9.674043616 | 3.416034906 | 0 | 0 | 0 | 0 |
| E014-T069-S174- | 0 | 8.952996887 | 3.315130992 | 0 | 0 | 0 | 0 |
| E014-T071-S102- | 0 | 11.1762243 | 3.605994935 | 0 | 0 | 0 | 0 |
| E014-T072-S054- | 0 | 0 | 0 | 14.81984912 | 3.983663935 | 0 | 0 |
| E014-T072-S059- | 0 | 16.40381309 | 4.121331523 | 0 | 0 | 0 | 0 |
| E014-T072-S082- | 0 | 8.472299067 | 3.243714632 | 0 | 0 | 0 | 0 |
| E014-T072-S136- | 0 | 0 | 0 | 6.430099824 | 2.893381594 | 0 | 0 |
| E014-T073-S120- | 0 | 0 | 0 | 3.551864665 | 2.186457664 | 0 | 0 |
| E014-T074-S161- | 0 | 10.15474144 | 3.479585167 | 0 | 0 | 0 | 0 |
| E014-T075-S064- | 0 | 9.553869161 | 3.399700098 | 11.75789682 | 3.673318611 | 0 | 0 |
| E014-T076-S058- | 0 | 7.330641746 | 3.058427637 | 0 | 0 | 0 | 0 |
| E014-T076-S121- | 0 | 10.21482866 | 3.487335674 | 0 | 0 | 0 | 0 |
| E014-T076-S177- | 0 | 7.751252338 | 3.129489487 | 0 | 0 | 0 | 0 |
| E014-T076-X001- | 0 | 0 | 0 | 5.266557951 | 2.647673228 | 0 | 0 |
| E014-T077-S106- | 0 | 6.609595017 | 2.927819675 | 0 | 0 | 0 | 0 |
| E014-T077-S193- | 0 | 7.93151402 | 3.158904754 | 0 | 0 | 0 | 0 |
| E014-T077-S194- | 0 | 8.41221184 | 3.234533792 | 0 | 0 | 0 | 0 |
| E014-T078-S176- | 0 | 9.433694706 | 3.38317822 | 0 | 0 | 0 | 0 |
| E014-T079-S062- | 0 | 6.609595017 | 2.927819675 | 0 | 0 | 0 | 0 |
| E014-T079-S084- | 0 | 9.253433024 | 3.358035124 | 0 | 0 | 0 | 0 |
| E014-T079-S121- | 0 | 8.111775703 | 3.187732234 | 0 | 0 | 0 | 0 |
| E014-T079-S180- | 0 | 8.952996887 | 3.315130992 | 0 | 0 | 0 | 0 |
| E014-T080-S174- | 0 | 0 | 0 | 13.41135106 | 3.849133689 | 0 | 0 |
| E014-T081-S104- | 0 | 0 | 0 | 3.245669435 | 2.085992048 | 0 | 0 |
| E014-T081-S137- | 0 | 3.905669783 | 2.294450124 | 0 | 0 | 0 | 0 |
| E014-T082-S104- | 0 | 18.86738941 | 4.312330409 | 0 | 0 | 0 | 0 |
| E014-T082-S176- | 0 | 9.253433024 | 3.358035124 | 0 | 0 | 0 | 0 |
| E013-T050-S186- | 0.004325154 | 0 | - | 9.002139754 | 3.316010341 | 0 | - |
| E014-T008-X001- | 0.004325154 | 4.686803739 | 2.501391593 | 0 | - | 0 | - |
| E014-T024-S175- | 0.004325154 | 0 | - | 7.287446467 | 3.044701224 | 0 | - |
| E012-T021-S144- | 0.008650307 | 7.991601248 | 3.15615197 | 0 | - | 0 | - |
| E012-T075-S138- | 0.008650307 | 8.892909659 | 3.293968815 | 0 | - | 0 | - |
| E013-T005-S197- | 0.008650307 | 8.41221184 | 3.222107704 | 0 | - | 0 | - |
| E014-T035-S138- | 0.008650307 | 0 | - | 3.613103711 | 2.19331164 | 0 | - |
| E012-T055-S142- | 0.030276075 | 95.35842993 | 6.547308005 | 108.8830237 | 6.736793729 | 0 | - |
| E011-T030-S099- | 0.034601228 | 8.532386295 | 3.203762608 | 0 | - | 0 | - |
| E012-T074-S083- | 0.034601228 | 9.734130843 | 3.375058665 | 0 | - | 0 | - |
| E014-T044-S072- | 0.038926382 | 0 | - | 6.858773146 | 2.919210679 | 0 | - |
| E012-T006-S142- | 0.047576689 | 7.751252338 | 3.062433626 | 40.84644364 | 5.319977253 | 0 | - |
| E013-T056-S158- | 0.051901842 | 7.871426793 | 3.076166066 | 0 | - | 0 | - |
| E013-T047-S117- | 0.060552149 | 0 | - | 54.99266326 | 5.722350335 | 0 | - |
| E013-T023-S103- | 0.07352761 | 9.193345796 | 3.247196469 | 0 | - | 0 | - |
| E013-T072-S191- | 0.082177917 | 0 | - | 9.553291167 | 3.285683379 | 0 | - |
| E013-T073-S186- | 0.134079759 | 7.631077883 | 2.928018633 | 107.2295694 | 6.576428795 | 0 | - |
| E014-T073-S109- | 0.164355833 | 8.472299067 | 3.024182611 | 0 | - | 0 | - |
| E013-T037-S064- | 1.98957061 | 12.79857944 | 2.206509559 | 14.32993675 | 2.358341553 | 1.563447628 | - |
| E013-T074-S186- | 0.198957061 | 11.65692212 | 3.40007472 | 0 | - | 0 | - |
| E014-T065-S077- | 0.203282215 | 11.8371838 | 3.415281788 | 0 | - | 0 | - |
| E013-T029-S194- | 0.255184057 | 4.146018692 | 2.035557765 | 0 | - | 0 | - |
| E012-T020-S169- | 0.285460131 | 0 | - | 7.287446467 | 2.688642781 | 0 | - |
| E013-T051-S154- | 0.285460131 | 9.433694706 | 3.020893355 | 0 | - | 0 | - |
| E013-T055-S120- | 0.315736206 | 17.3051215 | 3.798305175 | 0 | - | 0 | - |
| E014-T011-S135- | 0.376288355 | 4.686803739 | 2.046835246 | 0 | -0.46078277 | 0 | -0.46078277 |
| E014-T003-X001- | 0.389263815 | 7.631077883 | 2.635220153 | 0 | - | 0 | - |
| E012-T073-S085- | 0.415214736 | 5.768373833 | 2.257788279 | 3.858059894 | 1.779359301 | 0 | - |
| E012-T073-S198- | 0.41953989 | 0 | -0.50542339 | 43.7246788 | 4.977575826 | 0 | -0.50542339 |
| E013-T044-S138- | 0.441165657 | 9.073171341 | 2.805209876 | 0 | - | 0 | - |
| E011-T077-S081- | 0.449815964 | 7.030205609 | 2.469567148 | 0 | -0.53586978 | 0 | -0.53586978 |
| E014-T024-S189- | 0.449815964 | 9.493781933 | 2.85559303 | 0 | -0.53586978 | 0 | -0.53586978 |
| E014-T016-S156- | 0.454141118 | 9.193345796 | 2.809388482 | 0 | - | 0 | - |
| E014-T057-S099- | 0.454141118 | 8.592473522 | 2.721735595 | 0 | - | 0 | - |
| E013-T053-S115- | 0.462791425 | 0 | - | 18.1267576 | 3.708796347 | 0 | - |
| E013-T080-S168- | 0.462791425 | 9.674043616 | 2.867310832 | 0 | - | 0 | - |
| E011-T009-S054- | 0.471441732 | 0 | - | 5.450275089 | 2.132130276 | 0 | - |
| E013-T057-S202- | 0.471441732 | 7.93151402 | 2.60167434 | 0 | - | 0 | - |
| E013-T072-S192- | 0.471441732 | 9.493781933 | 2.834232397 | 0 | - | 0 | - |
| E012-T070-S147- | 0.475766885 | 8.17186293 | 2.635749946 | 0 | - | 0 | - |
| E012-T013-S195- | 0.484417192 | 8.952996887 | 2.745234377 | 0 | - | 0 | - |
| E014-T066-X001- | 0.484417192 | 20.24939564 | 3.83945329 | 0 | - | 0 | - |
| E012-T065-S192- | 0.493067499 | 10.39509034 | 2.932061069 | 0 | - | 0 | - |
| E012-T070-S077- | 0.493067499 | 7.510903428 | 2.511032893 | 0 | - | 0 | - |
| E011-T056-S116- | 0.497392653 | 9.854305298 | 2.857742907 | 0 | - | 0 | - |
| E012-T033-S064- | 0.501717806 | 10.39509034 | 2.923726722 | 0 | - | 0 | - |
| E012-T077-S170- | 0.50604296 | 0 | - | 13.22763392 | 3.239860931 | 0 | - |
| E012-T002-S186- | 0.510368113 | 0 | - | 15.43223958 | 3.443557004 | 0 | - |
| E012-T019-S063- | 0.510368113 | 10.99596262 | 2.989576815 | 0 | - | 0 | - |
| E012-T041-S189- | 0.510368113 | 10.69552648 | 2.95298469 | 0 | - | 0 | - |
| E012-T071-S137- | 0.514693267 | 15.44241745 | 3.440324852 | 0 | -0.59902567 | 0 | -0.59902567 |
| E013-T024-S177- | 0.514693267 | 9.373607479 | 2.775820111 | 0 | -0.59902567 | 0 | -0.59902567 |
| E013-T032-S147- | 0.523343574 | 7.150380064 | 2.419625973 | 0 | - | 0 | - |
| E013-T069-S176- | 0.523343574 | 8.292037385 | 2.608753596 | 0 | - | 0 | - |
| E013-T080-S102- | 0.523343574 | 8.472299067 | 2.636473269 | 0 | - | 0 | - |
| E013-T011-S158- | 0.527668727 | 8.832822432 | 2.686273859 | 0 | - | 0 | - |
| E011-T028-S121- | 0.531993881 | 9.073171341 | 2.71703552 | 0 | - | 0 | - |
| E012-T052-S069- | 0.531993881 | 9.854305298 | 2.824784953 | 0 | - | 0 | - |
| E012-T001-S117- | 0.536319034 | 0 | - | 6.552577916 | 2.297491323 | 0 | - |
| E011-T053-X001- | 0.540644188 | 0 | - | 10.34939876 | 2.881010258 | 0 | - |
| E013-T003-S171- | 0.540644188 | 10.87578816 | 2.94641765 | 0 | - | 0 | - |
| E013-T032-S102- | 0.553619648 | 8.892909659 | 2.670761551 | 0 | - | 0 | - |
| E013-T037-S177- | 0.562269955 | 9.854305298 | 2.79655172 | 0 | - | 0 | - |
| E011-T057-S192- | 0.566595109 | 9.674043616 | 2.768402547 | 0 | - | 0 | - |
| E014-T052-S101- | 0.566595109 | 8.532386295 | 2.6052050 58 | 0 | - | 0 | - |
| E014-T065-S171- | 0.566595109 | 8.41221184 | 2.586901433 | 0 | - | 0 | - |
| E013-T033-S175- | 0.570920262 | 9.073171341 | 2.680836101 | 0 | - | 0 | - |
| E013-T059-S145- | 0.570920262 | 10.57535203 | 2.881374211 | 0 | - | 0 | - |
| E011-T075-S180- | 0.575245416 | 0 | - | 54.74770707 | 5.145263951 | 0 | - |
| E013-T078-S190- | 0.575245416 | 6.669682244 | 2.283590197 | 0 | - | 0 | - |
| E011-T004-S054- | 0.579570569 | 9.734130843 | 2.764601081 | 0 | - | 0 | - |
| E014-T065-S116- | 0.579570569 | 7.871426793 | 2.489633759 | 0 | - | 0 | - |
| E014-T028-S183- | 0.583895723 | 0 | - | 9.492052121 | 2.727747617 | 0 | - |
| E014-T063-S054- | 0.588220876 | 0 | - | 6.001426502 | 2.140237329 | 0 | - |
| E012-T012-S103- | 0.59254603 | 17.42529595 | 3.532280816 | 0 | - | 0 | - |
| E012-T057-S104- | 0.59254603 | 17.48538318 | 3.536977973 | 0 | - | 0 | - |
| E013-T060-S116- | 0.59254603 | 8.652560749 | 2.599576657 | 0 | - | 0 | - |
| E012-T058-S136- | 0.596871183 | 9.193345796 | 2.674307827 | 0 | - | 0 | - |
| E013-T073-S081- | 0.601196337 | 0 | -0.67915022 | 8.573466432 | 2.579891181 | 0 | -0.67915022 |
| E014-T022-S064- | 0.601196337 | 8.472299067 | 2.564564412 | 0 | -0.67915022 | 0 | -0.67915022 |
| E012-T082-S058- | 0.60552149 | 9.253433024 | 2.674993148 | 0 | - | 0 | - |
| E014-T042-S197- | 0.60552149 | 9.553869161 | 2.716658122 | 0 | - | 0 | - |
| E012-T064-S192- | 0.609846644 | 8.652560749 | 2.583988467 | 0 | - | 0 | - |
| E014-T006-S085- | 0.609846644 | 10.5152648 | 2.83855242 | 0 | - | 0 | - |
| E014-T047-S193- | 0.618496951 | 0 | - | 6.7975341 | 2.268363309 | 0 | - |
| E014-T071-X001- | 0.622822104 | 6.849943927 | 2.27417749 | 0 | - | 0 | - |
| E013-T032-S106- | 0.648773025 | 20.18930842 | 3.683871789 | 0 | - | 0 | - |
| E012-T067-S085- | 0.657423332 | 0 | - | 6.430099824 | 2.164439457 | 0 | - |
| E013-T062-S171- | 0.657423332 | 8.892909659 | 2.577452766 | 0 | - | 0 | - |
| E012-T030-S129- | 0.661748486 | 8.892909659 | 2.573692863 | 0 | - | 0 | - |
| E013-T042-S142- | 0.661748486 | 10.03456698 | 2.731256072 | 0 | - | 0 | - |
| E014-T047-S064- | 0.666073639 | 10.03456698 | 2.727505943 | 0 | - | 0 | - |
| E014-T057-S175- | 0.674723946 | 10.21482866 | 2.743412366 | 0 | - | 0 | - |
| E011-T015-S121- | 0.69202456 | 9.613956388 | 2.649140111 | 0 | -0.75875051 | 0 | -0.75875051 |
| E014-T036-S141- | 0.696349714 | 7.991601248 | 2.406144436 | 0 | - | 0 | - |
| E013-T006-S058- | 0.705000021 | 6.188984425 | 2.076016221 | 0 | - | 0 | - |
| E012-T074-S054- | 0.713650328 | 9.373607479 | 2.597773026 | 0 | - | 0 | - |
| E013-T040-S176- | 0.722300635 | 11.65692212 | 2.877517718 | 0 | - | 0 | - |
| E014-T073-S104- | 2.954079841 | 9.734130843 | 1.440791472 | 56.27868322 | 3.856584419 | 1.291543692 | - |
| E011-T003-S058- | 0.726625788 | 10.09465421 | 2.683837357 | 0 | - | 0 | - |
| E011-T028-S155- | 0.726625788 | 19.40817446 | 3.563119791 | 0 | - | 0 | - |
| E013-T065-S141- | 0.730950942 | 9.433694706 | 2.591613384 | 0 | - | 0 | - |
| E012-T021-S054- | 0.739601249 | 7.631077883 | 2.310784091 | 0 | - | 0 | - |
| E014-T063-S196- | 0.743926402 | 8.17186293 | 2.394875638 | 0 | - | 0 | - |
| E011-T044-S098- | 0.76555217 | 10.21482866 | 2.667216223 | 0 | - | 0 | - |
| E012-T072-S169- | 0.76555217 | 8.532386295 | 2.432717966 | 0 | - | 0 | - |
| E012-T075-S199- | 0.769877323 | 28.96204362 | 4.081414759 | 0 | - | 0 | - |
| E011-T035-S058- | 0.77852763 | 8.532386295 | 2.42215403 | 0 | - | 0 | - |
| E012-T042-S086- | 0.782852784 | 8.832822432 | 2.46341801 | 0 | -0.83418758 | 0 | -0.83418758 |
| E013-T020-S192- | 0.782852784 | 9.914392525 | 2.6 139723 5 | 0 | -0.83418758 | 0 | -0.83418758 |
| E013-T015-S084- | 0.787177937 | 0 | - | 6.858773146 | 2.136620826 | 0 | - |
| E013-T051-S180- | 0.787177937 | 0 | - | 6.981251237 | 2.158931658 | 0 | - |
| E011-T049-S103- | 0.791503091 | 10.39509034 | 2.669169925 | 0 | - | 0 | - |
| E013-T073-S103- | 0.791503091 | 8.472299067 | 2.402544099 | 0 | - | 0 | - |
| E013-T012-S176- | 0.804478551 | 8.772735204 | 2.437180407 | 0 | - | 0 | - |
| E011-T039-S115- | 0.808803705 | 9.493781933 | 2.536426958 | 0 | - | 0 | - |
| E012-T031-S168- | 0.813128858 | 0 | -0.85848146 | 6.736295054 | 2.093161358 | 0 | -0.85848146 |
| E011-T014-S191- | 0.817454012 | 11.35648598 | 2.765277755 | 0 | - | 0 | - |
| E012-T039-S059- | 0.817454012 | 7.871426793 | 2.287247293 | 0 | - | 0 | - |
| E011-T007-S098- | 0.821779165 | 0 | - | 7.838597881 | 2.278469437 | 0 | - |
| E012-T019-S168- | 0.821779165 | 0 | - | 6.613816962 | 2.0632718 | 0 | - |
| E012-T030-S197- | 0.821779165 | 7.93151402 | 2.293556666 | 0 | - | 0 | - |
| E014-T044-S171- | 0.830429472 | 9.013084114 | 2.451632313 | 0 | - | 0 | - |
| E013-T065-S103- | 0.834754626 | 8.472299067 | 2.368127497 | 51.50203764 | 4.838714376 | 0 | - |
| E014-T052-S137- | 0.834754626 | 9.553869161 | 2.524112964 | 0 | - | 0 | - |
| E014-T069-S098- | 0.834754626 | 16.46390031 | 3.250716761 | 7.532402651 | 2.217364915 | 0 | - |
| E012-T057-S176- | 0.839079779 | 8.592473522 | 2.382918812 | 13.22763392 | 2.951639789 | 0 | - |
| E013-T020-S135- | 0.839079779 | 8.772735204 | 2.fsS | 0 | - | 0 | - |
| E013-T058-S174- | 0.843404933 | 6.489420562 | 2.022481089 | 0 | - | 0 | - |
| E011-T082-S186- | 0.847730086 | 8.772735204 | 2.403008377 | 0 | - | 0 | - |
| E012-T030-S130- | 0.847730086 | 8.892909659 | 2.420640877 | 0 | - | 0 | - |
| E014-T002-S106- | 0.847730086 | 0 | - | 7.593641697 | 2.217515601 | 0 | - |
| E012-T033-S135- | 0.85205524 | 18.6270405 | 3.405643616 | 0 | - | 0 | - |
| E013-T030-S069- | 0.85205524 | 10.21482866 | 2.598208545 | 0 | - | 0 | - |
| E011-T077-S130- | 0.856380393 | 11.1762243 | 2.71350257 | 0 | - | 0 | - |
| E014-T012-S161- | 0.856380393 | 18.74721496 | 3.411084927 | 351.6958408 | 7.569788388 | 0 | - |
| E012-T079-S136- | 0.860705547 | 16.40381309 | 3.225481753 | 0 | -0.89584977 | 0 | -0.89584977 |
| E014-T022-S171- | 0.860705547 | 9.553869161 | 2.503850329 | 0 | -0.89584977 | 0 | -0.89584977 |
| E014-T049-S138- | 0.860705547 | 7.871426793 | 2.253316383 | 0 | -0.89584977 | 0 | -0.89584977 |
| E013-T055-S141- | 0.873681007 | 9.974479753 | 2.550205289 | 0 | - | 0 | - |
| E013-T048-S136- | 0.878006161 | 10.03456698 | 2.554756316 | 0 | - | 0 | - |
| E014-T049-S102- | 0.878006161 | 28.96204362 | 3.995862328 | 0 | - | 0 | - |
| E011-T032-S174- | 0.882331314 | 0 | - | 14.81984912 | 3.071143352 | 0 | - |
| E012-T074-S196- | 0.882331314 | 8.712647977 | 2.367344091 | 0 | - | 0 | - |
| E011-T072-S136- | 0.886656468 | 8.832822432 | 2.381773837 | 0 | - | 0 | - |
| E013-T047-S116- | 0.886656468 | 8.292037385 | 2.300163205 | 0 | - | 0 | - |
| E013-T044-S106- | 0.890981621 | 10.27491589 | 2.575909425 | 0 | - | 0 | - |
| E013-T055-S115- | 0.890981621 | 9.373607479 | 2.45571044 | 0 | - | 0 | - |
| E014-T042-S085- | 0.890981621 | 7.270554519 | 2.12884872 | 0 | - | 0 | - |
| E012-T047-S116- | 0.895306775 | 11.41657321 | 2.711763779 | 0 | - | 0 | - |
| E012-T063-S121- | 0.895306775 | 9.133258569 | 2.418594891 | 0 | - | 0 | - |
| E013-T014-S084- | 0.895306775 | 7.811339565 | 2.216929983 | 0 | - | 0 | - |
| E014-T069-S106- | 0.895306775 | 10.5152648 | 2.6030443 | 0 | - | 0 | - |
| E014-T075-S141- | 0.895306775 | 8.41221184 | 2.312102409 | 0 | - | 0 | - |
| E011-T030-S120- | 0.899631928 | 0 | -0.92571991 | 18.8003871 | 3.381736821 | 0 | -0.92571991 |
| E013-T072-S082- | 0.899631928 | 8.592473522 | 2.336182968 | 0 | -0.92571991 | 0 | -0.92571991 |
| E014-T041-S087- | 0.899631928 | 17.78581932 | 3.305852224 | 0 | -0.92571991 | 0 | -0.92571991 |
| E013-T014-S064- | 0.903957082 | 8.772735204 | 2.359761444 | 0 | - | 0 | - |
| E011-T011-X001- | 0.908282235 | 10.15474144 | 2.547310606 | 0 | - | 0 | - |
| E014-T049-S059- | 0.908282235 | 8.892909659 | 2.374120341 | 0 | - | 0 | - |
| E011-T045-S142- | 0.912607389 | 7.330641746 | 2.122886883 | 0 | - | 0 | - |
| E012-T016-S137- | 0.912607389 | 9.79421807 | 2.49664608 | 0 | - | 0 | - |
| E013-T031-S069- | 0.912607389 | 9.734130843 | 2.48859272 | 0 | - | 0 | - |
| E013-T055-S186- | 0.912607389 | 7.150380064 | 2.091326582 | 14.02374152 | 2.973631488 | 0 | - |
| E012-T013-S098- | 0.916932542 | 8.292037385 | 2.27719539 | 0 | - | 0 | - |
| E012-T025-S104- | 0.916932542 | 0 | - | 8.573466432 | 2.320241832 | 0 | - |
| E014-T012-S129- | 0.916932542 | 9.734130843 | 2.485333905 | 0 | - | 0 | - |
| E012-T035-S138- | 0.921257696 | 8.952996887 | 2.373079954 | 0 | - | 0 | - |
| E013-T069-S106- | 0.921257696 | 11.8371838 | 2.740205798 | 0 | - | 0 | - |
| E014-T038-S103- | 0.921257696 | 7.991601248 | 2.226527019 | 0 | - | 0 | - |
| E012-T021-S137- | 0.925582849 | 9.133258569 | 2.395731076 | 0 | - | 0 | - |
| E012-T030-S141- | 0.929908003 | 9.79421807 | 2.483654757 | 0 | - | 0 | - |
| E013-T017-S194- | 0.93 | 9.07 | 2.38 | 0.00 | -0.95 | 0.00 | -0.95 |
| E013-T051-S176- | 0.93 | 8.83 | 2.35 | 0.00 | -0.95 | 0.00 | -0.95 |
| E013-T065-S121- | 0.93 | 14.60 | 3.02 | 0.00 | -0.95 | 0.00 | -0.95 |
| E014-T055-S100- | 0.94 | 9.79 | 2.48 | 0.00 | -0.95 | 0.00 | -0.95 |
| E012-T077-S103- | 0.94 | 8.41 | 2.28 | 0.00 | -0.96 | 0.00 | -0.96 |
| E013-T051-S109- | 0.94 | 10.64 | 2.58 | 0.00 | -0.96 | 0.00 | -0.96 |
| E012-T034-S193- | 0.95 | 9.67 | 2.45 | 0.00 | -0.96 | 0.00 | -0.96 |
| E012-T048-S087- | 0.95153377 | 9.914392525 | 2.483551502 | 0 | - | 0 | - |
| E012-T053-S109- | 0.95153377 | 8.17186293 | 2.232606367 | 0 | - | 0 | - |
| E013-T074-S130- | 0.95153377 | 7.691165111 | 2.154941165 | 0 | - | 0 | - |
| E012-T031-S169- | 0.955858924 | 8.892909659 | 2.33859259 | 0 | - | 0 | - |
| E012-T064-S099- | 0.955858924 | 13.33936449 | 2.874106869 | 0 | - | 0 | - |
| E012-T012-S189- | 0.960184077 | 10.75561371 | 2.584288811 | 0 | - | 0 | - |
| E014-T041-S063- | 0.960184077 | 8.352124612 | 2.254305012 | 0 | - | 0 | - |
| E014-T059-S202- | 0.960184077 | 8.892909659 | 2.335405761 | 0 | - | 0 | - |
| E011-T042-S109- | 0.964509231 | 8.712647977 | 2.305695727 | 0 | - | 0 | - |
| E012-T030-S062- | 0.964509231 | 10.69552648 | 2.573715955 | 0 | - | 0 | - |
| E014-T050-S136- | 0.964509231 | 12.55823053 | 2.786928055 | 7.226207421 | 2.066058503 | 0 | - |
| E012-T073-S186- | 0.968834384 | 8.892909659 | 2.329053144 | 0 | - | 0 | - |
| E013-T082-S116- | 0.968834384 | 18.56695327 | 3.313005471 | 0 | - | 0 | - |
| E014-TO55-S103- | 0.968834384 | 18.44677882 | 3.304117542 | 0 | - | 0 | - |
| E014-T058-S135- | 0.968834384 | 8.41221184 | 2.257192033 | 0 | - | 0 | - |
| E012-T007-S142- | 0.973159538 | 10.27491589 | 2.514537158 | 0 | - | 0 | - |
| E013-T052-S126- | 0.973159538 | 20.30948287 | 3.432916069 | 0 | - | 0 | - |
| E012-T032-S183- | 0.981809845 | 10.75561371 | 2.568459411 | 0 | - | 0 | - |
| E012-T054-S098- | 0.981809845 | 8.652560749 | 2.284093187 | 0 | - | 0 | - |
| E013-T046-S077- | 0.981809845 | 8.772735204 | 2.30194386 | 0 | - | 0 | - |
| E013-T046-S137- | 0.981809845 | 7.811339565 | 2.152542823 | 0 | - | 0 | - |
| E014-T009-S109- | 0.981809845 | 8.952996887 | 2.32831245 | 0 | - | 0 | - |
| E014-T018-S169- | 0.981809845 | 10.81570094 | 2.575814769 | 0 | - | 0 | - |
| E014-T032-S198- | 0.981809845 | 30.16378817 | 3.974980166 | 0 | - | 0 | - |
| E011-T010-S186- | 0.986134998 | 0 | - | 8.083554064 | 2.193293195 | 0 | - |
| E011-T078-S109- | 0.986134998 | 9.493781933 | 2.401499124 | 0 | - | 0 | - |
| E013-T059-X001- | 0.986134998 | 7.871426793 | 2.159202465 | 0 | - | 0 | - |
| E014-T003-S154- | 0.986134998 | 10.69552648 | 2.557921215 | 0 | - | 0 | - |
| E014-T044-S085- | 0.986134998 | 9.433694706 | 2.393214534 | 0 | - | 0 | - |
| E012-T018-S202- | 0.990460152 | 1.742529595 | 0.4624052 | 7.348685513 | 2.068447076 | 0 | - |
| E012-T080-S145- | 0.990460152 | 9.914392525 | 2.45505794 | 0 | - | 0 | - |
| E011-T041-S202- | 0.994785305 | 10.03456698 | 2.467724631 | 0 | -0.99623348 | 0 | -0.99623348 |
| E012-T010-S058- | 0.994785305 | 9.914392525 | 2.451926449 | 0 | -0.99623348 | 0 | -0.99623348 |
| E012-T042-S063- | 0.994785305 | 18.98756387 | 3.324797258 | 0 | -0.99623348 | 0 | -0.99623348 |
| E013-T041-S104- | 0.994785305 | 9.79421807 | 2.435953353 | 0 | -0.99623348 | 0 | -0.99623348 |
| E013-T070-S199- | 0.994785305 | 17.78581932 | 3.235338653 | 0 | -0.99623348 | 0 | -0.99623348 |
| E014-T014-S154- | 0.994785305 | 9.073171341 | 2.336212574 | 96.81893163 | 5.615808322 | 0 | -0.99623348 |
| E014-T021-S117- | 0.994785305 | 8.772735204 | 2.292528921 | 0 | -0.99623348 | 0 | -0.99623348 |
| E014-T041-S082- | 0.994785305 | 8.772735204 | 2.292528921 | 0 | -0.99623348 | 0 | -0.99623348 |
| E014-T051-S121- | 0.994785305 | 7.510903428 | 2.093078801 | 4.041777032 | 1.337698837 | 0 | -0.99623348 |
| E011-T062-S062- | 0.999110459 | 14.42093458 | 2.947460108 | 0 | - | 0 | - |
| E012-T001-S142- | 0.999110459 | 7.030205609 | 2.006078739 | 0 | - | 0 | - |
| E014-T031-S186- | 0.999110459 | 10.69552648 | 2.548526713 | 23.94446696 | 3.641289747 | 0 | - |
| E014-T056-S103- | 0.999110459 | 10.03456698 | 2.464599922 | 0 | - | 0 | - |
| E014-T074-S106- | 0.999110459 | 10.5152648 | 2.526117493 | 0 | - | 0 | - |
| E012-T028-S115- | 1.003435612 | 8.111775703 | 2.18525609 | 0 | - | 0 | - |
| E012-T039-S176- | 1.003435612 | 8.292037385 | 2.213518814 | 0 | - | 0 | - |
| E013-T012-S064- | 1.003435612 | 14.66128349 | 2.966654402 | 0 | - | 0 | - |
| E013-T019-S116- | 1.003435612 | 6.609595017 | 1.925343531 | 29.4559811 | 3.92617753 | 0 | - |
| E014-T033-S176- | 1.003435612 | 7.93151402 | 2.15642861 | 0 | - | 0 | - |
| E014-T057-X001- | 1.003435612 | 11.41657321 | 2.631719017 | 0 | - | 0 | - |
| E014-T061-S186- | 1.003435612 | 0 | - | 17.57560619 | 3.212861242 | 0 | - |
| E014-T066-S115- | 1.003435612 | 18.80730218 | 3.3054843 45 | 0 | - | 0 | - |
| E011-T008-S186- | 1.007760766 | 0.060087227 | - | 19.04534329 | 3.319607844 | 0 | - |
| E011-T075-S196- | 1.007760766 | 8.772735204 | 2.283175026 | 0 | - | 0 | - |
| E011-T082-S142- | 1.007760766 | 8.41221184 | 2.228946416 | 0 | - | 0 | - |
| E012-T010-S143- | 1.007760766 | 9.373607479 | 2.369258406 | 0 | - | 0 | - |
| E012-T023-S154- | 1.007760766 | 10.21482866 | 2.481748299 | 0 | - | 0 | - |
| E012-T045-S136- | 1.007760766 | 9.133258569 | 2.335438898 | 0 | - | 0 | - |
| E012-T069-S116- | 1.007760766 | 9.013084114 | 2.318227124 | 0 | - | 0 | - |
| E012-T073-S192- | 1.007760766 | 0 | - | 19.53525566 | 3.354443628 | 0 | - |
| E013-T031-S176- | 1.007760766 | 11.05604985 | 2.586098006 | 0 | - | 0 | - |
| E014-T054-S098- | 1.007760766 | 11.8371838 | 2.676669461 | 0 | - | 0 | - |
| E013-T064-S077- | 1.012085919 | 9.013084114 | 2.315122588 | 0 | - | 0 | - |
| E014-T019-S100- | 1.012085919 | 10.21482866 | 2.478643763 | 0 | - | 0 | - |
| E011-T057-S195- | 1.016411073 | 8.592473522 | 2.250113096 | 0 | - | 0 | - |
| E012-T003-S115- | 1.016411073 | 9.433694706 | 2.371388438 | 0 | - | 0 | - |
| E012-T059-S193- | 1.016411073 | 9.313520251 | 2.354675156 | 0 | - | 0 | - |
| E013-T047-S077- | 1.016411073 | 8.892909659 | 2.294605121 | 0 | - | 0 | - |
| E014-T058-S197- | 1.016411073 | 10.99596262 | 2.572687245 | 0 | - | 0 | - |
| E011-T025-S100- | 1.020736226 | 7.510903428 | 2.074431267 | 0 | - | 0 | - |
| E011-T055-S138- | 1.020736226 | 9.373607479 | 2.359964767 | 0 | - | 0 | - |
| E011-T069-S165- | 1.020736226 | 9.493781933 | 2.376581796 | 0 | - | 0 | - |
| E012-T021-S175- | 1.020736226 | 8.832822432 | 2.282724576 | 19.16782138 | 3.319102327 | 0 | - |
| E013-T038-S121- | 1.020736226 | 9.073171341 | 2.31756504 | 0 | - | 0 | - |
| E013-T060-S083- | 1.020736226 | 7.510903428 | 2.074431267 | 0 | - | 0 | - |
| E013-T069-S183- | 1.020736226 | 7.390728973 | 2.053915141 | 0 | - | 0 | - |
| E014-T012-S174- | 1.020736226 | 8.712647977 | 2.26498366 | 0 | - | 0 | - |
| E014-T028-S069- | 1.020736226 | 8.231950157 | 2.191754421 | 0 | - | 0 | - |
| E014-T057-S098- | 1.020736226 | 9.133258569 | 2.32614526 | 0 | - | 0 | - |
| E012-T008-S157- | 1.02506138 | 11.47666044 | 2.623194285 | 0 | - | 0 | - |
| E013-T005-S098- | 1.02506138 | 9.193345796 | 2.331590128 | 0 | - | 0 | - |
| E013-T013-S183- | 1.02506138 | 9.493781933 | 2.373497174 | 0 | - | 0 | - |
| E013-T047-S171- | 1.02506138 | 0 | - | 7.961075972 | 2.145706333 | 0 | - |
| E014-T037-X001- | 1.02506138 | 8.352124612 | 2.207328516 | 0 | - | 0 | - |
| E014-T067-S186- | 1.02506138 | 18.74721496 | 3.285611656 | 0 | - | 0 | - |
| E014-T072-S081- | 1.02506138 | 9.734130843 | 2.406167837 | 0 | - | 0 | - |
| E011-T039-S100- | 1.029386533 | 9.193345796 | 2.328512086 | 0 | - | 0 | - |
| E013-T037-S161- | 1.029386533 | 8.592473522 | 2.240859199 | 0 | - | 0 | - |
| E013-T073-S054- | 1.029386533 | 0 | - | 8.45098834 | 2.219421529 | 0 | - |
| E013-T075-S058- | 1.029386533 | 13.03892835 | 2.790317229 | 0 | - | 0 | - |
| E013-T075-S171- | 1.029386533 | 25.35680997 | 3.699060185 | 0 | - | 0 | - |
| E014-T018-S190- | 1.029386533 | 0 | - | 9.675769259 | 2.395224446 | 0 | - |
| E014-T036-S169- | 1.029386533 | 9.974479753 | 2.435036967 | 0 | - | 0 | - |
| E012-T023-S069- | 1.033711687 | 9.974479753 | 2.431965478 | 0 | - | 0 | - |
| E012-T077-X001- | 1.033711687 | 14.48102181 | 2.928313626 | 7.164968375 | 2.005332131 | 0 | - |
| E013-T055-S085- | 1.033711687 | 0 | - | 15.0648053 | 2.981716424 | 0 | - |
| E014-T004-S142- | 1.033711687 | 8.592473522 | 2.237787711 | 0 | - | 0 | - |
| E014-T029-S169- | 1.033711687 | 10.03456698 | 2.439842944 | 6.858773146 | 1.95018894 | 0 | - |
| E014-T035-S103- | 1.033711687 | 9.193345796 | 2.325440598 | 0 | - | 0 | - |
| E011-T026-S155- | 1.03803684 | 10.87578816 | 2.542771229 | 0 | -1.02718013 | 0 | -1.02718013 |
| E013-T079-S137- | 1.03803684 | 8.292037385 | 2.188814828 | 0 | -1.02718013 | 0 | -1.02718013 |
| E011-T055-S085- | 1.042361994 | 0 | - | 14.02374152 | 2.878933646 | 0 | - |
| E012-T058-S059- | 1.042361994 | 8.231950157 | 2.176396839 | 0 | - | 0 | - |
| E013-T047-S130- | 1.042361994 | 0.901308411 | - | 30.98695725 | 3.969173262 | 0 | - |
| E014-T078-X001- | 1.042361994 | 8.111775703 | 2.157493638 | 0 | - | 0 | - |
| E011-T036-S186- | 1.046687147 | 23.07349533 | 3.556083131 | 0 | - | 0 | - |
| E013-T037-S154- | 1.046687147 | 8.592473522 | 2.228612286 | 0 | - | 0 | - |
| E011-T031-S186- | 1.051012301 | 25.05637384 | 3.667228274 | 7.103729329 | 1.982249844 | 0 | - |
| E011-T069-S064- | 1.051012301 | 0 | - | 13.71754629 | 2.843129116 | 0 | - |
| E014-T071-5059- | 1.051012301 | 8.17186293 | 2.16087865 | 0 | - | 0 | - |
| E014-T077-S059- | 1.051012301 | 7.210467291 | 2.00112819 | 0 | - | 0 | - |
| E011-T064-S198- | 1.055337454 | 8.592473522 | 2.222527596 | 0 | - | 0 | - |
| E012-T009-S176- | 1.055337454 | 8.111775703 | 2.148356953 | 0 | - | 0 | - |
| E013-T071-S183- | 1.055337454 | 10.21482866 | 2.447960393 | 0 | - | 0 | - |
| E014-T046-S083- | 1.055337454 | 7.93151402 | 2.119529472 | 0 | - | 0 | - |
| E011-T026-S085- | 1.059662608 | 7.991601248 | 2.126170028 | 0 | -1.04240803 | 0 | -1.04240803 |
| E012-T026-S143- | 1.059662608 | 7.691165111 | 2.077141564 | 0 | -1.04240803 | 0 | -1.04240803 |
| E013-T058-S104- | 1.059662608 | 0 | -1.04240803 | 7.777358835 | 2.091378859 | 0 | -1.04240803 |
| E014-T012-S148- | 1.059662608 | 9.914392525 | 2.4057519 | 0 | -1.04240803 | 0 | -1.04240803 |
| E012-T012-S177- | 1.063987761 | 8.472299067 | 2.198280216 | 0 | - | 0 | - |
| E012-T023-S085- | 1.068312915 | 11.8371838 | 2.633802369 | 0 | - | 0 | - |
| E012-T034-S197- | 1.068312915 | 8.352124612 | 2.176839686 | 0 | - | 0 | - |
| E013-T052-S156- | 1.068312915 | 0 | - | 91.12370036 | 5.477045989 | 0 | - |
| E014-T065-S083- | 1.068312915 | 8.832822432 | 2.249151123 | 0 | - | 0 | - |
| E011-T034-S077- | 1.072638068 | 8.532386295 | 2.201369207 | 0 | -1.05146821 | 0 | -1.05146821 |
| E012-T078-S120- | 1.072638068 | 8.41221184 | 2.183065582 | 0 | -1.05146821 | 0 | -1.05146821 |
| E013-T015-S099- | 1.072638068 | 8.352124612 | 2.173825944 | 0 | -1.05146821 | 0 | -1.05146821 |
| E013-T026-S104- | 1.072638068 | 8.952996887 | 2.263662783 | 8.818422616 | 2.244023057 | 0 | -1.05146821 |
| E014-T072-S174- | 1.072638068 | 9.914392525 | 2.39669172 | 0 | -1.05146821 | 0 | -1.05146821 |
| E011-T019-S106- | 1.076963222 | 9.493781933 | 2.336987141 | 0 | -1.05447567 | 0 | -1.05447567 |
| E011-T026-S198- | 1.076963222 | 0 | -1.05447567 | 36.00855901 | 4.155311389 | 0 | -1.05447567 |
| E012-T027-S058- | 1.076963222 | 0 | -1.05447567 | 7.593641697 | 2.048793957 | 0 | -1.05447567 |
| E012-T036-S130- | 1.076963222 | 8.231950157 | 2.152159765 | 0 | -1.05447567 | 0 | -1.05447567 |
| E012-T067-5121- | 1.076963222 | 0 | -1.05447567 | 7.654880743 | 2.059038271 | 0 | -1.05447567 |
| E013-T013-S098- | 1.081288375 | 8.352124612 | 2.16781728 | 0 | - | 0 | - |
| E013-T045-S137- | 1.081288375 | 9.433694706 | 2.325701347 | 0 | - | 0 | - |
| E014-T032-S141- | 1.081288375 | 105.693433 | 5.679850698 | 146.4837979 | 6.14693579 | 0 | - |
| E011-T001-S165- | 1.085613529 | 0 | - | 16.77949859 | 3.091670887 | 0 | - |
| E012-T066-5121- | 1.085613529 | 10.09465421 | 2.411320952 | 0 | - | 0 | - |
| E014-T032-S199- | 1.085613529 | 8.712647977 | 2.219392828 | 8.940900708 | 2.252904729 | 0 | - |
| E011-T007-S193- | 1.089938682 | 10.15474144 | 2.416124553 | 71.03729329 | 5.107211455 | 0 | - |
| E011-T058-5084- | 1.089938682 | 9.193345796 | 2.28609515 | 6.552577916 | 1.853508548 | 0 | - |
| E011-T080-S175- | 1.089938682 | 9.313520251 | 2.3030043 23 | 0 | - | 0 | - |
| E013-T029-S155- | 1.089938682 | 0 | - | 8.573466432 | 2.195580786 | 0 | - |
| E014-T030-S072- | 1.089938682 | 0 | - | 63.62736873 | 4.950612734 | 0 | - |
| E014-T021-S129- | 1.094263836 | 0 | - | 30.1296106 | 3.893772421 | 0 | - |
| E011-T001-S102- | 1.098588989 | 11.1762243 | 2.536575294 | 7.716119789 | 2.054266383 | 0 | - |
| E011-T003-S130- | 1.098588989 | 9.914392525 | 2.378740289 | 0 | - | 0 | - |
| E013-T033-S121- | 1.098588989 | 10.15474144 | 2.410165526 | 0 | - | 0 | - |
| E013-T062-S175- | 1.098588989 | 9.253433024 | 2.288615483 | 0 | - | 0 | - |
| E013-T065-S168- | 1.098588989 | 9.073171341 | 2.263026413 | 0 | - | 0 | - |
| E013-T068-X001- | 1.098588989 | 9.493781933 | 2.322043169 | 0 | - | 0 | - |
| E014-T035-S135- | 1.098588989 | 8.292037385 | 2.146575318 | 0 | - | 0 | - |
| E014-T037-S098- | 1.098588989 | 9.914392525 | 2.378740289 | 0 | - | 0 | - |
| E011-T007-S177- | 1.102914143 | 8.652560749 | 2.19852178 | 0 | - | 0 | - |
| E011-T057-5186- | 1.102914143 | 9.734130843 | 2.351743525 | 0 | - | 0 | - |
| E013-T010-S191- | 1.102914143 | 0 | - | 7.899836927 | 2.081388952 | 0 | - |
| E013-T036-S104- | 1.102914143 | 9.253433024 | 2.285645175 | 0 | - | 0 | - |
| E014-T013-S169- | 1.102914143 | 9.253433024 | 2.285645175 | 0 | - | 0 | - |
| E014-T039-S087- | 1.102914143 | 7.871426793 | 2.076776203 | 0 | - | 0 | - |
| E011-T066-S098- | 1.107239296 | 9.133258569 | 2.265672119 | 0 | - | 0 | - |
| E011-T069-S120- | 1.107239296 | 10.33500312 | 2.427358728 | 0 | - | 0 | - |
| E013-T030-S141- | 1.107239296 | 20.00904673 | 3.317584643 | 0 | - | 0 | - |
| E014-T077-S165- | 1.107239296 | 9.553869161 | 2.324345944 | 0 | - | 0 | - |
| E012-T059-S168- | 1.11156445 | 10.27491589 | 2.416732484 | 0 | - | 0 | - |
| E013-T061-S183- | 1.11156445 | 10.45517757 | 2.439615636 | 0 | - | 0 | - |
| E014-T022-S099- | 1.11156445 | 9.253433024 | 2.279722842 | 0 | - | 0 | - |
| E014-T040-S161- | 1.11156445 | 17.00468536 | 3.091988201 | 225.29845 | 6.743770611 | 0 | - |
| E011-T017-S083- | 1.115889603 | 7.691165111 | 2.038285237 | 0 | - | 0 | - |
| E011-T035-S116- | 1.115889603 | 10.27491589 | 2.413780409 | 0 | - | 0 | - |
| E011-T065-S063- | 1.115889603 | 8.111775703 | 2.106467877 | 0 | - | 0 | - |
| E012-T003-S196- | 1.115889603 | 9.553869161 | 2.318435742 | 7.042490283 | 1.926377931 | 0 | - |
| E012-T074-S099- | 1.115889603 | 8.832822432 | 2.216341233 | 0 | - | 0 | - |
| E013-T004-S109- | 1.115889603 | 8.352124612 | 2.144029797 | 0 | - | 0 | - |
| E013-T057-S117- | 1.115889603 | 9.073171341 | 2.251181698 | 0 | - | 0 | - |
| E012-T016-S058- | 1.120214757 | 10.33500312 | 2.41850248 | 0 | - | 0 | - |
| E012-T021-5174- | 1.120214757 | 0 | - | 18.8003871 | 3.223246328 | 0 | - |
| E012-T062-S186- | 1.120214757 | 11.29639876 | 2.535953548 | 92.71591556 | 5.46601177 | 0 | - |
| E012-T065-S136- | 1.120214757 | 7.991601248 | 2.084367655 | 0 | - | 0 | - |
| E013-T033-S195- | 1.120214757 | 10.15474144 | 2.395374765 | 0 | - | 0 | - |
| E013-T050-S143- | 1.120214757 | 7.751252338 | 2.045279084 | 0 | - | 0 | - |
| E013-T054-S054- | 1.120214757 | 11.05604985 | 2.507474979 | 0 | - | 0 | - |
| E013-T058-S183- | 1.120214757 | 0 | - | 8.022315018 | 2.089287256 | 0 | - |
| E014-T055-S186- | 1.120214757 | 0 | - | 15.79967385 | 2.986150917 | 0 | - |
| E014-T066-S058- | 1.120214757 | 0 | - | 79.61075972 | 5.248690111 | 0 | - |
| E012-T080-S137- | 1.267269976 | 13.21919003 | 2.648811192 | 12.55400442 | 2.579691051 | 0.067975984 | - |
| E011-T036-S059- | 1.12453991 | 0 | - | 7.899836927 | 2.066628456 | 0 | - |
| E012-T006-S195- | 1.12453991 | 8.832822432 | 2.210455144 | 0 | - | 0 | - |
| E012-T026-S135- | 1.12453991 | 9.013084114 | 2.236664054 | 0 | - | 0 | - |
| E014-T047-S098- | 1.12453991 | 27.1594268 | 3.728395617 | 0 | - | 0 | - |
| E014-T074-S098- | 1.12453991 | 10.69552648 | 2.460734457 | 0 | - | 0 | - |
| E013-T019-S165- | 1.128865064 | 0 | - | 27.92500495 | 3.764160792 | 0 | - |
| E014-T030-S195- | 1.128865064 | 9.974479753 | 2.365996136 | 0 | - | 0 | - |
| E014-T040-S141- | 1.128865064 | 11.65692212 | 2.571770203 | 0 | - | 0 | - |
| E011-T057-S054- | 1.133190217 | 15.20206854 | 2.925093494 | 75.01783128 | 5.155253345 | 0 | - |
| E013-T038-S189- | 1.133190217 | 0 | - | 39.62166272 | 4.251164771 | 0 | - |
| E011-T080-S106- | 1.137515371 | 8.772735204 | 2.192827608 | 2.816996113 | 0.836502923 | 0 | - |
| E012-T020-S130- | 1.137515371 | 17.90599377 | 3.144836986 | 0 | - | 0 | - |
| E012-T057-S120- | 1.137515371 | 0 | - | 8.14479311 | 2.097015737 | 0 | - |
| E014-T082-S158- | 1.137515371 | 7.570990656 | 2.00352717 | 0 | - | 0 | - |
| E012-T058-S174- | 1.141840524 | 8.41221184 | 2.135682727 | 0 | - | 0 | - |
| E013-T062-S165- | 1.141840524 | 0 | - | 12.30904823 | 2.635484434 | 0 | - |
| E014-T004-S120- | 1.141840524 | 9.854305298 | 2.341344423 | 0 | - | 0 | - |
| E014-T035-S142- | 1.141840524 | 10.21482866 | 2.38848461 | 0 | - | 0 | - |
| E014-T045-S154- | 1.141840524 | 7.871426793 | 2.050315087 | 0 | - | 0 | - |
| E014-T047-S141- | 1.141840524 | 11.23631153 | 2.514245773 | 0 | - | 0 | - |
| E011-T059-S189- | 1.146165678 | 10.5152648 | 2.42371423 | 0 | - | 0 | - |
| E012-T001-S072- | 1.146165678 | 0 | - | 10.65559399 | 2.441189172 | 0 | - |
| E012-T030-S142- | 1.146165678 | 8.111775703 | 2.085970782 | 0 | - | 0 | - |
| E013-T009-S102- | 1.146165678 | 9.613956388 | 2.306129169 | 0 | - | 0 | - |
| E013-T016-S180- | 1.146165678 | 10.03456698 | 2.362196659 | 0 | - | 0 | - |
| E013-T033-S103- | 1.146165678 | 10.21482866 | 2.385574222 | 0 | - | 0 | - |
| E013-T067-S145- | 1.146165678 | 9.914392525 | 2.346398478 | 0 | - | 0 | - |
| E014-T018-S195- | 1.150490831 | 7.691165111 | 2.014883613 | 0 | -1.10466598 | 0 | -1.10466598 |
| E014-T076-S106- | 1.150490831 | 9.433694706 | 2.27851224 | 0 | -1.10466598 | 0 | -1.10466598 |
| E012-T037-S194- | 1.154815985 | 7.691165111 | 2.011984921 | 0 | - | 0 | - |
| E012-T046-S141- | 1.154815985 | 20.3695701 | 3.309921307 | 0 | - | 0 | - |
| E013-T012-S121- | 1.154815985 | 8.472299067 | 2.136149959 | 0 | - | 0 | - |
| E013-T017-S147- | 1.154815985 | 9.313520251 | 2.258900266 | 0 | - | 0 | - |
| E013-T053-S183- | 1.154815985 | 10.81570094 | 2.455068639 | 0 | - | 0 | - |
| E013-T073-S154- | 1.159141138 | 9.013084114 | 2.213356948 | 0 | - | 0 | - |
| E014-T051-S155- | 1.159141138 | 8.41221184 | 2.124076239 | 0 | - | 0 | - |
| E011-T015-S081- | 1.163466292 | 8.111775703 | 2.074387591 | 0 | - | 0 | - |
| E011-T047-S085- | 1.163466292 | 8.712647977 | 2.166520031 | 0 | - | 0 | - |
| E012-T024-S165- | 1.163466292 | 9.253433024 | 2.244690482 | 0 | - | 0 | - |
| E013-T002-S169- | 1.163466292 | 19.10773832 | 3.216334271 | 0 | - | 0 | - |
| E013-T058-S141- | 1.163466292 | 17.66564486 | 3.108968804 | 0 | - | 0 | - |
| E014-T021-S084- | 1.167791445 | 9.133258569 | 2.224800306 | 0 | - | 0 | - |
| E011-T010-S130- | 1.172116599 | 9.79421807 | 2.313085285 | 0 | - | 0 | - |
| E011-T076-S171- | 1.172116599 | 9.433694706 | 2.264076671 | 0 | - | 0 | - |
| E012-T042-S106- | 1.172116599 | 7.871426793 | 2.030064603 | 0 | - | 0 | - |
| E013-T006-S191- | 1.172116599 | 0 | - | 29.63969824 | 3.818228635 | 0 | - |
| E013-T007-S143- | 1.172116599 | 19.82878505 | 3.261405235 | 0 | - | 0 | - |
| E014-T009-S063- | 1.172116599 | 9.493781933 | 2.272361262 | 0 | - | 0 | - |
| E011-T025-S169- | 1.176441752 | 9.253433024 | 2.236063714 | 0 | -1.12197141 | 0 | -1.12197141 |
| E013-T082-S199- | 1.176441752 | 10.15474144 | 2.357613757 | 0 | -1.12197141 | 0 | -1.12197141 |
| E012-T021-S099- | 1.180766906 | 8.231950157 | 2.081799861 | 0 | - | 0 | - |
| E012-T042-S069- | 1.180766906 | 8.532386295 | 2.128001843 | 0 | - | 0 | - |
| E013-T056-S115- | 1.180766906 | 8.892909659 | 2.181559329 | 0 | - | 0 | - |
| E013-T079-S169- | 1.180766906 | 0 | - | 22.41349081 | 3.424432569 | 0 | - |
| E014-T038-S189- | 1.180766906 | 8.892909659 | 2.181559329 | 0 | - | 0 | - |
| E014-T063-S137- | 1.180766906 | 10.99596262 | 2.459641453 | 0 | - | 0 | - |
| E011-T044-S121- | 1.185092059 | 8.952996887 | 2.18743693 | 0 | - | 0 | - |
| E012-T073-S180- | 1.185092059 | 9.373607479 | 2.247151719 | 0 | - | 0 | - |
| E012-T029-S175- | 1.189417213 | 9.313520251 | 2.235918039 | 0 | - | 0 | - |
| E012-T051-S098- | 1.189417213 | 10.87578816 | 2.43940446 | 0 | - | 0 | - |
| E011-T028-S168- | 1.193742366 | 15.32224299 | 2.895373315 | 0 | - | 0 | - |
| E011-T071-S120- | 1.193742366 | 0 | - | 22.47472986 | 3.419642546 | 0 | - |
| E013-T032-S126- | 1.193742366 | 9.674043616 | 2.282640801 | 0 | - | 0 | - |
| E013-T070-S085- | 1.193742366 | 10.21482866 | 2.353941569 | 0 | - | 0 | - |
| E013-T072-S115- | 1.193742366 | 8.111775703 | 2.054338129 | 0 | - | 0 | - |
| E012-T020-S062- | 1.19806752 | 9.734130843 | 2.287897771 | 0 | - | 0 | - |
| E013-T079-S121- | 1.19806752 | 9.253433024 | 2.221799421 | 0 | - | 0 | - |
| E014-T014-S143- | 1.202392673 | 26.37829284 | 3.63588887 | 0 | - | 0 | - |
| E013-T050-S169- | 1.206717827 | 9.193345796 | 2.207653601 | 0 | - | 0 | - |
| E013-T021-S136- | 1.21104298 | 14.84154517 | 2.840914087 | 0 | -1.14472707 | 0 | -1.14472707 |
| E013-T082-S161- | 1.21104298 | 10.33500312 | 2.357985813 | 0 | -1.14472707 | 0 | -1.14472707 |
| E014-T021-S154- | 1.21104298 | 9.073171341 | 2.187718984 | 0 | -1.14472707 | 0 | -1.14472707 |
| E014-T028-S165- | 1.21104298 | 0 | -1.14472707 | 34.7837781 | 4.016506742 | 0 | -1.14472707 |
| E014-T029-S117- | 1.21104298 | 0 | -1.14472707 | 43.96963499 | 4.346152199 | 0 | -1.14472707 |
| E011-T027-S176- | 1.215368134 | 9.553869161 | 2.252153643 | 0 | - | 0 | - |
| E013-T029-S154- | 1.215368134 | 9.253433024 | 2.210488669 | 0 | - | 0 | - |
| E013-T070-S170- | 1.215368134 | 9.013084114 | 2.176268044 | 0 | - | 0 | - |
| E011-T080-X001- | 1.219693287 | 10.39509034 | 2.359980116 | 0 | - | 0 | - |
| E012-T007-S083- | 1.219693287 | 10.09465421 | 2.321432456 | 45.74556732 | 4.39639732 | 0 | - |
| E013-T044-S082- | 1.219693287 | 10.03456698 | 2.31359777 | 0 | - | 0 | - |
| E014-T067-S085- | 1.219693287 | 10.5152648 | 2.37511534 | 4.164255124 | 1.218199933 | 0 | - |
| E012-T045-S115- | 1.224018441 | 19.34808723 | 3.193652529 | 0 | -1.15316875 | 0 | -1.15316875 |
| E013-T044-S137- | 1.224018441 | 9.974479753 | 2.302911895 | 0 | -1.15316875 | 0 | -1.15316875 |
| E013-T070-S176- | 1.224018441 | 9.974479753 | 2.302911895 | 0 | -1.15316875 | 0 | -1.15316875 |
| E014-T017-S116- | 1.224018441 | 8.472299067 | 2.090545881 | 0 | -1.15316875 | 0 | -1.15316875 |
| E011-T044-S189- | 1.228343594 | 9.854305298 | 2.284223785 | 0 | - | 0 | - |
| E012-T050-S109- | 1.228343594 | 19.34808723 | 3.1908495 76 | 0 | - | 0 | - |
| E013-T039-S104- | 1.228343594 | 9.253433024 | 2.202063422 | 0 | - | 0 | - |
| E011-T030-S106- | 1.232668748 | 10.27491589 | 2.336275546 | 0 | -1.15876922 | 0 | -1.15876922 |
| E013-T073-S058- | 1.232668748 | 0 | -1.15876922 | 29.94589347 | 3.792906851 | 0 | -1.15876922 |
| E014-T039-S105- | 1.232668748 | 11.41657321 | 2.475425941 | 0 | -1.15876922 | 0 | -1.15876922 |
| E012-T057-S194- | 1.236993901 | 10.69552648 | 2.386323579 | 0 | - | 0 | - |
| E012-T065-S077- | 1.236993901 | 11.47666044 | 2.479598599 | 0 | - | 0 | - |
| E011-T072-S197- | 1.241319055 | 8.111775703 | 2.023384201 | 0 | - | 0 | - |
| E013-T057-S137- | 1.241319055 | 0 | - | 15.55471767 | 2.884822469 | 0 | - |
| E013-T060-S194- | 1.249969362 | 9.013084114 | 2.153909144 | 0 | - | 0 | - |
| E012-T081-S064- | 1.254294515 | 26.31820561 | 3.599114808 | 0 | - | 0 | - |
| E011-T016-S083- | 1.258619669 | 9.313520251 | 2.191023584 | 0 | - | 0 | - |
| E012-T033-S143- | 1.262944822 | 10.93587539 | 2.399031067 | 0 | - | 0 | - |
| E014-T045-S100- | 1.262944822 | 17.18494704 | 3.006471412 | 0 | - | 0 | - |
| E014-T074-S100- | 1.262944822 | 8.472299067 | 2.065513224 | 0 | - | 0 | - |
| E014-T001-S170- | 1.267269976 | 9.734130843 | 2.243177283 | 0 | - | 0 | - |
| E012-T075-S059- | 1.271595129 | 10.15474144 | 2.295879444 | 0 | - | 0 | - |
| E012-T066-S186- | 1.275920283 | 10.15474144 | 2.293135141 | 0 | - | 0 | - |
| E013-T029-S141- | 1.275920283 | 12.73849221 | 2.593701747 | 0 | - | 0 | - |
| E013-T014-S143- | 1.28457059 | 0 | -1.19192302 | 11.57417968 | 2.460469359 | 0 | -1.19192302 |
| E012-T050-S177- | 1.288895743 | 10.93587539 | 2.382580723 | 0 | - | 0 | - |
| E014-T044-S195- | 1.288895743 | 0 | - | 53.27796997 | 4.567643108 | 0 | - |
| E014-T064-S083- | 1.288895743 | 8.952996887 | 2.120479241 | 0 | - | 0 | - |
| E014-T063-S142- | 1.29754605 | 8.712647977 | 2.079770896 | 0 | - | 0 | - |
| E011-T005-S064- | 1.301871204 | 8.712647977 | 2.077057561 | 0 | - | 0 | - |
| E013-T051-S106- | 1.301871204 | 10.57535203 | 2.330177051 | 7.471163605 | 1.87975304 | 0 | - |
| E012-T033-S102- | 1.353773046 | 8.832822432 | 2.06263037 | 0 | -1.23497522 | 0 | -1.23497522 |
| E011-T048-S072- | 1.362423353 | 9.133258569 | 2.100758751 | 0 | - | 0 | - |
| E011-T070-S059- | 1.37107366 | 9.974479753 | 2.210540163 | 0 | - | 0 | - |
| E012-T082-S177- | 1.37107366 | 8.832822432 | 2.052065108 | 0 | - | 0 | - |
| E013-T082-S100- | 1.37107366 | 21.63140187 | 3.254713566 | 0 | - | 0 | - |
| E013-T038-S126- | 1.379723967 | 21.45114019 | 3.23792257 | 0 | -1.25079424 | 0 | -1.25079424 |
| E013-T046-S098- | 1.379723967 | 8.892909659 | 2.055600663 | 0 | -1.25079424 | 0 | -1.25079424 |
| E014-T072-S186- | 1.379723967 | 12.37796885 | 2.490992946 | 0 | -1.25079424 | 0 | -1.25079424 |
| E011-T066-S085- | 1.392699427 | 8.472299067 | 1.985075456 | 162.2834717 | 6.092595777 | 0 | - |
| E011-T078-S082- | 1.401349734 | 10.09465421 | 2.207947264 | 0 | - | 0 | - |
| E014-T026-S121- | 1.401349734 | 10.03456698 | 2.200112577 | 0 | - | 0 | - |
| E013-T045-S176- | 1.410000041 | 16.644162 | 2.872085835 | 7.042490283 | 1.738609117 | 0 | - |
| E014-T054-S145- | 1.414325195 | 9.79421807 | 2.160566822 | 0 | - | 0 | - |
| E013-T039-S116- | 1.431625809 | 9.734130843 | 2.142212237 | 0 | - | 0 | - |
| E013-T004-S177- | 1.448926423 | 9.734130843 | 2.131984045 | 0 | - | 0 | - |
| E011-T057-S104- | 1.45757673 | 9.553869161 | 2.102463637 | 0 | - | 0 | - |
| E013-T021-S083- | 1.461901883 | 18.92747664 | 3.016913866 | 3.368147527 | 0.827248316 | 0 | - |
| E013-T054-S116- | 1.461901883 | 16.52398754 | 2.831485923 | 0 | - | 0 | - |
| E013-T013-S142- | 1.47055219 | 9.493781933 | 2.086629277 | 0 | - | 0 | - |
| E013-T050-X001- | 1.47055219 | 12.55823053 | 2.456263468 | 0 | - | 0 | - |
| E014-T059-S186- | 1.474877344 | 0 | - | 15.92215194 | 2.773484112 | 0 | - |
| E011-T040-S154- | 1.479202497 | 9.734130843 | 2.114257361 | 0 | - | 0 | - |
| E012-T019-S058- | 1.487852804 | 9.193345796 | 2.034654635 | 0 | -1.31490113 | 0 | -1.31490113 |
| E014-T077-S183- | 1.505153418 | 20.00904673 | 3.068039839 | 12.61524346 | 2.442251917 | 0 | - |
| E012-T022-S069- | 1.509478572 | 10.15474144 | 2.15219754 | 0 | - | 0 | - |
| E014-T013-S083- | 1.509478572 | 9.854305298 | 2.112807861 | 0 | - | 0 | - |
| E013-T038-S100- | 1.513803725 | 11.41657321 | 2.304323151 | 0 | -1.32987201 | 0 | -1.32987201 |
| E012-T011-S202- | 1.522454032 | 11.1762243 | 2.271166957 | 0 | - | 0 | - |
| E013-T046-S168- | 1.522454032 | 0 | - | 14.2686977 | 2.597677133 | 0 | - |
| E013-T072-S098- | 1.526779186 | 38.03521496 | 3.949404716 | 265.1038299 | 6.718545871 | 0 | - |
| E014-T055-S115- | 1.526779186 | 10.99596262 | 2.247177433 | 0 | - | 0 | - |
| E013-T002-S102- | 1.535429493 | 9.493781933 | 2.049232655 | 0 | - | 0 | - |
| E014-T003-S143- | 1.535429493 | 6.429333335 | 1.551002602 | 67.30171149 | 4.751619669 | 0 | - |
| E014-T061-S109- | 1.539754646 | 9.914392525 | 2.103470798 | 0 | - | 0 | - |
| E012-T047-S064- | 1.5440798 | 9.974479753 | 2.108936721 | 0 | - | 0 | - |
| E014-T019-S063- | 1.55705526 | 18.44677882 | 2.926975962 | 0 | - | 0 | - |
| E011-T002-S147- | 1.570030721 | 19.10773832 | 2.967893309 | 0 | - | 0 | - |
| E013-T065-S183- | 1.574355874 | 11.29639876 | 2.255952448 | 0 | - | 0 | - |
| E013-T017-S165- | 1.583006181 | 5.107414331 | 1.241510626 | 146.3000808 | 5.833563315 | 0 | - |
| E014-T070-S109- | 1.608957102 | 19.88887228 | 3.00118948 | 0 | - | 0 | - |
| E011-T044-X001- | 1.613282256 | 17.54547041 | 2.827132001 | 0 | - | 0 | - |
| E013-T017-S109- | 1.613282256 | 14.96171963 | 2.610681227 | 0 | - | 0 | - |
| E011-T037-S177- | 1.617607409 | 9.914392525 | 2.059911193 | 0 | - | 0 | - |
| E013-T007-S175- | 1.617607409 | 18.08625546 | 2.866213445 | 0 | - | 0 | - |
| E011-T030-S138- | 1.621932563 | 9.613956388 | 2.017260042 | 0 | - | 0 | - |
| E011-T038-S157- | 1.64355833 | 18.80730218 | 2.905479329 | 0 | -1.40248116 | 0 | -1.40248116 |
| E012-T030-S143- | 1.647883484 | 14.2406729 | 2.525015057 | 0 | -1.40483964 | 0 | -1.40483964 |
| E011-T060-S186- | 1.660858944 | 11.05604985 | 2.179793349 | 0 | - | 0 | - |
| E013-T069-S137- | 1.691135019 | 10.39509034 | 2.082125683 | 0 | - | 0 | - |
| E013-T057-S072- | 1.695460172 | 10.09465421 | 2.041261205 | 0 | - | 0 | - |
| E013-T033-S197- | 1.71708594 | 13.15910281 | 2.381597749 | 0 | - | 0 | - |
| E014-T070-S135- | 1.721411093 | 18.32660437 | 2.828161372 | 0 | - | 0 | - |
| E014-T068-S186- | 1.751687168 | 19.04765109 | 2.865044843 | 0 | - | 0 | - |
| E013-T028-S115- | 1.756012321 | 30.04361371 | 3.493642265 | 0 | - | 0 | - |
| E013-T016-S109- | 1.760337475 | 17.54547041 | 2.748150298 | 0 | -1.46484466 | 0 | -1.46484466 |
| E011-T030-S104- | 1.764662628 | 14.60119626 | 2.496481312 | 0 | - | 0 | - |
| E012-T013-S069- | 1.768987782 | 10.21482866 | 2.017976987 | 0 | - | 0 | - |
| E012-T069-S154- | 1.768987782 | 10.15474144 | 2.01022648 | 0 | - | 0 | - |
| E013-T068-S102- | 1.794938703 | 11.29639876 | 2.137347308 | 0 | - | 0 | - |
| E014-T020-S142- | 1.799263856 | 14.30076013 | 2.450483941 | 0 | - | 0 | - |
| E013-T062-S186- | 1.993895764 | 32.92780063 | 3.502372012 | 33.13032385 | 3.510958206 | 0.067975984 | - |
| E014-T002-S104- | 1.80358901 | 20.42965732 | 2.934262001 | 0 | - | 0 | - |
| E011-T056-S129- | 1.833865084 | 0 | - | 82.06032156 | 4.873316476 | 0 | - |
| E014-T067-S054- | 1.833865084 | 0 | - | 12.49276537 | 2.251343082 | 0 | - |
| E014-T006-S141- | 1.838190238 | 15.74285359 | 2.560502238 | 0 | - | 0 | - |
| E013-T020-S117- | 1.864141159 | 0 | - | 40.35653128 | 3.851940684 | 0 | - |
| E012-T080-S142- | 1.868466312 | 10.63543925 | 2.020174196 | 0 | - | 0 | - |
| E014-T073-S145- | 1.868466312 | 17.966081 | 2.725070123 | 0 | - | 0 | - |
| E013-T019-S101- | 1.872791466 | 0 | - | 30.74200106 | 3.465867903 | 0 | - |
| E014-T022-S186- | 1.89009208 | 0 | - | 18.31047474 | 2.740196269 | 0 | - |
| E013-T028-S195- | 1.898742387 | 14.84154517 | 2.450214031 | 0 | - | 0 | - |
| E012-T042-S194- | 1.907392694 | 10.75561371 | 2.015552007 | 0 | - | 0 | - |
| E012-T057-S082- | 1.911717847 | 17.84590655 | 2.694308728 | 7.532402651 | 1.551081488 | 0 | - |
| E012-T017-S186- | 1.920368154 | 0 | - | 22.84216414 | 3.029293036 | 0 | - |
| E012-T047-S176- | 1.929018461 | 13.69988785 | 2.327315957 | 0 | - | 0 | - |
| E014-T028-S138- | 1.929018461 | 20.48974455 | 2.875159142 | 0 | - | 0 | - |
| E012-T017-S168- | 1.941993922 | 10.99596262 | 2.027682761 | 0 | - | 0 | - |
| E014-T037-S121- | 1.954969382 | 27.33968848 | 3.261608812 | 16.71825954 | 2.584021808 | 0 | - |
| E013-T029-S145- | 1.972269996 | 0 | - | 36.74342757 | 3.666588364 | 0 | - |
| E013-T063-S175- | 1.972269996 | 11.05604985 | 2.020120208 | 0 | - | 0 | - |
| E014-T061-S116- | 1.998220917 | 18.6270405 | 2.710664056 | 0 | - | 0 | - |
| E012-T036-S183- | 2.006871224 | 19.46826169 | 2.767053596 | 0 | - | 0 | - |
| E014-T011-S177- | 2.045797606 | 18.38669159 | 2.670174641 | 0 | - | 0 | - |
| E011-T045-S174- | 2.050122759 | 17.48538318 | 2.599445736 | 0 | - | 0 | - |
| E013-T045-S186- | 2.050122759 | 0 | - | 59.34063552 | 4.306190678 | 0 | - |
| E014-T019-S177- | 2.06309822 | 21.09061683 | 2.850370169 | 0 | - | 0 | - |
| E014-T052-S165- | 2.071748527 | 0 | - | 17.08569382 | 2.557716927 | 0 | - |
| E014-T074-S169- | 2.071748527 | 16.76433645 | 2.531851783 | 0 | - | 0 | - |
| E014-T045-S085- | 2.07607368 | 13.94023676 | 2.280041046 | 0 | -1.62109006 | 0 | -1.62109006 |
| E012-T024-S157- | 2.080398834 | 13.51962617 | 2.236815249 | 0 | - | 0 | - |
| E014-T079-S098- | 2.080398834 | 17.84590655 | 2.613062134 | 0 | - | 0 | - |
| E012-T001-S168- | 2.110674908 | 12.73849221 | 2.142924144 | 0 | - | 0 | - |
| E014-T019-S121- | 2.110674908 | 17.66564486 | 2.585085818 | 0 | - | 0 | - |
| E012-T006-S186- | 2.115000062 | 0 | - | 25.23048693 | 3.073940492 | 0 | - |
| E013-T030-S192- | 2.115000062 | 18.68712773 | 2.659948546 | 0 | - | 0 | - |
| E013-T046-S120- | 2.115000062 | 11.47666044 | 2.00192773 | 0 | - | 0 | - |
| E013-T001-S190- | 2.119325215 | 76.43095329 | 4.633604525 | 52.91053569 | 4111261367 | 0 | - |
| E013-T064-S115- | 2.119325215 | 21.45114019 | 2.847482836 | 2.449561838 | 0.145179149 | 0 | - |
| E013-T011-S141- | 2.127975522 | 23.85462929 | 2.990213457 | 66.81179912 | 4.438235193 | 0 | - |
| E012-T001-S177- | 2.14960129 | 37.91504051 | 3.627086745 | 0 | - | 0 | - |
| E013-T045-S146- | 2.171227057 | 15.9832025 | 2.420995449 | 0 | - | 0 | - |
| E012-T014-S058- | 2.179877364 | 15.38233022 | 2.365097548 | 0 | - | 0 | - |
| E012-T032-S186- | 2.188527671 | 5.227588786 | 0.965783283 | 11.94161396 | 2.021055242 | 0 | - |
| E011-T072-S199- | 2.192852825 | 12.85866667 | 2.117870505 | 0 | - | 0 | - |
| E012-T069-X001- | 2.192852825 | 29.92343926 | 3.275782824 | 0 | - | 0 | - |
| E013-T017-S115- | 2.197177978 | 11.89727103 | 2.012194872 | 0 | - | 0 | - |
| E014-T044-S102- | 2.210153439 | 15.32224299 | 2.346125163 | 0 | - | 0 | - |
| E013-T027-S072- | 2.218803746 | 0 | - | 33.49775813 | 3.421906088 | 0 | - |
| E014-T010-S084- | 2.231779206 | 18.74721496 | 2.611248655 | 0 | - | 0 | - |
| E013-T046-S064- | 2.240429513 | 9.613956388 | 1.711705568 | 16.59578145 | 2.44097263 | 0 | - |
| E014-T047-S177- | 2.24907982 | 19.40817446 | 2.651044045 | 0 | - | 0 | - |
| E014-T055-S059- | 2.257730127 | 12.43805608 | 2.044385456 | 0 | - | 0 | - |
| E011-T003-S169- | 2.262055281 | 18.38669159 | 2.571213487 | 0 | - | 0 | - |
| E012-T024-S085- | 2.266380434 | 18.92747664 | 2.608994301 | 0 | - | 0 | - |
| E014-T029-S137- | 2.275030741 | 14.36084735 | 2.229677448 | 0 | - | 0 | - |
| E011-T019-S186- | 2.292331355 | 0 | - | 102.636641 | 4.976280809 | 0 | - |
| E014-T009-S183- | 2.300981662 | 18.26651714 | 2.545128766 | 0 | - | 0 | - |
| E014-T021-S077- | 2.300981662 | 17.12485982 | 2.457002808 | 0 | - | 0 | - |
| E013-T033-S137- | 2.305306816 | 19.82878505 | 2.655722587 | 0 | - | 0 | - |
| E014-T048-S115- | 2.305306816 | 18.74721496 | 2.578793096 | 0 | - | 0 | - |
| E013-T055-S177- | 2.32260743 | 18.50686605 | 2.553594263 | 0 | - | 0 | - |
| E013-T013-S109- | 2.326932583 | 18.98756387 | 2.586838107 | 0 | - | 0 | - |
| E012-T069-S168- | 2.331257737 | 13.39945172 | 2.111874996 | 0 | - | 0 | - |
| E012-T006-S085- | 2.33558289 | 0 | - | 17.75932332 | 2.49159699 | 0 | - |
| E012-T069-S143- | 2.339908044 | 21.0305296 | 2.72162389 | 0 | - | 0 | - |
| E014-T072-S143- | 2.348558351 | 30.46422431 | 3.232100363 | 0 | - | 0 | - |
| E014-T072-S142- | 2.357208658 | 31.84623054 | 3.290393703 | 0 | - | 0 | - |
| E013-T012-S137- | 2.365858965 | 41.88079752 | 3.671285106 | 0 | - | 0 | - |
| E014-T014-S168- | 2.391809886 | 0 | - | 58.17709365 | 4.124911631 | 0 | - |
| E014-T016-S054- | 2.426411114 | 13.5797134 | 2.089192193 | 15.12604435 | 2.234622428 | 0 | - |
| E012-T055-S054- | 2.443711728 | 22.53271028 | 2.772631201 | 120.2122472 | 5.137427284 | 0 | -1.78396438 |
| E013-T019-S170- | 2.486963263 | 18.32660437 | 2.470545112 | 0 | - | 0 | - |
| E014-T015-S180- | 2.491288416 | 17.60555764 | 2.413902182 | 0 | - | 0 | - |
| E013-T057-S077- | 2.504263877 | 20.79018069 | 2.636494838 | 0 | - | 0 | - |
| E011-T019-S105- | 2.530214798 | 18.20642991 | 2.443761503 | 15.30976149 | 2.207907811 | 0 | - |
| E013-T047-S186- | 2.538865105 | 15.02180686 | 2.178678182 | 6.981251237 | 1.173328169 | 0 | -1.82328677 |
| E013-T048-S098- | 2.56 | 19.23 | 2.51 | 49.24 | 3.82 | 0.00 | -1.83 |
| E011-T019-S059- | 2.56 | 13.82 | 2.06 | 0.00 | -1.83 | 0.00 | -1.83 |
| E013-T065-S084- | 2.57 | 16.34 | 2.28 | 0.00 | -1.84 | 0.00 | -1.84 |
| E011-T029-S143- | 2.60 | 16.16 | 2.25 | 0.00 | -1.85 | 0.00 | -1.85 |
| E014-T044-S175- | 2.62 | 13.58 | 2.01 | 0.00 | -1.86 | 0.00 | -1.86 |
| E013-T030-S101- | 2.63 | 0.00 | -1.86 | 51.26 | 3.85 | 0.00 | -1.86 |
| E013-T027-S186- | 2.65 | 8.71 | 1.41 | 98.90 | 4.78 | 0.00 | -1.87 |
| E013-T001-X001- | 2.68 | 20.13 | 2.52 | 0.00 | -1.88 | 0.00 | -1.88 |
| E014-T047-S186- | 2.69 | 18.93 | 2.43 | 82.67 | 4.50 | 0.00 | -1.88 |
| E012-T026-S180- | 2.71 | 14.84 | 2.09 | 0.00 | -1.89 | 0.00 | -1.89 |
| E014-T081-S102- | 2.73 | 16.22 | 2.21 | 0.00 | -1.90 | 0.00 | -1.90 |
| E014-T035-S199- | 2.74 | 0.00 | -1.90 | 30.19 | 3.06 | 0.00 | -1.90 |
| E012-T029-S157- | 2.75 | 14.60 | 2.06 | 0.00 | -1.91 | 0.00 | -1.91 |
| E012-T020-S156- | 2.75512278 | 17.18494704 | 2.27581274 | 0 | -1.90886008 | 0 | -1.90886008 |
| E012-T051-S116- | 2.798374315 | 15.02180686 | 2.076582867 | 0 | - | 0 | - |
| E012-T057-S143- | 2.811349775 | 14.42093458 | 2.016516284 | 0 | - | 0 | - |
| E013-T005-S059- | 2.832975543 | 10.81570094 | 1.624168518 | 15.92215194 | 2.142376346 | 0 | - |
| E012-T039-S126- | 2.837300696 | 0 | - | 44.21459117 | 3.558624693 | 0 | - |
| E012-T070-S130- | 2.837300696 | 19.40817446 | 2.410983411 | 0 | - | 0 | - |
| E013-T016-S136- | 2.850276157 | 18.02616823 | 2.304951209 | 0 | - | 0 | - |
| E013-T032-S087- | 2.85460131 | 0 | - | 21.43366608 | 2.541011851 | 0 | - |
| E012-T031-S116- | 2.876227078 | 18.50686605 | 2.33125702 | 0 | - | 0 | - |
| E012-T078-S069- | 2.880552231 | 19.22791278 | 2.382013584 | 0 | - | 0 | - |
| E013-T021-S137- | 2.884877385 | 18.38669159 | 2.319125654 | 0 | - | 0 | - |
| E012-T001-S120- | 2.902177999 | 17.90599377 | 2.276492192 | 0 | - | 0 | - |
| E013-T063-S130- | 2.915153459 | 19.40817446 | 2.382006375 | 0 | - | 0 | - |
| E013-T046-S195- | 2.932454073 | 25.71733334 | 2.764274198 | 0 | - | 0 | - |
| E012-T016-S186- | 2.936779227 | 20.73009346 | 2.464606666 | 0 | - | 0 | - |
| E014-T044-S158- | 2.954079841 | 0 | - | 76.97748075 | 4.301643638 | 0 | - |
| E013-T056-X001- | 2.997331376 | 19.34808723 | 2.347784103 | 0 | - | 0 | - |
| E014-T077-S085- | 3.001656529 | 0 | - | 36.80466661 | 3.239895084 | 0 | - |
| E013-T046-X001- | 3.01463199 | 0 | - | 20.69879753 | 2.434275445 | 0 | - |
| E013-T071-S085- | 3.01463199 | 20.54983178 | 2.424336956 | 0 | - | 0 | - |
| E013-T019-X001- | 3.023282297 | 18.56695327 | 2.28197426 | 0 | - | 0 | - |
| E014-T073-S137- | 3.031932604 | 18.02616823 | 2.238441611 | 0 | - | 0 | - |
| E014-T057-S121- | 3.036257757 | 15.80294081 | 2.057623539 | 0 | - | 0 | - |
| E014-T030-S145- | 3.044908064 | 16.94459813 | 2.149370797 | 0 | - | 0 | - |
| E014-T045-S186- | 3.049233218 | 14.48102181 | 1.934780055 | 479.3792516 | 6.890381292 | 0 | - |
| E012-T075-S137- | 3.057883525 | 20.30948287 | 2.392696222 | 0 | - | 0 | - |
| E012-T030-S199- | 3.079509292 | 20.12922119 | 2.372772059 | 0 | - | 0 | - |
| E012-T002-S143- | 3.088159599 | 42.12114643 | 3.39887211 | 20.27012421 | 2.379305033 | 0 | - |
| E014-T014-S193- | 3.109785367 | 16.70424922 | 2.106960709 | 0 | - | 0 | - |
| E012-T078-S137- | 3.127085981 | 18.80730218 | 2.262836994 | 0 | - | 0 | - |
| E014-T032-S109- | 3.127085981 | 19.10773832 | 2.284555419 | 0 | - | 0 | - |
| E014-T002-S194- | 3.131411134 | 18.32660437 | 2.225881641 | 0 | - | 0 | - |
| E012-T020-S109- | 3.140061441 | 21.15070405 | 2.419628472 | 0 | - | 0 | - |
| E013-T020-S186- | 3.153036902 | 16.88451091 | 2.106472069 | 95.22671644 | 4.534198904 | 0 | - |
| E014-T042-S186- | 3.161687209 | 0 | - | 21.67862226 | 2.446092557 | 0 | - |
| E012-T006-S121- | 3.166012362 | 9.974479753 | 1.397413525 | 41.88750742 | 3.363818445 | 0 | - |
| E014-T077-S186- | 3.191963283 | 18.92747664 | 2.249061052 | 54.44151184 | 3.725268613 | 0 | - |
| E014-T049-S137- | 3.222239358 | 18.32660437 | 2.19450791 | 0 | - | 0 | - |
| E013-T061-S195- | 3.226564512 | 0 | -2.07948547 | 21.80110036 | 2.431546073 | 0 | -2.07948547 |
| E013-T038-S183- | 3.235214819 | 0 | - | 29.82341537 | 2.863519675 | 0 | - |
| E013-T007-S058- | 3.239539972 | 0 | - | 35.51864665 | 3.106653668 | 0 | - |
| E014-T030-X001- | 3.26116574 | 13.45953894 | 1.762701479 | 68.58773146 | 4.029512905 | 0 | - |
| E014-T034-S186- | 3.265490893 | 0.180261682 | -1.85360502 | 315.0748914 | 6.21141084 | 0 | - |
| E011-T013-S177- | 3.278466354 | 19.46826169 | 2.258222933 | 0 | - | 0 | - |
| E014-T034-S109- | 3.300092121 | 20.85026792 | 2.345211497 | 0 | - | 0 | - |
| E012-T006-X001- | 3.304417275 | 17.18494704 | 2.078854878 | 0 | - | 0 | - |
| E013-T080-S183- | 3.304417275 | 13.94023676 | 1.795313165 | 16.28958622 | 2.006013496 | 0 | - |
| E014-T035-S109- | 3.304417275 | 0 | - | 49.48114912 | 3.551854905 | 0 | - |
| E012-T047-S121- | 3.321717889 | 20.3695701 | 2.30588108 | 7.042490283 | 0.896037388 | 0 | -2.1116049 |
| E012-T014-S083- | 3.343343656 | 19.10773832 | 2.210872807 | 0 | - | 0 | - |
| E014-T023-S136- | 3.34766881 | 17.72573209 | 2.106708172 | 0 | - | 0 | - |
| E014-T073-S058- | 3.34766881 | 22.23227415 | 2.417816433 | 0 | - | 0 | - |
| E012-T074-S121- | 3.382270038 | 20.18930842 | 2.27358621 | 0 | - | 0 | - |
| E013-T073-S115- | 3.386595191 | 27.09933957 | 2.679362741 | 5.205318905 | 0.50040378 | 0 | - |
| E012-T052-S117- | 3.442822187 | 21.0305296 | 2.30995587 | 0 | - | 0 | - |
| E014-T017-X001- | 3.53365041 | 24.27523988 | 2.47897973 | 0 | - | 0 | - |
| E013-T062-S195- | 3.568251638 | 8.592473522 | 1.070260755 | 22.84216414 | 2.383801167 | 0 | - |
| E011-T077-S186- | 3.633128941 | 27.70021184 | 2.631002647 | 126.2749127 | 4.779817433 | 0 | - |
| E012-T030-S135- | 3.641779248 | 21.21079128 | 2.258510973 | 0 | - | 0 | - |
| E011-T022-S202- | 3.654754708 | 20.42965732 | 2.202831731 | 0 | - | 0 | - |
| E013-T014-X001- | 3.663405015 | 19.22791278 | 2.116891825 | 0 | - | 0 | - |
| E013-T056-S104- | 3.732607471 | 19.82878505 | 2.137871516 | 11.88037491 | 1.444467414 | 0 | - |
| E013-T028-S186- | 3.771533853 | 21.99192524 | 2.268602263 | 173.7351733 | 5.194573127 | 0 | - |
| E011-T062-S186- | 3.775859006 | 68.98013709 | 3.87311334 | 333.262888 | 6.129079131 | 0 | - |
| E013-T015-S104- | 3.775859006 | 7.030205609 | 0.749676682 | 56.58487845 | 3.591857865 | 0 | - |
| E014-T060-S168- | 3.806135081 | 19.16782555 | 2.069106442 | 0 | - | 0 | - |
| E014-T014-S142- | 3.849386616 | 0 | - | 58.36081078 | 3.613636615 | 0 | - |
| E013-T010-S137- | 3.901288458 | 18.6270405 | 2.001609688 | 0 | - | 0 | - |
| E014-T002-S186- | 3.909938765 | 4.806978194 | 0.242082585 | 63.4436516 | 3.714261307 | 0 | - |
| E014-T057-S058- | 3.909938765 | 19.58843614 | 2.068057313 | 0 | - | 0 | - |
| E014-T019-X001- | 3.922914225 | 25.05637384 | 2.404051816 | 10.96178922 | 1.280848694 | 0 | - |
| E013-T029-S126- | 3.9531903 | 19.58843614 | 2.055404295 | 0 | -2.30835805 | 0 | -2.30835805 |
| E014-T073-S062- | 3.96616576 | 35.57163864 | 2.880520948 | 0 | - | 0 | - |
| E011-T077-S138- | 3.974816067 | 10.21482866 | 1.172692488 | 63.4436516 | 3.695323153 | 0 | - |
| E011-T073-S104- | 3.987791528 | 21.0305296 | 2.143031105 | 6.613816962 | 0.610218721 | 0 | - |
| E012-T011-S183- | 4.005092142 | 20.54983178 | 2.106208074 | 0 | - | 0 | - |
| E011-T009-S138- | 4.035368216 | 9.493781933 | 1.05936553 | 66.3831258 | 3.742218169 | 0 | - |
| E013-T051-S126- | 4.095920365 | 25.65724611 | 2.38711311 | 0 | - | 0 | - |
| E014-T044-S141- | 4.199724049 | 0 | - | 22.84216414 | 2.197008228 | 0 | - |
| E013-T065-S100- | 4.29920258 | 0 | -2.40577528 | 62.15763163 | 3.575109887 | 0 | -2.40577528 |
| E012-T005-S177- | 4.662515474 | 0 | - | 29.76217633 | 2.44164258 | 0 | - |
| E012-T057-S069- | 4.666840627 | 12.01744549 | 1.199829831 | 98.59486397 | 4.135454811 | 0 | - |
| E013-T006-S062- | 4.809570693 | 26.25811839 | 2.230182515 | 0 | - | 0 | - |
| E012-T032-S054- | 4.935000144 | 0 | - | 32.33421626 | 2.489683837 | 0 | - |
| E014-T026-S102- | 5.133957205 | 28.30108412 | 2.256064038 | 0 | - | 0 | - |
| E013-T007-S176- | 5.172883587 | 0 | - | 55.54381467 | 3.195352727 | 0 | - |
| E013-T022-S138- | 5.198834508 | 49.93248599 | 3.038517243 | 300.4387594 | 5.603724134 | 0 | - |
| E013-T020-S157- | 5.306963345 | 28.36117135 | 2.218892071 | 0 | - | 0 | - |
| E012-T028-S199- | 5.916809989 | 8.832822432 | 0.507498765 | 31.41563057 | 2.228510907 | 0 | - |
| E014-T073-S192- | 6.037914287 | 9.553869161 | 0.584552154 | 36.9271447 | 2.430010915 | 0 | - |
| E014-T042-S177- | 6.12874251 | 440.9200749 | 5.953994047 | 52.48186237 | 2.907330188 | 0 | - |
| E012-T061-S186- | 6.206595273 | 8.892909659 | 0.4570770 77 | 54.13531661 | 2.935586995 | 0 | - |
| E012-T076-5168- | 6.414202641 | 24.27523988 | 1.769361333 | 102.1467286 | 3.798262709 | 0 | - |
| E014-T057-S135- | 6.427178102 | 30.76466044 | 2.096536518 | 6.246382686 | - | 0 | - |
| E012-T046-S186- | 6.578558475 | 0 | - | 125.9687175 | 4.066405822 | 0 | - |
| E014-T068-S143- | 6.600184242 | 29.56291589 | 2.007675894 | 0 | - | 0 | - |
| E012-T077-S186- | 6.816441917 | 9.974479753 | 0.48956861 | 262.593029 | 5.075656372 | 0 | - |
| E014-T029-S186- | 7.019724132 | 15.50250468 | 1.041060491 | 105.5761152 | 3.732187731 | 0 | - |
| E014-T012-S176- | 7.599294701 | 0 | - | 36.49847138 | 2.124541543 | 0 | - |
| E012-T021-S186- | 8.261043186 | 19.10773832 | 1.118504203 | 125.7850004 | 3.775065552 | 0 | - |
| E013-T021-S186- | 8.745460378 | 9.073171341 | 0.047715714 | 84.08121008 | 3.126038307 | 0 | -3.28473034 |
| E013-T057-S186- | 9.57588985 | 28.18090966 | 1.464245801 | 135.5832477 | 3.690929583 | 0 | - |
| E014-T017-S186- | 15.04720903 | 87.06639254 | 2.456269168 | 261.8581604 | 4.033890217 | 0 | - |

**Table 19. Constructs with Particularly Noteworthy Enrichments in Libraries 1A and 1.1A.**

| | | | | | P3 | P4 |
|---|---|---|---|---|---|---|
| BlockSequence | Gene | Cytokine receptor? | Contains ITAM motif? | Gene | Cytokine receptor? | Contains ITAM motif? |
| M001-S116-S044 | IL6R | Y | N | CD8B | N | N |
| M024-S192-S045 | MYD88 | N | N | CD8B | N | N |
| M001-S047-S102 | CD27 | Y | N | IL1RL1 | Y | N |
| M048-S195-S043 | MYD88 | N | N | CD8A | N | N |
| M012-S216-S211 | TNFRSF18 | Y | N | TNFRSF4 | Y | N |
| M030-S170-S194 | IL31RA | Y | N | MYD88 | N | N |

**Table 20. Constructs with Particularly Noteworthy Enrichments in Libraries 2B and 2.1B.**

| | P3 | | | P4 | | |
|---|---|---|---|---|---|---|
| BlockSequence | Gene | Cytokine receptor? | Contains ITAM motif? | Gene | Cytokine receptor? | Contains ITAM motif? |
| M007-5049-5051 | CD28 | N | N | CD40 | Y | N |
| M007-S050-S039 | CD40 | Y | N | CD3G | N | Y |
| M012-S050-S043 | CD40 | Y | N | CD8A | N | N |
| M012-S161-S213 | IL22RA1 | Y | N | TNFRSF9 | Y | N |
| M030-S142-S049 | IL13RA2 | Y | N | CD28 | N | N |
| M001-S145-S130 | IL17RB | Y | N | IL10RB | Y | N |
| M018-S085-S039 | IFNGR2 | Y | N | CD3G | N | Y |
| M018-S075-S053 | FCGR2C | Y | Y | CD79B | N | Y |
| M012-S135-S074 | IL11RA | Y | N | FCER1G | Y | Y |
| M007-S214-S077 | TNFRSF14 | Y | N | GHR | Y | N |

**Table 21. Constructs with Particularly Noteworthy Enrichments in Libraries 3A and 3.1A.**

| | P3 | | | P4 | | |
|---|---|---|---|---|---|---|
| BlockSequence | Gene | Cytokine receptor? | Contains ITAM motif? | Gene | Cytokine receptor? | Contains ITAM motif? |
| E008/E013-T041-S186-S050 | MPL | Y | N | CD40 | Y | N |
| E006/E011-T077-S186-S211 | MPL | Y | N | TNFRSF4 | Y | N |
| E007/E012-T021-S186-S051 | MPL | Y | N | CD40 | Y | N |
| E009/E014-T041-S186-S053 | MPL | Y | N | CD79B | N | Y |
| E007/E012-T073-S186-S053 | MPL | Y | N | CD79B | N | Y |
| E006/E011-T017-S186-S051 | MPL | Y | N | CD40 | Y | N |
| E006/E011-T031-S186-S211 | MPL | Y | N | TNFRSF4 | Y | N |
| E006/E011-T011-S186-S050 | MPL | Y | N | CD40 | Y | N |
| E006/E011-T011-S186-S047 | MPL | Y | N | CD27 | Y | N |
| E007/E012-T001-S186-S050 | MPL | Y | N | CD40 | Y | N |
| E006/E011-T041-S186-S051 | MPL | Y | N | CD40 | Y | N |
| E008/E013-T028-S186-S076 | MPL | Y | N | FCGR2A | Y | Y |
| E009/E014-T029-S199-S053 | OSMR | Y | N | CD79B | N | Y |
| E009/E014-T062-S186-S216 | MPL | Y | N | TNFRSF18 | Y | N |
| E007/E012-T006-S058-S051 | CSF2RA | Y | N | CD40 | Y | N |
| E009/E014-T076-S186-S211 | MPL | Y | N | TNFRSF4 | Y | N |
| E007/E012-T001-S186-S047 | MPL | Y | N | CD27 | Y | N |

**Table 22. Constructs with Particularly Noteworthy Enrichments in Libraries 3B and 3.1B.**

| | P3 | | | P4 | | |
|---|---|---|---|---|---|---|
| BlockSequence | Gene | Cytokine receptor? | Contains ITAM motif? | Gene | Cytokine receptor? | Contains ITAM motif? |
| E007/E012-T017-S186-S051 | MPL | Y | N | CD40 | Y | N |
| E007/E012-T073-S186-S053 | MPL | Y | N | CD79B | N | Y |
| E008/E013-T028-S186-S047 | MPL | Y | N | CD27 | Y | N |
| E006/E011-T011-S186-S047 | MPL | Y | N | CD27 | Y | N |
| E007/E012-T082-S176-S214 | LEPR | Y | N | TNFRSF14 | Y | N |
| E006/E011-T046-S186-S052 | MPL | Y | N | CD79A | N | Y |
| E008/E013-T029-S186-S052 | MPL | Y | N | CD79A | N | Y |
| E009/E014-T011-S186-S053 | MPL | Y | N | CD79B | N | Y |
| E008/E013-T032-S186-S039 | MPL | Y | N | CD3G | N | Y |
| E007/E012-T034-S186-S051 | MPL | Y | N | CD40 | Y | N |
| E007/E012-T041-S192-S213 | MYD88 | N | N | TNFRSF9 | Y | N |
| E006/E011-T014-S069-S213 | EPOR | Y | N | TNFRSF9 | Y | N |
| E006/E011-T022-S186-S053 | MPL | Y | N | CD79B | N | Y |
| E006/E011-T023-S115-S075 | IL5RA | Y | N | FCGR2C | Y | Y |
| E006/E011-T029-S106-S213 | IL2RG | Y | N | TNFRSF9 | Y | N |
| E006/E011-T032-S155-S080 | IL18RAP | Y | N | ICOS | N | N |
| E006/E011-T041-S186-S216 | MPL | Y | N | TNFRSF18 | Y | N |
| E006/E011-T057-S135-S080 | IL11RA | Y | N | ICOS | N | N |
| E006/E011-T072-S191-X002 | MYD88 | N | N | N/A | N | N |
| E006/E011-T077-S186-S216 | MPL | Y | N | TNFRSF18 | Y | N |
| E006/E011-T080-S141-S080 | IL13RA1 | Y | N | ICOS | N | N |
| E007/E012-T001-X001-S214 | N/A | N | N | TNFRSF14 | Y | N |
| E007/E012-T007-S059-S211 | CSF2RA | Y | N | TNFRSF4 | Y | N |
| E007/E012-T016-S186-S052 | MPL | Y | N | CD79A | N | Y |
| E007/E012-T031-S186-S053 | MPL | Y | N | CD79B | N | Y |
| E007/E012-T044-S102-S052 | IL1RL1 | Y | N | CD79A | N | Y |
| E007/E012-T044-S142-X002 | IL13RA2 | Y | N | N/A | N/A | N/A |
| E007/E012-T055-S069-S053 | EPOR | Y | N | CD79B | N | Y |
| E007/E012-T063-S176-S216 | LEPR | Y | N | TNFRSF18 | Y | N |
| E007/E012-T065-S157-S075 | IL20RB | Y | N | FCGR2C | Y | Y |
| E008/E013-T008-S085-X002 | IFNGR2 | Y | N | N/A | N/A | N/A |
| E008/E013-T011-S085-S048 | IFNGR2 | Y | N | CD28 | N | N |
| E008/E013-T021-S109-X002 | IL3RA | Y | N | N/A | N/A | N/A |
| E008/E013-T021-S168-S211 | IL27RA | Y | N | TNFRSF4 | Y | N |
| E008/E013-T032-S064-S214 | CSF3R | Y | N | TNFRSF14 | Y | N |
| E008/E013-T037-S170-S215 | IL31RA | Y | N | TNFRSF18 | Y | N |
| E008/E013-T038-S176-S048 | LEPR | Y | N | CD28 | N | N |
| E008/E013-T039-S137-S216 | IL12RB1 | Y | N | TNFRSF18 | Y | N |
| E008/E013-T041-S141-S053 | IL13RA1 | Y | N | CD79B | N | Y |
| E008/E013-T045-S177-S048 | LEPR | Y | N | CD28 | N | N |
| E008/E013-T048-S109-S074 | IL3RA | Y | N | FCER1G | Y | Y |
| E008/E013-T073-S199-S075 | OSMR | Y | N | FCGR2C | Y | Y |
| E009/E014-T001-S157-S074 | IL20RB | Y | N | FCER1G | Y | Y |
| E009/E014-T005-S196-S049 | MYD88 | N | N | CD28 | N | N |
| E009/E014-T011-S130-X002 | IL10RB | Y | N | N/A | N/A | N/A |
| E009/E014-T013-S155-X002 | IL18RAP | Y | N | N/A | N/A | N/A |
| E009/E014-T017-S186-S076 | MPL | Y | N | FCGR2A | Y | Y |
| E009/E014-T021-S142-S080 | IL13RA2 | Y | N | ICOS | N | N |
| E009/E014-T023-S082-S076 | IFNAR2 | Y | N | FCGR2A | Y | Y |
| E009/E014-T038-S196-S037 | MYD88 | N | N | CD3D | N | Y |
| E009/E014-T055-S186-S052 | MPL | Y | N | CD79A | N | Y |
| E009/E014-T060-S175-S053 | LEPR | Y | N | CD79B | N | Y |
| E009/E014-T070-S085-S212 | IFNGR2 | Y | N | TNFRSF8 | Y | N |
| E008/E013-T026-S054-S213 | CRLF2 | Y | N | TNFRSF9 | Y | N |
| E009/E014-T007-S120-S053 | IL7R | Y | N | CD79B | N | Y |
| E007/E012-T045-S186-S211 | MPL | Y | N | TNFRSF4 | Y | N |
| E008/E013-T073-S186-X002 | MPL | Y | N | N/A | N/A | N/A |
| E008/E013-T074-S186-X002 | MPL | Y | N | N/A | N/A | N/A |
| E007/E012-T055-S186-S053 | MPL | Y | N | CD79B | N | Y |
| E008/E013-T036-S186-S053 | MPL | Y | N | CD79B | N | Y |
| E007/E012-T017-S058-S053 | CSF2RA | Y | N | CD79B | N | Y |
| E008/E013-T030-S189-S080 | MYD88 | N | N | ICOS | N | N |
| E006/E011-T029-S081-S047 | IFNAR1 | Y | N | CD27 | Y | N |
| E009/E014-T044-S194-S050 | MYD88 | N | N | CD40 | Y | N |
| E006/E011-T028-S121-X002 | IL7R | Y | N | N/A | N/A | N/A |
| E008/E013-T028-S186-S053 | MPL | Y | N | CD79B | N | Y |
| E009/E014-T078-S142-S213 | IL13RA2 | Y | N | TNFRSF9 | Y | N |
| E009/E014-T041-S186-S051 | MPL | Y | N | CD40 | Y | N |
| E008/E013-T006-S186-S050 | MPL | Y | N | CD40 | Y | N |
| E006/E011-T028-S186-S075 | MPL | Y | N | FCGR2C | Y | Y |
| E006/E011-T040-S120-S038 | IL7R | Y | N | CD3E | N | Y |
| E007/E012-T044-S115-S211 | IL5RA | Y | N | TNFRSF4 | Y | N |
| E009/E014-T039-S176-S075 | LEPR | Y | N | FCGR2C | Y | Y |
| E007/E012-T028-S186-S050 | MPL | Y | N | CD40 | Y | N |
| E008/E013-T031-S202-S050 | PRLR | Y | N | CD40 | Y | N |
| E007/E012-T072-S192-S053 | MYD88 | N | N | CD79B | N | Y |
| E006/E011-T065-X001-S051 | N/A | N/A | N/A | CD40 | Y | N |
| E007/E012-T030-S062-X002 | CSF3R | Y | N | N/A | N/A | N/A |
| E007/E012-T073-S186-X002 | MPL | Y | N | N/A | N/A | N/A |
| E009/E014-T056-S186-S053 | MPL | Y | N | CD79B | N | Y |
| E008/E013-T046-S137-X002 | IL12RB1 | Y | N | N/A | N/A | N/A |
| E006/E011-T016-S136-S076 | IL12RB1 | Y | N | FCGR2A | Y | Y |
| E007/E012-T032-S142-S037 | IL13RA2 | Y | N | CD3D | N | Y |
| E007/E012-T065-S120-S215 | IL7R | Y | N | TNFRSF18 | Y | N |
| E009/E014-T077-S186-S047 | MPL | Y | N | CD27 | Y | N |
| E009/E014-T001-S126-S051 | IL9R | Y | N | CD40 | Y | N |
| E006/E011-T030-S121-S039 | IL7R | Y | N | CD3G | N | Y |
| E008/E013-T006-S176-S213 | LEPR | Y | N | TNFRSF9 | Y | N |
| E009/E014-T032-S130-S215 | IL10RB | Y | N | TNFRSF18 | Y | N |
| E008/E013-T041-S186-S039 | MPL | Y | N | CD3G | N | Y |
| E009/E014-T021-S186-S047 | MPL | Y | N | CD27 | Y | N |
| E008/E013-T026-S137-S214 | IL12RB1 | Y | N | TNFRSF14 | Y | N |
| E007/E012-T029-S116-S075 | IL6R | Y | N | FCGR2C | Y | Y |
| E008/E013-T026-S106-S049 | IL2RG | Y | N | CD28 | N | N |
| E007/E012-T032-S168-S075 | IL27RA | Y | N | FCGR2C | Y | Y |
| E006/E011-T031-S186-S052 | MPL | Y | N | CD79A | N | Y |
| E007/E012-T043-S186-S051 | MPL | Y | N | CD40 | Y | N |
| E008/E013-T066-S130-S075 | IL10RB | Y | N | FCGR2C | Y | Y |
| E009/E014-T019-S143-S074 | IL15RA | Y | N | FCER1G | Y | Y |
| E006/E011-T010-S130-X002 | IL10RB | Y | N | N/A | N/A | N/A |
| E009/E014-T065-S083-S215 | IFNAR2 | Y | N | TNFRSF18 | Y | N |
| E007/E012-T041-S130-S075 | IL10RB | Y | N | FCGR2C | Y | Y |
| E007/E012-T044-S199-S053 | OSMR | Y | N | CD79B | N | Y |
| E007/E012-T075-S059-X002 | CSF2RA | Y | N | N/A | N/A | N/A |
| E008/E013-T015-S170-S214 | IL31RA | Y | N | TNFRSF14 | Y | N |
| E006/E011-T030-X001-S076 | N/A | N/A | N/A | FCGR2A | Y | Y |
| E009/E014-T072-S186-X002 | MPL | Y | N | N/A | N/A | N/A |
| E008/E013-T032-S176-S211 | LEPR | Y | N | TNFRSF4 | Y | N |
| E009/E014-T075-S136-S052 | IL12RB1 | Y | N | CD79A | N | Y |
| E007/E012-T041-S186-S052 | MPL | Y | N | CD79A | N | Y |
| E008/E013-T028-S186-S039 | MPL | Y | N | CD3G | N | Y |
| E008/E013-T062-S186-X002 | MPL | Y | N | N/A | N/A | N/A |
| E007/E012-T044-S186-S051 | MPL | Y | N | CD40 | Y | N |
| E007/E012-T014-S058-X002 | CSF2RA | Y | N | N/A | N/A | N/A |
| E008/E013-T001-S186-S075 | MPL | Y | N | FCGR2C | Y | Y |
| E009/E014-T029-S137-X002 | IL12RB1 | Y | N | N/A | N/A | N/A |
| E006/E011-T004-S194-S038 | MYD88 | N | N | CD3E | N | Y |
| E007/E012-T016-S186-X002 | MPL | Y | N | N/A | N/A | N/A |
| E009/E014-T031-S186-S075 | MPL | Y | N | FCGR2C | Y | Y |
| E009/E014-T069-S137-S039 | IL12RB1 | Y | N | CD3G | N | Y |
| E007/E012-T049-S143-S216 | IL15RA | Y | N | TNFRSF18 | Y | N |
| E007/E012-T028-S186-S053 | MPL | Y | N | CD79B | N | Y |
| E008/E013-T011-S186-S037 | MPL | Y | N | CD3D | N | Y |
| E009/E014-T031-S186-S039 | MPL | Y | N | CD3G | N | Y |
| E009/E014-T020-S186-S050 | MPL | Y | N | CD40 | Y | N |
| E008/E013-T016-S186-X002 | MPL | Y | N | N/A | N/A | N/A |
| E009/E014-T046-S186-S053 | MPL | Y | N | CD79B | N | Y |

**Table 23. Constructs with Particularly Noteworthy Enrichments in Libraries 4B and 4.1B.**

| | P3 | | | P4 | | |
|---|---|---|---|---|---|---|
| BlockSequence | Gene | Cytokine receptor? | Contains ITAM motif? | Gene | Cytokine receptor? | Contains ITAM motif? |
| E007/E012-T078-S154-S047 | IL18R1 | Y | N | CD27 | Y | N |
| E008/E013-T062-S186-X002 | MPL | Y | N | N/A | N/A | N/A |
| E008/E013-T055-S186-S050 | MPL | Y | N | CD40 | Y | N |
| E009/E014-T057-S186-S050 | MPL | Y | N | CD40 | Y | N |
| E007/E012-T077-S054-S053 | CRLF2 | Y | N | CD79B | N | Y |
| E007/E012-T034-S135-S211 | IL11RA | Y | N | TNFRSF4 | Y | N |
| E009/E014-T071-X001-S216 | N/A | N/A | N/A | TNFRSF18 | Y | N |
| E009/E014-T011-S141-S037 | IL13RA1 | Y | N | CD3D | N | Y |
| E008/E013-T041-S186-S037 | MPL | Y | N | CD3D | N | Y |
| E006/E011-T038-S106-S039 | IL2RG | Y | N | CD3G | N | Y |
| E006/E011-T011-S121-X002 | IL7R | Y | N | N/A | N/A | N/A |
| E007/E012-T007-S085-S215 | IFNGR2 | Y | N | TNFRSF18 | Y | N |
| E006/E011-T041-S186-S050 | MPL | Y | N | CD40 | Y | N |
| E007/E012-T008-S064-S051 | CSF3R | Y | N | CD40 | Y | N |
| E006/E011-T041-S186-S047 | MPL | Y | N | CD27 | Y | N |
| E008/E013-T045-S186-S051 | MPL | Y | N | CD40 | Y | N |
| E008/E013-T003-S104-S216 | IL2RA | Y | N | TNFRSF18 | Y | N |
| E006/E011-T019-S186-S053 | MPL | Y | N | CD79B | N | Y |
| E008/E013-T071-S064-S080 | CSF3R | Y | N | ICOS | N | N |
| E006/E011-T021-S054-X002 | CRLF2 | Y | N | N/A | N/A | N/A |
| E006/E011-T003-S135-X002 | IL11RA | Y | N | N/A | N/A | N/A |
| E009/E014-T020-S199-S213 | OSMR | Y | N | TNFRSF9 | Y | N |
| E008/E013-T027-S121-S211 | IL7R | Y | N | TNFRSF4 | Y | N |
| E009/E014-T032-S195-X002 | MYD88 | N | N | N/A | N/A | N/A |
| E009/E014-T050-S171-X002 | IL31RA | Y | N | N/A | N/A | N/A |
| E008/E013-T069-S083-X002 | IFNAR2 | Y | N | N/A | N/A | N/A |
| E008/E013-T026-S116-S038 | IL6R | Y | N | CD3E | N | Y |
| E009/E014-T072-S195-X002 | MYD88 | N | N | N/A | N/A | N/A |
| E007/E012-T047-S058-S211 | CSF2RA | Y | N | TNFRSF4 | Y | N |
| E008/E013-T046-S142-S080 | IL13RA2 | Y | N | ICOS | N | N |
| E006/E011-T065-S186-S076 | MPL | Y | N | FCGR2A | Y | Y |
| E006/E011-T062-S069-X002 | EPOR | Y | N | N/A | N/A | N/A |
| E007/E012-T047-S098-X002 | IL1R1 | Y | N | N/A | N/A | N/A |
| E009/E014-T069-S099-S048 | IL1R1 | Y | N | CD28 | N | N |
| E008/E013-T039-S141-S050 | IL13RA1 | Y | N | CD40 | Y | N |
| E006/E011-T052-S130-S052 | IL10RB | Y | N | CD79A | N | Y |
| E008/E013-T041-S186-X002 | MPL | Y | N | N/A | N/A | N/A |
| E007/E012-T019-S120-X002 | IL7R | Y | N | N/A | N/A | N/A |
| E008/E013-T045-S186-S053 | MPL | Y | N | CD79B | N | Y |
| E006/E011-T003-S170-S039 | IL31RA | Y | N | CD3G | N | Y |
| E007/E012-T047-S058-S051 | CSF2RA | Y | N | CD40 | Y | N |
| E007/E012-T069-S109-X002 | IL3RA | Y | N | N/A | N/A | N/A |
| E009/E014-T019-S130-S075 | IL10RB | Y | N | FCGR2C | Y | Y |
| E006/E011-T047-S054-S053 | CRLF2 | Y | N | CD79B | N | Y |

## Claims

1. Use of replication incompetent recombinant retroviral particles for genetically modifying T cells, comprising: contacting peripheral blood mononuclear cells comprising the T cells ex vivo without requiring prior activation, with replication incompetent recombinant retroviral particles to form a reaction mixture, wherein the replication incompetent recombinant retroviral particles comprise:
a) a pseudotyping element on their surface, and
b) a polypeptide on their surface, wherein the polypeptide is a cytokine or a cytokine fusion polypeptide, or is capable of binding to CD3 and/or CD28,
wherein said contacting facilitates transduction of the T cells by the replication incompetent recombinant retroviral particles, thereby producing genetically modified T cells, and wherein the contacting is performed for less than 10 hours before the T cells are washed.

2. The use of claim 1, wherein the replication incompetent recombinant retroviral particles comprise a polynucleotide comprising one or more transcriptional units, wherein each transcriptional unit is operatively linked to a promoter active in T cells, wherein the one or more transcriptional units encode a first polypeptide, wherein the first polypeptide comprises:
a) an antigen-specific targeting region, a transmembrane domain, and an intracellular activating domain; or
b) a lymphoproliferative element comprising a chimeric cytokine receptor comprising an intracellular signaling domain that promotes proliferation and/or survival of T cells.

3. The use of claim 2, wherein the polypeptide on their surface is fused to a heterologous membrane attachment sequence.

4. The use of claim 1, wherein the polypeptide is capable of binding to CD3.

5. The use of claim 4, wherein the replication incompetent recombinant retroviral particles comprise a polynucleotide comprising one or more transcriptional units, wherein each transcriptional unit is operatively linked to a promoter active in T cells, wherein the one or more transcriptional units encode a first polypeptide, wherein:
a) the first polypeptide comprises an antigen-specific targeting region, a transmembrane domain, and an intracellular activating domain;
b) the first polypeptide comprises a lymphoproliferative element comprising a chimeric cytokine receptor comprising an intracellular signaling domain that promotes proliferation and/or survival of T cells; or
c) wherein the one or more transcriptional units encode a second polypeptide, wherein the first polypeptide comprises a chimeric antigen receptor comprising an antigen-specific targeting region, a transmembrane domain, and an intracellular activating domain or the first polypeptide comprises a defined TCR, and wherein the second polypeptide comprises a lymphoproliferative element comprising a chimeric cytokine receptor comprising an intracellular signaling domain that promotes proliferation and/or survival of T cells.

6. The use of any one of claims 1 to 5, wherein the polypeptide on their surface comprises an antibody.

7. The use of claim 6, wherein the antibody is an scFv.

8. The use of any one of claims 1 to 5, wherein the polypeptide on their surface comprises an anti-CD3 scFv.

9. The use of any one of claims 1 to 5, wherein the polypeptide on their surface is the cytokine fusion polypeptide, wherein the cytokine fusion polypeptide comprises IL-7.

10. The use of claim 9, wherein the cytokine fusion polypeptide comprises the amino acid sequence of SEQ ID NO:286.

11. The use of any one of claims 1 to 5, wherein the polypeptide on their surface is the cytokine fusion polypeptide, wherein the cytokine fusion polypeptide comprises IL-15.

12. The use of any one of claims 1 to 5, wherein the contacting is performed for less than 6 hours.

13. The use of any one of claims 2 to 3, wherein one or more transcriptional units encode a second polypeptide, wherein the first polypeptide comprises a chimeric antigen receptor comprising an antigen-specific targeting region, a transmembrane domain, and an intracellular activating domain or the first polypeptide comprises a defined TCR, and wherein the second polypeptide comprises a lymphoproliferative element comprising a chimeric cytokine receptor comprising an intracellular signaling domain that promotes proliferation and/or survival of T cells.

14. The use of any one of claims 2 or 3, wherein the replication incompetent recombinant retroviral particles encode the lymphoproliferative element comprising the chimeric cytokine receptor, wherein the chimeric cytokine receptor of the lymphoproliferative element is constitutively active.

15. The use of any one of claims 1 to 5, wherein the T cells comprise resting T cells, and wherein the T cells are not incubated with or exposed to bovine serum before or during the genetically modifying.

16. The use of any one of claims 2 or 3, wherein the replication incompetent recombinant retroviral particles encode the lymphoproliferative element comprising the chimeric cytokine receptor, wherein the chimeric cytokine receptor comprising an intracellular signaling domain that promotes proliferation of T cells comprises an intracellular signaling domain from CD2, CD3D, CD3E, CD3G, CD4, CD8A, CD8B, CD27, CD28, CD40, CD79A, CD79B, CRLF2, CFS2RB, CSF2RA, CSF3R, EPOR, FCER1G, FCGR2C, FCGRA2, GHR, ICOS, IFNAR1, IFNAR2, IFNGR1, IFNGR2, IFNLR1, IL1R1, IL1RAP, IL1RL1, IL1RL2, IL2RA, IL2RB, IL2RG, IL3RA, IL4R, IL5RA, IL6R, IL6ST, IL7RA, IL9R, IL10RA, IL10RB, IL11RA, IL12RB1, IL12RB2, IL13RA1, IL13RA2, IL15RA, IL17RA, IL17RB, IL17RC, IL17RD, IL17RE, IL18R1, IL18RAP, IL20RA, IL20RB, IL21R, IL22RA1, IL23R, IL27RA, IL31RA, LEPR, LIFR, LMP1, MPL, MYD88, OSMR, PRLR, TNFRSF4, TNFRSF8, TNFRSF9, TNFRSF14, or TNFRSF18.

17. The use of any one of claims 2 or 3, wherein the replication incompetent recombinant retroviral particles encode the lymphoproliferative element comprising the chimeric cytokine receptor, wherein the lymphoproliferative element is a chimeric lymphoproliferative element further comprising a second intracellular signaling domain.

## Patentansprüche

1. Verwendung von replikationsinkompetenten rekombinanten retroviralen Partikeln zur genetischen Modifizierung von T-Zellen, umfassend: Kontaktieren von peripheren mononukleären Blutzellen, die die T-Zellen umfassen, ex vivo, ohne eine vorherige Aktivierung zu erfordern, mit replikationsinkompetenten rekombinanten retroviralen Partikeln, um ein Reaktionsgemisch zu bilden, wobei die replikationsinkompetenten rekombinanten retroviralen Partikel umfassen:
a) ein Pseudotypisierungselement auf ihrer Oberfläche und
b) ein Polypeptid auf ihrer Oberfläche, wobei das Polypeptid ein Zytokin oder ein Zytokinfusionspolypeptid ist oder imstande ist, an CD3 und/oder CD28 zu binden,
wobei das Kontaktieren die Transduktion der T-Zellen durch die replikationsinkompetenten rekombinanten retroviralen Partikel erleichtert, wodurch genetisch modifizierte T-Zellen produziert werden und wobei das Kontaktieren für weniger als 10 Stunden durchgeführt wird, bevor die T-Zellen gewaschen werden.

2. Verwendung nach Anspruch 1, wobei die replikationsinkompetenten rekombinanten retroviralen Partikel ein Polynukleotid umfassen, das eine oder mehrere Transkriptionseinheiten umfasst, wobei jede Transkriptionseinheit operativ mit einem in T-Zellen aktiven Promotor verbunden ist, wobei die eine oder mehreren Transkriptionseinheiten ein erstes Polypeptid kodieren, wobei das erste Polypeptid umfasst:
a) eine antigenspezifische Targeting-Region, eine Transmembrandomäne und eine intrazelluläre Aktivierungsdomäne; oder
b) ein lymphoproliferatives Element, umfassend einen chimären Zytokinrezeptor umfassend eine intrazelluläre Signaldomäne, die die Proliferation und/oder das Überleben von T-Zellen fördert.

3. Verwendung nach Anspruch 2, wobei das Polypeptid auf ihrer Oberfläche an eine heterologe Membrananheftungssequenz fusioniert ist.

4. Verwendung nach Anspruch 1, wobei das Polypeptid imstande ist, an CD3 zu binden.

5. Verwendung nach Anspruch 4, wobei die replikationsinkompetenten rekombinanten retroviralen Partikel ein Polynukleotid umfassen, das eine oder mehrere Transkriptionseinheiten umfasst, wobei jede Transkriptionseinheit operativ mit einem in T-Zellen aktiven Promotor verbunden ist, wobei die eine oder mehreren Transkriptionseinheiten ein erstes Polypeptid kodieren, wobei:
a) das erste Polypeptid eine antigenspezifische Targeting-Region, eine Transmembrandomäne und eine intrazelluläre Aktivierungsdomäne umfasst;
b) das erste Polypeptid ein lymphoproliferatives Element umfassend einen chimären Zytokinrezeptor umfassend eine intrazelluläre Signaldomäne, die die Proliferation und/oder das Überleben von T-Zellen fördert, umfasst; oder
c) wobei die eine oder mehreren Transkriptionseinheiten ein zweites Polypeptid kodieren, wobei das erste Polypeptid einen chimären Antigenrezeptor umfasst, der eine antigenspezifische Targeting-Region, eine Transmembrandomäne und eine intrazelluläre Aktivierungsdomäne umfasst oder das erste Polypeptid einen definierten TCR umfasst und wobei das zweite Polypeptid ein lymphoproliferatives Element umfassend einen chimären Zytokinrezeptor umfassend eine intrazelluläre Signaldomäne, die die Proliferation und/oder das Überleben von T-Zellen fördert, umfasst.

6. Verwendung nach einem der Ansprüche 1 bis 5, wobei das Polypeptid auf ihrer Oberfläche einen Antikörper umfasst.

7. Verwendung nach Anspruch 6, wobei der Antikörper ein scFv ist.

8. Verwendung nach einem der Ansprüche 1 bis 5, wobei das Polypeptid auf ihrer Oberfläche ein Anti-CD3-scFv umfasst.

9. Verwendung nach einem der Ansprüche 1 bis 5, wobei das Polypeptid auf ihrer Oberfläche das Zytokinfusionspolypeptid ist, wobei das Zytokinfusionspolypeptid IL-7 umfasst.

10. Verwendung nach Anspruch 9, wobei das Zytokinfusionspolypeptid die Aminosäuresequenz von SEQ ID NO: 286 umfasst.

11. Verwendung nach einem der Ansprüche 1 bis 5, wobei das Polypeptid auf ihrer Oberfläche das Zytokinfusionspolypeptid ist, wobei das Zytokinfusionspolypeptid IL-15 umfasst.

12. Verwendung nach einem der Ansprüche 1 bis 5, wobei das Kontaktieren für weniger als 6 Stunden durchgeführt wird.

13. Verwendung nach einem der Ansprüche 2 bis 3, wobei eine oder mehrere Transkriptionseinheiten ein zweites Polypeptid kodieren, wobei das erste Polypeptid einen chimären Antigenrezeptor umfassend eine antigenspezifische Targeting-Region, eine Transmembrandomäne und eine intrazelluläre Aktivierungsdomäne umfasst oder das erste Polypeptid einen definierten TCR umfasst und wobei das zweite Polypeptid ein lymphoproliferatives Element umfassend einen chimären Zytokinrezeptor umfassend eine intrazelluläre Signaldomäne, die die Proliferation und/oder das Überleben von T-Zellen fördert, umfasst.

14. Verwendung nach einem der Ansprüche 2 oder 3, wobei die replikationsinkompetenten rekombinanten retroviralen Partikel das lymphoproliferative Element kodieren, das den chimären Zytokinrezeptor umfasst, wobei der chimäre Zytokinrezeptor des lymphoproliferativen Elements konstitutiv aktiv ist.

15. Verwendung nach einem der Ansprüche 1 bis 5, wobei die T-Zellen ruhende T-Zellen umfassen und wobei die T-Zellen vor oder während dem genetischen Modifizieren nicht mit Rinderserum inkubiert oder diesem ausgesetzt sind.

16. Verwendung nach einem der Ansprüche 2 oder 3, wobei die replikationsinkompetenten rekombinanten retroviralen Partikel das lymphoproliferative Element kodieren, das den chimären Zytokinrezeptor umfasst, wobei der chimäre Zytokinrezeptor umfassend eine intrazelluläre Signaldomäne, die die Proliferation von T-Zellen fördert, eine intrazelluläre Signaldomäne von CD2, CD3D, CD3E, CD3G, CD4, CD8A, CD8B, CD27, CD28, CD40, CD79A, CD79B, CRLF2, CFS2RB, CSF2RA, CSF3R, EPOR, FCER1G, FCGR2C, FCGRA2, GHR, ICOS, IFNAR1, IFNAR2, IFNGR1, IFNGR2, IFNLR1, IL1R1, IL1RAP, IL1RL1, IL1RL2, IL2RA, IL2RB, IL2RG, IL3RA, IL4R, IL5RA, IL6R, IL6ST, IL7RA, IL9R, IL10RA, IL10RB, IL11RA, IL12RB1, IL12RB2, IL13RA1, IL13RA2, IL15RA, IL17RA, IL17RB, IL17RC, IL17RD, IL17RE, IL18R1, IL18RAP, IL20RA, IL20RB, IL21R, IL22RA1, IL23R, IL27RA, IL31RA, LEPR, LIFR, LMP1, MPL, MYD88, OSMR, PRLR, TNFRSF4, TNFRSF8, TNFRSF9, TNFRSF14 oder TNFRSF18 umfasst.

17. Verwendung nach einem der Ansprüche 2 oder 3, wobei die replikationsinkompetenten rekombinanten retroviralen Partikel das lymphoproliferative Element kodieren, das den chimären Zytokinrezeptor umfasst, wobei das lymphoproliferative Element ein chimäres lymphoproliferatives Element ist, das ferner eine zweite intrazelluläre Signaldomäne umfasst.

## Revendications

1. Utilisation de particules rétrovirales recombinantes incapables de se répliquer pour la modification génétique de lymphocytes T, comprenant : la mise en contact ex vivo, sans nécessiter d'activation préalable, de cellules mononuclées du sang périphérique comprenant les lymphocytes T avec des particules rétrovirales recombinantes incapables de se répliquer pour former un mélange réactionnel, dans laquelle les particules rétrovirales recombinantes incapables de se répliquer comprennent :
a) un élément de pseudotypage à leur surface, et
b) un polypeptide à leur surface, ledit polypeptide étant une cytokine ou un polypeptide de fusion de cytokine, ou étant capable de se lier à CD3 et/ou CD28,
dans laquelle ladite mise en contact facilite la transduction des lymphocytes T par les particules rétrovirales recombinantes incapables de se répliquer, produisant ainsi des lymphocytes T génétiquement modifiés, et dans laquelle la mise en contact est effectuée pendant moins de 10 heures avant que les lymphocytes T ne soient lavés.

2. Utilisation de la revendication 1, dans laquelle les particules rétrovirales recombinantes incapables de se répliquer comprennent un polynucléotide comprenant une ou plusieurs unités transcriptionnelles, dans laquelle chaque unité transcriptionnelle est fonctionnellement liée à un promoteur actif dans les lymphocytes T, dans laquelle la ou les unités transcriptionnelles codent pour un premier polypeptide, dans laquelle le premier polypeptide comprend :
a) une région de ciblage spécifique d'un antigène, un domaine transmembranaire et un domaine d'activation intracellulaire ; ou
b) un élément lymphoprolifératif comprenant un récepteur de cytokine chimérique comprenant un domaine de signalisation intracellulaire qui favorise la prolifération et/ou la survie de lymphocytes T.

3. Utilisation de la revendication 2, dans laquelle le polypeptide à leur surface est fusionné à une séquence de fixation membranaire hétérologue.

4. Utilisation de la revendication 1, dans laquelle le polypeptide est capable de se lier à CD3.

5. Utilisation de la revendication 4, dans laquelle les particules rétrovirales recombinantes incapables de se répliquer comprennent un polynucléotide comprenant une ou plusieurs unités transcriptionnelles, dans laquelle chaque unité transcriptionnelle est fonctionnellement liée à un promoteur actif dans les lymphocytes T, dans laquelle la ou les unités transcriptionnelles codent pour un premier polypeptide, dans laquelle :
a) le premier polypeptide comprend une région de ciblage spécifique d'un antigène, un domaine transmembranaire et un domaine d'activation intracellulaire ;
b) le premier polypeptide comprend un élément lymphoprolifératif comprenant un récepteur de cytokine chimérique comprenant un domaine de signalisation intracellulaire qui favorise la prolifération et/ou la survie des lymphocytes T ; ou
c) dans laquelle la ou les unités transcriptionnelles codent pour un second polypeptide, dans laquelle le premier polypeptide comprend un récepteur d'antigène chimérique comprenant une région de ciblage spécifique d'un antigène, un domaine transmembranaire et un domaine d'activation intracellulaire ou le premier polypeptide comprend un TCR défini, et dans laquelle le second polypeptide comprend un élément lymphoprolifératif comprenant un récepteur de cytokine chimérique comprenant un domaine de signalisation intracellulaire qui favorise la prolifération et/ou la survie des lymphocytes T.

6. Utilisation de l'une quelconque des revendications 1 à 5, dans laquelle le polypeptide à leur surface comprend un anticorps.

7. Utilisation de la revendication 6, dans laquelle l'anticorps est un scFv.

8. Utilisation de l'une quelconque des revendications 1 à 5, dans laquelle le polypeptide à leur surface comprend un scFv anti-CD3.

9. Utilisation de l'une quelconque des revendications 1 à 5, dans laquelle le polypeptide à leur surface est le polypeptide de fusion de cytokine, dans laquelle le polypeptide de fusion de cytokine comprend l'IL-7.

10. Utilisation de la revendication 9, dans laquelle le polypeptide de fusion de cytokine comprend la séquence d'acides aminés de SEQ ID N° : 286.

11. Utilisation de l'une quelconque des revendications 1 à 5, dans laquelle le polypeptide à leur surface est le polypeptide de fusion de cytokine, dans laquelle le polypeptide de fusion de cytokine comprend l'IL-15.

12. Utilisation de l'une quelconque des revendications 1 à 5, dans laquelle la mise en contact est effectuée pendant moins de 6 heures.

13. Utilisation de l'une quelconque des revendications 2 à 3, dans laquelle une ou plusieurs unités transcriptionnelles codent pour un second polypeptide, dans laquelle le premier polypeptide comprend un récepteur d'antigène chimérique comprenant une région de ciblage spécifique d'un antigène, un domaine transmembranaire et un domaine d'activation intracellulaire ou le premier polypeptide comprend un TCR défini, et dans laquelle le second polypeptide comprend un élément lymphoprolifératif comprenant un récepteur de cytokine chimérique comprenant un domaine de signalisation intracellulaire qui favorise la prolifération et/ou la survie des lymphocytes T.

14. Utilisation de l'une quelconque des revendications 2 ou 3, dans laquelle les particules rétrovirales recombinantes incapables de se répliquer codent pour l'élément lymphoprolifératif comprenant le récepteur de cytokine chimérique, dans laquelle le récepteur de cytokine chimérique de l'élément lymphoprolifératif est constitutivement actif.

15. Utilisation de l'une quelconque des revendications 1 à 5, dans laquelle les lymphocytes T comprennent des lymphocytes T au repos, et dans laquelle les lymphocytes T ne sont pas incubés avec ou exposés au sérum bovin avant ou pendant la modification génétique.

16. Utilisation de l'une quelconque des revendications 2 ou 3, dans laquelle les particules rétrovirales recombinantes incapables de se répliquer codent pour l'élément lymphoprolifératif comprenant le récepteur de cytokine chimérique, dans laquelle le récepteur de cytokine chimérique comprenant un domaine de signalisation intracellulaire qui favorise la prolifération des lymphocytes T comprend un domaine de signalisation intracellulaire provenant de CD2, CD3D, CD3E, CD3G, CD4, CD8A, CD8B, CD27, CD28, CD40, CD79A, CD79B, CRLF2, CFS2RB, CSF2RA, CSF3R, EPOR, FCER1G, FCGR2C, FCGRA2, GHR, ICOS, IFNAR1, IFNAR2, IFNGR1, IFNGR2, IFNLR1, IL1R1, IL1RAP, IL1RL1, IL1RL2, IL2RA, IL2RB, IL2RG, IL3RA, IL4R, IL5RA, IL6R, IL6ST, IL7RA, IL9R, IL10RA, IL10RB, IL11RA, IL12RB1, IL12RB2, IL13RA1, IL13RA2, IL15RA, IL17RA, IL17RB, IL17RC, IL17RD, IL17RE, IL18R1, IL18RAP, IL20RA, IL20RB, IL21R, IL22RA1, IL23R, IL27RA, IL31RA, LEPR, LIFR, LMP1, MPL, MYD88, OSMR, PRLR, TNFRSF4, TNFRSF8, TNFRSF9, TNFRSF14, ou TNFRSF18.

17. Utilisation de l'une quelconque des revendications 2 ou 3, dans laquelle les particules rétrovirales recombinantes incapables de se répliquer codent pour l'élément lymphoprolifératif comprenant le récepteur de cytokine chimérique, dans laquelle l'élément lymphoprolifératif est un élément lymphoprolifératif chimérique comprenant en outre un second domaine de signalisation intracellulaire.
